# EUROPEAN PATENT SPECIFICATION

(11) **EP 4 188 924 B1**
(45) Date of publication and mention of the grant of the patent: **18.06.2025**
(21) Application number: 21752127.7
(22) Date of filing: 30.07.2021
(51) Int. Cl.: C07D 417/14, C07D 471/04, C07D 471/10, C07D 487/04, C07D 487/08, C07D 487/10, C07D 491/048, C07D 491/10, C07D 498/04, C07D 498/10, A61K 31/5377, A61P 35/00

(54) **ANTAGONIST COMPOUNDS**
ANTAGONISTISCHE VERBINDUNGEN
COMPOSÉS ANTAGONISTES

(30) Priority: 31.07.2020 GB 202011996
(43) Date of publication of application: 07.06.2023
(73) Proprietor: Adorx therapeutics Limited, Edinburgh EH2 4JY (GB)
(72) Inventor: MCCARTHY, Clive, Edinburgh EH2 4JY (GB); MOULTON, Ben, Alderley Edge Mereside Cheshire SK10 4TG (GB); WALKER, Edward, Richard, Abingdon Oxfordshire OX14 4RZ (GB); MCMAHON, Pearl, Siobhan, Abingdon Oxfordshire OX14 4RZ (GB)
(74) Representative: HGF
(86) International application number: PCT/GB2021/051984
(87) International publication number: WO 2022/023772

(56) References cited:
- EP-A1- 1 205 478
- EP-A1- 1 700 856
- WO-A1-2020/010197
- WO-A2-01/74811
- US-A1- 2005 080 113

## Description

### INTRODUCTION

The present invention relates to certain compounds that function as antagonists of the adenosine A2a receptor. Additionally, some of the compounds are also antagonists of both A2a and the A2b receptor. The present invention also relates to processes for the preparation of these compounds, to pharmaceutical compositions comprising them, and to their use in the treatment of diseases or conditions in which adenosine A2a and/or A2b receptor activity is implicated, such as, for example, cancer.

### BACKGROUND OF THE INVENTION

A number of immunosuppressive pathways are active in the tumour microenvironment which enable tumour cells to evade elimination by cytotoxic T cells and can diminish the clinical response of patients to immunotherapy with anti-checkpoint antibodies. The anti-PD-1 antibodies pembrolizumab and nivolumab and anti-PD-L1 antibodies durvalumab, avelumab and atezolizumab are approved for the treatment of number of solid tumours including non-small cell lung cancer, head and neck squamous cancer and urothelial cancer. However, only 20-30% of patients respond to checkpoint blockade and the side effects of such treatments are significant (Sukari et al, 2016). Consequently, other approaches to enhance the cytotoxic potential of the tumour microenvironment are actively being investigated. This includes agents that could be used as monotherapies or, more likely, used in combination with checkpoint inhibitors and cytotoxic agents to enhance their efficacy.

One approach that has attracted attention is to interfere with the production and/or action of adenosine in the tumour microenvironment (Vijayan et al, 2017). Adenosine has immunosuppressive properties and is present in the tumour microenvironment at high concentrations. Recent studies estimate the concentration of adenosine to be about 10µM in human tumours compared to <1 µM in normal tissue (Houthuys et al 2017). Adenosine is formed at both intracellular and extracellular sites by two distinct pathways that involve two different substrates. Intracellular adenosine is derived from AMP and S-adenosyl homocysteine whilst the high extracellular adenosine concentrations observed during metabolic stress are associated with the release and degradation of precursor adenine nucleotides (ATP, ADP and AMP) by the concerted action of CD39 and CD73 (Vijayan et al, 2017).

CD39 and CD73 are upregulated in the tumour microenvironment in response to hypoxia. CD73 represents a putative patient stratification method for adenosine antagonists as its expression on tumour cells is also associated with poor overall prognosis in many different cancer types suggesting that adenosine production contributes to the undesirable immunosuppressive phenotype of the tumour microenvironment (Gao et al 2014; Loi et al, 2013;). CD73 expression by tumour-infiltrating immune cells is also important in promoting tumour immune suppression as CD73 negative Treg cells fail to suppress effector T cell functions (Deaglio et al, 2007; Reinhardt et al, (2017). Furthermore, patients resistant to anti-PD1 treatment have elevated levels of CD73 (Reinhardt et al, 2017).

Adenosine regulates cell function via occupancy of specific GPCRs on the cell surface of the P1 purinoceptor subtypes. The P1 receptor family is further subdivided into A1, A2a, A2b and A3.

A2 receptors are subdivided into A2a and A2b, based on high and low affinity for adenosine, respectively. A2a is expressed by lymphocytes and activation of A2a leads to suppression of cytokine production and other effector functions. Tumour growth is inhibited by genetic ablation of A2a in syngeneic mouse models and this effect has been demonstrated to be due to enhanced lymphocyte activation and cytotoxic function (Ohta et al, 2006; Waickman et al 2012; Beavis et al, 2013; Mittal et al, 2014; Cekic et al, 2014). A2a-/- mice show an increased response to inhibition of checkpoint pathways such as PD-1, with an improvement in both tumour free survival and overall survival. Adenosine-mediated A2a activation also limits the efficacy of ant-CTLA4 treatment (lannone et al, 2014).

The effects of genetic deficiency of A2a in mouse models is mimicked by pharmacological blockade of A2a. A2a antagonists have been shown to enhance the cytotoxic CD8+T cells and to enhance the ability of NK cells prevent metastasis of CD73-expressing tumours (Beavis et al, 2013). Importantly, A2a antagonists enhance the efficacy of anti-PD1 antibodies (Beavis et al, 2015).

These findings have prompted the development of selective A2a antagonists for use in cancer immunotherapy and clinical trials are ongoing with CPI-444, the first selective A2a antagonist to be evaluated in cancer, being used as both as a monotherapy and in combination with the anti-PDL1 antibody atezolizumab. The preliminary data indicated that the compound was well tolerated and showed early indications of reducing tumour size and enhancing CD8+T infiltration into tumour tissue.

The adenosine A2b receptor also plays a key role in cancer progression through modulation of the tumour microenvironment. A2b is expressed by cells of myeloid origin and the high concentrations of adenosine found within the tumour microenvironment can modify the behaviour of tumour associated macrophages, myeloid-derived suppressor cells and dendritic cells. Activation of A2b leads to polarisation of immunosuppressive M2 macrophages (Csoka et al, 2012), formation of myeloid-derived suppressive cells (Rhyzov et al, 2011) and promotes the immunosuppressive and pro-angiogenic phenotype of dendritic cells (Novitskiy et al, 2008; Wilson et al, 2009). Blockade of A2b has been shown to reduce tumour size in a number of mouse cancer models (Cekic et al, 2012; Sorrentino et al, 2015; lannone et al, 2013), an effect associated with diminished infiltration of MDSCs and production of VEGF (Sorrentino et al, 2015; lannone et al, 2013).

Consequently, dual inhibition of both A2a and A2b is an attractive approach to reduce immunosuppression within the tumour microenvironment to inhibit growth.

### SUMMARY OF THE INVENTION

According to a first aspect of the present invention, there is provided a compound, or a pharmaceutically acceptable salt, hydrate or solvate thereof, as defined herein.

According to a further aspect of the present invention, there is provided a pharmaceutical composition comprising a compound as defined herein, or a pharmaceutically acceptable salt, hydrate or solvate thereof, in admixture with a pharmaceutically acceptable diluent or carrier.

According to a further aspect of the present invention, there is provided a method of antagonising adenosine A2a receptors, or A2a and A2b receptors, *in vitro* or *in vivo,* said method comprising contacting a cell with an effective amount of a compound or a pharmaceutically acceptable salt, hydrate or solvate thereof as defined herein.

According to a further aspect of the present invention, there is provided a method of selectively antagonising adenosine A2a receptors, or A2a and A2b receptors *in vitro* or *in vivo,* said method comprising contacting a cell with an effective amount of a compound, or a pharmaceutically acceptable salt, hydrate or solvate thereof, as defined herein.

According to a further aspect of the present invention, there is provided a method of inhibiting cell proliferation, *in vitro* or *in vivo,* said method comprising contacting a cell with an effective amount of a compound or a pharmaceutically acceptable salt, hydrate or solvate thereof as defined herein, or a pharmaceutical composition as defined herein. Suitably, the compound or pharmaceutical composition is administered in combination with one or more additional anti proliferative agents (e.g. checkpoint inhibitors and/or cytotoxic agents).

According to a further aspect of the present invention, there is provided a method of treating a disease or disorder associated with adenosine A2a and/or A2b receptor activity in a patient in need of such treatment, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a method of treating a proliferative disorder in a patient in need of such treatment, said method comprising administering to said patient a therapeutically effective amount of a compound or a pharmaceutically acceptable salt, hydrate or solvate thereof as defined herein, or a pharmaceutical composition as defined herein. Suitably, the compound or pharmaceutical composition is administered in combination with one or more additional antiproliferative agents (e.g. checkpoint inhibitors and/or cytotoxic agents).

According to a further aspect of the present invention, there is provided a method of treating cancer in a patient in need of such treatment, said method comprising administering to said patient a therapeutically effective amount of a compound or a pharmaceutically acceptable salt, hydrate or solvate thereof as defined herein, or a pharmaceutical composition as defined herein. Suitably, the compound or pharmaceutical composition is administered in combination with one or more additional anticancer agents (e.g. checkpoint inhibitors and/or cytotoxic agents).

According to a further aspect of the present invention, there is provided a compound, or a pharmaceutically acceptable salt, hydrate or solvate thereof, or a pharmaceutical composition as defined herein for use in therapy.

According to a further aspect of the present invention, there is provided a compound or a pharmaceutically acceptable salt, hydrate or solvate thereof as defined herein, or a pharmaceutical composition as defined herein, for use in the treatment of a proliferative condition. Suitably, the compound or pharmaceutical composition is administered in combination with one or more additional antiproliferative agents (e.g. checkpoint inhibitors and/or cytotoxic agents).

According to a further aspect of the present invention, there is provided a compound, or a pharmaceutically acceptable salt, hydrate or solvate thereof, or a pharmaceutical composition as defined herein for use in the treatment of cancer. In a particular embodiment, the cancer is human cancer. Suitably, the compound or pharmaceutical composition is administered in combination with one or more additional anticancer agents (e.g. checkpoint inhibitors and/or cytotoxic agents).

According to a further aspect of the present invention, there is provided a compound, or a pharmaceutically acceptable salt, hydrate or solvate thereof, as defined herein for use as an adenosine A2a and/or A2b antagonist. In an embodiment, the compounds of the invention are selective adenosine A2a and A2b antagonists.

According to a further aspect of the present invention, there is provided a compound, or a pharmaceutically acceptable salt, hydrate or solvate thereof, as defined herein for use in the treatment of a disease or disorder in which adenosine A2a and/or A2b is implicated.

According to a further aspect of the present invention, there is provided the use of a compound, or a pharmaceutically acceptable salt, hydrate or solvate thereof, as defined herein in the manufacture of a medicament for the treatment of a proliferative condition. Suitably, the compound or pharmaceutical composition is administered in combination with one or more additional anti proliferative agents (e.g. checkpoint inhibitors and/or cytotoxic agents).

According to a further aspect of the present invention, there is provide the use of a compound, or a pharmaceutically acceptable salt, hydrate or solvate thereof, as defined herein in the manufacture of a medicament for the treatment of cancer. Suitably, the cancer is a human cancer. Suitably, the compound or pharmaceutical composition is administered in combination with one or more additional anticancer agents (e.g. checkpoint inhibitors and/or cytotoxic agents).

According to a further aspect of the present invention, there is provided a use of a compound, or a pharmaceutically acceptable salt, hydrate or solvate thereof, as defined herein in the manufacture of a medicament for use as an adenosine A2a and/or A2b antagonist.

According to a further aspect of the present invention, there is provided a use of a compound, or a pharmaceutically acceptable salt, hydrate or solvate thereof, as defined herein in the manufacture of a medicament for the treatment of a disease or disorder in which adenosine A2a is implicated.

According to a further aspect of the present invention, there is provided a process for preparing a compound, or a pharmaceutically acceptable salt, hydrate or solvate thereof, as defined herein.

According to a further aspect of the present invention, there is provided a compound, or a pharmaceutically acceptable salt, hydrate or solvate thereof, obtainable by, or obtained by, or directly obtained by a process of preparing a compound as defined herein.

According to a further aspect of the present invention, there are provided novel intermediates as defined herein which are suitable for use in any one of the synthetic methods set out herein.

Features, including optional, suitable, and preferred features in relation to one aspect of the invention may also be features, including optional, suitable and preferred features in relation to any other aspect of the invention. Any references in the description to methods of treatment which comprise use of a compound or a pharmaceutical composition of the invention and which constitute a method for treatment of the human or animal body by surgery or therapy or a diagnostic method practised on the human or animal body are to be interpreted as references to the compound or pharmaceutical composition for use in those methods.

### DETAILED DESCRIPTION OF THE INVENTION

### Definitions

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

In this specification the term "alkyl" includes both straight and branched chain alkyl groups. References to individual alkyl groups such as "propyl" are specific for the straight chain version only and references to individual branched chain alkyl groups such as "isopropyl" are specific for the branched chain version only. For Example, "(1-6C)alkyl" includes (1-4C)alkyl, (1-3C)alkyl, propyl, isopropyl and t-butyl. A similar convention applies to other radicals, for example "phenyl(1-6C)alkyl" includes phenyl(1-4C)alkyl, benzyl, 1-phenylethyl and 2-phenylethyl.

The term "(m-nC)" or "(m-nC) group" used alone or as a prefix, refers to any group having m to n carbon atoms.

An "alkylene," "alkenylene," or "alkynylene" group is an alkyl, alkenyl, or alkynyl group that is positioned between and serves to connect two other chemical groups. Thus, "(1-6C)alkylene" means a linear saturated divalent hydrocarbon radical of one to six carbon atoms or a branched saturated divalent hydrocarbon radical of three to six carbon atoms, for example, methylene, ethylene, propylene, 2-methylpropylene, pentylene, and the like.

"(2-6C)alkenylene" means a linear divalent hydrocarbon radical of two to six carbon atoms or a branched divalent hydrocarbon radical of three to six carbon atoms, containing at least one double bond, for example, as in ethenylene, 2,4-pentadienylene, and the like.

"(2-6C)alkynylene" means a linear divalent hydrocarbon radical of two to six carbon atoms or a branched divalent hydrocarbon radical of three to six carbon atoms, containing at least one triple bond, for example, as in ethynylene, propynylene, and butynylene and the like.

"(3-8C)cycloalkyl" means a hydrocarbon ring containing from 3 to 8 carbon atoms, for example, cyclopropyl, cyclobutyl, cyclopentyl, cyclohexyl, cycloheptyl or bicyclo[2.2.1]heptyl.

"(3-8C)cycloalkenyl" means a hydrocarbon ring containing at least one double bond, for example, cyclobutenyl, cyclopentenyl, cyclohexenyl or cycloheptenyl, such as 3-cyclohexen-1-yl, or cyclooctenyl.

"(3-8C)cycloalkyl-(1-6C)alkylene" means a (3-8C)cycloalkyl group covalently attached to a (1-6C)alkylene group, both of which are defined herein.

The term "halo" or "halogeno" refers to fluoro, chloro, bromo and iodo.

The term "heterocyclyl", "heterocyclic" or "heterocycle" means a non-aromatic saturated or partially saturated monocyclic, fused, bridged, or spiro bicyclic heterocyclic ring system(s). Monocyclic heterocyclic rings contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles contain from 7 to 17 member atoms, suitably 7 to 12 member atoms, in the ring. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles containing nitrogen include, for example, azetidinyl, pyrrolidinyl, piperidinyl, piperazinyl, tetrahydrotriazinyl, tetrahydropyrazolyl, and the like. Typical sulfur containing heterocycles include tetrahydrothienyl, dihydro-1,3-dithiol, tetrahydro-2H-thiopyran, and hexahydrothiepine. Other heterocycles include dihydrooxathiolyl, tetrahydrooxazolyl, tetrahydro-oxadiazolyl, tetrahydrodioxazolyl, tetrahydrooxathiazolyl, hexahydrotriazinyl, tetrahydrooxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO₂ groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O) or thioxo (=S) substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group via any suitable atom, such as via a carbon or nitrogen atom. However, reference herein to piperidino or morpholino refers to a piperidin-1-yl or morpholin-4-yl ring that is linked via the ring nitrogen.

By "bridged ring systems" is meant ring systems in which two rings share more than two atoms, see for example Advanced Organic Chemistry, by Jerry March, 4th Edition, Wiley Interscience, pages 131-133, 1992. Examples of bridged heterocyclyl ring systems include, aza-bicyclo[2.2.1]heptane, 2-oxa-5-azabicyclo[2.2.1]heptane, aza-bicyclo[2.2.2]octane, aza-bicyclo[3.2.1]octane and quinuclidine.

By "spiro bicyclic ring systems" we mean that the two ring systems share one common spiro carbon atom, i.e. the heterocyclic ring is linked to a further carbocyclic or heterocyclic ring through a single common spiro carbon atom. Examples of spiro ring systems include 6-azaspiro[3.4]octane, 2-oxa-6-azaspiro[3.4]octane, 2-azaspiro[3.3]heptanes, 2-oxa-6-azaspiro[3.3]heptanes, 7-oxa-2-azaspiro[3.5]nonane, 6-oxa-2-azaspiro[3.4]octane, 2-oxa-7-azaspiro[3.5]nonane and 2-oxa-6-azaspiro[3.5]nonane.

"Heterocyclyl(1-6C)alkyl" means a heterocyclyl group covalently attached to a (1-6C)alkylene group, both of which are defined herein.

The term "heteroaryl" or "heteroaromatic" means an aromatic mono-, bi-, or polycyclic ring incorporating one or more (for example 14, particularly 1, 2 or 3) heteroatoms selected from nitrogen, oxygen or sulfur. The term heteroaryl includes both monovalent species and divalent species. Examples of heteroaryl groups are monocyclic and bicyclic groups containing from five to twelve ring members, and more usually from five to ten ring members. The heteroaryl group can be, for example, a 5- or 6-membered monocyclic ring or a 9- or 10-membered bicyclic ring, for example a bicyclic structure formed from fused five and six membered rings or two fused six membered rings. Each ring may contain up to about four heteroatoms typically selected from nitrogen, sulfur and oxygen. Typically, the heteroaryl ring will contain up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general, the number of basic nitrogen atoms present in the heteroaryl group, including any amino group substituents of the ring, will be less than five.

Examples of heteroaryl include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, isoindolyl, benzothienyl, benzoxazolyl, benzimidazolyl, benzothiazolyl, benzothiazolyl, indazolyl, purinyl, benzofurazanyl, quinolyl, isoquinolyl, quinazolinyl, quinoxalinyl, cinnolinyl, pteridinyl, naphthyridinyl, carbazolyl, phenazinyl, benzisoquinolinyl, pyridopyrazinyl, thieno[2,3b]-furanyl-, 2H-furo[3,2b]-pyranyl-, 5H-pyrido[2,3-d]-ooxazinyl-, 1H-pyrazolo[4,3-d]-oxazolyl, 4H-imidazo[4,5d]thiazolyl, pyrazino[2,3d]pyridazinyl, -imidazo[2,1b]thiazolyl, -imidazo[1,2b][1,2,4]-triazinyl. "Heteroaryl" also covers partially aromatic bi- or polycyclic ring systems wherein at least one ring is an aromatic ring and one or more of the other ring(s) is a nonaromatic, saturated or partially saturated ring, provided at least one ring contains one or more heteroatoms selected from nitrogen, oxygen or -sulfur-. Examples of partially aromatic heteroaryl groups include for example, tetrahydroisoquinolinyl, tetrahydroquinolinyl, 2-oxo-1,2,3,4-tetrahydroquinolinyl, dihydrobenzthienyl, dihydrobenzfuranyl, 2,3-dihydro-benzo[1,4]dioxinyl, benzo[1,3]dioxolyl, 2,2-dioxo-1,3-dihydro-2-benzothienyl, 4,5,6,7-tetrahydrobenzofuranyl, indolinyl, 1,2,3,4-tetrahydro-1,8-naphthyridinyl, 1,2,3,4-tetrahydropyrido[2,3-b]pyrazinyl, 3,4-dihydro-2*H*-pyrido[3,2-*b*][1,4]oxazinyl and 6,8-dihydro-5H-[1,2,4]triazolo[4,3-a]pyrazinyl.

Examples of five membered heteroaryl groups include but are not limited to pyrrolyl, furanyl, thienyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered heteroaryl groups include but are not limited to pyridyl, pyrazinyl, pyridazinyl, pyrimidinyl and triazinyl.

A bicyclic heteroaryl group may be, for example, a group selected from:
a benzene ring fused to a 5- or 6-membered ring containing 1, 2 or 3 ring heteroatoms;
a pyridine ring fused to a 5- or 6-membered ring containing 1, 2 or 3 ring heteroatoms;
a pyrimidine ring fused to a 5- or 6-membered ring containing 1 or 2 ring heteroatoms;
a pyrrole ring fused to a 5- or 6-membered ring containing 1, 2 or 3 ring heteroatoms;
a pyrazole ring fused to a 5- or 6-membered ring containing 1 or 2 ring heteroatoms;
a pyrazine ring fused to a 5- or 6-membered ring containing 1 or 2 ring heteroatoms;
an imidazole ring fused to a 5- or 6-membered ring containing 1 or 2 ring heteroatoms;
an oxazole ring fused to a 5- or 6-membered ring containing 1 or 2 ring heteroatoms;
an isoxazole ring fused to a 5- or 6-membered ring containing 1 or 2 ring heteroatoms;
a thiazole ring fused to a 5- or 6-membered ring containing 1 or 2 ring heteroatoms;
an isothiazole ring fused to a 5- or 6-membered ring containing 1 or 2 ring heteroatoms;
a thiophene ring fused to a 5- or 6-membered ring containing 1, 2 or 3 ring heteroatoms;
a furan ring fused to a 5- or 6-membered ring containing 1, 2 or 3 ring heteroatoms;
a cyclohexyl ring fused to a 5- or 6-membered heteroaromatic ring containing 1, 2 or 3 ring heteroatoms; and
a cyclopentyl ring fused to a 5- or 6-membered heteroaromatic ring containing 1, 2 or 3 ring heteroatoms.

Particular Examples of bicyclic heteroaryl groups containing a six membered ring fused to a five membered ring include but are not limited to benzfuranyl, benzthiophenyl, benzimidazolyl, benzoxazolyl, benzisoxazolyl, benzthiazolyl, benzisothiazolyl, isobenzofuranyl, indolyl, isoindolyl, indolizinyl, indolinyl, isoindolinyl, purinyl (e.g., adeninyl, guaninyl), indazolyl, benzodioxolyl and pyrazolopyridinyl groups.

Particular Examples of bicyclic heteroaryl groups containing two fused six membered rings include but are not limited to quinolinyl, isoquinolinyl, chromanyl, thiochromanyl, chromenyl, isochromenyl, chromanyl, isochromanyl, benzodioxanyl, quinolizinyl, benzoxazinyl, benzodiazinyl, pyridopyridinyl, quinoxalinyl, quinazolinyl, cinnolinyl, phthalazinyl, naphthyridinyl and pteridinyl groups.

"Heteroaryl(1-6C)alkyl" means a heteroaryl group covalently attached to a (1-6C)alkylene group, both of which are defined herein. Examples of heteroaralkyl groups include pyridin-3-ylmethyl, 3-(benzofuran-2-yl)propyl, and the like.

The term "aryl" means a cyclic or polycyclic aromatic ring having from 5 to 12 carbon atoms. The term aryl includes both monovalent species and divalent species. Examples of aryl groups include, but are not limited to, phenyl, biphenyl, naphthyl and the like. In particular embodiment, an aryl is phenyl.

The term "aryl(1-6C)alkyl" means an aryl group covalently attached to a (1-6C)alkylene group, both of which are defined herein. Examples of aryl-(1-6C)alkyl groups include benzyl, phenylethyl, and the like.

This specification also makes use of several composite terms to describe groups comprising more than one functionality. Such terms will be understood by a person skilled in the art. For Example heterocyclyl(m-nC)alkyl comprises (m-nC)alkyl substituted by heterocyclyl.

The term "optionally substituted" refers to either groups, structures, or molecules that are substituted and those that are not substituted. The term "wherein a/any CH, CH₂, CH₃ group or heteroatom (i.e. NH) within a R¹ group is optionally substituted" suitably means that (any) one of the hydrogen radicals of the R¹ group is substituted by a relevant stipulated group.

Where optional substituents are chosen from "one or more" groups it is to be understood that this definition includes all substituents being chosen from one of the specified groups or the substituents being chosen from two or more of the specified groups.

The phrase "compound of the invention" means those compounds which are disclosed herein, both generically and specifically.

### Compounds of the invention

In a first aspect, the present invention relates to compounds, or pharmaceutically acceptable salts, hydrates or solvates thereof, having the structural formula I shown below: 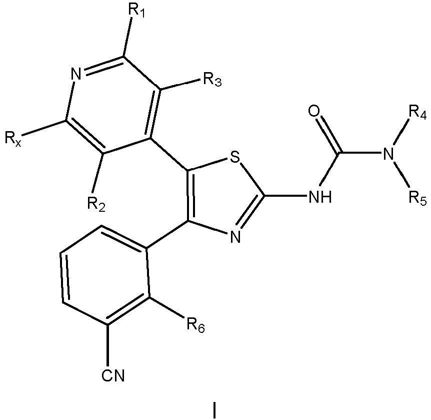 wherein:
Rₓ is selected from methyl, CD₃, chloro and CF₃;
R₁ is selected from (1-3C)alkyl, halo, (1-3C)haloalkyl, (1-3C)haloalkoxy, (1-3C)hydroxyalkyl, cyano, cyclopropyl, oxetan-3-yl, -(CR_{1C}R_{1D})_{q1}-OR_{1A}, -(CR_{1C}R_{1D})_{q1}-C(O)R_{1A}, -(CR_{1C}R_{1D})_{q1}-C(O)OR_{1A}, -(CR_{1C}R_{1D})_{q1}-OC(O)R_{1A}, -( CR_{1C}R_{1D})_{q1}-C(O)N(R_{1B})R_{1A}, -(CR_{1C}R_{1D})_{q1}-N(R_{1B})C(O)R_{1A}, -( CR_{1C}R_{1D})_{q1}-S(O)ₚR_{1A} (where p is 0, 1 or 2), -(CR_{1C}R_{1D})_{q1}-SO₂N(R_{1B})R_{1A}, or -(CR_{1C}R_{1D})_{q1}-N(R_{1B})SO₂R_{1A},
   wherein q1 is 0, 1, or 2; and
   wherein R_{1A} and R_{1B} are each independently selected from hydrogen, (1-2C)alkyl, (3-4C)cycloalkyl or (3-4C)cycloalkyl(1-2C)alkyl;
   wherein R_{1C} and R_{1D} are each independently selected from hydrogen or (1-2C)alkyl;
R₂ and R₃ are both hydrogen or one of R₂ and R₃ is hydrogen and the other is selected from fluoro, methyl or methoxy;
R₄ and R₅ are linked such that, together with the nitrogen atom to which they are attached,
they form heterocyclic ring which is optionally substituted on any available carbon atom by one or more R_{10C} substituent groups,
   wherein R_{10C} is selected from oxo, thioxo, halo, or cyano substituents, or a group of the formula:

      -[CR₇ₐR_{7b}]ₙ-L-Z
   wherein
   n is 0 to 6;
   R₇ₐ and R_{7b} are each independently selected from hydrogen, fluoro or (1-2C)alkyl;
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -C(S)N(Rₐ)-, -N(Rₐ)C(S)-,
   -N(Rₐ)C(S)N(R_{b})-, -S(O)₂N(Rₐ)-, or -N(Rₐ)SO₂-, wherein Rₐ and R_{b} are each independently selected from hydrogen or (1-2C)alkyl; and
   Z is selected from hydrogen, (1-6C)alkyl, (3-6C)cycloalkyl, (3-6C)cycloalkyl(1-2C)alkyl, aryl, aryl(1-2C)alkyl, heterocyclyl, heterocyclyl(1-2C)alkyl, heteroaryl or heteroaryl(1-2C)alkyl; and wherein Z is optionally substituted by one or more substituents selected from (1-6C)alkyl, halo, (1-6C)haloalkyl, (1-6C)haloalkoxy, cyano, nitro, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1, 2 or 3) or oxo, wherein any alkyl moiety in a substituent group on Z is optionally further substituted by cyano, halo, hydroxy, amino, oxo, (1-2C)alkyl or (1-2C)alkoxy and R_{c} and R_{d} are each independently selected from hydrogen, (1-6C)alkyl, (1-6C)haloalkyl or (3-6C)cycloalkyl;
and wherein any available N atoms are optionally in the form of a N-oxide or are optionally substituted by one or more R_{10N},
   wherein R_{10N} is -S(O)₂NH₂ or selected from:
      (i) -Z₁;
      (ii) -L₁ₐ-Z₁; or
      (iii) -[CR₈ₐR_{8b}]₁₋₆-L_{1b}-Z₁;
   wherein
   R₈ₐ and R_{8b} are each independently selected from hydrogen, fluoro or (1-2C)alkyl;
   L₁ₐ is selected from -C(O)-, -S(O)₂-, -C(O)O-, -C(O)N(Rₐ₁)-, -S(O)₂N(Rₐ₁)- or -N(Rₐ₁)-, wherein Rₐ₁ is selected from hydrogen or (1-2C)alkyl;
   L_{1b} is absent or selected from -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, -N(Rₐ₂)C(S)N(R_{b2})-, -S(O)₂N(Rₐ₂)-, or -N(Rₐ₂)SO₂, wherein Rₐ₂ and R_{b2} are each independently selected from hydrogen or (1-2C)alkyl; and
   Z₁ is selected from (1-6C)alkyl, (3-6C)cycloalkyl, aryl, heterocyclyl, heteroaryl, (3-6C)cycloalkyl(1-2C)alkyl, aryl(1-2C)alkyl, heterocyclyl(1-2C)alkyl or heteroaryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-6C)alkyl, halo, (1-6C)haloalkoxy, cyano, nitro, - NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen, (1-6C)alkyl, (1-6C)haloalkyl or (3-6C)cycloalkyl;
and wherein any S atoms present in the heterocyclic ring may optionally be present as S(=O), S(=O)₂ or S(=O)(=NRₑ) wherein Rₑ is selected from hydrogen, (1-3C)alkyl or (2-3C)alkanoyl; and
R₆ is selected from hydrogen, halo, methyl, methoxy and trifluoromethyl.

In a second aspect, the present invention relates to compounds, or pharmaceutically acceptable salts, hydrates or solvates thereof, having the structural formula I' (a sub definition of Formula I) shown below: 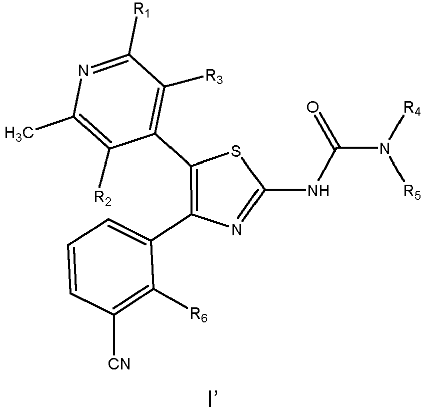 wherein:
R₁ is selected from (1-2C)alkyl, halo, (1-2C)haloalkyl, (1-2C)haloalkoxy, cyano, -(CH₂)_{q1}-OR_{1A}, -(CH₂)_{q1}-C(O)R_{1A}, -(CH₂)_{q1}-C(O)OR_{1A}, -(CH₂)_{q1}-OC(O)R_{1A}, -(CH₂)_{q1}-C(O)N(R_{1B})R_{1A}, -(CH₂)_{q1}-N(R_{1B})C(O)R_{1A}, -(CH₂)_{q1}-S(O)ₚR_{1A} (where p is 0, 1 or 2), -(CH₂)_{q1}-SO₂N(R_{1B})R_{1A}, or -(CH₂)_{q1}-N(R_{1B})SO₂R_{1A},
   wherein q1 is 0, 1, or 2; and
   wherein R_{1A} and R_{1B} are each independently selected from hydrogen, (1-2C)alkyl, (3-4C)cycloalkyl or (3-4C)cycloalkyl(1-2C)alkyl;
R₂ and R₃ are both hydrogen or one of R₂ and R₃ is hydrogen and the other is selected from fluoro, methyl or methoxy;
R₄ and R₅ are linked such that, together with the nitrogen atom to which they are attached, they form heterocyclic ring which is optionally substituted on any available carbon atom by one or more R_{10C} substituent groups,
   wherein R_{10C} is selected from oxo, thioxo, halo, or cyano substituents, or a group of the formula:

      -[CR₇ₐR_{7b}]ₙ₋L-Z
   wherein
   n is 0 to 6;
   R₇ₐ and R_{7b} are each independently selected from hydrogen, fluoro or (1-2C)alkyl;
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -C(S)N(Rₐ)-, -N(Rₐ)C(S)-, -N(Rₐ)C(S)N(R_{b})-, -S(O)₂N(Rₐ)-, or -N(Rₐ)SO₂-, wherein Rₐ and R_{b} are each independently selected from hydrogen or (1-2C)alkyl; and
   Z is selected from hydrogen, (1-6C)alkyl, (3-6C)cycloalkyl, (3-6C)cycloalkyl(1-2C)alkyl, aryl, aryl(1-2C)alkyl, heterocyclyl, heterocyclyl(1-2C)alkyl, heteroaryl or heteroaryl(1-2C)alkyl; and wherein Z is optionally substituted by one or more substituents selected from (1-6C)alkyl, halo, (1-6C)haloalkyl, (1-6C)haloalkoxy, cyano, nitro, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1, 2 or 3) or oxo,
   wherein any alkyl moiety in a substituent group on Z is optionally further substituted by cyano, halo, hydroxy, amino, oxo, (1-2C)alkyl or (1-2C)alkoxy and R_{c} and R_{d} are each independently selected from hydrogen, (1-6C)alkyl, (1-6C)haloalkyl or (3-6C)cycloalkyl;
and wherein any available N atoms are optionally in the form of a N-oxide or are optionally substituted by one or more R_{10N},
   wherein R_{10N} is -S(O)₂NH₂ or selected from:
      (i) -Z₁;
      (ii) -L₁ₐ-Z₁; or
      (iii) -[CR₈ₐR_{8b}]₁₋₆-L_{1b}-Z₁;
   wherein
   R₈ₐ and R_{8b} are each independently selected from hydrogen, fluoro or (1-2C)alkyl;
   L₁ₐ is selected from -C(O)-, -S(O)₂-, -C(O)O-, -C(O)N(Rₐ₁)-, -S(O)₂N(Rₐ₁)- or -N(Rₐ₁)-, wherein Rₐ₁ is selected from hydrogen or (1-2C)alkyl;
   L_{1b} is absent or selected from -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, -N(Rₐ₂)C(S)N(R_{b2})-, -S(O)₂N(Rₐ₂)-, or -N(Rₐ₂)SO₂, wherein Rₐ₂ and R_{b2} are each independently selected from hydrogen or (1-2C)alkyl; and
   Z₁ is selected from (1-6C)alkyl, (3-6C)cycloalkyl, aryl, heterocyclyl, heteroaryl, (3-6C)cycloalkyl(1-2C)alkyl, aryl(1-2C)alkyl, heterocyclyl(1-2C)alkyl or heteroaryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-6C)alkyl, halo, (1-6C)haloalkoxy, cyano, nitro, - NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen, (1-6C)alkyl, (1-6C)haloalkyl or (3-6C)cycloalkyl;
and wherein any S atoms present in the heterocyclic ring may optionally be present as S(=O), S(=O)₂ or S(=O)(=NRₑ) wherein Rₑ is selected from hydrogen, (1-3C)alkyl or (2-3C)alkanoyl; and
R₆ is selected from hydrogen, halo, methyl, methoxy and trifluoromethyl.

Particular compounds of the invention include, for example, compounds of the formula I, or pharmaceutically acceptable salts, hydrates and/or solvates thereof, wherein, unless otherwise stated, each of Rₓ, R₁, R₂, R₃, R₄, R₅ and R₆ have any of the meanings defined hereinbefore or in any of paragraphs (1) to (32) hereinafter:-
(1) R₁ is selected from (1-2C)alkyl, halo, (1-2C)haloalkyl, (1-2C)haloalkoxy, cyano, -(CH₂)_{q1}-OR_{1A} and -(CH₂)_{q1}-C(O)R_{1A};
   wherein q1 is 0 or 1; and
   wherein R_{1A} is selected from hydrogen or (1-2C)alkyl;
(1a) R₁ is selected from (1-3C)alkyl, halo, (1-3C)haloalkyl, (1-3C)haloalkoxy, (1-3C)hydroxyalkyl, cyano, cyclopropyl, oxetan-3-yl, -(CH₂)_{q1}-OR_{1A} and -(CH₂)_{q1}-C(O)R_{1A};
   wherein q1 is 0 or 1; and
   wherein R_{1A} is selected from hydrogen or (1-2C)alkyl;
(2) R₁ is selected from (1-2C)alkyl, halo, (1-2C)haloalkyl and -C(O)R_{1A};
   wherein R_{1A} is selected from hydrogen or methyl;
(2a) R₁ is selected from (1-3C)alkyl, halo, (1-3C)haloalkyl, (1-3C)haloalkoxy, (1-3C)hydroxyalkyl, cyano, cyclopropyl, oxetan-3-yl and -OR_{1A};
(3) R₁ is selected from methyl, halo and -CF₃;
(3a) R₁ is selected from methyl, CD₃, isopropyl, halo, (1-3C)hydroxyalkyl, cyano, cyclopropyl, oxetan-3-yl, -OMe, -CH₂F, -CHF₂ and -CF₃;
(3b) R₁ is selected from methyl, CD₃, isopropyl, halo, (1-3C)hydroxyalkyl, cyano, cyclopropyl, oxetan-3-yl, -OMe, CHF₂ and -CF₃;
(4) R₁ is selected from methyl and halo;
(4a) R₁ is selected from methyl, CD₃, isopropyl, hydroxymethyl, 2-hydroxyisopropyl, cyclopropyl, oxetan-3-yl, and halo;
(5) R₁ is selected from methyl, fluoro or chloro;
(6) R₁ is selected from methyl or chloro;
(7) R₁ is methyl;
(7a) R₁ is methyl or CD₃;
(8) R₁ is chloro;
(8a) R₁ is CD₃;
(8b) R₁ is isopropyl;
(8c) R₁ is hydroxymethyl;
(8d) R₁ is 2-hydroxyisopropyl;
(8e) R₁ is cyclopropyl;
(8f) R₁ is oxetan-3-yl
(9) R₂ and R₃ are both hydrogen or one of R₂ and R₃ is hydrogen and the other is fluoro or methyl;
(10) R₂ and R₃ are both hydrogen;
(11) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, thioxo, halo, or cyano substituents, or a group of the formula:

   -[CH₂]ₙ-L-Z

   wherein
   n is 0 to 4;
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -S(O)₂N(Rₐ)-, or -N(Rₐ)SO₂-, wherein Rₐ and R_{b} are each independently selected from hydrogen or (1-2C)alkyl; and
   Z is selected from hydrogen, (1-6C)alkyl, (3-6C)cycloalkyl, (3-6C)cycloalkyl(1-2C)alkyl, aryl, aryl(1-2C)alkyl, heterocyclyl, heterocyclyl(1-2C)alkyl, heteroaryl or heteroaryl(1-2C)alkyl; and wherein Z is optionally substituted by one or more substituents selected from (1-4C)alkyl, halo, (1-4C)haloalkyl, (1-4C)haloalkoxy, cyano, nitro, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}Rₑ (where y is 0, 1 or 2), -SO₂N(R_{c})R_{d}, -N(R_{c})SO₂R_{d}, -(CH₂)_{z}NR_{c}R_{d} (where z is 1, 2 or 3) or oxo,
   wherein any alkyl moiety in a substituent group on Z is optionally further substituted by cyano, halo, hydroxy, amino, oxo, (1-2C)alkyl or (1-2C)alkoxy and R_{c} and R_{d} are each independently selected from hydrogen, (1-4C)alkyl, (1-4C)haloalkyl or (3-6C)cycloalkyl;

   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₄-L_{1b}-Z₁; wherein
         R₈ₐ and R_{8b} are both hydrogen;
         L₁ₐ is selected from -C(O)-, -S(O)₂-, -C(O)O-, -C(O)N(Rₐ₁)- or -S(O)₂N(Rₐ₁)-, wherein Rₐ₁ is hydrogen or methyl;
         L_{1b} is absent or selected from -O-, -S-, -SO-, -SO₂₋, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, -N(Rₐ₂)C(S)N(R_{b2})-, -S(O)₂N(Rₐ₂)-, or -N(Rₐ₂)SO₂, wherein Rₐ₂ and R_{b2} are each independently selected from hydrogen or methyl; and
         Z₁ is selected from (1-6C)alkyl, (3-6C)cycloalkyl, aryl, heterocyclyl, heteroaryl, (3-6C)cycloalkyl(1-2C)alkyl, aryl(1-2C)alkyl, heterocyclyl(1-2C)alkyl or heteroaryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-4C)alkyl, halo, (1-4C)haloalkoxy, cyano, nitro, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen, (1-4C)alkyl, (1-4C)haloalkyl or (3-6C)cycloalkyl;
   and wherein any S atoms present in the heterocyclic ring may optionally be present as S(=O), S(=O)₂ or S(=O)(=NRₑ) wherein Rₑ is selected from hydrogen,(1-3C)alkyl or (2-3C)alkanoyl;
(12) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, or cyano substituents, or a group of the formula:

   -[CH₂]ₙ-L-Z

   wherein
   n is 0 to 2;
   L is absent or selected from -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)-, or -N(Rₐ₂)SO₂-, wherein Rₐ₂ and R_{b2} are each independently selected from hydrogen or methyl; and
   Z is selected from hydrogen, (1-6C)alkyl, (3-6C)cycloalkyl, (3-6C)cycloalkyl(1-2C)alkyl, aryl, heterocyclyl or heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1 or 2) or oxo;
   wherein any alkyl moiety in a substituent group on Z₁ is optionally further substituted by cyano, halo, hydroxy, amino, oxo, (1-2C)alkyl or (1-2C)alkoxy and R_{c} and R_{d} are each independently selected from hydrogen, (1-2C)alkyl, (1-2C)haloalkyl or (3-6C)cycloalkyl;

   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₂-L_{1b}-Z₁; wherein
         R₈ₐ and R_{8b} are both hydrogen;
         L₁ₐ is selected from -C(O)-, -S(O)₂- or -S(O)₂N(Rₐ₁)-, wherein Rₐ₁ is hydrogen or methyl;
         L_{1b} is absent or selected from -O-, -S-, -SO-, -SO₂₋, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)-, or -N(Rₐ₂)SO₂, wherein Rₐ₂ and R_{b2} are each independently selected from hydrogen or methyl; and
         Z₁ is selected from (1-6C)alkyl, (3-6C)cycloalkyl, aryl, heterocyclyl, heteroaryl, (3-6C)cycloalkyl(1-2C)alkyl or aryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, nitro, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen or (1-2C)alkyl;
   and wherein any S atoms present in the heterocyclic ring may optionally be present as S(=O), S(=O)₂ or S(=O)(=NRₑ) wherein Rₑ is selected from hydrogen,(1-3C)alkyl or (2-3C)alkanoyl;
(13) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, or cyano substituents, or a group of the formula:

   -[CH₂]ₙ-L-Z

   wherein
   n is 0 to 2;
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -S(O)₂N(Rₐ)-, or -N(Rₐ)SO₂-, wherein Rₐ and R_{b} are hydrogen; and
   Z is selected from hydrogen, (1-6C)alkyl, (3-6C)cycloalkyl, aryl, heterocyclyl or heteroaryl; and wherein Z is optionally substituted by one or more substituents selected from (1-2C)alkyl, halo, (1-2C)haloalkyl, (1-2C)haloalkoxy, cyano, - NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, - S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1 or 2) or oxo;
   wherein R_{c} and R_{d} are each independently selected from hydrogen, (1-2C)alkyl or (1-2C)haloalkyl;

   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₂-L_{1b}-Z₁;
      wherein
      R₈ₐ and R_{8b} are both hydrogen;
      L₁ₐ is selected from -C(O)-, -S(O)₂- or -S(O)₂N(Rₐ₁)-, wherein Rₐ₁ is hydrogen;
      L_{1b} is absent or selected from -O-, -S-, -SO-, -SO₂₋, -N(Rₐ₂)-, -C(O)N(Rₐ₂)-, - N(Rₐ₂)C(O)-, -S(O)₂N(Rₐ₂)- or -N(Rₐ₂)SO₂, wherein Rₐ₂ is hydrogen; and
      Z₁ is selected from (1-6C)alkyl, (3-6C)cycloalkyl, aryl, heterocyclyl, heteroaryl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NR_{c}R_{d} (where z is 1 or 2); and wherein Rₑ and R_{f} are each independently selected from hydrogen or methyl;
   and wherein any S atoms present in the heterocyclic ring may optionally be present as S(=O), S(=O)₂ or S(=O)(=NRₑ) wherein Rₑ is selected from hydrogen, (1-2C)alkyl or (2C)alkanoyl;
(14) R₄ and R₅ are linked such that, together with the nitrogen atom to which they are attached, they form heterocyclic ring which is optionally substituted on any available carbon atom by one or more R_{10C} substituent groups,
   wherein R_{10C} is selected from oxo, halo, or cyano, or a group of the formula:

      -[CH₂]ₙ-L-Z
   wherein
      n is 0 to 2;
      L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)-, or -N(Rₐ)SO₂-, wherein Rₐ and R_{b} are hydrogen; and
      Z is selected from hydrogen, (1-4C)alkyl, (3-6C)cycloalkyl, phenyl, a 4-7 membered heterocyclyl or a 5 or 6-membered heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, - C(O)OR_{c}, -OC(O)R_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1 or 2) or oxo;
      wherein R_{c} and R_{d} are each independently selected from hydrogen or (1-2C)alkyl;
   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₂-L_{1b}-Z₁; wherein
         R₈ₐ and R_{8b} are both hydrogen;
         L₁ₐ is selected from -C(O)-, -S(O)₂- or -S(O)₂N(Rₐ₁)-, wherein Rₐ₁ is hydrogen;
         L_{1b} is absent or selected from -O-, -S-, -SO-, -SO₂₋, -N(Rₐ₂)-, -C(O)N(Rₐ₂)-, -N(Rₐ₂)C(O)-, -S(O)₂N(Rₐ₂)- or -N(Rₐ₂)SO₂, wherein Rₐ₂ is hydrogen; and
         Z₁ is selected from (1-4C)alkyl, phenyl, a 4-7 membered heterocyclyl or a 5 or 6-membered heteroaryl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NR_{c}R_{d} (where z is 1 or 2); and wherein Rₑ and R_{f} are each independently selected from hydrogen or methyl;
   and wherein any S atoms present in the heterocyclic ring may optionally be present as S(=O), S(=O)₂ or S(=O)(=NRₑ) wherein Rₑ is selected from hydrogen, (1-2C)alkyl or (2C)alkanoyl;
(15) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, or cyano substituents, or a group of the formula:

   -[CH₂]ₙ-L-Z

   wherein
   n is 0 to 2;
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)-, or -N(Rₐ)SO₂-, wherein Rₐ and R_{b} are hydrogen; and
   Z is selected from hydrogen, (1-4C)alkyl, (3-6C)cycloalkyl, phenyl, a 4-7 membered heterocyclyl or a 5 or 6-membered heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, - C(O)N(R_{c})R_{d}, -N(R_{c})C(O)R_{d} or -S(O)_{y}Rₑ (where y is 0, 1 or 2) or oxo;
   wherein R_{c} and R_{d} are each independently selected from hydrogen or (1-2C)alkyl;

   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₂-L_{1b}-Z₁; wherein
         R₈ₐ and R_{8b} are both hydrogen;
         L₁ₐ is selected from -C(O)-, -S(O)₂- or -S(O)₂N(Rₐ₁)-, wherein Rₐ₁ is hydrogen; L_{1b} is absent or selected from -O-, -S-, -SO-, -SO₂₋, -N(Rₐ₂)-, -C(O)N(Rₐ₂)-, -N(Rₐ₂)C(O)-, -S(O)₂N(Rₐ₂)- or -N(Rₐ₂)SO₂, wherein Rₐ₂ is hydrogen; and
         Z₁ is selected from (1-4C)alkyl, phenyl, a 4-7 membered heterocyclyl or a 5 or 6-membered heteroaryl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, -NRₑR_{f}, -ORₑ, -C(O)Rₑ, -C(O)N(Rₑ)R_{f}, -N(Rₑ)C(O)R_{f}, -S(O)_{y}Rₑ (where y is 0, 1 or 2); and wherein Rₑ and R_{f} are each independently selected from hydrogen or methyl;
   and wherein any S atoms present in the heterocyclic ring may optionally be present as S(=O), S(=O)₂ or S(=O)(=NRₑ) wherein Rₑ is selected from hydrogen, methyl or (2C)alkanoyl;
(15a) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, or cyano substituents, or a group of the formula:

   -[CH₂]ₙ-L-Z

   wherein
   n is 0 to 2;
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)-, or -N(Rₐ)SO₂-, wherein Rₐ and R_{b} are hydrogen; and
   Z is selected from hydrogen, (1-4C)alkyl or (3-6C)cycloalkyl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, - C(O)N(R_{c})R_{d}, -N(R_{c})C(O)R_{d} or -S(O)_{y}Rₑ (where y is 0, 1 or 2) or oxo;
   wherein R_{c} and R_{d} are each independently selected from hydrogen or (1-2C)alkyl;

   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₂-L_{1b}-Z₁;
      wherein
      R₈ₐ and R_{8b} are both hydrogen;
      L₁ₐ is selected from -C(O)-, -S(O)₂- or -S(O)₂N(Rₐ₁)-, wherein Rₐ₁ is hydrogen;
      L_{1b} is absent or selected from -O-, -S-, -SO-, -SO₂₋, -N(Rₐ₂)-, -C(O)N(Rₐ₂)-, -N(Rₐ₂)C(O)-, -S(O)₂N(Rₐ₂)- or -N(Rₐ₂)SO₂, wherein Rₐ₂ is hydrogen; and
      Z₁ is selected from (1-4C)alkyl, wherein Z₁ is optionally substituted by one or more substituents selected from (1-2C)alkyl, halo, (1-2C)haloalkoxy, cyano, - NRₑR_{f}, -ORₑ, -C(O)Rₑ, -C(O)N(Rₑ)R_{f}, -N(Rₑ)C(O)R_{f}, -S(O)_{y}Rₑ (where y is 0, 1 or 2); and wherein Rₑ and R_{f} are each independently selected from hydrogen or methyl;
   and wherein any S atoms present in the heterocyclic ring may optionally be present as S(=O), S(=O)₂ or S(=O)(=NRₑ) wherein Rₑ is selected from hydrogen, methyl or (2C)alkanoyl;
(15b) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, or cyano substituents, or a group of the formula:

   -L-Z

   wherein
   L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)-, or -N(Rₐ)SO₂-, wherein Rₐ and R_{b} are hydrogen; and
   Z is selected from hydrogen, (1-4C)alkyl or (3-6C)cycloalkyl;

   and wherein any available N atoms are optionally in the form of a N-oxide or are optionally substituted by one or more R_{10N}, wherein R_{10N} is -S(O)₂NH₂ or (1-4C)alkyl,
   and wherein any S atoms present in the heterocyclic ring may optionally be present as S(=O), S(=O)₂ or S(=O)(=NRₑ) wherein Rₑ is selected from hydrogen, methyl or (2C)alkanoyl;
(15c) R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, cyano, (1-4C)alkyl or (3-6C)cycloalkyl;
   and wherein any available N atoms are optionally in the form of a N-oxide or are optionally substituted by one or more R_{10N}, wherein R_{10N} is -S(O)₂NH₂ or (1-4C)alkyl, and wherein any S atoms present in the heterocyclic ring may optionally be present as S(=O), S(=O)₂ or S(=O)(=NRₑ) wherein Rₑ is selected from hydrogen, methyl or (2C)alkanoyl;
(16) R₄ and R₅ are linked such that, together with the nitrogen atom to which they are attached, they form a 4 to 12 membered heterocyclic ring and wherein the heterocyclic ring is optionally substituted with one or more R_{10C} or R_{10N} substituent groups, wherein R_{10c} and R_{10N} are as defined in any one of paragraphs (11) to (15c) above;
(17) R₄ and R₅ are linked such that, together with the nitrogen atom to which they are attached, they form a 4 to 8 membered monocyclic or bridged heterocyclic ring or a 7 to 12-membered bicyclic or spiro bicyclic heterocyclic ring, and wherein the heterocyclic ring is optionally substituted with one or more R_{10C} or R_{10N} substituent groups, wherein R_{10c} and R_{10N} are as defined in any one of paragraphs (11) to (15c) above;
(18) R₄ and R₅ are linked such that, together with the nitrogen atom to which they are attached, they form a 4 to 7 membered monocyclic or bridged heterocyclic ring or a 7 to 11-membered bicyclic or spiro bicyclic heterocyclic ring, and wherein the heterocyclic ring is optionally substituted with one or more R_{10C} or R_{10N} substituent groups, wherein R_{10c} and R_{10N} are as defined in any one of paragraphs (11) to (15c) above;
(19) R₄ and R₅ are linked such that, together with the nitrogen atom to which they are attached, they form a 5 or 6 membered monocyclic heterocyclic ring or a 7 to 10-membered bicyclic or spiro bicyclic heterocyclic ring, and wherein the heterocyclic ring is optionally substituted with one or more R_{10C} or R_{10N} substituent groups, wherein R_{10c} and R_{10N} are as defined in any one of paragraphs (11) to (15c) above;
(20) R₄ and R₅ are linked such that, together with the nitrogen atom to which they are attached, they form a heterocyclic ring selected from any one of the following options: 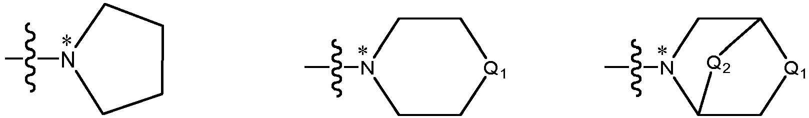 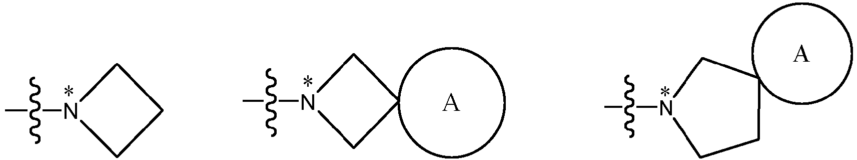 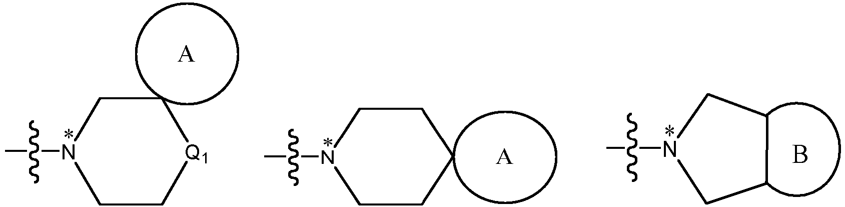 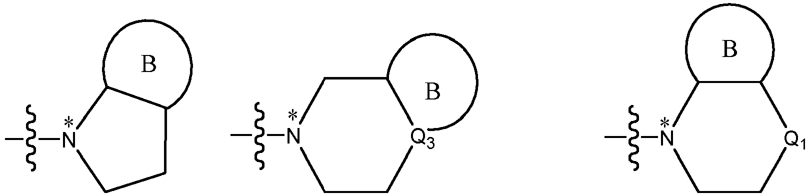
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-, -CHR_{10C}-, -C(R_{10c})₂-, -CHR_{10C}-CH₂-, -CH₂-CHR_{10C}-, -CHR_{10C}-CHR_{10C}-, -C(R_{10c})₂-CH₂-, or -CH₂-C(R_{10c})₂-;
   Q₃ is CH, CR_{10C} or N;
   Ring A is a spiro-fused 4, 5 or 6 membered carbocyclic or heterocyclic ring;
   Ring B is a fused 4, 5 or 6 membered carbocyclic or heterocyclic ring;
   wherein Rₑ is selected from hydrogen or methyl;
   and wherein each of the heterocyclic ring systems is optionally substituted with one or more R_{10c} or R_{10N} substituent groups as defined in any one of paragraphs (11) to (15c) above;
(20a) R₄ and R₅ are linked such that, together with the nitrogen atom to which they are attached, they form a heterocyclic ring selected from any one of the following options: 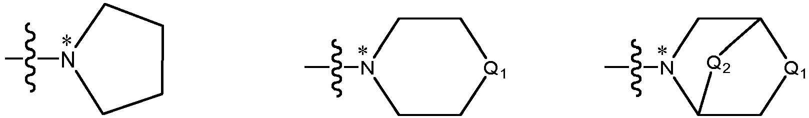 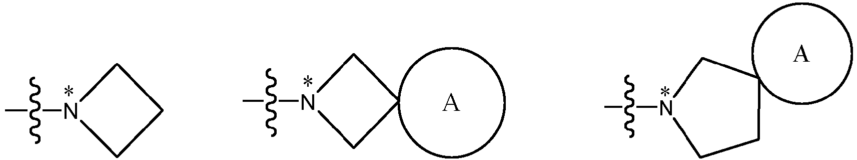 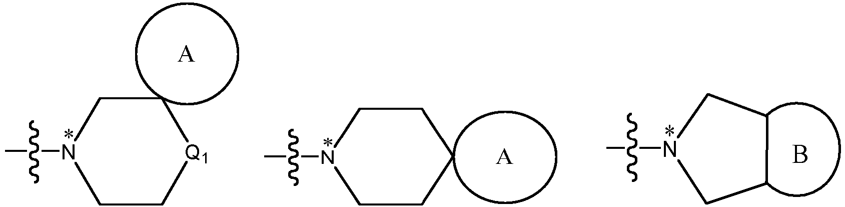 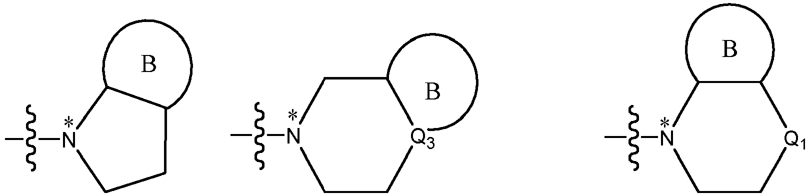 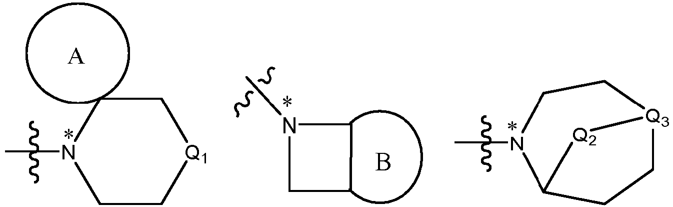
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-, -CHR_{10C}-, -C(R_{10c})₂-, -CHR_{10C}-CH₂-, -CH₂-CHR_{10C}-, -CHR_{10C}-CHR_{10C}-, -C(R_{10c})₂-CH₂-, or -CH₂-C(R_{10c})₂-;
   Q₃ is CH, CR_{10C} or N;
   Ring A is a spiro-fused 4, 5 or 6 membered carbocyclic or heterocyclic ring;
   Ring B is a fused 4, 5 or 6 membered carbocyclic or heterocyclic ring;
   wherein Rₑ is selected from hydrogen or methyl;
   and wherein each of the heterocyclic ring systems is optionally substituted with one or more R_{10c} or R_{10N} substituent groups as defined in any one of paragraphs (11) to (15c) above;
(21) R₄ and R₅ are linked such that, together with the nitrogen atom to which they are attached, they form a heterocyclic ring selected from any one of the following options: 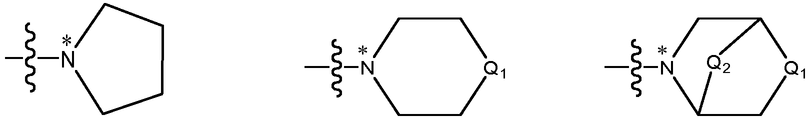 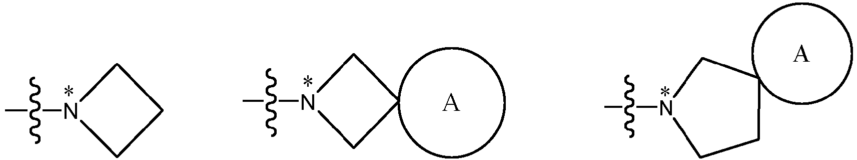 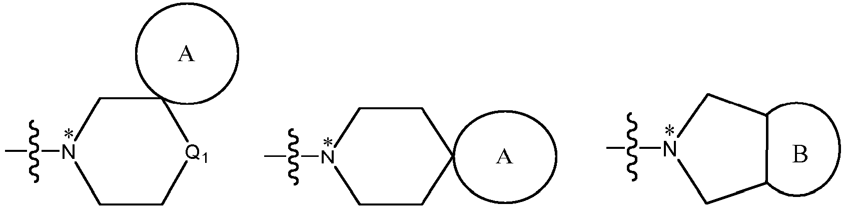 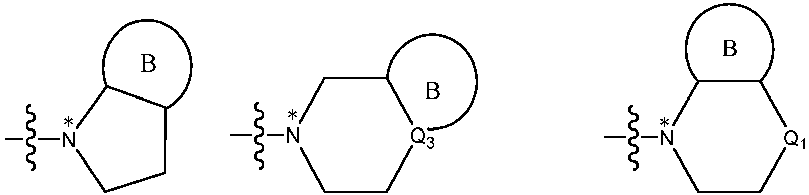
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-, -CHR_{10C}-, or -C(R_{10c})₂-;
   Q₃ is CH, CR_{10C} or N;
   Ring A is a spiro-fused 4, 5 or 6 membered carbocyclic or heterocyclic ring;
   Ring B is a fused 4, 5 or 6 membered carbocyclic or heterocyclic ring;
   wherein Rₑ is selected from hydrogen, (1-3C)alkyl or (2-3C)alkanoyl;
   and wherein each of the heterocyclic ring systems is optionally substituted with one or more R_{10c} or R_{10N} substituent groups as defined in any one of paragraphs (11) to (15c) above;
(21a) R₄ and R₅ are linked such that, together with the nitrogen atom to which they are attached, they form a heterocyclic ring selected from any one of the following options: 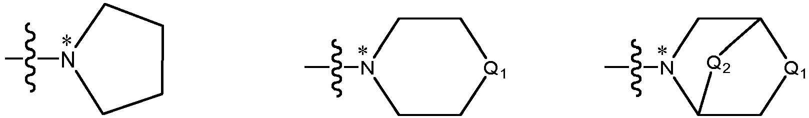 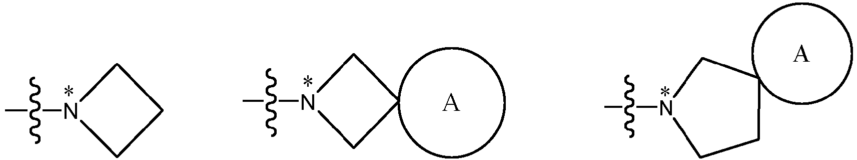 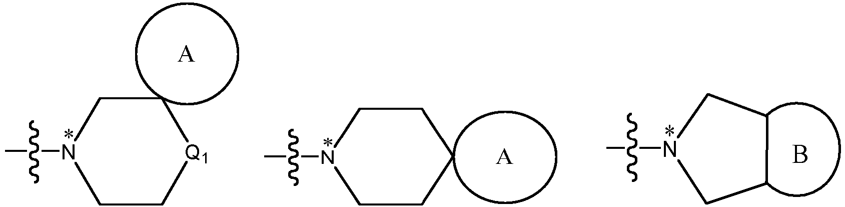 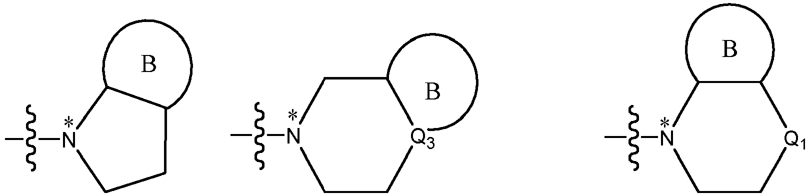 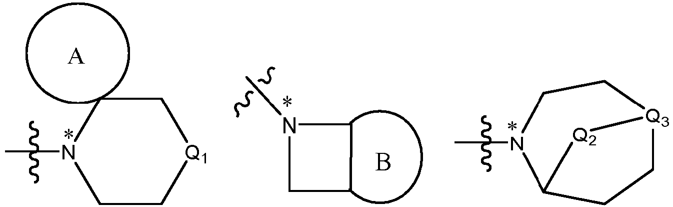
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-, -CHR_{10C}-, or -C(R_{10c})₂-;
   Q₃ is CH, CR_{10C} or N;
   Ring A is a spiro-fused 4, 5 or 6 membered carbocyclic or heterocyclic ring;
   Ring B is a fused 4, 5 or 6 membered carbocyclic or heterocyclic ring;
   wherein Rₑ is selected from hydrogen, (1-3C)alkyl or (2-3C)alkanoyl;
   and wherein each of the heterocyclic ring systems is optionally substituted with one or more R_{10c} or R_{10N} substituent groups as defined in any one of paragraphs (11) to (15c) above;
(21b) R₄ and R₅ are linked such that, together with the nitrogen atom to which they are attached, they form a heterocyclic ring selected from any one of the following options: 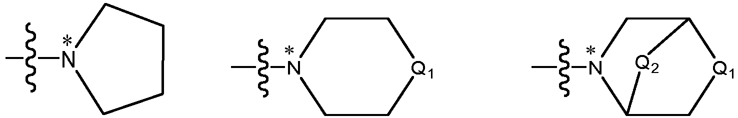 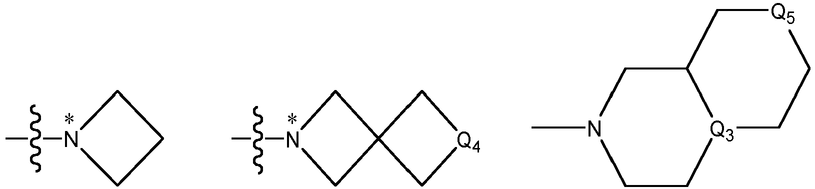 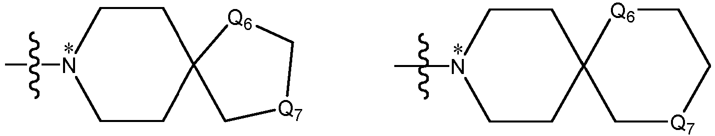
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-;
   Q₃ is CH, CR_{10C} or N;
   Q₄ is O, NH, S, S(O), S(O)₂, S(O)(=NRₑ), N-R_{10N}, CH₂, CHR_{10C} or C(R_{10c})₂;
   Q₅ is O, NH, S, S(O), S(O)₂, S(O)(=NRₑ), N-R_{10N}, CH₂, CHR_{10C} or C(R_{10c})₂;
   Q₆ is O, NH, S, S(O), S(O)₂, S(O)(=NRₑ), N-R_{10N}, CH₂, CHR_{10C} or C(R_{10c})₂;
   Q₇ is O, NH, S, S(O), S(O)₂, S(O)(=NRₑ), N-R_{10N}, CH₂, CHR_{10C} or C(R_{10c})₂;
   wherein Rₑ is selected from hydrogen, (1-2C)alkyl or (2C)alkanoyl;
   and wherein each of the heterocyclic ring systems is optionally substituted with one or more R_{10c} or R_{10N} substituent groups as defined in any one of paragraphs (11) to (15c) above;
(22) R₄ and R₅ are linked such that, together with the nitrogen atom to which they are attached, they form a heterocyclic ring selected from any one of the following options: 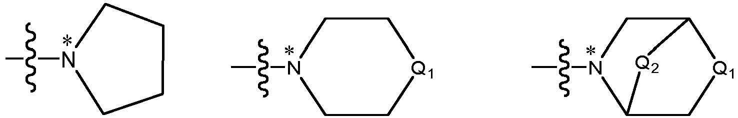 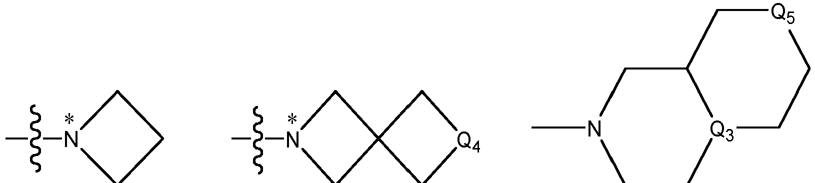 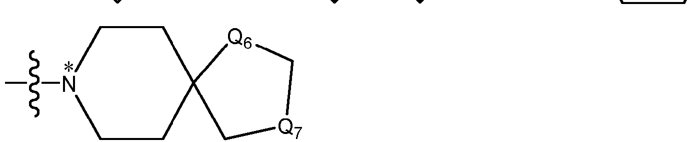
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-;
   Q₃ is CH, CR_{10C} or N;
   Q₄ is O, NH, S, S(O), S(O)₂, S(O)(=NRₑ), N-R_{10N}, CH₂, CHR_{10C} or C(R_{10c})₂;
   Q₅ is O, NH, S, S(O), S(O)₂, S(O)(=NRₑ), N-R_{10N}, CH₂, CHR_{10C} or C(R_{10c})₂;
   Q₆ is O, NH, S, S(O), S(O)₂, S(O)(=NRₑ), N-R_{10N}, CH₂, CHR_{10C} or C(R_{10c})₂;
   Q₇ is O, NH, S, S(O), S(O)₂, S(O)(=NRₑ), N-R_{10N}, CH₂, CHR_{10C} or C(R_{10c})₂;
   wherein Rₑ is selected from hydrogen, (1-2C)alkyl or (2C)alkanoyl;
   and wherein each of the heterocyclic ring systems is optionally substituted with one or more R_{10c} or R_{10N} substituent groups as defined in any one of paragraphs (11) to (15c) above;
(22a) R₄ and R₅ are linked such that, together with the nitrogen atom to which they are attached, they form a heterocyclic ring selected from any one of the following options: 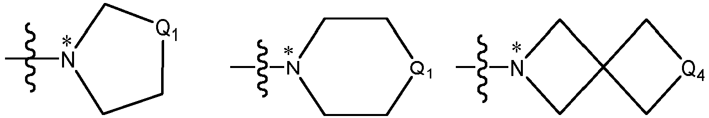
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₄ is O, NH, S, S(O), S(O)₂, S(O)(=NRₑ), N-R_{10N}, CH₂, CHR_{10C} or C(R_{10c})₂;
   wherein Rₑ is selected from hydrogen, (1-2C)alkyl or (2C)alkanoyl;
   and wherein each of the heterocyclic ring systems is optionally substituted with one or more R_{10c} or R_{10N} substituent groups as defined in any one of paragraphs (11) to (15c) above;
(22b) R₄ and R₅ are linked such that, together with the nitrogen atom to which they are attached, they form a heterocyclic ring selected from any one of the following options: 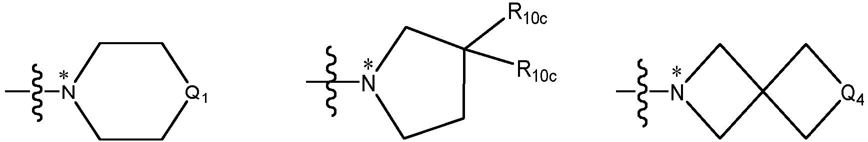
   wherein * denotes the N atom to which R₄ and R₅ are attached;
   Q₁ is O, NH, N-R_{10N}, CH₂, CHR_{10C} or C(R_{10c})₂;
   Q₄ is O, NH, N-R_{10N}, CH₂, CHR_{10C} or C(R_{10c})₂;
   wherein Rₑ is selected from hydrogen, methyl or (2C)alkanoyl;
   and R_{10c} or R_{10N} are as defined in any one of paragraphs (11) to (15c) above;
(23) R₄ and R₅ are linked such that, together with the nitrogen atom to which they are attached, they form a heterocyclic ring selected from any one of the following options: 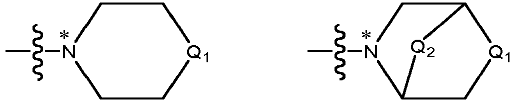
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-;
   wherein Rₑ is selected from hydrogen, methyl or (2C)alkanoyl;
   and R_{10c} or R_{10N} are as defined in any one of paragraphs (11) to (15c) above;
(24) R₄ and R₅ are linked such that, together with the nitrogen atom to which they are attached, they form a heterocyclic ring of the structure: 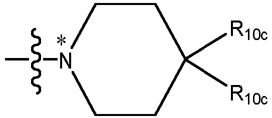
   wherein * denotes the N atom to which R₄ and R₅ are attached;
   and each R_{10c} is independently selected from the options defined in any one of paragraphs (11) to (15c) above;
(25) R₆ is selected from hydrogen, halo or methyl;
(26) R₆ is selected from hydrogen or methyl;
(27) R₆ is hydrogen;
(28) Rₓ is selected from methyl, CD₃ or chloro;
(29) Rₓ is selected from methyl or CD₃;
(30) Rₓ is methyl;
(31) Rₓ is CD₃
(32) Rₓ is chloro;

Suitably, a heteroaryl or heterocyclyl group as defined herein is a monocyclic heteroaryl or mono, bicyclic, spiro bicyclic or bridged heterocyclyl group comprising one, two or three heteroatoms selected from N, O or S.

Suitably, a heteroaryl is a 5- or 6-membered heteroaryl ring comprising one, two or three heteroatoms selected from N, O or S.

Suitably, a heterocyclyl group is a 4-, 5-, 6-, 7-, 8-, 9-, 10-, 11-, or 12- membered heterocyclyl ring comprising one, two or three heteroatoms selected from N, O or S. Most suitably, a heterocyclyl group is a 5-, 6- or 7-membered ring comprising one, two or three heteroatoms selected from N, O or S [e.g. morpholinyl (e.g. 4-morpholinyl), pyridinyl, piperazinyl, homopiperazinyl or pyrrolidinonyl] or a 7-, 8-, 9- or 10-membered spiro bicyclic ring system comprising one, two or three heteroatoms selected from N, O or S.

Suitably, an aryl group is phenyl.

Suitably, R₁ is as defined in paragraphs (1) to (8) above. In an embodiment, R₁ is as defined in paragraph (6) above.

Suitably, R₁ is as defined in paragraphs (1) to (8f) above. More suitably, R₁ is as defined in paragraphs (1a), (2a), (3a), (3b), (4a), (7a), (8a), (8b), (8c), (8d), (8e) or (8f) above. In an embodiment, R₁ is as defined in paragraph (4a) or (7a) above.

Suitably, R₂ and R₃ are as defined in any one of paragraphs (9) or (10) above. Most suitably, R₂ and R₃ are as defined in paragraph (10) above.

Suitably, R₄ and R₅ are as defined in any one of paragraphs (11) to (24) above. More suitably, R₄ and R₅ are as defined in paragraph (15), (15a), (15b) or (15c), (20a), (21a), (21b), (22a) or (22b) above. In certain groups of compounds of the invention, R₄ and R₅ are as defined in paragraph (16), (17), (18), (20), (21), (22) (23), (24), (20a), (21a), (21b), (22a) or (22b) above.

Suitably, R₄ and R₅ are as defined in any one of paragraphs (11) to (24) above. More suitably, R₄ and R₅ are as defined in paragraph (15) above. Even more suitably, R₄ and R₅ are as defined in paragraph (16), (17) or (18) above. In certain groups of compounds of the invention, R₄ and R₅ are as defined in paragraph (20), (21), (22) (23) or (24) above.

Suitably, Rₓ is as defined in any one of paragraphs (28) to (31) above. Most suitably, Rₓ is as defined in paragraph (29) above.

Suitably, R_{10c} and R_{10N} are as defined in any one of paragraphs (11) to (15c) above. More suitably, R_{10c} and R_{10N} are as defined in any one of paragraphs (15) to (15c) above. Even more suitably, R_{10c} and R_{10N} are as defined in any one of paragraphs (15a), (15b) or (15c) above.

Suitably, R₆ is as defined in any one of paragraphs (25) to (27) above. More suitably, R₆ is as defined in paragraph (27) above.

In a particular group of compounds of Formula I above, R₁ is as defined in any one of paragraphs (1), (2), (3), (4), (5), (6), (7) or (8) and R₂, R₃, R₄, R₅ and R₆ each have any one of the definitions set out herein.

In a particular group of compounds of Formula I above, R₁ is as defined in any one of paragraphs (1a), (2a), (3a), (3b), (4a), (7a), (8a), (8b), (8c), (8d), (8e), (8f) above, and R_{z} R₂, R₃, R₄, R₅ and R₆ each have any one of the definitions set out herein.

In a particular group of compounds of Formula I above, R₂ and R₃ are hydrogen, i.e. the compounds have the structural formula IA (a sub-formula of Formula I) shown below: 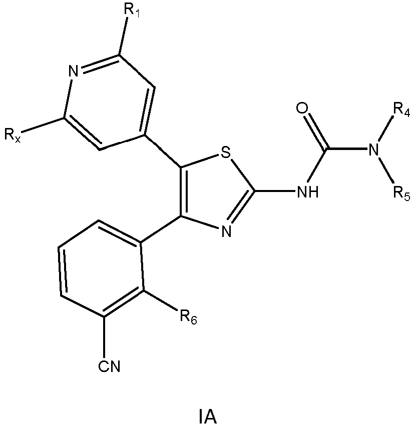 wherein Rₓ, R₁, R₄, R₅, R₆ and Rₓ each have any one of the definitions set out herein.

In an embodiment of the compounds of formula IA:
Rₓ is as defined in any one of paragraphs (28) to (31) above
R₁ is as defined in any one of paragraph (1) to (8f) above;
R₄ and R₅ are as defined in any one of paragraphs (11) to (24) above; and
R₆ is as defined in any one of paragraphs (25) to (27) above;

In another embodiment of the compounds of formula IA:
Rₓ is as defined in paragraph (29) above;
R₁ is as defined in paragraphs (1a), (2a), (3a), (3b), (4a), (7a), (8a), (8b), (8c), (8d), (8e) or (8f) above;
R₄ and R₅ are as defined in paragraph (12) above; and
R₆ is as defined in paragraphs (25) above.

In another embodiment of the compounds of formula IA:
Rₓ is as defined in paragraph (29) above;
R₁ is as defined in paragraph (4a) above;
R₄ and R₅ are as defined in paragraph (15), (15a), (15b), (15c) or (16) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (16) above; and
R₆ is as defined in paragraphs (27) above.

In another embodiment of the compounds of formula IA':
Rₓ is as defined in paragraph (29) above;
R₁ is as defined in paragraph (4a) above;
R₄ and R₅ are as defined in paragraph (17) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (18) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (19) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (20) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (21) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (22) above; and
R₆ is as defined in paragraphs (27) above.

In another embodiment of the compounds of formula IA:
Rₓ is as defined in paragraph (29) above;
R₁ is as defined in paragraph (6) above;
R₄ and R₅ are as defined in paragraph (23) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (24) above; and
R₆ is as defined in paragraphs (27) above.

In another embodiment of the compounds of formula IA:
Rₓ is as defined in paragraph (29) above;
R₁ is as defined in paragraph (4a) above;
R₄ and R₅ are as defined in paragraph (20a), (21a), (21b), (22a) or (22b) above; and
R₆ is as defined in paragraphs (27) above.

In a particular group of compounds of Formula I above, R₆ is hydrogen, i.e. the compounds have the structural formula IB (a sub-formula of Formula I') shown below: 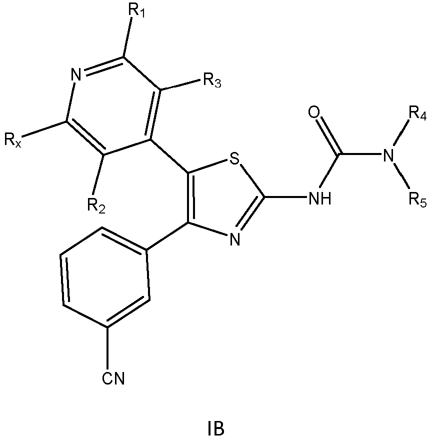 wherein Rₓ, R₁, R₂, R₃, R₄ and R₅ each have any one of the definitions set out herein.

In an embodiment of the compounds of formula IB:
Rₓ is as defined in any one of paragraphs (28) to (31) above
R₁ is as defined in any one of paragraph (1) to (8f) above;
R₂ and R₃ are as defined in paragraph (9) or (10); and
R₄ and R₅ are as defined in any one of paragraphs (11) to (24) above.

In another embodiment of the compounds of formula IB:
Rₓ is as defined in paragraph (29) above;
R₁ is as defined in paragraphs (1a), (2a), (3a), (3b), (4a), (7a), (8a), (8b), (8c), (8d), (8e) or (8f) above;
R₂ and R₃ are as defined in paragraph (9); and
R₄ and R₅ are as defined in paragraph (12) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (15), (15a), (15b) or (15c) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (16) above.

In another embodiment of the compounds of formula IB:
R₁ is as defined in paragraph (6) above;
R₂ and R₃ are as defined in paragraph (10); and
R₄ and R₅ are as defined in paragraph (17) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (18) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (19) above.

In another embodiment of the compounds of formula IB:
R₁ is as defined in paragraph (6) above;
R₂ and R₃ are as defined in paragraph (10); and
R₄ and R₅ are as defined in paragraph (20) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (21) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (22) above.

In another embodiment of the compounds of formula IB:
R₁ is as defined in paragraph (6) above;
R₂ and R₃ are as defined in paragraph (10); and
R₄ and R₅ are as defined in paragraph (23) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (24) above.

In another embodiment of the compounds of formula IB:
Rₓ is as defined in paragraph (29) above;
R₁ is as defined in paragraph (4a) above;
R₂ and R₃ are as defined in paragraph (10); and
R₄ and R₅ are as defined in paragraph (20a), (21a), (21b), (22a) or (22b) above.

In a particular group of compounds of Formula I above, R₂, R₃ and R₆ are hydrogen, i.e. the compounds have the structural formula IC (a sub-formula of Formula I) shown below: 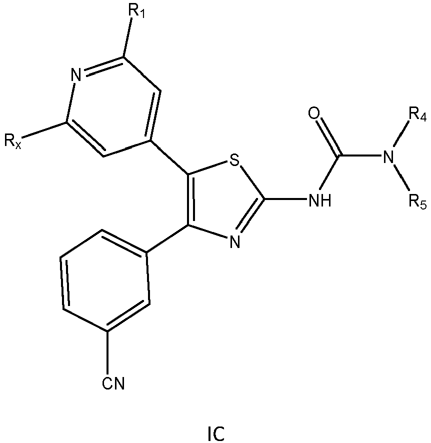 wherein Rₓ, R₁, R₄ and R₅ each have any one of the definitions set out herein.

In an embodiment of the compounds of formula IC:
Rₓ is as defined in any one of paragraphs (28) to (31) above
R₁ is as defined in any one of paragraph (1) to (8f) above; and
R₄ and R₅ are as defined in any one of paragraphs (11) to (24) above.

In another embodiment of the compounds of formula IC:
Rₓ is as defined in paragraph (29) above;
R₁ is as defined in paragraphs (1a), (2a), (3a), (3b), (4a), (7a), (8a), (8b), (8c), (8d), (8e) or (8f) above; and
R₄ and R₅ are as defined in paragraph (12) above.

In another embodiment of the compounds of formula IC:
Rₓ is as defined in paragraph (29) above;
R₁ is as defined in paragraph (4a) above; and
R₄ and R₅ are as defined in paragraph (15), (15a), (15b) or (15c) above.

In another embodiment of the compounds of formula IC:
Rₓ is as defined in paragraph (29) above;
R₁ is as defined in paragraph (4a) above; and
R₄ and R₅ are as defined in paragraph (16) above.

In another embodiment of the compounds of formula IC:
Rₓ is as defined in paragraph (29) above;
R₁ is as defined in paragraph (4a) above; and
R₄ and R₅ are as defined in paragraph (17) above.

In another embodiment of the compounds of formula IC:
Rₓ is as defined in paragraph (29) above;
R₁ is as defined in paragraph (4a) above; and
R₄ and R₅ are as defined in paragraph (18) above.

In another embodiment of the compounds of formula IC:
Rₓ is as defined in paragraph (29) above;
R₁ is as defined in paragraph (4a) above; and
R₄ and R₅ are as defined in paragraph (19) above.

In another embodiment of the compounds of formula IC:
Rₓ is as defined in paragraph (29) above;
R₁ is as defined in paragraph (4a) above; and
R₄ and R₅ are as defined in paragraph (20) above.

In another embodiment of the compounds of formula IC:
Rₓ is as defined in paragraph (29) above;
R₁ is as defined in paragraph (4a) above; and
R₄ and R₅ are as defined in paragraph (21) above.

In another embodiment of the compounds of formula IC:
Rₓ is as defined in paragraph (29) above;
R₁ is as defined in paragraph (4a) above; and
R₄ and R₅ are as defined in paragraph (22) above.

In another embodiment of the compounds of formula IC':
Rₓ is as defined in paragraph (29) above;
R₁ is as defined in paragraph (4a) above; and
R₄ and R₅ are as defined in paragraph (23) above.

In another embodiment of the compounds of formula IC:
Rₓ is as defined in paragraph (29) above;
R₁ is as defined in paragraph (4a) above; and
R₄ and R₅ are as defined in paragraph (24) above.

In another embodiment of the compounds of formula IC:
Rₓ is as defined in paragraph (29) above;
R₁ is as defined in paragraph (4a) above; and
R₄ and R₅ are as defined in paragraph (20a), (21a), (21b), (22a) or (22b) above; and

In a particular group of compounds of Formula I above, R₂ and R₃ are hydrogen, i.e. the compounds have the structural formula IA' (a sub-formula of Formula I) shown below: 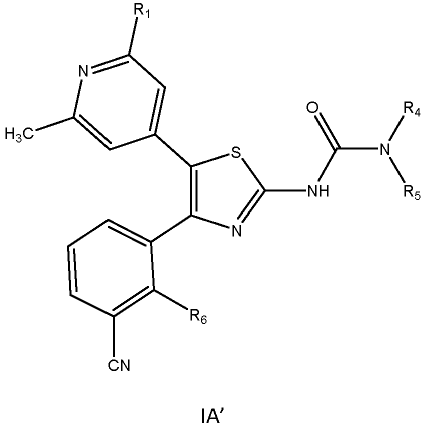 wherein R₁, R₄, R₅ and R₆ each have any one of the definitions set out herein.

In an embodiment of the compounds of formula IA':
R₁ is as defined in any one of paragraph (1) to (8) above;
R₄ and R₅ are as defined in any one of paragraphs (11) to (24) above; and
R₆ is as defined in any one of paragraphs (25) to (27) above.

In another embodiment of the compounds of formula IA':
R₁ is as defined in paragraph (2) above;
R₄ and R₅ are as defined in paragraph (12) above; and
R₆ is as defined in paragraphs (25) above.

In another embodiment of the compounds of formula IA':
R₁ is as defined in paragraph (6) above;
R₄ and R₅ are as defined in paragraph (15), (15a), (15b) or (15c) above; and
R₆ is as defined in paragraphs (27) above.

In another embodiment of the compounds of formula IA':
R₁ is as defined in paragraph (6) above;
R₄ and R₅ are as defined in paragraph (16) above; and
R₆ is as defined in paragraphs (27) above.

In another embodiment of the compounds of formula IA':
R₁ is as defined in paragraph (6) above;
R₄ and R₅ are as defined in paragraph (17) above; and
R₆ is as defined in paragraphs (27) above.

In another embodiment of the compounds of formula IA':
R₁ is as defined in paragraph (6) above;
R₄ and R₅ are as defined in paragraph (18) above; and
R₆ is as defined in paragraphs (27) above.

In another embodiment of the compounds of formula IA':
R₁ is as defined in paragraph (6) above;
R₄ and R₅ are as defined in paragraph (19) above; and
R₆ is as defined in paragraphs (27) above.

In another embodiment of the compounds of formula IA':
R₁ is as defined in paragraph (6) above;
R₄ and R₅ are as defined in paragraph (20) above; and
R₆ is as defined in paragraphs (27) above.

In another embodiment of the compounds of formula IA':
R₁ is as defined in paragraph (6) above;
R₄ and R₅ are as defined in paragraph (21) above; and
R₆ is as defined in paragraphs (27) above.

In another embodiment of the compounds of formula IA':
R₁ is as defined in paragraph (6) above;
R₄ and R₅ are as defined in paragraph (22) above; and
R₆ is as defined in paragraphs (27) above.

In another embodiment of the compounds of formula IA':
R₁ is as defined in paragraph (6) above;
R₄ and R₅ are as defined in paragraph (23) above; and
R₆ is as defined in paragraphs (27) above.

In another embodiment of the compounds of formula IA':
R₁ is as defined in paragraph (6) above;
R₄ and R₅ are as defined in paragraph (24) above; and
R₆ is as defined in paragraphs (27) above.

In a particular group of compounds of Formula I above, R₆ is hydrogen, i.e. the compounds have the structural formula IB' (a sub-formula of Formula I) shown below: 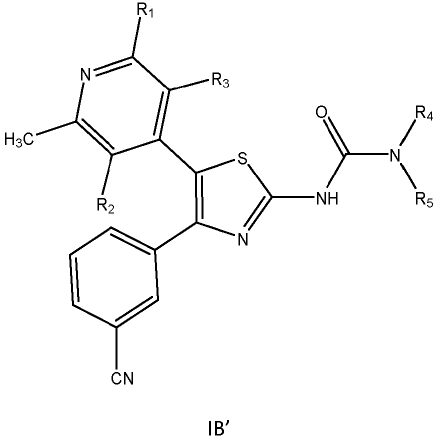 wherein R₁, R₂, R₃, R₄ and R₅ each have any one of the definitions set out herein.

In an embodiment of the compounds of formula IB':
R₁ is as defined in any one of paragraph (1) to (8) above;
R₂ and R₃ are as defined in paragraph (9) or (10); and
R₄ and R₅ are as defined in any one of paragraphs (11) to (24) above.

In another embodiment of the compounds of formula IB':
R₁ is as defined in paragraph (2) above;
R₂ and R₃ are as defined in paragraph (9); and
R₄ and R₅ are as defined in paragraph (12) above.

In another embodiment of the compounds of formula IB':
R₁ is as defined in paragraph (6) above;
R₂ and R₃ are as defined in paragraph (10); and
R₄ and R₅ are as defined in paragraph (15), (15a), (15b) or (15c) above.

In another embodiment of the compounds of formula IB':
R₁ is as defined in paragraph (6) above;
R₂ and R₃ are as defined in paragraph (10); and
R₄ and R₅ are as defined in paragraph (16) above.

In another embodiment of the compounds of formula IB':
R₁ is as defined in paragraph (6) above;
R₂ and R₃ are as defined in paragraph (10); and
R₄ and R₅ are as defined in paragraph (17) above.

In another embodiment of the compounds of formula IB':
R₁ is as defined in paragraph (6) above;
R₂ and R₃ are as defined in paragraph (10); and
R₄ and R₅ are as defined in paragraph (18) above.

In another embodiment of the compounds of formula IB':
R₁ is as defined in paragraph (6) above;
R₂ and R₃ are as defined in paragraph (10); and
R₄ and R₅ are as defined in paragraph (19) above.

In another embodiment of the compounds of formula IB':
R₁ is as defined in paragraph (6) above;
R₂ and R₃ are as defined in paragraph (10); and
R₄ and R₅ are as defined in paragraph (20) above.

In another embodiment of the compounds of formula IB':
R₁ is as defined in paragraph (6) above;
R₂ and R₃ are as defined in paragraph (10); and
R₄ and R₅ are as defined in paragraph (21) above.

In another embodiment of the compounds of formula IB':
R₁ is as defined in paragraph (6) above;
R₂ and R₃ are as defined in paragraph (10); and
R₄ and R₅ are as defined in paragraph (22) above.

In another embodiment of the compounds of formula IB':
R₁ is as defined in paragraph (6) above;
R₂ and R₃ are as defined in paragraph (10); and
R₄ and R₅ are as defined in paragraph (23) above.

In another embodiment of the compounds of formula IB':
R₁ is as defined in paragraph (6) above;
R₂ and R₃ are as defined in paragraph (10); and
R₄ and R₅ are as defined in paragraph (24) above.

In a particular group of compounds of Formula I above, R₂, R₃ and R₆ are hydrogen, i.e. the compounds have the structural formula IC' (a sub-formula of Formula I) shown below: 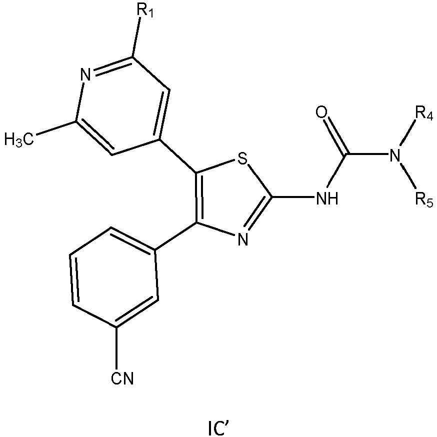 wherein R₁, R₄ and R₅ each have any one of the definitions set out herein.

In an embodiment of the compounds of formula IC':
R₁ is as defined in any one of paragraph (1) to (8) above; and
R₄ and R₅ are as defined in any one of paragraphs (11) to (24) above.

In another embodiment of the compounds of formula IC':
R₁ is as defined in paragraph (2) above; and
R₄ and R₅ are as defined in paragraph (12) above.

In another embodiment of the compounds of formula IC':
R₁ is as defined in paragraph (6) above; and
R₄ and R₅ are as defined in paragraph (15), (15a), (15b) or (15c) above.

In another embodiment of the compounds of formula IC':
R₁ is as defined in paragraph (6) above; and
R₄ and R₅ are as defined in paragraph (16) above.

In another embodiment of the compounds of formula IC':
R₁ is as defined in paragraph (6) above; and
R₄ and R₅ are as defined in paragraph (17) above.

In another embodiment of the compounds of formula IC':
R₁ is as defined in paragraph (6) above; and
R₄ and R₅ are as defined in paragraph (18) above.

In another embodiment of the compounds of formula IC':
R₁ is as defined in paragraph (6) above; and
R₄ and R₅ are as defined in paragraph (19) above.

In another embodiment of the compounds of formula IC':
R₁ is as defined in paragraph (6) above; and
R₄ and R₅ are as defined in paragraph (20) above.

In another embodiment of the compounds of formula IC':
R₁ is as defined in paragraph (6) above; and
R₄ and R₅ are as defined in paragraph (21) above.

In another embodiment of the compounds of formula IC':
R₁ is as defined in paragraph (6) above; and
R₄ and R₅ are as defined in paragraph (22) above.

In another embodiment of the compounds of formula IC':
R₁ is as defined in paragraph (6) above; and
R₄ and R₅ are as defined in paragraph (23) above.

In another embodiment of the compounds of formula IC':
R₁ is as defined in paragraph (6) above; and
R₄ and R₅ are as defined in paragraph (24) above.

Particular compounds of the present invention include any of the compounds described in the example section of the present application, or a pharmaceutically acceptable salt or solvate thereof, and, in particular, any of the following:
N-[5-(2-chloro-6-methyl-4-pyridyl)-4-(3-cyanophenyl)thiazol-2-yl]morpholine-4-carboxamide
N-[5-(2-chloro-6-methyl-4-pyridyl)-4-(3-cyanophenyl)thiazol-2-yl]-3-(1-hydroxy-1-methylethyl)pyrrolidine-1-carboxamide
(2R)-N-[5-(2-chloro-6-methyl-4-pyridyl)-4-(3-cyanophenyl)thiazol-2-yl]-2-(1-hydroxy-1-methylethyl)pyrrolidine-1-carboxamide
N-[5-(2-chloro-6-methyl-4-pyridyl)-4-(3-cyanophenyl)thiazol-2-yl]piperazine-1-carboxamide (3R)-N-[5-(2-chloro-6-methyl-4-pyridyl)-4-(3-cyanophenyl)thiazol-2-yl]-3-(1-hydroxy-1-methylethyl)piperazine-1-carboxamide
N-[5-(2-chloro-6-methyl-4-pyridyl)-4-(3-cyanophenyl)thiazol-2-yl]-4-(oxetan-3-yl)piperazine-1-carboxamide
N-[5-(2-chloro-6-methyl-4-pyridyl)-4-(3-cyanophenyl)thiazol-2-yl]-1-imino-1-oxo-1,4-thiazinane-4-carboxamide
N-[5-(2-chloro-6-methyl-4-pyridyl)-4-(3-cyanophenyl)thiazol-2-yl]pyrrolidine-1-carboxamide
N-[5-(2-chloro-6-methyl-4-pyridyl)-4-(3-cyanophenyl)thiazol-2-yl]-2-(2-hydroxy-2-methylpropyl)pyrrolidine-1-carboxamide
N-[5-(2-chloro-6-methyl-4-pyridyl)-4-(3-cyanophenyl)thiazol-2-yl]-3-(1-hydroxy-1-methylethyl)morpholine-4-carboxamide
(2S)-4-[[5-(2-chloro-6-methyl-4-pyridyl)-4-(3-cyanophenyl)thiazol-2-yl]carbamoyl]piperazine-2-carboxylic acid
N-[5-(2-chloro-6-methyl-4-pyridyl)-4-(3-cyanophenyl)thiazol-2-yl]-4-methyl-piperazine-1-carboxamide
N-[5-(2-chloro-6-methyl-4-pyridyl)-4-(3-cyanophenyl)thiazol-2-yl]-4-hydroxy-4-methylpiperidine-1-carboxamide
N-[5-(2-chloro-6-methyl-4-pyridyl)-4-(3-cyanophenyl)thiazol-2-yl]-4-cyano-4-methylpiperidine-1-carboxamide
(3S)-N-[5-(2-chloro-6-methyl-4-pyridyl)-4-(3-cyanophenyl)thiazol-2-yl]-3-(1-hydroxy-1-methylethyl)piperazine-1-carboxamide
N-[4-(3-cyanophenyl)-5-(2,6-dimethyl-4-pyridyl)thiazol-2-yl]-4-methyl-piperazine-1-carboxamide
4-cyano-N-[4-(3-cyanophenyl)-5-(2,6-dimethyl-4-pyridyl)thiazol-2-yl]piperidine-1-carboxamide
(3R)-3-cyano-N-[4-(3-cyanophenyl)-5-(2,6-dimethyl-4-pyridyl)thiazol-2-yl]pyrrolidine-1-carboxamide
N-[4-(3-cyanophenyl)-5-(2,6-dimethyl-4-pyridyl)thiazol-2-yl]-3,4,6,7,9,9a-hexahydro-1H-pyrazino[2,1-c][1,4]oxazine-8-carboxamide
N-[4-(3-cyanophenyl)-5-(2,6-dimethyl-4-pyridyl)thiazol-2-yl]-2-oxa-6-azaspiro[3.3]heptane-6-carboxamide
N-[4-(3-cyanophenyl)-5-(2,6-dimethyl-4-pyridyl)thiazol-2-yl]-3-oxo-piperazine-1-carboxamide
N4-[4-(3-cyanophenyl)-5-(2,6-dimethyl-4-pyridyl)thiazol-2-yl]morpholine-2,4-dicarboxamide
N-[4-(3-cyanophenyl)-5-(2,6-dimethyl-4-pyridyl)thiazol-2-yl]-4-methyl-3-oxo-piperazine-1-carboxamide
(2R)-N-[4-(3-cyanophenyl)-5-(2,6-dimethyl-4-pyridyl)thiazol-2-yl]-2-(hydroxymethyl)morpholine-4-carboxamide
4-acetyl-N-[4-(3-cyanophenyl)-5-(2,6-dimethyl-4-pyridyl)thiazol-2-yl]piperazine-1-carboxamide
N-[4-(3-cyanophenyl)-5-(2,6-dimethyl-4-pyridyl)thiazol-2-yl]-4-methylsulfonyl-piperazine-1-carboxamide
N-[4-(3-cyanophenyl)-5-(2,6-dimethyl-4-pyridyl)thiazol-2-yl]-4-sulfamoyl-piperazine-1-carboxamide
N-[4-(3-cyanophenyl)-5-(2,6-dimethyl-4-pyridyl)thiazol-2-yl]-1-imino-1-oxo-1,4-thiazinane-4-carboxamide
N-[4-(3-cyanophenyl)-5-(2,6-dimethyl-4-pyridyl)thiazol-2-yl]-2,4-dioxo-1,3,8-triazaspiro[4.5]decane-8-carboxamide
(2S)-N-[4-(3-cyanophenyl)-5-(2,6-dimethyl-4-pyridyl)thiazol-2-yl]-2-(hydroxymethyl)morpholine-4-carboxamide
4-cyano-N-[4-(3-cyanophenyl)-5-(2,6-dimethyl-4-pyridyl)thiazol-2-yl]-4-methoxy-piperidine-1-carboxamide
4-cyano-N-[4-(3-cyanophenyl)-5-(2,6-dimethyl-4-pyridyl)thiazol-2-yl]-4-(hydroxymethyl)piperidine-1-carboxamide
N-[4-(3-cyanophenyl)-5-(2,6-dimethyl-4-pyridyl)thiazol-2-yl]-1-oxo-1,4-thiazinane-4-carboxamide
N-[4-(3-cyanophenyl)-5-(2,6-dimethyl-4-pyridyl)thiazol-2-yl]-3-(hydroxymethyl)azetidine-1-carboxamide
3-cyano-N-[4-(3-cyanophenyl)-5-(2,6-dimethyl-4-pyridyl)thiazol-2-yl]azetidine-1-carboxamide
N-[4-(3-cyanophenyl)-5-(2,6-dimethyl-4-pyridyl)thiazol-2-yl]-1,1-dioxo-1,4-thiazinane-4-carboxamide
N-[4-(3-cyanophenyl)-5-(2,6-dimethyl-4-pyridyl)thiazol-2-yl]-1,3-dimethyl-2,4-dioxo-1,3,8-triazaspiro[4.5]decane-8-carboxamide
N 1-[4-(3-cyanophenyl)-5-(2,6-dimethyl-4-pyridyl)thiazol-2-yl]piperidine-1,4-dicarboxamide
N-[4-(3-cyanophenyl)-5-(2,6-dimethyl-4-pyridyl)thiazol-2-yl]-4-(2-hydroxyethyl)piperazine-1-carboxamide
N-[4-(3-cyanophenyl)-5-(2,6-dimethyl-4-pyridyl)thiazol-2-yl]-3-hydroxy-azetidine-1-carboxamide
N-[4-(3-cyanophenyl)-5-(2,6-dimethyl-4-pyridyl)thiazol-2-yl]-4-(2-hydroxyethyl)-3-oxo-piperazine-1-carboxamide
4,4-dicyano-N-[4-(3-cyanophenyl)-5-(2,6-dimethyl-4-pyridyl)thiazol-2-yl]piperidine-1-carboxamide
N-[4-(3-cyanophenyl)-5-(2,6-dimethyl-4-pyridyl)thiazol-2-yl]-3-(1-hydroxy-1-methylethyl)azetidine-1-carboxamide
N-[4-(3-cyanophenyl)-5-(2,6-dimethyl-4-pyridyl)thiazol-2-yl]-4-(2-hydroxy-2-methylpropyl)piperazine-1-carboxamide
N-[4-(3-cyanophenyl)-5-(2,6-dimethyl-4-pyridyl)thiazol-2-yl]-1-methyl-1,7-diazaspiro[3.4]octane-7-carboxamide
N-[4-(3-cyanophenyl)-5-(2,6-dimethyl-4-pyridyl)thiazol-2-yl]-4-(2-hydroxy-1,1-dimethylethyl)piperazine-1-carboxamide
(8aS)-N-[4-(3-cyanophenyl)-5-(2,6-dimethyl-4-pyridyl)thiazol-2-yl]-3,4,6,7,8,8a-hexahydro-1H-pyrrolo[1,2-a]pyrazine-2-carboxamide
(8aR)-N-[4-(3-cyanophenyl)-5-(2,6-dimethyl-4-pyridyl)thiazol-2-yl]-3,4,6,7,8,8a-hexahydro-1H-pyrrolo[1,2-a]pyrazine-2-carboxamide
N-[4-(3-cyanophenyl)-5-(2,6-dimethyl-4-pyridyl)thiazol-2-yl]-4-(oxetan-3-yl)piperazine-1-carboxamide
(3S)-N-[4-(3-cyanophenyl)-5-(2,6-dimethyl-4-pyridyl)thiazol-2-yl]-3-hydroxy-pyrrolidine-1-carboxamide
4-cyano-N-[4-(3-cyanophenyl)-5-(2,6-dimethyl-4-pyridyl)thiazol-2-yl]-4-(methoxymethyl)piperidine-1-carboxamide
4-cyano-N-[4-(3-cyanophenyl)-5-(2,6-dimethyl-4-pyridyl)thiazol-2-yl]-4-fluoro-piperidine-1-carboxamide
(3S)-3-cyano-N-[4-(3-cyanophenyl)-5-(2,6-dimethyl-4-pyridyl)thiazol-2-yl]pyrrolidine-1-carboxamide
N-[4-(3-cyanophenyl)-5-(2,6-dimethyl-4-pyridyl)thiazol-2-yl]-3-hydroxy-3-methyl-azetidine-1-carboxamide
(2R)-4-[[5-(2-chloro-6-methyl-4-pyridyl)-4-(3-cyanophenyl)thiazol-2-yl]carbamoyl]piperazine-2-carboxylic acid methyl (2R)-4-[[5-(2-chloro-6-methyl-4-pyridyl)-4-(3-cyanophenyl)thiazol-2-yl]carbamoyl]piperazine-2-carboxylate
(3S)-N-[5-(2-chloro-6-methyl-4-pyridyl)-4-(3-cyanophenyl)thiazol-2-yl]-3-(hydroxymethyl)piperazine-1-carboxamide
(3R)-N-[5-(2-chloro-6-methyl-4-pyridyl)-4-(3-cyanophenyl)thiazol-2-yl]-3-(hydroxymethyl)piperazine-1-carboxamide
(3S)-N-[5-(2-chloro-6-methyl-4-pyridyl)-4-(3-cyanophenyl)thiazol-2-yl]-3-methyl-piperazine-1-carboxamide
(3R)-N-[5-(2-chloro-6-methyl-4-pyridyl)-4-(3-cyanophenyl)thiazol-2-yl]-3-methyl-piperazine-1-carboxamide
(1S,4S)-N-[5-(2-chloro-6-methyl-4-pyridyl)-4-(3-cyanophenyl)thiazol-2-yl]-2,5-diazabicyclo[2.2.1]heptane-2-carboxamide
(3R)-N 1-[5-(2-chloro-6-methyl-4-pyridyl)-4-(3-cyanophenyl)thiazol-2-yl]piperazine-1,3-dicarboxamide
(3S)-N1-[5-(2-chloro-6-methyl-4-pyridyl)-4-(3-cyanophenyl)thiazol-2-yl]piperazine-1,3-dicarboxamide
4-amino-N-[5-(2-chloro-6-methyl-4-pyridyl)-4-(3-cyanophenyl)thiazol-2-yl]-4-methylpiperidine-1-carboxamide
4-cyano-N-[4-(3-cyanophenyl)-5-(2,6-dimethyl-4-pyridyl)thiazol-2-yl]-4-methyl-piperidine-1-carboxamide
(3S)-N-[4-(3-cyanophenyl)-5-(2,6-dimethyl-4-pyridyl)thiazol-2-yl]-3-methylsulfonyl-pyrrolidine-1-carboxamide
N-[4-(3-cyanophenyl)-5-(2,6-dimethyl-4-pyridyl)thiazol-2-yl]-2-oxa-7-azaspiro[3.5]nonane-7-carboxamide
N-[5-(2-chloro-6-methyl-4-pyridyl)-4-(3-cyanophenyl)thiazol-2-yl]-2-oxa-6-azaspiro[3.3]heptane-6-carboxamide
(1R,4R)-5-acetyl-N-[4-(3-cyanophenyl)-5-(2,6-dimethyl-4-pyridyl)thiazol-2-yl]-2,5-diazabicyclo[2.2.1]heptane-2-carboxamide
(1S,4S)-N-[4-(3-cyanophenyl)-5-(2,6-dimethyl-4-pyridyl)thiazol-2-yl]-5-methyl-2,5-diazabicyclo[2.2.1]heptane-2-carboxamide
N-[4-(3-cyanophenyl)-5-(2,6-dimethyl-4-pyridyl)thiazol-2-yl]-3-methyl-2-oxo-1,3,8-triazaspiro[4.5]decane-8-carboxamide
N-[5-[2,6-bis(trideuteriomethyl)-4-pyridyl]-4-(3-cyanophenyl)thiazol-2-yl]-2-oxa-6-azaspiro[3.3]heptane-6-carboxamide
N-[4-(3-cyanophenyl)-5-(3-fluoro-2,6-dimethyl-4-pyridyl)thiazol-2-yl]-2-oxa-6-azaspiro[3.3]heptane-6-carboxamide
(4aS,7aR)-N-[4-(3-cyanophenyl)-5-[2-(difluoromethyl)-6-methyl-4-pyridyl]thiazol-2-yl]-4-methyl-2,3,4a,5,7,7a-hexahydropyrrolo[3,4-b][1,4]oxazine-6-carboxamide
N-[4-(3-cyanophenyl)-5-[2-(difluoromethyl)-6-methyl-4-pyridyl]thiazol-2-yl]-9-methyl-6-oxa-2,9-diazaspiro[4.5]decane-2-carboxamide
N-[4-(3-cyanophenyl)-5-[2-(difluoromethyl)-6-methyl-4-pyridyl]thiazol-2-yl]-6-oxa-2,9-diazaspiro[4.5]decane-2-carboxamide
(4aS,7aS)-N-[4-(3-cyanophenyl)-5-[2-(difluoromethyl)-6-methyl-4-pyridyl]thiazol-2-yl]-4-methyl-2,3,4a,5,7,7a-hexahydropyrrolo[3,4-b][1,4]oxazine-6-carboxamide
N-[4-(3-cyanophenyl)-5-(2,6-dimethyl-4-pyridyl)thiazol-2-yl]-2-oxo-1,3,8-triazaspiro[4.5]decane-8-carboxamide
3-cyano-N-[4-(3-cyanophenyl)-5-[2-methyl-6-(oxetan-3-yl)-4-pyridyl]thiazol-2-yl]-3-methyl-pyrrolidine-1-carboxamide
4-cyano-N-[4-(3-cyanophenyl)-5-[2-methyl-6-(oxetan-3-yl)-4-pyridyl]thiazol-2-yl]-4-methylpiperidine-1-carboxamide
N-[4-(3-cyanophenyl)-5-(2,6-dimethyl-4-pyridyl)thiazol-2-yl]-3-morpholino-azetidine-1-carboxamide
N-[4-(3-cyanophenyl)-5-(2,6-dimethyl-4-pyridyl)thiazol-2-yl]-4-tetrahydropyran-4-yl-piperazine-1-carboxamide;
N-[4-(3-cyanophenyl)-5-(2,6-dimethyl-4-pyridyl)thiazol-2-yl]-3-pyrrolidin-1-yl-azetidine-1-carboxamide
N-[4-(3-cyanophenyl)-5-(2,6-dimethyl-4-pyridyl)thiazol-2-yl]-3-(dimethylamino)azetidine-1-carboxamide
N-[4-(3-cyanophenyl)-5-(2,6-dimethyl-4-pyridyl)thiazol-2-yl]-4-(morpholinomethyl)piperidine-1-carboxamide;
N-[4-(3-cyanophenyl)-5-(2,6-dimethyl-4-pyridyl)thiazol-2-yl]-3-methyl-2-oxo-1-oxa-3,8-diazaspiro[4.5]decane-8-carboxamide
N-[4-(3-cyanophenyl)-5-(2,6-dimethyl-4-pyridyl)thiazol-2-yl]-3-methyl-3,6-diazabicyclo[3.2.0]heptane-6-carboxamide
N-[4-(3-cyanophenyl)-5-(2,6-dimethyl-4-pyridyl)thiazol-2-yl]-1,4-diazabicyclo[3.2.1]octane-4-carboxamide
N-[4-(3-cyanophenyl)-5-(2,6-dimethyl-4-pyridyl)thiazol-2-yl]-3-oxo-5,6,8,8a-tetrahydro-1H-oxazolo[3,4-a]pyrazine-7-carboxamide
N-[4-(3-cyanophenyl)-5-(2,6-dimethyl-4-pyridyl)thiazol-2-yl]-2,2-dioxo-3,4,6,7,9,9a-hexahydro-1H-pyrazino[2,1-c][1,4]thiazine-8-carboxamide
N-[4-(3-cyanophenyl)-5-(2,6-dimethyl-4-pyridyl)thiazol-2-yl]-4-oxo-3,6,7,8,9,9a-hexahydro-1H-pyrido[1,2-a]pyrazine-2-carboxamide
N-[4-(3-cyanophenyl)-5-(2,6-dimethyl-4-pyridyl)thiazol-2-yl]-9-oxo-3,4,6,7,8,9a-hexahydro-1H-pyrazino[1,2-a]pyrazine-2-carboxamide
N-[4-(3-cyanophenyl)-5-(2,6-dimethyl-4-pyridyl)thiazol-2-yl]-6-oxo-1,3,4,8,9,9a-hexahydropyrazino[1,2-c][1,3]oxazine-2-carboxamide
N-[4-(3-cyanophenyl)-5-(2,6-dimethyl-4-pyridyl)thiazol-2-yl]-6-oxo-1,3,4,7,8,8a-hexahydropyrrolo[1,2-a]pyrazine-2-carboxamide
N-[4-(3-cyanophenyl)-5-[2-(difluoromethyl)-6-methyl-4-pyridyl]thiazol-2-yl]-1-oxo-1,4-thiazinane-4-carboxamide
N-[4-(3-cyanophenyl)-5-(2,6-dimethyl-4-pyridyl)thiazol-2-yl]-3-sulfamoyl-pyrrolidine-1-carboxamide
N-[4-(3-cyanophenyl)-5-(2,6-dimethyl-4-pyridyl)thiazol-2-yl]-3-oxo-2,7-diazaspiro[3.5]nonane-7-carboxamide
N-[4-(3-cyanophenyl)-5-(2,6-dimethyl-4-pyridyl)thiazol-2-yl]-4-pyrazol-1-yl-piperidine-1-carboxamide
N-[4-(3-cyanophenyl)-5-(2,6-dimethyl-4-pyridyl)thiazol-2-yl]-4-(1,2,4-oxadiazol-3-yl)piperidine-1-carboxamide
N-[4-(3-cyanophenyl)-5-(2,6-dimethyl-4-pyridyl)thiazol-2-yl]-1,3-dioxo-5,6,8,8a-tetrahydroimidazo[1,5-a]pyrazine-7-carboxamide
N-[4-(3-cyanophenyl)-5-(2,6-dimethyl-4-pyridyl)thiazol-2-yl]-1-oxo-2,8-diazaspiro[4.5]decane-8-carboxamide
N-[4-(3-cyanophenyl)-5-(2,6-dimethyl-4-pyridyl)thiazol-2-yl]-4-(1H-imidazol-2-yl)piperidine-1-carboxamide
N-[4-(3-cyanophenyl)-5-(2,6-dimethyl-4-pyridyl)thiazol-2-yl]-10-oxo-3,9-diazaspiro[5.5]undecane-3-carboxamide
N-[4-(3-cyanophenyl)-5-(2,6-dimethyl-4-pyridyl)thiazol-2-yl]-6,8-dihydro-5H-imidazo[1,2-a]pyrazine-7-carboxamide
N 1-[4-(3-cyanophenyl)-5-(2,6-dimethyl-4-pyridyl)thiazol-2-yl]-N4-methyl-piperidine-1,4-dicarboxamide
N-[4-(3-cyanophenyl)-5-(2,6-dimethyl-4-pyridyl)thiazol-2-yl]-8-oxa-2-azaspiro[4.5]decane-2-carboxamide
(3S)-N 1-[4-(3-cyanophenyl)-5-(2,6-dimethyl-4-pyridyl)thiazol-2-yl]pyrrolidine-1,3-dicarboxamide
N-[4-(3-cyanophenyl)-5-(2,6-dimethyl-4-pyridyl)thiazol-2-yl]-4-(2-oxoimidazolidin-1-yl)piperidine-1-carboxamide
N-[4-(3-cyanophenyl)-5-(2,6-dimethyl-4-pyridyl)thiazol-2-yl]-4-(1,2,4-oxadiazol-5-yl)piperidine-1-carboxamide
N-[4-(3-cyanophenyl)-5-(2,6-dimethyl-4-pyridyl)thiazol-2-yl]-1-methylimino-1-oxo-1,4-thiazinane-4-carboxamide
N 1-[4-(3-cyanophenyl)-5-(2,6-dimethyl-4-pyridyl)thiazol-2-yl]-N4-(2-hydroxyethyl)piperidine-1,4-dicarboxamide
N-[4-(3-cyanophenyl)-5-(2,6-dimethyl-4-pyridyl)thiazol-2-yl]-6-oxo-7-oxa-2,5-diazaspiro[3.5]nonane-2-carboxamide
N-[4-(3-cyanophenyl)-5-(2,6-dimethyl-4-pyridyl)thiazol-2-yl]-6,9-dioxo-1,3,4,7,8,9a-hexahydropyrazino[1,2-a]pyrazine-2-carboxamide
(3R)-N-[4-(3-cyanophenyl)-5-(2,6-dimethyl-4-pyridyl)thiazol-2-yl]-3-(1-hydroxy-1-methylethyl)pyrrolidine-1-carboxamide
N-[4-(3-cyanophenyl)-5-(2,6-dimethyl-4-pyridyl)thiazol-2-yl]-4-oxo-6,7,9,9a-tetrahydro-1H-pyrazino[2,1-c][1,4]oxazine-8-carboxamide
N-[4-(3-cyanophenyl)-5-(2,6-dimethyl-4-pyridyl)thiazol-2-yl]-2-methyl-3-oxo-2,8-diazaspiro[4.5]decane-8-carboxamide
N-[4-(3-cyanophenyl)-5-(2,6-dimethyl-4-pyridyl)thiazol-2-yl]-8-methyl-6,9-dioxo-3,4,7,9a-tetrahydro-1H-pyrazino[1,2-a]pyrazine-2-carboxamide
N-[4-(3-cyanophenyl)-5-(2,6-dimethyl-4-pyridyl)thiazol-2-yl]-3-oxo-2,8-diazaspiro[4.5]decane-8-carboxamide
N-[4-(3-cyanophenyl)-5-(2,6-dimethyl-4-pyridyl)thiazol-2-yl]-3-oxo-2,9-diazaspiro[5.5]undecane-9-carboxamide
4-cyano-N-[4-(3-cyanophenyl)-5-(2,6-dimethyl-4-pyridyl)thiazol-2-yl]-4-(1-hydroxy-1-methylethyl)piperidine-1-carboxamide
N-[4-(3-cyanophenyl)-5-(2,6-dimethyl-4-pyridyl)thiazol-2-yl]-6-oxo-3,4,7,8,9,9a-hexahydro-1H-pyrazino[1,2-c]pyrimidine-2-carboxamide
N-[4-(3-cyanophenyl)-5-(2,6-dimethyl-4-pyridyl)thiazol-2-yl]-6-oxo-3,4,7,8,9,9a-hexahydro-1H-pyrido[1,2-a]pyrazine-2-carboxamide
N-[4-(3-cyanophenyl)-5-(2,6-dimethyl-4-pyridyl)thiazol-2-yl]-6-hydroxy-6-methyl-2-azaspiro[3.3]heptane-2-carboxamide
N-[4-(3-cyanophenyl)-5-(2,6-dimethyl-4-pyridyl)thiazol-2-yl]-2-oxo-1,8-diazaspiro[4.5]decane-8-carboxamide
N-[4-(3-cyanophenyl)-5-(2,6-dimethyl-4-pyridyl)thiazol-2-yl]-2,2-dioxo-2λ^{6}-thia-6-azaspiro[3.3]heptane-6-carboxamide
N-[4-(3-cyanophenyl)-5-(2,6-dimethyl-4-pyridyl)thiazol-2-yl]-2-(1-hydroxy-1-methylethyl)morpholine-4-carboxamide
(3S)-N-[4-(3-cyanophenyl)-5-(2,6-dimethyl-4-pyridyl)thiazol-2-yl]-3-(1-hydroxy-1-methylethyl)pyrrolidine-1-carboxamide
(3aR,6aS)-N-[4-(3-cyanophenyl)-5-(2,6-dimethyl-4-pyridyl)thiazol-2-yl]-1,3,3a,4,6,6a-hexahydrofuro[3,4-c]pyrrole-5-carboxamide
N-[4-(3-cyanophenyl)-5-(2,6-dimethyl-4-pyridyl)thiazol-2-yl]-2-oxo-3-oxa-1,9-diazaspiro[5.5]undecane-9-carboxamide
(1S,5S)-N-[4-(3-cyanophenyl)-5-(2,6-dimethyl-4-pyridyl)thiazol-2-yl]-6-methyl-3,6-diazabicyclo[3.2.2]nonane-3-carboxamide
N-[4-(3-cyanophenyl)-5-(2,6-dimethyl-4-pyridyl)thiazol-2-yl]-1-methyl-2-oxo-1,3,8-triazaspiro[4.5]decane-8-carboxamide
(3aS,6aS)-N-[4-(3-cyanophenyl)-5-(2,6-dimethyl-4-pyridyl)thiazol-2-yl]-3-oxo-1,2,3a,4,6,6a-hexahydropyrrolo[3,4-c]pyrrole-5-carboxamide
N-[4-(3-cyanophenyl)-5-(2,6-dimethyl-4-pyridyl)thiazol-2-yl]-6-oxa-2-azaspiro[3.4]octane-2-carboxamide
N-[4-(3-cyanophenyl)-5-(2,6-dimethyl-4-pyridyl)thiazol-2-yl]-7-oxa-2-azaspiro[3.5]nonane-2-carboxamide
N-[4-(3-cyanophenyl)-5-(2,6-dimethyl-4-pyridyl)thiazol-2-yl]-6-oxo-2,7-diazaspiro[3.4]octane-2-carboxamide
N-[4-(3-cyanophenyl)-5-(2,6-dimethyl-4-pyridyl)thiazol-2-yl]-2-oxo-1-oxa-3,8-diazaspiro[4.5]decane-8-carboxamide
N-[4-(3-cyanophenyl)-5-(2,6-dimethyl-4-pyridyl)thiazol-2-yl]-6-oxo-2,5,7-triazaspiro[3.4]octane-2-carboxamide
N-[4-(3-cyanophenyl)-5-(2,6-dimethyl-4-pyridyl)thiazol-2-yl]-2-oxo-1,9-diazaspiro[5.5]undecane-9-carboxamide
N-[4-(3-cyanophenyl)-5-(2,6-dimethyl-4-pyridyl)thiazol-2-yl]-5-oxo-2,6-diazaspiro[3.4]octane-2-carboxamide
N-[4-(3-cyanophenyl)-5-(2,6-dimethyl-4-pyridyl)thiazol-2-yl]-3-methyl-2,4-dioxo-1,3,8-triazaspiro[4.5]decane-8-carboxamide
N-[4-(3-cyanophenyl)-5-(2,6-dimethyl-4-pyridyl)thiazol-2-yl]-4-ethyl-piperazine-1-carboxamide
N-[4-(3-cyanophenyl)-5-[2-methyl-6-(trifluoromethyl)-4-pyridyl]thiazol-2-yl]-4-(oxetan-3-yl)piperazine-1-carboxamide
1-[[4-(3-cyanophenyl)-5-(2,6-dimethyl-4-pyridyl)thiazol-2-yl]carbamoyl]piperidine-4-carboxylic acid
(3S)-1-[[4-(3-cyanophenyl)-5-(2,6-dimethyl-4-pyridyl)thiazol-2-yl]carbamoyl]pyrrolidine-3-carboxylic acid
3-cyano-N-[4-(3-cyanophenyl)-5-(2,6-dimethyl-4-pyridyl)thiazol-2-yl]-3-(hydroxymethyl)azetidine-1-carboxamide
(3R)-1-[[4-(3-cyanophenyl)-5-(2,6-dimethyl-4-pyridyl)thiazol-2-yl]carbamoyl]pyrrolidine-3-carboxylic acid
4-cyano-N-[4-(3-cyanophenyl)-5-(3-fluoro-2,6-dimethyl-4-pyridyl)thiazol-2-yl]-4-methylpiperidine-1-carboxamide
N-[4-(3-cyanophenyl)-5-(3-fluoro-2,6-dimethyl-4-pyridyl)thiazol-2-yl]-3-hydroxy-3-methyl-azetidine-1-carboxamide
3-cyano-N-[4-(3-cyanophenyl)-5-(3-fluoro-2,6-dimethyl-4-pyridyl)thiazol-2-yl]-3-methyl-azetidine-1-carboxamide
N-[4-(3-cyanophenyl)-5-(3-fluoro-2,6-dimethyl-4-pyridyl)thiazol-2-yl]-3-oxo-2,7-diazaspiro[3.5]nonane-7-carboxamide
3-cyano-N-[4-(3-cyanophenyl)-5-(2,6-dimethyl-4-pyridyl)thiazol-2-yl]-3-methyl-azetidine-1-carboxamide
1-[[4-(3-cyanophenyl)-5-(2,6-dimethyl-4-pyridyl)thiazol-2-yl]carbamoyl]azetidine-3-carboxylic acid
(2S)-N-[4-(3-cyanophenyl)-5-(2,6-dimethyl-4-pyridyl)thiazol-2-yl]-2-(hydroxymethyl)pyrrolidine-1-carboxamide
(2S)-N-[4-(3-cyanophenyl)-5-(2,6-dimethyl-4-pyridyl)thiazol-2-yl]-2-methyl-azetidine-1-carboxamide
(2S)-N-[4-(3-cyanophenyl)-5-(2,6-dimethyl-4-pyridyl)thiazol-2-yl]-2-(hydroxymethyl)azetidine-1-carboxamide
(3R)-N-[4-(3-cyanophenyl)-5-(2,6-dimethyl-4-pyridyl)thiazol-2-yl]-3-methyl-morpholine-4-carboxamide
(3S)-N-[4-(3-cyanophenyl)-5-(2,6-dimethyl-4-pyridyl)thiazol-2-yl]-3-(1-hydroxy-1-methylethyl)piperazine-1-carboxamide
(3S)-N-[4-(3-cyanophenyl)-5-(2,6-dimethyl-4-pyridyl)thiazol-2-yl]-3-(hydroxymethyl)morpholine-4-carboxamide
N-[4-(3-cyanophenyl)-5-(2,6-dimethyl-4-pyridyl)thiazol-2-yl]-2-methyl-1-oxo-2,8-diazaspiro[4.5]decane-8-carboxamide
(2R)-N-[4-(3-cyanophenyl)-5-(2,6-dimethyl-4-pyridyl)thiazol-2-yl]-2-methyl-azetidine-1-carboxamide
N-[4-(3-cyano-2-methyl-phenyl)-5-(2,6-dimethyl-4-pyridyl)thiazol-2-yl]-2-oxa-6-azaspiro[3.3]heptane-6-carboxamide
(3R)-N1-[4-(3-cyanophenyl)-5-(2,6-dimethyl-4-pyridyl)thiazol-2-yl]pyrrolidine-1,3-dicarboxamide
N-[4-(3-cyanophenyl)-5-(2,6-dimethyl-4-pyridyl)thiazol-2-yl]-2-oxa-7-azaspiro[4.4]nonane-7-carboxamide
N-[4-(3-cyanophenyl)-5-(2,6-dimethyl-4-pyridyl)thiazol-2-yl]-2-oxa-7-azaspiro[3.4]octane-7-carboxamide
(2S)-N-[4-(3-cyanophenyl)-5-(2,6-dimethyl-4-pyridyl)thiazol-2-yl]-2-(1-hydroxy-1-methylethyl)azetidine-1-carboxamide
(2R)-N-[4-(3-cyanophenyl)-5-(2,6-dimethyl-4-pyridyl)thiazol-2-yl]-2-(1-hydroxy-1-methylethyl)azetidine-1-carboxamide
N-[4-(3-cyanophenyl)-5-(2,6-dimethyl-4-pyridyl)thiazol-2-yl]-3-morpholino-pyrrolidine-1-carboxamide
(3R)-N-[4-(3-cyanophenyl)-5-(2,6-dimethyl-4-pyridyl)thiazol-2-yl]-3-methyl-5-oxo-piperazine-1-carboxamide
(3S)-N-[4-(3-cyanophenyl)-5-(2,6-dimethyl-4-pyridyl)thiazol-2-yl]-3-methyl-morpholine-4-carboxamide
N-[4-(3-cyanophenyl)-5-(2,6-dimethyl-4-pyridyl)thiazol-2-yl]-6-oxa-1-azaspiro[3.3]heptane-1-carboxamide
(3R)-N-[4-(3-cyanophenyl)-5-(2,6-dimethyl-4-pyridyl)thiazol-2-yl]-3-(hydroxymethyl)morpholine-4-carboxamide
N-[4-(3-cyanophenyl)-5-(2,6-dimethyl-4-pyridyl)thiazol-2-yl]-1,3-dimethyl-2-oxo-1,3,8-triazaspiro[4.5]decane-8-carboxamide
(3R)-N-[4-(3-cyanophenyl)-5-(2,6-dimethyl-4-pyridyl)thiazol-2-yl]-3-methylsulfonyl-pyrrolidine-1-carboxamide
(3S)-N-[4-(3-cyanophenyl)-5-(2,6-dimethyl-4-pyridyl)thiazol-2-yl]-3-methyl-5-oxo-piperazine-1-carboxamide
(2R)-N-[4-(3-cyanophenyl)-5-(2,6-dimethyl-4-pyridyl)thiazol-2-yl]-2-(hydroxymethyl)azetidine-1-carboxamide
N-[4-(3-cyanophenyl)-5-(2,6-dimethyl-4-pyridyl)thiazol-2-yl]-6-methyl-5-oxo-2,6-diazaspiro[3.4]octane-2-carboxamide
N-[4-(3-cyanophenyl)-5-(2,6-dimethyl-4-pyridyl)thiazol-2-yl]-1-oxa-6-azaspiro[3.3]heptane-6-carboxamide
N-[4-(3-cyanophenyl)-5-(2,6-dimethyl-4-pyridyl)thiazol-2-yl]-1-methyl-2-oxo-1,8-diazaspiro[4.5]decane-8-carboxamide
N-[4-(3-cyanophenyl)-5-(2,6-dimethyl-4-pyridyl)thiazol-2-yl]-4-methylsulfonyl-piperidine-1-carboxamide
N-[4-(3-cyanophenyl)-5-(2,6-dimethyl-4-pyridyl)thiazol-2-yl]-4-hydroxy-4-methyl-piperidine-1-carboxamide
N-[4-(3-cyanophenyl)-5-[2-(difluoromethyl)-6-methyl-4-pyridyl]thiazol-2-yl]-2-oxa-6-azaspiro[3.3]heptane-6-carboxamide
(4aR,7aS)-N-[4-(3-cyanophenyl)-5-(2,6-dimethyl-4-pyridyl)thiazol-2-yl]-4-methyl-2,3,4a,5,7,7a-hexahydropyrrolo[3,4-b][1,4]oxazine-6-carboxamide
N-[4-(3-cyanophenyl)-5-[2-methyl-6-(trifluoromethyl)-4-pyridyl]thiazol-2-yl]-2-oxa-6-azaspiro[3.3]heptane-6-carboxamide
N-[4-(3-cyanophenyl)-5-(2,6-dimethyl-4-pyridyl)thiazol-2-yl]piperazine-1-carboxamide (3R)-N-[4-(3-cyanophenyl)-5-(2,6-dimethyl-4-pyridyl)thiazol-2-yl]-3-methyl-piperazine-1-carboxamide
N-[4-(3-cyanophenyl)-5-(2,6-dimethyl-4-pyridyl)thiazol-2-yl]-5,8-diazaspiro[3.5]nonane-5-carboxamide
trans-N-[4-(3-cyanophenyl)-5-(2,6-dimethyl-4-pyridyl)thiazol-2-yl]-2,5-dimethyl-piperazine-1-carboxamide
4-amino-4-cyano-N-[4-(3-cyanophenyl)-5-(2,6-dimethyl-4-pyridyl)thiazol-2-yl]piperidine-1-carboxamide
N-[4-(3-cyanophenyl)-5-(2,6-dimethyl-4-pyridyl)thiazol-2-yl]-1-oxa-4,9-diazaspiro[5.5]undecane-9-carboxamide
N-[4-(3-cyanophenyl)-5-(2,6-dimethyl-4-pyridyl)thiazol-2-yl]-3,8-diazabicyclo[3.2.1]octane-8-carboxamide
N-[4-(3-cyanophenyl)-5-(2,6-dimethyl-4-pyridyl)thiazol-2-yl]-3,6-diazabicyclo[3.1.1]heptane-6-carboxamide
(1R,4R)-N-[4-(3-cyanophenyl)-5-(2,6-dimethyl-4-pyridyl)thiazol-2-yl]-2,5-diazabicyclo[2.2.2]octane-2-carboxamide
cis-(2S,5S)-N-[4-(3-cyanophenyl)-5-(2,6-dimethyl-4-pyridyl)thiazol-2-yl]-2,5-dimethylpiperazine-1-carboxamide
(2R,6R)-N-[4-(3-cyanophenyl)-5-(2,6-dimethyl-4-pyridyl)thiazol-2-yl]-2,6-dimethyl-piperazine-1-carboxamide
N-[4-(3-cyanophenyl)-5-(2,6-dimethyl-4-pyridyl)thiazol-2-yl]-2,2-dimethyl-piperazine-1-carboxamide 1-acetylimino-N-[4-(3-cyanophenyl)-5-(2,6-dimethyl-4-pyridyl)thiazol-2-yl]-1-oxo-1,4-thiazinane-4-carboxamide
N-[4-(3-cyanophenyl)-5-[2-(hydroxymethyl)-6-methyl-4-pyridyl]thiazol-2-yl]-2-oxa-6-azaspiro[3.3]heptane-6-carboxamide
N-[4-(3-cyanophenyl)-5-[2-(hydroxymethyl)-6-methyl-4-pyridyl]thiazol-2-yl]-3-oxo-2,7-diazaspiro[3.5]nonane-7-carboxamide
(3R)-N-[4-(3-cyanophenyl)-5-[2-(hydroxymethyl)-6-methyl-4-pyridyl]thiazol-2-yl]-3-(1-hydroxy-1-methyl-ethyl)pyrrolidine-1-carboxamide
N-[4-(3-cyanophenyl)-5-[2-(hydroxymethyl)-6-methyl-4-pyridyl]thiazol-2-yl]-1-oxo-1,4-thiazinane-4-carboxamide
N-[4-(3-cyanophenyl)-5-[2-(hydroxymethyl)-6-methyl-4-pyridyl]thiazol-2-yl]-4-(oxetan-3-yl)piperazine-1-carboxamide
N-[4-(3-cyanophenyl)-5-[2-(hydroxymethyl)-6-methyl-4-pyridyl]thiazol-2-yl]-1,3-dimethyl-2,4-dioxo-1,3,8-triazaspiro[4.5]decane-8-carboxamide
4-cyano-N-[4-(3-cyanophenyl)-5-[2-(hydroxymethyl)-6-methyl-4-pyridyl]thiazol-2-yl]piperidine-1-carboxamide
4-cyano-N-[4-(3-cyanophenyl)-5-[2-(hydroxymethyl)-6-methyl-4-pyridyl]thiazol-2-yl]-4-(hydroxymethyl)piperidine-1-carboxamide
4,4-dicyano-N-[4-(3-cyanophenyl)-5-[2-(hydroxymethyl)-6-methyl-4-pyridyl]thiazol-2-yl]piperidine-1-carboxamide
4-cyano-N-[4-(3-cyanophenyl)-5-[2-(hydroxymethyl)-6-methyl-4-pyridyl]thiazol-2-yl]-4-methoxy-piperidine-1-carboxamide
N-[4-(3-cyanophenyl)-5-(3-fluoro-2,6-dimethyl-4-pyridyl)thiazol-2-yl]-1-oxo-1,4-thiazinane-4-carboxamide
1-cyanoimino-N-[4-(3-cyanophenyl)-5-(2,6-dimethyl-4-pyridyl)thiazol-2-yl]-1-oxo-1,4-thiazinane-4-carboxamide
4-cyano-N-[4-(3-cyanophenyl)-5-[2-(1-hydroxy-1-methyl-ethyl)-6-methyl-4-pyridyl]thiazol-2-yl]-4-methyl-piperidine-1-carboxamide
N-[4-(3-cyanophenyl)-5-[2-(1-hydroxy-1-methyl-ethyl)-6-methyl-4-pyridyl]thiazol-2-yl]-6-oxa-2-azaspiro[3.4]octane-2-carboxamide
N-[4-(3-cyanophenyl)-5-[2-(1-hydroxy-1-methyl-ethyl)-6-methyl-4-pyridyl]thiazol-2-yl]-2-oxa-6-azaspiro[3.3]heptane-6-carboxamide
N-[4-(3-cyanophenyl)-5-[2-(1-hydroxyethyl)-6-methyl-4-pyridyl]thiazol-2-yl]-2-oxa-6-azaspiro[3.3]heptane-6-carboxamide
rac-(3S,5R)-N-[4-(3-cyanophenyl)-5-(2,6-dimethyl-4-pyridyl)thiazol-2-yl]-3,5-dimethylpiperazine-1-carboxamide
(3S)-N-[4-(3-cyanophenyl)-5-(2,6-dimethyl-4-pyridyl)thiazol-2-yl]-3-(trifluoromethyl)piperazine-1-carboxamide
N-[4-(3-cyanophenyl)-5-(2,6-dimethyl-4-pyridyl)thiazol-2-yl]-5-oxa-2,8-diazaspiro[3.5]nonane-2-carboxamide
N-[4-(3-cyanophenyl)-5-(2,6-dimethyl-4-pyridyl)thiazol-2-yl]-3,8-diazabicyclo[3.2.1]octane-3-carboxamide
N-[4-(3-cyanophenyl)-5-(2,6-dimethyl-4-pyridyl)thiazol-2-yl]-6-oxa-2,9-diazaspiro[4.5]decane-2-carboxamide
(3R)-N-[4-(3-cyanophenyl)-5-(2,6-dimethyl-4-pyridyl)thiazol-2-yl]-3-(trifluoromethyl)piperazine-1-carboxamide
(3S)-3-cyano-N-[4-(3-cyanophenyl)-5-(2,6-dimethyl-4-pyridyl)thiazol-2-yl]piperazine-1-carboxamide
(3R)-3-cyano-N-[4-(3-cyanophenyl)-5-(2,6-dimethyl-4-pyridyl)thiazol-2-yl]piperazine-1-carboxamide
N-[4-(3-cyanophenyl)-5-(2,6-dimethyl-4-pyridyl)thiazol-2-yl]-4,7-diazaspiro[2.5]octane-7-carboxamide
(3S,5S)-N-[4-(3-cyanophenyl)-5-(2,6-dimethyl-4-pyridyl)thiazol-2-yl]-3,5-dimethyl-piperazine-1-carboxamide
(2S)-N-[4-(3-cyanophenyl)-5-(2,6-dimethyl-4-pyridyl)thiazol-2-yl]-2-methyl-piperazine-1-carboxamide
(2R)-N-[4-(3-cyanophenyl)-5-(2,6-dimethyl-4-pyridyl)thiazol-2-yl]-2-(hydroxymethyl)piperazine-1-carboxamide
(2S)-N-[4-(3-cyanophenyl)-5-(2,6-dimethyl-4-pyridyl)thiazol-2-yl]-2-(hydroxymethyl)piperazine-1-carboxamide
N-[4-(3-cyanophenyl)-5-(2,6-dimethyl-4-pyridyl)thiazol-2-yl]-3,6-diazabicyclo[3.1.1]heptane-3-carboxamide
(2R)-N-[4-(3-cyanophenyl)-5-(2,6-dimethyl-4-pyridyl)thiazol-2-yl]-2-methyl-piperazine-1-carboxamide
N-[4-(3-cyanophenyl)-5-(2,6-dimethyl-4-pyridyl)thiazol-2-yl]-9-oxa-2,6-diazaspiro[4.5]decane-2-carboxamide
(3R)-N-[4-(3-cyanophenyl)-5-[2-methyl-6-(trifluoromethyl)-4-pyridyl]thiazol-2-yl]-3-methylpiperazine-1-carboxamide
(4aS,7aS)-N-[4-(3-cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide
(4aS,7aR)-N-[4-(3-cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide
rac-(4aS,7aS)-N-[4-(3-cyanophenyl)-5-(2,6-dimethyl-4-pyridyl)thiazol-2-yl]-3,4,4a,5,7,7a-hexahydro-2H-pyrrolo[3,4-b][1,4]oxazine-6-carboxamide
N-[4-(3-cyanophenyl)-5-(2,6-dimethyl-4-pyridyl)thiazol-2-yl]-2-cyclopropyl-piperazine-1-carboxamide
N-[4-(3-cyanophenyl)-5-(2,6-dimethyl-4-pyridyl)thiazol-2-yl]-2-(cyclopropylmethyl)piperazine-1-carboxamide
N-[4-(3-cyanophenyl)-5-(2,6-dimethyl-4-pyridyl)thiazol-2-yl]-3,3-dimethyl-piperazine-1-carboxamide
N-[4-(3-cyanophenyl)-5-(2-methoxy-6-methyl-4-pyridyl)thiazol-2-yl]-1-imino-1-oxo-1,4-thiazinane-4-carboxamide
N-[4-(3-cyanophenyl)-5-[2-(difluoromethyl)-6-methyl-4-pyridyl]thiazol-2-yl]-1-imino-1-oxo-1,4-thiazinane-4-carboxamide
N-[4-(3-cyanophenyl)-5-(2,6-dichloro-4-pyridyl)thiazol-2-yl]-2-oxa-6-azaspiro[3.3]heptane-6-carboxamide
N-[4-(3-cyanophenyl)-5-[2-methyl-6-(trifluoromethyl)-4-pyridyl]thiazol-2-yl]-1-oxo-1,4-thiazinane-4-carboxamide
N-[4-(3-cyanophenyl)-5-(2-methoxy-6-methyl-4-pyridyl)thiazol-2-yl]-1-oxo-1,4-thiazinane-4-carboxamide
N-[4-(3-cyanophenyl)-5-[2-methyl-6-(oxetan-3-yl)-4-pyridyl]thiazol-2-yl]-2-oxa-6-azaspiro[3.3]heptane-6-carboxamide
N-[5-(2-cyano-6-methyl-4-pyridyl)-4-(3-cyanophenyl)thiazol-2-yl]-1-oxo-1,4-thiazinane-4-carboxamide
N-[4-(3-cyanophenyl)-5-[2-(fluoromethyl)-6-methyl-4-pyridyl]thiazol-2-yl]-2-oxa-6-azaspiro[3.3]heptane-6-carboxamide
N-[4-(3-cyanophenyl)-5-(2-cyclopropyl-6-methyl-4-pyridyl)thiazol-2-yl]-2-oxa-6-azaspiro[3.3]heptane-6-carboxamide
N-[4-(3-cyanophenyl)-5-(2-isopropyl-6-methyl-4-pyridyl)thiazol-2-yl]-2-oxa-6-azaspiro[3.3]heptane-6-carboxamide
N-[4-(3-cyanophenyl)-5-(2-ethyl-6-methyl-4-pyridyl)thiazol-2-yl]-2-oxa-6-azaspiro[3.3]heptane-6-carboxamide
N-[4-(3-cyanophenyl)-5-(2,6-dimethyl-1-oxido-pyridin-1-ium-4-yl)thiazol-2-yl]-2-oxa-6-azaspiro[3.3]heptane-6-carboxamide
4-cyano-N-[4-(3-cyano-2-methyl-phenyl)-5-(2,6-dimethyl-4-pyridyl)thiazol-2-yl]-4-methylpiperidine-1-carboxamide
N-[5-(2-acetamido-6-methyl-4-pyridyl)-4-(3-cyanophenyl)thiazol-2-yl]-2-oxa-6-azaspiro[3.3]heptane-6-carboxamide
N-[5-[2-(acetamidomethyl)-6-methyl-4-pyridyl]-4-(3-cyanophenyl)thiazol-2-yl]-2-oxa-6-azaspiro[3.3]heptane-6-carboxamide
N-[4-(3-cyanophenyl)-5-[2-(methoxymethyl)-6-methyl-4-pyridyl]thiazol-2-yl]-2-oxa-6-azaspiro[3.3]heptane-6-carboxamide
N-[4-(3-cyanophenyl)-5-[2-(1-methoxyethyl)-6-methyl-4-pyridyl]thiazol-2-yl]-2-oxa-6-azaspiro[3.3]heptane-6-carboxamide
N-[5-[2-chloro-6-(1-hydroxy-1-methyl-ethyl)-4-pyridyl]-4-(3-cyanophenyl)thiazol-2-yl]-2-oxa-6-azaspiro[3.3]heptane-6-carboxamide
4-cyano-N-[4-(3-cyanophenyl)-5-[2-(hydroxymethyl)-6-methyl-4-pyridyl]thiazol-2-yl]-4-methylpiperidine-1-carboxamide
N-[4-(3-cyanophenyl)-5-[2-(hydroxymethyl)-6-(trifluoromethyl)-4-pyridyl]thiazol-2-yl]-2-oxa-6-azaspiro[3.3]heptane-6-carboxamide
3-cyano-N-[4-(3-cyanophenyl)-5-(2,6-dimethyl-4-pyridyl)thiazol-2-yl]-3-methyl-pyrrolidine-1-carboxamide
N-[5-[2,6-bis(trideuteriomethyl)-4-pyridyl]-4-(3-cyanophenyl)thiazol-2-yl]-4-cyano-4-methylpiperidine-1-carboxamide
N-[4-(3-cyanophenyl)-5-[2-[(1R)-1-hydroxyethyl]-6-methyl-4-pyridyl]thiazol-2-yl]-2-oxa-6-azaspiro[3.3]heptane-6-carboxamide
N-[4-(3-cyanophenyl)-5-[2-[(1S)-1-hydroxyethyl]-6-methyl-4-pyridyl]thiazol-2-yl]-2-oxa-6-azaspiro[3.3]heptane-6-carboxamide
(3R)-3-cyano-N-[4-(3-cyanophenyl)-5-(2,6-dimethyl-4-pyridyl)thiazol-2-yl]-3-methyl-pyrrolidine-1-carboxamide
(3S)-3-cyano-N-[4-(3-cyanophenyl)-5-(2,6-dimethyl-4-pyridyl)thiazol-2-yl]-3-methyl-pyrrolidine-1-carboxamide.

Particular compounds of the present invention include any of the compounds described in the example section of the present application, or a pharmaceutically acceptable salt or solvate thereof, and, in particular, any of the following:
N-[5-(2-chloro-6-methyl-4-pyridyl)-4-(3-cyanophenyl)thiazol-2-yl]morpholine-4-carboxamide;
N-[5-(2-chloro-6-methyl-4-pyridyl)-4-(3-cyanophenyl)thiazol-2-yl]-3-(1-hydroxy-1-methylethyl)pyrrolidine-1-carboxamide;
(2R)-N-[5-(2-chloro-6-methyl-4-pyridyl)-4-(3-cyanophenyl)thiazol-2-yl]-2-(1-hydroxy-1-methylethyl)pyrrolidine-1-carboxamide;
N-[5-(2-chloro-6-methyl-4-pyridyl)-4-(3-cyanophenyl)thiazol-2-yl]piperazine-1-carboxamide;
N-[5-(2-chloro-6-methyl-4-pyridyl)-4-(3-cyanophenyl)thiazol-2-yl]pyrrolidine-1-carboxamide;
N-[5-(2-chloro-6-methyl-4-pyridyl)-4-(3-cyanophenyl)thiazol-2-yl]-2-(2-hydroxy-2-methylpropyl)pyrrolidine-1-carboxamide;
N-[5-(2-chloro-6-methyl-4-pyridyl)-4-(3-cyanophenyl)thiazol-2-yl]-3-(1-hydroxy-1-methylethyl)morpholine-4-carboxamide;
(2S)-4-[[5-(2-chloro-6-methyl-4-pyridyl)-4-(3-cyanophenyl)thiazol-2-yl]carbamoyl]piperazine-2-carboxylic acid;
N-[5-(2-chloro-6-methyl-4-pyridyl)-4-(3-cyanophenyl)thiazol-2-yl]-4-methyl-piperazine-1-carboxamide;
N-[5-(2-chloro-6-methyl-4-pyridyl)-4-(3-cyanophenyl)thiazol-2-yl]-4-hydroxy-4-methylpiperidine-1-carboxamide;
N-[5-(2-chloro-6-methyl-4-pyridyl)-4-(3-cyanophenyl)thiazol-2-yl]-4-cyano-4-methylpiperidine-1-carboxamide;
(3S)-N-[5-(2-chloro-6-methyl-4-pyridyl)-4-(3-cyanophenyl)thiazol-2-yl]-3-(1-hydroxy-1-methylethyl)piperazine-1-carboxamide formate;
(3R)-N-[5-(2-chloro-6-methyl-4-pyridyl)-4-(3-cyanophenyl)thiazol-2-yl]-3-(1-hydroxy-1-methylethyl)piperazine-1-carboxamide formate
N-[5-(2-chloro-6-methyl-4-pyridyl)-4-(3-cyanophenyl)thiazol-2-yl]-4-(oxetan-3-yl)piperazine-1-carboxamide;
N-[5-(2-chloro-6-methyl-4-pyridyl)-4-(3-cyanophenyl)thiazol-2-yl]-1-imino-1-oxo-1,4-thiazinane-4-carboxamide;
N-[4-(3-cyanophenyl)-5-(2,6-dimethyl-4-pyridyl)thiazol-2-yl]-4-methyl-piperazine-1-carboxamide;
4-cyano-N-[4-(3-cyanophenyl)-5-(2,6-dimethyl-4-pyridyl)thiazol-2-yl]piperidine-1-carboxamide;
N-[4-(3-cyanophenyl)-5-(2,6-dimethyl-4-pyridyl)thiazol-2-yl]-1-imino-1-oxo-1,4-thiazinane-4-carboxamide;
N-[4-(3-cyanophenyl)-5-(2,6-dimethyl-4-pyridyl)thiazol-2-yl]-4-(2-hydroxyethyl)piperazine-1-carboxamide;
N-[4-(3-cyanophenyl)-5-(2,6-dimethyl-4-pyridyl)thiazol-2-yl]-4-(oxetan-3-yl)piperazine-1-carboxamide;
(3S)-N-[4-(3-cyanophenyl)-5-(2,6-dimethyl-4-pyridyl)thiazol-2-yl]-3-hydroxy-pyrrolidine-1-carboxamide;
4-cyano-N-[4-(3-cyanophenyl)-5-(2,6-dimethyl-4-pyridyl)thiazol-2-yl]-4-(methoxymethyl)piperidine-1-carboxamide;
4-cyano-N-[4-(3-cyanophenyl)-5-(2,6-dimethyl-4-pyridyl)thiazol-2-yl]-4-fluoro-piperidine-1-carboxamide;
(3S)-3-cyano-N-[4-(3-cyanophenyl)-5-(2,6-dimethyl-4-pyridyl)thiazol-2-yl]pyrrolidine-1-carboxamide;
N-[4-(3-cyanophenyl)-5-(2,6-dimethyl-4-pyridyl)thiazol-2-yl]-3-hydroxy-3-methyl-azetidine-1-carboxamide;
(3R)-3-cyano-N-[4-(3-cyanophenyl)-5-(2,6-dimethyl-4-pyridyl)thiazol-2-yl]pyrrolidine-1-carboxamide;
N-[4-(3-cyanophenyl)-5-(2,6-dimethyl-4-pyridyl)thiazol-2-yl]-3,4,6,7,9,9a-hexahydro-1H-pyrazino[2,1-c][1,4]oxazine-8-carboxamide;
N-[4-(3-cyanophenyl)-5-(2,6-dimethyl-4-pyridyl)thiazol-2-yl]-2-oxa-6-azaspiro[3.3]heptane-6-carboxamide;
N-[4-(3-cyanophenyl)-5-(2,6-dimethyl-4-pyridyl)thiazol-2-yl]-3-oxo-piperazine-1-carboxamide
N4-[4-(3-cyanophenyl)-5-(2,6-dimethyl-4-pyridyl)thiazol-2-yl]morpholine-2,4-dicarboxamide
N-[4-(3-cyanophenyl)-5-(2,6-dimethyl-4-pyridyl)thiazol-2-yl]-4-methyl-3-oxo-piperazine-1-carboxamide;
(2R)-N-[4-(3-cyanophenyl)-5-(2,6-dimethyl-4-pyridyl)thiazol-2-yl]-2-(hydroxymethyl)morpholine-4-carboxamide;
4-acetyl-N-[4-(3-cyanophenyl)-5-(2,6-dimethyl-4-pyridyl)thiazol-2-yl]piperazine-1-carboxamide;
N-[4-(3-cyanophenyl)-5-(2,6-dimethyl-4-pyridyl)thiazol-2-yl]-4-methylsulfonyl-piperazine-1-carboxamide;
N-[4-(3-cyanophenyl)-5-(2,6-dimethyl-4-pyridyl)thiazol-2-yl]-4-sulfamoyl-piperazine-1-carboxamide;
N-[4-(3-cyanophenyl)-5-(2,6-dimethyl-4-pyridyl)thiazol-2-yl]-2,4-dioxo-1,3,8-triazaspiro[4.5]decane-8-carboxamide;
(2S)-N-[4-(3-cyanophenyl)-5-(2,6-dimethyl-4-pyridyl)thiazol-2-yl]-2-(hydroxymethyl)morpholine-4-carboxamide;
4-cyano-N-[4-(3-cyanophenyl)-5-(2,6-dimethyl-4-pyridyl)thiazol-2-yl]-4-methoxy-piperidine-1-carboxamide;
4-cyano-N-[4-(3-cyanophenyl)-5-(2,6-dimethyl-4-pyridyl)thiazol-2-yl]-4-(hydroxymethyl)piperidine-1-carboxamide;
N-[4-(3-cyanophenyl)-5-(2,6-dimethyl-4-pyridyl)thiazol-2-yl]-1-oxo-1,4-thiazinane-4-carboxamide;
N-[4-(3-cyanophenyl)-5-(2,6-dimethyl-4-pyridyl)thiazol-2-yl]-3-(hydroxymethyl)azetidine-1-carboxamide;
3-cyano-N-[4-(3-cyanophenyl)-5-(2,6-dimethyl-4-pyridyl)thiazol-2-yl]azetidine-1-carboxamide;
N-[4-(3-cyanophenyl)-5-(2,6-dimethyl-4-pyridyl)thiazol-2-yl]-1,1-dioxo-1,4-thiazinane-4-carboxamide
N-[4-(3-cyanophenyl)-5-(2,6-dimethyl-4-pyridyl)thiazol-2-yl]-1,3-dimethyl-2,4-dioxo-1,3,8-triazaspiro[4.5]decane-8-carboxamide
N 1-[4-(3-cyanophenyl)-5-(2,6-dimethyl-4-pyridyl)thiazol-2-yl]piperidine-1,4-dicarboxamide
N-[4-(3-cyanophenyl)-5-(2,6-dimethyl-4-pyridyl)thiazol-2-yl]-3-hydroxy-azetidine-1-carboxamide
N-[4-(3-cyanophenyl)-5-(2,6-dimethyl-4-pyridyl)thiazol-2-yl]-4-(2-hydroxyethyl)-3-oxo-piperazine-1-carboxamide;
4,4-Dicyano-N-[4-(3-cyanophenyl)-5-(2,6-dimethyl-4-pyridyl)thiazol-2-yl]piperidine-1-carboxamide;
N-[4-(3-cyanophenyl)-5-(2,6-dimethyl-4-pyridyl)thiazol-2-yl]-3-(1-hydroxy-1-methylethyl)azetidine-1-carboxamide;
(2R)-4-[[5-(2-chloro-6-methyl-4-pyridyl)-4-(3-cyanophenyl)thiazol-2-yl]carbamoyl]piperazine-2-carboxylic acid;
methyl (2R)-4-[[5-(2-chloro-6-methyl-4-pyridyl)-4-(3-cyanophenyl)thiazol-2-yl]carbamoyl]piperazine-2-carboxylate;
(3S)-N-[5-(2-chloro-6-methyl-4-pyridyl)-4-(3-cyanophenyl)thiazol-2-yl]-3-(hydroxymethyl)piperazine-1-carboxamide;
(3R)-N-[5-(2-chloro-6-methyl-4-pyridyl)-4-(3-cyanophenyl)thiazol-2-yl]-3-(hydroxymethyl)piperazine-1-carboxamide;
(3S)-N-[5-(2-chloro-6-methyl-4-pyridyl)-4-(3-cyanophenyl)thiazol-2-yl]-3-methyl-piperazine-1-carboxamide;
(3R)-N-[5-(2-chloro-6-methyl-4-pyridyl)-4-(3-cyanophenyl)thiazol-2-yl]-3-methyl-piperazine-1-carboxamide;
(1S,4S)-N-[5-(2-chloro-6-methyl-4-pyridyl)-4-(3-cyanophenyl)thiazol-2-yl]-2,5-diazabicyclo[2.2.1]heptane-2-carboxamide;
(3R)-N 1-[5-(2-chloro-6-methyl-4-pyridyl)-4-(3-cyanophenyl)thiazol-2-yl]piperazine-1,3-dicarboxamide;
(3S)-N1-[5-(2-chloro-6-methyl-4-pyridyl)-4-(3-cyanophenyl)thiazol-2-yl]piperazine-1,3-dicarboxamide;
4-Amino-N-[5-(2-chloro-6-methyl-4-pyridyl)-4-(3-cyanophenyl)thiazol-2-yl]-4-methylpiperidine-1-carboxamide;
4-cyano-N-[4-(3-cyanophenyl)-5-(2,6-dimethyl-4-pyridyl)thiazol-2-yl]-4-methyl-piperidine-1-carboxamide;
N-[4-(3-cyanophenyl)-5-(2,6-dimethyl-4-pyridyl)thiazol-2-yl]piperazine-1-carboxamide;
(3R)-N-[4-(3-cyanophenyl)-5-(2,6-dimethyl-4-pyridyl)thiazol-2-yl]-3-methyl-piperazine-1-carboxamide;
4-Amino-4-cyano-N-[4-(3-cyanophenyl)-5-(2,6-dimethyl-4-pyridyl)thiazol-2-yl]piperidine-1-carboxamide; and
1-acetylimino-N-[4-(3-cyanophenyl)-5-(2,6-dimethyl-4-pyridyl)thiazol-2-yl]-1-oxo-1,4-thiazinane-4-carboxamide.

The various functional groups and substituents making up the compounds of the formula (I) are typically chosen such that the molecular weight of the compound of the formula (I) does not exceed 1000. More usually, the molecular weight of the compound will be less than 900, for example less than 800, or less than 750, or less than 700, or less than 650. More preferably, the molecular weight is less than 600 and, for example, is 550 or less.

A suitable pharmaceutically acceptable salt of a compound of the invention is, for example, an acidaddition salt of a compound of the invention which is sufficiently basic, for example, an acidaddition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamine, trimethylamine, piperidine, morpholine or tris(2hydroxyethyl)amine.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are nonsuperimposable mirror images of each other are termed "enantiomers". When a compound has an asymmetric center-, for example, it is bonded to four different groups, a pair of enantiomers is possible. An enantiomer can be characterized by the absolute configuration of its asymmetric center and is described by the R and Ssequencing rules of Cahn and Prelog, or by the manner in which the molecule rotates the plane of polarized light and designated as dextrorotatory or -levorotatory- (i.e., as (+) or (-)-isomers respectively). A chiral compound can exist as either individual enantiomer or as a mixture thereof. A mixture containing equal proportions of the enantiomers is called a "racemic mixture".

The compounds of this invention may possess one or more asymmetric centers; such compounds can therefore be produced as individual (R) or (S)stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are wellknown in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E and Z isomers). It is to be understood that the present invention encompasses all optical, diastereoisomers and geometric isomers and mixtures thereof that possess antiproliferative activity.

The present invention also encompasses compounds of the invention as defined herein which comprise one or more isotopic substitutions. For Example, H may be in any isotopic form, including 1H, 2H(D), and 3H (T); C may be in any isotopic form, including 12C, 13C, and 14C; and O may be in any isotopic form, including 160 and18O; and the like.

It is also to be understood that certain compounds of the formula (I) may exist in solvated as well as unsolvated forms such as, for example, hydrated forms. It is to be understood that the invention encompasses all such solvated forms that possess antiproliferative activity.

It is also to be understood that certain compounds of the formula I may exhibit polymorphism, and that the invention encompasses all such forms that possess antiproliferative activity.

Compounds of the formula I may exist in a number of different tautomeric forms and references to compounds of the formula I include all such forms. For the avoidance of doubt, where a compound can exist in one of several tautomeric forms, and only one is specifically described or shown, all others are nevertheless embraced by formula I. Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

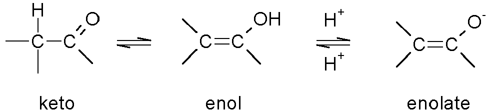

Compounds of the formula I containing an amine function may also form N-oxides. A reference herein to a compound of the formula I that contains an amine function also includes the N-oxide. Where a compound contains several amine functions, one or more than one nitrogen atom may be oxidised to form an N-oxide. Particular Examples of N-oxides are the N-oxides of a *tertiary* amine or a nitrogen atom of a nitrogen-containing heterocycle. N-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with m-chloroperoxybenzoic acid (mCPBA), for example, in an inert solvent such as dichloromethane.

The compounds of formula (I) may be administered in the form of a prodrug which is broken down in the human or animal body to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include *in vivo* cleavable ester derivatives that may be formed at a carboxy group or a hydroxy group in a compound of the formula (I) and in-vivo cleavable amide derivatives that may be formed at a carboxy group or an amino group in a compound of the formula (I).

Accordingly, the present invention includes those compounds of the formula (I) as defined hereinbefore when made available by organic synthesis and when made available within the human or animal body by way of cleavage of a pro-drug thereof. Accordingly, the present invention includes those compounds of the formula I that are produced by organic synthetic means and also such compounds that are produced in the human or animal body by way of metabolism of a precursor compound, that is a compound of the formula (I) may be a synthetically-produced compound or a metabolically-produced compound.

A suitable pharmaceutically acceptable pro-drug of a compound of the formula (I) is one that is based on reasonable medical judgement as being suitable for administration to the human or animal body without undesirable pharmacological activities and without undue toxicity.

Various forms of pro-drug have been described, for example in the following documents :-
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

A suitable pharmaceutically acceptable pro-drug of a compound of the formula I that possesses a carboxy group is, for example, an *in vivo* cleavable ester thereof. An *in vivo* cleavable ester of a compound of the formula I containing a carboxy group is, for example, a pharmaceutically acceptable ester which is cleaved in the human or animal body to produce the parent acid. Suitable pharmaceutically acceptable esters for carboxy include C1-6alkyl esters such as methyl, ethyl and *tert*-butyl, C1-6alkoxymethyl esters such as methoxymethyl esters, C1-6alkanoyloxymethyl esters such as pivaloyloxymethyl esters, 3-phthalidyl esters, C3-8cycloalkylcarbonyloxy- C1-6alkyl esters such as cyclopentylcarbonyloxymethyl and 1-cyclohexylcarbonyloxyethyl esters, 2-oxo-1,3-dioxolenylmethyl esters such as 5-methyl-2-oxo-1,3-dioxolen-4-ylmethyl esters and C1-6alkoxycarbonyloxy- C1-6alkyl esters such as methoxycarbonyloxymethyl and 1-methoxycarbonyloxyethyl esters.

A suitable pharmaceutically acceptable pro-drug of a compound of the Formula (I) that possesses a hydroxy group is, for example, an *in vivo* cleavable ester or ether thereof. An *in vivo* cleavable ester or ether of a compound of the formula I containing a hydroxy group is, for example, a pharmaceutically acceptable ester or ether which is cleaved in the human or animal body to produce the parent hydroxy compound. Suitable pharmaceutically acceptable ester forming groups for a hydroxy group include inorganic esters such as phosphate esters (including phosphoramidic cyclic esters). Further suitable pharmaceutically acceptable ester forming groups for a hydroxy group include C1-10alkanoyl groups such as acetyl, benzoyl, phenylacetyl and substituted benzoyl and phenylacetyl groups, C1-10alkoxycarbonyl groups such as ethoxycarbonyl, N,N -(C1-6)2carbamoyl, 2-dialkylaminoacetyl and 2-carboxyacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-4alkyl)piperazin-1-ylmethyl. Suitable pharmaceutically acceptable ether forming groups for a hydroxy group include α-acyloxyalkyl groups such as acetoxymethyl and pivaloyloxymethyl groups.

A suitable pharmaceutically acceptable pro-drug of a compound of the formula (I) that possesses a carboxy group is, for example, an *in vivo* cleavable amide thereof, for example an amide formed with an amine such as ammonia, a C1-4alkylamine such as methylamine, a (C1-4alkyl)2amine such as dimethylamine, N-ethyl-N-methylamine or diethylamine, a C1-4alkoxy- C2-4alkylamine such as 2-methoxyethylamine, a phenyl-C1-4alkylamine such as benzylamine and amino acids such as glycine or an ester thereof.

A suitable pharmaceutically acceptable pro-drug of a compound of the formula I that possesses an amino group is, for example, an *in vivo* cleavable amide derivative thereof. Suitable pharmaceutically acceptable amides from an amino group include, for example an amide formed with C1-10alkanoyl groups such as an acetyl, benzoyl, phenylacetyl and substituted benzoyl and phenylacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-4alkyl)piperazin-1-ylmethyl.

The *in vivo* effects of a compound of the formula (I) may be exerted in part by one or more metabolites that are formed within the human or animal body after administration of a compound of the formula (I). As stated hereinbefore, the *in vivo* effects of a compound of the formula (I) may also be exerted by way of metabolism of a precursor compound (a pro-drug).

Though the present invention may relate to any compound or particular group of compounds defined herein by way of optional, preferred or suitable features or otherwise in terms of particular embodiments, the present invention may also relate to any compound or particular group of compounds that specifically excludes said optional, preferred or suitable features or particular embodiments.

Suitably, the present invention excludes any individual compounds not possessing the biological activity defined herein.

### Synthesis

The compounds of the present invention can be prepared by any suitable technique known in the art. Particular methods for forming compounds of formula I defined herein are shown in the accompanying examples.

In the description of the synthetic methods described herein and in any referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilised.

It will be appreciated that during the synthesis of the compounds of the invention in the processes defined herein, or during the synthesis of certain starting materials, it may be desirable to protect certain substituent groups to prevent their undesired reaction. The skilled chemist will appreciate when such protection is required, and how such protecting groups may be put in place, and later removed.

For Examples of protecting groups see one of the many general texts on the subject, for example, 'Protective Groups in Organic Synthesis' by Theodora Green (publisher: John Wiley & Sons). Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule.

Thus, if reactants include, for example, groups such as amino, carboxy or hydroxy it may be desirable to protect the group in some of the reactions mentioned herein.

By way of Example, a suitable protecting group for an amino or alkylamino group is, for example, an acyl group, for example an alkanoyl group such as acetyl, an alkoxycarbonyl group, for example a methoxycarbonyl, ethoxycarbonyl or tbutoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively an acyl group such as a tertbutoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladiumoncarbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively, an arylmethyl group such as a benzyl group may be removed, for example, by hydrogenation over a catalyst such as palladiumoncarbon.

A suitable protecting group for a carboxy group is, for example, an esterifying group, for example a methyl or an ethyl group which may be removed, for example, by hydrolysis with a base such as sodium hydroxide, or for example a tbutyl group which may be removed, for example, by treatment with an acid, for example an organic acid such as trifluoroacetic acid, or for example a benzyl group which may be removed, for example, by hydrogenation over a catalyst such as palladiumoncarbon.

Resins may also be used as a protecting group.

The methodology employed to synthesise a compound of formula (I) will vary depending on the nature of R₁, R₂, R₃, R₄, R₅ and R₆ and any substituent groups associated therewith. Suitable processes for their preparation are described further in the accompanying Examples.

Once a compound of formula (I) has been synthesised by any one of the processes defined herein, the processes may then further comprise one or more of the additional steps of:
(i) removing any protecting groups present;
(ii) converting the compound formula (I) into another compound of formula (I);
(iii) forming a pharmaceutically acceptable salt, hydrate or solvate of the compound of formula I; and/or
(iv) forming a prodrug of the compound of formula I.

An Example of (ii) above is when a compound of formula (I) is synthesised and then one or more of the groups of R₁, R₂, R₃, R₄, R₅ and R₆ may be further reacted to change the nature of the group and provide an alternative compound of formula (I).

The resultant compounds of formula (I) can be isolated and purified using techniques well known in the art.

### Biological Activity

The biological assays described in the example section (Biological Examples 1 to 3) may be used to measure the pharmacological effects of the compounds of the present invention.

Although the pharmacological properties of the compounds of formula I vary with structural change, as expected, the compounds of the invention were found to be active in the assays described in Biological Examples 1, 2 andd 3.

In general, in terms of adenosine A2a antagonism, the compounds of the invention demonstrate an IC₅₀ of 1 µM or less in the assay described in Biological Example 1, with preferred compounds of the invention demonstrating an IC₅₀ of 200 nM or less and the most preferred compounds of the invention demonstrating an IC₅₀ of 50 nM or less.

In general, in terms of adenosine A2b antagonism, the compounds of the invention demonstrate an IC₅₀ of 1 µM or less in the assay described in Biological Example 1, with preferred compounds of the invention demonstrating an IC₅₀ of 200 nM or less and the most preferred compounds of the invention demonstrating an IC₅₀ of 50 nM or less.

Suitably the IC₅₀ at the adenosine A1 or A3 receptors of the compounds of the invention in the assay described in Biological Example 1 is at least two-fold higher than the IC₅₀ at the adenosine A2a receptor, and more suitably it is at least 5-fold higher, and even more suitably it is at least 10-fold higher.

### Pharmaceutical Compositions

According to a further aspect of the invention there is provided a pharmaceutical composition which comprises a compound of the invention as defined hereinbefore, or a pharmaceutically acceptable salt, hydrate or solvate thereof, in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

An effective amount of a compound of the present invention for use in therapy is an amount sufficient to treat or prevent a proliferative condition referred to herein, slow its progression and/or reduce the symptoms associated with the condition.

The amount of active ingredient that is combined with one or more excipients to produce a single dosage form will necessarily vary depending upon the individual treated and the particular route of administration. For Example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the formula I will naturally vary according to the nature and severity of the conditions, the age and sex of the animal or patient and the route of administration, according to well-known principles of medicine.

In using a compound of the invention for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### Therapeutic Uses and Applications

The present invention provides compounds that function as antagonists of adenosine A2 receptors, especially adenosine A2a receptors.

According to a further aspect of the present invention, there is provided a method of antagonising adenosine A2a receptors *in vitro* or *in vivo,* said method comprising contacting a cell with an effective amount of a compound or a pharmaceutically acceptable salt, hydrate or solvate thereof as defined herein.

According to a further aspect of the present invention, there is provided a method of selectively antagonising adenosine A2a receptros *in vitro* or *in vivo,* said method comprising contacting a cell with an effective amount of a compound, or a pharmaceutically acceptable salt, hydrate or solvate thereof, as defined herein.

According to a further aspect of the present invention, there is provided a method of inhibiting cell proliferation, *in vitro* or *in vivo,* said method comprising contacting a cell with an effective amount of a compound or a pharmaceutically acceptable salt, hydrate or solvate thereof as defined herein, or a pharmaceutical composition as defined herein. Suitably, the compound or pharmaceutical composition is administered in combination with one or more additional anti proliferative agents (e.g. checkpoint inhibitors and/or cytotoxic agents).

According to a further aspect of the present invention, there is provided a method of treating a disease or disorder in which adenosine A2a receptor activity is implicated in a patient in need of such treatment, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a method of treating a proliferative disorder in a patient in need of such treatment, said method comprising administering to said patient a therapeutically effective amount of a compound or a pharmaceutically acceptable salt, hydrate or solvate thereof as defined herein, or a pharmaceutical composition as defined herein. Suitably, the compound or pharmaceutical composition is administered in combination with one or more additional anti proliferative agents (e.g. checkpoint inhibitors and/or cytotoxic agents).

According to a further aspect of the present invention, there is provided a method of treating cancer in a patient in need of such treatment, said method comprising administering to said patient a therapeutically effective amount of a compound or a pharmaceutically acceptable salt, hydrate or solvate thereof as defined herein, or a pharmaceutical composition as defined herein. Suitably, the compound or pharmaceutical composition is administered in combination with one or more additional anticancer agents (e.g. checkpoint inhibitors and/or cytotoxic agents).

According to a further aspect of the present invention, there is provided a compound, or a pharmaceutically acceptable salt, hydrate or solvate thereof, or a pharmaceutical composition as defined herein for use in therapy.

According to a further aspect of the present invention, there is provided a compound or a pharmaceutically acceptable salt, hydrate or solvate thereof as defined herein, or a pharmaceutical composition as defined herein, for use in the treatment of a proliferative condition. Suitably, the compound or pharmaceutical composition is administered in combination with one or more additional anti proliferative agents (e.g. checkpoint inhibitors and/or cytotoxic agents).

According to a further aspect of the present invention, there is provided a compound, or a pharmaceutically acceptable salt, hydrate or solvate thereof, or a pharmaceutical composition as defined herein for use in the treatment of cancer. In a particular embodiment, the cancer is human cancer. Suitably, the compound or pharmaceutical composition is administered in combination with one or more additional anticancer agents (e.g. checkpoint inhibitors and/or cytotoxic agents).

According to a further aspect of the present invention, there is provided a compound, or a pharmaceutically acceptable salt, hydrate or solvate thereof, as defined herein for use as an adenosine A2a antagonist. In an embodiment, the compounds of the invention are selective adenosine A2a antagonists. In an alternative embodiment, certain compounds of the invention are selective adenosine A2a and adenosine A2b antagonists.

According to a further aspect of the present invention, there is provided a compound, or a pharmaceutically acceptable salt, hydrate or solvate thereof, as defined herein for use in the treatment of a disease or disorder in which adenosine A2a is implicated.

According to a further aspect of the present invention, there is provided the use of a compound, or a pharmaceutically acceptable salt, hydrate or solvate thereof, as defined herein in the manufacture of a medicament for the treatment of a proliferative condition. Suitably, the compound or pharmaceutical composition is administered in combination with one or more additional antiproliferative agents (e.g. checkpoint inhibitors and/or cytotoxic agents).

According to a further aspect of the present invention, there is provide the use of a compound, or a pharmaceutically acceptable salt, hydrate or solvate thereof, as defined herein in the manufacture of a medicament for the treatment of cancer. Suitably, the cancer is a human cancer. Suitably, the compound or pharmaceutical composition is administered in combination with one or more additional anticancer agents (e.g. checkpoint inhibitors and/or cytotoxic agents).

According to a further aspect of the present invention, there is provided a use of a compound, or a pharmaceutically acceptable salt, hydrate or solvate thereof, as defined herein in the manufacture of a medicament for use as an adenosine A2a antagonist.

According to a further aspect of the present invention, there is provided a use of a compound, or a pharmaceutically acceptable salt, hydrate or solvate thereof, as defined herein in the manufacture of a medicament for use as an adenosine A2a antagonist.

According to a further aspect of the present invention, there is provided a use of a compound, or a pharmaceutically acceptable salt, hydrate or solvate thereof, as defined herein in the manufacture of a medicament for the treatment of a disease or disorder in which adenosine A2a receptor activity is implicated.

The term "proliferative disorder" are used interchangeably herein and pertain to an unwanted or uncontrolled cellular proliferation of excessive or abnormal cells which is undesired, such as, neoplastic or hyperplastic growth, whether *in vitro* or *in vivo.* Examples of proliferative conditions include, but are not limited to, pre-malignant and malignant cellular proliferation, including but not limited to, malignant neoplasms and tumours, cancers, leukemias, psoriasis, bone diseases, fibroproliferative disorders (e.g., of connective tissues), and atherosclerosis. Any type of cell may be treated, including but not limited to, lung, colon, breast, ovarian, prostate, liver, pancreas, brain, and skin.

The anti-proliferative effects of the compounds of the present invention have particular application in the treatment of human cancers (by virtue of their adenosine A2a antagonist activity).

More specifically, there is provided a compound of general formula (I) for use in the treatment of cancer, particularly solid tumours, for example non-small cell or small cell lung cancer, head and neck squamous cancer and urothelial cancer.

More specifically, there is provided a compound of general formula (I) for use in the treatment of cancer, for example, lung cancer, such as as small cell lung cancer or non-small cell lung cancer.

There is also provided the use of a compound of general formula (I) in the manufacture of a medicament for the treatment of cancer, particularly solid tumours, for example non-small cell or small cell lung cancer, head and neck squamous cancer and urothelial cancer.

The invention further provides a method for the treatment of cancer, particularly solid tumours, for example non-small cell or small cell lung cancer, head and neck squamous cancer and urothelial cancer, the method comprising administering to a patient in need of such treatment an effective amount of a compound of general formula (I).

The patient to be treated is suitably a mammal and more suitably a human.

### Routes of Administration

The compounds of the invention or pharmaceutical compositions comprising these compounds may be administered to a subject by any convenient route of administration, whether systemically/ peripherally or topically (i.e., at the site of desired action).

Routes of administration include, but are not limited to, oral (e.g, by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, intrasternal, and intratumoural; by implant of a depot or reservoir, for example, subcutaneously or intramuscularly.

The compounds of the invention or pharmaceutical compositions comprising these compounds may be administered by intratumoural delivery.

### Combination Therapies

The compounds of formula I are useful for the treatment and/or prophylaxis of proliferative disorders, such as, for example, cancer. A compound of formula I defined herein may be used in combination with one or more additional anti proliferative/anticancer therapies, such as, for example, chemotherapy with one or more additional antiproliferative/anticancer agents, radiotherapy and/or conventional surgery.

An additional antiproliferative/anticancer agent may be included in the pharmaceutical composition with a compound of formula (I) as defined herein or, alternatively, it may be administered separately, either at the same time as the compound of formula (I) or at an earlier or later time.

Therefore, in a further aspect of the present invention there is provided a product comprising a compound of general formula (I) and an additional agent useful in the treatment or prevention of cancer as a combined preparation for simultaneous, sequential or separate use in the treatment of cancer.

The present invention also provides a compound of general formula (I) in combination with one or more additional antiproliferative/anticancer agents for use in the treatment of cancer as a combined preparation for simultaneous, sequential or separate use in the treatment of treatment of cancer.

In particular, the combination therapy defined herein is suitable for the treatment of solid tumours for example non-small cell or small cell lung cancer, head and neck squamous cancer and urothelial cancer.

Suitable additional anti proliferative/anti-cancer agents that may be used in combination with a compound of formula I defined herein [either separately or as part of a combined pharmaceutical composition or a combined preparation with the compounds of general formula (I)] include:
1) other forms of cancer immunotherapy and anti-cancer chemotherapeutic agents;
2) adenosine pathway modulators, including, but not limited to A2b antagonists, CD73 inhibitors and CD39 inhibitors;
3) anti-PD-1 and PDL-1 antibodies including, but not limited to, cetrelimab pembrolizumab, nivolumab, durvalumab, avelumab and atezolizumab; and
4) anti-CTLA4 antibodies including, but not limited to, ipilimumab.

The compounds of of formula I defined herein are particulalrly suited to use in combination with anti-PD-1 and PDL-1 antibodies including, but not limited to, Cetrelimab, pembrolizumab, nivolumab, durvalumab, avelumab and atezolizumab.

Suitably, the anti-PD1 antibody is one of the antibodies disclosed in U.S. Publication No. 2019/0225689 or U.S. Publication No. 2017/0121409, such as cetrelimab. Cetrelimab (JNJ-63723283, CET) is a fully human immunoglobulin (Ig) G4 kappa monoclonal antibody that binds to programmed death receptor-1 (PD-1) with high affinity and specificity. Cetrelimab has shown activity in solid tumors. Rutkowski P, et al. Journal of Clinical Oncology. 2019;37(8):31.

The compounds of of formula I defined herein are particulalrly suited to use in combination with adenosine pathway modulators, including, but not limited to A2b antagonists, CD73 inhibitors and CD39 inhibitors.

The A2a antagonists of general formula (I) can also be used in combination with cell-based immunotherapy and cancer vaccines that include, but are not limited to CART cell therapy.

Examples of the additional antiproliferative/anticancer chemotherapeutic agents include, but are not limited to, any one or more of the following:
MEK (e.g. MEK1, MEK2, or MEK1 and MEK2) inhibitors (e.g. XL518, CI-1040, PD035901,selumetinib/ AZD6244, GSK1 120212/ trametinib, GDC-0973, ARRY-162, ARRY-300, AZD8330, PD0325901, U0126, PD98059, TAK-733, PD3 18088, AS703026, BAY 869766), alkylating agents (e.g., cyclophosphamide, ifosfamide, chlorambucil, busulfan, melphalan, mechlorethamine, uramustine, thiotepa, nitrosoureas, nitrogen mustards (e.g., mechloroethamine, cyclophosphamide, chlorambucil, meiphalan), ethylenimine and methylmelamines (e.g., hexamethlymelamine, thiotepa), alkyl sulfonates (e.g., busulfan), nitrosoureas (e.g., carmustine, lomusitne, semustine, streptozocin), triazenes (decarbazine)), anti-metabolites (e.g., 5-azathioprine, leucovorin, capecitabine, fludarabine, gemcitabine, pemetrexed, raltitrexed, folic acid analog (e.g., methotrexate), or pyrimidine analogs (e.g., fluorouracil, floxouridine, Cytarabine), purine analogs (e.g., mercaptopurine, thioguanine, pentostatin), etc.), plant alkaloids (e.g., vincristine, vinblastine, vinorelbine, vindesine, podophyllotoxin, paclitaxel, docetaxel, etc.), topoisomerase inhibitors (e.g., irinotecan, topotecan, amsacrine, etoposide (VP16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), platinum-based compounds or platinum containing agents (e.g. cisplatin, oxaloplatin, arboplatin), anthracenedione (e.g., mitoxantrone), substituted urea (e.g., hydroxyurea), methyl hydrazine derivative (e.g., procarbazine), adrenocortical suppressant (e.g., mitotane, aminoglutethimide), epipodophyllotoxins (e.g., etoposide), antibiotics (e.g., daunorubicin, doxorubicin, bleomycin), enzymes (e.g., L-asparaginase), inhibitors of mitogen-activated protein kinase signaling (e.g. U0126, PD98059, PD184352, PD0325901, ARRY-142886, SB239063, SP600125, BAY 43-9006, wortmannin, or LY294002, Syk inhibitors, mTOR inhibitors, antibodies (e.g., rituxan), gossyphol, genasense, polyphenol E, Chlorofusin, all trans-retinoic acid (ATRA), bryostatin, tumor necrosis factor-related apoptosis-inducing ligand (TRAIL), 5-aza-2'-deoxycytidine, all trans retinoic acid, doxorubicin, vincristine, etoposide, gemcitabine, imatinib (Gleevec.RTM.), geldanamycin, 17-N-Allylamino-17-Demethoxygeldanamycin (17-AAG), flavopiridol, LY294002, bortezomib, trastuzumab, BAY 11-7082, PKC412, PD184352, 20-epi-I, 25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein- 1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cisporphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; 9-dioxamycin; diphenyl spiromustine; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fiudarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatinA; marimastat; masoprocol; maspin; matrilysin inhibitors; matrix metalloproteinase inhibitors; menogaril; merbarone; meterelin; methioninase; metoclopramide; MIF inhibitor; mifepristone;miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzyl guanine; octreotide; okicenone; oligonucleotides; onapristone; ondansetron; ondansetron; oracin; oral cytokine inducer; ormaplatin; osaterone; oxaliplatin; oxaunomycin; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-basedimmune modulator; protein kinase C inhibitor; protein kinase C inhibitors, microalgal; proteintyrosine phosphatase inhibitors; purine nucleoside phosphorylase inhibitors; purpurins; pyrazoloacridine; pyridoxylated hemoglobin polyoxyethylerie conjugate; raf antagonists; raltitrexed; ramosetron; ras farnesyl protein transferase inhibitors; ras inhibitors; ras-GAPinhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen-binding protein; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stemcell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; zinostatin stimalamer, Adriamycin, Dactinomycin, Bleomycin, Vinblastine, Cisplatin, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cladribine; crisnatol mesylate; cyclophosphamide; cytarabine; dacarbazine; daunorubicin hydrochloride; decitabine; dexormaplatin; dezaguanine; dezaguanine mesylate; diaziquone; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; fluorocitabine; fosquidone; fostriecin sodium; gemcitabine; gemcitabine hydrochloride; hydroxyurea; idarubicin hydrochloride; ifosfamide; iimofosine; interleukin 2 (including recombinant interleukin 2, or rIL.sub.2), interferon alfa-2a; interferon alfa-2b;
interferon alfa-nl; interferon alfa-n3; interferon beta- la; interferon gamma-lb; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazoie; nogalamycin; ormaplatin; oxisuran; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride, agents that arrest cells in the G2-M phases and/or modulate the formation or stability of microtubules, (e.g. Taxol.TM (i.e. paclitaxel), Taxotere.TM, compounds comprising the taxane skeleton, Erbulozole (i.e. R-55104), Dolastatin 10 (i.e. DLS- 10 and NSC-376128), Mivobulin isethionate (i.e. as CI-980), Vincristine, NSC-639829, Discodermolide (i.e. as NVP-XX-A-296), ABT-751 (Abbott, i.e. E-7010), Altorhyrtins (e.g. Altorhyrtin A and Altorhyrtin C), Spongistatins (e.g. Spongistatin 1, Spongistatin 2, Spongistatin 3, Spongistatin 4, Spongistatin 5, Spongistatin 6, Spongistatin 7, Spongistatin 8, and Spongistatin 9), Cemadotin hydrochloride (i.e. LU-103793 and NSC-D-669356), Epothilones (e.g. Epothilone A, Epothilone B, Epothilone C (i.e. desoxyepothilone A or dEpoA), Epothilone D (i.e. KOS-862, dEpoB, and desoxyepothilone B), Epothilone E, Epothilone F, Epothilone B N-oxide, Epothilone A N-oxide, 16-aza-epothilone B, 21-aminoepothilone B (i.e. BMS-3 10705), 21- hydroxyepothilone D (i.e. Desoxyepothilone F and dEpoF), 26-fluoroepothilone, Auristatin PE (i.e. NSC-654663), Soblidotin (i.e. TZT-1027), , Vincristine sulfate, Cryptophycin 52 (i.e. LY-355703), Vitilevuamide, Tubulysin A, Canadensol, Centaureidin (i.e. NSC-106969), Oncocidin Al (i.e. BTO-956 and DF E), Fijianolide B, Laulimalide, Narcosine (also known as NSC-5366), Nascapine, Hemiasterlin, Vanadocene acetylacetonate, Monsatrol, Inanocine (i.e. NSC-698666), Eleutherobins (such as Desmethyleleutherobin, Desaetyleleutherobin, Isoeleutherobin A, and ZEleutherobin), Caribaeoside, Caribaeolin, Halichondrin B, Diazonamide A, Taccalonolide A, Diozostatin, (-)-Phenylahistin (i.e. NSCL-96F037), Myoseverin B, Resverastatin phosphate sodium, steroids (e.g., dexamethasone), finasteride, aromatase inhibitors, gonadotropin-releasing hormone agonists (GnRH) such as goserelin or leuprolide, adrenocorticosteroids (e.g., prednisone), progestins (e.g., hydroxyprogesterone caproate, megestrol acetate, medroxyprogesterone acetate), estrogens (e.g., diethlystilbestrol, ethinyl estradiol), antiestrogen (e.g., tamoxifen), androgens (e.g., testosterone propionate, fluoxymesterone), antiandrogen (e.g., flutamide), immunostimulants (e.g., Bacillus Calmette-Guerin (BCG), levamisole, interleukin-2, alpha-interferon, etc.), monoclonal antibodies (e.g., anti-CD20, anti-F£ER2, anti-CD52, anti- ULA-DR, and anti-VEGF monoclonal antibodies), immunotoxins (e.g., anti-CD33 monoclonal antibody-calicheamicin conjugate, anti-CD22 monoclonal antibody-pseudomonas exotoxin conjugate, etc.), radioimmunotherapy (e.g., anti-CD20 monoclonal antibody conjugated to In, 0Y, or I, etc.), triptolide, homoharringtonine, dactinomycin, doxorubicin, epirubicin, topotecan, itraconazole, vindesine, cerivastatin, vincristine, deoxyadenosine, sertraline, pitavastatin, irinotecan, clofazimine, 5-nonyloxytryptamine, vemurafenib, dabrafenib, erlotinib, gefitinib, EGFR inhibitors, epidermal growth factor receptor (EGFR)-targeted therapy or therapeutic (e.g. gefitinib (Iressa ^{™}), erlotinib (Tarceva ^{™}), cetuximab (Erbitux^{™}), lapatinib (Tykerb^{™}), panitumumab (Vectibix^{™}), vandetanib (Caprelsa^{™}), afatinib/BIBW2992, CI-1033/canertinib, neratinib/HKI-272, CP-724714, TAK-285, AST-1306, ARRY334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035,BMS-599626), sorafenib, imatinib, sunitinib, dasatinib, hormonal therapies, or the like.

As indicated above, the combination therapy of the present invention may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a combination for use in the treatment of a cancer (for example a cancer involving a solid tumour) comprising a compound of the invention as defined hereinbefore, or a pharmaceutically acceptable salt, hydrate or solvate thereof, and one or more additional antiproliferative/anticancer agents.

According to this aspect of the invention there is also provided a combination for use in the treatment of a proliferative condition, such as cancer (for example a cancer involving a solid tumour), comprising a compound of the invention as defined hereinbefore, or a pharmaceutically acceptable salt, hydrate or solvate thereof, and one or more additional antiproliferative/anticancer agents selected from those listed above.

In a further aspect of the invention there is provided a compound of the invention or a pharmaceutically acceptable salt, hydrate or solvate thereof, for use in the treatment of cancer in combination with another anti-tumour agent, optionally selected from one listed herein above.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

According to a further aspect of the invention there is provided a pharmaceutical composition which comprises a compound of the invention, or a pharmaceutically acceptable salt, hydrate or solvate thereof, in combination with an anti-tumour agent (optionally selected from one listed herein above), in association with a pharmaceutically acceptable diluent or carrier.

### EXAMPLES

### General Conditions:

Mass spectra were run on LC-MS systems using electrospray ionization. These were run using either a Waters Acquity H-Class UPLC with PDA and QDa mass detection, a Waters Acquity Classic UPLC with PDA and SQ mass detection, an Acquity LC (binary pump/PDA detector) + ZQ Mass Spectrometer or Acquity i-Class (quaternary pump/PDA detector) + Quattro Micro Mass Spectrometer, a Waters Acquity LC system with Waters PDA and ELS detectors or a Shimadzu LCMS-2010EV system. [M+H]+ refers to mono-isotopic molecular weights.

NMR spectra were run on either a Bruker Ultrashield 500 MHz NMR spectrometer, a Bruker Avance III HD 400 MHz NMR spectrometer or a Bruker Avance III HD 500 MHz. Spectra were recorded at 298K and were referenced using the solvent peak.

The following examples are intended to illustrate the invention and are not to be construed as being limitations thereon. Temperatures are given in degrees centigrade. If not mentioned otherwise, all evaporations are performed *in vacuo,* preferably between about 15 mm Hg and 100 mm Hg (= 20-133 mbar). The structure of final products, intermediates and starting materials is confirmed by standard analytical methods, e.g., microanalysis and spectroscopic characteristics, e.g., MS, IR, and NMR. Abbreviations used are those conventional in the art. If not defined, the terms have their generally accepted meanings.

### Abbreviations

- app: apparent
- br: broad
- CDI: 1,1'-carbonyldiimidazole
- d: doublet
- dd: doublet of doublets
- DCM: dichloromethane
- DIPEA: diisopropylethylamine
- DMF: *N*,*N*-dimethylformamide
- DMA: Dimethylacetamide
- EtOAc: ethyl acetate
- HPLC: high pressure liquid chromatography
- LC-MS: liquid chromatography and mass spectrometry
- MeOH: MeOH
- MeCN: acetonitrile
- MS: mass spectrometry
- m: multiplet
- mins: minute(s)
- mL: milliliter(s)
- m/z: mass to charge ratio
- NMR: nuclear magnetic resonance
- ppm: parts per million
- Rt: retention time
- s: singlet
- t: triplet
- TBAF: tetra-n-butylammonium fluoride
- TFA: trifluoroacetic acid
- THF: tetrahydrofuran

Referring to the examples that follow, compounds of the preferred embodiments were synthesized using the methods described herein, or other methods, which are known in the art.

The various starting materials, intermediates, and compounds of the preferred embodiments may be isolated and purified, where appropriate, using conventional techniques such as precipitation, filtration, crystallization, evaporation, distillation, and chromatography. Unless otherwise stated, all starting materials are obtained from commercial suppliers and used without further purification. Salts may be prepared from compounds by known salt-forming procedures.

If not indicated otherwise, the analytical HPLC conditions are as follows:

### Method 2A

| | |
|---|---|
| Column: | Kinetex Core-Shell C18 2.1 x 50 mm 5 µm |
| Column Temp | 40 °C |
| Eluents: | A: H₂0+0.1% formic acid, B: MeCN+ 0.1% formic acid |
| Flow Rate: | 1.2 mL/min |
| Gradient: | 0-1.83 min 5-100 B, 1.83- 2.25 min 100% B, 2.25-2.26 min 100-5% B |

### Method 2.5B

| | |
|---|---|
| Column: | Phenomenex Gemini-NX C18 2 x 50 mm 3 µm |
| Column Temp: | 40 °C |
| Eluents: | A: 2 mM ammonium bicarbonate, buffered to pH10, B: MeCN |
| Flow Rate: | 1 mL/min |
| Gradient: | 0-1.80 min 1-100% B, 1.80-2.10 min 100% B, 2.10-2.30 min 100-1%B |

### Method 2B

| | |
|---|---|
| Column: | Acquity UPLC BEH C18 2.1 x 50 mm, 1.7 µm |
| Column Temp: | 50 °C |
| Eluents: | A: H₂O, 0.1% formic acid B: MeCN |
| Flow Rate: | 0.8 mL/min |
| Gradient: | 0.0-1.8 min 2-98% B, 1.8-2.1 min 98% B, 2.1-2.5 min 98%A |

### Method 3A

| | |
|---|---|
| Column: | Acquity LC BEH C18 2.1 x 50 mm 1.7 µm |
| Column Temp: | 50 °C |
| Eluents: | A: H₂O, 0.1% formic acid, B: MeCN |
| Flow Rate: | 0.6 mL/min |
| Gradient: | 0.2-2.5 mins 2-98% B, 2.5-3.3 mins 98% B, 3.3-3.5 mins 98% A |

### Method 3B

| | |
|---|---|
| Column: | Acquity LC BEH C18 2.1 x 50 mm 1.7 µm |
| Column Temp: | 50 °C |
| Eluents: | A: H₂O, 0.1% ammonia, B: MeCN |
| Flow Rate: | 0.6 mL/min |
| Gradient: | 0.2-2.5 mins 2-98% B, 2.5-3.3 mins 98% B, 3.3-3.5 mins 98% A |

### Method 5A

| | |
|---|---|
| Column: | YMC-Triart C18 2 x 50 mm, 5 µm. |
| Flow rate: | 0.8 mL/min. |
| Eluents: | A: H₂O, B: MeCN, C: 50% H₂O / 50% MeCN + 1.0% formic acid |
| Gradient: | 0.0-4.0 min 0-95% B, 5% C; 4.0-4.4 min 95% B, 5%C; 4.4-4.5 min 95% A, 5% B |

### Method 5B

| | |
|---|---|
| Column: | Waters Alliance ZQ MS, YMC-Triart C18 50 x 2 mm, 5 µm |
| Column Temp: | room temperature |
| Eluents: | A: H₂O, B: CH₃CN, C: 50% H₂O / 50% CH₃CN + 1.0% ammonia (aq.) |
| Flow rate: | 0.8 mL/min. |
| Gradient: | 0.0 - 4.0 mins 0-95% B, 5% C; 4.0-4.4 mins 95% B, 5%C; 4.4-4.5 mins 95% A, 5%B |

### Method 7A

| | |
|---|---|
| Column: | Phenomenex Kinetix-XB C18 2.1 x 100 mm, 1.7 µm |
| Column Temp: | 40 °C |
| Eluents: | A: H₂O 0.1% formic acid, B: MeCN, 0.1% formic acid |
| Flow Rate: | 0.6 mL/min |
| Gradient: | 0-5.3.0 min 5-100% B, 5.3-5.8 min 100% B, 5.8-5.82 min 100-5% B, 5.82-7.00 min 5% B |

### Method 7B

| | |
|---|---|
| Column: | Waters LC^{®} BEH^{™} C18, 2.1 mm x 100 mm, 1.7 µm |
| Column Temp: | 40 °C |
| Eluents: | A: 2 mM ammonium bicarbonate buffered to pH10, B: MeCN |
| Flow Rate: | 0.6 mL/min |
| Gradient: | 0-5.3.0 min 5-100% B, 5.3-5.8 min 100% B, 5.8-5.82 min 100-5% B, 5.82-7.00 min 5% B |

### Method 8A

| | |
|---|---|
| Column: | Acquity UPLC BEH C18 2.1 x 100 mm, 1.7 µm |
| Column Temp: | 50 °C |
| Eluents: | A: H₂O, 0.1% formic acid B: MeCN |
| Flow Rate: | 0.6 mL/min |
| Gradient: | 0.5-6.5 min 2-98% B 6.5-7.5 min 98% B |

### Method 8B

| | |
|---|---|
| Column: | Acquity UPLC BEH C18 2.1 x 100 mm, 1.7 µm |
| Column Temp: | 50 °C |
| Eluents: | A: H₂O, 0.1% ammonia B: MeCN |
| Flow Rate: | 0.6 mL/min |
| Gradient: | 0.5-6.5 min 2-98% B 6.5-7.5 min 98% B |

### Method 15A

| | |
|---|---|
| Column: | YMC-Triart C18 2 x 50 mm, 5 µm. |
| Flow rate: | 0.8 mL/min. |
| Eluents: | A: H₂O, B: MeCN, C: 50% H₂O / 50% MeCN + 1.0% formic acid |
| Gradient: | 0.0-12.0 min 0-95% B, 5% C; 12.0-14.0 min 95% B, 5% C; 14.0-14.2 min 95% A, 5% B |

### Method 15B

| | |
|---|---|
| Column: | YMC-Triart C18 50 x 2 mm, 5 µm. |
| Flow rate: | 0.8 mL/min. |
| Eluents: | A: H₂O, B: MeCN, C: 50% H₂O / 50% MeCN + 1.0% ammonia (aq.) |
| Gradient: | 0.0-12.0 min 0-95% B, 5% C; 12.0-14.0 min 95% B, 5% C; 14.0-14.2 min 95% A, 5% B |

### Preparative HPLC using acidic pH, early elution method

Purifications by were performed on a Gilson LC system using Waters Sunfire C18 columns (30 mm x 100 mm, 10 µM; temperature: room temperature) and a gradient of 10-95% B (A= 0.1% formic acid in water; B= 0.1% formic acid in MeCN) over 14.44 min then 95% B for 2.11 min, with an injection volume of 1500 µL and a flow rate of 40 mL/min. UV spectra were recorded at 215 nm using a Gilson detector.

### Preparative HPLC using basic pH, early elution method

Purifications by preparative HPLC (basic pH, early elution method) were performed on a Gilson LC system using Waters Xbridge C18 columns (30 mm x 100 mm, 10 µM; temperature: room temperature) and a gradient of 10-95% (A= 0.2% ammonium hydroxide in water; B= 0.2% ammonium hydroxide in MeCN) over 14.44 min then 95% B for 2.11 min, with an injection volume of 1500 µL and a flow rate of 40 mL/min. UV spectra were recorded at 215 nm using a Gilson detector.

### Example 1

### N-[5-(2-Chloro-6-methyl-4-pyridyl)-4-(3-cyanophenyl)thiazol-2-yl]morpholine-4-carboxamide

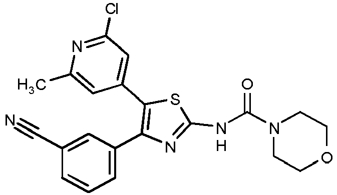

### Step 1: N-[4-(3-Cyanophenyl)thiazol-2-yl]morpholine-4-carboxamide

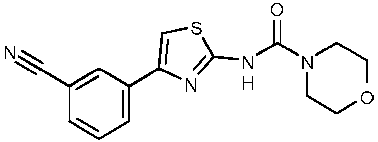

To a stirred solution of 3-(2-aminothiazol-4-yl)benzonitrile (Intermediate A)(653 mg, 3.08 mmol) in THF (10 mL) at 0 °C was added sodium hydride (60% in mineral oil) (150 mg, 3.75 mmol). After stirring at room temperature for 20 mins, morpholine-4-carbonyl chloride (520 µL, 4.55 mmol) was added and stirring continued for 18 h. The resulting mixture was diluted with THF (10 mL) then retreated with sodium hydride (60% in mineral oil) (150 mg, 3.75 mmol). After stirring for 10 mins, morpholine-4-carbonyl chloride (520 µL, 4.55 mmol) was added and stirring continued for 4 h. The reaction was quenched with water (20 mL) and extracted with EtOAc (2 x 40 mL). The aqueous layer was acidified to pH 5 using 1M HCl and extracted with EtOAc (2 x 30 mL). The combined organic extracts were washed with brine (30 mL), dried over MgSO₄ and concentrated *in vacuo.* The resulting brown oil was purified by chromatography on silica eluting with 0-100% EtOAc in heptane to afford the title compound as a beige solid.
LC-MS (Method 2A): Rt 1.06 mins; MS m/z 315.1 = [M+H]+
¹H NMR (400 MHz, DMSO-*d*₆) δ 11.05 (s, 1H), 8.31 (t, *J* = 1.5 Hz, 1H), 8.21 (dt, *J* = 7.9, 1.3 Hz, 1H), 7.76 (dt, *J* = 7.7, 1.3 Hz, 1H), 7.73 (s, 1H), 7.63 (t, *J* = 7.8 Hz, 1H), 3.67 - 3.58 (m, 4H), 3.56 - 3.48 (m, 4H).

### Step 2: N-[5-Bromo-4-(3-cyanophenyl)thiazol-2-yl]morpholine-4-carboxamide

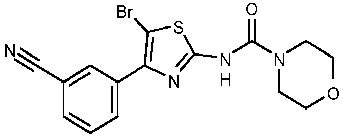

To a solution of N-[4-(3-cyanophenyl)thiazol-2-yl]morpholine-4-carboxamide (step 1) (790 mg, 2.34 mmol) in THF (20 mL) was added NBS (499 mg, 2.8mmol) and the reaction mixture was stirred at room temperature for 30 mins. The resulting mixture was diluted with EtOAc (50 mL) and washed with water (30 mL), sat. NaHCO₃ solution (2 x 30 mL) and brine (30 mL). The organic layer was dried over MgSO₄ and concentrated *in vacuo* to afford the title compound as a yellow solid.
LC-MS (Method 2A): Rt 1.18 mins; MS m/z 393.0/395.0 = [M+H]+
¹H NMR (500 MHz, DMSO-*d*₆) δ 11.35 (s, 1H), 8.25 (t, *J* = 1.5 Hz, 1H), 8.21 (dt, J= 8.0, 1.4 Hz, 1H), 7.88 (dt, *J* = 7.7, 1.3 Hz, 1H), 7.71 (t, *J* = 7.9 Hz, 1H), 3.63 - 3.56 (m, 4H), 3.55 - 3.48 (m, 4H).

### Step 3: N-[5-(2-Chloro-6-methyl-4-pyridyl)-4-(3-cyanophenyl)thiazol-2-yl]morpholine-4-carboxamide

A stirred solution of N-[5-bromo-4-(3-cyanophenyl)thiazol-2-yl]morpholine-4-carboxamide (step 2) (50 mg, 0.13 mmol), (2-chloro-6-methyl-4-pyridyl)boronic acid (87 mg, 0.51 mmol) and cesium carbonate (124 mg, 0.38 mmol) in monoglyme (3 mL) and water (0.5 mL) was degassed with nitrogen for 5 mins. Bis(triphenylphosphine)palladium(ll) dichloride (18 mg, 0.03 mmol) was added and the reaction mixture was heated at 85 °C for 7.5 h. After cooling to room temperature, the mixture was diluted with DCM (5 mL) and water (5 mL). The organic layer was separated, passed through a hydrophobic frit and concentrated *in vacuo.* The crude product was purified by C18 reverse phase chromatography eluting with acetonitrile/water/0.1% formic acid followed by further purification by C18 reverse phase chromatography eluting with acetonitrile/water/0.1% ammonia to afford the title compound as a bright yellow powder.
LC-MS (Method 2.5B): Rt 3.58 mins; MS m/z 440.1/442.1 = [M+H]+
¹H NMR (400MHz, DMSO-d6) δ 11.40 (br s, 1H), 7.90 (t, *J* = 1.4Hz, 1H), 7.84 (dt, *J* = 7.7, 1.3Hz, 1H), 7.72 (dt, *J* = 7.9, 1.3Hz, 1H), 7.58 (t, *J* = 7.9Hz, 1H), 7.14 (s, 1H), 7.01 (s, 1H), 3.62 - 3.57 (m, 4H), 3.54 - 3.49 (m, 4H), 2.39 (s, 3H)

### Example 2

### N-[5-(2-Chloro-6-methyl-4-pyridyl)-4-(3-cyanophenyl)thiazol-2-yl]-3-(1-hydroxy-1-methyl-ethyl)pyrrolidine-1-carboxamide

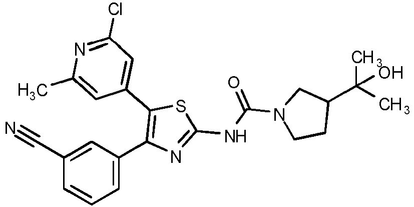

### Step 1: N-[5-(2-Chloro-6-methyl-4-pyridyl)-4-(3-cyanophenyl)thiazol-2-yl]imidazole-1-carboxamide

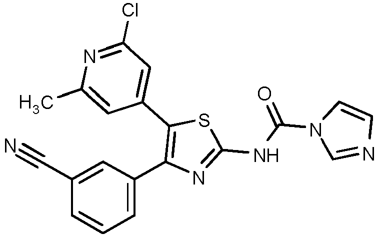

A solution of 3-[2-amino-5-(2-chloro-6-methyl-4-pyridyl)thiazol-4-yl]benzonitrile (Intermediate D) (73 mg, 0.17 mmol) and CDI (55 mg, 0.34 mmol) in DMA (0.5 mL) was stirred at room temperature for 24 h. A further portion of CDI (55 mg, 0.34 mmol) was added and stirring continued at room temperature for 2 h and then at 50 °C for 1 h. After cooling to room temperature, the resulting precipitate was collected by vacuum filtration to afford the title compound as a pale yellow solid.
LC-MS (Method 7B): Rt 3.76 mins; MS m/z 385.1 = [M-(C₃N₂H₅)+(OCH₃)]+; methyl carbamate observed from reaction of title compound with MeOH.
¹H NMR (400 MHz, DMSO-d6) δ 8.43 (s, 1H), 8.11 (d, *J* = 3.6 Hz, 1H), 7.90 (d, *J* = 1.5 Hz, 1H), 7.84 (d, *J* = 1.4 Hz, 1H), 7.83 - 7.79 (m, 1H), 7.76 - 7.72 (m, 1H), 7.69 (dt, *J* = 7.9, 1.4 Hz, 1H), 7.56 (t, *J* = 7.8 Hz, 1H), 7.01 (s, 1H), 6.91 (s, 1H), 2.35 (s, 3H).

### Step 2: N-[5-(2-Chloro-6-methyl-4-pyridyl)-4-(3-cyanophenyl)thiazol-2-yl]-3-(1-hydroxy-1-methyl-ethyl)pyrrolidine-1-carboxamide

To a stirred solution of N-[5-(2-chloro-6-methyl-4-pyridyl)-4-(3-cyanophenyl)thiazol-2-yl]imidazole-1-carboxamide (step 1) (59 mg, 0.09 mmol) in DMA (0.5 mL) was added 2-pyrrolidin-3-ylpropan-2-ol (22 mg, 0.17 mmol) and the reaction mixture was stirred at room temperature for 30 mins. A further portion of 2-pyrrolidin-3-ylpropan-2-ol (22 mg, 0.17 mmol) was added and stirring continued for 3 days. DIPEA (30 µL, 0.17 mmol) was added followed by further 2-pyrrolidin-3-ylpropan-2-ol (22 mg, 0.17 mmol) and the mixture was heated to 50 °C for 18 h. The resulting mixture was diluted with water (3 mL) and extracted with EtOAc (3 x 3 mL). The combined organic extracts were filtered through a phase separating cartridge and concentrated *in vacuo.* Purification of the crude product by chromatography on silica eluting with 0-100% EtOAc in DCM followed by 0-10% MeOH in EtOAc afforded the title compound as an off-white solid.
LC-MS (Method 7B): Rt 3.23 mins; MS m/z 482.3/484.2 = [M+H]+
¹H NMR (400 MHz, DMSO-*d*₆) δ 11.05 (s, 1H), 7.92 (t, *J* = 1.5 Hz, 1H), 7.85 (dt, *J* = 7.7, 1.4 Hz, 1H), 7.73 (dt, *J* = 7.9, 1.3 Hz, 1H), 7.59 (t, *J* = 7.9 Hz, 1H), 7.18 (s, 1H), 7.06 (s, 1H), 4.37 (s, 1H), 3.71 - 3.41 (m, 4H), 2.40 (s, 3H), 2.21 (s, 1H), 1.83 (s, 2H), 1.12 (s, 6H).

### Example 3

### (2R)-N-[5-(2-Chloro-6-methyl-4-pyridyl)-4-(3-cyanophenyl)thiazol-2-yl]-2-(1-hydroxy-1-methyl-ethyl)pyrrolidine-1-carboxamide

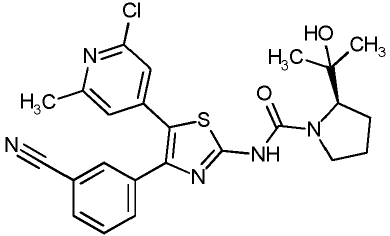

A solution of 3-[2-amino-5-(2-chloro-6-methyl-4-pyridyl)thiazol-4-yl]benzonitrile (Intermediate D (52 mg, 0.16 mmol) and CDI (31 mg, 0.19 mmol) in DMA (0.5 mL) was stirred at 50 °C for 22 h. Further CDI (31 mg, 0.19 mmol) was added and stirring was continued at 50 °C for a total of 46 h. The resulting mixture was allowed to cool to room temperature before being treated with 2-[(2R)-pyrrolidin-2-yl]propan-2-ol hydrochloride (40 mg, 0.24 mmol) and DIPEA (55 µL, 0.32 mmol). Stirring was continued at room temperature for 1 h and then at 50 °C for 1 h. After cooling to room temperature, the mixture was diluted with water (3 mL) and extracted with EtOAc (3 x 3 mL). The combined organic extracts were filtered through a phase separating cartridge and concentrated *in vacuo.* The resulting orange solid was purified by chromatography on silica eluting with 0-100% EtOAc in DCM followed by a second purification by chromatography on silica eluting with 0-100% EtOAc in heptane to afford the title compound as an off-white solid.
LC-MS (Method 7B): Rt 3.62 mins; MS m/z 482.3/484.3 = [M+H]+
¹H NMR (400 MHz, DMSO-*d*₆) δ 12.00 (s, 1H), 7.88 (t, *J* = 1.5 Hz, 1H), 7.85 (dt, *J* = 7.7, 1.4 Hz, 1H), 7.69 (dt, *J* = 8.0, 1.2 Hz, 1H), 7.58 (t, *J* = 7.8 Hz, 1H), 7.21 - 7.16 (m, 1H), 7.08 - 7.03 (m, 1H), 4.01 - 3.82 (m, 3H), 2.40 (s, 3H), 2.11 - 2.00 (m, 1H), 1.90 - 1.60 (m, 4H), 1.19 (s, 3H), 1.10 (s, 3H).

The compounds of the following tabulated Examples (Table Ex3) were prepared analogously to Example 3 from 3-[2-amino-5-(2-chloro-6-methyl-4-pyridyl)thiazol-4-yl]benzonitrile (Intermediate D) and the appropriate amine.

**Table Ex3**

| **Ex.** | **Structure and Name** | **Retention Time, [M+H]+, ¹H NMR** |
|---|---|---|
| 3.1 | 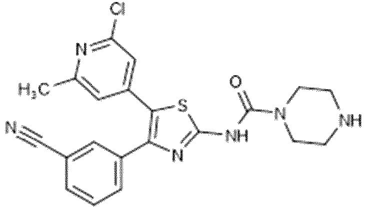 N-[5-(2-Chloro-6-methyl-4-pyridyl)-4-(3-cyanophenyl)thiazol-2-yl]piperazine-1-carboxamide | LC-MS (Method 7A): Rt 1.98 mins; MS m/z 439.1/441.2 = [M+H]+ |
| | | ¹H NMR (400 MHz, DMSO-d6) δ 7.91 (t,J= 1.5 Hz, 1H), 7.85 (dt,J= 7.7, 1.3 Hz, 1H), 7.72 (dt,J= 7.9, 1.3 Hz, 1H), 7.59 (t,J= 7.8 Hz, 1H), 7.16 (s, 1H), 7.03 (s, 1H), 3.50 - 3.43 (m, 4H), 2.75 - 2.69 (m, 4H), 2.39 (s, 3H). 2 x NH protons not observed |
| 3.2 | 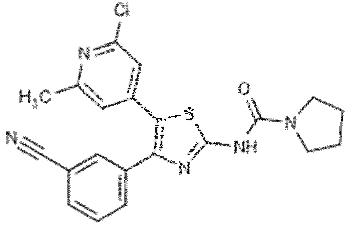 N-[5-(2-Chloro-6-methyl-4-pyridyl)-4-(3-cyanophenyl)thiazol-2-yl]pyrrolidine-1-carboxamide | LC-MS (Method 7B): Rt 3.51 mins; MS m/z 424.2/426.2 = [M+H]+ |
| | | ¹H NMR (500 MHz, DMSO-d6) δ 11.08 (s, 1H), 7.92 (t, *J =* 1.4 Hz, 1H), 7.85 (dt, J = 7.7, 1.3 Hz, 1H), 7.73 (dt, *J* = 7.9, 1.3 Hz, 1H), 7.59 (t, *J* = 7.8 Hz, 1H), 7.18 (s, 1H), 7.06 (s, 1H), 3.47 - 3.37 (m, 4H), 2.40 (s, 3H), 1.91 - 1.81 (m, 4H). |
| 3.3 | 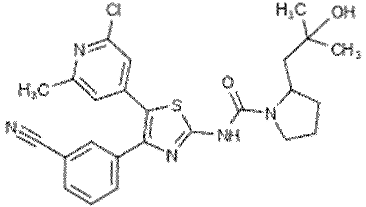 N-[5-(2-Chloro-6-methyl-4-pyridyl)-4-(3-cyanophenyl)thiazol-2-yl]-2-(2-hydroxy-2-methylpropyl)pyrrolidine-1-carboxamide | (Method 7B): Rt 3.87 mins; MS m/z 496.3/498.3 = [M+H]+ |
| | | ¹H NMR (400 MHz, DMSO-d6) δ 7.90 (t, *J* = 1.2 Hz, 1H), 7.84 (dt, *J* = 7.7, 1.4 Hz, 1H), 7.71 (d, *J = 7.7* Hz, 1H), 7.58 (t, *J* = 7.8 Hz, 1H), 7.15 (s, 1H), 7.02 (s, 1H), 4.13 - 4.03 (m, 1H), 3.46 - 3.34 (m, 3H), 2.39 (s, 3H), 1.92 - 1.64 (m, 6H), 1.17 (s, 3H), 1.16 (s, 3H). NH proton not observed. |
| 3.4 | 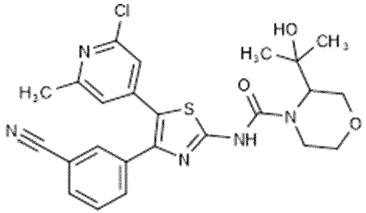 N-[5-(2-Chloro-6-methyl-4-pyridyl)-4-(3-cyanophenyl)thiazol-2-yl]-3-(1-hydroxy-1-methyl-ethyl)morpholine-4-carboxamide | LC-MS (Method 7A): Rt 3.34 mins; MS m/z 498.1/500.1 = [M+H]+ |
| | | ¹H NMR (400 MHz, DMSO-d6) δ 7.90 (s, 1H), 7.85 (d, *J = 7.8* Hz, 1H), 7.71 (d, *J =* 8.1 Hz, 1H), 7.58 (t, *J* = 7.8 Hz, 1H), 7.17 (s, 1H), 7.05 (s, 1H), 4.19 - 4.06 (m, 1H), 4.03 - 3.93 (m, 2H), 3.86 - 3.78 (m, 1H), 3.51 (dd, J = 12.3, 4.6 Hz, 1H), 3.48 - 3.38 (m, 2H), 2.40 (s, 3H), 1.22 (s, 3H), 1.20 (s, 3H). NH and OH protons not observed. |
| 3.5 | 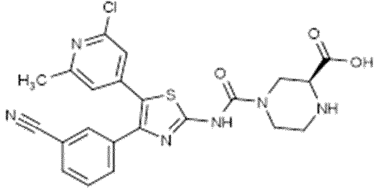 (2S)-4-[[5-(2-Chloro-6-methyl-4-pyridyl)-4-(3-cyanophenyl)thiazol-2-yl]carbamoyl]piperazine-2-carboxylic acid | LC-MS (Method 7A): Rt 2.20 mins; MS m/z 483.1/485.1 = [M+H]+ |
| | | ¹H NMR (500 MHz, DMSO-d6) δ 7.91 (t, *J* = 1.5 Hz, 1H), 7.85 (dt, *J* = 7.8, 1.2 Hz, 1H), 7.72 (dt, *J* = 8.0, 1.3 Hz, 1H), 7.58 (t, *J* = 7.9 Hz, 1H), 7.16 (s, 1H), 7.03 (s, 1H), 4.38 (d, *J* = 11.4 Hz, 2H), 4.08 - 4.00 (m, 1H), 3.08 (d, *J* = 13.0 Hz, 1H), 2.89 - 2.83 (m, 1H), 2.39 (s, 3H). NH and COOH protons not observed. 1 x CH₂ signal under H₂O signal. |
| 3.6 | 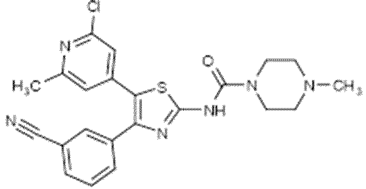 N-[5-(2-Chloro-6-methyl-4-pyridyl)-4-(3-cyanophenyl)thiazol-2-yl]-4-methyl-piperazine-1-carboxamide | LC-MS (Method 7A): Rt 2.00 mins; MS m/z 453.2/455.1 = [M+H]+ |
| | | ¹H NMR (400 MHz, DMSO-d6) δ 7.91 (t, *J* = 1.5 Hz, 1H), 7.85 (dt, *J* = 7.7, 1.4 Hz, 1H), 7.72 (dt, *J* = 7.9, 1.3 Hz, 1H), 7.59 (t, *J* = 7.9 Hz, 1H), 7.18 (s, 1H), 7.06 (s, 1H), 3.57 - 3.50 (m, 4H), 2.40 (s, 3H), 2.35 - 2.30 (m, 4H), 2.20 (s, 3H). NH proton not observed |
| 3.7 | 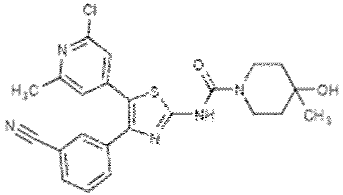 N-[5-(2-Chloro-6-methyl-4-pyridyl)-4-(3-cyanophenyl)thiazol-2-yl]-4-hydroxy-4-methyl-piperidine-1-carboxamide | LC-MS (Method 7A): Rt 3.16 mins; MS m/z 468.1/470.1 = [M+H]+ |
| | | ¹H NMR (400 MHz, DMSO-d6) δ 11.28 (br s, 1H), 7.91 (t, *J* = 1.5 Hz, 1H), 7.85 (dt, *J* = 7.7, 1.4 Hz, 1H), 7.72 (dt, *J* = 7.9, 1.3 Hz, 1H), 7.59 (t, *J* = 7.8 Hz, 1H), 7.17 (s, 1H), 7.05 (s, 1H), 4.39 (s, 1H), 3.82 (dt, *J* = 13.4, 3.7 Hz, 2H), 3.28 - 3.22 (m, 2H), 2.40 (s, 3H), 1.51 - 1.39 (m, 4H), 1.14 (s, 3H). |
| 3.8 | 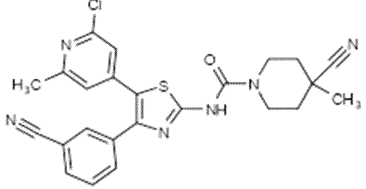 N-[5-(2-Chloro-6-methyl-4-pyridyl)-4-(3-cyanophenyl)thiazol-2-yl]-4-cyano-4-methyl-piperidine-1-carboxamide | LC-MS (Method 7A): Rt 3.63 mins; MS m/z 477.1/479.1 = [M+H]+ |
| | | ¹H NMR (400 MHz, DMSO-d6) δ 11.46 (br s, 1H), 7.91 (t, *J* = 1.5 Hz, 1H), 7.85 (dt, *J* = 7.7, 1.3 Hz, 1H), 7.72 (dt, *J* = 7.9, 1.3 Hz, 1H), 7.59 (t, *J* = 7.8 Hz, 1H), 7.20 - 7.16 (m, 1H), 7.06 (s, 1H), 4.23 (d, *J* = 14.2 Hz, 2H), 3.04 (t, *J* = 12.3 Hz, 2H), 2.40 (s, 3H), 1.92 (d, *J* = 12.7 Hz, 2H), 1.53 (td, *J* = 13.6, 3.9 Hz, 2H), 1.37 (s, 3H). |
| 3.9 | 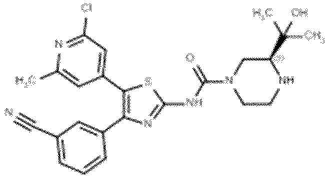 (3S)-N-[5-(2-Chloro-6-methyl-4-pyridyl)-4-(3-cyanophenyl)thiazol-2-yl]-3-(1-hydroxy-1-methylethyl)piperazine-1-carboxamide | LC-MS (Method 7A): Rt 2.08 mins; MS m/z 497.2/499.1 = [M+H]+ |
| | | ¹H NMR (500 MHz, DMSO-d6) δ 7.92 (t, *J* = 1.5 Hz, 1H), 7.85 (dt, *J* = 7.7, 1.4 Hz, 1H), 7.72 (dt, *J* = 7.9, 1.2 Hz, 1H), 7.59 (t, = 7.8 Hz, 1H), 7.17 (s, 1H), 7.05 (s, 1H), 4.48 (s, 1H), 4.20 (d, *J* = 12.4 Hz, 1H), 4.10 (d, *J* = 12.9 Hz, 1H), 2.99 (dt, *J* = 11.7, 2.1 Hz, 1H), 2.78 (t, *J* = 11.7 Hz, 1H), 2.62 - 2.53 (m, 2H), 2.40 (s, 3H), 2.38 - 2.35 (m, 1H), 1.15 (s, 3H), 1.11 (s, 3H). 2 x NH protons not observed. |
| 3.10 | 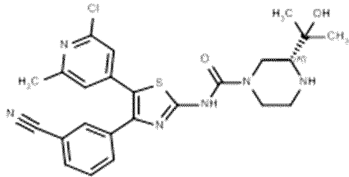 3R)-N-[5-(2-Chloro-6-methyl-4-pyridyl)-4-(3-cyanophenyl)thiazol-2-yl]-3-(1-hydroxy-1-methylethyl)piperazine-1-carboxamide | LC-MS (Method 7A): Rt 2.06 mins; MS m/z 497.2/499.1 = [M+H]+ |
| | | ¹H NMR (500 MHz, DMSO-d6) δ 7.92 (t, *J* = 1.5 Hz, 1H), 7.85 (dt, *J* = 7.7, 1.4 Hz, 1H), 7.73 (dt, *J* = 7.9, 1.2 Hz, 1H), 7.59 (t, *J =* 7.8 Hz, 1H), 7.17 (s, 1H), 7.05 (s, 1H), 4.49 (s, 1H), 4.20 (d, *J* = 12.4 Hz, 1H), 4.10 (d, *J* = 12.6 Hz, 1H), 2.99 (d, *J* = 11.6 Hz, 1H), 2.78 (t, *J* = 11.6 Hz, 1H), 2.58 (tt, *J* = 13.3, 6.9 Hz, 2H), 2.40 (s, 3H), 2.39 - 2.36 (m, 1H), 1.15 (s, 3H), 1.11 (s, 3H). 2 x NH protons not observed. |
| 3.11 | 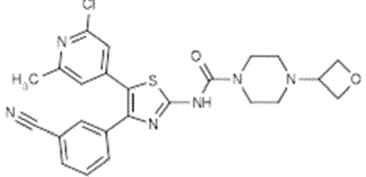 N-[5-(2-Chloro-6-methyl-4-pyridyl)-4-(3-cyanophenyl)thiazol-2-yl]-4-(oxetan-3-yl)piperazine-1-carboxamide | LC-MS (Method 7A): Rt 2.18 mins; MS m/z 495.2/497.2 = [M+H]+ |
| | | ¹H NMR (400 MHz, DMSO-d6) δ 11.35 (br s, 1H), 7.91 (s, 1H), 7.85 (d, *J* = 7.7 Hz, 1H), 7.72 (d, *J =* 7.9 Hz, 1H), 7.59 (t, *J* = 7.8 Hz, 1H), 7.18 (s, 1H), 7.06 (s, 1H), 4.54 (t, *J* = 6.5 Hz, 2H), 4.44 (d, *J =* 6.0 Hz, 2H), 3.60 - 3.52 (m, 4H), 3.43 (p, *J* = 6.1 Hz, 1H), 2.40 (s, 3H), 2.31 - 2.24 (m, 4H). |
| 3.12 | 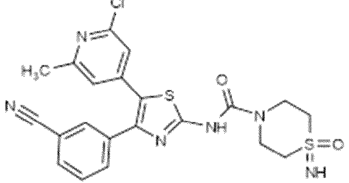 N-[5-(2-Chloro-6-methyl-4-pyridyl)-4-(3-cyanophenyl)thiazol-2-yl]-1-imino-1-oxo-1,4-thiazinane-4-carboxamide | LC-MS (Method 8B): Rt 3.12 mins; MS m/z 487.2 = [M+H]+ |
| | | ¹H NMR (500 MHz, DMSO-d6) δ 11.61 (br s, 1H), 7.91 (br s, 1H), 7.86 (d, *J* = 7.7 Hz, 1H), 7.72 (d, *J =* 7.9 Hz, 1H), 7.59 (t, *J =* 7.8 Hz, 1H), 7.17 (s, 1H), 7.05 (s, 1H), 4.11 (m, 2H), 3.82 (s, 1H), 3.78 - 3.67 (m, 2H), 3.13 - 3.00 (m, 4H), 2.40 (s, 3H). |

### Example 4

### N-[4-(3-Cyanophenyl)-5-(2,6-dimethyl-4-pyridyl)thiazol-2-yl]-4-methyl-piperazine-1-carboxamide

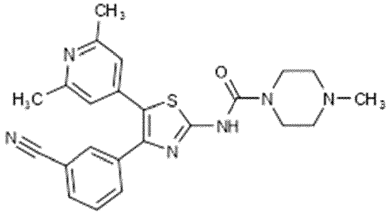

A solution of 3-[2-amino-5-(2,6-dimethyl-4-pyridyl)thiazol-4-yl]benzonitrile (Intermediate C) (50 mg, 0.16 mmol) and CDI (40 mg, 0.24 mmol) in DMA (0.5 mL) was stirred at 50 °C for 18 h. The resulting mixture was treated with 1-methylpiperazine (36 µL, 0.33 mmol) and stirring continued at 50 °C for 1 h. After cooling to room temperature, the crude mixture was purified by preparative HPLC using acidic conditions, early elution method to afford the title compound.
LC-MS (Method 7B): Rt 2.43 mins; MS m/z 433.3 = [M+H]+
¹H NMR (500 MHz, DMSO-d6) δ 11.27 (br s, 1H), 7.89 (t, *J* = 1.5 Hz, 1H), 7.82 (dt, *J* = 7.7, 1.4 Hz, 1H), 7.70 (dt, *J* = 8.0, 1.2 Hz, 1H), 7.56 (t, *J* = 7.8 Hz, 1H), 6.93 (s, 2H), 3.55 - 3.50 (m, 4H), 2.36 (s, 6H), 2.34 - 2.30 (m, 4H), 2.20 (s, 3H).

### Example 4.1

### 4-Cyano-N-[4-(3-cyanophenyl)-5-(2,6-dimethyl-4-pyridyl)thiazol-2-yl]piperidine-1-carboxamide

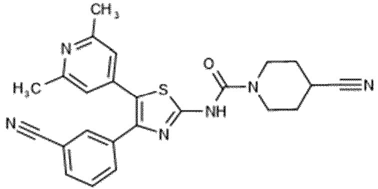

A solution of 3-[2-amino-5-(2,6-dimethyl-4-pyridyl)thiazol-4-yl]benzonitrile (Intermediate C) (50 mg, 0.16 mmol) and CDI (53 mg, 0.33 mmol) in DMA (0.5 mL) was stirred at 50 °C for 18 h. The resulting mixture was treated with piperidine-4-carbonitrile (36 mg, 0.33 mmol) and DIPEA (90 µL, 0.49 mmol) and stirring continued at 50 °C for 30 minutes. After cooling to room temperature, purification of the crude material by C18 reverse phase chromatography eluting with 5-20% MeCN in water (+0.1% ammonium hydroxide modifier) afforded a solid that was suspended in Et₂O (2 mL), collected by filtration, washed with Et₂O (2 x 1.5 mL) and dried to afford the title compound as a yellow solid.
LC-MS (Method 8B): Rt 4.17 mins; MS m/z 443.3 = [M+H]+
¹H NMR (500 MHz, DMSO-d6) δ 11.28 (s, 1H), 7.88 (s, 1H), 7.81 (d, *J* = 7.9 Hz, 1H), 7.70 (d, J = 7.9 Hz, 1H), 7.56 (t, *J* = 7.9 Hz, 1H), 6.92 (s, 2H), 3.83 - 3.76 (m, 2H), 3.45-3.38 (m, 2H), 3.14 - 3.08 (m, 1H), 2.36 (s, 6H), 1.95 - 1.86 (m, 2H), 1.75 - 1.65 (m, 2H).
The compounds of the following tabulated Examples (Table Ex4) were prepared analogously to Example 4 or Example 4.1 from 3-[2-amino-5-(2,6-dimethyl-4-pyridyl)thiazol-4-yl]benzonitrile (Intermediate C) and the appropriate amine.

**Table Ex4**

| **Ex.** | **Structure and Name** | **Retention Time, [M+H]+, ¹H NMR** |
|---|---|---|
| 4.2 | 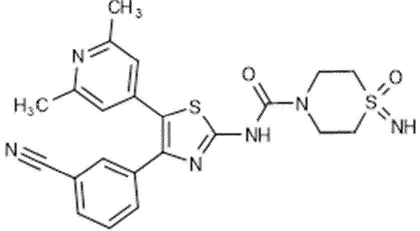 N-[4-(3-Cyanophenyl)-5-(2,6-dimethyl-4-pyridyl)thiazol-2-yl]-1-imino-1-oxo-1,4-thiazinane-4-carboxamide | LC-MS (Method 8B): Rt 2.65 mins; MS m/z 467.2 = [M+H]+ ¹H NMR (500 MHz, DMSO-d6) δ 11.51 (br s, 1H), 7.88 (d, J = 1.7 Hz, 1H), 7.82 (d, J= 7.8 Hz, 1H), 7.71 (d, *J* = 7.8 Hz, 1H), 7.56 (t, *J* = 7.8 Hz, 1H), 6.93 (s, 2H), 4.16 - 4.04 (m, 2H), 3.81 (s, 1H), 3.78 - 3.66 (m, 2H), 3.13 - 3.02 (m, 4H), 2.36 (s, 6H). |
| 4.3 | 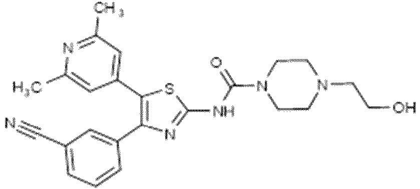 N-[4-(3-Cyanophenyl)-5-(2,6-dimethyl-4-pyridyl)thiazol-2-yl]-4-(2-hydroxyethyl)piperazine-1-carboxamide | LC-MS (Method 8B): Rt 3.73 mins; MS m/z 463.3 = [M+H]+ ¹H NMR (500 MHz, DMSO-d6) δ 11.24 (s, 1H), 7.89 (d, *J* = 1.7 Hz, 1H), 7.82 (dt, *J* = 7.9, 1.8 Hz, 1H), 7.71 (dt, *J* = 8.1, 1.6 Hz, 1H), 7.56 (t, *J* = 7.8 Hz, 1H), 6.93 (s, 2H), 4.44 (t, *J* = 5.4 Hz, 1H), 3.56 - 3.49 (m, 6H), 2.46 - 2.40 (m, 6H), 2.36 (s, 6H). |
| 4.4 | 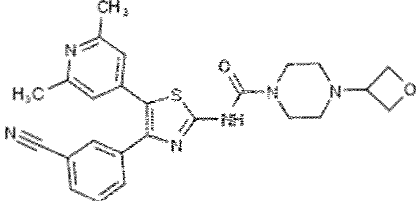 N-[4-(3-Cyanophenyl)-5-(2,6-dimethyl-4-pyridyl)thiazol-2-yl]-4-(oxetan-3-yl)piperazine-1-carboxamide | LC-MS (Method 8B): Rt 3.77 mins; MS m/z 475.3 = [M+H]+ ¹H NMR (500 MHz, DMSO-d6) δ 11.25 (s, 1H), 7.88 (t, *J* = 1.6 Hz, 1H), 7.82 (dt, J = 7.8, 1.6 Hz, 1H), 7.70 (dt, J = 7.8, 1.6 Hz, 1H), 7.56 (t, *J* = 7.8 Hz, 1H), 6.92 (s, 2H), 4.54 (apr t, *J* = 6.5 Hz, 2H), 4.45 (apr t, *J* = 6.1 Hz, 2H), 3.60 - 3.52 (m, 4H), 3.43 (apr p, *J* = 6.3 Hz, 1H), 2.36 (s, 6H), 2.30 - 2.25 (m, 4H). |
| 4.5 | 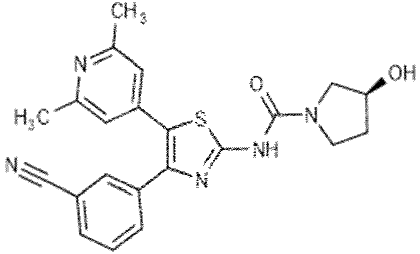 (3S)-N-[4-(3-Cyanophenyl)-5-(2,6-dimethyl-4-pyridyl)thiazol-2-yl]-3-hydroxy-pyrrolidine-1-carboxamide | LC-MS (Method 8B): Rt 3.66 mins; MS m/z 420.3 = [M+H]+ ¹H NMR (500 MHz, DMSO-d6) δ 11.01 (s, 1H), 7.89 (t, *J* = 1.7 Hz, 1H), 7.81 (dt, J = 7.8, 1.4 Hz, 1H), 7.71 (dt, *J* = 8.1, 1.5 Hz, 1H), 7.56 (t, *J* = 7.8 Hz, 1H), 6.93 (s, 2H), 5.01 (br s, 1H), 4.31 (br s, 1H), 3.49 (br s, 3H), 2.36 (s, 6H), 2.00 - 1.76 (m, 2H). OH proton not observed. |
| 4.6 | 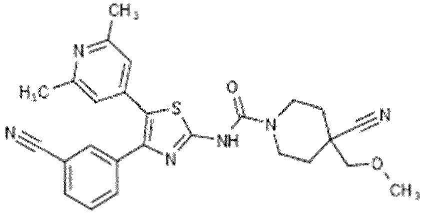 4-Cyano-N-[4-(3-cyanophenyl)-5-(2,6-dimethyl-4-pyridyl)thiazol-2-yl]-4-(methoxymethyl)piperidine-1-carboxamide | LC-MS (Method 8B): Rt 3.88 mins; MS m/z 487.2 = [M+H]+ ¹H NMR (500 MHz, DMSO-d6) δ 11.32 (s, 1H), 7.88 (s, 1H), 7.82 (dt, *J* = 7.8, 1.4 Hz, 1H), 7.70 (dt, *J =* 7.8, 1.4 Hz, 1H), 7.56 (t, *J* = 7.8 Hz, 1H), 6.92 (s, 2H), 4.26 (d, *J* = 13.7 Hz, 2H), 3.46 (s, 2H), 3.34 (s, 3H), 3.03 (t, *J* = 13.0 Hz, 2H), 2.36 (s, 6H), 1.93 (d, *J* = 13.7 Hz, 2H), 1.61 - 1.50 (m, 2H). |
| 4.7 | 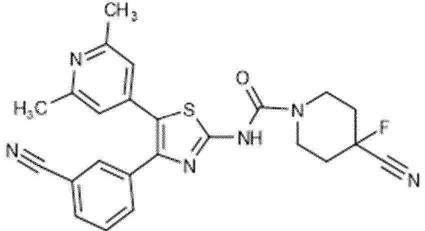 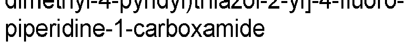 4-Cyano-N-[4-(3-cyanophenyl)-5-(2,6-dimethyl-4-pyridyl)thiazopl-2-yl]-4-fluoro-piperidine-1-carboxamide | LC-MS (Method 8B): Rt 3.68 mins; MS m/z 461.2 = [M+H]+ ¹H NMR (500 MHz, DMSO-d6) δ 11.42 (s, 1H), 7.89 (s, 1H), 7.82 (d, *J* = 7.9 Hz, 1H), 7.71 (d, *J* = 7.9 Hz, 1H), 7.56 (t, *J* = 7.9 Hz, 1H), 6.92 (s, 2H), 3.81 - 3.72 (m, 2H), 3.63 - 3.54 (m, 2H), 2.36 (s, 6H), 2.33 - 2.22 (m, 2H), 2.22 - 2.13 (m, 2H). |
| 4.8 | 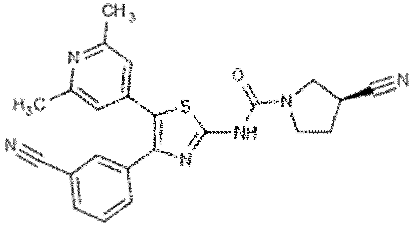 (3S)-3-Cyano-N-[4-(3-cyanophenyl)-5-(2,6-dimethyl-4-pyridyl)thiazol-2-yl]pyrrolidine-1-carboxamide | LC-MS (Method 8B): Rt 3.88 mins; MS m/z 429.3 = [M+H]+ ¹H NMR (500 MHz, DMSO-d6) δ 11.22 (s, 1H), 7.89 (s, 1H), 7.82 (d, *J* = 7.8 Hz, 1H), 7.71 (d, *J* = 7.8 Hz, 1H), 7.56 (t, *J* = 7.8 Hz, 1H), 6.93 (s, 2H), 3.80 - 3.74 (m, 1H), 3.64 - 3.52 (m, 4H), 2.36 (s, 6H), 2.32 - 2.26 (m, 1H), 2.23 - 2.15 (m, 1H). |
| 4.9 | 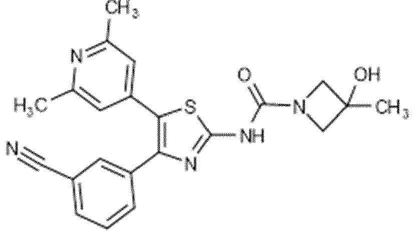 N-[4-(3-Cyanophenyl)-5-(2,6-dimethyl-4-pyridyl)thiazol-2-yl]-3-hydroxy-3-methyl-azetidine-1-carboxamide | LC-MS (Method 8B): Rt 3.71 mins; MS m/z 420.3 = [M+H]+ ¹H NMR (500 MHz, DMSO-d6) δ 11.29 (s, 1H), 7.87 (s, 1H), 7.81 (d, *J* = 7.8 Hz, 1H), 7.70 (d, *J* = 7.8 Hz, 1H), 7.55 (t, *J* = 7.8 Hz, 1H), 6.92 (s, 2H), 5.65 (s, 1H), 3.94 - 3.82 (m, 4H), 2.36 (s, 6H), 1.38 (s, 3H). |
| 4.10 | 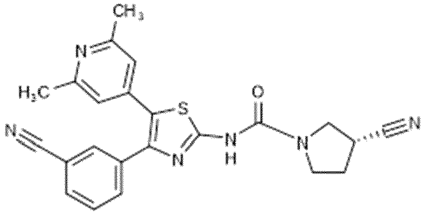 (3R)-3-Cyano-N-[4-(3-cyanophenyl)-5-(2,6-dimethyl-4-pyridyl)thiazol-2-yl]pyrrolidine-1-carboxamide | LC-MS (Method 8B): Rt 3.95 mins; MS m/z 429.3 = [M+H]+ ¹H NMR (400 MHz, DMSO-d6) δ 11.23 (s, 1H), 7.89 (t, *J* = 1.7 Hz, 1H), 7.82 (dt, J = 7.9, 1.4 Hz, 1H), 7.71 (dt, J = 7.9, 1.4 Hz, 1H), 7.56 (t, *J* = 7.9 Hz, 1H), 6.93 (s, 2H), 3.81 - 3.73 (m, 1H), 3.67 - 3.46 (m, 4H), 2.36 (s, 6H), 2.31 - 2.26 (m, 1H), 2.24 - 2.15 (m, 1H). |
| 4.11 | 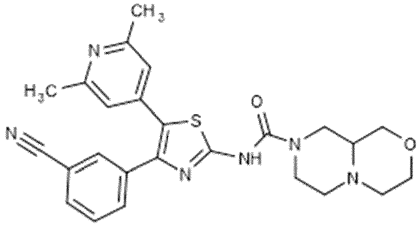 N-[4-(3-Cyanophenyl)-5-(2,6-dimethyl-4-pyridyl)thiazol-2-yl]-3,4,6,7,9,9a-hexahydro-1H-pyrazino[2,1-c][1,4]oxazine-8-carboxamide | LC-MS (Method 8B): Rt 3.75 mins; MS m/z 475.3 = [M+H]+ ¹H NMR (500 MHz, DMSO-d6) δ 11.27 (s, 1H), 7.89 (d, J = 2.1 Hz, 1H), 7.83 (d, J = 7.7 Hz, 1H), 7.71 (d, J = 8.0 Hz, 1H), 7.57 (t, J = 7.8 Hz, 1H), 6.94 (s, 2H), 4.17 (d, J = 13.1 Hz, 1H), 4.04 (d, J = 12.8 Hz, 1H), 3.76 (dd, J = 11.6, 3.0 Hz, 1H), 3.69 (dd, J = 11.1, 3.0 Hz, 1H), 3.53 (td, J = 11.4, 2.4 Hz, 1H), 3.12 (t, J = 10.5 Hz, 1H), 3.00 (t, J = 12.6 Hz, 1H), 2.76 (d, J = 11.4 Hz, 1H), 2.70 - 2.61 (m, 1H), 2.58 - 2.52 (m, 1H), 2.37 (s, 6H), 2.22 (td, J = 11.8, 3.3 Hz, 1H), 2.13 (dd, J = 11.9, 3.2 Hz, 1H), 2.06 (d, J = 11.1 Hz, 1H). |
| 4.13 | 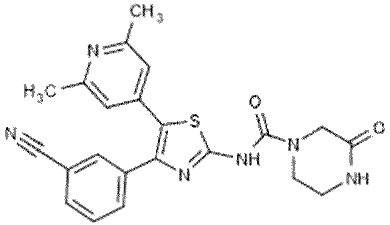 N-[4-(3-Cyanophenyl)-5-(2,6-dimethyl-4-pyridyl)thiazol-2-yl]-3-oxo-piperazine-1-carboxamide | LC-MS (Method 8B): Rt 2.95 mins; MS m/z 433.3 = [M+H]+ ¹H NMR (500 MHz, DMSO-d6) δ 11.38 (s, 1H), 8.14 (s, 1H), 7.90 (s, 1H), 7.83 (d, *J* = 7.7 Hz, 1H), 7.72 (d, *J* = 8.0 Hz, 1H), 7.57 (t, *J = 7.8* Hz, 1H), 6.94 (s, 2H), 4.08 (s, 2H), 3.71 (t, *J* = 5.4 Hz, 2H), 3.26 (q, *J* = 6.1, 4.3 Hz, 2H), 2.37 (s, 6H). |
| 4.14 | 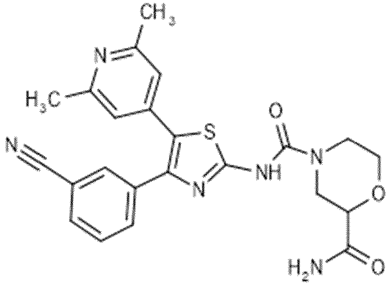 N4-[4-(3-Cyanophenyl)-5-(2,6-dimethyl-4-pyridyl)thiazol-2-yl]morpholine-2,4-dicarboxamide | LC-MS (Method 8B): Rt 3.01 mins; MS m/z 461.2 = [M+H]+ ¹H NMR (500 MHz, DMSO-d6) δ 11.44 (s, 1H), 7.90 (s, 1H), 7.83 (d, *J* = 7.7 Hz, 1H), 7.71 (d, *J* = 8.0 Hz, 1H), 7.57 (t, *J* = 7.8 Hz, 1H), 7.40 (s, 1H), 7.33 (s, 1H), 6.94 (s, 2H), 4.31 (d, *J =* 13.4 Hz, 1H), 4.02 (d, *J* = 13.4 Hz, 1H), 3.94 (d, *J* = 11.6 Hz, 1H), 3.90 (dd, *J* = 10.1, 3.0 Hz, 1H), 3.61 - 3.52 (m, 1H), 3.09 (t, *J* = 12.1 Hz, 1H), 2.95 (s, 1H), 2.37 (s, 6H). |
| 4.15 | 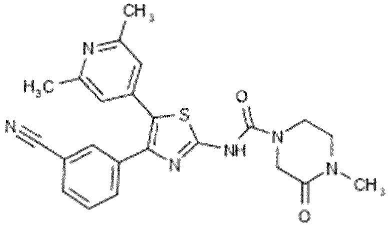 N-[4-(3-Cyanophenyl)-5-(2,6-dimethyl-4-pyridyl)thiazol-2-yl]-4-methyl-3-oxo-piperazine-1-carboxamide | LC-MS (Method 8B): Rt 3.50 mins; MS m/z 447.3 = [M+H]+ ¹H NMR (500 MHz, DMSO-d6) δ 11.44 (s, 1H), 7.90 (s, 1H), 7.83 (d, *J* = 7.7 Hz, 1H), 7.71 (d, *J* = 7.9 Hz, 1H), 7.57 (t, *J* = 7.8 Hz, 1H), 6.94 (s, 2H), 4.13 (s, 2H), 3.80 (t, *J* = 5.4 Hz, 2H), 3.39 (dd, *J* = 6.4, 4.3 Hz, 2H), 2.88 (s, 3H), 2.37 (s, 6H). |
| 4.16 | 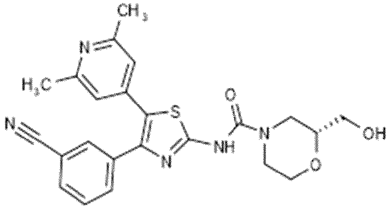 (2R)-N-[4-(3-Cyanophenyl)-5-(2,6-dimethyl-4-pyridyl)thiazol-2-yl]-2-(hydroxymethyl)morpholine-4-carboxamide | LC-MS (Method 8B): Rt 3.16 mins; MS m/z 450.3 = [M+H]+ ¹H NMR (500 MHz, DMSO-d6) δ 11.31 (s, 1H), 7.90 (s, 1H), 7.83 (d, *J* = 7.7 Hz, 1H), 7.71 (d, *J* = 7.9 Hz, 1H), 7.57 (t, *J* = 7.8 Hz, 1H), 6.94 (s, 2H), 4.83 (t, *J* = 5.4 Hz, 1H), 4.17 (d, *J* = 13.3 Hz, 1H), 4.05 (d, *J* = 13.3 Hz, 1H), 3.88 (dd, J = 11.5, 3.2 Hz, 1H), 3.50 - 3.42 (m, 2H), 3.40 (d, *J* = 4.6 Hz, 2H), 2.99 (t, *J* = 11.8 Hz, 1H), 2.74 (t, *J* = 11.6 Hz, 1H), 2.37 (s, 6H). |
| 4.17 | 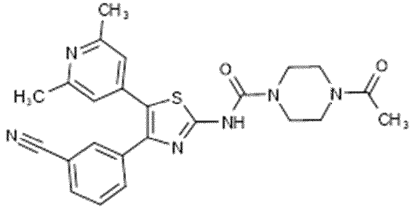 4-Acetyl-N-[4-(3-cyanophenyl)-5-(2,6-dimethyl-4-pyridyl)thiazol-2-yl]piperazine-1-carboxamide | LC-MS (Method 8B): Rt 3.45 mins; MS m/z 461.3 = [M+H]+ ¹H NMR (500 MHz, DMSO-d6) δ 11.34 (s, 1H), 7.89 (t, *J* = 1.9 Hz, 1H), 7.82 (d, *J* = 7.8 Hz, 1H), 7.71 (d, *J* = 7.8 Hz, 1H), 7.56 (t, *J = 7.8* Hz, 1H), 6.93 (s, 2H), 3.60 - 3.55 (m, 2H), 3.53 - 3.45 (m, 6H), 2.36 (s, 6H), 2.03 (s, 3H). |
| 4.18 | 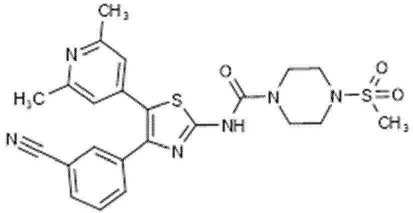 N-[4-(3-cyanophenyl)-5-(2,6-dimethyl-4-pyridyl)thiazol-2-yl]-4-methylsulfonyl-piperazine-1-carboxamide | LC-MS (Method 8B): Rt 3.22 mins; MS m/z 497.3 = [M+H]+ ¹H NMR (500 MHz, DMSO-d6) δ 11.42 (s, 1H), 7.89 (s, 1H), 7.83 (d, *J* = 7.7 Hz, 1H), 7.71 (d, *J* = 8.0 Hz, 1H), 7.56 (t, *J* = 7.9 Hz, 1H), 6.93 (s, 2H), 3.73 - 3.61 (m, 4H), 3.19 - 3.12 (m, 4H), 2.91 (s, 3H), 2.36 (s, 6H). |
| 4.19 | 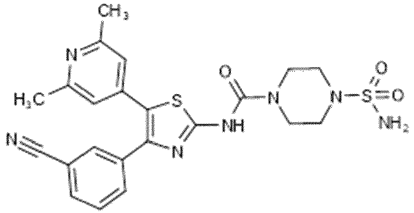 N-[4-(3-cyanophenyl)-5-(2,6-dimethyl-4-pyridyl)thiazol-2-yl]-4-sulfamoyl-piperazine-1-carboxamide | LC-MS (Method 8B): Rt 3.29 mins; MS m/z 498.3 = [M+H]+ ¹H NMR (500 MHz, DMSO-d6) δ 11.39 (s, 1H), 7.89 (s, 1H), 7.83 (d, *J* = 7.8 Hz, 1H), 7.70 (d, *J* = 7.8 Hz, 1H), 7.56 (t, *J* = 7.8 Hz, 1H), 6.93 (s, 2H), 6.88 (s, 2H), 3.64 (apr br t ,J = 5.1 Hz, 4H), 2.98 (apr br t ,J = 5.1 Hz, 4H), 2.36 (s, 6H). |
| 4.20 | 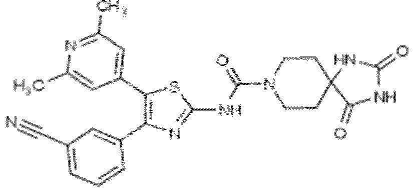 N-[4-(3-Cyanophenyl)-5-(2,6-dimethyl-4-pyridyl)thiazol-2-yl]-2,4-dioxo-1,3,8-triazaspiro[4.5]decane-8-carboxamide | LC-MS (Method 8B): Rt 3.33 mins; MS m/z 502.3 = [M+H]+ ¹H NMR (500 MHz, DMSO-d6) δ 11.34 (s, 1H), 10.75 (s, 1H), 8.58 (s, 1H), 7.89 (s, 1H), 7.82 (d,J = 7.8 Hz, 1H), 7.70 (d,*J* = 7.8 Hz, 1H), 7.56 *(t,J* = 7.8 Hz, 1H), 6.93 (s, 2H), 4.10 - 4.03 (m, 2H), 3.31 - 3.25 (m, 2H), 2.36 (s, 6H), 1.82 - 1.74 (m, 2H), 1.64 - 1.56 (m, 2H). |
| 4.21 | 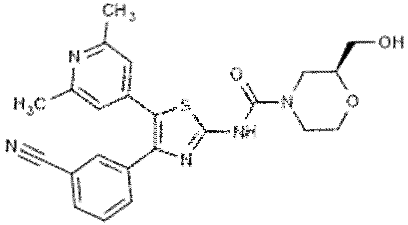 (2S)-N-[4-(3-Cyanophenyl)-5-(2,6-dimethyl-4-pyridyl)thiazol-2-yl]-2-(hydroxymethyl)morpholine-4-carboxamide | LC-MS (Method 8B): Rt 3.18 mins; MS m/z 450.3 = [M+H]+ ¹H NMR (500 MHz, DMSO-d6) δ 11.29 (s, 1H), 7.89 (t, *J* = 1.8 Hz, 1H), 7.82 (d, *J* = 7.8 Hz, 1H), 7.70 (d, *J* = 7.8 Hz, 1H), 7.56 (t, *J = 7.8* Hz, 1H), 6.93 (s, 2H), 4.82 (t, *J =* 5.5 Hz, 1H), 4.17 (d, *J =* 13.2 Hz, 1H), 4.04 (d, *J =* 13.2 Hz, 1H), 3.90 - 3.84 (m, 1H), 3.50 - 3.42 (m, 2H), 3.42 - 3.38 (m, 2H), 3.02 - 2.94 (m, 1H), 2.77 - 2.69 (m, 1H), 2.36 (s, 6H). |
| 4.22 | 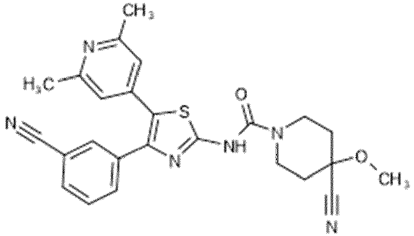 4-Cyano-N-[4-(3-cyanophenyl)-5-(2,6-dimethyl-4-pyridyl)thiazol-2-yl]-4-methoxy-piperidine-1-carboxamide | LC-MS (Method 8B): Rt 3.93 mins; MS m/z 473.3 = [M+H]+ ¹H NMR (500 MHz, DMSO-d6) δ 11.37 (s, 1H), 7.89 (s, 1H), 7.82 (d, *J* = 7.8 Hz, 1H), 7.70 (d, *J* = 7.8 Hz, 1H), 7.56 (t, *J* = 7.8 Hz, 1H), 6.93 (s, 2H), 3.75 - 3.65 (m, 2H), 3.55 - 3.47 (m, 2H), 3.40 (s, 3H), 2.36 (s, 6H), 2.17 - 2.09 (m, 2H), 1.93-1.86 (m, 2H). |
| 4.23 | 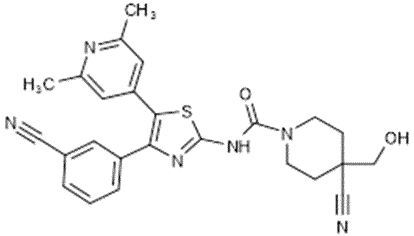 4-Cyano-N-[4-(3-cyanophenyl)-5-(2,6-dimethyl-4-pyridyl)thiazol-2-yl]-4-(hydroxymethyl)piperidine-1-carboxamide (Prepared using Intermediate E) | LC-MS (Method 8B): Rt 3.66 mins; MS m/z 473.3 = [M+H]+ ¹H NMR (500 MHz, DMSO-d6) δ 11.32 (s, 1H), 7.88 (s, 1H), 7.82 (d, *J* = 7.8 Hz, 1H), 7.70 (d, *J* = 7.8 Hz, 1H), 7.56 (t, *J* = 7.8 Hz, 1H), 6.92 (s, 2H), 5.50 (t, *J* = 5.7 Hz, 1H), 4.28 (d, *J* = 13.9 Hz, 2H), 3.50 (d, *J* = 5.7 Hz, 2H), 3.00 (t, *J* = 13.0 Hz, 2H), 2.35 (s, 6H), 1.88 (d, *J* = 13.6 Hz, 2H), 1.53 - 1.45 (m, 2H). |
| 4.24 | 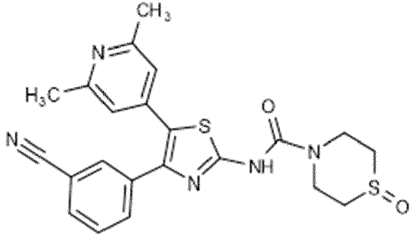 N-[4-(3-Cyanophenyl)-5-(2,6-dimethyl-4-pyridyl)thiazol-2-yl]-1-oxo-1,4-thiazinane-4-carboxamide | LC-MS (Method 8B): Rt 3.25 mins; MS m/z 452.2 = [M+H]+ ¹H NMR (500 MHz, DMSO-d6) δ 11.46 (s, 1H), 7.90 (s, 1H), 7.83 (d, J = 7.8 Hz, 1H), 7.71 (d, J = 7.8 Hz, 1H), 7.57 (t, J = 7.8 Hz, 1H), 6.94 (s, 2H), 4.12 (d, J = 14.6 Hz, 2H), 3.79 (t, J = 13.0 Hz, 2H), 2.94 (t, J = 12.5 Hz, 2H), 2.76 (d, J = 13.6 Hz, 2H), 2.37 (s, 6H). |
| 4.25 | 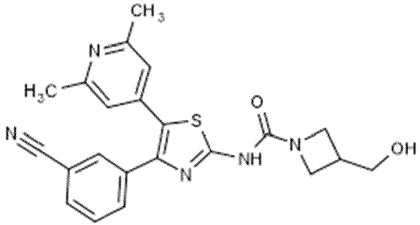 N-[4-(3-Cyanophenyl)-5-(2,6-dimethyl-4-pyridyl)thiazol-2-yl]-3-(hydroxymethyl)azetidine-1-carboxamide | LC-MS (Method 8B): Rt 3.66 mins; MS m/z 420.3 = [M+H]+ ¹H NMR (500 MHz, DMSO-d6) δ 11.26 (s, 1H), 7.88 (s, 1H), 7.82 (d, *J* = 7.7 Hz, 1H), 7.70 (d, *J* = 7.9 Hz, 1H), 7.56 (t, *J* = 7.8 Hz, 1H), 6.93 (s, 2H), 4.84 (t, *J* = 5.3 Hz, 1H), 4.03 (s, 2H), 3.77 (s, 2H), 3.53 (t, *J* = 5.7 Hz, 2H), 2.72 - 2.66 (m, 1H), 2.36 (s, 6H). |
| 4.26 | 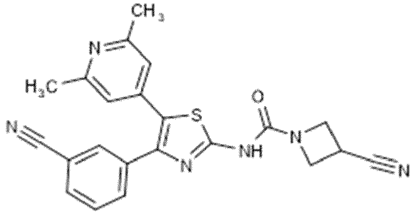 3-Cyano-N-[4-(3-cyanophenyl)-5-(2,6-dimethyl-4-pyridyl)thiazol-2-yl]azetidine-1-carboxamide | LC-MS (Method 8B): Rt 3.58 mins; MS m/z 415.3 = [M+H]+ ¹H NMR (500 MHz, DMSO-d6) δ 11.51 (s, 1H), 7.87 (s, 1H), 7.82 (d, *J* = 7.7 Hz, 1H), 7.69 (d, *J* = 7.9 Hz, 1H), 7.56 (t, *J* = 7.8 Hz, 1H), 6.93 (s, 2H), 4.32 (t, *J* = 9.0 Hz, 2H), 4.19 (t, *J* = 7.3 Hz, 2H), 3.85 - 3.75 (m, 1H), 2.36 (s, 6H). |
| 4.27 | 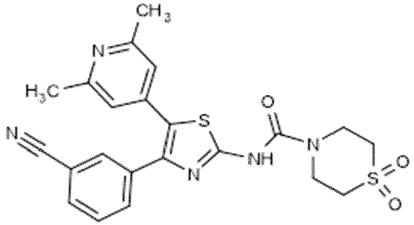 N-[4-(3-Cyanophenyl)-5-(2,6-dimethyl-4-pyridyl)thiazol-2-yl]-1,1-dioxo-1,4-thiazinane-4-carboxamide | LC-MS (Method 8B): Rt 3.20 mins; MS m/z 468.2 = [M+H]+ ¹H NMR (500 MHz, DMSO-d6) δ 11.58 (s, 1H), 7.89 (s, 1H), 7.83 (d, J = 7.8 Hz, 1H), 7.70 (d, J = 7.8 Hz, 1H), 7.56 (t, J = 7.8 Hz, 1H), 6.92 (s, 2H), 4.01 - 3.92 (m, 4H), 3.25 - 3.18 (m, 4H), 2.36 (s, 6H). |
| 4.28 | 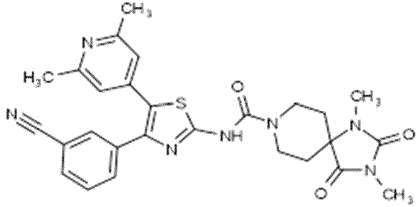 N-[4-(3-Cyanophenyl)-5-(2,6-dimethyl-4-pyridyl)thiazol-2-yl]-1,3-dimethyl-2,4-dioxo-1,3,8-triazaspiro[4.5]decane-8-carboxamide | LC-MS (Method 8B): Rt 4.09 mins; MS m/z 530.3 = [M+H]+ ¹H NMR (500 MHz, DMSO-d6) δ 11.33 (s, 1H), 7.90 (s, 1H), 7.83 (d, *J* = 7.7 Hz, 1H), 7.71 (d, *J* = 7.9 Hz, 1H), 7.57 (t, *J = 7.8* Hz, 1H), 6.94 (s, 2H), 4.25 (d, *J* = 13.1 Hz, 2H), 3.48 (t, *J* = 13.0 Hz, 2H), 2.87 (s, 3H), 2.77 (s, 3H), 2.37 (s, 6H), 1.93 (td, J = 13.2, 4.7 Hz, 2H), 1.73 (d, *J* = 13.3 Hz, 2H). |
| 4.29 | 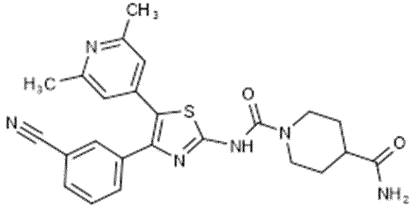 N1-[4-(3-Cyanophenyl)-5-(2,6-dimethyl-4-pyridyl)thiazol-2-yl]piperidine-1,4-dicarboxamide | LC-MS (Method 8B): Rt 3.60 mins; MS m/z 461.3 = [M+H]+ ¹H NMR (500 MHz, DMSO-d6) δ 11.23 (s, 1H), 7.90 (s, 1H), 7.83 (d, *J* = 7.7 Hz, 1H), 7.71 (d, *J* = 8.0 Hz, 1H), 7.57 (t, *J* = 7.8 Hz, 1H), 7.30 (s, 1H), 6.93 (s, 2H), 6.83 (s, 1H), 4.19 (d, *J* = 13.3 Hz, 2H), 2.90 (t, *J* = 12.7 Hz, 2H), 2.36 (s, 6H), 2.36 - 2.29 (m, 1H), 1.74 (d, *J* = 12.6 Hz, 2H), 1.47 (qd, *J* = 12.4, 3.9 Hz, 2H). |
| 4.30 | 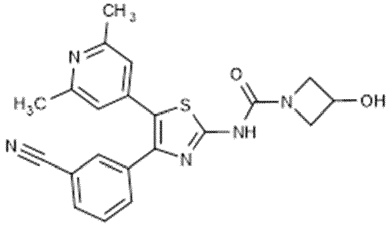 N-[4-(3-Cyanophenyl)-5-(2,6-dimethyl-4-pyridyl)thiazol-2-yl]-3-hydroxy-azetidine-1-carboxamide | LC-MS (Method 8B): Rt 3.33 mins; MS m/z 406.3 = [M+H]+ ¹H NMR (500 MHz, DMSO-*d*₆) δ 11.28 (s, 1H), 7.88 (s, 1H), 7.82 (d, *J* = 7.8 Hz, 1H), 7.70 (d, *J =* 7.8 Hz, 1H), 7.56 (t, *J =* 7.8 Hz, 1H), 6.94 (s, 2H), 5.74 (d, *J =* 6.5 Hz, 1H), 4.50 - 4.43 (m, 1H), 4.28 - 4.19 (m, 2H), 3.81 - 3.74 (m, 2H), 2.36 (s, 6H). |
| 4.31 | 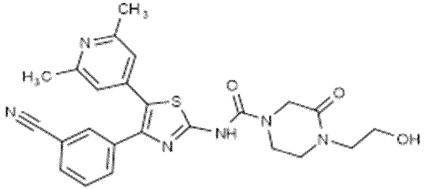 N-[4-(3-Cyanophenyl)-5-(2,6-dimethyl-4-pyridyl)thiazol-2-yl]-4-(2-hydroxyethyl)-3-oxo-piperazine-1-carboxamide | LC-MS (Method 8B): Rt 3.29 mins; MS m/z 477.3 = [M+H]+ ¹H NMR (500 MHz, DMSO-d6) δ 11.42 (s, 1H), 7.90 (s, 1H), 7.83 (d, *J* = 7.7 Hz, 1H), 7.72 (d, *J* = 7.9 Hz, 1H), 7.57 (t, *J* = 7.8 Hz, 1H), 6.94 (s, 2H), 4.77 (t, *J* = 5.5 Hz, 1H), 4.13 (s, 2H), 3.78 (t, *J* = 5.4 Hz, 2H), 3.53 (q, *J* = 5.8 Hz, 2H), 3.48 (t, *J* = 5.4 Hz, 2H), 3.39 (t, *J* = 5.9 Hz, 2H), 2.37 (s, 6H). |
| 4.32 | 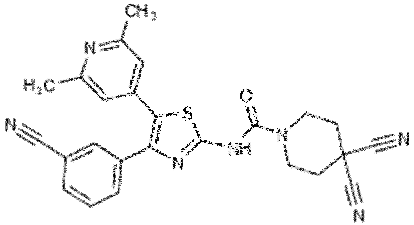 4,4-Dicyano-N-[4-(3-cyanophenyl)-5-(2,6-dimethyl-4-pyridyl)thiazol-2-yl]piperidine-1-carboxamide (Prepared using Intermediate F) | LC-MS (Method 8B): Rt 3.86 mins; MS m/z 468.3 = [M+H]+ ¹H NMR (500 MHz, Methanol-d4) δ 7.92 (s, 1H), 7.72 (t, *J* = 7.2 Hz, 2H), 7.52 (t, *J = 7.8* Hz, 1H), 7.02 (s, 2H), 3.83 (t, *J* = 5.5 Hz, 4H), 2.45 (s, 6H), 2.38 (t, *J* = 5.5 Hz, 4H). NH proton not observed. |
| 4.33 | 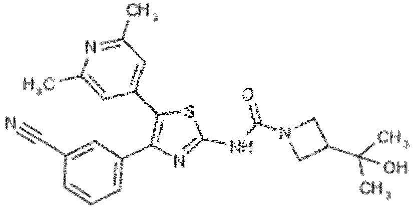 N-[4-(3-Cyanophenyl)-5-(2,6-dimethyl-4-pyridyl)thiazol-2-yl]-3-(1-hydroxy-1-methyl-ethyl)azetidine-1-carboxamide | LC-MS (Method 8B): Rt 3.93 mins; MS m/z 448.3 = [M+H]+ ¹H NMR (500 MHz, DMSO-d6) δ 11.25 (s, 1H), 7.88 (s, 1H), 7.82 (d, *J* = 7.6 Hz, 1H), 7.70 (d, *J* = 7.9 Hz, 1H), 7.56 (t, *J* = 7.8 Hz, 1H), 6.93 (s, 2H), 4.58 (s, 1H), 3.93 (s, 4H), 2.59 (p *,J* = 7.6 Hz, 1H), 2.36 (s, 6H), 1.04 (s, 6H). |
| 4.34 | 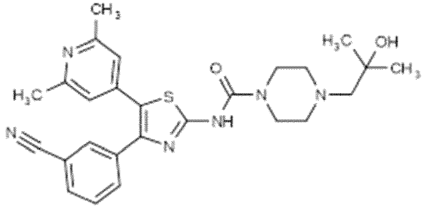 N-[4-(3-Cyanophenyl)-5-(2,6-dimethyl-4-pyridyl)thiazol-2-yl]-4-(2-hydroxy-2-methyl-propyl)piperazine-1-carboxamide | LC-MS (Method 7B): Rt 2.94 mins; MS m/z = 491.4 [M+H]+ 1H NMR (400 MHz, DMSO-d6) δ 11.18 (s, 1H), 7.88 (t, J = 1.5 Hz, 1H), 7.82 (dt, J = 7.7, 1.3 Hz, 1H), 7.70 (dt, J = 8.0, 1.3 Hz, 1H), 7.56 (t, J = 7.8 Hz, 1H), 6.92 (s, 2H), 4.13 (s, 1H), 3.55 - 3.48 (m, 4H), 2.55 - 2.52 (m, 4H), 2.36 (s, 6H), 2.22 (s, 2H), 1.10 (s, 6H). |
| 4.36 | 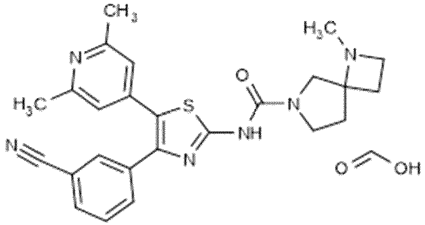 N-[4-(3-Cyanophenyl)-5-(2,6-dimethyl-4-pyridyl)thiazol-2-yl]-1-methyl-1,7-diazaspiro[3.4]octane-7-carboxamide formate salt | LC-MS (Method 7B): Rt 2.75 mins; MS m/z 459.4 = [M+H]+ 1H NMR (500 MHz, DMSO-d6) δ 11.04 (s, 1H), 8.25 (s, 1H), 7.89 (t, J = 1.6 Hz, 1H), 7.82 (dt, J = 7.7, 1.4 Hz, 1H), 7.71 (dt, J = 7.7, 1.3 Hz, 1H), 7.56 (dd, J = 7.8 Hz, 1H), 6.93 (s, 2H), 3.59 - 3.45 (m, 3H), 3.12 - 3.08 (m, 1H), 3.03 - 2.98 (m, 1H), 2.36 (s, 6H), 2.13 (s, 3H), 2.10 - 2.02 (m, 2H), 2.01 - 1.88 (m, 3H). |
| 4.37 | 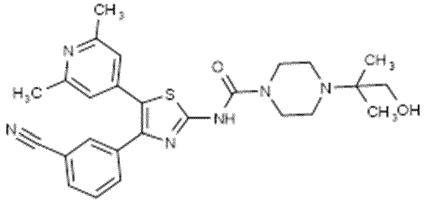 N-[4-(3-Cyanophenyl)-5-(2,6-dimethyl-4-pyridyl)thiazol-2-yl]-4-(2-hydroxy-1,1-dimethyl-ethyl)piperazine-1-carboxamide | LC-MS (Method 7B): Rt 2.99 mins; MS m/z 491.4 = [M+H]+ 1H NMR (500 MHz, Methanol-d4) δ 7.90 (t, J = 1.7 Hz, 1H), 7.71 (dt, J = 7.9, 1.5 Hz, 1H), 7.68 (dt, J = 7.8, 1.4 Hz, 1H), 7.49 (dd, J = 7.8, 7.8 Hz, 1H), 7.00 (s, 2H), 3.61 - 3.56 (m, 4H), 3.46 (s, 2H), 2.74 - 2.68 (m, 4H), 2.43 (s, 6H), 1.07 (s, 6H). Exchangeable protons not observed. |
| 4.38 | 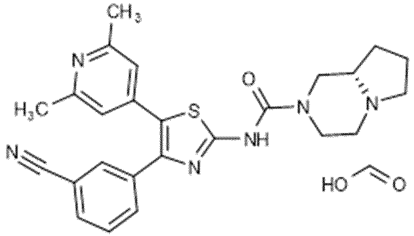 (8aS)-N-[4-(3-Cyanophenyl)-5-(2,6-dimethyl-4-pyridyl)thiazol-2-yl]-3,4,6,7,8,8a-hexahydro-1H-pyrrolo[1,2-a]pyrazine-2-carboxamide formate salt | LC-MS (Method 7B): Rt 2.93 mins; MS m/z 459.4 = [M+H]+ 1H NMR (400 MHz, Methanol-d4) δ 7.92 (t, J = 1.8 Hz, 1H), 7.76 - 7.68 (m, 2H), 7.51 (dd, J = 7.9, 7.9 Hz, 1H), 7.03 (s, 2H), 4.36 (d, J = 13.3 Hz, 1H), 4.22 (d, J = 13.2 Hz, 1H), 3.23 - 3.14 (m, 3H), 2.87 (dd, J = 13.0, 10.4 Hz, 1H), 2.46 (s, 6H), 2.43 - 2.36 (m, 2H), 2.33 - 2.23 (m, 1H), 2.06 - 1.97 (m, 1H), 1.97 - 1.84 (m, 2H), 1.62 - 1.47 (m, 1H). Exchangeable protons not observed |
| 4.39 | 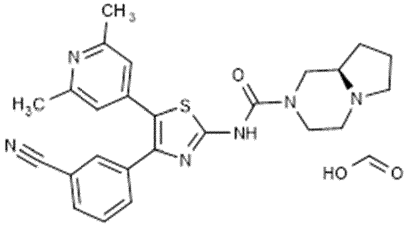 (8aR)-N-[4-(3-Cyanophenyl)-5-(2,6-dimethyl-4-pyridyl)thiazol-2-yl]-3,4,6,7,8,8a-hexahydro-1H-pyrrolo[1,2-a]pyrazine-2-carboxamide formate salt | LC-MS (Method 7B): Rt 2.93 mins; MS m/z 459.4 = [M+H]+ 1H NMR (400 MHz, Methanol-d4) δ 7.91 (t, J = 1.7 Hz, 1H), 7.74 - 7.70 (m, 1H), 7.70 - 7.66 (m, 1H), 7.49 (dd, J = 7.8, 7.8 Hz, 1H), 7.01 (s, 2H), 4.34 (d, J = 12.9 Hz, 1H), 4.20 (d, J = 13.2 Hz, 1H), 3.18 - 3.09 (m, 3H), 2.79 (dd, J = 12.7, 10.7 Hz, 1H), 2.44 (s, 6H), 2.33 - 2.23 (m, 2H), 2.18 - 2.08 (m, 1H), 1.99 - 1.92 (m, 1H), 1.90 - 1.79 (m, 2H), 1.50 (td, J = 11.2, 6.8 Hz, 1H). Exchangeable protons not observed |

### Example 4.12

### N-[4-(3-Cyanophenyl)-5-(2,6-dimethyl-4-pyridyl)thiazol-2-yl]-2-oxa-6-azaspiro[3.3]heptane-6-carboxamide

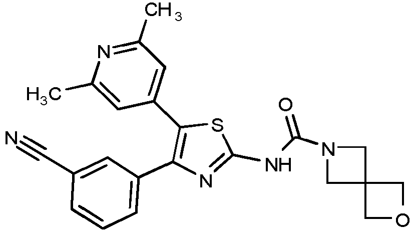

CDI (1.27 g, 7.83 mmol) and DIPEA (2.05 mL, 11.75 mmol) were added to a stirred solution of 3-[2-amino-5-(2,6-dimethyl-4-pyridyl)thiazol-4-yl]benzonitrile (Intermediate C)(1.2 g, 3.92 mmol) in DMA (12 mL) and the solution was heated at 50 °C overnight. 2-Oxa-6-azaspiro[3.3]heptane (0.73 mL, 7.83 mmol) and additional DIPEA (2.05 mL, 11.75 mmol) were added and the solution was stirred at 50 °C for 40 mins. The resulting mixture was allowed to cool to room temperature before being diluted with EtOAc (150 mL), washed with 90% brine (100 mL), 50% brine (3 X 100 mL), dried over MgSO₄ and concentrated *in vacuo.* Purification by chromatography on silica eluting with a gradient of 0 to 4% MeOH in DCM afforded the title compound as an off-white solid.
LC-MS (Method 8B): Rt 2.82 mins; MS m/z 432.0 = [M+H]+
¹H NMR (500 MHz, DMSO-d6) δ 11.35 (s, 1H), 7.88 (t, J = 1.7 Hz, 1H), 7.82 (dt, J = 7.7, 1.4 Hz, 1H), 7.70 (dt, J = 8.0, 1.5 Hz, 1H), 7.56 (t, J = 7.8 Hz, 1H), 6.93 (s, 2H), 4.68 (s, 4H), 4.21 (s, 4H), 2.36 (s, 6H).

### Example 5: (2R)-4-[[5-(2-Chloro-6-methyl-4-pyridyl)-4-(3-cyanophenyl)thiazol-2-yl]carbamoyl]piperazine-2-carboxylic acid

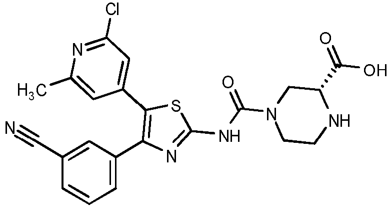

and

### Example 5.1: Methyl (2R)-4-[[5-(2-chloro-6-methyl-4-pyridyl)-4-(3-cyanophenyl)thiazol-2-yl]carbamoyl]piperazine-2-carboxylate

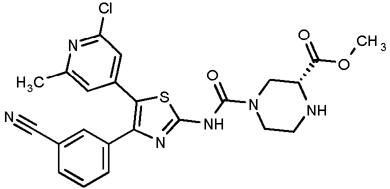

### Step 1: (2R)-1-tert-Butoxycarbonyl-4-[[5-(2-chloro-6-methyl-4-pyridyl)-4-(3-cyanophenyl)thiazol-2-yl]carbamoyl]piperazine-2-carboxylic acid

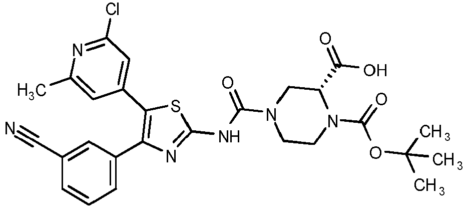

A solution of 3-[2-amino-5-(2-chloro-6-methyl-4-pyridyl)thiazol-4-yl]benzonitrile (Intermediate D) (50 mg, 0.15 mmol) and CDI (50 mg, 0.31 mmol) in DMA (0.5 mL) was stirred at 50 °C for 20 h. The reaction mixture was treated with (2R)-1-*tert*-butoxycarbonylpiperazine-2-carboxylic acid (106 mg, 0.46 mmol) and DIPEA (53 µL, 0.31 mmol) and stirring continued heating at 50 °C for 1 h. After cooling to room temperature, the resulting mixture was diluted with water (2 mL) and extracted with EtOAc (3 x 2 mL). The combined organic extracts were filtered through a phase separating cartridge and concentrated *in vacuo.* The crude product was dissolved in DCM (4 mL) and heptane (10 mL) added resulting in the formation of a precipitate. The solid was collected under vacuum filtration to afford the title compound as a brown solid.
LC-MS (Method 2A): Rt 1.25 mins; MS m/z 583.2/585.2 = [M+H]+
¹H NMR (400 MHz, DMSO-d6) δ 7.91 (t, J = 1.5 Hz, 1H), 7.86 (dt, *J* = 7.7, 1.3 Hz, 1H), 7.72 (dt, *J* = 7.9, 1.3 Hz, 1H), 7.59 (t, *J =* 7.8 Hz, 1H), 7.18 (s, 1H), 7.06 (s, 1H), 4.68 - 4.57 (m, 1H), 4.51 (d, *J* = 30.0 Hz, 2H), 4.17 - 4.08 (m, 2H), 3.77 - 3.69 (m, 2H), 2.40 (s, 3H), 1.39 (d, *J* = 7.0 Hz, 9H). NH and OH protons not observed

### Step 2: (2R)-4-[[5-(2-Chloro-6-methyl-4-pyridyl)-4-(3-cyanophenyl)thiazol-2-yl]carbamoyl]piperazine-2-carboxylic acid and methyl (2R)-4-[[5-(2-chloro-6-methyl-4-pyridyl)-4-(3-cyanophenyl)thiazol-2-yl]carbamoyl]piperazine-2-carboxylate

To a solution of (2R)-1-*tert*-butoxycarbonyl-4-[[5-(2-chloro-6-methyl-4-pyridyl)-4-(3-cyanophenyl)thiazol-2-yl]carbamoyl]piperazine-2-carboxylic acid (step 1)(94 mg, 0.13 mmol) in DCM (2 mL) and MeOH (0.2 mL) was added 4M HCl in 1,4-dioxane (322 µL, 1.29 mmol) and the reaction mixture was stirred at room temperature for 16 h. The resulting mixture was concentrated *in vacuo* and purification of the crude mixture by HPLC using acidic conditions, early elution method afforded the following compounds:

### Example 5: (2R)-4-[[5-(2-Chloro-6-methyl-4-pyridyl)-4-(3-cyanophenyl)thiazol-2-yl]carbamoyl]piperazine-2-carboxylic acid as an off-white solid.

LC-MS (Method 7A): Rt 2.08 mins; MS m/z 483.2/485.1 = [M+H]+ (99% @ 215nm)
¹H NMR (500 MHz, DMSO-d6) δ 7.91 (t, *J* = 1.5 Hz, 1H), 7.85 (dt, *J* = 7.7, 1.3 Hz, 1H), 7.72 (dt, *J* = 7.9, 1.3 Hz, 1H), 7.59 (t, *J =* 7.8 Hz, 1H), 7.17 (s, 1H), 7.05 (s, 1H), 4.37 (dd, *J* = 10.9, 3.2 Hz, 2H), 4.02 (d, *J* = 13.0 Hz, 1H), 3.11 (dd, *J* = 9.5, 6.1 Hz, 1H), 2.92 - 2.85 (m, 1H), 2.40 (s, 3H). 2 x NH and COOH protons not observed. 2 x CH signals under H₂O signal.

### Example 5.1: Methyl (2R)-4-[[5-(2-chloro-6-methyl-4-pyridyl)-4-(3-cyanophenyl)thiazol-2-yl]carbamoyl]piperazine-2-carboxylate as an off-white solid.

LC-MS (Method 7A): Rt 2.10 mins; MS m/z 497.2/499.1 = [M+H]+ (99% @ 215nm)
¹H NMR (500 MHz, DMSO-d6) δ 7.92 (t, *J* = 1.5 Hz, 1H), 7.85 (dt, *J* = 7.7, 1.3 Hz, 1H), 7.72 (dt, *J* = 8.0, 1.4 Hz, 1H), 7.59 (t, *J =* 7.8 Hz, 1H), 7.18 (s, 1H), 7.06 (s, 1H), 4.07 - 4.00 (m, 1H), 3.74 (d, *J =* 12.9 Hz, 1H), 3.66 (s, 3H), 3.46 (dd, *J* = 8.3, 3.5 Hz, 1H), 3.22 - 3.17 (m, 1H), 2.91 (dt, *J* = 11.9, 3.3 Hz, 1H), 2.62 - 2.58 (m, 1H), 2.40 (s, 3H). 2 x NH protons not observed. 1 x CH signal under H₂O signal.

The compounds of the following tabulated Examples (Table Ex5) were prepared analogously to Example 5 from 3-[2-amino-5-(2-chloro-6-methyl-4-pyridyl)thiazol-4-yl]benzonitrile (Intermediate D) and the appropriate amine (step 1) followed by deprotection using 4M HCl in 1,4-dioxane (step 2).

**Table Ex5**

| **Ex.** | **Structure and Name** | **Retention Time, [M+H]+, ¹H NMR** |
|---|---|---|
| 5.2 | 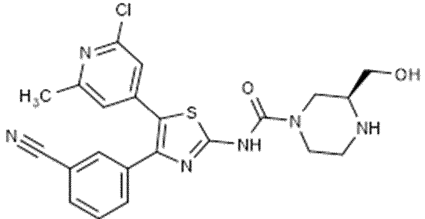 | LC-MS (Method 7A): Rt 1.96 mins; MS m/z 469.2/471.1 = [M+H]+ |
| | | ¹H NMR (400 MHz, DMSO-d6) δ 7.91 (t, *J* = 1.5 Hz, 1H), 7.85 (dt, *J* = 7.7, 1.3 Hz, 1H), 7.72 (dt, *J* = 8.0, 1.3 Hz, 1H), 7.59 (t, *J* = 7.8 Hz, 1H), 7.17 (s, 1H), 7.05 (s, 1H), 4.15 (d, *J* = 10.9 Hz, 1H), 4.07 (d, *J* = 12.7 Hz, 1H), 2.91 (dd, *J* = 26.9, 12.8 Hz, 2H), 2.66 - 2.55 (m, 3H), 2.40 (s, 3H). 2 x NH and OH protons not observed. 1 x CH₂ signal under H₂O signal. |
| | (3S)-N-[5-(2-Chloro-6-methyl-4-pyridyl)-4-(3-cyanophenyl)thiazol-2-yl]-3-(hydroxymethyl)piperazine-1-carboxamide | |
| | | Intermediate Data: |
| | Prepared via: | LC-MS (Method 7A): Rt 3.60 mins; MS m/z 569.2 = [M+H]+ |
| | 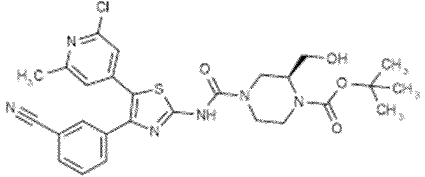 | ¹H NMR (400 MHz, DMSO-d6) δ 11.32 (s, 1H), 7.91 (t, *J* = 1.3 Hz, 1H), 7.86 (dt, *J* = 7.7, 1.2 Hz, 1H), 7.72 (dt, *J* = 7.9, 1.3 Hz, 1H), 7.59 (t, *J* = 7.8 Hz, 1H), 7.19 (s, 1H), 7.07 (s, 1H), 4.87 (s, 1H), 4.21 (d, *J* = 12.8 Hz, 1H), 4.08 - 3.94 (m, 2H), 3.76 (d, *J* = 11.6 Hz, 1H), 3.45 - 3.36 (m, 2H), 3.12 (dd, *J* = 13.3, 3.5 Hz, 1H), 3.05 - 2.97 (m, 2H), 2.40 (s, 3H), 1.42 (s, 9H). |
| | *tert-Butyl* (2S)-4-[[5-(2-chloro-6-methyl-4-pyridyl)-4-(3-cyanophenyl)thiazol-2-yl]carbamoyl]-2-(hydroxymethyl)piperazine-1-carboxylate | |
| 5.3 | 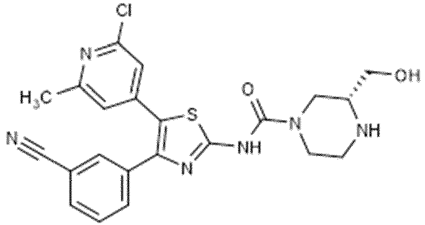 | LC-MS (Method 7A): Rt 1.92 mins; MS m/z 469.2/471.1 = [M+H]+ |
| | | ¹H NMR (400 MHz, DMSO-d6) δ 7.92 (t, *J =* 1.5 Hz, 1H), 7.86 (dt, *J* = 7.7, 1.4 Hz, 1H), 7.73 (dt, *J* = 7.9, 1.3 Hz, 1H), 7.60 (t, *J* = 7.8 Hz, 1H), 7.18 (s, 1H), 7.06 (s, 1H), 4.15 (d, *J =* 10.5 Hz, 1H), 4.07 (d, *J* = 12.2 Hz, 1H), 2.91 (dd, *J* = 26.5, 12.2 Hz, 2H), 2.66 - 2.57 (m, 3H), 2.41 (s, 3H). 2 x NH and OH protons not observed. 1 x CH₂ signal under H₂O signal. |
| | (3R)-N-[5-(2-Chloro-6-methyl-4-pyridyl)-4-(3-cyanophenyl)thiazol-2-yl]-3-(hydroxymethyl)piperazine-1-carboxamide | |
| | | Intermediate Data: |
| | Prepared via: | LC-MS (Method 7A): Rt 3.60 mins; MS m/z 569.2 = [M+H]+ |
| | 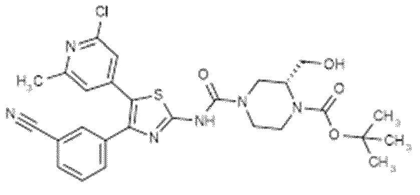 | |
| | | ¹H NMR (400 MHz, DMSO-d6) δ 7.91 (t, *J =* 1.4 Hz, 1H), 7.86 (dt, *J* = 7.7, 1.3 Hz, 1H), 7.72 (dt, *J* = 8.0, 1.3 Hz, 1H), 7.59 (t, *J* = 7.8 Hz, 1H), 7.18 (s, 1H), 7.06 (s, 1H), 4.22 (d, *J =* 13.0 Hz, 1H), 4.08 - 3.95 (m, 2H), 3.79 - 3.72 (m, 1H), 3.46 - 3.37 (m, 2H), 3.11 (dd, *J* = 13.7, 3.7 Hz, 1H), 3.00 (t, *J =* 8.4 Hz, 2H), 2.40 (s, 3H), 1.41 (s, 9H). NH and OH protons not observed. |
| | *tert*-Butyl (2R)-4-[5-(2-chloro-6-methyl-4-pyridyl)-4-(3-cyanophenyl)thiazol-2-yl]carbamoyl]-2-(hydroxymethyl)piperazine-1-carboxylate | |
| 5.4 | 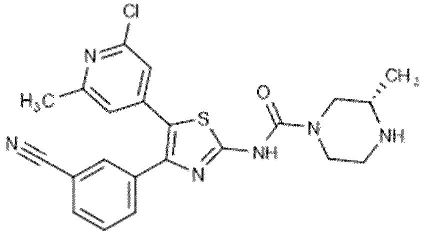 | LC-MS (Method 7A): Rt 2.02 mins; MS m/z 453.2/455.2 = [M+H]+ |
| | | ¹H NMR (500 MHz, DMSO-d6) δ 7.91 (t, *J =* 1.4 Hz, 1H), 7.85 (dt, *J* = 7.7, 1.3 Hz, 1H), 7.72 (dt, *J* = 7.9, 1.4 Hz, 1H), 7.59 (t, *J =* 7.8 Hz, 1H), 7.17 (s, 1H), 7.05 (s, 1H), 4.09 (t, *J =* 10.9 Hz, 2H), 2.92 (d, *J* = 11.9 Hz, 1H), 2.87 - 2.80 (m, 1H), 2.71 - 2.64 (m, 2H), 2.53 - 2.52 (m, 1H), 2.40 (s, 3H), 1.00 (d, *J* = 6.3 Hz, 3H). 2 x NH protons not observed |
| | (3S)-N-[5-(2-Chloro-6-methyl-4-pyridyl)-4-(3-cyanophenyl)thiazol-2-yl]-3-methylpiperazine-1-carboxamide | |
| | | Intermediate Data: |
| | Prepared via: | LC-MS (Method 2A): Rt 1.39 mins; MS m/z 553.1/555.0 = [M+H]+ |
| | 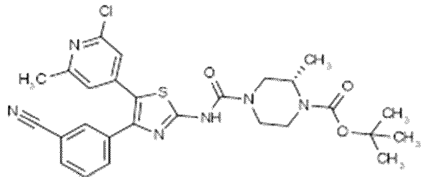 | ¹H NMR (500 MHz, DMSO-d6) δ 7.92 (t, *J =* 1.5 Hz, 1H), 7.86 (dt, *J* = 7.7, 1.4 Hz, 1H), 7.73 (dt, *J* = 7.9, 1.3 Hz, 1H), 7.59 (t, *J =* 7.8 Hz, 1H), 7.19 (s, 1H), 7.06 (s, 1H), 4.19 - 4.10 (m, 2H), 4.03 (dd, *J* = 10.2, 2.9 Hz, 1H), 3.74 (dt, *J* = 12.9, 2.5 Hz, 1H), 3.17 - 3.11 (m, 1H), 3.08 - 3.01 (m, 1H), 2.98 - 2.91 (m, 1H), 2.40 (s, 3H), 1.41 (s, 9H), 1.07 (d, *J* = 6.7 Hz, 3H). NH proton not observed. |
| | *tert*-Butyl (2S)-4-[[5-(2-chloro-6-methyl-4-pyridyl)-4-(3-cyanophenyl)thiazol-2-yl]carbamoyl]-2-methyl-piperazine-1-carboxylate | |
| 5.5 | 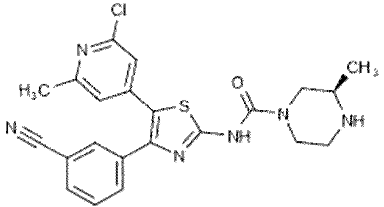 | LC-MS (Method 7A): Rt 2.03 mins; MS m/z 453.2/455.2 = [M+H]+ |
| | | 1H NMR (500 MHz, DMSO-d6) δ 7.92 (t, *J =* 1.5 Hz, 1H), 7.86 (dt, *J* = 7.7, 1.4 Hz, 1H), 7.72 (dt, *J* = 7.9, 1.2 Hz, 1H), 7.59 (t, *J =* 7.8 Hz, 1H), 7.19 - 7.17 (m, 1H), 7.06 (s, 1H), 4.15 (t, *J* = 11.0 Hz, 2H), 3.08 (d, *J* = 12.4 Hz, 1H), 3.02 - 2.97 (m, 1H), 2.93 - 2.89 (m, 1H), 2.82 - 2.77 (m, 1H), 2.74 - 2.68 (m, 1H), 2.40 (s, 3H), 1.09 (d, *J* = 6.4 Hz, 3H). 2 x NH protons not observed |
| | (3R)-N-[5-(2-Chloro-6-methyl-4-pyridyl)-4-(3-cyanophenyl)thiazol-2-yl]-3-methylpiperazine-1-carboxamide | |
| | Prepared via: | Intermediate Data: |
| | 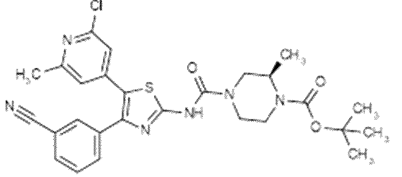 | LC-MS (Method 2A): Rt 1.40 mins; MS m/z 553.1/555.0 = [M+H]+ |
| | | ¹H NMR (500 MHz, DMSO-d6) δ 11.41 (br s, 1H), 7.92 (t, *J* = 1.4 Hz, 1H), 7.86 (dt, *J* = 7.7, 1.4 Hz, 1H), 7.73 (dt, *J* = 7.9, 1.4 Hz, 1H), 7.59 (t, *J =* 7.8 Hz, 1H), 7.19 (s, 1H), 7.07 (s, 1H), 4.18 - 4.10 (m, 2H), 4.02 (d, *J =* 13.4 Hz, 1H), 3.74 (dq, *J* = 12.9, 2.5, 0.2 Hz, 1H), 3.17 - 3.12 (m, 1H), 3.05 (t, *J* = 11.5 Hz, 1H), 2.95 (t, *J =* 12.1 Hz, 1H), 2.40 (s, 3H), 1.41 (s, 9H), 1.07 (d, *J* = 6.7 Hz, 3H). |
| | *tert*-Butyl (2R)-4-[[5-(2-chloro-6-methyl-4-pyridyl)-4-(3-cyanophenyl)thiazol-2-yl]carbamoyl]-2-methyl-piperazine-1-carboxylate | |
| 5.6 | 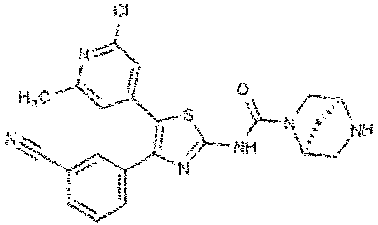 | LC-MS (Method 7A): Rt 1.99 mins; MS m/z 451.2/453.2 = [M+H]+ |
| | | ¹H NMR (500 MHz, Methanol-d4) δ 7.93 (t, *J =* 1.4 Hz, 1H), 7.76 - 7.68 (m, 2H), 7.52 (t, *J* = 7.8 Hz, 1H), 7.15 (s, 1H), 7.11 (s, 1H), 4.82 (s, 1H), 4.24 (s, 1H), 3.67 (d, *J* = 10.2 Hz, 1H), 3.59 (d, *J* = 10.5 Hz, 1H), 3.28 (s, 1H), 3.23 (d, *J* = 10.8 Hz, 1H), 2.45 (s, 3H), 2.11 (d, *J* = 10.6 Hz, 1H), 1.95 (d, *J* = 10.7 Hz, 1H). 2 x NH protons not observed. |
| | (1S,4S)-N-[5-(2-Chloro-6-methyl-4-pyridyl)-4-(3-cyanophenyl)thiazol-2-yl]-2,5-diazabicyclo[2.2.1]heptane-2-carboxamide | |
| | | Intermediate Data: |
| | | LC-MS (Method 7A): Rt 3.85 mins; MS m/z 551.2 = [M+H]+ |
| | Prepared via: | |
| | 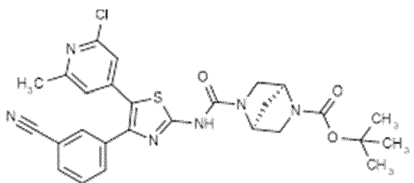 | ¹H NMR (400 MHz, DMSO-d6) δ 11.00 (br s, 1H), 7.91 (t, *J =* 1.3 Hz, 1H), 7.85 (dt, *J* = 7.7, 1.3 Hz, 1H), 7.72 (dt, *J* = 7.9, 1.2 Hz, 1H), 7.59 (t, *J =* 7.8 Hz, 1H), 7.18 (s, 1H), 7.06 (s, 1H), 4.98 - 4.58 (m, 1H), 4.42 (d, *J* = 19.1 Hz, 1H), 3.55 - 3.36 (m, 4H), 3.24 (d, *J* = 10.0 Hz, 2H), 2.40 (s, 3H), 1.39 (s, 9H). |
| | *tert*-Butyl (1S,4S)-5-[[5-(2-chloro-6-methyl-4-pyridyl)-4-(3-cyanophenyl)thiazol-2-yl]carbamoyl]-2,5-diazabicyclo[2.2.1]heptane-2-carboxylate | |
| 5.7 | 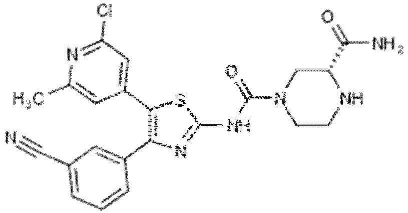 | LC-MS (Method 7B): Rt 2.11 mins; MS m/z 482.2/484.2 = [M+H]+ |
| | | ¹H NMR (400 MHz, DMSO-d6) δ 11.28 (s, 1H), 7.92 (t, *J* = 1.5 Hz, 1H), 7.85 (dt, *J* = 7.7, 1.3 Hz, 1H), 7.72 (dt, *J* = 7.9, 1.2 Hz, 1H), 7.59 (t, *J* = 7.8 Hz, 1H), 7.34 (s, 1H), 7.19 (s, 1H), 7.18 (s, 1H), 7.06 (s, 1H), 4.08 (dd, *J* = 12.9, 2.2 Hz, 1H), 3.85 (d, *J* = 13.3 Hz, 1H), 3.20 (dd, *J* = 9.2, 3.2 Hz, 1H), 3.08 - 2.99 (m, 2H), 2.90 (dt, *J* = 12.9, 3.2 Hz, 1H), 2.66 - 2.59 (m, 1H), 2.40 (s, 3H). NH proton not observed. |
| | (3R)-N 1-[5-(2-Chloro-6-methyl-4-pyridyl)-4-(3-cyanophenyl)thiazol-2-yl]piperazine-1,3-dicarboxamide | |
| | Prepared via: | |
| | 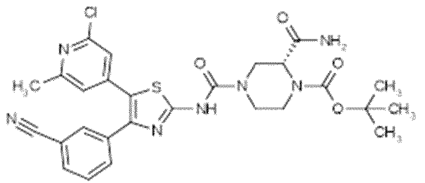 | Intermediate Data: |
| | | LC-MS (Method 2A): Rt 1.19 mins; MS m/z 582.2/584.2 = [M+H]+ |
| | | ¹H NMR (400 MHz, DMSO-d6) δ 11.31 (s, 1H), 7.91 (s, 1H), 7.86 (d, *J* = 7.8 Hz, 1H), 7.72 (d, *J* = 8.1 Hz, 1H), 7.59 (t, *J =* 7.8 Hz, 1H), 7.46 (s, 1H), 7.19 (s, 1H), 7.13 (s, 1H), 7.07 (s, 1H), 4.50 - 4.35 (m, 3H), 4.07 - 3.99 (m, 1H), 3.74 - 3.68 (m, 1H), 3.43 - 3.35 (m, 2H), 2.40 (s, 3H), 1.39 (s, 9H). |
| | *tert*-Butyl (2R)-2-carbamoyl-4-[[5-(2-chloro-6-methyl-4-pyridyl)-4-(3-cyanophenyl)thiazol-2-yl]carbamoyl]piperazine-1-carboxylate | |
| 5.8 | 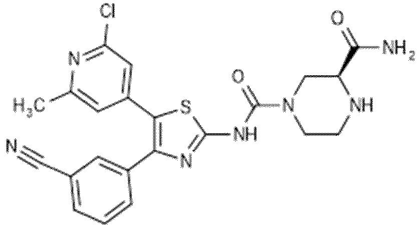 | LC-MS (Method 7B): Rt 2.12 mins; MS m/z 482.2/484.2 = [M+H]+ |
| | | ¹H NMR (400 MHz, DMSO-d6) δ 11.29 (s, 1H), 7.92 (t, *J =* 1.5 Hz, 1H), 7.85 (dt, *J* = 7.7, 1.3 Hz, 1H), 7.72 (dt, *J* = 7.9, 1.2 Hz, 1H), 7.59 (t, *J =* 7.9 Hz, 1H), 7.34 (s, 1H), 7.21 - 7.17 (m, 2H), 7.06 (s, 1H), 4.08 (dd, *J* = 13.2, 2.4 Hz, 1H), 3.85 (d, *J =* 12.5 Hz, 1H), 3.21 (dd, *J =* 9.3, 3.2 Hz, 1H), 3.10 - 2.98 (m, 2H), 2.90 (dt, *J* = 12.4, 2.9 Hz, 1H), 2.66 - 2.59 (m, 1H), 2.40 (s, 3H). NH proton not observed. |
| | (3S)-N 1-[5-(2-Chloro-6-methyl-4-pyridyl)-4-(3-cyanophenyl)thiazol-2-yl]piperazine-1,3-dicarboxamide | |
| | Prepared via: | |
| | 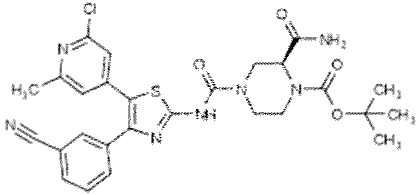 | Intermediate Data: |
| | | LC-MS (Method 7A): Rt 3.37 mins; MS m/z 582.2/584.2 = [M+H]+ |
| | | ¹H NMR (400 MHz, DMSO-d6) δ 11.31 (s, 1H), 7.94 - 7.89 (m, 1H), 7.86 (d, *J* = 7.7 Hz, 1H), 7.72 (dt, *J* = 7.9, 1.3 Hz, 1H), 7.59 (t, *J* = 7.8 Hz, 1H), 7.46 (s, 1H), 7.19 (s, 1H), 7.13 (s, 1H), 7.07 (s, 1H), 4.56 - 4.34 (m, 3H), 4.08 - 3.99 (m, 1H), 3.76 - 3.66 (m, 1H), 3.42 - 3.34 (m, 2H), 2.40 (s, 3H), 1.38 (s, 9H). |
| | *tert*-Butyl (2S)-2-carbamoyl-4-[[5-(2-chloro-6-methyl-4-pyridyl)-4-(3-cyanophenyl)thiazol-2-yl]carbamoyl]piperazine-1-carboxylate | |
| 5.9 | 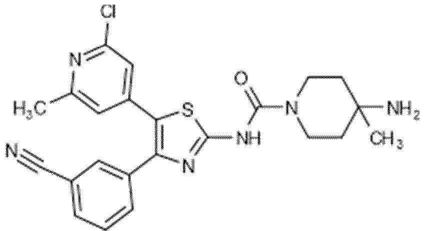 | LC-MS (Method 7A): Rt 2.08 mins; MS m/z 467.2/469.1 = [M+H]+ |
| | | ¹H NMR (500 MHz, DMSO-d6) δ 7.90 (t, *J =* 1.5 Hz, 1H), 7.84 (dt, *J* = 7.7, 1.3 Hz, 1H), 7.72 (dt, *J* = 7.9, 1.3 Hz, 1H), 7.58 (t, *J = 7.8* Hz, 1H), 7.12 (s, 1H), 6.99 (s, 1H), 3.81 - 3.72 (m, 2H), 3.42 - 3.37 (m, 2H), 2.38 (s, 3H), 1.53 (t, *J* = 5.4 Hz, 4H), 1.22 (s, 3H). 3 x NH protons not observed. 1 x CH₂ signal under H₂O signal. |
| | 4-Amino-N-[5-(2-chloro-6-methyl-4-pyridyl)-4-(3-cyanophenyl)thiazol-2-yl]-4-methyl-piperidine-1-carboxamide | |
| | | Intermediate Data: |
| | Prepared via: | LC-MS (Method 2A): Rt 1.38 mins; MS m/z 567.2/569.2 = [M+H]+ |
| | 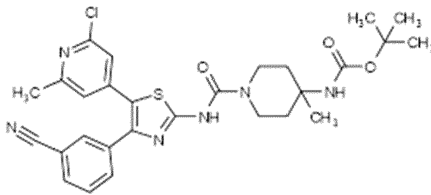 | |
| | | ¹H NMR (400 MHz, DMSO-d6) δ 11.25 (s, 1H), 7.92 (s, 1H), 7.85 (d, *J =* 7.8 Hz, 1H), 7.72 (d, *J =* 8.0 Hz, 1H), 7.59 (t, *J = 7.8* Hz, 1H), 7.18 (s, 1H), 7.06 (s, 1H), 6.59 (s, 1H), 3.77 (d, *J =* 13.6 Hz, 2H), 3.20 (t, *J* = 11.5 Hz, 2H), 2.40 (s, 3H), 2.05 (d, *J* = 13.3 Hz, 2H), 1.39 (s, 9H), 1.38 - 1.33 (m, 2H), 1.23 (s, 3H). |
| | *tert*-Butyl N-[1-[[5-(2-chloro-6-methyl-4-pyridyl)-4-(3-cyanophenyl)thiazol-2-yl]carbamoyl]-4-methyl-4-piperidyl]carbamate | |

### Example 6

### 4-Cyano-N-[4-(3-cyanophenyl)-5-(2,6-dimethyl-4-pyridyl)thiazol-2-yl]-4-methylpiperidine-1-carboxamide

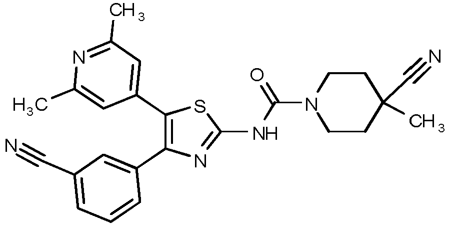

A solution of 3-[2-amino-5-(2,6-dimethyl-4-pyridyl)thiazol-4-yl]benzonitrile (Intermediate C) (50 mg, 0.16 mmol) and CDI (40 mg, 0.24 mmol) in DMA (0.5 mL) was stirred at 50°C for 18 h. The resulting mixture was treated with 4-methylpiperidine-4-carbonitrile hydrochloride (52 mg, 0.33 mmol) and DIPEA (85 µL, 0.49 mmol) and stirring continued at 50°C for 1 h. After cooling to room temperature, the crude mixture was purified by preparative HPLC using acidic conditions, early elution method to afford to afford the title compound as a colorless solid.

### Example 6 was also prepared using the following procedure:

CDI (1.27 g, 7.83mmol) and DIPEA (2.05 mL, 11.75 mmol) were added to a stirred solution of 3-[2-amino-5-(2,6-dimethyl-4-pyridyl)thiazol-4-yl]benzonitrile (Intermediate C) (1.20 g, 3.92 mmol) in DMA (12 mL) and the solution was heated at 50 °C overnight. 4-Methylpiperidine-4-carbonitrile hydrochloride (1.26 g, 7.83 mmol) and additional DIPEA (2.05 mL, 11.75 mmol) were added and the solution was stirred at 50 °C for 30 mins. The resulting mixture was cooled to room temperature before being diluted with EtOAc (150 mL), washed with 90% aqueous brine (100 mL), 50% aqueous brine (3 x 100 mL) dried over MgSO₄ and concentrated *in vacuo.* Purification by chromatography on silica eluting with a gradient of 1 to 3% MeOH in DCM afforded a yellow solid. Addition of MeOH (15 mL) afforded a white solid which was collected by filtration, washed with MeOH (2 mL), Et₂O (20 mL) and dried to afford the title compound as a colorless solid.
LC-MS (Method 8B): Rt 3.24 mins; MS m/z 457.2 = [M+H]+
1H NMR (500 MHz, DMSO-d6) δ 11.31 (s, 1H), 7.89 (t, J = 1.8 Hz, 1H), 7.82 (dt, J = 7.7, 1.4 Hz, 1H), 7.70 (dt, J = 8.0, 1.5 Hz, 1H), 7.56 (t, J = 7.9 Hz, 1H), 6.93 (s, 2H), 4.22 (d, J = 14.0 Hz, 2H), 3.03 (t, J = 12.6 Hz, 2H), 2.36 (s, 6H), 1.92 (d, J = 13.4 Hz, 2H), 1.53 (td, J = 13.2, 4.0 Hz, 2H), 1.37 (s, 3H).

The compounds of the following tabulated Examples (Table Ex6) were prepared analogously to Example 6 from either 3-[2-amino-5-(2,6-dimethyl-4-pyridyl)thiazol-4-yl]benzonitrile (Intermediate C) or the appropriate starting Intermediate and the appropriate amine.

**Table Ex6**

| **Ex.** | **Structure and Name** | **Retention Time, [M+H]+, ¹H NMR** |
|---|---|---|
| 6.1 | 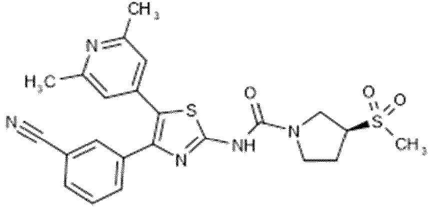 (3S)-N-[4-(3-Cyanophenyl)-5-(2,6-dimethyl-4-pyridyl)thiazol-2-yl]-3-methylsulfonyl-pyrrolidine-1-carboxamide | LC-MS (Method 8B): Rt 3.69 mins; |
| | | MS m/z 482.2 = [M+H]+ |
| | | 1H NMR (500 MHz, DMSO-d6) δ 11.20 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 6.93 (s, 2H), 4.07 - 3.97 (m, 1H), 3.93 - 3.84 (m, 1H), 3.84 - 3.76 (m, 1H), 3.66 - 3.60 (m, 1H), 3.60 - 3.50 (m, 1H), 3.07 (s, 3H), 2.40 - 2.28 (m, 8H). |
| 6.2 | 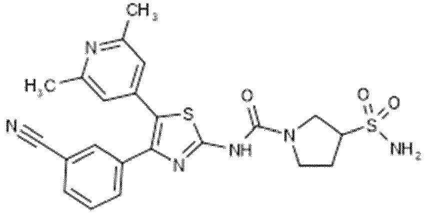 N-[4-(3-Cyanophenyl)-5-(2,6-dimethyl-4-pyridyl)thiazol-2-yl]-3-sulfamoyl-pyrrolidine-1-carboxamide | LC-MS (Method 8B): Rt 3.57 mins; |
| | | MS m/z 483.2 = [M+H]+ |
| | | 1H NMR (500 MHz, DMSO-d6) δ 11.17 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 7.09 (s, 2H), 6.93 (s, 2H), 3.85 - 3.74 (m, 3H), 3.67 - 3.60 (m, 1H), 3.57 - 3.50 (m, 1H), 2.36 (s, 6H), 2.33 - 2.25 (m, 2H). |
| 6.3 | 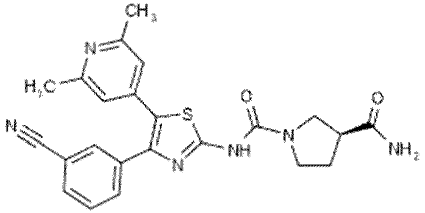 (3S)-N1-[4-(3-Cyanophenyl)-5-(2,6-dimethyl-4-pyridyl)thiazol-2-yl]pyrrolidine-1,3-dicarboxamide | LC-MS (Method 8B): Rt 3.51 mins; |
| | | MS m/z 447.3 = [M+H]+ |
| | | 1H NMR (500 MHz, DMSO-d6) δ 11.05 (s, 1H), 7.89 (s, 1H), 7.81 (d,J= 7.8 Hz, 1H), 7.71 (d,J= 7.8 Hz, 1H), 7.56 (t,J= 7.8 Hz, 1H), 7.49 (s, 1H), 6.99 (s, 1H), 6.92 (s, 2H), 3.64 - 3.49 (m, 3H), 3.00 - 2.91 (m, 1H), 2.36 (s, 6H), 2.13 - 1.92 (m, 3H). |
| 6.4 | 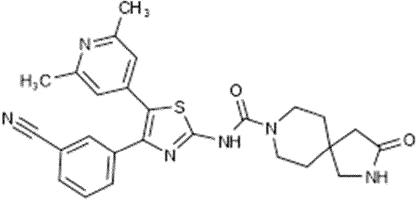 N-[4-(3-Cyanophenyl)-5-(2,6-dimethyl-4-pyridyl)thiazol-2-yl]-3-oxo-2,8-diazaspiro[4.5]decane-8-carboxamide | LC-MS (Method 8B): Rt 3.84 mins; |
| | | MS m/z 487.3 = [M+H]+ |
| | | 1H NMR (500 MHz, DMSO-d6) δ 11.24 (s, 1H), 7.89 (s, 1H), 7.82 (d, J= 7.6 Hz, 1H), 7.70 (d, J= 8.0 Hz, 1H), 7.60 - 7.51 (m, 2H), 6.92 (s, 2H), 3.66 - 3.59 (m, 2H), 3.45 - 3.40 (m, 2H), 3.07 (s, 2H), 2.36 (s, 6H), 2.11 (s, 2H), 1.54 (t, J= 5.7 Hz, 4H). |
| 6.5 | 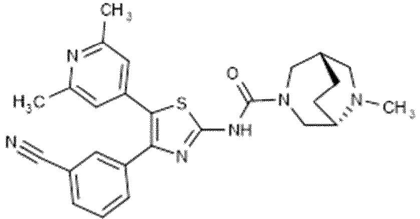 (1S,5S)-N-[4-(3-Cyanophenyl)-5-(2,6-dimethyl-4-pyridyl)thiazol-2-yl]-6-methyl-3,6-diazabicyclo[3.2.2]nonane-3-carboxamide | LC-MS (Method 7A): Rt 1.00 mins; |
| | | MS m/z 473.3 = [M+H]+ |
| | | 1H NMR (500 MHz, DMSO-d6) δ 7.89 (t, J = 1.7 Hz, 1H), 7.81 (dt, J = 7.7, 1.4 Hz, 1H), 7.70 (dt, J = 7.9, 1.4 Hz, 1H), 7.56 (dd, J = 7.8, 7.8 Hz, 1H), 6.92 (s, 2H), 4.13 (dd, J = 13.9, 6.1 Hz, 1H), 3.99 - 3.92 (m, 1H), 3.43 - 3.37 (m, 2H), 2.84 (t, J = 5.3 Hz, 1H), 2.75 - 2.68 (m, 2H), 2.36 (s, 6H), 2.31 (s, 3H), 2.14 - 2.10 (m, 1H), 1.91 - 1.85 (m, 1H), 1.58 - 1.48 (m, 3H). NH proton not observed |
| 6.6 | 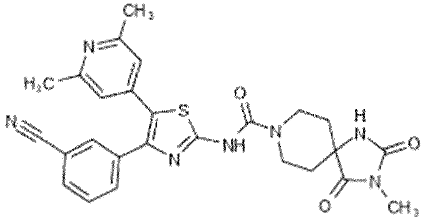 N-[4-(3-Cyanophenyl)-5-(2,6-dimethyl-4-pyridyl)thiazol-2-yl]-3-methyl-2,4-dioxo-1,3,8-triazaspiro[4.5]decane-8-carboxamide | LC-MS (Method 8B): Rt 3.37 mins; |
| | | MS m/z 516.3 = [M+H]+ |
| | | 1H NMR (500 MHz, Methanol-d4) δ 7.93 (s, 1H), 7.76 - 7.68 (m, 2H), 7.51 (t, J= 7.9 Hz, 1H), 7.02 (s, 2H), 4.13 (dt, J= 14.0, 4.9 Hz, 2H), 3.51 (ddd, J= 13.7, 9.9, 3.3 Hz, 2H), 2.99 (s, 3H), 2.45 (s, 6H), 2.04 (ddd, J= 13.9, 9.9, 4.2 Hz, 2H), 1.76 (dt, J= 13.9, 4.3 Hz, 2H). 2 x NH protons not observed. |
| 6.7 | 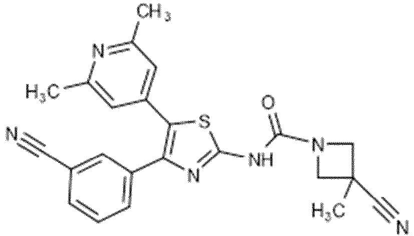 3-Cyano-N-[4-(3-cyanophenyl)-5-(2,6-dimethyl-4-pyridyl)thiazol-2-yl]-3-methyl-azetidine-1-carboxamide | LC-MS (Method 8B): Rt 3.54 mins; |
| | | MS m/z 429.3 = [M+H]+ |
| | | 1H NMR (500 MHz, DMSO-d6) δ 11.54 (s, 1H), 7.87 (s, 1H), 7.82 (d,J= 7.8 Hz, 1H), 7.70 (d,J= 7.8 Hz, 1H), 7.56 (t,J= 7.8 Hz, 1H), 6.93 (s, 2H), 4.34 (d,J= 8.8 Hz, 2H), 4.01 (d,J= 8.8 Hz, 2H), 2.36 (s, 6H), 1.63 (s, 3H). |
| 6.8 | 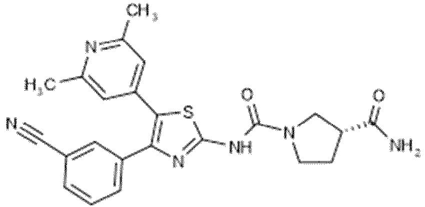 (3R)-N 1-[4-(3-Cyanophenyl)-5-(2,6-dimethyl-4-pyridyl)thiazol-2-yl]pyrrolidine-1,3-dicarboxamide | LC-MS (Method 8B): Rt 3.51 mins; |
| | | MS m/z 447.3 = [M+H]+ |
| | | 1H NMR (500 MHz, DMSO-d6) δ 11.05 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 7.49 (s, 1H), 6.99 (s, 1H), 6.93 (s, 2H), 3.64 - 3.52 (m, 3H), 2.99 - 2.92 (m, 1H), 2.36 (s, 6H), 2.14 - 1.92 (m, 3H). |
| 6.9 | 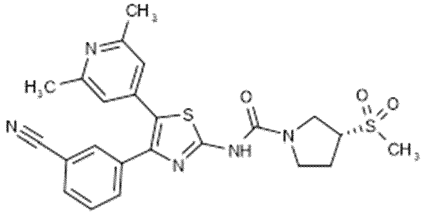 (3R)-N-[4-(3-Cyanophenyl)-5-(2,6-dimethyl-4-pyridyl)thiazol-2-yl]-3-methylsulfonyl-pyrrolidine-1-carboxamide | LC-MS (Method 8B): Rt 3.70 mins; |
| | | MS m/z 482.2 = [M+H]+ |
| | | 1H NMR (500 MHz, DMSO-d6) δ 11.20 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 6.93 (s, 2H), 4.06 - 3.98 (m, 1H), 3.93 - 3.85 (m, 1H), 3.85 - 3.76 (m, 1H), 3.67 - 3.60 (m, 1H), 3.60 - 3.51 (m, 1H), 3.07 (s, 3H), 2.41 - 2.26 (m, 8H). |
| 6.10 | 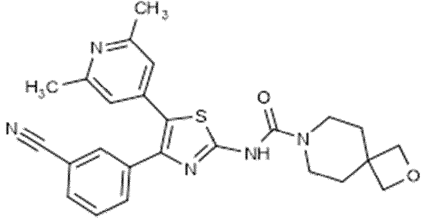 N-[4-(3-Cyanophenyl)-5-(2,6-dimethyl-4-pyridyl)thiazol-2-yl]-2-oxa-7-azaspiro[3.5]nonane-7-carboxamide | LC-MS (Method 8B): Rt 4.15 mins; |
| | | MS m/z 460.3 = [M+H]+ |
| | | 1H NMR (500 MHz, DMSO-d6) δ 11.26 (s, 1H), 7.89 (s, 1H), 7.83 (d, J= 7.7 Hz, 1H), 7.71 (d, J= 7.9 Hz, 1H), 7.56 (t, J= 7.8 Hz, 1H), 6.93 (s, 2H), 4.34 (s, 4H), 3.46 (t, J= 5.6 Hz, 4H), 2.36 (s, 6H), 1.79 (t, J= 5.6 Hz, 4H). |
| 6.11 | 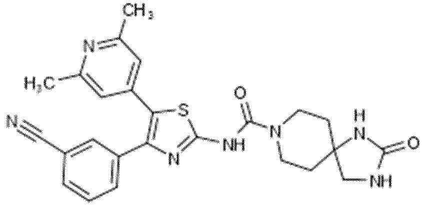 N-[4-(3-Cyanophenyl)-5-(2,6-dimethyl-4-pyridyl)thiazol-2-yl]-2-oxo-1,3,8-triazaspiro[4.5]decane-8-carboxamide | LC-MS (Method 8B): Rt 3.63 mins; |
| | | MS m/z 488.3 = [M+H]+ |
| | | 1H NMR (500 MHz, DMSO-d6) δ 11.28 (s, 1H), 7.89 (s, 1H), 7.82 (d, J= 7.7 Hz, 1H), 7.71 (d, J= 8.0 Hz, 1H), 7.57 (t, J= 7.8 Hz, 1H), 6.93 (s, 2H), 6.78 (s, 1H), 6.22 (s, 1H), 3.65 - 3.51 (m, 4H), 3.14 (s, 2H), 2.36 (s, 6H), 1.66 - 1.53 (m, 4H). |
| 6.12 | 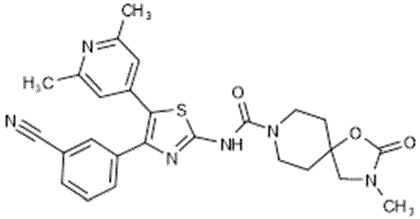 N-[4-(3-Cyanophenyl)-5-(2,6-dimethyl-4-pyridyl)thiazol-2-yl]-3-methyl-2-oxo-1-oxa-3,8-diazaspiro[4.5]decane-8-carboxamide | LC-MS (Method 8B): Rt 3.93 mins; |
| | | MS m/z 503.3 = [M+H]+ |
| | | 1H NMR (500 MHz, Methanol-d4) δ 7.93 (s, 1H), 7.76 - 7.68 (m, 2H), 7.51 (t, J = 7.8 Hz, 1H), 7.03 (s, 2H), 4.04 - 3.96 (m, 2H), 3.55 - 3.45 (m, 2H), 3.45 (s, 2H), 2.90 (s, 3H), 2.46 (s, 6H), 2.03 - 1.96 (m, 2H), 1.94 - 1.84 (m, 2H). NH proton not observed. |
| 6.13 | 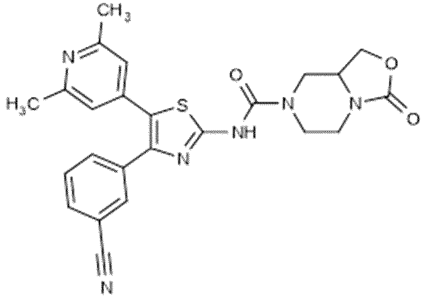 N-[4-(3-Cyanophenyl)-5-(2,6-dimethyl-4-pyridyl)thiazol-2-yl]-3-oxo-5,6,8,8a-tetrahydro-1H-oxazolo[3,4-a]pyrazine-7-carboxamide | LC-MS (Method 8B): Rt 3.69 mins; |
| | | MS m/z 475.3 = [M+H]+ |
| | | 1H NMR (500 MHz, DMSO-d6) δ 11.40 (s, 1H), 7.89 (d, J= 1.8 Hz, 1H), 7.83 (d, J= 7.7 Hz, 1H), 7.71 (d, J= 8.0 Hz, 1H), 7.57 (t, J= 7.8 Hz, 1H), 6.94 (s, 2H), 4.41 (t, J= 8.5 Hz, 1H), 4.36 (d, J= 13.3 Hz, 1H), 4.24 (d, J= 13.3 Hz, 1H), 4.00 (dd, J= 8.9, 5.6 Hz, 1H), 3.89 - 3.83 (m, 1H), 3.62 (dd, J= 13.5, 3.4 Hz, 1H), 3.08 - 2.99 (m, 1H), 2.89 (dt, J= 25.1, 12.7 Hz, 2H), 2.37 (s, 6H). |
| 6.14 | 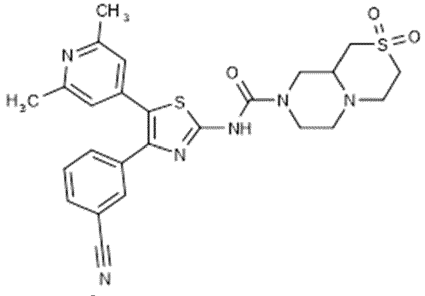 N-[4-(3-Cyanophenyl)-5-(2,6-dimethyl-4-pyridyl)thiazol-2-yl]-2,2-dioxo-3,4,6,7,9,9a-hexahydro-1H-pyrazino[2,1-c][1,4]thiazine-8-carboxamide | LC-MS (Method 8B): Rt 3.58 mins; |
| | | MS m/z 523.2 = [M+H]+ |
| | | 1H NMR (500 MHz, DMSO-d6) δ 11.31 (s, 1H), 7.89 (s, 1H), 7.83 (d, J= 7.7 Hz, 1H), 7.71 (d, J= 8.2 Hz, 1H), 7.57 (t, J= 7.8 Hz, 1H), 6.94 (s, 2H), 4.11 (dd, J= 21.0, 13.4 Hz, 2H), 3.24 (td, J= 13.3, 4.0 Hz, 1H), 3.19 - 3.11 (m, 3H), 3.02 (dt, J= 24.7, 12.7 Hz, 2H), 2.90 (d, J= 11.7 Hz, 1H), 2.83 - 2.74 (m, 1H), 2.69 - 2.61 (m, 2H), 2.37 (s, 6H), 2.22 (td, J= 11.6, 2.9 Hz, 1H). |
| 6.15 | 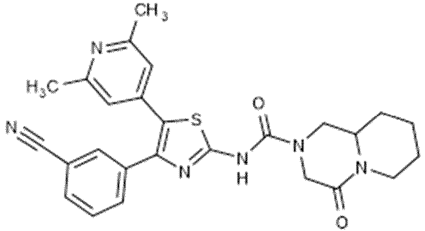 N-[4-(3-Cyanophenyl)-5-(2,6-dimethyl-4-pyridyl)thiazol-2-yl]-4-oxo-3,6,7,8,9,9a-hexahydro-1H-pyrido[1,2-a]pyrazine-2-carboxamide | LC-MS (Method 8B): Rt 3.95 mins; |
| | | MS m/z 487.3 = [M+H]+ |
| | | 1H NMR (500 MHz, DMSO-d6) δ 11.47 (s, 1H), 7.89 (s, 1H), 7.83 (d, J = 7.9 Hz, 1H), 7.71 (d, J = 7.9 Hz, 1H), 7.56 (t, J = 7.9 Hz, 1H), 6.93 (s, 2H), 4.46 (d, J = 12.9 Hz, 1H), 4.27 (d, J = 17.4 Hz, 1H), 4.09 (d, J = 12.1 Hz, 1H), 4.03 (d, J = 17.4 Hz, 1H), 3.48 - 3.38 (m, 3H), 2.36 (s, 6H), 1.78 (d, J = 13.0 Hz, 1H), 1.72 (d, J = 13.0 Hz, 1H), 1.65 (d, J = 13.0 Hz, 1H), 1.45 (dd, J = 15.1, 11.2 Hz, 1H), 1.30 - 1.14 (m, 2H). |
| 6.16 | 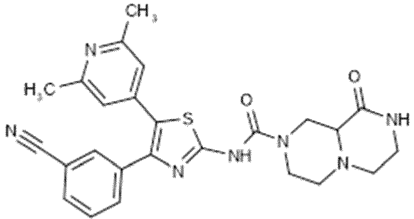 N-[4-(3-Cyanophenyl)-5-(2,6-dimethyl-4-pyridyl)thiazol-2-yl]-9-oxo-3,4,6,7,8,9a-hexahydro-1H-pyrazino[1,2-a]pyrazine-2-carboxamide | LC-MS (Method 8B): Rt 3.39 mins; |
| | | MS m/z 488.2 = [M+H]+ |
| | | 1H NMR (500 MHz, DMSO-d6) δ 11.41 (s, 1H), 7.90 (d, J= 1.8 Hz, 1H), 7.85 - 7.80 (m, 2H), 7.71 (d, J= 8.0 Hz, 1H), 7.57 (t, J= 7.8 Hz, 1H), 6.94 (s, 2H), 4.56 (d, J= 13.1 Hz, 1H), 4.16 (d, J= 13.0 Hz, 1H), 3.31 - 3.24 (m, 1H), 3.08 (d, J= 11.7 Hz, 1H), 2.99 (t, J= 12.2 Hz, 1H), 2.90 (d, J= 11.2 Hz, 2H), 2.73 (t, J= 12.1 Hz, 1H), 2.58 (d, J= 10.6 Hz, 1H), 2.45 - 2.39 (m, 1H), 2.37 (s, 6H), 2.22 - 2.14 (m, 1H). |
| 6.17 | 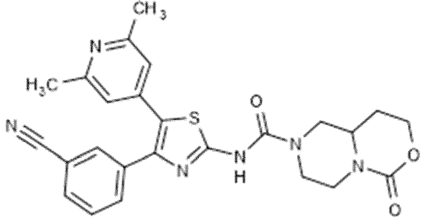 N-[4-(3-Cyanophenyl)-5-(2,6-dimethyl-4-pyridyl)thiazol-2-yl]-6-oxo-1,3,4,8,9,9a-hexahydropyrazino[1,2-c][1,3]oxazine-2-carboxamide | LC-MS (Method 8B): Rt 3.64 mins; |
| | | MS m/z 489.2 = [M+H]+ |
| | | 1H NMR (500 MHz, DMSO-d6) δ 11.36 (s, 1H), 7.88 (s, 1H), 7.82 (d, J= 7.8 Hz, 1H), 7.70 (d, J= 7.8 Hz, 1H), 7.56 (t, J= 7.8 Hz, 1H), 6.92 (s, 2H), 4.29 - 4.08 (m, 5H), 3.52 - 3.45 (m, 1H), 2.96 - 2.82 (m, 2H), 2.75 (t, J= 11.9 Hz, 1H), 2.36 (s, 6H), 2.14 - 2.07 (m, 1H), 1.76 - 1.68 (m, 1H). |
| 6.18 | 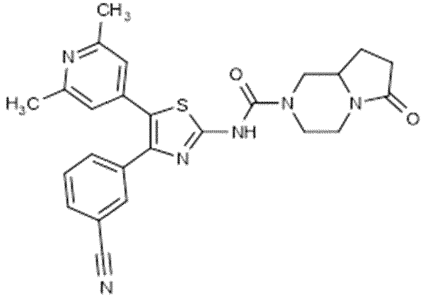 N-[4-(3-Cyanophenyl)-5-(2,6-dimethyl-4-pyridyl)thiazol-2-yl]-6-oxo-1,3,4,7,8,8a-hexahydropyrrolo[1,2-a]pyrazine-2-carboxamide | LC-MS (Method 8B): Rt 3.65 mins; |
| | | MS m/z 473.2 = [M+H]+ |
| | | 1H NMR (500 MHz, DMSO-d6) δ 11.39 (s, 1H), 7.90 (s, 1H), 7.83 (d, J= 7.7 Hz, 1H), 7.71 (d, J= 7.9 Hz, 1H), 7.57 (t, J= 7.8 Hz, 1H), 6.94 (s, 2H), 4.38 (d, J= 12.2 Hz, 1H), 4.27 (d, J= 9.3 Hz, 1H), 3.89 - 3.81 (m, 1H), 3.55 (dtd, J= 11.1, 7.1, 3.6 Hz, 1H), 2.78 (d, J= 9.2 Hz, 2H), 2.67 (d, J= 12.1 Hz, 1H), 2.37 (s, 6H), 2.34 - 2.20 (m, 2H), 2.13 (qd, J= 12.2, 10.1, 4.2 Hz, 1H), 1.63 - 1.52 (m, 1H). |
| 6.19 | 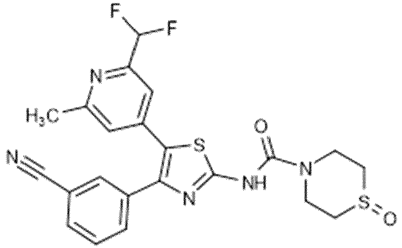 N-[4-(3-Cyanophenyl)-5-[2-(difluoromethyl)-6-methyl-4-pyridyl]thiazol-2-yl]-1-oxo-1,4-thiazinane-4-carboxamide (Prepared from Intermediate G) | LC-MS (Method 8B): Rt 2.93 mins; |
| | | MS m/z 488.1 = [M+H]+ |
| | | ¹H NMR (500 MHz, Methanol-d4) δ 7.89 (s, 1H), 7.72 (d, J = 7.8 Hz, 2H), 7.52 (t, J = 7.9 Hz, 1H), 7.31 (s, 1H), 7.27 (s, 1H), 6.59 (t, J = 55.3 Hz, 1H), 4.23 (d, J = 14.7 Hz, 2H), 4.02 - 3.95 (m, 2H), 3.02 (t, J = 12.9 Hz, 2H), 2.88 (d, J = 14.0 Hz, 2H), 2.51 (s, 3H). |
| 6.20 | 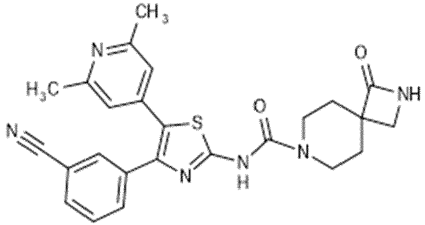 N-[4-(3-Cyanophenyl)-5-(2,6-dimethyl-4-pyridyl)thiazol-2-yl]-3-oxo-2,7-diazaspiro[3.5]nonane-7-carboxamide | LC-MS (Method 8B): Rt 3.70 mins; |
| | | MS m/z 473.2 = [M+H]+ |
| | | 1H NMR (500 MHz, Methanol-d4) δ 7.91 (s, 1H), 7.72 (d, J = 7.8 Hz, 1H), 7.69 (d, J = 7.8 Hz, 1H), 7.49 (t, J = 7.8 Hz, 1H), 7.01 (s, 2H), 3.97 - 3.89 (m, 2H), 3.56 - 3.47 (m, 2H), 3.24 (s, 2H), 2.44 (s, 6H), 2.01 - 1.93 (m, 2H), 1.92 - 1.84 (m, 2H). 2 x NH protons not observed. |
| 6.21 | 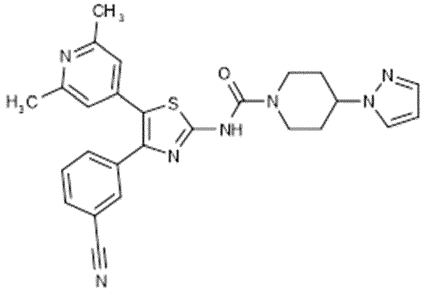 N-[4-(3-Cyanophenyl)-5-(2,6-dimethyl-4-pyridyl)thiazol-2-yl]-4-pyrazol-1-yl-piperidine-1-carboxamide | LC-MS (Method 8B): Rt 4.07 mins; |
| | | MS m/z 484.2 = [M+H]+ |
| | | 1H NMR (500 MHz, DMSO-d6) δ 11.34 (s, 1H), 7.90 (t, J = 1.7 Hz, 1H), 7.85 - 7.79 (m, 2H), 7.72 (dt, J = 8.1, 1.4 Hz, 1H), 7.57 (t, J = 7.8 Hz, 1H), 7.45 (d, J = 1.8 Hz, 1H), 6.94 (s, 2H), 6.25 (t, J = 2.0 Hz, 1H), 4.46 (td, J = 11.3, 5.5 Hz, 1H), 4.32 (d, J = 13.6 Hz, 2H), 3.07 (t, J = 13.0 Hz, 2H), 2.37 (s, 6H), 2.04 (d, J = 11.8 Hz, 2H), 1.86 (qd, J = 12.2, 3.9 Hz, 2H). |
| 6.22 | 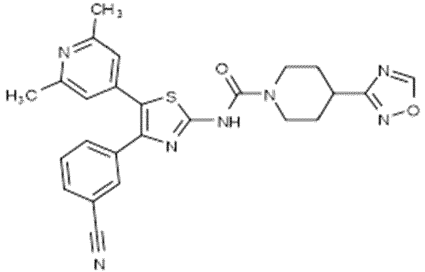 N-[4-(3-Cyanophenyl)-5-(2,6-dimethyl-4-pyridyl)thiazol-2-yl]-4-(1,2,4-oxadiazol-3-yl)piperidine-1-carboxamide | LC-MS (Method 8B): Rt 4.22 mins; |
| | | MS m/z 486.2 = [M+H]+ |
| | | 1H NMR (500 MHz, DMSO-d6) δ 11.30 (s, 1H), 9.54 (s, 1H), 7.90 (s, 1H), 7.83 (d, J = 7.7 Hz, 1H), 7.72 (d, J = 7.9 Hz, 1H), 7.57 (t, J = 7.8 Hz, 1H), 6.94 (s, 2H), 4.21 (d, J = 13.5 Hz, 2H), 3.19 (s, 1H), 3.13 (t, J = 12.4 Hz, 2H), 2.37 (s, 6H), 2.01 (d, J = 13.1 Hz, 2H), 1.71 - 1.60 (m, 2H). |
| 6.23 | 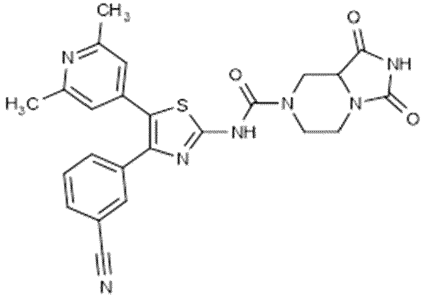 N-[4-(3-Cyanophenyl)-5-(2,6-dimethyl-4-pyridyl)thiazol-2-yl]-1,3-dioxo-5,6,8,8a-tetrahydroimidazo[1,5-a]pyrazine-7-carboxamide | LC-MS (Method 8B): Rt 2.96 mins; |
| | | MS m/z 488.1 = [M+H]+ |
| | | 1H NMR (500 MHz, DMSO-d6) δ 11.58 (s, 1H), 11.03 (s, 1H), 7.90 (s, 1H), 7.84 (d, J= 7.7 Hz, 1H), 7.71 (d, J= 7.9 Hz, 1H), 7.57 (t, J= 7.8 Hz, 1H), 6.94 (s, 2H), 4.50 (d, J= 13.0 Hz, 1H), 4.23 (d, J= 10.8 Hz, 1H), 4.17 (dd, J= 11.3, 4.6 Hz, 1H), 3.87 (d, J= 10.0 Hz, 1H), 3.06 (t, J= 12.2 Hz, 1H), 2.96 (q, J= 11.9, 10.4 Hz, 2H), 2.37 (s, 6H). |
| 6.24 | 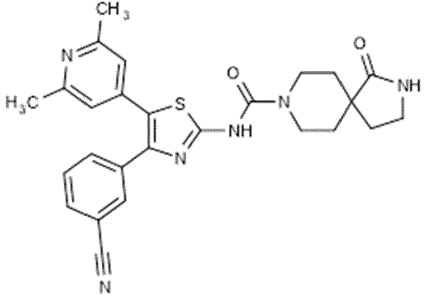 N-[4-(3-Cyanophenyl)-5-(2,6-dimethyl-4-pyridyl)thiazol-2-yl]-1-oxo-2,8-diazaspiro[4.5]decane-8-carboxamide | LC-MS (Method 8B): Rt 3.69 mins; |
| | | MS m/z 487.2 = [M+H]+ |
| | | 1H NMR (500 MHz, DMSO-d6) δ 11.25 (s, 1H), 7.90 (s, 1H), 7.83 (d, J= 7.8 Hz, 1H), 7.71 (d, J= 8.0 Hz, 1H), 7.63 (s, 1H), 7.57 (t, J= 7.8 Hz, 1H), 6.94 (s, 2H), 4.09 (d, J= 13.5 Hz, 2H), 3.20 (t, J= 6.8 Hz, 2H), 3.10 (t, J= 12.5 Hz, 2H), 2.37 (s, 6H), 2.01 (t, J= 6.8 Hz, 2H), 1.60 (td, J= 12.7, 12.1, 4.1 Hz, 2H), 1.41 (d,J = 13.6 Hz, 2H). |
| 6.25 | 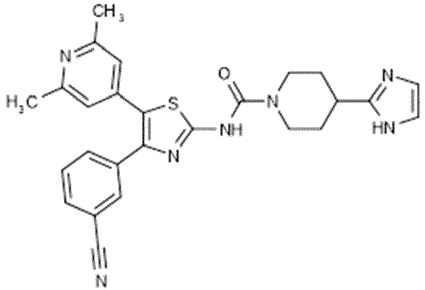 N-[4-(3-Cyanophenyl)-5-(2,6-dimethyl-4-pyridyl)thiazol-2-yl]-4-(1H-imidazol-2-yl)piperidine-1-carboxamide | LC-MS (Method 8B): Rt 3.66 mins; |
| | | MS m/z 484.2 = [M+H]+ |
| | | 1H NMR (500 MHz, DMSO-d6) δ 11.71 (s, 1H), 11.28 (s, 1H), 7.90 (d, J= 2.0 Hz, 1H), 7.83 (d, J= 7.7 Hz, 1H), 7.72 (d, J= 7.9 Hz, 1H), 7.57 (t, J= 7.8 Hz, 1H), 6.99 (s, 1H), 6.94 (s, 2H), 6.77 (s, 1H), 4.22 (d, J= 13.4 Hz, 2H), 3.05 (t, J= 12.5 Hz, 2H), 2.95 (s, 1H), 2.37 (s, 6H), 1.92 (d, J= 12.6 Hz, 2H), 1.70 - 1.58 (m, 2H). |
| 6.26 | 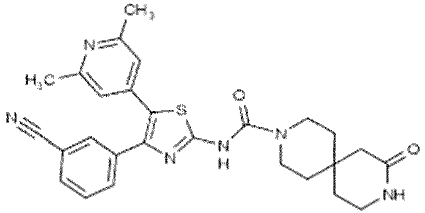 N-[4-(3-Cyanophenyl)-5-(2,6-dimethyl-4-pyridyl)thiazol-2-yl]-10-oxo-3,9-diazaspiro[5.5]undecane-3-carboxamide | LC-MS (Method 8B): Rt 3.84 mins; |
| | | MS m/z 501.2 = [M+H]+ |
| | | ¹H NMR (500 MHz, DMSO-d6) δ 11.21 (s, 1H), 7.89 (s, 1H), 7.82 (d,J= 7.8 Hz, 1H), 7.70 (d,J= 7.8 Hz, 1H), 7.56 (t,J= 7.8 Hz, 1H), 7.43 (s, 1H), 6.92 (s, 2H), 3.61 - 3.47 (m, 4H), 3.20 - 3.12 (m, 2H), 2.36 (s, 6H), 2.09 (s, 2H), 1.67 - 1.59 (m, 2H), 1.47 - 1.34 (m, 4H). |
| 6.27 | 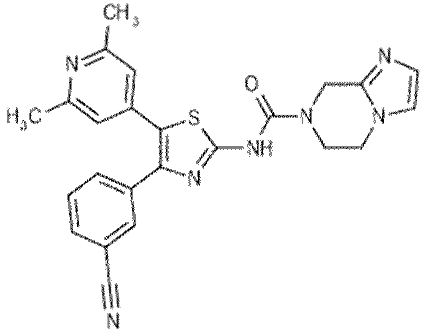 N-[4-(3-Cyanophenyl)-5-(2,6-dimethyl-4-pyridyl)thiazol-2-yl]-6,8-dihydro-5H-imidazo[1,2-a]pyrazine-7-carboxamide | LC-MS (Method 8B): Rt 3.04 mins; |
| | | MS m/z 456.2 = [M+H]+ |
| | | 1H NMR (500 MHz, DMSO-d6) δ 11.62 (s, 1H), 7.90 (s, 1H), 7.83 (d, J= 7.7 Hz, 1H), 7.72 (d, J= 8.0 Hz, 1H), 7.57 (t, J= 7.8 Hz, 1H), 7.13 (d, J= 1.3 Hz, 1H), 6.94 (s, 2H), 6.90 (d, J= 1.3 Hz, 1H), 4.77 (s, 2H), 4.07 (t ,J= 5.3 Hz, 2H), 3.99 (t, J= 5.3 Hz, 2H), 2.36 (s, 6H). |
| 6.28 | 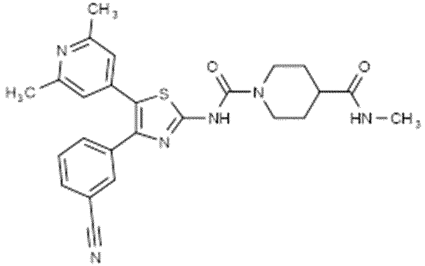 N1-[4-(3-Cyanophenyl)-5-(2,6-dimethyl-4-pyridyl)thiazol-2-yl]-N4-methyl-piperidine-1,4-dicarboxamide | LC-MS (Method 8B): Rt 3.45 mins; |
| | | MS m/z 475.2 = [M+H]+ |
| | | 1H NMR (500 MHz, DMSO-d6) δ 11.22 (s, 1H), 7.89 (s, 1H), 7.82 (d, J= 7.7 Hz, 1H), 7.78 (d, J= 5.0 Hz, 1H), 7.71 (d, J= 7.9 Hz, 1H), 7.56 (t, J= 7.8 Hz, 1H), 6.93 (s, 2H), 4.19 (d, J= 13.3 Hz, 2H), 2.89 (t, J= 12.9 Hz, 2H), 2.57 (d, J= 4.5 Hz, 3H), 2.36 (s, 6H), 2.31 (s, 1H), 1.70 (d, J= 12.5 Hz, 2H), 1.54 - 1.42 (m, 2H). |
| 6.29 | 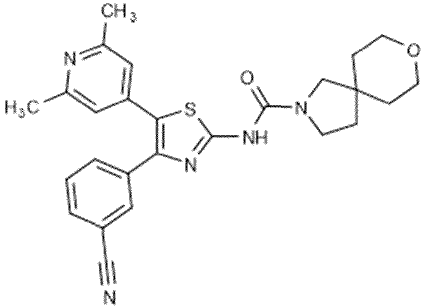 N-[4-(3-Cyanophenyl)-5-(2,6-dimethyl-4-pyridyl)thiazol-2-yl]-8-oxa-2-azaspiro[4.5]decane-2-carboxamide | LC-MS (Method 8B): Rt 4.28 mins; |
| | | MS m/z 474.2 = [M+H]+ |
| | | 1H NMR (500 MHz, DMSO-d6) δ 10.99 (s, 1H), 7.89 (t, J= 1.7 Hz, 1H), 7.82 (d, J= 7.8 Hz, 1H), 7.71 (d, J= 7.8 Hz, 1H), 7.56 (t, J= 7.8 Hz, 1H), 6.93 (s, 2H), 3.68 - 3.58 (m, 2H), 3.57 - 3.45 (m, 4H), 3.44 - 3.31 (m, 2H), 2.36 (s, 6H), 1.81 (s, 2H), 1.55 - 1.44 (m, 4H). |
| 6.30 | 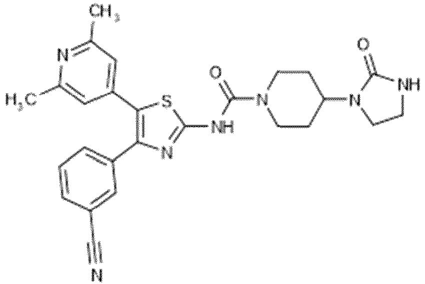 N-[4-(3-Cyanophenyl)-5-(2,6-dimethyl-4-pyridyl)thiazol-2-yl]-4-(2-oxoimidazolidin-1-yl)piperidine-1-carboxamide | LC-MS (Method 8B): Rt 3.82 mins; |
| | | MS m/z 502.2 = [M+H]+ |
| | | 1H NMR (500 MHz, DMSO-d6) δ 11.24 (s, 1H), 7.89 (s, 1H), 7.82 (d, J= 7.8 Hz, 1H), 7.70 (d, J= 7.8 Hz, 1H), 7.56 (t, J= 7.8 Hz, 1H), 6.93 (s, 2H), 6.29 (s, 1H), 4.30 (d, J= 13.3 Hz, 2H), 3.77 - 3.69 (m, 1H), 3.29 - 3.24 (m, 2H), 3.22 - 3.16 (m, 2H), 2.90 (t, J= 12.8 Hz, 2H), 2.36 (s, 6H), 1.63 - 1.48 (m, 4H). |
| 6.31 | 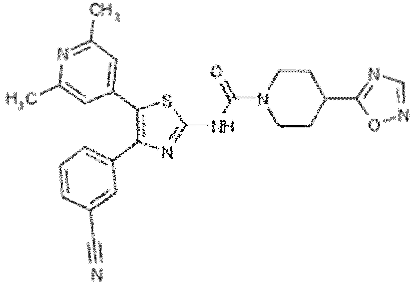 N-[4-(3-Cyanophenyl)-5-(2,6-dimethyl-4-pyridyl)thiazol-2-yl]-4-(1,2,4-oxadiazol-5-yl)piperidine-1-carboxamide | LC-MS (Method 5B): Rt 1.57 mins; |
| | | MS m/z 486.2 = [M+H]+ |
| | | 1H NMR (500 MHz, Methanol-d4) δ 8.61 (s, 1H), 7.91 (s, 1H), 7.76 - 7.66 (m, 2H), 7.49 (t, J = 7.8 Hz, 1H), 7.01 (s, 2H), 4.24 (d, J = 13.8 Hz, 2H), 3.44 - 3.39 (m, 1H), 3.28 - 3.21 (m, 2H), 2.44 (s, 6H), 2.24 - 2.15 (m, 2H), 1.93 ~ 1.83 (m, 2H). NH proton not observed |
| 6.32 | 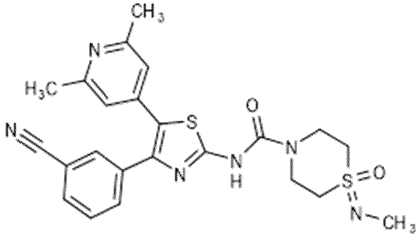 N-[4-(3-Cyanophenyl)-5-(2,6-dimethyl-4-pyridyl)thiazol-2-yl]-1-methylimino-1-oxo-1,4-thiazinane-4-carboxamide (Prepared from Intermediate S) | LC-MS (Method 8B): Rt 3.09 mins; |
| | | MS m/z 481.1 = [M+H]+ |
| | | 1H NMR (500 MHz, DMSO-d6) δ 11.55 (s, 1H), 7.89 (s, 1H), 7.83 (d, J = 7.9 Hz, 1H), 7.70 (d, J = 7.9 Hz, 1H), 7.56 (t, J = 7.9 Hz, 1H), 6.93 (s, 2H), 4.14 (d, J = 14.8 Hz, 2H), 3.67 - 3.59 (m, 2H), 3.27 - 3.21 (m, 2H), 3.16 - 3.08 (m, 2H), 2.66 (s, 3H), 2.36 (s, 6H). |
| 6.33 | 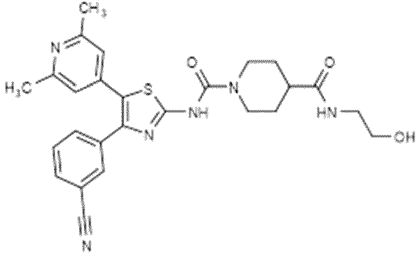 N1-[4-(3-Cyanophenyl)-5-(2,6-dimethyl-4-pyridyl)thiazol-2-yl]-N4-(2-hydroxyethyl)piperidine-1,4-dicarboxamide | LC-MS (Method 8B): Rt 3.55 mins; |
| | | MS m/z 505.2 = [M+H]+ |
| | | 1H NMR (500 MHz, Methanol-d4) δ 7.93 (t, J= 1.7 Hz, 1H), 7.72 (ddt, J= 13.4, 7.8, 1.5 Hz, 2H), 7.51 (t, J= 7.8 Hz, 1H), 7.02 (s, 2H), 4.29 (d, J= 13.4 Hz, 2H), 3.62 (t, J= 5.8 Hz, 2H), 3.02 (t, J= 12.7 Hz, 2H), 2.56 - 2.48 (m, 1H), 2.45 (s, 6H), 1.89 (d ,J= 12.1 Hz, 2H), 1.71 (qd, J= 12.3, 4.1 Hz, 2H). 2 x NH, 1 x OH and 1 x CH₂ signal not observed. |
| 6.34 | 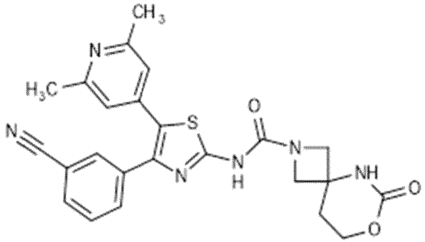 N-[4-(3-Cyanophenyl)-5-(2,6-dimethyl-4-pyridyl)thiazol-2-yl]-6-oxo-7-oxa-2,5-diazaspiro[3.5]nonane-2-carboxamide | LC-MS (Method 8B): Rt 3.44 mins; |
| | | MS m/z 475.2 = [M+H]+ |
| | | 1H NMR (500 MHz, DMSO-d6) δ 11.46 (s, 1H), 7.99 (s, 1H), 7.87 (s, 1H), 7.82 (d,J= 7.8 Hz, 1H), 7.70 (d,J= 7.8 Hz, 1H), 7.56 (t,J= 7.8 Hz, 1H), 6.92 (s, 2H), 4.16 (t,J= 5.3 Hz, 2H), 4.08 - 3.94 (m, 4H), 2.36 (s, 6H), 2.13 (t,J= 5.3 Hz, 2H). |
| 6.35 | 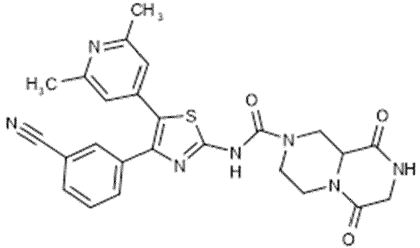 N-[4-(3-Cyanophenyl)-5-(2,6-dimethyl-4-pyridyl)thiazol-2-yl]-6,9-dioxo-1,3,4,7,8,9a-hexahydropyrazino[1,2-a]pyrazine-2-carboxamide | LC-MS (Method 8B): Rt 2.72 mins; |
| | | MS m/z 502.2 = [M+H]+ |
| | | 1H NMR (500 MHz, DMSO-d6) δ 11.59 (s, 1H), 8.30 (s, 1H), 7.90 (s, 1H), 7.83 (d, J= 7.8 Hz, 1H), 7.72 (d, J= 7.8 Hz, 1H), 7.57 (t, J= 7.8 Hz, 1H), 6.94 (s, 2H), 4.60 (d, J= 13.3 Hz, 1H), 4.31 (d, J= 13.3 Hz, 1H), 4.25 (d, J= 13.3 Hz, 1H), 4.08 (d, J= 10.3 Hz, 1H), 3.94 - 3.81 (m, 2H), 3.04 - 2.90 (m, 2H), 2.74 (t, J= 12.6 Hz, 1H), 2.37 (s, 6H). |
| 6.36 | 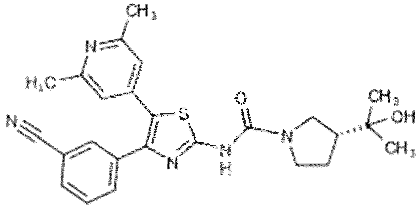 (3R)-N-[4-(3-Cyanophenyl)-5-(2,6-dimethyl-4-pyridyl)thiazol-2-yl]-3-(1-hydroxy-1-methyl-ethyl)pyrrolidine-1-carboxamide | LC-MS (Method 8B): Rt 4.10 mins; |
| | | MS m/z 462.2 = [M+H]+ |
| | | 1H NMR (500 MHz, DMSO-d6) δ 10.88 (s, 1H), 7.89 (s, 1H), 7.81 (d,J= 7.8 Hz, 1H), 7.71 (d,J= 7.8 Hz, 1H), 7.55 (t,J= 7.8 Hz, 1H), 6.92 (s, 2H), 4.38 (s, 1H), 3.68 - 3.49 (m, 3H), 2.36 (s, 6H), 2.26 - 2.15 (m, 1H), 1.90 - 1.73 (m, 2H), 1.11 (s, 6H). 1 x CH proton not observed. |
| 6.37 | 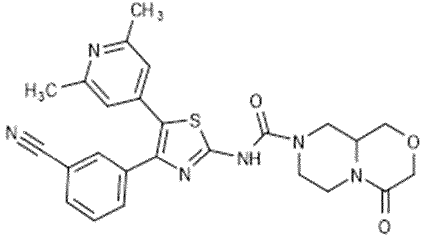 N-[4-(3-Cyanophenyl)-5-(2,6-dimethyl-4-pyridyl)thiazol-2-yl]-4-oxo-6,7,9,9a-tetrahydro-1H-pyrazino[2,1-c][1,4]oxazine-8-carboxamide | LC-MS (Method 8B): Rt 3.02 mins; |
| | | MS m/z 489.1 = [M+H]+ |
| | | 1H NMR (500 MHz, DMSO-d6) δ 11.37 (s, 1H), 7.89 (s, 1H), 7.82 (d, J = 7.9 Hz, 1H), 7.71 (d, J = 7.9 Hz, 1H), 7.56 (t, J = 7.9 Hz, 1H), 6.93 (s, 2H), 4.38 (d, J = 13.1 Hz, 1H), 4.29 - 4.18 (m, 2H), 4.07 (s, 2H), 4.04 - 3.97 (m, 1H), 3.63 - 3.54 (m, 2H), 2.90 (t, J = 12.3 Hz, 1H), 2.84 - 2.77 (m, 1H), 2.74 (td, J = 12.6, 2.6 Hz, 1H), 2.36 (s, 6H). |
| 6.38 | 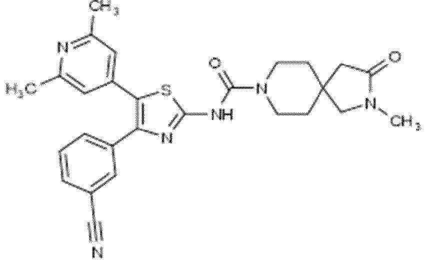 N-[4-(3-Cyanophenyl)-5-(2,6-dimethyl-4-pyridyl)thiazol-2-yl]-2-methyl-3-oxo-2,8-diazaspiro[4.5]decane-8-carboxamide | LC-MS (Method 8B): Rt 3.80 mins; |
| | | MS m/z 501.3 = [M+H]+ |
| | | 1H NMR (500 MHz, DMSO-d6) δ 11.24 (s, 1H), 7.90 (s, 1H), 7.83 (d, J= 7.7 Hz, 1H), 7.71 (d, J= 8.0 Hz, 1H), 7.56 (t, J= 7.8 Hz, 1H), 6.93 (s, 2H), 3.65 - 3.57 (m, 2H), 3.51 - 3.43 (m, 2H), 3.20 (s, 2H), 2.72 (s, 3H), 2.36 (s, 6H), 2.21 (s, 2H), 1.60 - 1.50 (m, 4H). |
| 6.39 | 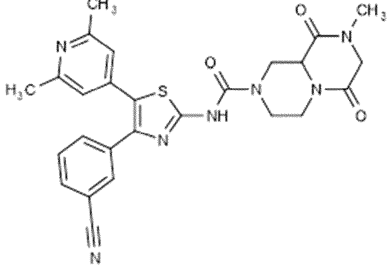 N-[4-(3-Cyanophenyl)-5-(2,6-dimethyl-4-pyridyl)thiazol-2-yl]-8-methyl-6,9-dioxo-3,4,7,9a-tetrahydro-1H-pyrazino[1,2-a]pyrazine-2-carboxamide | LC-MS (Method 8B): Rt 2.32 mins; |
| | | MS m/z 516.3 = [M+H]+ |
| | | 1H NMR (500 MHz, DMSO-d6) δ 11.58 (s, 1H), 7.90 (s, 1H), 7.84 (d, J = 7.7 Hz, 1H), 7.72 (d, J = 8.0 Hz, 1H), 7.57 (t, J = 7.8 Hz, 1H), 6.94 (s, 2H), 4.63 (d, J = 13.4 Hz, 1H), 4.33 (d, J = 13.0 Hz, 1H), 4.26 (d, J = 13.5 Hz, 1H), 4.10 (d, J = 11.1 Hz, 1H), 4.07 - 3.96 (m, 2H), 3.02 - 2.87 (m, 2H), 2.86 (s, 3H), 2.74 (dd, J = 14.3, 11.1 Hz, 1H), 2.37 (s, 6H). |
| 6.40 | 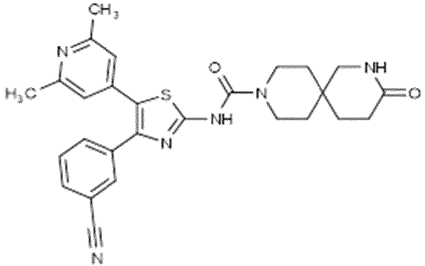 N-[4-(3-Cyanophenyl)-5-(2,6-dimethyl-4-pyridyl)thiazol-2-yl]-3-oxo-2,9-diazaspiro[5.5]undecane-9-carboxamide | LC-MS (Method 8B): Rt 3.94 mins; |
| | | MS m/z 501.3 = [M+H]+ |
| | | 1H NMR (500 MHz, DMSO-d6) δ 11.22 (s, 1H), 7.90 (s, 1H), 7.83 (d, J= 7.7 Hz, 1H), 7.71 (d, J= 7.9 Hz, 1H), 7.57 (t, J= 7.8 Hz, 1H), 7.42 (s, 1H), 6.94 (s, 2H), 3.60 - 3.48 (m, 4H), 3.00 (d, J= 2.6 Hz, 2H), 2.37 (s, 6H), 2.16 (t,J= 7.0 Hz, 2H), 1.67 (t, J= 7.0 Hz, 2H), 1.45 (t, J= 5.9 Hz, 4H). |
| 6.41 | 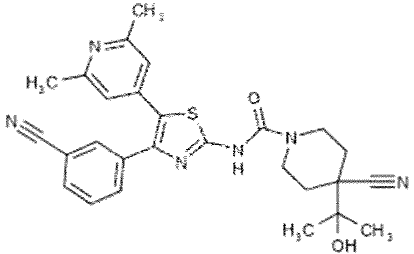 4-Cyano-N-[4-(3-cyanophenyl)-5-(2,6-dimethyl-4-pyridyl)thiazol-2-yl]-4-(1-hydroxy-1-methyl-ethyl)piperidine-1-carboxamide | LC-MS (Method 8B): Rt 4.10 mins; |
| | | MS m/z 501.3 = [M+H]+ |
| | | 1H NMR (500 MHz, DMSO-d6) δ 11.34 (s, 1H), 7.89 (s, 1H), 7.82 (d, J = 7.8 Hz, 1H), 7.70 (d, J = 7.8 Hz, 1H), 7.56 (t, J = 7.8 Hz, 1H), 6.92 (s, 2H), 5.12 (s, 1H), 4.38 (d, J = 13.4 Hz, 2H), 2.94 (t, J = 13.4 Hz, 2H), 2.36 (s, 6H), 1.88 (d, J = 13.4 Hz, 2H), 1.63 (td, J = 13.4, 4.1 Hz, 2H), 1.23 (s, 6H). |
| 6.42 | 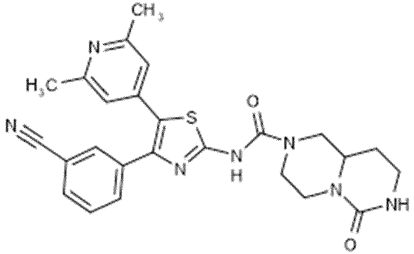 N-[4-(3-Cyanophenyl)-5-(2,6-dimethyl-4-pyridyl)thiazol-2-yl]-6-oxo-3,4,7,8,9,9a-hexahydro-1H-pyrazino[1,2-c]pyrimidine-2-carboxamide | LC-MS (Method 8B): Rt 3.21 mins; |
| | | MS m/z 488.3 = [M+H]+ |
| | | 1H NMR (500 MHz, DMSO-d6) δ 11.31 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 6.93 (s, 2H), 6.46 (s, 1H), 4.27 - 4.12 (m, 3H), 3.28 - 3.23 (m, 1H), 3.17 - 3.07 (m, 2H), 2.83 (t, J= 12.6 Hz, 1H), 2.69-2.56 (m, 2H), 2.36 (s, 6H), 1.97 - 1.92 (m, 1H), 1.64 - 1.55 (m, 1H). |
| 6.43 | 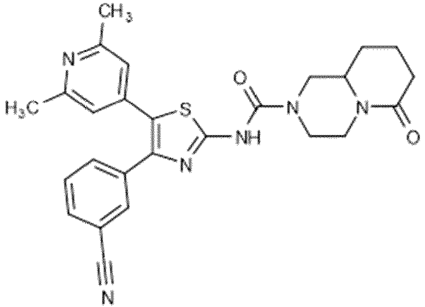 N-[4-(3-Cyanophenyl)-5-(2,6-dimethyl-4-pyridyl)thiazol-2-yl]-6-oxo-3,4,7,8,9,9a-hexahydro-1H-pyrido[1,2-a]pyrazine-2-carboxamide | LC-MS (Method 8B): Rt 3.96 mins; |
| | | MS m/z 487.3 = [M+H]+ |
| | | 1H NMR (500 MHz, DMSO-d6) δ 11.35 (s, 1H), 7.90 (s, 1H), 7.83 (d, J= 7.8 Hz, 1H), 7.71 (d, J= 7.8 Hz, 1H), 7.57 (t, J= 7.8 Hz, 1H), 6.94 (s, 2H), 4.45 (d, J= 13.1 Hz, 1H), 4.23 (d, J= 13.1 Hz, 2H), 2.87 - 2.81 (m, 1H), 2.74 - 2.68 (m, 1H), 2.64 - 2.57 (m, 2H), 2.37 (s, 6H), 2.29 - 2.22 (m, 2H), 2.00 - 1.93 (m, 1H), 1.82 - 1.74 (m, 1H), 1.69 - 1.59 (m, 1H), 1.49 - 1.39 (m, 1H). |
| 6.44 | 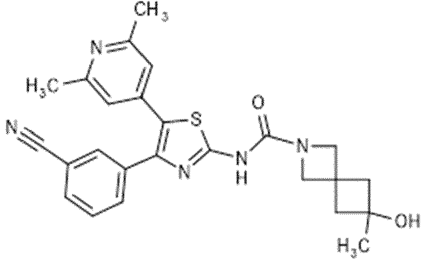 N-[4-(3-Cyanophenyl)-5-(2,6-dimethyl-4-pyridyl)thiazol-2-yl]-6-hydroxy-6-methyl-2-azaspiro[3.3]heptane-2-carboxamide | LC-MS (Method 8B): Rt 3.98 mins; |
| | | MS m/z 460.3 = [M+H]+ |
| | | 1H NMR (500 MHz, DMSO-d6) δ 11.25 (s, 1H), 7.87 (s, 1H), 7.81 (d, J= 7.8 Hz, 1H), 7.69 (d, J= 7.8 Hz, 1H), 7.55 (t, J= 7.8 Hz, 1H), 6.92 (s, 2H), 4.91 (s, 1H), 4.10 - 3.91 (m, 4H), 2.35 (s, 6H), 2.18 (s, 4H), 1.16 (s, 3H). |
| 6.45 | 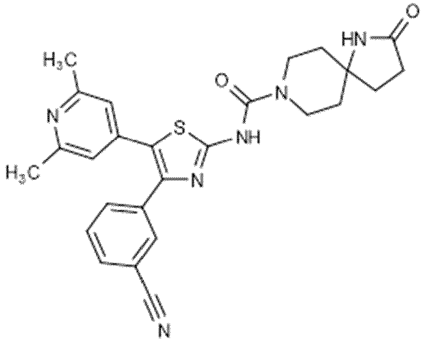 N-[4-(3-Cyanophenyl)-5-(2,6-dimethyl-4-pyridyl)thiazol-2-yl]-2-oxo-1,8-diazaspiro[4.5]decane-8-carboxamide | LC-MS (Method 8B): Rt 3.85 mins; |
| | | MS m/z 487.3 = [M+H]+ |
| | | 1H NMR (500 MHz, DMSO-d6) δ 11.27 (s, 1H), 8.05 (s, 1H), 7.89 (d, J = 1.9 Hz, 1H), 7.83 (d, J = 7.7 Hz, 1H), 7.71 (d, J = 8.1 Hz, 1H), 7.57 (t, J = 7.8 Hz, 1H), 6.93 (s, 2H), 3.67 (dt, J = 12.3, 5.8 Hz, 2H), 3.53 - 3.45 (m, 2H), 2.36 (s, 6H), 2.22 (t, J = 7.9 Hz, 2H), 1.88 (t, J = 8.0 Hz, 2H), 1.63 - 1.53 (m, 4H). |
| 6.46 | 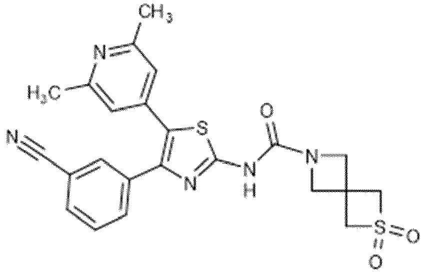 N-[4-(3-Cyanophenyl)-5-(2,6-dimethyl-4-pyridyl)thiazol-2-yl]-2,2-dioxo-2λ^{6}-thia-6-azaspiro[3.3]heptane-6-carboxamide | LC-MS (Method 8B): Rt 3.69 mins; |
| | | MS m/z 480.2 = [M+H]+ |
| | | 1H NMR (500 MHz, DMSO-d6) δ 11.48 (s, 1H), 7.87 (s, 1H), 7.82 (d, J= 7.8 Hz, 1H), 7.70 (d, J= 7.8 Hz, 1H), 7.56 (t, J= 7.8 Hz, 1H), 6.92 (s, 2H), 4.47 (s, 4H), 4.27 (s, 4H), 2.36 (s, 6H). |
| 6.47 | 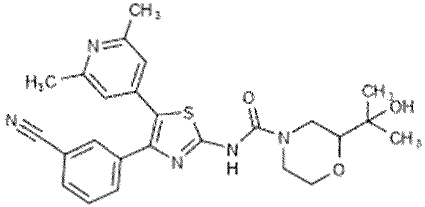 N-[4-(3-Cyanophenyl)-5-(2,6-dimethyl-4-pyridyl)thiazol-2-yl]-2-(1-hydroxy-1-methyl-ethyl)morpholine-4-carboxamide | LC-MS (Method 8B): Rt 3.63 mins; |
| | | MS m/z 478.3 = [M+H]+ |
| | | 1H NMR (500 MHz, DMSO-d6) δ 11.34 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 6.93 (s, 2H), 4.51 (s, 1H), 4.25 (d, J= 13.1 Hz, 1H), 4.05 (d, J= 13.1 Hz, 1H), 3.92 (d, J= 11.5 Hz, 1H), 3.44 (t, J= 11.5 Hz, 1H), 3.10 (d, J= 10.7 Hz, 1H), 2.93 (t, J= 12.2 Hz, 1H), 2.77 (t, J= 12.2 Hz, 1H), 2.36 (s, 6H), 1.13 (s, 3H), 1.09 (s, 3H). |
| 6.48 | 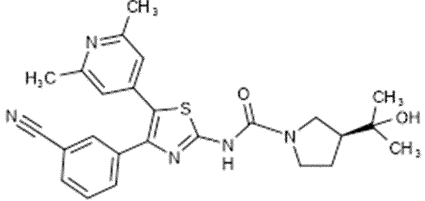 (3S)-N-[4-(3-Cyanophenyl)-5-(2,6-dimethyl-4-pyridyl)thiazol-2-yl]-3-(1-hydroxy-1-methyl-ethyl)pyrrolidine-1-carboxamide | LC-MS (Method 15B): Rt 6.86 mins; |
| | | MS m/z 462.4 = [M+H]+ |
| | | 1H NMR (500 MHz, DMSO-d6) δ 10.96 (s, 1H), 7.89 (s, 1H), 7.81 (d, J= 7.8 Hz, 1H), 7.70 (d, J= 7.8 Hz, 1H), 7.55 (t, J= 7.8 Hz, 1H), 6.93 (s, 2H), 4.39 (s, 1H), 3.74- 3.47 (m, 3H), 3.33 - 3.21 (m, 1H),2.36 (s, 6H), 2.26 - 2.15 (m, 1H), 1.92 - 1.72 (m, 2H), 1.11 (s, 6H). |
| 6.49 | 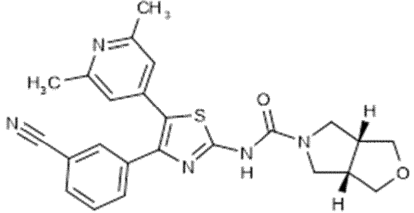 (3aR,6aS)-N-[4-(3-Cyanophenyl)-5-(2,6-dimethyl-4-pyridyl)thiazol-2-yl]-1,3,3a,4,6,6a-hexahydrofuro[3,4-c]pyrrole-5-carboxamide | LC-MS (Method 8B): Rt 4.04 mins; |
| | | MS m/z 446.3 = [M+H]+ |
| | | 1H NMR (500 MHz, DMSO-d6) δ 11.06 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 6.92 (s, 2H), 3.82 - 3.76 (m, 2H), 3.67 - 3.61 (m, 2H), 3.55 - 3.51 (m, 2H), 3.43 - 3.39 (m, 2H), 2.98 - 2.92 (m, 2H), 2.36 (s, 6H). |
| 6.50 | 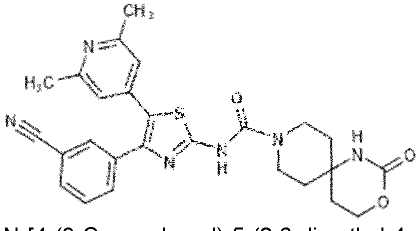 N-[4-(3-Cyanopnenyl)-5-(2,b-dimethyl-4-pyridyl)thiazol-2-yl]-2-oxo-3-oxa-1,9-diazaspiro[5.5]undecane-9-carboxamide | LC-MS (Method 8B): Rt 2.58 mins; |
| | | MS m/z 503.3 = [M+H]+ |
| | | ¹H NMR (500 MHz, DMSO-d6) δ 11.26 (s, 1H), 7.89 (s, 1H), 7.82 (d, J= 7.8 Hz, 1H), 7.70 (d, J= 7.8 Hz, 1H), 7.56 (t, J= 7.8 Hz, 1H), 7.35 (s, 1H), 6.92 (s, 2H), 4.18 (t, J= 5.0 Hz, 2H), 3.85 (d, J= 13.6 Hz, 2H), 2.36 (s, 6H), 1.89 (t, J= 5.0 Hz, 2H), 1.70 - 1.54 (m, 4H). 1 x CH₂ signal not observed. |
| 6.51 | 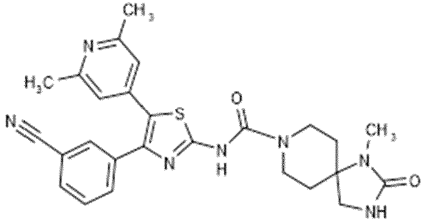 N-[4-(3-Cyanophenyl)-5-(2,6-dimethyl-4-pyridyl)thiazol-2-yl]-1-methyl-2-oxo-1,3,8-triazaspiro[4.5]decane-8-carboxamide | LC-MS (Method 8B): Rt 2.67 mins; |
| | | MS m/z 500.5 = [M-H]- |
| | | 1H NMR (500 MHz, DMSO-d6) δ 11.31 (s, 1H), 7.90 (s, 1H), 7.83 (d, J= 7.8 Hz, 1H), 7.71 (d, J= 7.8 Hz, 1H), 7.57 (t, J= 7.8 Hz, 1H), 6.93 (s, 2H), 6.39 (s, 1H), 4.25 (d, J= 13.4 Hz, 2H), 3.23 (s, 2H), 2.93 (t, J= 13.4 Hz, 2H), 2.52 (s, 3H), 2.37 (s, 6H), 1.78 (td, J= 13.4, 4.0 Hz, 2H), 1.49 (d, J= 12.7 Hz, 2H). |
| 6.52 | 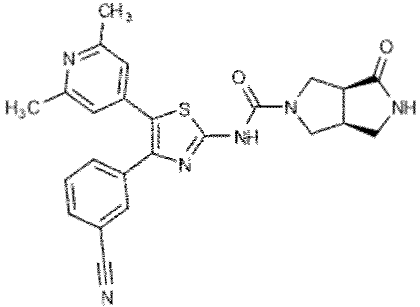 (3aS,6aS)-N-[4-(3-Cyanophenyl)-5-(2,6-dimethyl-4-pyridyl)thiazol-2-yl]-3-oxo-1,2,3a,4,6,6a-hexahydropyrrolo[3,4-c]pyrrole-5-carboxamide | LC-MS (Method 8B): Rt 3.58 mins; |
| | | MS m/z 459.3 = [M+H]+ |
| | | 1H NMR (500 MHz, DMSO-d6) δ 11.14 (s, 1H), 7.90 (s, 1H), 7.85 - 7.79 (m, 2H), 7.72 (d, J = 8.0 Hz, 1H), 7.57 (t, J = 7.8 Hz, 1H), 6.94 (s, 2H), 3.86 (t, J = 10.0 Hz, 1H), 3.80 (d, J = 10.9 Hz, 1H), 3.55 (t, J = 9.2 Hz, 1H), 3.44 (dd, J = 10.3, 6.0 Hz, 1H), 3.20 (d, J = 10.7 Hz, 1H), 3.07 (d, J = 10.3 Hz, 2H), 3.04 - 2.99 (m, 1H), 2.36 (s, 6H). |
| 6.53 | 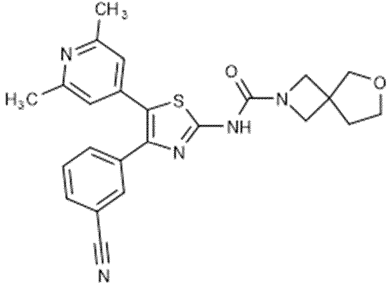 N-[4-(3-Cyanophenyl)-5-(2,6-dimethyl-4-pyridyl)thiazol-2-yl]-6-oxa-2-azaspiro[3.4]octane-2-carboxamide | LC-MS (Method 8B): Rt 3.95 mins; |
| | | MS m/z 446.3 = [M+H]+ |
| | | 1H NMR (500 MHz, DMSO-d6) δ 11.39 - 11.35 (m, 1H), 7.88 (s, 1H), 7.82 (d, J= 7.7 Hz, 1H), 7.70 (d, J= 7.9 Hz, 1H), 7.56 (t, J= 7.8 Hz, 1H), 6.93 (s, 2H), 4.01 (s, 4H), 3.77 (s, 2H), 3.71 (t, J= 7.0 Hz, 2H), 2.36 (s, 6H), 2.12 (t, J= 7.0 Hz, 2H) |
| 6.54 | 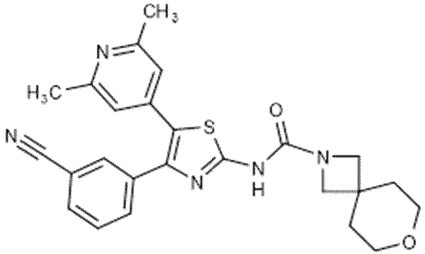 N-[4-(3-Cyanophenyl)-5-(2,6-dimethyl-4-pyridyl)thiazol-2-yl]-7-oxa-2-azaspiro[3.5]nonane-2-carboxamide | LC-MS (Method 8B): Rt 3.28 mins; |
| | | MS m/z 458.4 = [M-H]- |
| | | 1H NMR (500 MHz, DMSO-d6) δ 11.31 (s, 1H), 7.87 (s, 1H), 7.82 (d, J= 7.8 Hz, 1H), 7.70 (d, J= 7.8 Hz, 1H), 7.56 (t, J= 7.8 Hz, 1H), 6.92 (s, 2H), 3.86 - 3.68 (m, 4H), 3.57 - 3.45 (m, 4H), 2.36 (s, 6H), 1.75 - 1.64 (m, 4H). |
| 6.55 | 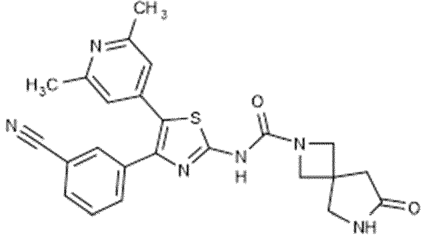 N-[4-(3-Cyanophenyl)-5-(2,6-dimethyl-4-pyridyl)thiazol-2-yl]-6-oxo-2,7-diazaspiro[3.4]octane-2-carboxamide | LC-MS (Method 8B): Rt 2.59 mins; |
| | | MS m/z 457.4 = [M-H]- |
| | | 1H NMR (500 MHz, DMSO-d6) δ 11.37 (s, 1H), 7.87 (s, 1H), 7.82 (d, J= 7.8 Hz, 1H), 7.70 (d, J= 7.8 Hz, 1H), 7.65 (s, 1H), 7.56 (t, J= 7.8 Hz, 1H), 6.92 (s, 2H), 4.08 - 3.86 (m, 4H), 3.44 (s, 2H), 3.31 (s, 2H), 2.36 (s, 6H). |
| 6.56 | 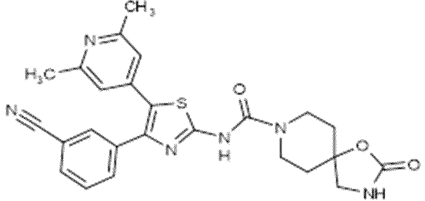 N-[4-(3-Cyanophenyl)-5-(2,6-dimethyl-4-pyridyl)thiazol-2-yl]-2-oxo-1-oxa-3,8-diazaspiro[4.5]decane-8-carboxamide | LC-MS (Method 8B): Rt 2.90 mins; |
| | | MS m/z 487.4 = [M-H]- |
| | | 1H NMR (500 MHz, DMSO-d6) δ 11.32 (s, 1H), 7.89 (s, 1H), 7.82 (d, J= 7.7 Hz, 1H), 7.71 (d, J= 7.9 Hz, 1H), 7.61 - 7.50 (m, 2H), 6.92 (s, 2H), 3.85 - 3.76 (m, 2H), 3.43 - 3.38 (m, 2H), 3.28 (s, 2H), 2.36 (s, 6H), 1.86 - 1.70 (m, 4H). |
| 6.57 | 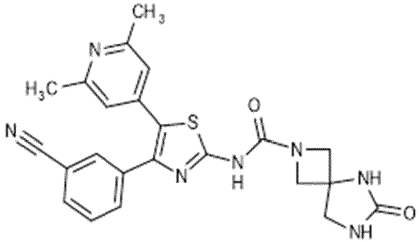 N-[4-(3-Cyanophenyl)-5-(2,6-dimethyl-4-pyridyl)thiazol-2-yl]-6-oxo-2,5,7-triazaspiro[3.4]octane-2-carboxamide | LC-MS (Method 8B): Rt 2.57 mins; |
| | | MS m/z 458.3 = [M-H]- |
| | | 1H NMR (500 MHz, DMSO-d6) δ 11.41 (s, 1H), 7.87 (s, 1H), 7.82 (d, J= 7.8 Hz, 1H), 7.70 (d, J= 7.8 Hz, 1H), 7.56 (t, J= 7.8 Hz, 1H), 7.09 (s, 1H), 6.93 (s, 2H), 6.40 (s, 1H), 4.17 - 4.02 (m, 4H), 3.54 (s, 2H), 2.36 (s, 6H). |
| 6.58 | 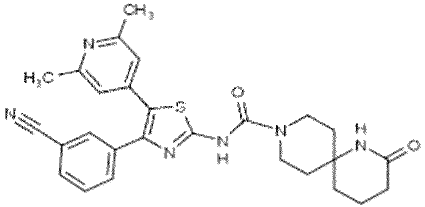 N-[4-(3-Cyanophenyl)-5-(2,6-dimethyl-4-pyridyl)thiazol-2-yl]-2-oxo-1,9-diazaspiro[5.5]undecane-9-carboxamide | LC-MS (Method 8B): Rt 3.05 mins; |
| | | MS m/z 499.4 = [M-H]- |
| | | 1H NMR (500 MHz, DMSO-d6) δ 11.24 (s, 1H), 7.90 (s, 1H), 7.83 (d, J= 7.6 Hz, 1H), 7.71 (d, J= 8.0 Hz, 1H), 7.57 (t, J= 7.8 Hz, 2H), 6.93 (s, 2H), 3.87 - 3.80 (m, 2H), 2.37 (s, 6H), 2.12 (s, 2H), 1.72 - 1.65 (m, 4H), 1.63 - 1.57 (m, 4H). 1 x CH₂ not observed. |
| 6.59 | 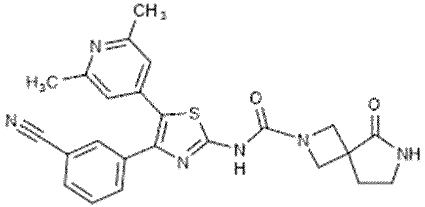 N-[4-(3-Cyanophenyl)-5-(2,6-dimethyl-4-pyridyl)thiazol-2-yl]-5-oxo-2,6-diazaspiro[3.4]octane-2-carboxamide | LC-MS (Method 8B): Rt 3.53 mins; |
| | | MS m/z 459.2 = [M+H]+ |
| | | 1H NMR (500 MHz, DMSO-d6) δ 11.43 (s, 1H), 7.90 - 7.86 (m, 2H), 7.83 (d, J= 7.7 Hz, 1H), 7.71 (d, J= 7.9 Hz, 1H), 7.56 (t, J= 7.8 Hz, 1H), 6.94 (s, 2H), 4.02 (d, J= 74.8 Hz, 4H), 3.17 (t, J= 6.7 Hz, 2H), 2.37 (s, 6H), 2.37 - 2.32 (m, 2H). |
| 6.60 | 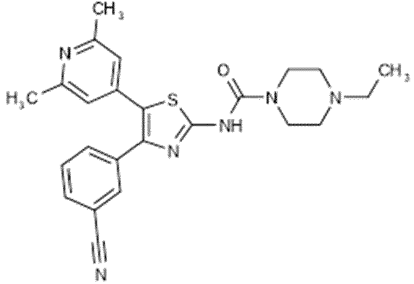 N-[4-(3-Cyanophenyl)-5-(2,6-dimethyl-4-pyridyl)thiazol-2-yl]-4-ethyl-piperazine-1-carboxamide | LC-MS (Method 8B): Rt 4.25 mins; |
| | | MS m/z 447.3 = [M+H]+ |
| | | 1H NMR (500 MHz, DMSO-d6) δ 11.27 (s, 1H), 7.89 (s, 1H), 7.83 (d, J= 7.7 Hz, 1H), 7.71 (d, J= 8.0 Hz, 1H), 7.57 (t, J= 7.8 Hz, 1H), 6.93 (s, 2H), 3.53 (t, J= 5.1 Hz, 4H), 2.40 - 2.31 (m, 12H), 1.02 (t, J= 7.1 Hz, 3H). |
| 6.61 | 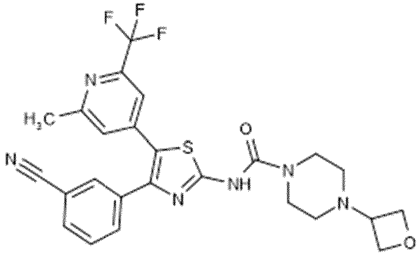 N-[4-(3-Cyanophenyl)-5-[2-methyl-6-(trifluoromethyl)-4-pyridyl]thiazol-2-yl]-4-(oxetan-3-yl)piperazine-1-carboxamide (Prepared from Intermediate H) | LC-MS (Method 8B): Rt 4.34 mins; |
| | | MS m/z 529.2 = [M-H]+ |
| | | H NMR (500 MHz, Methanol-d4) δ 7.92 (s, 1H), 7.75 - 7.69 (m, 2H), 7.52 (t, J= 7.8 Hz, 1H), 7.45 (s, 1H), 7.36 (s, 1H), 4.71 (t, J= 6.6 Hz, 2H), 4.63 (t, J= 6.2 Hz, 2H), 3.66 (t, J= 4.9 Hz, 4H), 3.55 (p, J= 6.3 Hz, 1H), 2.55 (s, 3H), 2.42 (t, J= 5.0 Hz, 4H). NH proton not observed. |
| 6.62 | 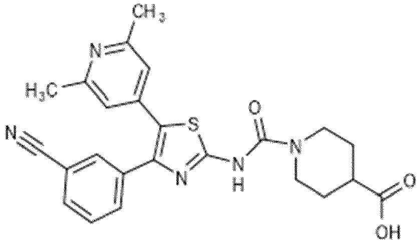 1-[[4-(3-Cyanophenyl)-5-(2,6-dimethyl-4-pyridyl)thiazol-2-yl]carbamoyl]piperidine-4-carboxylic acid | LC-MS (Method 8B): Rt 2.39 mins; |
| | | MS m/z 462.2 = [M+H]+ |
| | | 1H NMR (500 MHz, Methanol-d4) δ 7.91 (s, 1H), 7.72 (d, J= 8.0 Hz, 1H), 7.69 (d, J= 7.7 Hz, 1H), 7.49 (t, J= 7.9 Hz, 1H), 7.01 (s, 2H), 4.15 (d, J= 13.5 Hz, 2H), 3.10 (t, J= 12.4 Hz, 2H), 2.59 - 2.53 (m, 1H), 2.44 (s, 6H), 1.99 (d, J= 13.5 Hz, 2H), 1.73 - 1.62 (m, 2H). NH and OH protons not observed. |
| 6.63 | 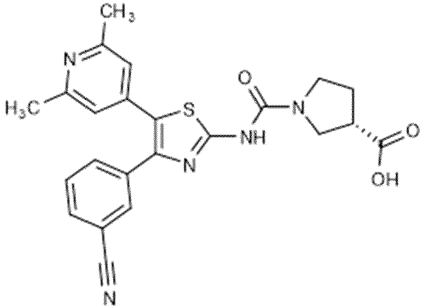 (3S)-1-[[4-(3-Cyanophenyl)-5-(2,6-dimethyl-4-pyridyl)thiazol-2-yl]carbamoyl]pyrrolidine-3-carboxylic acid | LC-MS (Method 8B): Rt 2.91 mins; |
| | | MS m/z 448.2 = [M+H]+ |
| | | 1H NMR (500 MHz, Methanol-d4) δ 7.93 (s, 1H), 7.72 (dd, J = 16.0, 7.8 Hz, 2H), 7.51 (t, J = 7.8 Hz, 1H), 7.06 (s, 2H), 3.82 - 3.72 (m, 2H), 3.65 (dt, J = 10.0, 6.8 Hz, 1H), 3.58 (dt, J = 10.1, 7.1 Hz, 1H), 3.23 (s, 1H), 2.46 (s, 6H), 2.29 (s, 2H). NH and OH protons not observed. |
| 6.64 | 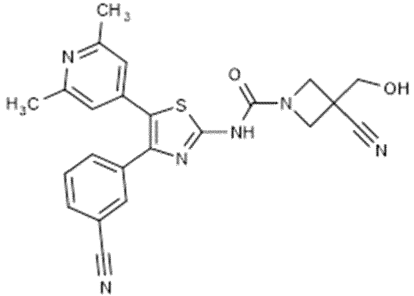 3-Cyano-N-[4-(3-cyanophenyl)-5-(2,6-dimethyl-4-pyridyl)thiazol-2-yl]-3-(hydroxymethyl)azetidine-1-carboxamide | LC-MS (Method 8B): Rt 2.98 mins; |
| | | MS m/z 445.2 = [M+H]+ |
| | | 1H NMR (500 MHz, DMSO-d6) δ 11.57 (s, 1H), 7.87 (s, 1H), 7.83 (d, J= 7.7 Hz, 1H), 7.70 (d, J= 8.3 Hz, 1H), 7.57 (t, J= 7.8 Hz, 1H), 6.94 (s, 2H), 5.77 (t, J= 5.7 Hz, 1H), 4.28 - 4.24 (m, 2H), 4.13 - 4.07 (m, 2H), 3.78 (d, J= 5.7 Hz, 2H), 2.37 (s, 6H). |
| 6.65 | 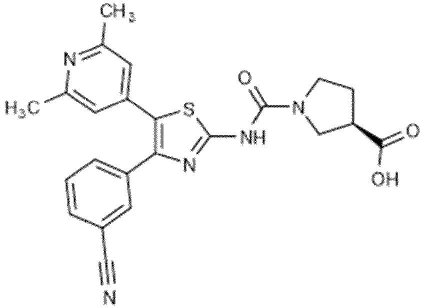 (3R)-1-[[4-(3-Cyanophenyl)-5-(2,6-dimethyl-4-pyridyl)thiazol-2-yl]carbamoyl]pyrrolidine-3-carboxylic acid | LC-MS (Method 8B): Rt 2.42 mins; |
| | | MS m/z 448.2 = [M+H]+ |
| | | 1H NMR (500 MHz, Methanol-d4) δ 7.93 (s, 1H), 7.77 - 7.69 (m, 2H), 7.51 (t, J= 7.8 Hz, 1H), 7.08 (s, 2H), 3.82 - 3.72 (m, 2H), 3.65 (dt, J= 10.3, 6.9 Hz, 1H), 3.58 (dt, J= 10.3, 7.2 Hz, 1H), 3.24 (s, 1H), 2.47 (s, 6H), 2.32 - 2.27 (m, 2H). NH and OH protons not observed. |
| 6.66 | 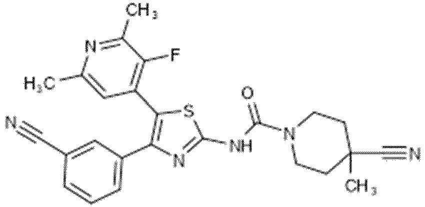 4-Cyano-N-[4-(3-cyanophenyl)-5-(3-fluoro-2,6-dimethyl-4-pyridyl)thiazol-2-yl]-4-methyl-piperidine-1-carboxamide (Prepared from Intermediate M) | LC-MS (Method 8B): Rt 3.66 mins; |
| | | MS m/z 473.3 = [M-H]- |
| | | 1H NMR (500 MHz, DMSO-d6) δ 11.38 (s, 1H), 7.88 (s, 1H), 7.80 (d, J= 7.8 Hz, 1H), 7.67 (d, J= 7.8 Hz, 1H), 7.54 (t, J= 7.8 Hz, 1H), 7.10 (d, J= 4.5 Hz, 1H), 4.23 (d, J= 13.6 Hz, 2H), 3.07 - 2.98 (m, 2H), 2.40 - 2.32 (m, 6H), 1.92 (d, J= 13.6 Hz, 2H), 1.57 - 1.49 (m, 2H), 1.37 (s, 3H). |
| 6.67 | 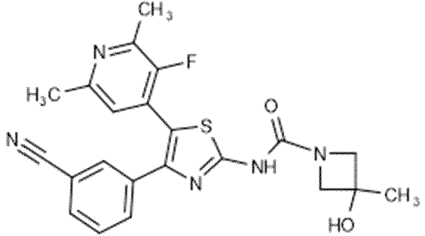 N-[4-(3-Cyanophenyl)-5-(3-fluoro-2,6-dimethyl-4-pyridyl)thiazol-2-yl]-3-hydroxy-3-methyl-azetidine-1-carboxamide (Prepared from Intermediate M) | LC-MS (Method 8B): Rt 3.06 mins; |
| | | MS m/z 436.3 = [M-H]- |
| | | 1H NMR (500 MHz, DMSO-d6) δ 11.36 (s, 1H), 7.87 (s, 1H), 7.80 (d, J= 7.7 Hz, 1H), 7.66 (d, J= 7.9 Hz, 1H), 7.54 (t, J= 7.9 Hz, 1H), 7.10 (d, J= 4.9 Hz, 1H), 5.67 (s, 1H), 3.96 - 3.81 (m, 4H), 2.40 - 2.33 (m, 6H), 1.38 (s, 3H). |
| 6.68 | 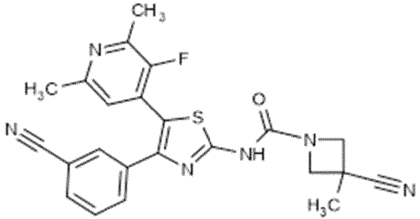 3-Cyano-N-[4-(3-cyanophenyl)-5-(3-fluoro-2,6-dimethyl-4-pyridyl)thiazol-2-yl]-3-methyl-azetidine-1-carboxamide (Prepared from Intermediate M) | LC-MS (Method 8B): Rt 3.05 mins; |
| | | MS m/z 445.3 = [M-H]- |
| | | 1H NMR (500 MHz, DMSO-d6) δ 11.61 (s, 1H), 7.86 (s, 1H), 7.81 (d, J= 7.8 Hz, 1H), 7.66 (d, J= 7.8 Hz, 1H), 7.54 (t, J= 7.8 Hz, 1H), 7.10 (d, J= 5.0 Hz, 1H), 4.41 - 4.28 (m, 2H), 4.07 - 3.95 (m, 2H), 2.41 - 2.32 (m, 6H), 1.63 (s, 3H). |
| 6.69 | 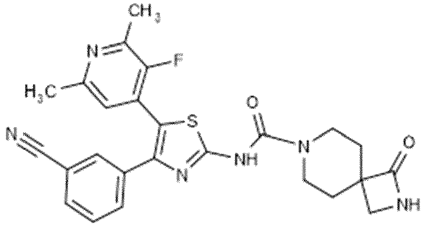 N-[4-(3-Cyanophenyl)-5-(3-fluoro-2,6-dimethyl-4-pyridyl)thiazol-2-yl]-3-oxo-2,7-diazaspiro[3.5]nonane-7-carboxamide (Prepared from Intermediate M) | LC-MS (Method 8B): Rt 3.08 mins; |
| | | MS m/z 489.3 = [M-H]- |
| | | 1H NMR (500 MHz, Methanol-d4) δ 7.93 (s, 1H), 7.74 - 7.65 (m, 2H), 7.49 (t, J = 7.9 Hz, 1H), 7.13 (d, J = 5.0 Hz, 1H), 3.99 - 3.92 (m, 2H), 3.58 - 3.52 (m, 2H), 3.26 (s, 2H), 2.53 - 2.36 (m, 6H), 2.03 - 1.95 (m, 2H), 1.94 - 1.86 (m, 2H). 2 x NH protons not observed. |
| 6.70 | 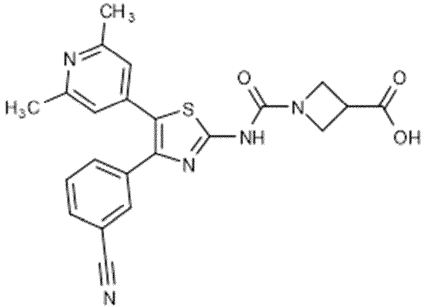 1-[[4-(3-Cyanophenyl)-5-(2,6-dimethyl-4-pyridyl)thiazol-2-yl]carbamoyl]azetidine-3-carboxylic acid | LC-MS (Method 8A): Rt 2.04 mins; |
| | | MS m/z 434.8 = [M+H]+ |
| | | 1H NMR (500 MHz, DMSO-d6) δ 12.72 (s, 1H), 11.40 (s, 1H), 7.88 (s, 1H), 7.83 (d, J= 7.7 Hz, 1H), 7.70 (d, J= 7.9 Hz, 1H), 7.56 (t, J= 7.8 Hz, 1H), 6.94 (s, 2H), 4.29 - 4.17 (m, 2H), 4.12 - 4.01 (m, 2H), 3.44 (td, J= 9.2, 4.7 Hz, 1H), 2.37 (s, 6H). |
| 6.71 | 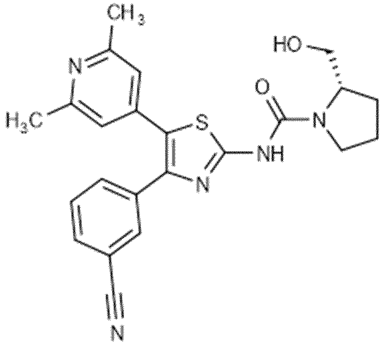 (2S)-N-[4-(3-Cyanophenyl)-5-(2,6-dimethyl-4-pyridyl)thiazol-2-yl]-2-(hydroxymethyl)pyrrolidine-1-carboxamide | LC-MS (Method 8B): Rt 3.14 mins; |
| | | MS m/z 432.4 = [M-H]- |
| | | 1H NMR (500 MHz, DMSO-d6) δ 11.09 (s, 1H), 7.89 (s, 1H), 7.82 (d, J= 7.7 Hz, 1H), 7.70 (d, J= 7.9 Hz, 1H), 7.56 (t, J= 7.8 Hz, 1H), 6.94 (s, 2H), 4.77 (v. br s, 1H), 4.01 (s, 1H), 3.51 - 3.47 (m, 4H), 2.37 (s, 6H), 1.93 - 1.81 (m, 4H). |
| 6.72 | 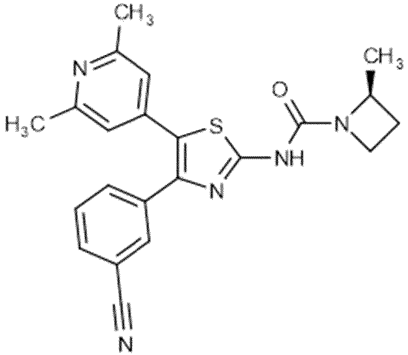 (2S)-N-[4-(3-Cyanophenyl)-5-(2,6-dimethyl-4-pyridyl)thiazol-2-yl]-2-methyl-azetidine-1-carboxamide | LC-MS (Method 8B): Rt 3.54 mins; |
| | | MS m/z 402.3 = [M-H]- |
| | | ¹H NMR (500 MHz, DMSO-d6) δ 11.15 (s, 1H), 7.88 (d, J= 1.9 Hz, 1H), 7.82 (d, J= 7.7 Hz, 1H), 7.70 (d, J= 7.9 Hz, 1H), 7.56 (t, J= 7.8 Hz, 1H), 6.93 (s, 2H), 4.47 - 4.41 (m, 1H), 4.06 (q, J= 8.2 Hz, 1H), 3.95 (t, J= 8.3 Hz, 1H), 2.36 (s, 7H), 1.81 (tt, J= 11.0, 6.5 Hz, 1H), 1.38 (d, J= 6.2 Hz, 3H). |
| 6.73 | 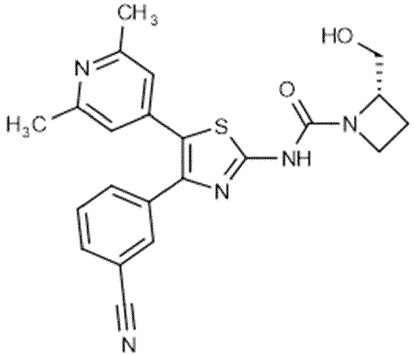 (2S)-N-[4-(3-Cyanophenyl)-5-(2,6-dimethyl-4-pyridyl)thiazol-2-yl]-2-(hydroxymethyl)azetidine-1-carboxamide | LC-MS (Method 8B): Rt 2.90 mins; |
| | | MS m/z 418.3 = [MH]- |
| | | 1H NMR (500 MHz, DMSO-d6) δ 11.06 (s, 1H), 7.86 (s, 1H), 7.82 (d, J= 7.7 Hz, 1H), 7.68 (d, J= 8.0 Hz, 1H), 7.56 (t, J= 7.8 Hz, 1H), 6.93 (s, 2H), 4.48 (s, 1H), 3.94 (q, J= 8.1 Hz, 1H), 3.86 - 3.82 (m, 1H), 3.78 - 3.71 (m, 1H), 3.66 - 3.59 (m, 1H), 2.36 (s, 6H), 2.27 - 2.18 (m, 1H), 2.03 (s, 1H). 1 proton not observed. |
| 6.74 | 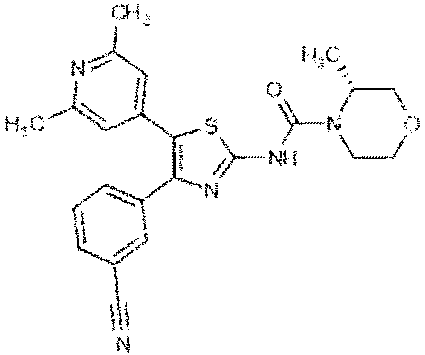 (3R)-N-[4-(3-Cyanophenyl)-5-(2,6-dimethyl-4-pyridyl)thiazol-2-yl]-3-methylmorpholine-4-carboxamide | LC-MS (Method 8B): Rt 4.07 mins; |
| | | MS m/z 434.3 = [M+H]+ |
| | | 1H NMR (500 MHz, Methanol-d4) δ 7.91 (s, 1H), 7.75 - 7.66 (m, 2H), 7.49 (t, J= 7.9 Hz, 1H), 7.01 (s, 2H), 4.28 - 4.21 (m, 1H), 3.94 (dd, J= 11.6, 3.6 Hz, 1H), 3.84 (d, J= 13.1 Hz, 1H), 3.78 - 3.72 (m, 1H), 3.67 (dd, J= 11.6, 3.2 Hz, 1H), 3.52 (dd, J= 13.1, 9.9 Hz, 1H), 3.36 (dd, J= 13.1, 3.6 Hz, 1H), 2.44 (s, 6H), 1.35 (d, J= 6.9 Hz, 3H). NH proton not observed. |
| 6.75 | 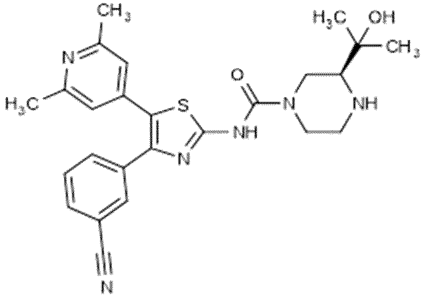 (3S)-N-[4-(3-Cyanophenyl)-5-(2,6-dimethyl-4-pyridyl)thiazol-2-yl]-3-(1-hydroxy-1-methyl-ethyl)piperazine-1-carboxamide (Prepared from Intermediate T) | LC-MS (Method 8B): Rt 1.38 mins; |
| | | MS m/z 477.3 = [M+H]+ |
| | | 1H NMR (500 MHz, Methanol-d4) δ 7.91 (t, J= 1.6 Hz, 1H), 7.73 - 7.67 (m, 2H), 7.49 (t, J= 7.8 Hz, 1H), 7.00 (s, 2H), 4.29 (apr d, J= 12.9 Hz, 1H), 4.13 (apr d, J= 12.9 Hz, 1H), 3.10 (apr d, J= 12.1 Hz, 1H), 2.97 (apr t, J= 12.5 Hz, 1H), 2.78 (apr dd, J= 12.2, 3.2 Hz, 1H), 2.74 (apr d, J= 12.1 Hz, 1H), 2.55 (apr dd, J= 11.0, 2.7 Hz, 1H), 2.44 (s, 6H), 1.28 (s, 3H), 1.25 (s, 3H). 2 x NH and 1 x OH protons not observed. |
| 6.76 | 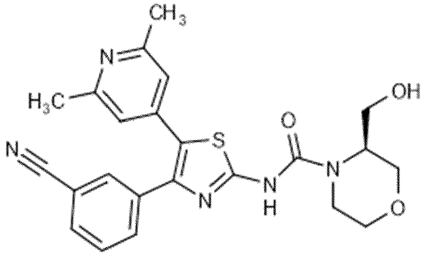 (3S)-N-[4-(3-Cyanophenyl)-5-(2,6-dimethyl-4-pyridyl)thiazol-2-yl]-3-(hydroxymethyl)morpholine-4-carboxamide | LC-MS (Method 8B): Rt 2.46 mins; |
| | | MS m/z 448.3 = [M-H]- |
| | | 1H NMR (500 MHz, DMSO-d6) δ 11.09 (s, 1H), 7.88 (s, 1H), 7.82 (d, J = 7.8 Hz, 1H), 7.70 (dt, J = 7.8, 1.5 Hz, 1H), 7.56 (t, J = 7.8 Hz, 1H), 6.93 (s, 2H), 4.92 (v br s, 1H), 4.12 (t, J = 7.2 Hz, 1H), 3.92 - 3.81 (m, 3H), 3.65 (dd, J = 10.6, 7.9 Hz, 1H), 3.60 - 3.53 (m, 1H), 3.47 (dd, J = 11.6, 3.3 Hz, 1H), 3.40 (dd, J = 12.1, 3.1 Hz, 1H), 3.15 - 3.07 (m, 1H), 2.36 (s, 6H). |
| 6.77 | 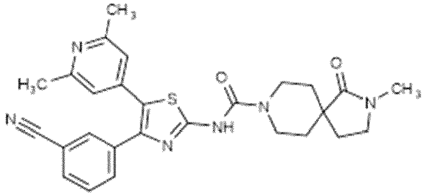 N-[4-(3-Cyanophenyl)-5-(2,6-dimethyl-4-pyridyl)thiazol-2-yl]-2-methyl-1-oxo-2,8-diazaspiro[4.5]decane-8-carboxamide | LC-MS (Method 8B): Rt 4.01 mins; |
| | | MS m/z 501.3 = [M+H]+ |
| | | 1H NMR (500 MHz, DMSO-d6) δ 11.24 (s, 1H), 7.90 (s, 1H), 7.82 (d, J= 7.7 Hz, 1H), 7.71 (d, J= 8.0 Hz, 1H), 7.56 (t, J= 7.8 Hz, 1H), 6.93 (s, 2H), 4.09 (d, J= 14.1 Hz, 2H), 3.31 - 3.28 (m, 2H), 3.10 (t, J= 12.5 Hz, 2H), 2.73 (s, 3H), 2.36 (s, 6H), 1.97 (t, J= 6.8 Hz, 2H), 1.61 (td, J= 12.8, 4.1 Hz, 2H), 1.39 (d, J= 13.3 Hz, 2H). |
| 6.78 | 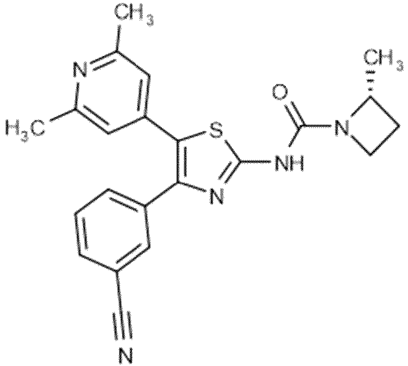 (2R)-N-[4-(3-Cyanophenyl)-5-(2,6-dimethyl-4-pyridyl)thiazol-2-yl]-2-methyl-azetidine-1-carboxamide | LC-MS (Method 8B): Rt 4.31 mins; |
| | | MS m/z 404.2 = [M+H]+ |
| | | 1H NMR (500 MHz, DMSO-d6) δ 11.15 (s, 1H), 7.88 (s, 1H), 7.82 (d, J = 7.7 Hz, 1H), 7.70 (d, J = 8.0 Hz, 1H), 7.56 (t, J = 7.9 Hz, 1H), 6.93 (s, 2H), 4.48 - 4.42 (m, 1H), 4.06 (q, J = 8.0 Hz, 1H), 3.94 (q, J = 8.2 Hz, 1H), 2.38 - 2.34 (m, 7H), 1.86 - 1.76 (m, 1H), 1.38 (d, J = 6.2 Hz, 3H). |
| 6.79 | 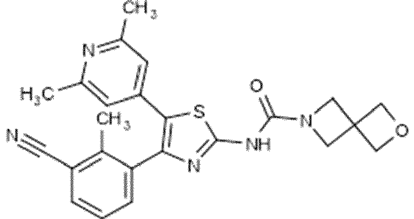 N-[4-(3-Cyano-2-methyl-phenyl)-5-(2,6-dimethyl-4-pyridyl)thiazol-2-yl]-2-oxa-6-azaspiro[3.3]heptane-6-carboxamide (Prepared from Intermediate O) | LC-MS (Method 8B): Rt 2.92 mins; |
| | | MS m/z 444.3 = [M-H]- |
| | | 1H NMR (500 MHz, DMSO-d6) δ 11.35 (s, 1H), 7.88 (dd, J = 7.8, 1.5 Hz, 1H), 7.52 (dd, J = 7.8, 1.5 Hz, 1H), 7.43 (t, J = 7.7 Hz, 1H), 6.66 (s, 2H), 4.67 (s, 4H), 4.20 (s, 4H), 2.27 (s, 6H), 2.26 (s, 3H). |
| 6.80 | 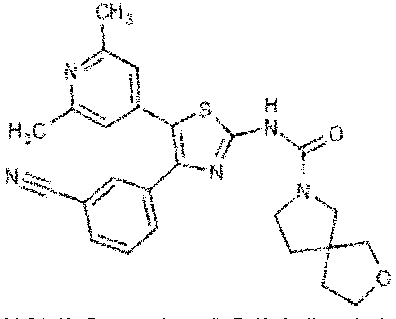 N-[4-(3-Cyanophenyl)-5-(2,6-dimethyl-4-pyridyl)thiazol-2-yl]-2-oxa-7-azaspiro[4.4]nonane-7-carboxamide | LC-MS (Method 8B): Rt 4.14 mins; |
| | | MS m/z 460.2 = [M+H]+ |
| | | 1H NMR (500 MHz, DMSO-d6) δ 11.02 (s, 1H), 7.89 (t, J = 1.5 Hz, 1H), 7.81 (dt, J = 7.9, 1.5 Hz, 1H), 7.71 (dt, J = 7.9, 1.5 Hz, 1H), 7.56 (t, J = 7.9 Hz, 1H), 6.92 (s, 2H), 3.82 - 3.75 (m, 2H), 3.58 - 3.48 (m, 4H), 3.44 - 3.39 (m, 2H), 2.36 (s, 6H), 1.95 - 1.81 (m, 4H). |
| 6.81 | 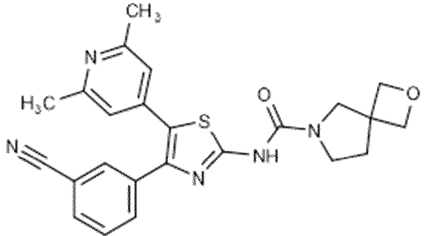 N-[4-(3-Cyanophenyl)-5-(2,6-dimethyl-4-pyridyl)thiazol-2-yl]-2-oxa-7-azaspiro[3.4]octane-7-carboxamide | LC-MS (Method 8B): Rt 3.88 mins; |
| | | MS m/z 446.2 = [M+H]+ |
| | | 1H NMR (500 MHz, DMSO-d6) δ 11.07 (s, 1H), 7.89 (d, J= 2.0 Hz, 1H), 7.82 (d, J= 7.7 Hz, 1H), 7.71 (d, J= 7.9 Hz, 1H), 7.56 (t, J= 7.8 Hz, 1H), 6.93 (s, 2H), 4.56 (d, J= 6.2 Hz, 2H), 4.49 (d, J= 6.2 Hz, 2H), 3.68 (s, 2H), 3.45 (s, 2H), 2.36 (s, 6H), 2.19 (s, 2H). |
| 6.82 | 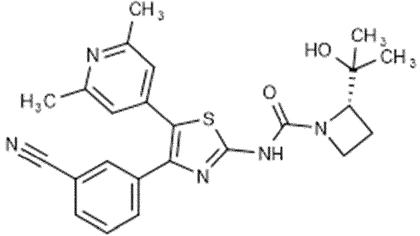 (2S)-N-[4-(3-Cyanophenyl)-5-(2,6-dimethyl-4-pyridyl)thiazol-2-yl]-2-(1-hydroxy-1-methyl-ethyl)azetidine-1-carboxamide | LC-MS (Method 8B): Rt 4.22 mins; |
| | | MS m/z 448.3 = [M+H]+ |
| | | 1H NMR (500 MHz, DMSO-d6) δ 11.37 (s, 1H), 7.85 (s, 1H), 7.81 (d, J= 7.7 Hz, 1H), 7.67 (d, J= 8.0 Hz, 1H), 7.55 (t, J= 7.8 Hz, 1H), 6.93 (s, 2H), 6.31 (s, 1H), 4.33 (t, J= 7.8 Hz, 1H), 3.92 (q, J= 8.3 Hz, 1H), 3.68 (s, 1H), 2.36 (s, 6H), 2.27 - 2.17 (m, 1H), 1.99 (p, J= 8.4 Hz, 1H), 1.27 (s, 3H), 1.10 (s, 3H). |
| 6.83 | 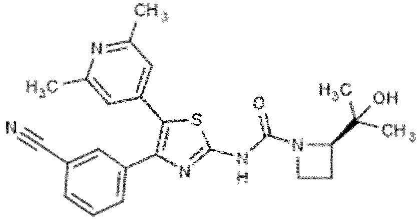 (2R)-N-[4-(3-Cyanophenyl)-5-(2,6-dimethyl-4-pyridyl)thiazol-2-yl]-2-(1-hydroxy-1-methyl-ethyl)azetidine-1-carboxamide | LC-MS (Method 8B): Rt 4.26 mins; |
| | | MS m/z 448.3 = [M+H]+ |
| | | 1H NMR (500 MHz, DMSO-d6) δ 11.34 (s, 1H), 7.85 (t, J = 1.4 Hz, 1H), 7.81 (dt, J = 7.8, 1.4 Hz, 1H), 7.66 (dt, J = 7.8, 1.5 Hz, 1H), 7.54 (t, J = 7.8 Hz, 1H), 6.92 (s, 2H), 6.31 (s, 1H), 4.32 (t, J = 7.8 Hz, 1H), 3.91 (q, J = 8.4 Hz, 1H), 3.71 - 3.64 (m, 1H), 2.36 (s, 6H), 2.25 - 2.17 (m, 1H), 2.02 - 1.94 (m, 1H), 1.26 (s, 3H), 1.09 (s, 3H). |
| 6.84 | 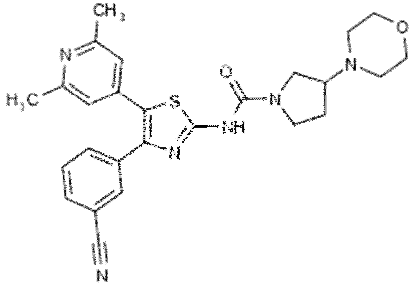 N-[4-(3-Cyanophenyl)-5-(2,6-dimethyl-4-pyridyl)thiazol-2-yl]-3-morpholino-pyrrolidine-1-carboxamide hemi formate salt | LC-MS (Method 8B): Rt 4.00 mins; |
| | | MS m/z 489.2 = [M+H]+ |
| | | 1H NMR (500 MHz, Methanol-d4) δ 8.18 (s, 0.5H), 7.91 (t, J = 1.6 Hz, 1H), 7.72 (dt, J = 7.8, 1.6 Hz, 1H), 7.69 (dt, J = 7.8, 1.6 Hz, 1H), 7.49 (t, J = 7.8 Hz, 1H), 7.04 (s, 2H), 3.84 (dd, J = 10.1, 7.1 Hz, 1H), 3.77 - 3.70 (m, 5H), 3.52 - 3.45 (m, 1H), 3.29 - 3.33 (m, 1H), 3.04 - 2.94 (m, 1H), 2.67 - 2.59 (m, 2H), 2.59 - 2.50 (m, 2H), 2.45 (s, 6H), 2.34 - 2.21 (m, 1H), 1.96 - 1.82 (m, 1H). NH proton not observed. |
| 6.85 | 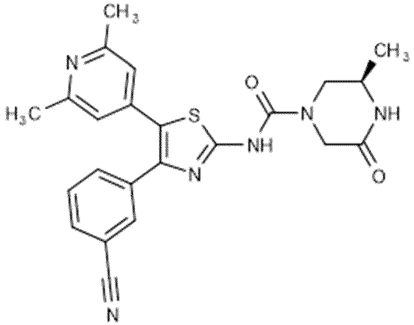 (3R)-N-[4-(3-Cyanophenyl)-5-(2,6-dimethyl-4-pyridyl)thiazol-2-yl]-3-methyl-5-oxo-piperazine-1-carboxamide | LC-MS (Method 8B): Rt 3.36 mins; |
| | | MS m/z 447.2 = [M+H]+ |
| | | 1H NMR (500 MHz, DMSO-d6) δ 11.38 (s, 1H), 8.14 (s, 1H), 7.89 (t, J= 1.6 Hz, 1H), 7.82 (dt, J= 7.8, 1.6 Hz, 1H), 7.71 (dt, J= 7.8, 1.6 Hz, 1H), 7.56 (t, J= 7.8 Hz, 1H), 6.93 (s, 2H), 4.18 (apr d, J= 17.6 Hz, 1H), 4.02 - 3.95 (m, 1H), 3.92 (apr d, J= 17.6 Hz, 1H), 3.58 - 3.49 (m, 1H), 3.22 - 3.14 (m, 1H), 2.36 (s, 6H), 1.10 (d, J= 6.4 Hz, 3H). |
| 6.86 | 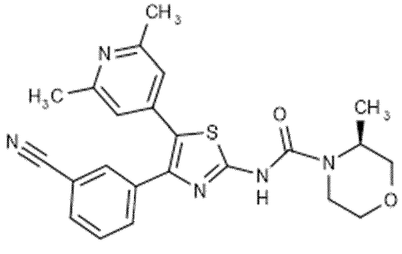 (3S)-N-[4-(3-Cyanopnenyl)-5-(2,6-dimethyl-4-pyridyl)thiazol-2-yl]-3-methylmorpholine-4-carboxamide | LC-MS (Method 8B): Rt 4.15 mins; |
| | | MS m/z 434.3 = [M+H]+ |
| | | 1H NMR (500 MHz, DMSO-d6) δ 11.23 (s, 1H), 7.89 (d, J = 1.5 Hz, 1H), 7.82 (d, J = 7.8 Hz, 1H), 7.71 (dt, J = 7.8, 1.5 Hz, 1H), 7.56 (t, J = 7.8 Hz, 1H), 6.92 (s, 2H), 4.30 - 4.23 (m, 1H), 3.91 - 3.82 (m, 2H), 3.65 (d, J = 11.5 Hz, 1H), 3.53 (dd, J = 11.5, 2.9 Hz, 1H), 3.41 - 3.36 (m, 1H), 3.17 (td, J = 13.1, 2.9 Hz, 1H), 2.36 (s, 6H), 1.21 (d, J = 6.7 Hz, 3H). |
| 6.87 | 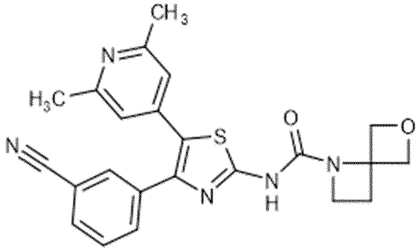 N-[4-(3-Cyanophenyl)-5-(2,6-dimethyl-4-pyridyl)thiazol-2-yl]-6-oxa-1-azaspiro[3.3]heptane-1-carboxamide | LC-MS (Method 8B): Rt 3.04 mins; |
| | | MS m/z 432.2 = [M+H]+ |
| | | 1H NMR (500 MHz, Methanol-d4) δ 7.89 (s, 1H), 7.71 (d, J = 8.0 Hz, 1H), 7.68 (d, J = 7.6 Hz, 1H), 7.49 (t, J = 7.9 Hz, 1H), 7.01 (s, 2H), 5.38 (d, J = 7.1 Hz, 2H), 4.72 (d, J = 7.1 Hz, 2H), 4.03 (t, J = 7.3 Hz, 2H), 2.61 (t, J = 7.4 Hz, 2H), 2.44 (s, 6H). NH proton not observed. |
| 6.88 | 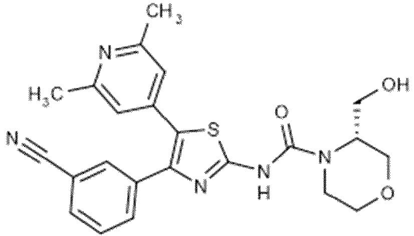 (3R)-N-[4-(3-Cyanophenyl)-5-(2,6-dimethyl-4-pyridyl)thiazol-2-yl]-3-(hydroxymethyl)morpholine-4-carboxamide | LC-MS (Method 8B): Rt 3.43 mins; |
| | | MS m/z 450.2 = [M+H]+ |
| | | 1H NMR (500 MHz, DMSO-d6) δ 11.07 (s, 1H), 7.89 (t, J= 1.8 Hz, 1H), 7.82 (dt, J= 7.7, 1.5 Hz, 1H), 7.71 (dt, J= 8.0, 1.5 Hz, 1H), 7.56 (t, J= 7.8 Hz, 1H), 6.93 (s, 2H), 4.92 (s, 1H), 4.12 (d, J= 8.3 Hz, 1H), 3.93 - 3.82 (m, 3H), 3.69 - 3.62 (m, 1H), 3.57 (t, J= 9.1 Hz, 1H), 3.48 (dd, J= 11.7, 3.3 Hz, 1H), 3.40 (td, J= 11.8, 3.1 Hz, 1H), 3.12 (td, J= 13.9, 13.2, 3.7 Hz, 1H), 2.37 (s, 6H). |
| 6.89 | 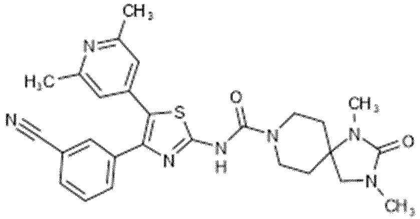 N-[4-(3-Cyanophenyl)-5-(2,6-dimethyl-4-pyridyl)thiazol-2-yl]-1,3-dimethyl-2-oxo-1,3,8-triazaspiro[4.5]decane-8-carboxamide | LC-MS (Method 8B): Rt 3.85 mins; |
| | | MS m/z 516.2 = [M+H]+ |
| | | 1H NMR (500 MHz, DMSO-d6) δ 11.30 (s, 1H), 7.89 (s, 1H), 7.83 (d, J= 7.7 Hz, 1H), 7.71 (d, J= 8.0 Hz, 1H), 7.57 (t, J= 7.8 Hz, 1H), 6.94 (s, 2H), 4.27 (d, J= 13.3 Hz, 2H), 3.25 (s, 2H), 2.99 - 2.90 (m, 2H), 2.67 (s, 3H), 2.56 (s, 3H), 2.37 (s, 6H), 1.81 (td, J= 13.1, 4.6 Hz, 2H), 1.47 (d, J= 12.8 Hz, 2H). |
| 6.90 | 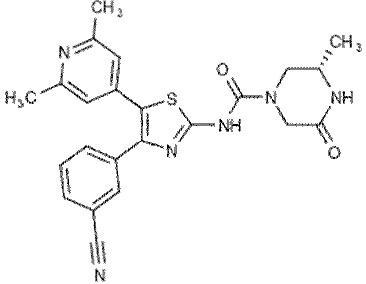 (3S)-N-[4-(3-Cyanophenyl)-5-(2,6-dimethyl-4-pyridyl)thiazol-2-yl]-3-methyl-5-oxo-piperazine-1-carboxamide | LC-MS (Method 8B): Rt 3.43 mins; |
| | | MS m/z 447.2 = [M+H]+ |
| | | 1H NMR (500 MHz, DMSO-d6) δ 11.38 (s, 1H), 8.16 (s, 1H), 7.90 (d, J= 1.8 Hz, 1H), 7.83 (dt, J= 7.8, 1.5 Hz, 1H), 7.71 (dt, J= 8.0, 1.5 Hz, 1H), 7.57 (t, J = 7.8 Hz, 1H), 6.94 (s, 2H), 4.18 (d, J= 17.6 Hz, 1H), 3.99 (d, J= 13.2 Hz, 1H), 3.93 (d, J= 17.6 Hz, 1H), 3.58 - 3.51 (m, 1H), 3.20 (dd, J= 13.5, 8.0 Hz, 1H), 2.37 (s, 6H), 1.10 (d, J= 6.4 Hz, 3H). |
| 6.91 | 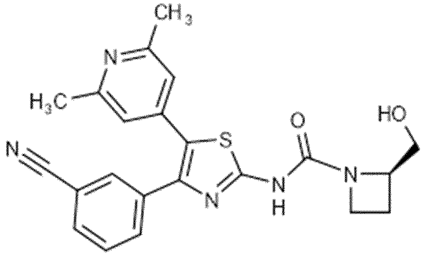 (2R)-N-[4-(3-Cyanophenyl)-5-(2,6-dimethyl-4-pyridyl)thiazol-2-yl]-2-(hydroxymethyl)azetidine-1-carboxamide | LC-MS (Method 8B): Rt 3.12 mins; |
| | | MS m/z 418.3 = [M-H]- |
| | | 1H NMR (500 MHz, DMSO-d6) δ 11.01 (s, 1H), 7.86 (d, J = 1.5 Hz, 1H), 7.82 (dt, J = 7.9, 1.5 Hz, 1H), 7.69 (dt, J = 7.9, 1.5 Hz, 1H), 7.55 (t, J = 7.9 Hz, 1H), 6.93 (s, 2H), 4.48 (s, 1H), 3.94 (q, J = 8.3 Hz, 1H), 3.84 (s, 1H), 3.75 (dd, J = 11.2, 3.5 Hz, 1H), 3.67 - 3.59 (m, 1H), 2.36 (s, 6H), 2.28 - 2.19 (m, 1H), 2.03 (s, 1H). One proton not observed |
| 6.92 | 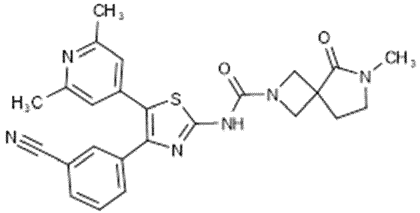 N-[4-(3-Cyanophenyl)-5-(2,6-dimethyl-4-pyridyl)thiazol-2-yl]-6-methyl-5-oxo-2,6-diazaspiro[3.4]octane-2-carboxamide | LC-MS (Method 8B): Rt 3.64 mins; |
| | | MS m/z 473.3 = [M+H]+ |
| | | 1H NMR (500 MHz, DMSO-d6) δ 11.41 (s, 1H), 7.87 (t, J = 1.5 Hz, 1H), 7.81 (dt, J = 7.7, 1.5 Hz, 1H), 7.70 (dt, J = 8.0, 1.5 Hz, 1H), 7.56 (apr t, J = 7.8 Hz, 1H), 6.93 (s, 2H), 4.09 (s, 2H), 3.94 (s, 2H), 3.27 (t, J = 6.8 Hz, 2H), 2.76 (s, 3H), 2.36 (s, 6H), 2.32 (t, J = 6.8 Hz, 2H) |
| 6.93 | 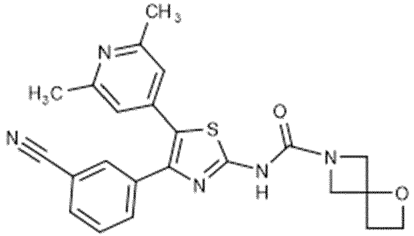 N-[4-(3-Cyanophenyl)-5-(2,6-dimethyl-4-pyridyl)thiazol-2-yl]-1-oxa-6-azaspiro[3.3]heptane-6-carboxamide | LC-MS (Method 8B): Rt 2.68 mins; |
| | | MS m/z 432.2 = [M+H]+ |
| | | 1H NMR (500 MHz, DMSO-d6) δ 11.35 (s, 1H), 7.87 (t, J= 1.5 Hz, 1H), 7.81 (dt, J= 7.8, 1.5 Hz, 1H), 7.69 (dt, J= 7.8, 1.5 Hz, 1H), 7.55 (t, J= 7.8 Hz, 1H), 6.92 (s, 2H), 4.41 (t, J= 7.5 Hz, 2H), 4.28 (d, J= 10.3 Hz, 2H), 4.12 (d, J= 10.3 Hz, 2H), 2.83 (t, J= 7.5 Hz, 2H), 2.36 (s, 6H). |
| 6.94 | 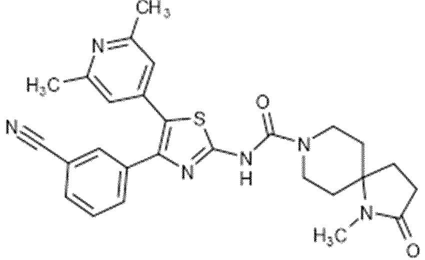 N-[4-(3-Cyanophenyl)-5-(2,6-dimethyl-4-pyridyl)thiazol-2-yl]-1-methyl-2-oxo-1,8-diazaspiro[4.5]decane-8-carboxamide | LC-MS (Method 8B): Rt 3.92 mins; |
| | | MS m/z 501.3 = [M+H]+ |
| | | 1H NMR (500 MHz, DMSO-d6) δ 11.29 (s, 1H), 7.89 (s, 1H), 7.82 (d, J= 7.8 Hz, 1H), 7.71 (d, J= 7.8 Hz, 1H), 7.56 (t, J= 7.8 Hz, 1H), 6.92 (s, 2H), 4.29 (d, J= 13.5 Hz, 2H), 2.96 (d, J= 14.5 Hz, 2H), 2.60 (s, 3H), 2.36 (s, 6H), 2.27 (t, J= 8.0 Hz, 2H), 1.97 (t, J= 8.0 Hz, 2H), 1.79 (t, J= 14.5, 10.1 Hz, 2H), 1.42 (d, J= 12.7 Hz, 2H). |
| 6.95 | 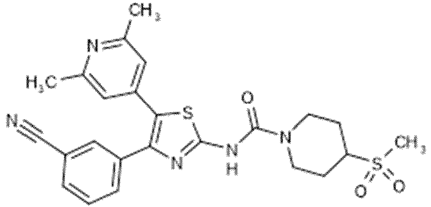 N-[4-(3-Cyanophenyl)-5-(2,6-dimethyl-4-pyridyl)thiazol-2-yl]-4-methylsulfonyl-piperidine-1-carboxamide | LC-MS (Method 8B): Rt 3.62 mins; |
| | | MS m/z 496.2 = [M+H]+ |
| | | 1H NMR (500 MHz, DMSO-d6) δ 11.32 (s, 1H), 7.89 (t, J= 1.7 Hz, 1H), 7.83 (dt, J= 7.8, 1.4 Hz, 1H), 7.71 (dt, J= 8.1, 1.4 Hz, 1H), 7.57 (t, J= 7.9 Hz, 1H), 6.93 (s, 2H), 4.36 (d, J= 13.5 Hz, 2H), 3.43 - 3.33 (m, 1H), 2.96 (s, 3H), 2.96 - 2.87 (m, 2H), 2.37 (s, 6H), 2.11 - 2.04 (m, 2H), 1.55 (qd, J= 12.4, 4.1 Hz, 2H). |
| 6.96 | 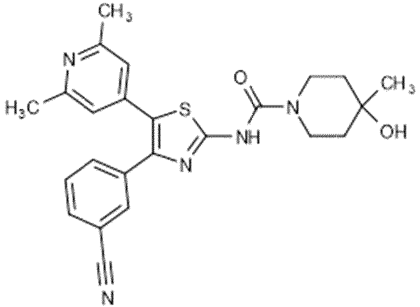 N-[4-(3-Cyanophenyl)-5-(2,6-dimethyl-4-pyridyl)thiazol-2-yl]-4-hydroxy-4-methylpiperidine-1-carboxamide | LC-MS (Method 8B): Rt 3.95 mins; |
| | | MS m/z 448.3 = [M+H]+ |
| | | 1H NMR (500 MHz, DMSO-d6) δ 11.16 (s, 1H), 7.89 (d, J= 1.8 Hz, 1H), 7.82 (dt, J= 7.8, 1.5 Hz, 1H), 7.71 (dt, J= 7.9, 1.5 Hz, 1H), 7.56 (t, J= 7.8 Hz, 1H), 6.93 (s, 2H), 4.40 (s, 1H), 3.83 (dt, J= 13.5, 4.2 Hz, 2H), 3.29 - 3.23 (m, 2H), 2.36 (s, 6H), 1.53 - 1.39 (m, 4H), 1.15 (s, 3H). |
| 6.97 | 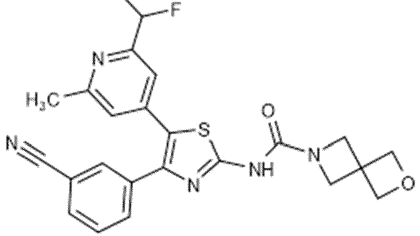 F. N-[4-(3-Cyanophenyl)-5-[2-(difluoromethyl)-6-methyl-4-pyridyl]thiazol-2-yl]-2-oxa-6-azaspiro[3.3]heptane-6-carboxamide (Prepared from Intermediate G) | LC-MS (Method 8B): Rt 3.77 mins; |
| | | MS m/z 468.2 = [M+H]+ |
| | | 1H NMR (500 MHz, DMSO-d6) δ 11.33 (br s, 1H), 7.88 (d, J= 1.8 Hz, 1H), 7.85 (dt, J= 7.8, 1.5 Hz, 1H), 7.72 (dt, J= 8.1, 1.5 Hz, 1H), 7.58 (t, J= 7.8 Hz, 1H), 7.36 (s, 1H), 7.20 (s, 1H), 6.83 (t, J= 54.9 Hz, 1H), 4.67 (s, 4H), 4.21 (s, 4H), 2.48 (s, 3H). |
| 6.98 | 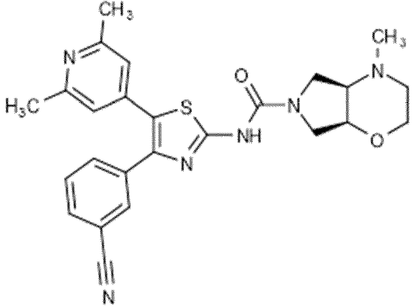 rac-(4aR,7aS)-N-[4-(3-Cyanophenyl)-5-(2,6-dimethyl-4-pyridyl)thiazol-2-yl]-4-methyl-2,3,4a,5,7,7a-hexahydropyrrolo[3,4-b][1,4]oxazine-6-carboxamide | LC-MS (Method 8B): Rt 4.00 mins; |
| | | MS m/z 475.3 = [M+H]+ |
| | | 1H NMR (500 MHz, DMSO-d6) δ 11.05 (s, 1H), 7.89 (t, J= 1.6 Hz, 1H), 7.81 (dt, J= 7.8, 1.6 Hz, 1H), 7.71 (dt, J= 7.8, 1.6 Hz, 1H), 7.56 (t, J= 7.8 Hz, 1H), 6.93 (s, 2H), 4.11 - 3.96 (m, 1H), 3.76 (dt, J= 11.2, 2.9 Hz, 1H), 3.65 - 3.35 (m, 6H), 2.62 - 2.53 (m, 1H), 2.36 (s, 7H), 2.29 (s, 3H). |
| 6.99 | 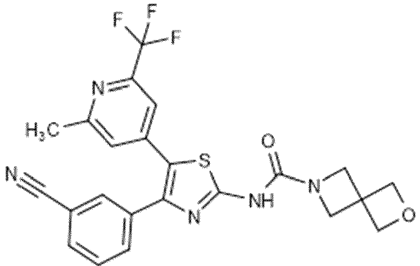 N-[4-(3-Cyanophenyl)-5-[2-methyl-6-(trifluoromethyl)-4-pyridyl]thiazol-2-yl]-2-oxa-6-azaspiro[3.3]heptane-6-carboxamide (Prepared from Intermediate H) | LC-MS (Method 8B): Rt 4.39 mins; |
| | | MS m/z 486.2 = [M+H]+ |
| | | 1H NMR (500 MHz, Methanol-d4) δ 7.90 (d, J= 1.7 Hz, 1H), 7.74 - 7.68 (m, 2H), 7.52 (t, J= 7.8 Hz, 1H), 7.44 (s, 1H), 7.36 (s, 1H), 4.82 (s, 4H), 4.31 (s, 4H), 2.55 (s, 3H). NH proton not observed. |
| 6.100 | 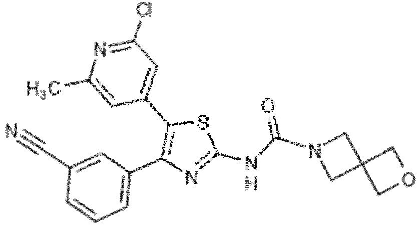 N-[5-(2-Chloro-6-methyl-4-pyridyl)-4-(3-cyanophenyl)thiazol-2-yl]-2-oxa-6-azaspiro[3.3]heptane-6-carboxamide (Prepared from Intermediate D) | LC-MS (Method 8B): Rt 4.17 mins; |
| | | MS m/z 452.1 = [M+H]+ |
| | | 1H NMR (500 MHz, DMSO-d6) δ 11.44 (s, 1H), 7.90 (t, J= 1.5 Hz, 1H), 7.85 (dt, J= 7.8, 1.5 Hz, 1H), 7.71 (dt, J= 7.8, 1.5 Hz, 1H), 7.59 (t, J= 7.8 Hz, 1H), 7.18 (s, 1H), 7.06 (s, 1H), 4.67 (s, 4H), 4.21 (s, 4H), 2.40 (s, 3H). |
| 6.101 | 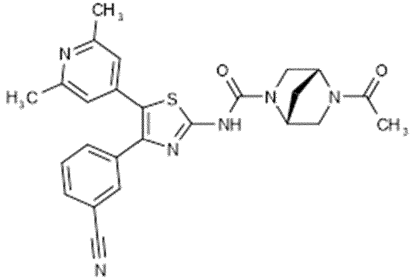 (1R,4R)-5-Acetyl-N-[4-(3-cyanophenyl)-5-(2,6-dimethyl-4-pyridyl)thiazol-2-yl]-2,5-diazabicyclo[2.2.1]heptane-2-carboxamide | LC-MS (Method 8B): Rt 3.21 mins; |
| | | MS m/z 473.2 = [M+H]+ |
| | | 1H NMR (500 MHz, Methanol-d4) δ 7.91 - 7.89 (m, 1H), 7.73 - 7.67 (m, 2H), 7.49 (t, J= 7.8 Hz, 1H), 7.01 (s, 2H), 3.68 - 3.61 (m, 2H), 3.60 - 3.56 (m, 1H), 3.53 - 3.45 (m, 3H), 2.44 (s, 6H), 2.16 (s, 1H), 2.06 (s, 1H), 2.01 (s, 3H). NH proton not observed. |
| 6.102 | 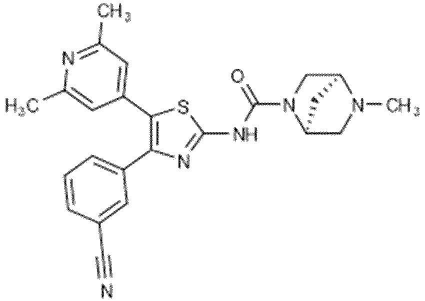 (1S,4S)-N-[4-(3-Cyanophenyl)-5-(2,6-dimethyl-4-pyridyl)thiazol-2-yl]-5-methyl-2,5-diazabicyclo[2.2.1]heptane-2-carboxamide | LC-MS (Method 8B): Rt 3.97 mins; |
| | | MS m/z 445.3 = [M+H]+ |
| | | 1H NMR (500 MHz, Methanol-d4) δ 7.92 (t, J = 1.7 Hz, 1H), 7.72 (ddt, J = 14.1, 7.8, 1.5 Hz, 2H), 7.50 (t, J = 7.8 Hz, 1H), 7.02 (s, 2H), 4.65 (s, 1H), 3.73 - 3.66 (m, 2H), 3.50 - 3.44 (m, 1H), 2.93 (d, J = 10.1 Hz, 1H), 2.88 (d, J = 10.3 Hz, 1H), 2.52 (s, 3H), 2.45 (s, 6H), 2.05 (d, J = 10.3 Hz, 1H), 1.91 (d, J = 10.4 Hz, 1H). NH proton not observed. |
| 6.103 | 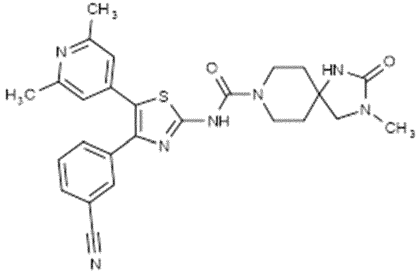 N-[4-(3-Cyanophenyl)-5-(2,6-dimethyl-4-pyridyl)thiazol-2-yl]-3-methyl-2-oxo-1,3,8-triazaspiro[4.5]decane-8-carboxamide | LC-MS (Method 8B): Rt 3.68 mins; |
| | | MS m/z 502.2 = [M+H]+ |
| | | 1H NMR (500 MHz, DMSO-d6) δ 11.27 (s, 1H), 7.89 (t, J= 1.7 Hz, 1H), 7.83 (dt, J= 7.8, 1.5 Hz, 1H), 7.71 (dt, J= 7.9, 1.5 Hz, 1H), 7.57 (t, J= 7.8 Hz, 1H), 6.93 (s, 2H), 6.92 (s, 1H), 3.68 - 3.49 (m, 4H), 3.16 (s, 2H), 2.63 (s, 3H), 2.37 (s, 6H), 1.68 - 1.52 (m, 4H). |
| 6.104 | 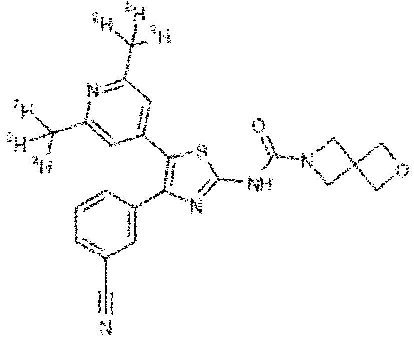 N-[5-[2,6-Bis(trideuteriomethyl)-4-pyridyl]-4-(3-cyanophenyl)thiazol-2-yl]-2-oxa-6-azaspiro[3.3]heptane-6-carboxamide (Prepared from Intermediate N) | LC-MS (Method 8B): Rt 2.83 mins; |
| | | MS m/z 438.3 = [M+H]+ |
| | | 1H NMR (500 MHz, DMSO-d6) δ 11.34 (br s, 1H), 7.87 (br t, J = 1.6 Hz, 1H), 7.81 (br dt, J = 7.7, 1.6 Hz, 1H), 7.69 (br dt, J = 8.0, 1.6 Hz, 1H), 7.55 (apr t, J = 7.8 Hz, 1H), 6.92 (s, 2H), 4.67 (s, 4H), 4.20 (s, 4H) |
| 6.105 | 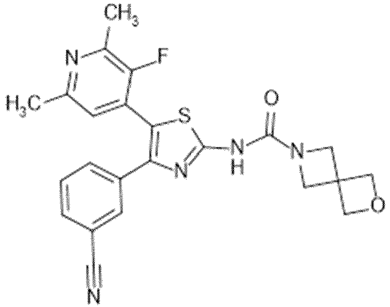 N-[4-(3-Cyanophenyl)-5-(3-fluoro-2,6-dimethyl-4-pyridyl)thiazol-2-yl]-2-oxa-6-azaspiro[3.3]heptane-6-carboxamide (Prepared from Intermediate M) | LC-MS (Method 8B): Rt 3.02 mins; |
| | | MS m/z 450.1 = [M+H]+ |
| | | 1H NMR (500 MHz, DMSO-d6) δ 11.41 (s, 1H), 7.86 (d, J = 1.9 Hz, 1H), 7.80 (dd, J = 7.7, 1.7 Hz, 1H), 7.67 - 7.64 (m, 1H), 7.54 (t, J = 7.8 Hz, 1H), 7.09 (d, J = 5.0 Hz, 1H), 4.67 (s, 4H), 4.21 (s, 4H), 2.37 (s, 3H), 2.36 (d, J = 3.1 Hz, 3H). |
| 6.106 | 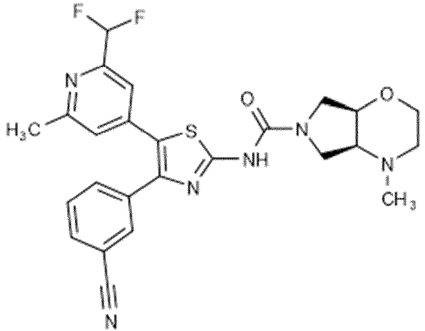 rac-(4aS,7aR)-N-[4-(3-Cyanophenyl)-5-[2-(difluoromethyl)-6-methyl-4-pyridyl]thiazol-2-yl]-4-methyl-2,3,4a,5,7,7a-hexahydropyrrolo[3,4-b][1,4]oxazine-6-carboxamide (Prepared from Intermediate G) | LC-MS (Method 8B): Rt 3.44 mins; |
| | | MS m/z 511.1 = [M+H]+ |
| | | 1H NMR (500 MHz, Methanol-d4) δ 7.81 (t,J= 1.7 Hz, 1H), 7.61 (dt, J= 7.3, 1.5 Hz, 2H), 7.40 (t, J= 7.8 Hz, 1H), 7.24 (s, 1H), 7.20 (s, 1H), 6.50 (t, J= 55.2 Hz, 1H), 4.16 - 3.93 (m, 1H), 3.79 (dt, J= 11.6, 2.7 Hz, 1H), 3.68 - 3.27 (m, 6H), 2.65 (td, J= 11.6, 3.4 Hz, 1H), 2.42 (s, 3H), 2.39 (s, 1H), 2.34 (s, 3H). NH proton not observed. |
| 6.107 | 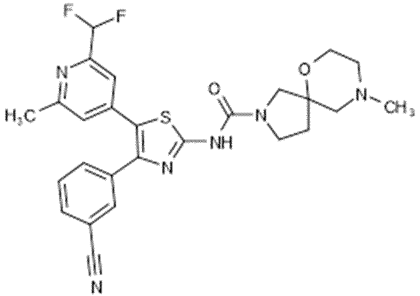 N-[4-(3-Cyanophenyl)-5-[2-(difluoromethyl)-6-methyl-4-pyridyl]thiazol-2-yl]-9-methyl-6-oxa-2,9-diazaspiro[4.5]decane-2-carboxamide (Prepared from Intermediate G) | LC-MS (Method 8B): Rt 3.41 mins; |
| | | MS m/z 525.4 = [M+H]+ |
| | | 1H NMR (500 MHz, DMSO-d6) δ 11.17 (s, 1H), 7.91 (s, 1H), 7.86 (d, J= 7.8 Hz, 1H), 7.74 (d, J= 8.0 Hz, 1H), 7.59 (t, J= 7.8 Hz, 1H), 7.38 (s, 1H), 7.22 (s, 1H), 6.84 (t, J= 54.9 Hz, 1H), 3.78 - 3.38 (m, 6H), 2.49 (s, 3H), 2.44 - 2.20 (m, 5H), 2.17 (s, 3H), 1.91 (s, 1H). |
| 6.108 | 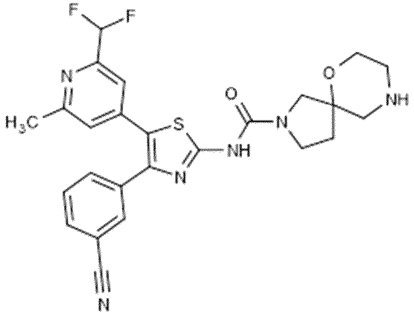 N-[4-(3-Cyanophenyl)-5-[2-(difluoromethyl)-6-methyl-4-pyridyl]thiazol-2-yl]-6-oxa-2,9-diazaspiro[4.5]decane-2-carboxamide (Prepared from Intermediate G) | LC-MS (Method 8B): Rt 2.81 mins; |
| | | MS m/z 511.1 = [M+H]+ |
| | | 1H NMR (500 MHz, Methanol-d4) δ 7.93 (t, J= 1.7 Hz, 1H), 7.73 (ddt, J= 7.8, 3.9, 1.3 Hz, 2H), 7.52 (t, J= 7.8 Hz, 1H), 7.36 (s, 1H), 7.32 (d, J= 1.6 Hz, 1H), 6.62 (t, J= 55.2 Hz, 1H), 3.84 - 3.72 (m, 3H), 3.71 - 3.58 (m, 2H), 3.44 (d, J= 11.4 Hz, 1H), 2.93 (q, J= 12.6 Hz, 2H), 2.86 (t, J= 4.9 Hz, 2H), 2.54 (s, 3H), 2.28 (s, 1H), 1.99 (s, 1H). 2 x NH protons not observed. |
| 6.109 | 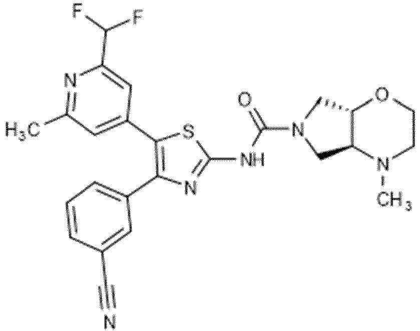 rac-(4aS,7aS)-N-[4-(3-Cyanophenyl)-5-[2-(difluoromethyl)-6-methyl-4-pyridyl]thiazol-2-yl]-4-methyl-2,3,4a,5,7,7a-hexahydropyrrolo[3,4-b][1,4]oxazine-6-carboxamide (Prepared from Intermediate G) | LC-MS (Method 8B): Rt 2.85 mins; |
| | | MS m/z 511.1 = [M+H]+ |
| | | 1H NMR (500 MHz, DMSO-d6) δ 11.20 (s, 1H), 7.91 (d, J= 2.0 Hz, 1H), 7.86 (d, J= 7.7 Hz, 1H), 7.74 (d, J= 8.0 Hz, 1H), 7.59 (t, J= 7.8 Hz, 1H), 7.38 (s, 1H), 7.22 (s, 1H), 6.84 (t, J= 54.9 Hz, 1H), 4.01 - 3.71 (m, 3H), 3.65 (td, J= 11.7, 2.7 Hz, 1H), 3.56 (s, 1H), 3.25 (d, J= 8.1 Hz, 1H), 3.13 (s, 1H), 2.97 (d, J= 18.3 Hz, 1H), 2.73 (d, J= 11.8 Hz, 1H), 2.49 (s, 3H), 2.17 (s, 3H), 2.14 - 2.05 (m, 1H). |
| 6.110 | 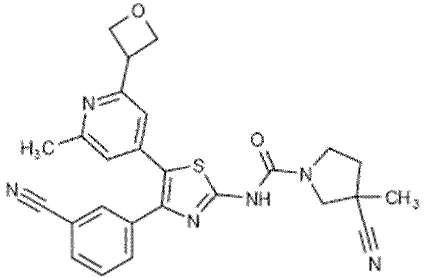 3-Cyano-N-[4-(3-cyanophenyl)-5-[2-methyl-6-(oxetan-3-yl)-4-pyridyl]thiazol-2-yl]-3-methyl-pyrrolidine-1-carboxamide (Prepared from Intermediate L) | LC-MS (Method 8B): Rt 2.81 mins; |
| | | MS m/z 485.1 = [M+H]+ (97% @ 254nm) |
| | | 1H NMR (500 MHz, DMSO-d6) δ 11.26 (s, 1H), 7.90 (br t, J = 1.6 Hz, 1H), 7.83 (br dt, J = 7.7, 1.6 Hz, 1H), 7.73 (br dt, J = 7.9, 1.6 Hz, 1H), 7.57 (apr t, J = 7.8 Hz, 1H), 7.07 (s, 1H), 6.99 (s, 1H), 4.81 (dd, J = 8.5, 5.6 Hz, 2H), 4.66 (dd, J = 6.9, 5.6 Hz, 2H), 4.27 (tt, J = 8.5, 6.9 Hz, 1H), 3.97 - 3.89 (m, 1H), 3.71 - 3.64 (m, 1H), 3.64 - 3.56 (m, 1H), 3.45 - 3.38 (m, 1H), 2.45 (s, 3H), 2.42 - 2.37 (m, 1H), 2.11 - 2.03 (m, 1H), 1.47 (s, 3H). |
| 6.111 | 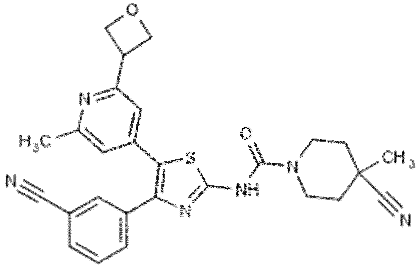 4-Cyano-N-[4-(3-cyanophenyl)-5-[2-methyl-6-(oxetan-3-yl)-4-pyridyl]thiazol-2-yl]-4-methyl-piperidine-1-carboxamide (Prepared from Intermediate L) | LC-MS (Method 8B): Rt 3.05 mins; |
| | | MS m/z 499.2 = [M+H]+ |
| | | 1H NMR (500 MHz, DMSO-d6) δ 11.34 (s, 1H), 7.90 (t, J= 1.8 Hz, 1H), 7.84 (d, J= 7.7 Hz, 1H), 7.73 (dt, J= 8.0, 1.5 Hz, 1H), 7.58 (t, J= 7.8 Hz, 1H), 7.07 (s, 1H), 6.99 (s, 1H), 4.82 (dd, J= 8.5, 5.6 Hz, 2H), 4.66 (t, J= 6.2 Hz, 2H), 4.33 - 4.26 (m, 1H), 4.23 (d, J= 13.8 Hz, 2H), 3.04 (t, J= 13.1 Hz, 2H), 2.45 (s, 3H), 1.93 (d, J= 13.7 Hz, 2H), 1.54 (td, J= 13.0, 3.8 Hz, 2H), 1.38 (s, 3H). |
| 6.112 | 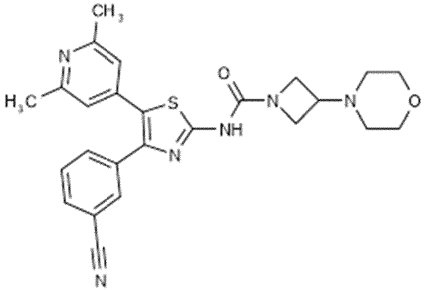 N-[4-(3-Cyanophenyl)-5-(2,6-dimethyl-4-pyridyl)thiazol-2-yl]-3-morpholino-azetidine-1-carboxamide | LC-MS (Method 8B): Rt 2.72 mins; |
| | | MS m/z 475.1 = [M+H]+ |
| | | 1H NMR (500 MHz, Methanol-d4) δ 7.89 (s, 1H), 7.70 (d, J= 8.0 Hz, 1H), 7.68 (d, J= 7.7 Hz, 1H), 7.48 (t, J= 7.8 Hz, 1H), 7.00 (s, 2H), 4.17 (t, J= 8.1 Hz, 2H), 4.05 - 3.93 (m, 2H), 3.77 - 3.64 (m, 4H), 3.29 - 3.23 (m, 1H), 2.47 - 2.44 (m, 4H), 2.43 (s, 6H). NH proton not observed. |
| 6.113 | 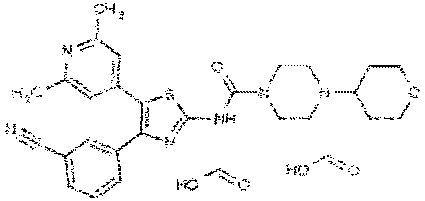 N-[4-(3-Cyanophenyl)-5-(2,6-dimethyl-4-pyridyl)thiazol-2-yl]-4-tetrahydropyran-4-yl-piperazine-1-carboxamide diformate salt | LC-MS (Method 7B): Rt 2.68 mins; |
| | | MS m/z 503.4 = [M+H]+ |
| | | 1H NMR (500 MHz, DMSO-d6) δ 11.27 (s, 1H), 8.14 (s, 2H), 7.89 (t, J = 1.5 Hz, 1H), 7.82 (dt, J = 7.7, 1.3 Hz, 1H), 7.70 (dt, J = 8.0, 1.4 Hz, 1H), 7.56 (t, J = 7.8 Hz, 1H), 6.93 (s, 2H), 3.88 (dd, J = 11.0, 3.5 Hz, 2H), 3.53 (s, 4H), 3.27 (t, J = 11.0 Hz, 2H), 2.43 (tt, J = 11.3, 3.6 Hz, 1H), 2.36 (s, 6H), 1.73 - 1.67 (m, 2H), 1.41 (qd, J = 12.2, 4.4 Hz, 2H). 2 x CH₂ signals not observed. |
| 6.114 | 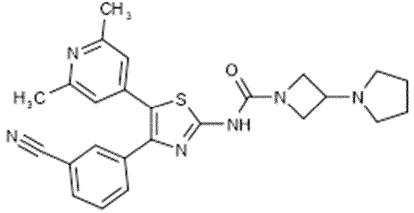 N-[4-(3-Cyanophenyl)-5-(2,6-dimethyl-4-pyridyl)thiazol-2-yl]-3-pyrrolidin-1-yl-azetidine-1-carboxamide | LC-MS (Method 7B): Rt 2.86 mins; |
| | | MS m/z 459.4 = [M+H]+ |
| | | 1H NMR (400 MHz, DMSO-d6) δ 11.29 (s, 1H), 7.87 (t, J = 1.6 Hz, 1H), 7.81 (dt, J = 7.8, 1.3 Hz, 1H), 7.70 (dt, J = 8.1, 1.5 Hz, 1H), 7.55 (dd, J = 7.8 Hz, 1H), 6.92 (s, 2H), 4.16 - 4.01 (m, 2H), 3.90 - 3.82 (m, 2H), 3.30 - 3.24 (m, 1H), 2.46 - 2.40 (m, 4H), 2.36 (s, 6H), 1.74 - 1.67 (m, 4H). |
| 6.115 | 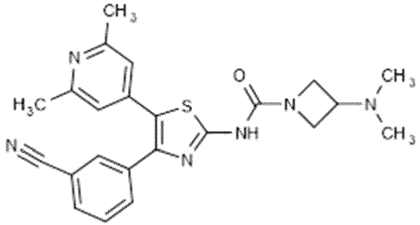 N-[4-(3-Cyanophenyl)-5-(2,6-dimethyl-4-pyridyl)thiazol-2-yl]-3-(dimethylamino)azetidine-1-carboxamide | LC-MS (Method 7B): Rt 2.58 mins; |
| | | MS m/z 433.3 = [M+H]+ |
| | | 1H NMR (400 MHz, DMSO-d6) δ 11.30 (s, 1H), 7.87 (t, J = 1.5 Hz, 1H), 7.81 (dt, J = 7.7, 1.2 Hz, 1H), 7.70 (dt, J = 7.8, 1.3 Hz, 1H), 7.56 (t, J = 7.8 Hz, 1H), 6.93 (s, 2H), 4.10 - 4.01 (m, 2H), 3.86 - 3.78 (m, 2H), 3.13 - 3.06 (m, 1H), 2.36 (s, 6H), 2.10 (s, 6H). |
| 6.116 | 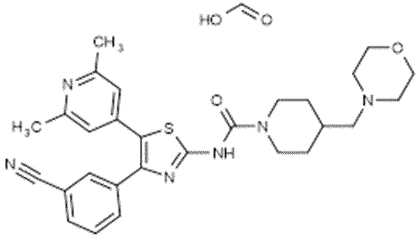 N-[4-(3-Cyanophenyl)-5-(2,6-dimethyl-4-pyridyl)thiazol-2-yl]-4-(morpholinomethyl)piperidine-1-carboxamide formate salt | LC-MS (Method 7B): Rt 3.38 mins; |
| | | MS m/z 517.3 = [M+H]+ |
| | | 1H NMR (500 MHz, DMSO-d6) δ 11.16 (s, 1H), 8.14 (s, 1H), 7.89 (t, J = 1.5 Hz, 1H), 7.82 (dt, J = 7.7, 1.3 Hz, 1H), 7.70 (dt, J = 8.0, 1.3 Hz, 1H), 7.56 (dd, J = 7.8 Hz, 1H), 6.92 (s, 2H), 4.22 - 4.15 (m, 2H), 3.61 - 3.53 (m, 4H), 2.89 - 2.79 (m, 2H), 2.36 (s, 6H), 2.35 - 2.27 (m, 4H), 2.16 - 2.11 (m, 2H), 1.81 - 1.69 (m, 3H), 1.07 - 0.97 (m, 2H). |
| 6.120 | 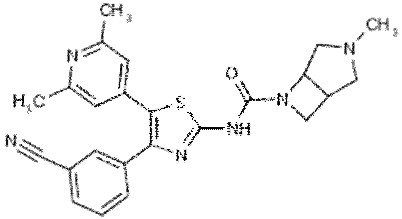 N-[4-(3-Cyanophenyl)-5-(2,6-dimethyl-4-pyridyl)thiazol-2-yl]-3-methyl-3,6-diazabicyclo[3.2.0]heptane-6-carboxamide | LC-MS (Method 7A): Rt 0.95 mins; |
| | | MS m/z 445.0 = [M+H]+ |
| | | 1H NMR (500 MHz, Methanol-d4) δ 7.89 (t, J = 1.7 Hz, 1H), 7.71 (dt, J = 7.9, 1.4 Hz, 1H), 7.68 (dt, J = 7.8, 1.4 Hz, 1H), 7.48 (dd, J = 7.9, 7.9 Hz, 1H), 7.00 (s, 2H), 4.22 - 4.15 (m, 1H), 3.87 - 3.82 (m, 1H), 3.40 (d, J = 11.3 Hz, 1H), 3.18 - 3.12 (m, 1H), 3.08 (d, J = 10.3 Hz, 1H), 2.45 (s, 3H), 2.43 (s, 6H), 2.18 (dd, J = 10.4, 5.9 Hz, 1H), 2.11 (dd, J = 11.3, 4.0 Hz, 1H). NH proton and 1 x CH proton not observed. |
| 6.121 | 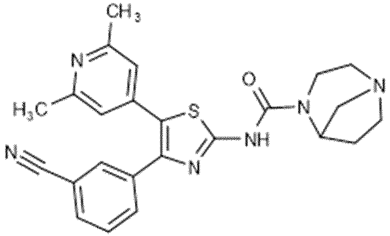 N-[4-(3-Cyanophenyl)-5-(2,6-dimethyl-4-pyridyl)thiazol-2-yl]-1,4-diazabicyclo[3.2.1]octane-4-carboxamide | LC-MS (Method 7A): Rt 0.91 mins; |
| | | MS m/z 445.3 = [M+H]+ |
| | | 1H NMR (500 MHz, Methanol-d4) δ 7.90 (t, J = 1.8 Hz, 1H), 7.71 (dt, J = 7.9, 1.5 Hz, 1H), 7.68 (dt, J = 7.8, 1.4 Hz, 1H), 7.48 (dd, J = 7.8, 7.8 Hz, 1H), 6.99 (s, 2H), 4.82 - 4.79 (m, 1H), 3.81 (dd, J = 13.6, 5.6 Hz, 1H), 3.38 - 3.32 (m, 1H), 3.14 - 3.07 (m, 1H), 3.06 - 2.97 (m, 3H), 2.79 (dd, J = 13.5, 4.8 Hz, 1H), 2.68 - 2.63 (m, 1H), 2.43 (s, 6H), 2.16 - 2.08 (m, 1H), 2.02 - 1.94 (m, 1H). NH proton not observed. |

### Example 7

### N-[4-(3-Cyanophenyl)-5-(2,6-dimethyl-4-pyridyl)thiazol-2-yl]piperazine-1-carboxamide

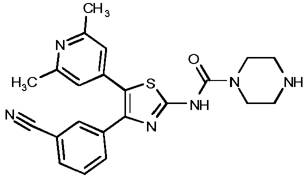

### Step 1: tert-Butyl 4-[[4-(3-cyanophenyl)-5-(2,6-dimethyl-4-pyridyl)thiazol-2-yl]carbamoyl]piperazine-1-carboxylate

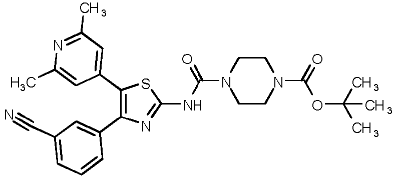

A solution of 3-[2-amino-5-(2,6-dimethyl-4-pyridyl)thiazol-4-yl]benzonitrile (Intermediate C) (50 mg, 0.16 mmol) and CDI (40 mg, 0.24 mmol) in DMA (0.5 mL) was stirred at 50 °C for 18 h. The resulting mixture was treated with *tert*-butyl piperazine-1-carboxylate (61 mg, 0.33 mmol) and stirring continued at 50 °C for 1 h. After cooling to room temperature, the crude mixture was diluted with water (10 mL) and the precipitate was collected by vacuum filtration. The solid was dissolved in DCM, filtered through a phase separating cartridge and concentrated *in vacuo.* Purification of the crude product by chromatography on silica eluting with 0-100% EtOAc in DCM afforded the title compound.
LC-MS (Method 7A): Rt 2.16 mins; MS m/z 519.3 = [M+H]+
¹H NMR (500 MHz, DMSO-d6) δ 11.30 (s, 1H), 7.89 (t, *J =* 1.4 Hz, 1H), 7.82 (d, *J =* 7.7 Hz, 1H), 7.71 (dt, *J* = 8.0, 1.2 Hz, 1H), 7.56 (t, *J =* 7.8 Hz, 1H), 6.93 (s, 2H), 3.55 - 3.49 (m, 4H), 3.39 - 3.34 (m, 4H), 2.36 (s, 6H), 1.42 (s, 9H).

### Step 2: N-[4-(3-Cyanophenyl)-5-(2,6-dimethyl-4-pyridyl)thiazol-2-yl]piperazine-1-carboxamide

A solution of *tert*-butyl 4-[[4-(3-cyanophenyl)-5-(2,6-dimethyl-4-pyridyl)thiazol-2-yl]carbamoyl]piperazine-1-carboxylate (step 1)(70 mg, 0.14 mmol) in DCM (2 mL), MeOH (2 drops) and 4M HCI in 1,4-dioxane (338 µL, 1.35 mmol) was stirred at room temperature for 22 h. The resulting mixture was concentrated *in vacuo* and purification of the crude product by preparative HPLC using basic conditions, early elution method to afford the title compound.
LC-MS (Method 7A): Rt 0.94 mins; MS m/z 419.2 = [M+H]+
¹H NMR (400 MHz, DMSO-d6) δ 7.88 (s, 1H), 7.81 (d, *J* = 7.7 Hz, 1H), 7.71 (d, *J =* 8.0 Hz, 1H), 7.56 (t, *J =* 7.8 Hz, 1H), 6.92 (s, 2H), 3.50 - 3.43 (m, 4H), 2.74 - 2.68 (m, 4H), 2.36 (s, 6H). 2 x NH protons not observed.

### Example 7.1

### (3R)-N-[4-(3-Cyanophenyl)-5-(2,6-dimethyl-4-pyridyl)thiazol-2-yl]-3-methyl-piperazine-1-carboxamide

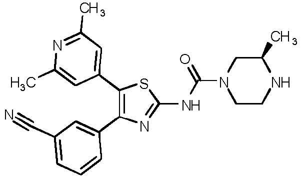

### Step 1: tert-Butyl (2R)-4-[[4-(3-cyanophenyl)-5-(2,6-dimethyl-4-pyridyl)thiazol-2-yl]carbamoyl]-2-methyl-piperazine-1-carboxylate

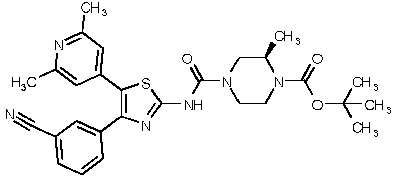

The title compound was prepared from 3-[2-amino-5-(2,6-dimethyl-4-pyridyl)thiazol-4-yl]benzonitrile (Intermediate C) and *tert-butyl* (2R)-2-methylpiperazine-1-carboxylate analogously to Example 7 step 1.
LC-MS (Method 2A): Rt 1.07 mins; MS m/z 533.3 = [M+H]+
¹H NMR (400 MHz, DMSO-d6) δ 11.30 (s, 1H), 7.89 (t, *J* = 1.5 Hz, 1H), 7.82 (dt, *J* = 7.7, 1.7 Hz, 1H), 7.71 (dt, *J* = 8.0, 1.3 Hz, 1H), 7.56 (t, *J =* 7.8 Hz, 1H), 6.93 (s, 2H), 4.20 - 4.09 (m, 2H), 4.02 (d, *J =* 13.7 Hz, 1H), 3.78 - 3.69 (m, 1H), 3.14 (dd, *J* = 13.7, 4.1 Hz, 1H), 3.09 - 3.00 (m, 1H), 2.99 - 2.89 (m, 1H), 2.36 (s, 6H), 1.42 (s, 9H), 1.07 (d, *J* = 6.7 Hz, 3H).

### Step 2: (3R)-N-[4-(3-Cyanophenyl)-5-(2,6-dimethyl-4-pyridyl)thiazol-2-yl]-3-methylpiperazine-1-carboxamide

The title compound was prepared from *tert-butyl* (2R)-4-[[4-(3-cyanophenyl)-5-(2,6-dimethyl-4-pyridyl)thiazol-2-yl]carbamoyl]-2-methyl-piperazine-1-carboxylate and 4M HCI in 1,4-dioxane analogously to Example 7 step 2.
LC-MS (Method 7A): Rt 1.00 mins; MS m/z 433.2 = [M+H]+
¹H NMR (400 MHz, DMSO-d6) δ 7.89 (t, *J* = 1.5 Hz, 1H), 7.82 (dt, *J* = 7.7, 1.3 Hz, 1H), 7.73 - 7.69 (m, 1H), 7.56 (t, *J =* 7.8 Hz, 1H), 6.93 (s, 2H), 4.18 - 4.08 (m, 2H), 3.06 (d, *J* = 11.6 Hz, 1H), 3.02 - 2.91 (m, 2H), 2.88 (s, 1H), 2.82 - 2.73 (m, 1H), 2.36 (s, 6H), 1.08 (d, *J* = 6.*3* Hz, 3H). 2x NH protons not observed.

### Example 7.2

### 4-Amino-4-cyano-N-[4-(3-cyanophenyl)-5-(2,6-dimethyl-4-pyridyl)thiazol-2-yl]piperidine-1-carboxamide

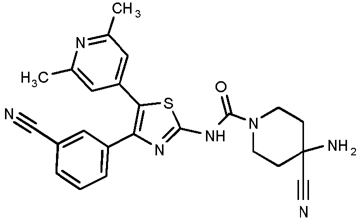

### Step 1: tert-Butyl N-[4-cyano-1-[[4-(3-cyanophenyl)-5-(2,6-dimethyl-4-pyridyl)thiazol-2-yl]carbamoyl]-4-piperidyl]carbamate

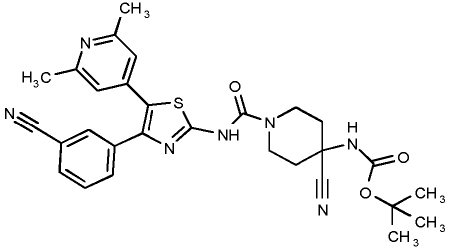

The title compound was prepared from 3-[2-amino-5-(2,6-dimethyl-4-pyridyl)thiazol-4-yl]benzonitrile (Intermediate C) and *tert*-butyl N-(4-cyano-4-piperidyl)carbamate analogously to Example 7 step 1.
LC-MS (Method 5B): Rt 2.95 mins; MS m/z 558.2 = [M+H]+
¹H NMR (500 MHz, DMSO-*d*₆) δ 11.34 (s, 1H), 7.88 (s, 1H), 7.82 (d, *J =* 7.9 Hz, 1H), 7.77 (s, 1H), 7.70 (d, *J* = 7.9 Hz, 1H), 7.56 (t, *J =* 7.9 Hz, 1H), 6.92 (s, 2H), 3.94 - 3.86 (m, 2H), 2.36 (s, 6H), 2.23 - 2.17 (m, 2H), 1.87 - 1.81 (m, 2H), 1.43 (s, 9H). 1 x CH₂ signal not observed.

### Step 2: 4-Amino-4-cyano-N-[4-(3-cyanophenyl)-5-(2,6-dimethyl-4-pyridyl)thiazol-2-yl]piperidine-1-carboxamide

The title compound was prepared from *tert*-butyl N-[4-cyano-1-[[4-(3-cyanophenyl)-5-(2,6-dimethyl-4-pyridyl)thiazol-2-yl]carbamoyl]-4-piperidyl]carbamate and 4M HCI in 1,4-dioxane analogously to Example 7 step 2.
LC-MS (Method 8B): Rt 3.30 mins; MS m/z 458.3 = [M+H]+
¹H NMR (500 MHz, DMSO-*d*₆) δ 11.31 (s, 1H), 7.88 (s, 1H), 7.82 (d, *J* = 7.7 Hz, 1H), 7.70 (d, *J* = 7.7 Hz, 1H), 7.56 (t, *J* = 7.7 Hz, 1H), 6.92 (s, 2H), 3.97 - 3.77 (m, 3H), 2.77 - 2.65 (m, 2H), 2.46 - 2.40 (m, 1H), 2.36 (s, 6H), 2.02 - 1.86 (m, 2H), 1.70 - 1.53 (m, 2H).

### Example 7.3

### N-[4-(3-Cyanophenyl)-5-(2,6-dimethyl-4-pyridyl)thiazol-2-yl]-1-oxa-4,9-diazaspiro[5.5]undecane-9-carboxamide

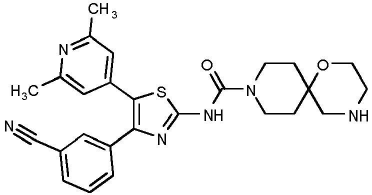

### Step 1: tert-Butyl 9-[[4-(3-cyanophenyl)-5-(2,6-dimethyl-4-pyridyl)thiazol-2-yl]carbamoyl]-1-oxa-4,9-diazaspiro[5.5]undecane-4-carboxylate

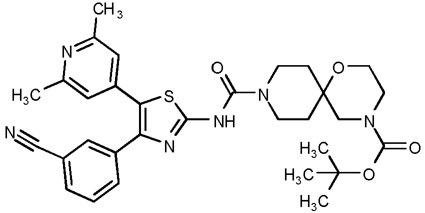

The title compound was prepared from 3-[2-amino-5-(2,6-dimethyl-4-pyridyl)thiazol-4-yl]benzonitrile (Intermediate C) and *tert*-butyl 1-oxa-4,9-diazaspiro[5.5]undecane-4-carboxylate analogously to Example 7 step 1.
LC-MS (Method 2A): Rt 0.77 mins; MS m/z 589.3 = [M+H]+
1H NMR (500 MHz, DMSO-d6) δ 11.22 (s, 1H), 7.89 (t, J = 1.5 Hz, 1H), 7.82 (dt, J = 7.7, 1.3 Hz, 1H), 7.70 (dt, J = 8.0, 1.3 Hz, 1H), 7.56 (t, J = 7.9 Hz, 1H), 6.92 (s, 2H), 3.86 - 3.78 (m, 2H), 3.64 - 3.59 (m, 2H), 3.36 - 3.32 (m, 2H), 3.28 - 3.16 (m, 4H), 2.36 (s, 6H), 1.75 - 1.69 (m, 2H), 1.52 - 1.44 (m, 2H), 1.41 (s, 9H).

### Step 2: N-[4-(3-Cyanophenyl)-5-(2,6-dimethyl-4-pyridyl)thiazol-2-yl]-1-oxa-4,9-diazaspiro[5.5]undecane-9-carboxamide

The title compound was prepared from *tert*-butyl 9-[[4-(3-cyanophenyl)-5-(2,6-dimethyl-4-pyridyl)thiazol-2-yl]carbamoyl]-1-oxa-4,9-diazaspiro[5.5]undecane-4-carboxylate (step 1) and 4M HCI in 1,4-dioxane analogously to Example 7 step 2.
LC-MS (Method 7B): Rt 2.68 mins; MS m/z 489.4 = [M+H]+
1H NMR (500 MHz, DMSO-d6) δ 7.88 (t, J = 1.7 Hz, 1H), 7.81 (dt, J = 7.9, 1.4 Hz, 1H), 7.70 (dt, J = 8.2, 1.4 Hz, 1H), 7.55 (dd, J = 7.8, 7.8 Hz, 1H), 6.91 (s, 2H), 3.83 (dt, J = 13.2, 4.1 Hz, 2H), 3.56 - 3.52 (m, 2H), 3.23 - 3.15 (m, 2H), 2.64 (td, J = 3.9, 2.1 Hz, 2H), 2.54 (s, 2H), 2.35 (s, 6H), 1.89 - 1.83 (m, 2H), 1.45 - 1.37 (m, 2H). 2 x NH protons not observed

### Example 7.4

### N-[4-(3-Cyanophenyl)-5-(2,6-dimethyl-4-pyridyl)thiazol-2-yl]-3,8-diazabicyclo[3.2.1]octane-8-carboxamide

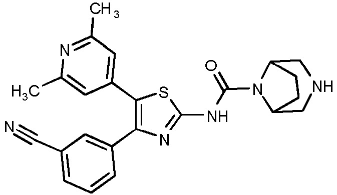

### Step 1: tert-Butyl 8-[[4-(3-cyanophenyl)-5-(2,6-dimethyl-4-pyridyl)thiazol-2-yl]carbamoyl]-3,8-diazabicyclo[3.2.1]octane-3-carboxylate

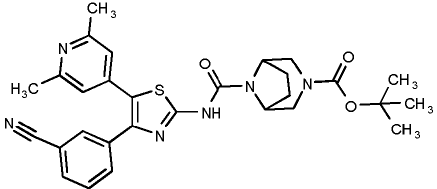

The title compound was prepared from 3-[2-amino-5-(2,6-dimethyl-4-pyridyl)thiazol-4-yl]benzonitrile (Intermediate C) and *tert*-butyl 3,8-diazabicyclo[3.2.1]octane-3-carboxylate analogously to Example 7 step 1.
LC-MS (Method 2A): Rt 0.77 mins; MS m/z 545.3 = [M+H]+
1H NMR (400 MHz, DMSO-D6) δ 11.37 (s, 1H), 7.88 (d, J = 1.7 Hz, 1H), 7.82 (dt, J = 7.8, 1.4 Hz, 1H), 7.70 (dt, J = 8.1, 1.4 Hz, 1H), 7.56 (dd, J = 7.8, 7.8 Hz, 1H), 6.93 (s, 2H), 4.60 - 4.49 (m, 2H), 3.80 - 3.66 (m, 2H), 3.09 - 3.02 (m, 1H), 2.96 - 2.89 (m, 1H), 2.36 (s, 6H), 1.89 - 1.80 (m, 2H), 1.62 (q, J = 7.0 Hz, 2H), 1.41 (s, 9H).

### Step 2: N-[4-(3-Cyanophenyl)-5-(2,6-dimethyl-4-pyridyl)thiazol-2-yl]-3,8-diazabicyclo[3.2.1]octane-8-carboxamide

The title compound was prepared from *tert*-butyl 8-[[4-(3-cyanophenyl)-5-(2,6-dimethyl-4-pyridyl)thiazol-2-yl]carbamoyl]-3,8-diazabicyclo[3.2.1]octane-3-carboxylate (step 1) and 4M HCI in 1,4-dioxane analogously to Example 7 step 2.
LC-MS (Method 7B): Rt 2.57 mins; MS m/z 445.4 = [M+H]+
1H NMR (500 MHz, Methanol-d4) δ 7.91 (t, J = 1.7 Hz, 1H), 7.72 (dt, J = 7.9, 1.4 Hz, 1H), 7.68 (dt, J = 7.8, 1.4 Hz, 1H), 7.48 (dd, J = 7.8, 7.8 Hz, 1H), 7.01 (s, 2H), 4.43 - 4.37 (m, 2H), 2.98 (dd, J = 13.1, 2.0 Hz, 2H), 2.74 (dd, J = 13.1, 2.1 Hz, 2H), 2.44 (s, 6H), 2.05 - 1.98 (m, 4H). 2 x NH protons not observed.

### Example 7.5

### N-[4-(3-Cyanophenyl)-5-(2,6-dimethyl-4-pyridyl)thiazol-2-yl]-3,6-diazabicyclo[3.1.1]heptane-6-carboxamide

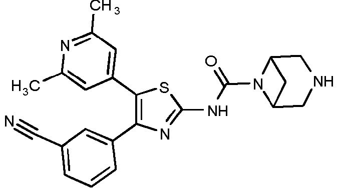

### Step 1: tert-Butyl 6-[[4-(3-cyanophenyl)-5-(2,6-dimethyl-4-pyridyl)thiazol-2-yl]carbamoyl]-3,6-diazabicyclo[3.1.1]heptane-3-carboxylate

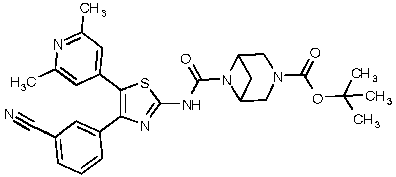

The title compound was prepared from 3-[2-amino-5-(2,6-dimethyl-4-pyridyl)thiazol-4-yl]benzonitrile (Intermediate C) and *tert-butyl* 3,6-diazabicyclo[3.1.1]heptane-3-carboxylate analogously to Example 7 step 1.
LC-MS (Method 2A): Rt 0.70 mins; MS m/z 531.3 = [M+H]+
1H NMR (500 MHz, DMSO-d6) δ 11.59 (s, 1H), 7.87 (t, J = 1.7 Hz, 1H), 7.82 (dt, J = 7.7, 1.4 Hz, 1H), 7.69 (dt, J = 8.0, 1.4 Hz, 1H), 7.56 (dd, J = 7.8, 7.8 Hz, 1H), 6.92 (s, 2H), 4.53 - 4.35 (m, 2H), 3.87 - 3.70 (m, 2H), 3.42 - 3.34 (m, 2H), 2.59 (q, J = 7.1 Hz, 1H), 2.35 (s, 6H), 1.45 (d, J = 8.9 Hz, 1H), 1.38 (s, 9H).

### Step 2: N-[4-(3-Cyanophenyl)-5-(2,6-dimethyl-4-pyridyl)thiazol-2-yl]-3,6-diazabicyclo[3.1.1]heptane-6-carboxamide

The title compound was prepared from *tert*-butyl 6-[[4-(3-cyanophenyl)-5-(2,6-dimethyl-4-pyridyl)thiazol-2-yl]carbamoyl]-3,6-diazabicyclo[3.1.1]heptane-3-carboxylate (step 1) and 4M HCI in 1,4-dioxane analogously to Example 7 step 2.
LC-MS (Method 7A): Rt 0.98 mins; MS m/z 431.3 = [M+H]+
1H NMR (500 MHz, Methanol-d4) δ 7.90 (t, J = 1.7 Hz, 1H), 7.71 (dt, J = 8.0, 1.4 Hz, 1H), 7.68 (dt, J = 7.8, 1.4 Hz, 1H), 7.51 - 7.45 (m, 1H), 7.01 (s, 2H), 4.43 - 4.38 (m, 2H), 3.48 (d, J = 13.2 Hz, 2H), 2.99 (dd, J = 13.0, 1.9 Hz, 2H), 2.76 - 2.70 (m, 1H), 2.44 (s, 6H), 1.84 (d, J = 8.8 Hz, 1H). 2 x NH protons not observed

### Example 7.6

### (1R,4R)-N-[4-(3-Cyanophenyl)-5-(2,6-dimethyl-4-pyridyl)thiazol-2-yl]-2,5-diazabicyclo[2.2.2]octane-2-carboxamide

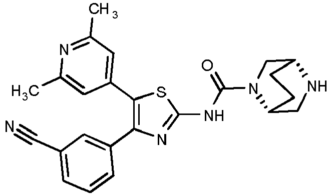

### Step 1: tert-Butyl (1R,4R)-5-[[4-(3-cyanophenyl)-5-(2,6-dimethyl-4-pyridyl)thiazol-2-yl]carbamoyl]-2,5-diazabicyclo[2.2.2]octane-2-carboxylate

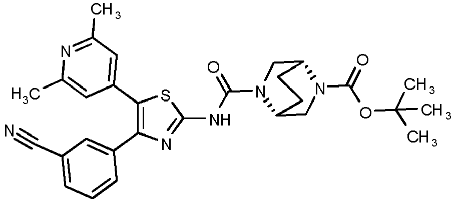

The title compound was prepared from 3-[2-amino-5-(2,6-dimethyl-4-pyridyl)thiazol-4-yl]benzonitrile (Intermediate C) and *tert-butyl* (2R,5R)-5-ethyl-2-methylpiperazine-1-carboxylate analogously to Example 7 step 1.
LC-MS (Method 2A): Rt 0.76 mins; MS m/z 545.3 = [M+H]+
1H NMR (500 MHz, DMSO-d6) δ 11.07 (s, 1H), 7.88 (d, J = 1.8 Hz, 1H), 7.82 (dt, J = 7.8, 1.5 Hz, 1H), 7.71 (dt, J = 8.0, 1.4 Hz, 1H), 7.56 (dd, J = 7.8, 7.8 Hz, 1H), 6.93 (s, 2H), 4.50 - 4.37 (m, 1H), 4.23 - 4.13 (m, 1H), 3.68 - 3.38 (m, 4H), 2.36 (s, 6H), 1.91 - 1.73 (m, 4H), 1.41 (d, J = 3.3 Hz, 9H).

### Step 2: (1R,4R)-N-[4-(3-Cyanophenyl)-5-(2,6-dimethyl-4-pyridyl)thiazol-2-yl]-2,5-diazabicyclo[2.2.2]octane-2-carboxamide

The title compound was prepared from *tert*-butyl (1R,4R)-5-[[4-(3-cyanophenyl)-5-(2,6-dimethyl-4-pyridyl)thiazol-2-yl]carbamoyl]-2,5-diazabicyclo[2.2.2]octane-2-carboxylate (step 1) and 4M HCI in 1,4-dioxane analogously to Example 7 step 2.
LC-MS (Method 7A): Rt 0.99 mins; MS m/z 445.3 = [M+H]+
1H NMR (500 MHz, Methanol-d4) δ 7.91 (t, J = 1.7 Hz, 1H), 7.72 (dt, J = 8.0, 1.5 Hz, 1H), 7.68 (dt, J = 7.8, 1.4 Hz, 1H), 7.49 (dd, J = 7.8, 7.8 Hz, 1H), 7.00 (s, 2H), 4.27 (s, 1H), 3.78 (d, J = 10.7 Hz, 1H), 3.64 (d, J = 10.7 Hz, 1H), 3.28 - 3.23 (m, 1H), 3.22 - 3.18 (m, 1H), 3.11 (dd, J = 11.5, 1.9 Hz, 1H), 2.43 (s, 6H), 2.08 - 1.98 (m, 2H), 1.97 - 1.89 (m, 1H), 1.88 - 1.80 (m, 1H). 2 x NH protons not observed.

### Example 7.7

### (2S,5S)-N-[4-(3-Cyanophenyl)-5-(2,6-dimethyl-4-pyridyl)thiazol-2-yl]-2,5-dimethylpiperazine-1-carboxamide

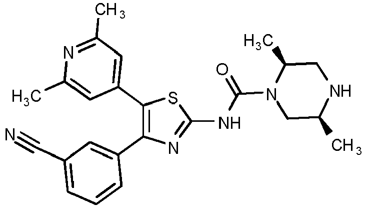

### Step 1: tert-Butyl (2S,5S)-4-[[4-(3-cyanophenyl)-5-(2,6-dimethyl-4-pyridyl)thiazol-2-yl]carbamoyl]-2,5-dimethyl-piperazine-1-carboxylate

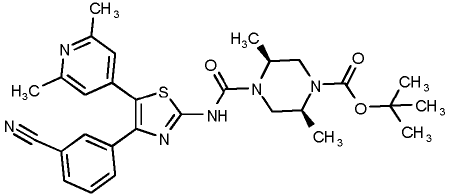

The title compound was prepared from 3-[2-amino-5-(2,6-dimethyl-4-pyridyl)thiazol-4-yl]benzonitrile (Intermediate C) and *tert*-butyl cis-(2S,5S)-2,5-dimethylpiperazine-1-carboxylate analogously to Example 7 step 1.
LC-MS (Method 7A): Rt 0.80 mins; MS m/z 547.4 = [M+H]+
1H NMR (400 MHz, DMSO-d6) δ 11.19 (s, 1H), 7.89 (t, J = 1.7 Hz, 1H), 7.82 (dt, J = 7.8, 1.4 Hz, 1H), 7.70 (dt, J = 8.0, 1.4 Hz, 1H), 7.56 (dd, J = 7.8, 7.8 Hz, 1H), 6.93 (s, 2H), 4.20 - 4.08 (m, 2H), 3.95 - 3.84 (m, 2H), 2.96 (dd, J = 14.6, 11.2 Hz, 1H), 2.88 (dd, J = 14.2, 10.7 Hz, 1H), 2.36 (s, 6H), 1.39 (s, 9H), 1.10 (d, J = 6.1 Hz, 3H), 1.06 (d, J = 6.1 Hz, 3H).

### Step 2: (2S,5S)-N-[4-(3-Cyanophenyl)-5-(2,6-dimethyl-4-pyridyl)thiazol-2-yl]-2,5-dimethylpiperazine-1-carboxamide

The title compound was prepared from *tert*-butyl (2S,5S)-4-[[4-(3-cyanophenyl)-5-(2,6-dimethyl-4-pyridyl)thiazol-2-yl]carbamoyl]-2,5-dimethyl-piperazine-1-carboxylate (step 1) and 4M HCI in 1,4-dioxane analogously to Example 7 step 2.
LC-MS (Method 7B): Rt 2.72 mins; MS m/z 447.3 = [M+H]+
1H NMR (500 MHz, Methanol-d4) δ 7.91 (t, J = 1.7 Hz, 1H), 7.71 (dt, J = 7.9, 1.4 Hz, 1H), 7.69 (dt, J = 7.8, 1.4 Hz, 1H), 7.49 (dd, J = 7.8, 7.8 Hz, 1H), 7.00 (s, 2H), 4.37 - 4.29 (m, 1H), 3.98 - 3.90 (m, 1H), 2.99 (dd, J = 12.9, 4.2 Hz, 1H), 2.90 (dd, J = 12.9, 1.5 Hz, 1H), 2.78 - 2.69 (m, 2H), 2.43 (s, 6H), 1.31 (d, J = 6.9 Hz, 3H), 1.16 (d, J = 5.8 Hz, 3H). 2 x NH protons not observed.

### Example 7.8

### (2R,6R)-N-[4-(3-Cyanophenyl)-5-(2,6-dimethyl-4-pyridyl)thiazol-2-yl]-2,6-dimethylpiperazine-1-carboxamide

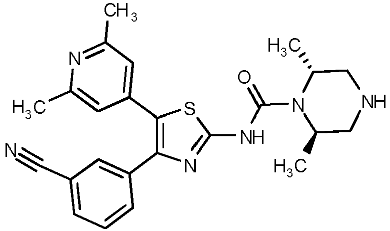

### Step 1: tert-Butyl (3R,5R)-4-[[4-(3-cyanophenyl)-5-(2,6-dimethyl-4-pyridyl)thiazol-2-yl]carbamoyl]-3,5-dimethyl-piperazine-1-carboxylate

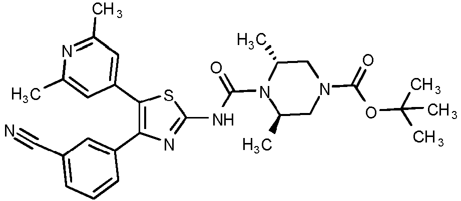

The title compound was prepared from 3-[2-amino-5-(2,6-dimethyl-4-pyridyl)thiazol-4-yl]benzonitrile (Intermediate C) and *tert*-butyl (3R,5R)-3,5-dimethylpiperazine-1-carboxylate analogously to Example 7 step 1.
LC-MS (Method 2A): Rt 0.81 mins; MS m/z 547.3 = [M+H]+
1H NMR (400 MHz, DMSO-d6) δ 11.16 (s, 1H), 7.90 (t, J = 1.7 Hz, 1H), 7.82 (dt, J = 7.8, 1.2 Hz, 1H), 7.71 (dt, J = 8.3, 1.2 Hz, 1H), 7.56 (dd, J = 7.8, 7.8 Hz, 1H), 6.92 (s, 2H), 4.39 - 4.29 (m, 2H), 3.61 - 3.45 (m, 4H), 2.35 (s, 6H), 1.43 (s, 9H), 1.23 - 1.17 (m, 6H).

### Step 2: (2R,6R)-N-[4-(3-Cyanophenyl)-5-(2,6-dimethyl-4-pyridyl)thiazol-2-yl]-2,6-dimethylpiperazine-1-carboxamide

The title compound was prepared from *tert*-butyl rac-(3R,5R)-4-[[4-(3-cyanophenyl)-5-(2,6-dimethyl-4-pyridyl)thiazol-2-yl]carbamoyl]-3,5-dimethyl-piperazine-1-carboxylate (step 1) and 4M HCI in 1,4-dioxane analogously to Example 7 step 2.
LC-MS (Method 7B): Rt 2.73 mins; MS m/z 447.4 = [M+H]+
1H NMR (400 MHz, Methanol-d4) δ 7.91 (t, J = 1.7 Hz, 1H), 7.72 (dt, J = 8.0, 1.5 Hz, 1H), 7.69 (dt, J = 7.8, 1.4 Hz, 1H), 7.49 (t, J = 7.8 Hz, 1H), 7.01 (s, 2H), 4.02 - 3.95 (m, 2H), 3.21 (dd, J = 13.0, 3.9 Hz, 2H), 2.78 (dd, J = 13.1, 4.5 Hz, 2H), 2.44 (s, 6H), 1.34 (d, J = 6.5 Hz, 6H). - 2 x NH protons not observed

### Example 7.9

### N-[4-(3-Cyanophenyl)-5-(2,6-dimethyl-4-pyridyl)thiazol-2-yl]-2,2-dimethyl-piperazine-1-carboxamide

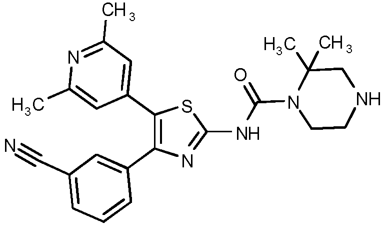

### Step 1: tert-Butyl 4-[[4-(3-cyanophenyl)-5-(2,6-dimethyl-4-pyridyl)thiazol-2-yl]carbamoyl]-3,3-dimethyl-piperazine-1-carboxylate

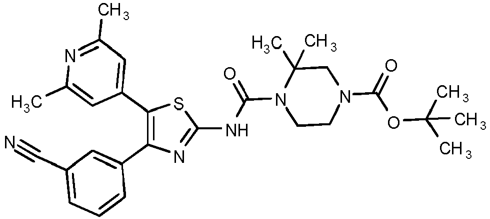

The title compound was prepared from 3-[2-amino-5-(2,6-dimethyl-4-pyridyl)thiazol-4-yl]benzonitrile (Intermediate C) and *tert*-butyl 3,3-dimethylpiperazine-1-carboxylate analogously to Example 7 step 1.
LC-MS (Method 2A): Rt 0.83 mins; MS m/z 547.3 = [M+H]+
1H NMR (500 MHz, DMSO-d6) δ 11.24 (s, 1H), 7.87 (t, J = 1.7 Hz, 1H), 7.82 (dt, J = 7.7, 1.5 Hz, 1H), 7.70 (dt, J = 8.1, 1.4 Hz, 1H), 7.56 (dd, J = 7.8, 7.8 Hz, 1H), 6.92 (s, 2H), 3.71 - 3.65 (m, 2H), 3.45 - 3.36 (m, 4H), 2.35 (s, 6H), 1.42 (s, 9H), 1.39 (s, 6H).

### Step 2: N-[4-(3-Cyanophenyl)-5-(2,6-dimethyl-4-pyridyl)thiazol-2-yl]-2,2-dimethyl-piperazine-1-carboxamide

The title compound was prepared from *tert*-butyl 4-[[4-(3-cyanophenyl)-5-(2,6-dimethyl-4-pyridyl)thiazol-2-yl]carbamoyl]-3,3-dimethyl-piperazine-1-carboxylate (step 1) and 4M HCI in 1,4-dioxane analogously to Example 7 step 2.
LC-MS (Method 7B): Rt 2.88 mins; MS m/z 447.4 = [M+H]+
1H NMR (400 MHz, Methanol-d4) δ 7.89 (t, J = 1.5 Hz, 1H), 7.72 - 7.67 (m, 2H), 7.49 (dd, J = 7.8, 7.8 Hz, 1H), 7.00 (s, 2H), 3.50 - 3.45 (m, 2H), 2.99 - 2.93 (m, 2H), 2.69 (s, 2H), 2.44 (s, 6H), 1.47 (s, 6H). 2 x NH protons not observed.

### Example 7.10

### N-[4-(3-Cyanophenyl)-5-(2,6-dimethyl-4-pyridyl)thiazol-2-yl]-5,8-diazaspiro[3.5]nonane-5-carboxamide

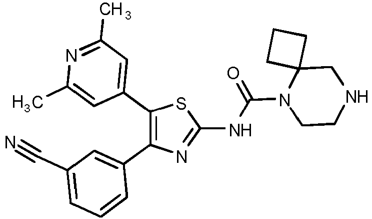

### Step 1: tert-Butyl 5-[[4-(3-cyanophenyl)-5-(2,6-dimethyl-4-pyridyl)thiazol-2-yl]carbamoyl]-5,8-diazaspiro[3.5]nonane-8-carboxylate

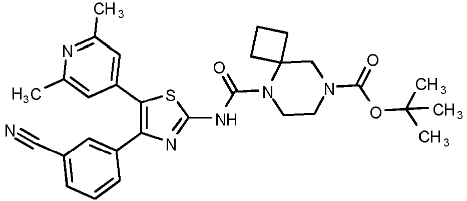

The title compound was prepared from 3-[2-amino-5-(2,6-dimethyl-4-pyridyl)thiazol-4-yl]benzonitrile (Intermediate C) and *tert*-butyl 5,8-diazaspiro[3.5]nonane-8-carboxylate analogously to Example 7 step 1.
LC-MS (Method 2A): Rt 0.83 mins; MS m/z 559.3 = [M+H]+
1H NMR (400 MHz, DMSO-d6) δ 11.40 (s, 1H), 7.87 (t, J = 1.7 Hz, 1H), 7.82 (dt, J = 7.8, 1.5 Hz, 1H), 7.70 (dt, J = 8.0, 1.4 Hz, 1H), 7.56 (dd, J = 7.8, 7.8 Hz, 1H), 6.93 (s, 2H), 3.53 (s, 2H), 3.50 - 3.44 (m, 2H), 3.29 - 3.25 (m, 2H), 2.35 (s, 6H), 2.28 - 2.16 (m, 3H), 2.04 - 1.97 (m, 2H), 1.81 - 1.75 (m, 1H), 1.42 (s, 9H).

### Step 2: N-[4-(3-Cyanophenyl)-5-(2,6-dimethyl-4-pyridyl)thiazol-2-yl]-5,8-diazaspiro[3.5]nonane-5-carboxamide

The title compound was prepared from *tert*-butyl 5-[[4-(3-cyanophenyl)-5-(2,6-dimethyl-4-pyridyl)thiazol-2-yl]carbamoyl]-5,8-diazaspiro[3.5]nonane-8-carboxylate (step 1) and 4M HCI in 1,4-dioxane analogously to Example 7 step 2.
LC-MS (Method 7B): Rt 2.48 mins; MS m/z 459.3 = [M+H]+
1H NMR (400 MHz, Methanol-d4) δ 7.89 (t, J = 1.7 Hz, 1H), 7.72 - 7.66 (m, 2H), 7.48 (dd, J = 7.8, 0.6 Hz, 1H), 6.99 (s, 2H), 3.51 - 3.47 (m, 2H), 3.01 (s, 2H), 2.73 - 2.69 (m, 2H), 2.43 (s, 6H), 2.33 - 2.25 (m, 4H), 1.88 - 1.69 (m, 2H). 2 x NH protons not observed.

### Example 7.11

### trans-N-[4-(3-Cyanophenyl)-5-(2,6-dimethyl-4-pyridyl)thiazol-2-yl]-2,5-dimethylpiperazine-1-carboxamide

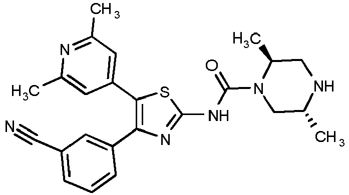

### Step 1: tert-Butyl trans-4-[[4-(3-cyanophenyl)-5-(2,6-dimethyl-4-pyridyl)thiazol-2-yl]carbamoyl]-2,5-dimethyl-piperazine-1-carboxylate

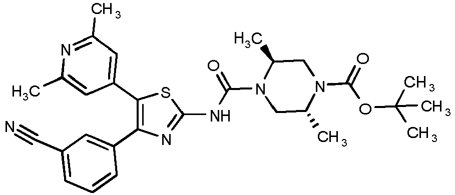

The title compound was prepared from 3-[2-amino-5-(2,6-dimethyl-4-pyridyl)thiazol-4-yl]benzonitrile (Intermediate C) and *tert*-butyl trans-2,5-dimethylpiperazine-1-carboxylate analogously to Example 7 step 1
LC-MS (Method 2A): Rt 0.81 mins; MS m/z 547.3 = [M+H]+
1H NMR (400 MHz, DMSO-d6) δ 11.29 (s, 1H), 7.89 (t, J = 1.7 Hz, 1H), 7.85 - 7.78 (m, 1H), 7.70 (dt, J = 8.2, 1.6 Hz, 1H), 7.56 (dd, J = 7.8, 7.8 Hz, 1H), 6.92 (s, 2H), 4.44 (d, J = 21.6 Hz, 2H), 4.31 - 4.21 (m, 1H), 4.21 - 4.11 (m, 1H), 3.92 - 3.85 (m, 1H), 3.65 - 3.56 (m, 1H), 2.36 (s, 6H), 1.42 (s, 9H), 1.15 - 1.01 (m, 6H).

### Step 2: trans-N-[4-(3-Cyanophenyl)-5-(2,6-dimethyl-4-pyridyl)thiazol-2-yl]-2,5-dimethylpiperazine-1-carboxamide

The title compound was prepared from *tert*-butyl trans-4-[[4-(3-cyanophenyl)-5-(2,6-dimethyl-4-pyridyl)thiazol-2-yl]carbamoyl]-2,5-dimethyl-piperazine-1-carboxylate (step 1) and 4M HCI in 1,4-dioxane analogously to Example 7 step 2.
LC-MS (Method 7A): Rt 1.10 mins; MS m/z 447.3 = [M+H]+
1H NMR (400 MHz, Methanol-d4) δ 7.91 (t, J = 1.7 Hz, 1H), 7.74 - 7.67 (m, 2H), 7.49 (dd, J = 7.8, 7.8 Hz, 1H), 7.00 (s, 2H), 4.33 - 4.26 (m, 1H), 3.74 (dd, J = 14.0, 2.2 Hz, 1H), 3.40 (dd, J = 13.6, 4.0 Hz, 1H), 3.26 - 3.17 (m, 2H), 2.60 (dd, J = 13.5, 2.9 Hz, 1H), 2.43 (s, 6H), 1.33 (d, J = 6.8 Hz, 3H), 1.22 (d, J = 6.8 Hz, 3H). 2 x NH protons not observed.

### Example 8

### 1-Acetylimino-N-[4-(3-cyanophenyl)-5-(2,6-dimethyl-4-pyridyl)thiazol-2-yl]-1-oxo-1,4-thiazinane-4-carboxamide

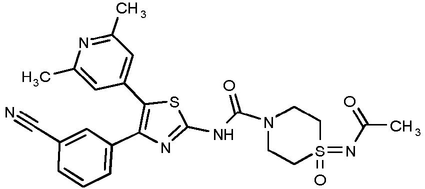

To a suspension of N-[4-(3-cyanophenyl)-5-(2,6-dimethyl-4-pyridyl)thiazol-2-yl]-1-imino-1-oxo-1,4-thiazinane-4-carboxamide (Example 4.2) (50 mg, 0.11 mmol) in DCM (2.5 mL) at 0 °C was added triethylamine (37 µL, 0.27 mmol) followed by acetyl chloride (10 µL, 0.14 mmol) and the mixture was stirred for 1 h. A further portion of acetyl chloride (10 µL, 0.14 mmol) was added and the reaction mixture was stirred at 0 °C for 30 mins after which time additional acetyl chloride (4 µL, 0.05 mmol) was added and the reaction mixture was stirred at 0 °C for 15 mins. The resulting mixture was concentrated *in vacuo* then partitioned between 10% MeOH in EtOAc (20 mL) and water (20 mL). The layers were separated and the aqueous layer was further extracted with 10% MeOH in EtOAc (2 x 20 mL). The combined organic extracts were concentrated *in vacuo* and purification of the crude material by C18 reverse phase chromatography eluting with 5-15% MeCN in water (+0.1% ammonium hydroxide modifier) afforded the title compound as an off-white solid.
LC-MS (Method 8B): Rt 2.16 mins; MS m/z 509.3 = [M+H]+
¹H NMR (500 MHz, DMSO-d6) δ 11.58 (s, 1H), 7.88 (s, 1H), 7.83 (d, J = 7.8 Hz, 1H), 7.70 (d, J = 7.8 Hz, 1H), 7.56 (t, *J =* 7.8 Hz, 1H), 6.92 (s, 2H), 4.26 - 4.18 (m, 2H), 3.77 - 3.65 (m, 4H), 3.53 - 3.47 (m, 2H), 2.36 (s, 6H), 1.99 (s, 3H).

### Example 9

### N-[4-(3-Cyanophenyl)-5-[2-(hydroxymethyl)-6-methyl-4-pyridyl]thiazol-2-yl]-2-oxa-6-azaspiro[3.3]heptane-6-carboxamide

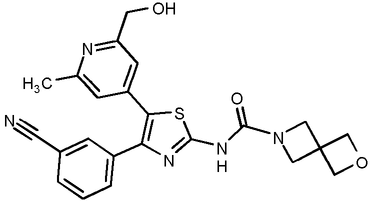

A solution of 3-[2-amino-5-[2-[[tert-butyl(dimethyl)silyl]oxymethyl]-6-methyl-4-pyridyl]thiazol-4-yl]benzonitrile (Intermediate I) (60 mg, 0.13 mmol) and CDI (49 mg, 0.30 mmol) in DMA (0.5 mL) was stirred at 50 °C overnight. The resulting mixture was treated with 2-oxa-6-azaspiro[3.3]heptane (30 mg, 0.30 mmol) in DMA (0.1 mL) and DIPEA (78 µL, 0.45 mmol) and stirring continued at 50 °C for 30 mins. The reaction mixture was cooled to room temperature and 1M TBAF in THF (211 µL, 0.21 mmol) was added. After stirring for 15 mins, the resulting mixture was purified by C18 reverse phase chromatography eluting with a gradient of 5 to 15% MeCN in water (+0.1% ammonium hydroxide modifier). The product fractions were combined, concentrated and loaded onto an Isolute^{®} SCX cartridge. The column was flushed with MeOH (30 mL) and the product was eluted with 0.7 M NH₃ in MeOH (30 mL). The solvent was removed *in vacuo* and the resulting material was triturated in 2:1 hexane/diethyl ether (1 mL) to yield a solid. The solid was purified further by chromatography on silica eluting with a gradient of 0 to 4% MeOH in DCM to afford the title compound as a yellow solid.
LC-MS (Method 8B): Rt 2.93 mins; MS m/z 448.3 = [M+H]+
1H NMR (500 MHz, DMSO-d6) δ 11.36 (s, 1H), 7.87 (d, J = 1.5 Hz, 1H), 7.81 (d, J = 7.8 Hz, 1H), 7.71 (dt, J = 7.8, 1.5 Hz, 1H), 7.55 (t, J = 7.8 Hz, 1H), 7.13 (s, 1H), 6.96 (s, 1H), 5.32 (t, J = 5.8 Hz, 1H), 4.67 (s, 4H), 4.46 (d, J = 5.8 Hz, 2H), 4.19 (s, 4H), 2.36 (s, 3H).

The compounds of the following tabulated Examples (Table Ex9) were prepared analogously to Example 9 from 3-[2-amino-5-[2-[[*tert-*butyl(dimethyl)silyl]oxymethyl]-6-methyl-4-pyridyl]thiazol-4-yl]benzonitrile (Intermediate I) and the appropriate amine.

**Table Ex9**

| **Ex.** | **Structure and Name** | **Retention Time, [M+H]+, ¹H NMR** |
|---|---|---|
| 9.1 | 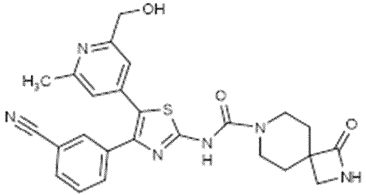 N-[4-(3-Cyanophenyl)-5-[2-(hydroxymethyl)-6-methyl-4-pyridyl]thiazol-2-yl]-3-oxo-2,7-diazaspiro[3.5]nonane-7-carboxamide | LC-MS (Method 8B): Rt 3.02 mins; |
| | | MS m/z 489.3 = [M+H]+ 1H NMR (500 MHz, DMSO-d6) δ 11.28 (s, 1H), 7.90 (d, J= 1.5 Hz, 1H), 7.85 - 7.77 (m, 2H), 7.73 (dt, J= 8.0, 1.5 Hz, 1H), 7.56 (t, J= 7.8 Hz, 1H), 7.15 (s, 1H), 6.98 (s, 1H), 5.32 (t, J= 5.8 Hz, 1H), 4.47 (d, J= 5.8 Hz, 2H), 3.87 (dt, J= 13.4, 5.0 Hz, 2H), 3.40 - 3.36 (m, 2H), 3.09 (s, 2H), 2.38 (s, 3H), 1.81 - 1.69 (m, 4H). |
| 9.2 | 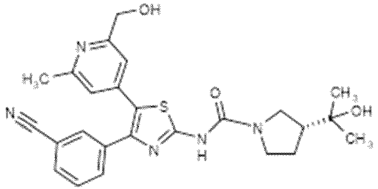 (3R)-N-[4-(3-Cyanophenyl)-5-[2-(hydroxymethyl)-6-methyl-4-pyridyl]thiazol-2-yl]-3-(1-hydroxy-1-methyl-ethyl)pyrrolidine-1-carboxamide | LC-MS (Method 8B): Rt 3.66 mins; |
| | | MS m/z 478.3 = [M+H]+ ¹H NMR (500 MHz, Methanol-*d*₄) δ 7.91 (t, *J* = 1.5 Hz, 1H), 7.73 (dt, *J* = 7.9, 1.5 Hz, 1H), 7.68 (dt, J = 7.9, 1.5 Hz, 1H), 7.49 (t, *J* = 7.9 Hz, 1H), 7.29 (s, 1H), 7.07 (s, 1H), 4.61 (s, 2H), 3.72 (t, *J =* 9.3 Hz, 1H), 3.68 - 3.63 (m, 1H), 3.46 - 3.41 (m, 1H), 3.41 - 3.35 (m, 1H), 2.45 (s, 3H), 2.43 - 2.34 (m, 1H), 2.09 - 1.98 (m, 1H), 1.98 - 1.85 (m, 1H), 1.26 (d, *J* = 1.8 Hz, 6H). |
| | | NH and 2 x OH protons not observed. |
| 9.3 | 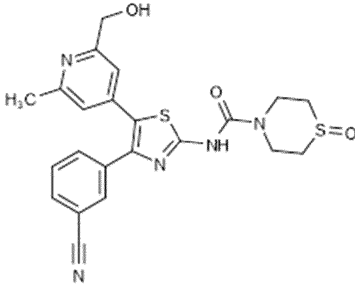 N-[4-(3-Cyanophenyl)-5-[2-(hydroxymethyl)-6-methyl-4-pyridyl]thiazol-2-yl]-1-oxo-1,4-thiazinane-4-carboxamide | LC-MS (Method 8B): Rt 2.71 mins; |
| | | MS m/z 468.2 = [M+H]+ 1H NMR (500 MHz, DMSO-d6) δ 11.45 (s, 1H), 7.89 (t, J = 1.5 Hz, 1H), 7.82 (d, J = 7.8 Hz, 1H), 7.72 (dt, J = 7.8, 1.5 Hz, 1H), 7.56 (t, J = 7.8 Hz, 1H), 7.14 (s, 1H), 6.98 (s, 1H), 5.32 (t, J = 5.8 Hz, 1H), 4.47 (d, J = 5.8 Hz, 2H), 4.16 - 4.07 (m, 2H), 3.79 (t, J = 13.1 Hz, 2H), 2.93 (t, J = 12.5 Hz, 2H), 2.75 (d, J = 13.7 Hz, 2H), 2.37 (s, 3H). |
| 9.4 | 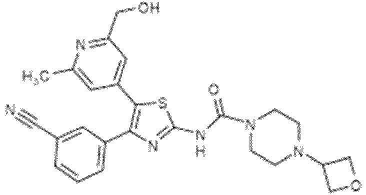 N-[4-(3-Cyanophenyl)-5-[2-(hydroxymethyl)-6-methyl-4-pyridyl]thiazol-2-yl]-4-(oxetan-3-yl)piperazine-1-carboxamide | LC-MS (Method 8B): Rt 2.97 mins; |
| | | MS m/z 491.3 = [M+H]+ 1H NMR (500 MHz, DMSO-d6) δ 11.27 (s, 1H), 7.89 (t, J= 1.5 Hz, 1H), 7.82 (dt, J= 7.8, 1.5 Hz, 1H), 7.72 (dt, J= 7.8, 1.5 Hz, 1H), 7.56 (t, J= 7.8 Hz, 1H), 7.14 (s, 1H), 6.98 (s, 1H), 5.32 (t, J= 5.8 Hz, 1H), 4.54 (t, J= 6.5 Hz, 2H), 4.51 - 4.40 (m, 4H), 3.57 (t, J= 4.9 Hz, 4H), 3.45 - 3.40 (m, 1H), 2.37 (s, 3H), 2.28 (t,J= 4.9 Hz, 4H). |
| 9.5 | 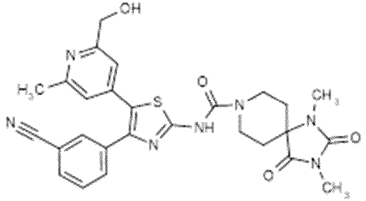 N-[4-(3-Cyanophenyl)-5-[2-(hydroxymethyl)-6-methyl-4-pyridyl]thiazol-2-yl]-1,3-dimethyl-2,4-dioxo-1,3,8-triazaspiro[4.5]decane-8-carboxamide | LC-MS (Method 8B): Rt 3.44 mins; |
| | | MS m/z 546.2 = [M+H]+ 1H NMR (500 MHz, DMSO-d6) δ 11.32 (s, 1H), 7.89 (d, J= 1.5 Hz, 1H), 7.82 (d, J= 7.9 Hz, 1H), 7.72 (dt, J= 7.9, 1.5 Hz, 1H), 7.56 (t, J= 7.9 Hz, 1H), 7.15 (s, 1H), 6.98 (s, 1H), 5.32 (t, J= 5.8 Hz, 1H), 4.47 (d, J= 5.8 Hz, 2H), 4.25 (d, J= 13.1 Hz, 2H), 3.49 (d, J= 13.1 Hz, 2H), 2.87 (s, 3H), 2.76 (s, 3H), 2.37 (s, 3H), 1.92 (td, J= 13.1, 4.6 Hz, 2H), 1.72 (d, J= 13.1 Hz, 2H). |
| 9.6 | 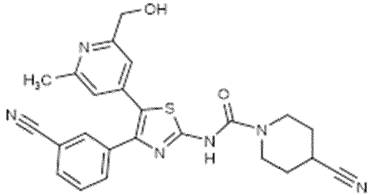 4-Cyano-N-[4-(3-cyanophenyl)-5-[2-(hydroxymethyl)-6-methyl-4-pyridyl]thiazol-2-yl]piperidine-1-carboxamide | LC-MS (Method 8B): Rt 3.60 mins; |
| | | MS m/z 459.3 = [M+H]+ 1H NMR (500 MHz, DMSO-d6) δ 11.30 (s, 1H), 7.89 (t, J = 1.5 Hz, 1H), 7.82 (dt, J = 7.8, 1.5 Hz, 1H), 7.72 (dt, J = 7.8, 1.5 Hz, 1H), 7.56 (t, J = 7.8 Hz, 1H), 7.14 (s, 1H), 6.97 (s, 1H), 5.32 (t, J = 5.8 Hz, 1H), 4.47 (d, J = 5.8 Hz, 2H), 3.85 - 3.73 (m, 2H), 3.41 - 3.34 (m, 2H), 3.14 - 3.08 (m, 1H), 2.37 (s, 3H), 1.95 - 1.86 (m, 2H), 1.75 - 1.65 (m, 2H). |
| 9.7 | 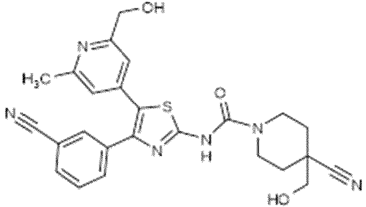 4-Cyano-N-[4-(3-cyanophenyl)-5-[2-(hydroxymethyl)-6-methyl-4-pyridyl]thiazol-2-yl]-4-(hydroxymethyl)piperidine-1-carboxamide (Prepared from amine Intermediate E) | LC-MS (Method 8B): Rt 3.00 mins; |
| | | MS m/z 489.2 = [M+H]+ 1H NMR (500 MHz, DMSO-d6) δ 11.33 (s, 1H), 7.90 (s, 1H), 7.83 (d, J= 7.8 Hz, 1H), 7.72 (d, J= 7.8 Hz, 1H), 7.57 (t, J= 7.8 Hz, 1H), 7.15 (s, 1H), 6.98 (s, 1H), 5.49 (t, J= 5.8 Hz, 1H), 5.33 (t, J= 5.8 Hz, 1H), 4.47 (d, J= 5.8 Hz, 2H), 4.28 (d, J= 14.0 Hz, 2H), 3.51 (d, J= 5.8 Hz, 2H), 3.02 (t, J= 13.0 Hz, 2H), 2.38 (s, 3H), 1.90 (d, J= 13.7 Hz, 2H), 1.51 (td, J= 13.4, 4.0 Hz, 2H). |
| 9.8 | 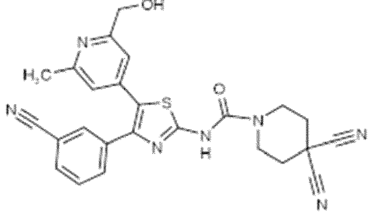 4,4-Dicyano-N-[4-(3-cyanophenyl)-5-[2-(hydroxymethyl)-6-methyl-4-pyridyl]thiazol-2-yl]piperidine-1-carboxamide (Prepared from amine Intermediate F) | LC-MS (Method 8B): Rt 3.51 mins; |
| | | MS m/z 484.2 = [M+H]+ 1H NMR (500 MHz, DMSO-d6) δ 11.43 (s, 1H), 7.90 (t, J= 1.8 Hz, 1H), 7.84 (dt, J= 7.7, 1.5 Hz, 1H), 7.73 (dt, J= 8.0, 1.4 Hz, 1H), 7.57 (t, J= 7.8 Hz, 1H), 7.15 (s, 1H), 6.99 (s, 1H), 5.33 (t, J= 5.8 Hz, 1H), 4.48 (d, J= 6.1 Hz, 2H), 3.70 (t, J= 5.4 Hz, 4H), 2.37 (d, J= 10.5 Hz, 7H). |
| 9.9 | 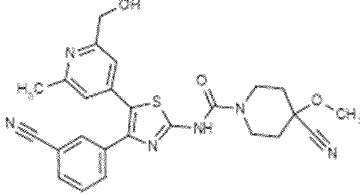 4-Cyano-N-[4-(3-cyanophenyl)-5-[2-(hydroxymethyl)-6-methyl-4-pyridyl]thiazol-2-yl]-4-methoxy-piperidine-1-carboxamide | LC-MS (Method 8B): Rt 3.92 mins; |
| | | MS m/z 489.2 = [M+H]+ 1H NMR (500 MHz, DMSO-d6) δ 11.37 (s, 1H), 7.90 (t, J= 1.7 Hz, 1H), 7.83 (d, J= 7.7 Hz, 1H), 7.72 (dt, J= 8.1, 1.4 Hz, 1H), 7.57 (t, J= 7.9 Hz, 1H), 7.17 - 7.13 (m, 1H), 6.99 (s, 1H), 5.33 (t, J= 5.8 Hz, 1H), 4.47 (d, J= 5.9 Hz, 2H), 3.70 (s, 2H), 3.57 - 3.49 (m, 2H), 3.41 (s, 3H), 2.38 (s, 3H), 2.18 - 2.10 (m, 2H), 1.94 - 1.87 (m, 2H). |

### Example 10

### N-[4-(3-Cyanophenyl)-5-(3-fluoro-2,6-dimethyl-4-pyridyl)thiazol-2-yl]-1-oxo-1,4-thiazinane-4-carboxamide

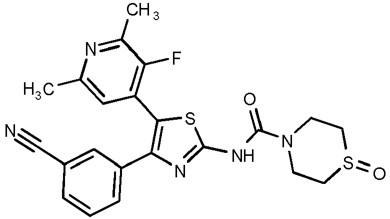

The title compound was prepared from 3-[2-amino-5-(3-fluoro-2,6-dimethyl-4-pyridyl)thiazol-4-yl]benzonitrile (Intermediate M) and 1,4-thiazinane 1-oxide hydrochloride analogously to Example 6.
LC-MS (Method 8A): Rt 2.64 mins; MS m/z 470.3 = [M+H]+
1H NMR (500 MHz, DMSO-d6) δ 11.51 (s, 1H), 7.89 (s, 1H), 7.81 (d, J = 7.8 Hz, 1H), 7.67 (d, J = 7.8 Hz, 1H), 7.55 (t, J = 7.8 Hz, 1H), 7.11 (d, J = 4.9 Hz, 1H), 4.12 (d, J = 14.7 Hz, 2H), 3.79 (t, J = 13.1 Hz, 2H), 2.94 (t, J = 12.5 Hz, 2H), 2.75 (d, J = 13.6 Hz, 2H), 2.41 - 2.32 (m, 6H).

### Example 11

### 1-Cyanoimino-N-[4-(3-cyanophenyl)-5-(2,6-dimethyl-4-pyridyl)thiazol-2-yl]-1-oxo-1,4-thiazinane-4-carboxamide

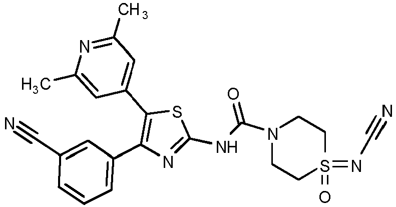

To a suspension of N-[4-(3-cyanophenyl)-5-(2,6-dimethyl-4-pyridyl)thiazol-2-yl]-1-imino-1-oxo-1,4-thiazinane-4-carboxamide (Example 4.2)(50 mg, 0.11 mmol) in DCM (2 mL) at 0 °C was added DMAP (14 mg, 0.12 mmol) followed by cyanogen bromide (23 mg, 0.21 mmol) and the mixture was stirred at 0 °C for 1h 10 mins. Further cyanogen bromide (3 mg, 0.03 mmol) and DMAP (2 mg, 0.02 mmol) were added and stirring continued at 0 °C for 1 h. Additional cyanogen bromide (6 mg, 0.05 mmol) and DMAP (3 mg, 0.03 mmol) were added and after stirring for 45 mins, the mixture was concentrated *in vacuo.* The crude product was purified by C18 reverse phase chromatography eluting with 5 to 40% MeCN in water with (0.1% ammonium hydroxide modifier) and further purified by chromatography on silica eluting with a gradient of 0 to 5% MeOH in DCM followed by purification by chromatography on silica eluting with a gradient of 0 to 80% EtOAc in DCM to afford the title compound as a yellow solid.
LC-MS (Method 8B): Rt 4.70 mins; MS m/z 492.0 = [M+H]+
1H NMR (500 MHz, DMSO-d6) δ 11.64 (s, 1H), 7.89 (s, 1H), 7.84 (d, J = 7.8 Hz, 1H), 7.70 (d, J = 7.8 Hz, 1H), 7.57 (t, J = 7.8 Hz, 1H), 6.93 (s, 2H), 4.40 (d, J = 13.7 Hz, 2H), 3.82 - 3.73 (m, 4H), 3.70 - 3.63 (m, 2H), 2.36 (s, 6H).

### Example 12

### 4-Cyano-N-[4-(3-cyanophenyl)-5-[2-(1-hydroxy-1-methyl-ethyl)-6-methyl-4-pyridyl]thiazol-2-yl]-4-methyl-piperidine-1-carboxamide

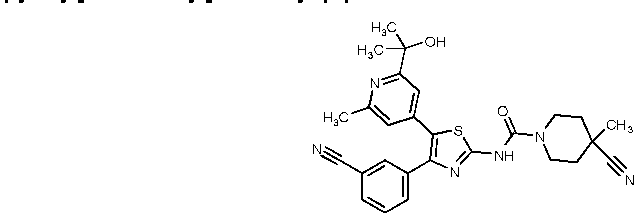

### Step 1: 4-Cyano-N-[4-(3-cyanophenyl)-5-[2-[1-[(4-methoxyphenyl)methoxy]-1-methyl-ethyl]-6-methyl-4-pyridyl]thiazol-2-yl]-4-methyl-piperidine-1-carboxamide

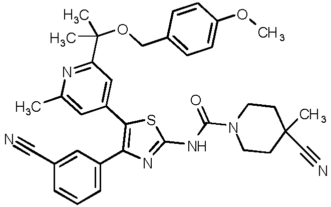

The title compound was prepared from 3-[2-amino-5-[2-[1-[(4-methoxyphenyl)methoxy]-1-methyl-ethyl]-6-methyl-4-pyridyl]thiazol-4-yl]benzonitrile (Intermediate J) and 4-methylpiperidine-4-carbonitrile analogously to Example 6.
LC-MS (Method 3B): Rt 2.37 mins; MS m/z 621.4 = [M+H]+
1H NMR (500 MHz, DMSO-d6) δ 11.34 (s, 1H), 7.84 (t, J = 1.8 Hz, 1H), 7.75 - 7.69 (m, 2H), 7.52 (t, J = 7.9 Hz, 1H), 7.18 - 7.13 (m, 2H), 7.00 - 6.95 (m, 2H), 6.86 - 6.80 (m, 2H), 4.25 - 4.18 (m, 2H), 4.11 (s, 2H), 3.75 (s, 3H), 3.08 - 2.99 (m, 2H), 2.48 (s, 3H), 1.95 - 1.88 (m, 2H), 1.57 - 1.50 (m, 2H), 1.45 (s, 6H), 1.37 (s, 3H).

### Step 2: 4-Cyano-N-[4-(3-cyanophenyl)-5-[2-(1-hydroxy-1-methyl-ethyl)-6-methyl-4-pyridyl]thiazol-2-yl]-4-methyl-piperidine-1-carboxamide

To a solution of 4-cyano-N-[4-(3-cyanophenyl)-5-[2-[1-[(4-methoxyphenyl)methoxy]-1-methyl-ethyl]-6-methyl-4-pyridyl]thiazol-2-yl]-4-methyl-piperidine-1-carboxamide (step 1) (77 mg, 0.12 mmol) in DCM (3.6 mL) was added trifluoroacetic acid (0.38 mL, 4.96 mmol) and the mixture stirred at room temperature for 45 mins. The reaction mixture was diluted with DCM (15 mL) and washed with sat. NaHCO₃ solution (2 x 20 mL) before being dried over Na₂SO₄ and the solvent removed *in vacuo.* Purification by chromatography on silica eluting with 3% MeOH in DCM afforded an oil which was suspended in 10% Et₂O/hexane causing a precipitate to form which was collected by filtration and dried to afford the title compound as an off-white solid.
LC-MS (Method 8B): Rt 4.20 mins; MS m/z 501.2 = [M+H]+
1H NMR (500 MHz, DMSO-d6) δ 11.33 (s, 1H), 7.85 (s, 1H), 7.83 (d, J = 7.8 Hz, 1H), 7.77 (d, J = 7.8 Hz, 1H), 7.59 (t, J = 7.8 Hz, 1H), 7.29 (s, 1H), 7.03 (s, 1H), 5.09 (s, 1H), 4.23 (apr d, J = 14.1 Hz, 2H), 3.04 (apr t, J = 13.1 Hz, 2H), 2.43 (s, 3H), 1.93 (apr d, J = 13.6 Hz, 2H), 1.58 - 1.50 (m, 2H), 1.38 (s, 3H), 1.36 (s, 6H).

### Example 12.1

### N-[4-(3-Cyanophenyl)-5-[2-(1-hydroxy-1-methyl-ethyl)-6-methyl-4-pyridyl]thiazol-2-yl]-6-oxa-2-azaspiro[3.4]octane-2-carboxamide

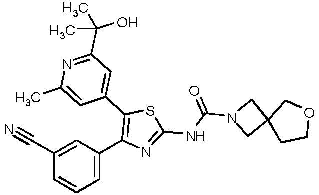

### Step 1: N-[4-(3-Cyanophenyl)-5-[2-[1-[(4-methoxyphenyl)methoxy]-1-methyl-ethyl]-6-methyl-4-pyridyl]thiazol-2-yl]-6-oxa-2-azaspiro[3.4]octane-2-carboxamide

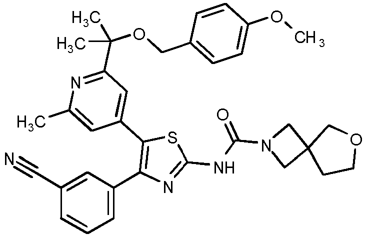

The title compound was prepared from 3-[2-amino-5-[2-[1-[(4-methoxyphenyl)methoxy]-1-methyl-ethyl]-6-methyl-4-pyridyl]thiazol-4-yl]benzonitrile (Intermediate J) and 6-oxa-2-azaspiro[3.4]octane analogously to Example 6.
LC-MS (Method 3B): Rt 2.17 mins; MS m/z 610.3 = [M+H]+ 1H NMR (500 MHz, Methanol-d4) δ 7.82 (t, J = 1.6 Hz, 1H), 7.69 (dt, J = 7.8, 1.6 Hz, 1H), 7.51 (dt, J = 7.8, 1.6 Hz, 1H), 7.38 (t, J = 7.8 Hz, 1H), 7.26 (d, J = 1.6 Hz, 1H), 7.15 (d, J = 1.6 Hz, 1H), 7.05 - 7.01 (m, 2H), 6.86 - 6.80 (m, 2H), 4.16 (s, 2H), 4.11 (s, 4H), 3.89 (s, 2H), 3.86 (t, J = 7.0 Hz, 2H), 3.80 (s, 3H), 2.54 (s, 3H), 2.23 (t, J = 7.0 Hz, 2H), 1.54 (s, 6H). 1 x NH proton not observed.

### Step 2: N-[4-(3-Cyanophenyl)-5-[2-(1-hydroxy-1-methyl-ethyl)-6-methyl-4-pyridyl]thiazol-2-yl]-6-oxa-2-azaspiro[3.4]octane-2-carboxamide

The title compound was prepared from N-[4-(3-cyanophenyl)-5-[2-[1-[(4-methoxyphenyl)methoxy]-1-methyl-ethyl]-6-methyl-4-pyridyl]thiazol-2-yl]-6-oxa-2-azaspiro[3.4]octane-2-carboxamide (step 1) and TFA analogously to Example 12 step 2. LC-MS (Method 8B): Rt 3.18 mins; MS m/z 490.1 = [M+H]+
1H NMR (500 MHz, DMSO-d6) δ 11.36 (s, 1H), 7.85 - 7.81 (m, 2H), 7.76 (dt, J = 7.8, 1.6 Hz, 1H), 7.60 - 7.56 (m, 1H), 7.29 (d, J = 1.6 Hz, 1H), 7.02 (d, J = 1.6 Hz, 1H), 5.09 (s, 1H), 4.02 (s, 4H), 3.78 (s, 2H), 3.72 (t, J = 7.0 Hz, 2H), 2.42 (s, 3H), 2.13 (t, J = 7.0 Hz, 2H), 1.36 (s, 6H).

### Example 12.2

### N-[4-(3-Cyanophenyl)-5-[2-(1-hydroxy-1-methyl-ethyl)-6-methyl-4-pyridyl]thiazol-2-yl]-2-oxa-6-azaspiro[3.3]heptane-6-carboxamide

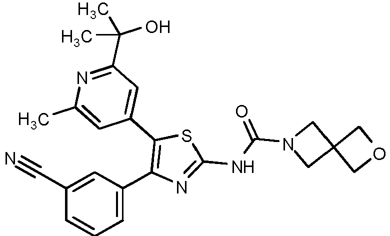

### Step 1: N-[4-(3-Cyanophenyl)-5-[2-[1-[(4-methoxyphenyl)methoxy]-1-methyl-ethyl]-6-methyl-4-pyridyl]thiazol-2-yl]-2-oxa-6-azaspiro[3.3]heptane-6-carboxamide

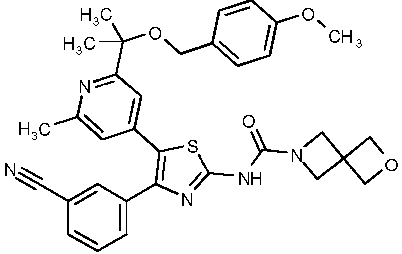

The title compound was prepared from 3-[2-amino-5-[2-[1-[(4-methoxyphenyl)methoxy]-1-methyl-ethyl]-6-methyl-4-pyridyl]thiazol-4-yl]benzonitrile (Intermediate J) and 2-oxa-6-azaspiro[3.3]heptane analogously to Example 6.
LC-MS (Method 3B): Rt 2.06 mins; MS m/z 596.3 = [M+H]+
1H NMR (500 MHz, DMSO-d6) δ 11.37 (br s, 1H), 7.83 (br t, J = 1.7 Hz, 1H), 7.74 - 7.71 (m, 1H), 7.73 - 7.68 (m, 1H), 7.52 (apr t, J = 7.8 Hz, 1H), 7.16 - 7.12 (m, 2H), 6.99 - 6.95 (m, 2H), 6.85 - 6.81 (m, 2H), 4.67 (s, 4H), 4.20 (s, 4H), 4.10 (s, 2H), 3.75 (s, 3H), 2.48 (s, 3H), 1.45 (s, 6H).

### Step 2: N-[4-(3-Cyanophenyl)-5-[2-(1-hydroxy-1-methyl-ethyl)-6-methyl-4-pyridyl]thiazol-2-yl]-2-oxa-6-azaspiro[3.3]heptane-6-carboxamide

The title compound was prepared from N-[4-(3-cyanophenyl)-5-[2-[1-[(4-methoxyphenyl)methoxy]-1-methyl-ethyl]-6-methyl-4-pyridyl]thiazol-2-yl]-2-oxa-6-azaspiro[3.3]heptane-6-carboxamide (step 1) and TFA analogously to Example 12 step 2. LC-MS (Method 8B): Rt 3.68 mins; MS m/z 476.2 = [M+H]+
1H NMR (500 MHz, DMSO-d6) δ 11.35 (s, 1H), 7.85 - 7.79 (m, 2H), 7.75 (dt, J = 7.9, 1.4 Hz, 1H), 7.61 - 7.54 (m, 1H), 7.27 (d, J = 1.6 Hz, 1H), 7.01 (d, J = 1.6 Hz, 1H), 5.08 (s, 1H), 4.67 (s, 4H), 4.20 (s, 4H), 2.41 (s, 3H), 1.35 (s, 6H).

### Example 12.3

### N-[4-(3-Cyanophenyl)-5-[2-(1-hydroxyethyl)-6-methyl-4-pyridyl]thiazol-2-yl]-2-oxa-6-azaspiro[3.3]heptane-6-carboxamide

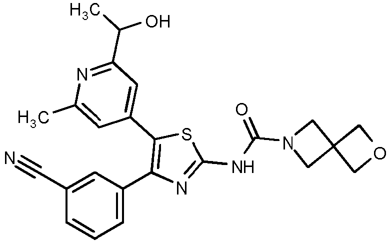

### Step 1: N-[4-(3-Cyanophenyl)-5-[2-[1-[(4-methoxyphenyl)methoxy]ethyl]-6-methyl-4-pyridyl]thiazol-2-yl]-2-oxa-6-azaspiro[3.3]heptane-6-carboxamide

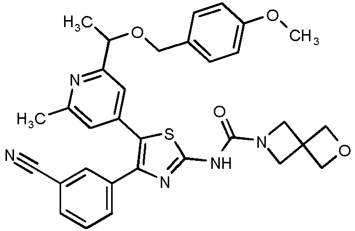

The title compound was prepared from 3-[2-amino-5-[2-[1-[(4-methoxyphenyl)methoxy]ethyl]-6-methyl-4-pyridyl]thiazol-4-yl]benzonitrile (Intermediate PA) and 2-oxa-6-azaspiro[3.3]heptane analogously to Example 6.
LC-MS (Method 3B): Rt 1.81 mins; MS m/z 580.4 = [M-H]-
1H NMR (500 MHz, DMSO-d6) δ 11.38 (br s, 1H), 7.85 (t, J = 1.7 Hz, 1H), 7.75 - 7.71 (m, 2H), 7.53 (apr t, J = 7.8 Hz, 1H), 7.16 (br d, J = 1.6 Hz, 1H), 7.06 - 7.02 (m, 2H), 6.98 (br d, J = 1.6 Hz, 1H), 6.88 - 6.84 (m, 2H), 4.67 (s, 4H), 4.42 (q, J = 6.5 Hz, 1H), 4.23 - 4.18 (m, 5H), 4.16 (d, J = 11.5 Hz, 1H), 3.75 (s, 3H), 2.46 (s, 3H), 1.27 (d, J = 6.5 Hz, 3H).

### Step 2: N-[4-(3-Cyanophenyl)-5-[2-(1-hydroxyethyl)-6-methyl-4-pyridyl]thiazol-2-yl]-2-oxa-6-azaspiro[3.3]heptane-6-carboxamide

The title compound was prepared from N-[4-(3-cyanophenyl)-5-[2-[1-[(4-methoxyphenyl)methoxy]ethyl]-6-methyl-4-pyridyl]thiazol-2-yl]-2-oxa-6-azaspiro[3.3]heptane-6-carboxamide (step 1) and TFA analogously to Example 12 step 2.
LC-MS (Method 8B): Rt 3.67 mins; MS m/z 462.2 = [M+H]+
1H NMR (500 MHz, DMSO-d6) δ 11.36 (s, 1H), 7.84 (br t, J = 1.5 Hz, 1H), 7.82 (br dt, J = 7.7, 1.5 Hz, 1H), 7.74 (br dt, J = 8.0, 1.5 Hz, 1H), 7.57 (apr t, J = 7.8 Hz, 1H), 7.12 (s, 1H), 7.01 (s, 1H), 5.23 (d, J = 4.5 Hz, 1H), 4.67 (s, 4H), 4.65 - 4.56 (m, 1H), 4.21 (s, 4H), 2.40 (s, 3H), 1.26 (d, J = 6.6 Hz, 3H).

### Example 12.3a and Example 12.3b

Racemic N-[4-(3-Cyanophenyl)-5-[2-(1-hydroxyethyl)-6-methyl-4-pyridyl]thiazol-2-yl]-2-oxa-6-azaspiro[3.3]heptane-6-carboxamide (Example 12.3) was dissolved to 50 mg/mL in EtOH/DCM (4:1) and purified by SFC to afford the individual enantiomers:

### Purification Conditions;

| | |
|---|---|
| Column Details | Lux iC5 (21.2mm x 250mm, 5um) |
| Column Temp | 40 °C |
| Flow Rate | 50 mL/min |
| BPR | 100 BarG |
| Detector Wavelength | 218 nm |
| Injection Volume | 250 uL (2 mg) |
| Isocratic Conditions | 50:50 EtOH:CO₂ |

### Chiral Purity Analysis Conditions;

| | |
|---|---|
| Column Details | Lux iC5 (4.6mm x 250mm, 5um) |
| Column Temp | 40 °C |
| Flow Rate | 4 mL/min |
| Detector Wavelength | 210-400 nm |
| Injection Volume | 1.0 uL |
| BPR | 125 BarG |
| Isocratic Conditions | 50:50 EtOH:CO₂ (0.2% v/v NH₃) |

### Example 12.3a

### N-[4-(3-Cyanophenyl)-5-[2-[(1R)-1-hydroxyethyl]-6-methyl-4-pyridyl]thiazol-2-yl]-2-oxa-6-azaspiro[3.3]heptane-6-carboxamide or N-[4-(3-cyanophenyl)-5-[2-[(1S)-1-hydroxyethyl]-6-methyl-4-pyridyl]thiazol-2-yl]-2-oxa-6-azaspiro[3.3]heptane-6-carboxamide

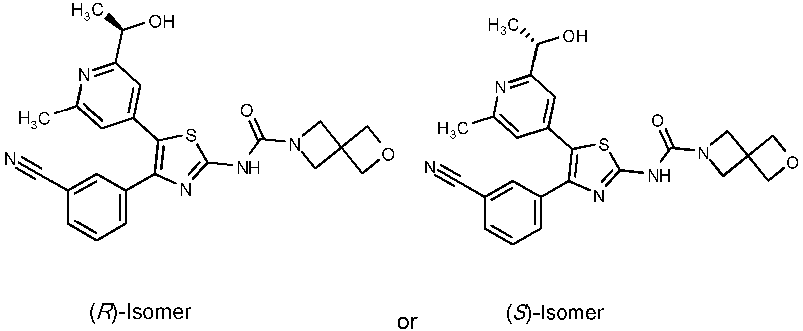

### First Eluted Peak: Chiral LCMS retention time: = 3.12 mins

LC-MS (Method 8B): Rt 2.56 mins; MS m/z 462.1 = [M+H]+
1H NMR (500 MHz, DMSO-d6) δ 11.36 (s, 1H), 7.84 (t, J = 1.6 Hz, 1H), 7.82 (dt, J = 7.8, 1.6 Hz, 1H), 7.74 (dt, J = 7.8, 1.6 Hz, 1H), 7.57 (t, J = 7.8 Hz, 1H), 7.12 (d, J = 1.7 Hz, 1H), 7.01 (d, J = 1.7 Hz, 1H), 5.23 (d, J = 4.5 Hz, 1H), 4.67 (s, 4H), 4.60 (td, J = 6.5, 4.5 Hz, 1H), 4.21 (s, 4H), 2.40 (s, 3H), 1.26 (d, J = 6.5 Hz, 3H).

### Example 12.3b

### N-[4-(3-Cyanophenyl)-5-[2-[(1R)-1-hydroxyethyl]-6-methyl-4-pyridyl]thiazol-2-yl]-2-oxa-6-azaspiro[3.3]heptane-6-carboxamide or N-[4-(3-cyanophenyl)-5-[2-[(1S)-1-hydroxyethyl]-6-methyl-4-pyridyl]thiazol-2-yl]-2-oxa-6-azaspiro[3.3]heptane-6-carboxamide

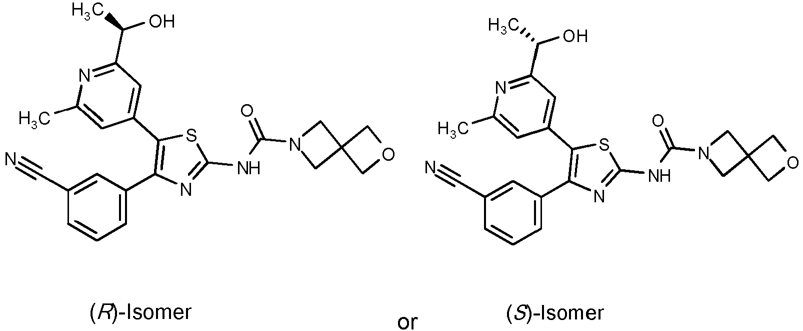

### Second Eluted Peak: Chiral LCMS retention time: = 4.12 mins

LC-MS (Method 8B): Rt 2.74 mins; MS m/z 462.1 = [M+H]+
1H NMR (500 MHz, DMSO-d6) δ 11.36 (s, 1H), 7.84 (t, J = 1.6 Hz, 1H), 7.82 (dt, J = 7.8, 1.6 Hz, 1H), 7.74 (dt, J = 7.8, 1.6 Hz, 1H), 7.57 (t, J = 7.8 Hz, 1H), 7.12 (d, J = 1.7 Hz, 1H), 7.01 (d, J = 1.7 Hz, 1H), 5.23 (d, J = 4.4 Hz, 1H), 4.67 (s, 4H), 4.63 - 4.58 (m, 1H), 4.21 (s, 4H), 2.40 (s, 3H), 1.26 (d, J = 6.5 Hz, 3H).

### Example 13

### (3S,5R)-N-[4-(3-Cyanophenyl)-5-(2,6-dimethyl-4-pyridyl)thiazol-2-yl]-3,5-dimethylpiperazine-1-carboxamide

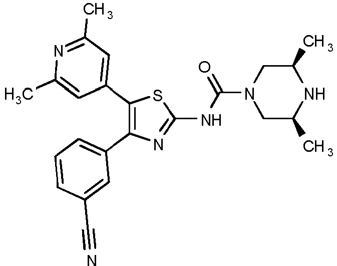

### Step 1: tert-Butyl rac-(2S,6R)-4-[[4-(3-cyanophenyl)-5-(2,6-dimethyl-4-pyridyl)thiazol-2-yl]carbamoyl]-2,6-dimethyl-piperazine-1-carboxylate

The title compound was prepared from 3-[2-amino-5-(2,6-dimethyl-4-pyridyl)thiazol-4-yl]benzonitrile (Intermediate C) and (2R,6S)-tert-butyl 2,6-dimethylpiperazine-1-carboxylate analogously to Example 6.
LC-MS (Method 2B): Rt 1.69 mins; MS m/z 547.2 = [M+H]+
1H NMR (500 MHz, DMSO-d6) δ 11.39 (s, 1H), 7.90 (d, J = 1.8 Hz, 1H), 7.83 (dt, J = 7.8, 1.5 Hz, 1H), 7.72 (dt, J = 8.0, 1.5 Hz, 1H), 7.57 (t, J = 7.8 Hz, 1H), 6.94 (s, 2H), 4.17 (d, J = 13.4 Hz, 2H), 4.13 - 4.01 (m, 2H), 3.12 - 3.04 (m, 2H), 2.37 (s, 6H), 1.43 (s, 9H), 1.15 (d, J = 2.5 Hz, 6H).

### Step 2: (3S,5R)-N-[4-(3-Cyanophenyl)-5-(2,6-dimethyl-4-pyridyl)thiazol-2-yl]-3,5-dimethylpiperazine-1-carboxamide

To a stirred solution of *tert*-butyl (2S,6R)-4-[[4-(3-cyanophenyl)-5-(2,6-dimethyl-4-pyridyl)thiazol-2-yl]carbamoyl]-2,6-dimethyl-piperazine-1-carboxylate (step 1) (100 mg, 0.14 mmol) in chloroform (2 mL) was added dropwise trimethylsilyl iodide (0.08 mL, 0.58 mmol) and the reaction mixture was stirred at room temperature for 30 mins. The resulting mixture was concentrated under a flow of nitrogen, chloroform (2 mL) was added and concentrated once again. Purification by C18 reverse phase chromatography eluting with a gradient of 15 to 40% MeCN in water (+0.1% ammonium hydroxide modifier) followed by freeze drying of the product fractions afforded the title compound as a white solid.
LC-MS (Method 8B): Rt 3.04 mins; MS m/z 447.2 = [M+H]+
1H NMR (500 MHz, DMSO-d6) δ 11.16 (s, 1H), 7.90 (t, J = 1.8 Hz, 1H), 7.82 (dt, J = 7.7, 1.5 Hz, 1H), 7.71 (dt, J = 8.0, 1.5 Hz, 1H), 7.56 (t, J = 7.8 Hz, 1H), 6.93 (s, 2H), 4.10 (d, J = 12.5 Hz, 2H), 2.74 - 2.57 (m, 2H), 2.37 (s, 6H), 2.35 - 2.27 (m, 2H), 0.99 (d, J = 6.2 Hz, 6H). 1 x NH proton not observed.

The compounds of the following tabulated Examples (Table Ex13) were prepared analogously to Example 13 from 3-[2-amino-5-(2,6-dimethyl-4-pyridyl)thiazol-4-yl]benzonitrile (Intermediate C)) and the appropriate amine (step 1) followed by deprotection using trimethylsilyl iodide (step 2).

**Table Ex13**

| **Ex.** | **Structure and Name** | **Retention Time, [M+H]+, ¹H NMR** |
|---|---|---|
| 13.1 | 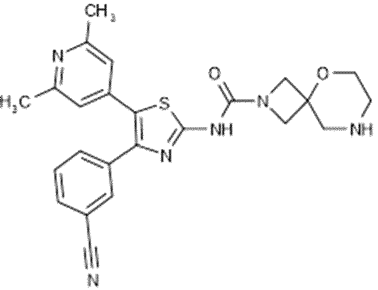 | LC-MS (Method 8B): Rt 2.75 mins; MS m/z 461.1 = [M+H]+ |
| | | 1H NMR (500 MHz, DMSO-d6) δ 11.37 (s, 1H), 7.88 (t, J = 1.7 Hz, 1H), 7.82 (dt, J = 7.7, 1.4 Hz, 1H), 7.71 (dt, J = 8.0, 1.5 Hz, 1H), 7.57 (t, J = 7.8 Hz, 1H), 6.94 (s, 2H), 3.99 - 3.88 (m, 2H), 3.88 - 3.65 (m, 2H), 3.54 (t, J = 4.6 Hz, 2H), 2.80 (s, 2H), 2.73 - 2.57 (m, 2H), 2.37 (s, 6H).1 x NH proton not observed. |
| | N- [4-(3-Cyanophenyl)-5-(2,6-dimethyl-4- | Intermediate Data: |
| | pyridyl)thiazol-2-yl]-5-oxa-2,8-diazaspiro[3.5]nonane-2-carboxamide | LC-MS (Method 2B): Rt 1.56 mins; MS m/z 561.2 = [M+H]+ |
| | | 1H NMR (500 MHz, DMSO-d6) δ 11.43 (s, 1H), 7.88 (s, 1H), 7.82 (dt, J = 7.7, 1.5 Hz, 1H), 7.71 (dt, J = 8.0, 1.5 Hz, 1H), 7.61 - 7.49 (m, 1H), 6.94 (s, 2H), 4.02 - 3.82 (m, 4H), 3.71 (d, J = 1.7 Hz, 1H), 3.59 (tt, J = 19.4, 4.9 Hz, 3H), 3.51 (s, 2H), 2.37 (s, 6H), 1.43 (s, 9H). |
| | Prepared via: | |
| | 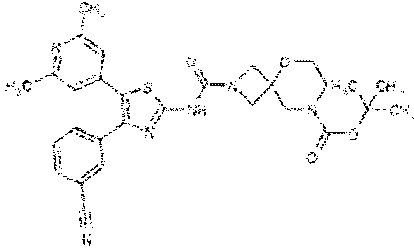 | |
| | *tert*-Butyl 2-[[4-(3-cyanophenyl)-5-(2,6-dimethyl-4-pyridyl)thiazol-2-yl]carbamoyl]-5-oxa-2,8-diazaspiro[3.5]nonane-8-carboxylate | |
| 13.2 | 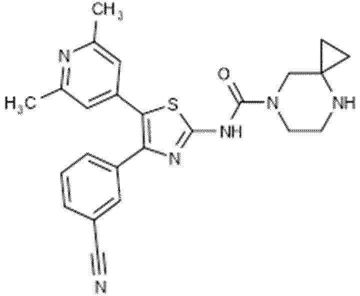 | LC-MS (Method 8A): Rt 1.82 mins; MS m/z 445.2 = [M+H]+ |
| | | 1H NMR (500 MHz, DMSO-d6) δ 11.10 (s, 1H), 7.89 (t, J = 1.9 Hz, 1H), 7.82 (d, J = 7.6 Hz, 1H), 7.71 (d, J = 8.0 Hz, 1H), 7.56 (t, J = 7.8 Hz, 1H), 6.93 (s, 2H), 3.53 - 3.42 (m, 2H), 3.43 - 3.38 (m, 2H), 2.76 (t, J = 5.0 Hz, 2H), 2.37 (s, 6H), 0.52 - 0.42 (m, 4H). 1 x NH proton not observed. |
| | | Intermediate Data: |
| | N-[4-(3-Cyanophenyl)-5-(2,6-dimethyl-4-pyridyl)thiazol-2-yl]-4,7-diazaspiro[2.5]octane-7-carboxamide | LC-MS (Method 2B): Rt 1.72 mins; MS m/z 545.3 = [M+H]+ |
| | | 1H NMR (500 MHz, DMSO-d6) δ 11.22 (s, 1H), 7.89 (s, 1H), 7.83 (d, J= 7.8 Hz, 1H), 7.71 (d, J= 8.0 Hz, 1H), 7.57 (t, J= 7.9 Hz, 1H), 6.93 (s, 2H), 3.53 (s, 2H), 3.44 (d, J= 16.6 Hz, 4H), 2.36 (s, 6H), 1.43 (s, 9H), 0.91 (s, 2H), 0.84 (s, 2H). |
| | Prepared via: | |
| | 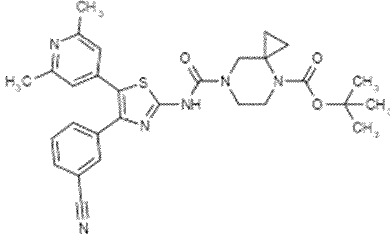 | |
| | *tert*-Butyl 7-[[4-(3-cyanophenyl)-5-(2,6-dimethyl-4-pyridyl)thiazol-2-yl]carbamoyl]-4, 7-diazaspiro[2.5]octane-4-carboxylate | |
| 13.3 | 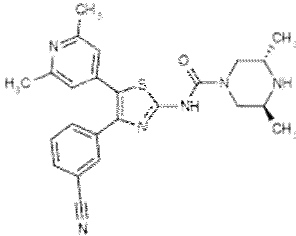 | LC-MS (Method 8A): Rt 1.80 mins; MS m/z 447.2 = [M+H]+ |
| | | 1H NMR (500 MHz, DMSO-d6) δ 7.89 (s, 1H), 7.81 (d, J = 7.7 Hz, 1H), 7.70 (d, J = 7.9 Hz, 1H), 7.55 (t, J = 7.8 Hz, 1H), 6.92 (s, 2H), 3.60 - 3.47 (m, 2H), 3.18 (dd, J = 13.0, 6.3 Hz, 2H), 3.12 - 3.03 (m, 2H), 2.36 (s, 6H), 1.01 (d, J = 6.4 Hz, 6H) 2 x NH protons not observed. |
| | (3S,5S)-N-[4-(3-Cyanophenyl)-5-(2,6-dimethyl-4-pyridyl)thiazol-2-yl]-3,5-dimethyl-piperazine-1-carboxamide Prepared via: | Intermediate Data: |
| | | LC-MS (Method 2B): Rt 1.70 mins; MS m/z 547.3 = [M+H]+ |
| | | 1H NMR (500 MHz, DMSO-d6) δ 11.19 (s, 1H), 7.90 (s, 1H), 7.83 (d, J = 7.7 Hz, 1H), 7.71 (d, J = 8.1 Hz, 1H), 7.57 (t, J = 7.8 Hz, 1H), 6.94 (s, 2H), 4.18 - 3.92 (m, 2H), 3.81 - 3.66 (m, 2H), 3.67 - 3.49 (m, 2H), 2.37 (s, 6H), 1.43 (s, 9H), 1.19 (s, 6H). |
| | 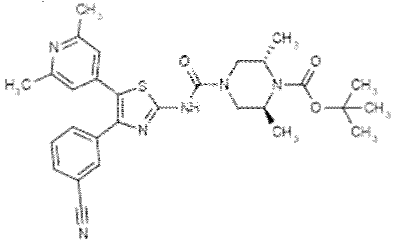 | |
| | *tert*-Butyl (2S,6S)-4-[[4-(3-cyanophenyl)-5-(2,6-dimethyl-4-pyridyl)thiazol-2-yl]carbamoyl]-2,6-dimethyl-piperazine-1-carboxylate | |
| 13.4 | 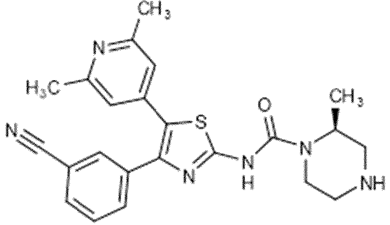 | LC-MS (Method 8B): Rt 3.84 mins; MS m/z 433.3 = [M+H]+ |
| | | 1H NMR (500 MHz, DMSO-d6) δ 7.90 (s, 1H), 7.83 (d, J = 7.8 Hz, 1H), 7.71 (d, J = 7.8 Hz, 1H), 7.57 (t, J = 7.8 Hz, 1H), 6.93 (s, 2H), 4.41 - 4.34 (m, 1H), 3.95 (d, J = 13.3 Hz, 1H), 3.05 (t, J = 12.5 Hz, 1H), 2.98 (d, J = 12.5 Hz, 1H), 2.87 - 2.80 (m, 1H), 2.65 - 2.59 (m, 2H), 2.36 (s, 6H), 1.23 (d, J = 6.9 Hz, 3H). 2 x NH protons not observed. |
| | (2S)-N-[4-(3-Cyanophenyl)-5-(2,6-dimethyl-4-pyridyl)thiazol-2-yl]-2-methyl-piperazine-1-carboxamide | |
| | | Intermediate Data: |
| | Prepared via: | LC-MS (Method 5B): Rt 3.32 mins; MS m/z 533.5 = [M+H]+ |
| | | |
| | 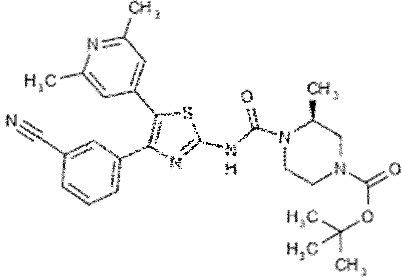 | 1H NMR (500 MHz, DMSO-d6) δ 11.28 (s, 1H), 7.89 (s, 1H), 7.82 (d, J= 7.7 Hz, 1H), 7.71 (d, J= 8.0 Hz, 1H), 7.56 (t, J= 7.9 Hz, 1H), 6.92 (s, 2H), 4.50 - 4.40 (m, 1H), 4.01 - 3.96 (m, 1H), 3.79 - 3.71 (m, 1H), 3.12 - 3.03 (m, 2H), 2.36 (s, 6H), 1.43 (s, 9H), 1.11 (d, J= 6.5 Hz, 3H). 2 x CH protons not observed. |
| | *tert*-Butyl (3S)-4-[[4-(3-cyanophenyl)-5-(2,6-dimethyl-4-pyridyl)thiazol-2-yl]carbamoyl]-3-methyl-piperazine-1-carboxylate | |
| 13.5 | 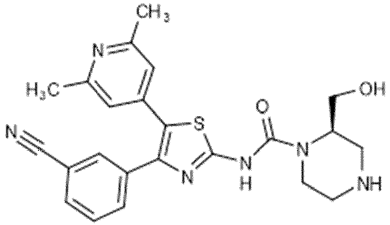 | LC-MS (Method 15B): Rt 5.62 mins; MS m/z 449.3 = [M+H]+ |
| | | 1H NMR (500 MHz, δ 7.89 (s, 1H), 7.75 - 7.65 (m, 2H), 7.49 (t,J= 7.9 Hz, 1H), 6.99 (s, 2H), 4.25 - 4.18 (m, 1H), 4.02 (d,J= 13.1 Hz, 1H), 3.90 (t,J= 11.1, 7.8 Hz, 1H), 3.81 (dd,J= 11.1, 6.6 Hz, 1H), 3.20 - 3.10 (m, 2H), 3.02 (d,J= 13.1 Hz, 1H), 2.88 (dd,J= 13.1, 4.5 Hz, 1H), 2.73 (td,J= 12.6, 3.7 Hz, 1H), 2.43 (s, 6H). OH and 2 x NH protonsnot observed. |
| | (2R)-N-[4-(3-Cyanophenyl)-5-(2,6-dimethyl-4-pyridyl)thiazol-2-yl]-2-(hydroxymethyl)piperazine-1-carboxamide | |
| | | Intermediate Data: |
| | Prepared via: | LC-MS (Method 5B): Rt 2.61 mins; MS m/z 549.5 = [M+H]+ |
| | | 1H NMR (500 MHz, DMSO-d6) δ 11.13 (s, 1H), 7.88 (s, 1H), 7.82 (d,J= 7.7 Hz, 1H), 7.70 (d,J= 8.0 Hz, 1H), 7.56 (t,J= 7.8 Hz, 1H), 6.92 (s, 2H), 4.93 (s, 1H), 4.33 - 4.23 (m, 1H), 4.00 - 3.91 (m, 2H), 3.89 - 3.75 (m, 2H), 3.46 (d,J= 7.2 |
| | 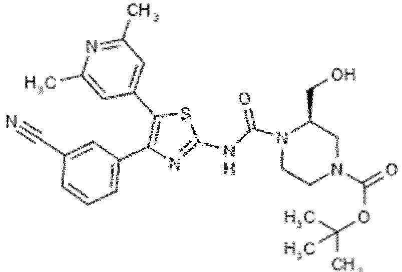 | Hz, 2H), 3.06 - 2.97 (m, 2H), 2.36 (s, 6H), 1.41 (s, 9H). |
| | *tert*-Butyl (3R)-4-[[4-(3-cyanophenyl)-5-(2,6-dimethyl-4-pyridyl)thiazol-2-yl]carbamoyl]-3-(hydroxymethyl)piperazine-1-carboxylate | |
| 13.6 | 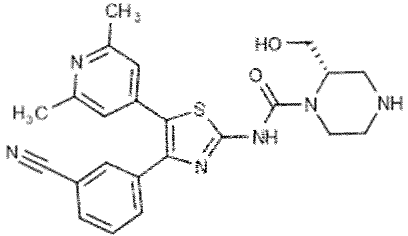 | LC-MS (Method 8B): Rt 3.48 mins; MS m/z 449.3 = [M+H]+ |
| | | 1H NMR (500 MHz, DMSO-d6) δ 10.77 (br s, 1H), 7.88 (s, 1H), 7.81 (d, J = 7.7 Hz, 1H), 7.70 (d, J = 8.0 Hz, 1H), 7.55 (apr t, J = 7.8 Hz, 1H), 6.92 (s, 2H), 4.78 (br s, 1H), 4.15 - 4.07 (m, 1H), 3.90 (d, J = 13.0 Hz, 1H), 3.69 - 3.58 (m, 2H), 3.00 - 2.90 (m, 2H), 2.87 (d, J = 12.4 Hz, 1H), 2.67 - 2.62 (m, 1H), 2.56 - 2.51 (m, 1H), 2.36 (s, 6H). 1 x NH not observed. |
| | (2S)-N-[4-(3-Cyanophenyl)-5-(2,6-dimethyl-4-pyridyl)thiazol-2-yl]-2-(hydroxymethyl)piperazine-1-carboxamide | |
| | | Intermediate Data: |
| | Prepared via: | LC-MS (Method 3B): Rt 1.66 mins; MS m/z 549.2 = [M+H]+ |
| | 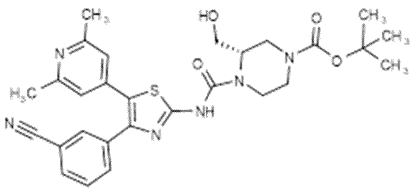 | 1H NMR (500 MHz, DMSO-d6) δ 11.11 (br s, 1H), 7.88 (t, J = 1.5 Hz, 1H), 7.82 (dt, J = 7.7, 1.5 Hz, 1H), 7.70 (dt, J = 8.1, 1.5 Hz, 1H), 7.56 (apr t, J = 7.8 Hz, 1H), 6.93 (s, 2H), 4.92 (br s, 1H), 4.28 (br s, 1H), 3.96 (dd, J = 24.8, 13.4 Hz, 2H), 3.86 (br s, 1H), 3.47 (d, J = 7.2 Hz, 2H), 3.10 - 2.97 (m, 2H), 2.87 (br s, 1H), 2.36 (s, 6H), 1.41 (s, 9H) |
| | *tert*-Butyl (3S)-4-[[4-(3-cyanophenyl)-5-(2,6-dimethyl-4-pyridyl)thiazol-2-yl]carbamoyl]-3-(hydroxymethyl)piperazine-1-carboxylate | |
| 13.7 | 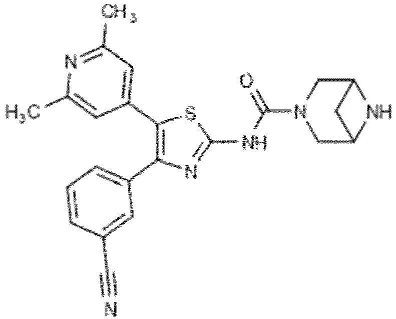 | LC-MS (Method 8B): Rt 3.64 mins; MS m/z 431.3 = [M+H]+ |
| | | 1H NMR (500 MHz, δ 7.92 (t, J = 1.5 Hz, 1H), 7.72 (dt, J = 8.0, 1.5 Hz, 1H), 7.69 (dt, J = 7.9, 1.5 Hz, 1H), 7.49 (apr t, J = 7.8 Hz, 1H), 7.01 (s, 2H), 3.86 - 3.74 (m, 6H), 2.79 - 2.71 (m, 1H), 2.44 (s, 6H), 1.63 (d, J = 9.4 Hz, 1H). 2 x NH protons not observed. |
| | | Intermediate Data: |
| | | LC-MS (Method 3B): Rt 1.85 mins; MS m/z 529.4 = [M-H]- |
| | N-[4-(3-Cyanophenyl)-5-(2,6-dimethyl-4-pyridyl)thiazol-2-yl]-3,6-diazabicyclo[3.1.1]heptane-3-carboxamide | |
| | | 1H NMR (500 MHz, DMSO-d6) δ 11.17 (s, 1H), 7.91 (d, J = 1.8 Hz, 1H), 7.83 (d, J = 7.7 Hz, 1H), 7.72 (d, J = 8.0 Hz, 1H), 7.57 (t, J = 7.8 Hz, 1H), 6.94 (s, 2H), 4.13 (d, J = 6.4 Hz, 3H), 3.96 (d, J = 12.1 Hz, 1H), 3.55 (s, 1H), 2.37 (s, 6H), 1.44 (d, J = 8.9 Hz, 1H), 1.36 (s, 9H). 2 x CH protons not observed. |
| | Prepared via: | |
| | 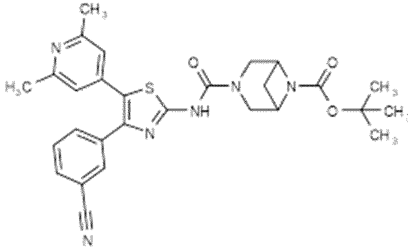 | |
| | *tert*-Butyl 3-[[4-(3-cyanophenyl)-5-(2,6-dimethyl-4-pyridyl)thiazol-2-yl]carbamoyl]-3,6-diazabicyclo[3.1.1]heptane-6-carboxylate | |
| 13.8 | 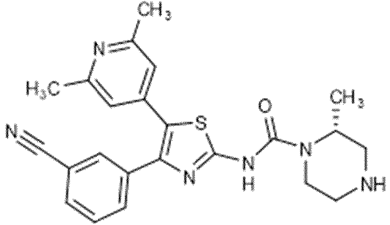 | LC-MS (Method 8B): Rt 3.89 mins; MS m/z 433.3 = [M+H]+ |
| | | 1H NMR (500 MHz, δ 7.92 (t, J = 1.5 Hz, 1H), 7.73 (dt, J = 7.9, 1.5 Hz, 1H), 7.71 (dt, J = 7.9, 1.5 Hz, 1H), 7.51 (t, J = 7.9 Hz, 1H), 7.02 (s, 2H), 4.39 - 4.33 (m, 1H), 3.94 (d, J = 13.6 Hz, 1H), 3.20 (td, J = 12.9, 3.3 Hz, 1H), 3.04 (d, J = 12.5 Hz, 1H), 2.95 (dd, J = 12.9, 4.1 Hz, 1H), 2.89 (d, J = 12.9 Hz, 1H), 2.72 (td, J = 12.6, 3.7 Hz, 1H), 2.45 (s, 6H), 1.35 (d, J = 6.8 Hz, 3H). 2 x NH protons not observed. |
| | (2R)-N-[4-(3-Cyanophenyl)-5-(2,6-dimethyl-4-pyridyl)thiazol-2-yl]-2-methyl-piperazine-1-carboxamide | |
| | | Intermediate Data: |
| | | LC-MS (Method 3B): Rt 1.94 mins; MS m/z 531.4 = [M-H]- |
| | | 1H NMR (500 MHz, DMSO-d6) δ 11.27 (s, 1H), 7.88 (t, J= 1.5 Hz, 1H), 7.81 (d, J= 7.8 Hz, 1H), 7.70 (dt, J= 7.8, 1.5 Hz, 1H), 7.56 (t, J= 7.8 Hz, 1H), 6.91 (s, 2H), 4.44 (s, 1H), 3.99 (d, J= 13.5 Hz, 1H), 3.96-3.82 (m, 2H), 3.74 (d, J= 13.5 |
| | Prepared via: | Hz, 1H), 3.12 - 3.02 (m, 2H), 2.35 (s, 6H), 1.42 (s, 9H), 1.10 (d, J= 6.7 Hz, 3H). |
| | 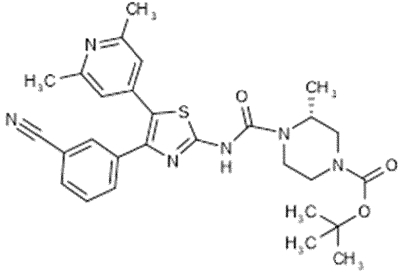 | |
| | *tert*-Butyl (3R)-4-[[4-(3-cyanophenyl)-5-(2,6-dimethyl-4-pyridyl)thiazol-2-yl]carbamoyl]-3-methyl-piperazine-1-carboxylate | |
| 13.9 | 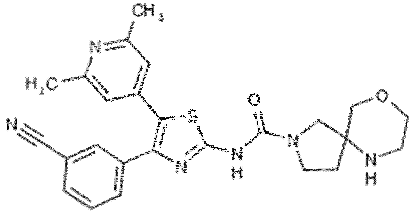 | LC-MS (Method 8B): Rt 3.67 mins; MS m/z 475.2 = [M+H]+ |
| | | 1H NMR (500 MHz, Methanol-d4) δ 7.91 (t, J= 1.5 Hz, 1H), 7.72 (dt, J= 7.9, 1.5 Hz, 1H), 7.69 (dt, J= 7.9, 1.5 Hz, 1H), 7.49 (t, J= 7.9 Hz, 1H), 7.00 (s, 2H), 3.78 - 3.43 (m, 8H), 2.99 - 2.81 (m, 2H), 2.43 (s, 6H), 2.12 - 2.02 (m, 1H), 1.99 - 1.88 (m, 1H). 2 x NH protons not observed. |
| | Prepared via: | |
| | 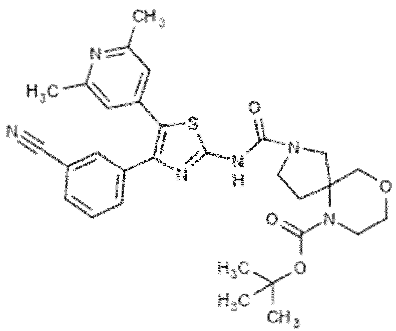 | |
| | | Intermediate Data: |
| | | LC-MS (Method 3B): Rt 1.96 mins; MS m/z 575.2 = [M+H]+ |
| | | 1H NMR (500 MHz, DMSO-d6) δ 11.06 (s, 1H), 7.89 (t, J = 1.5 Hz, 1H), 7.81 (d, J = 7.8 Hz, 1H), 7.71 (dt, J = 7.8, 1.5 Hz, 1H), 7.55 (t, J = 7.8 Hz, 1H), 6.92 (s, 2H), 3.81 - 3.73 (m, 1H), 3.71 - 3.60 (m, 4H), 3.54 - 3.43 (m, 5H), 2.42 - 2.32 (m, 7H), 2.14 - 2.04 (m, 1H), 1.41 (s, 9H). |
| | *tert*-Butyl 2-[[4-(3-cyanophenyl)-5-(2,6-dimethyl-4-pyridyl)thiazol-2-yl]carbamoyl]-9-oxa-2,6-diazaspiro[4.5]decane-6-carboxylate | |
| 13.10 | 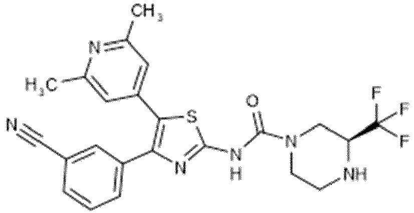 | LC-MS (Method 8B): Rt 4.13 mins; MS m/z 487.2 = [M+H]+ |
| | | 1H NMR (500 MHz, DMSO-d6) δ 11.40 (s, 1H), 7.89 (t, J = 1.5 Hz, 1H), 7.82 (dt, J = 7.8, 1.5 Hz, 1H), 7.71 (dt, J = 7.8, 1.5 Hz, 1H), 7.56 (t, J = 7.8 Hz, 1H), 6.92 (s, 2H), 4.15 (d, J = 12.9 Hz, 1H), 3.91 (d, J = 12.9 Hz, 1H), 3.45 - 3.37 (m, 1H), 3.15 - 3.04 (m, 2H), 3.03 |
| | (3S)-N-[4-(3-Cyanophenyl)-5-(2,6-dimethyl-4-pyridyl)thiazol-2-yl]-3- | |
| | (trifluoromethyl)piperazine-1-carboxamide | - 2.97 (m, 1H), 2.92 (d, J = 12.6 Hz, 1H), 2.70 - 2.63 (m, 1H), 2.36 (s, 6H). |
| | Prepared via: | Intermediate Data: |
| | 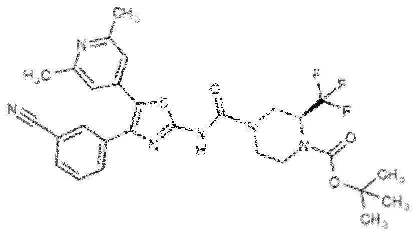 | LC-MS (Method 3B): Rt 1.92 mins; MS m/z 585.4 = [M-H]- |
| | | 1H NMR (500 MHz, DMSO-d6) δ 11.51 (s, 1H), 7.89 (t, J= 1.5 Hz, 1H), 7.83 (d, J= 7.8 Hz, 1H), 7.71 (d, J= 7.8 Hz, 1H), 7.57 (t, J= 7.8 Hz, 1H), 6.93 (s, 2H), 4.93 - 4.71 (m, 2H), 4.54 (d, J= 14.9 Hz, 1H), 4.30 - 4.14 (m, 2H), 3.99 - 3.88 (m, 2H), 2.36 (s, 6H), 1.44 (s, 9H). |
| | tert-Butyl (2S)-4-[[4-(3-cyanophenyl)-5-(2,6-dimethyl-4-pyridyl)thiazol-2-yl]carbamoyl]-2-(trifluoromethyl)piperazine-1-carboxylate | |
| 13.11 | 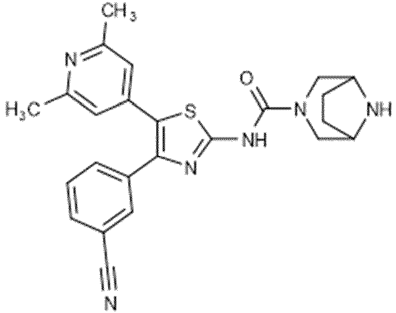 | LC-MS (Method 8B): Rt 3.57 mins; MS m/z 445.3 = [M+H]+ |
| | | 1H NMR (500 MHz, DMSO-d6) δ 7.88 (t,J= 1.8 Hz, 1H), 7.81 (dt,J= 7.7, 1.5 Hz, 1H), 7.71 (dt,J= 8.0, 1.5 Hz, 1H), 7.56 (t,J= 7.8 Hz, 1H), 6.91 (s, 2H), 3.84 (d,J= 12.1 Hz, 2H), 3.41 (s, 2H), 2.95 (d,J= 12.1 Hz, 2H), 2.36 (s, 6H), 1.67 - 1.61 (m, 2H), 1.57 (q,J= 7.9, 6.7 Hz, 2H). 2 x NH protons not observed. |
| | | Intermediate Data: |
| | | LC-MS (Method 3B): Rt 1.98 mins; MS m/z 543.4 = [M-H]- |
| | N-[4-(3-Cyanophenyl)-5-(2,6-dimethyl-4-pyridyl)thiazol-2-yl]-3,8-diazabicyclo[3.2.1]octane-3-carboxamide | |
| | | 1H NMR (500 MHz, DMSO-d6) δ 11.21 (s, 1H), 7.89 (t, J= 1.7 Hz, 1H), 7.83 (dt, J= 7.8, 1.4 Hz, 1H), 7.71 (dt, J= 8.0, 1.5 Hz, 1H), 7.57 (t, J= 7.8 Hz, 1H), 6.93 (s, 2H), 4.16 (s, 2H), 3.95 (d, J= 12.5 Hz, 2H), 3.04 (d, J= 12.6 Hz, 2H), 2.36 (s, 6H), 1.84 (s, 2H), 1.65 (s, 2H), 1.43 (s, 9H). |
| | Prepared via: | |
| | 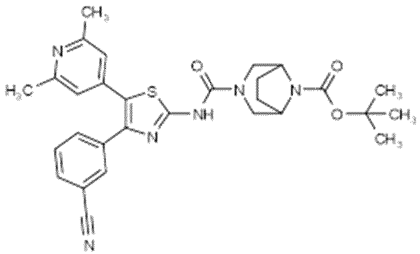 | |
| | *tert*-Butyl 3-[[4-(3-cyanophenyl)-5-(2,6-dimethyl-4-pyridyl)thiazol-2-yl]carbamoyl]-3,8-diazabicyclo[3.2.1]octane-8-carboxylate | |
| 13.12 | 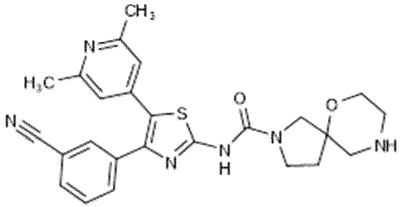 | LC-MS (Method 8B): Rt 3.81 mins; MS m/z 475.2 = [M+H]+ |
| | | 1H NMR (500 MHz, Methanol-d4) δ 7.92 (t, J= 1.6 Hz, 1H), 7.74 (dt, J= 7.8, 1.6 Hz, 1H), 7.70 (dt, J= 7.8, 1.6 Hz, 1H), 7.50 (t, J= 7.8 Hz, 1H), 7.02 (s, 2H), 3.84 - 3.70 (m, 3H), 3.70 - 3.57 (m, 2H), 3.43 (d, J= 11.4 Hz, 1H), 2.94 (d, J= 12.6 Hz, 1H), 2.88 (d, J= 12.6 Hz, 1H), 2.84 (t, J= 4.9 Hz, 2H), 2.45 (s, 6H), 2.34 - 2.20 (m, 1H), 2.06 - 1.92 (m, 1H). 2 x NH protons not observed. |
| | N-[4-(3-Cyanophenyl)-5-(2,6-dimethyl-4-pyridyl)thiazol-2-yl]-6-oxa-2,9-diazaspiro[4.5]decane-2-carboxamide | |
| | Prepared via: | |
| | 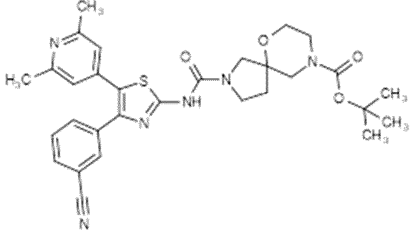 | Intermediate Data: |
| | | LC-MS (Method 3B): Rt 1.90 mins; MS m/z 573.4 = [M-H]- |
| | | 1H NMR (500 MHz, DMSO-d6) δ 11.10 (s, 1H), 7.89 (t,J= 1.5 Hz, 1H), 7.82 (dt,J= 7.8, 1.5 Hz, 1H), 7.71 (dt,J= 7.8, 1.5 Hz, 1H), 7.56 (t,J= 7.8 Hz, 1H), 6.93 (s, 2H), 3.72 - 3.34 (m, 10H), 2.37 (s, 6H), 2.12 - 2.01 (m, 1H), 1.98 - 1.89 (m, 1H), 1.42 (s, 9H). |
| | *tert*-Butyl 2-[[4-(3-cyanophenyl)-5-(2,6-dimethyl-4-pyridyl)thiazol-2-yl]carbamoyl]-6-oxa-2,9-diazaspiro[4.5]decane-9-carboxylate | |
| 13.13 | 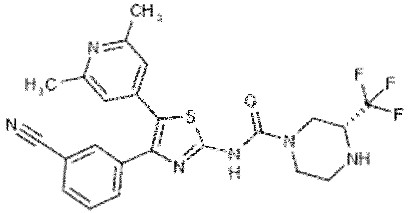 | LC-MS (Method 8B): Rt 4.07 mins; MS m/z 487.2 = [M+H]+ |
| | | 1H NMR (500 MHz, DMSO-d6) δ 11.41 (s, 1H), 7.89 (t, J= 1.6 Hz, 1H), 7.82 (dt, J= 7.8, 1.6 Hz, 1H), 7.71 (dt, J= 7.8, 1.4 Hz, 1H), 7.56 (t, J= 7.8 Hz, 1H), 6.93 (s, 2H), 4.18 - 4.09 (m, 1H), 3.94 - 3.86 (m, 1H), 3.47 - 3.38 (m, 1H), 3.16 - 3.07 (m, 2H), 3.03 - 2.97 (m, 1H), 2.96 - 2.89 (m, 1H), 2.71 - 2.64 (m, 1H), 2.36 (s, 6H). |
| | (3R)-N-[4-(3-Cyanophenyl)-5-(2,6-dimethyl-4-pyridyl)thiazol-2-yl]-3-(trifluoromethyl)piperazine-1-carboxamide | |
| | | Intermediate Data: |
| | Prepared via: | LC-MS (Method 3B): Rt 1.81 mins; MS m/z 585.4 = [M-H]- |
| | 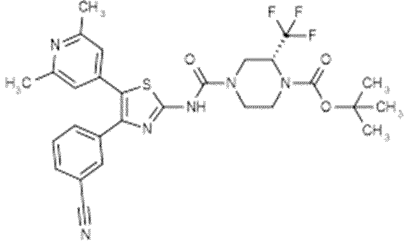 | |
| | | 1H NMR (500 MHz, DMSO-d6) δ 11.52 (br s, 1H), 7.90 (t, J= 1.5 Hz, 1H), 7.83 (dt, J= 7.8, 1.5 Hz, 1H), 7.71 (dt, J= 7.8, 1.5 Hz, 1H), 7.57 (t, J= 7.8 Hz, 1H), 6.94 (s, 2H), 4.92 - 4.72 (m, 1H), 4.54 (apr d, J= 14.8 Hz, 1H), 4.31 - 4.13 (m, 1H), 4.00 - 3.88 (m, 1H), 3.45 - 3.51 (m, 1H), 3.17 - 2.91 (m, 2H), 2.36 (s, 6H), 1.44 (s, 9H). |
| | *tert*-Butyl (2R)-4-[[4-(3-cyanophenyl)-5-(2,6-dimethyl-4-pyridyl)thiazol-2-yl]carbamoyl]-2-(trifluoromethyl)piperazine-1-carboxylate | |
| 13.14 | 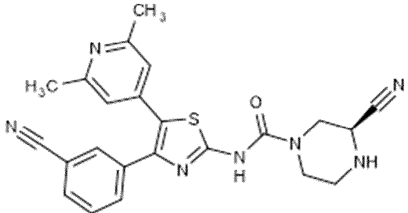 | LC-MS (Method 8B): Rt 2.02 mins; MS m/z 444.4 = [M+H]+ |
| | | 1H NMR (500 MHz, DMSO-d6) δ 11.39 (br s, 1H), 7.89 (t, J = 1.6 Hz, 1H), 7.82 (dt, J = 7.8, 1.6 Hz, 1H), 7.71 (dt, J = 7.8, 1.6 Hz, 1H), 7.56 (t, J = 7.8 Hz, 1H), 6.94 (s, 2H), 4.26 - 4.19 (m, 2H), 4.07 - 4.01 (m, 1H), 3.44 (br s, 1H), 3.27 - 3.19 (m, 1H), 3.05 - 2.98 (m, 1H), 2.84 - 2.77 (m, 2H), 2.36 (s, 6H). |
| | (3S)-3-Cyano-N-[4-(3-cyanophenyl)-5-(2,6-dimethyl-4-pyridyl)thiazol-2-yl]piperazine-1-carboxamide | |
| | | Intermediate Data: |
| | Prepared via: | LC-MS (Method 2B): Rt 1.34 mins; MS m/z 544.2 = [M+H]+ |
| | 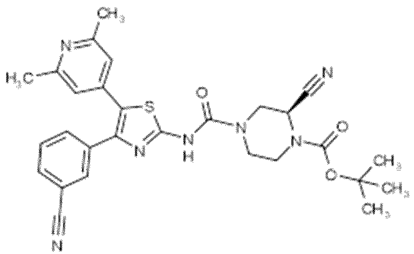 | 1H NMR (500 MHz, DMSO-d6) δ 11.60 (s, 1H), 7.90 (t, J= 1.6 Hz, 1H), 7.83 (dt, J= 7.9, 1.6 Hz, 1H), 7.71 (dt, J= 7.9, 1.6 Hz, 1H), 7.57 (t, J= 7.9 Hz, 1H), 6.94 (s, 2H), 5.31 (s, 1H), 4.52 (d, J= 14.0 Hz, 1H), 4.27 (d, J= 11.0 Hz, 1H), 3.89 (d, J= 11.0 Hz, 1H), 3.23 (d, J= 14.0 Hz, 1H), 3.05 - 2.91 (m, 2H), 2.36 (s, 6H), 1.45 (s, 9H). |
| | *tert*-Butyl (2S)-2-cyano-4-[[4-(3-cyanophenyl)-5-(2,6-dimethyl-4-pyridyl)thiazol-2-yl]carbamoyl]piperazine-1-carboxylate | |
| 13.15 | 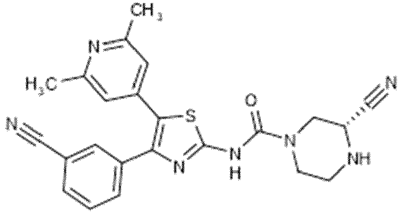 | LC-MS (Method 8A): Rt 1.93 mins; MS m/z 444.3 = [M+H]+ |
| | | 1H NMR (500 MHz, DMSO-d6) δ 11.40 (s, 1H), 7.89 (t, J = 1.8 Hz, 1H), 7.82 (d, J = 7.8 Hz, 1H), 7.71 (d, J = 7.8 Hz, 1H), 7.56 (t, J = 7.8 Hz, 1H), 6.94 (s, 2H), 4.26 - 4.19 (m, 2H), 4.04 (apr d, J = 13.0 Hz, 1H), 3.44 (s, 1H), 3.26 - 3.19 (m, 1H), 3.05 - 2.98 (m, 1H), 2.85 - 2.76 (m, 2H), 2.36 (s, 6H). |
| | (3R)-3-Cyano-N-[4-(3-cyanophenyl)-5-(2,6-dimethyl-4-pyridyl)thiazol-2-yl]piperazine-1-carboxamide | |
| | | Intermediate Data: |
| | Prepared via: | LC-MS (Method 2B): Rt 1.19 mins; MS m/z 544.4 = [M+H]+ |
| | 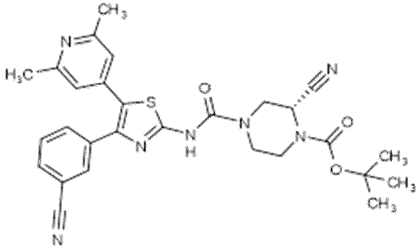 | |
| | | 1H NMR (500 MHz, DMSO-d6) δ 11.60 (s, 1H), 7.90 (t, J = 1.6 Hz, 1H), 7.83 (dt, J = 7.9, 1.6 Hz, 1H), 7.71 (dt, J = 7.9, 1.6 Hz, 1H), 7.57 (t, J = 7.9 Hz, 1H), 6.94 (s, 2H), 5.31 (s, 1H), 4.52 (d, J = 14.0 Hz, 1H), 4.27 (d, J = 11.0 Hz, 1H), 3.89 (d, J = 11.0 Hz, 1H), 3.23 (d, J = 14.0 Hz, 1H), 3.05 - 2.91 (m, 2H), 2.36 (s, 6H), 1.45 (s, 9H). |
| | *tert*-Butyl (2R)-2-cyano-4-[[4-(3-cyanophenyl)-5-(2,6-dimethyl-4-pyridyl)thiazol-2-yl]carbamoyl]piperazine-1-carboxylate | |

### Example 14

### (3R)-N-[4-(3-Cyanophenyl)-5-[2-methyl-6-(trifluoromethyl)-4-pyridyl]thiazol-2-yl]-3-methyl-piperazine-1-carboxamide

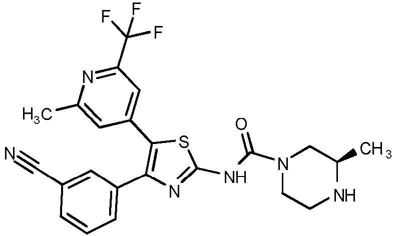

### Step 1: tert-Butyl (2R)-4-[[4-(3-cyanophenyl)-5-[2-methyl-6-(trifluoromethyl)-4-pyridyl]thiazol-2-yl]carbamoyl]-2-methyl-piperazine-1-carboxylate

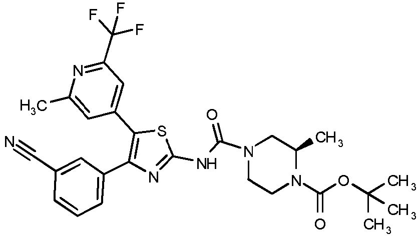

The title compound was prepared from 3-[2-amino-5-[2-methyl-6-(trifluoromethyl)-4-pyridyl]thiazol-4-yl]benzonitrile (Intermediate H) and *tert-butyl* (2R)-2-methylpiperazine-1-carboxylate analogously to Example 6.
LC-MS (Method 5B): Rt 3.29 mins; MS m/z 587.5 = [M+H]+
1H NMR (500 MHz, DMSO-d6) δ 11.46 (s, 1H), 7.92 (s, 1H), 7.87 (d, J = 7.7 Hz, 1H), 7.75 (d, J = 7.9 Hz, 1H), 7.60 (t, J = 7.8 Hz, 1H), 7.53 (s, 1H), 7.33 (s, 1H), 4.22 - 4.11 (m, 2H), 4.06 - 4.00 (m, 1H), 3.77 = 3.71 (m, 1H), 3.17 - 3.1 (m, 1H), 3.08 - 3.00 (m, 1H), 2.99 - 2.90 (m, 1H), 1.41 (s, 9H), 1.07 (d, J = 6.7 Hz, 3H). 1 x CH₃ signal not observed.

### Step 2: (3R)-N-[4-(3-Cyanophenyl)-5-[2-methyl-6-(trifluoromethyl)-4-pyridyl]thiazol-2-yl]-3-methyl-piperazine-1-carboxamide

To a solution of *tert-butyl* (2R)-4-[[4-(3-cyanophenyl)-5-[2-methyl-6-(trifluoromethyl)-4-pyridyl]thiazol-2-yl]carbamoyl]-2-methyl-piperazine-1-carboxylate (step 1) (83 mg, 0.14 mmol) in DCM (3.6 mL) was added TFA (0.71 mL, 9.27 mmol) and the reaction mixture was stirred at room temperature for 1 h. EtOAc (20 mL) and 1M NaOH (aq) (20 mL) were added and the layers were separated. The aqueous layer was extracted with EtOAc (4 x 15 mL) and the combined organic extracts were dried over Na₂SO₄ and concentrated *in vacuo* to afford a yellow oil. The crude product was purified by C18 reverse phase chromatography eluting with a gradient of 10 to 30% MeCN in water (+0.1% ammonium hydroxide modifier) to afford the title compound as a white solid.
LC-MS (Method 8B): Rt 4.37 mins; MS m/z 487.2 = [M+H]+
1H NMR (500 MHz, Methanol-d4) δ 7.92 (s, 1H), 7.72 (t, J = 9.1 Hz, 2H), 7.52 (t, J = 7.8 Hz, 1H), 7.44 (s, 1H), 7.36 (s, 1H), 4.14 (d, J = 12.9 Hz, 2H), 3.07 - 2.95 (m, 2H), 2.84 - 2.76 (m, 2H), 2.64 (t, J = 11.9 Hz, 1H), 2.55 (s, 3H), 1.14 (d, J = 6.4 Hz, 3H). 2 x NH protons not observed.

### Example 14.1

### rac-(4aS,7aS)-N-[4-(3-Cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide 2,2,2-trifluoroacetic acid salt

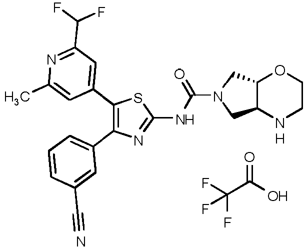

### Step 1: tert-Butyl rac-(4aS,7aS)-6-[[4-(3-cyanophenyl)-5-[2-(difluoromethyl)-6-methyl-4-pyridyl]thiazol-2-yl]carbamoyl]-2,3,4a,5,7,7a-hexahydropyrrolo[3,4-b][1,4]oxazine-4-carboxylate

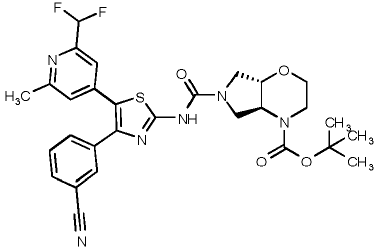

The title compound was prepared from 3-[2-amino-5-[2-(difluoromethyl)-6-methyl-4-pyridyl]thiazol-4-yl]benzonitrile (Intermediate G) and *tert-butyl* rac-(4aS,7aS)-3,4a,5,6,7,7a-hexahydro-2H-pyrrolo[3,4-b][1,4]oxazine-4-carboxylate analogously to Example 6.
LC-MS (Method 2B): Rt 1.49 mins; MS m/z 597.2 = [M+H]+ 1H NMR (500 MHz, DMSO-d6) δ 11.61 (s, 1H), 7.91 (s, 1H), 7.87 (d, J = 7.8 Hz, 1H), 7.78 - 7.72 (m, 1H), 7.60 (t, J = 7.8 Hz, 1H), 7.41 (s, 1H), 7.22 (s, 1H), 6.84 (t, J = 54.9 Hz, 1H), 4.21 (p, J = 7.1, 6.6 Hz, 1H), 4.14 (d, J = 12.7 Hz, 1H), 4.08 - 3.97 (m, 1H), 3.78 - 3.60 (m, 2H), 3.55 (ddd, J = 9.3, 7.2, 3.7 Hz, 1H), 3.29 - 3.09 (m, 3H), 3.01 (dt, J = 22.4, 9.8 Hz, 1H), 2.49 (s, 3H), 1.43 (d, J = 9.2 Hz, 9H).

### Step 2: rac-(4aS,7aS)-N-[4-(3-Cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide 2,2,2-trifluoroacetic acid salt

The title compound was prepared from *tert-*butyl rac-(4aS,7aS)-6-[[4-(3-cyanophenyl)-5-[2-(difluoromethyl)-6-methyl-4-pyridyl]thiazol-2-yl]carbamoyl]-2,3,4a,5,7,7a-hexahydropyrrolo[3,4-b][1,4]oxazine-4-carboxylate (step 1) and TFA analogously to Example 14 step 2.
LC-MS (Method 8B): Rt 2.35 mins; MS m/z 497.1 = [M+H]+
1H NMR (500 MHz, DMSO-d6) δ 11.72 (s, 1H), 9.00 (s, 2H), 7.93 - 7.86 (m, 2H), 7.74 (d, J = 7.9 Hz, 1H), 7.60 (t, J = 7.8 Hz, 1H), 7.38 (s, 1H), 7.22 (s, 1H), 6.85 (t, J = 54.9 Hz, 1H), 4.23 - 3.97 (m, 3H), 3.80 - 3.59 (m, 2H), 3.46 (dd, J = 10.9, 7.3 Hz, 1H), 3.27 - 3.12 (m, 3H), 2.97 (t, J = 11.0 Hz, 1H). Exchangeable protons not observed.

### Example 14.2

### rac-(4aS,7aR)-N-[4-(3-Cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide 2,2,2-trifluoroacetic acid salt

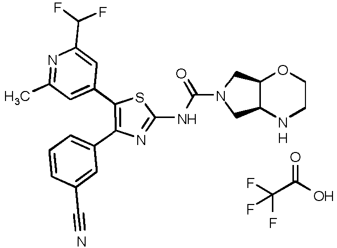

### Step 1: tert-Butyl rac-(4aS,7aR)-6-[[4-(3-cyanophenyl)-5-[2-(difluoromethyl)-6-methyl-4-pyridyl]thiazol-2-yl]carbamoyl]-2,3,4a,5,7,7a-hexahydropyrrolo[3,4-b][1,4]oxazine-4-carboxylate

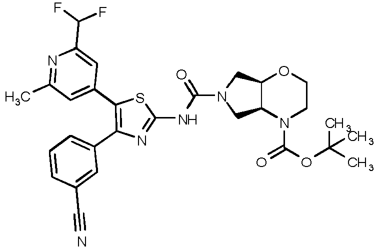

The title compound was prepared from 3-[2-amino-5-[2-(difluoromethyl)-6-methyl-4-pyridyl]thiazol-4-yl]benzonitrile (Intermediate G) and *tert-butyl* rac-(4aS,7aR)-3,4a,5,6,7,7a-hexahydro-2H-pyrrolo[3,4-b][1,4]oxazine-4-carboxylate analogously to Example 6.
LC-MS (Method 2B): Rt 1.48 mins; MS m/z 597.2 = [M+H]+
1H NMR (500 MHz, DMSO-d6) δ 11.23 (s, 1H), 7.91 (d, J = 1.7 Hz, 1H), 7.86 (dt, J = 7.7, 1.4 Hz, 1H), 7.74 (dt, J = 7.9, 1.5 Hz, 1H), 7.59 (t, J = 7.8 Hz, 1H), 7.39 (s, 1H), 7.22 (d, J = 1.6 Hz, 1H), 6.85 (t, J = 54.9 Hz, 1H), 4.44 (s, 1H), 3.99 (s, 1H), 3.86 (s, 2H), 3.75 - 3.41 (m, 5H), 3.05 (d, J = 59.6 Hz, 1H), 2.49 (s, 3H), 1.44 (s, 9H).

### Step 2: rac-(4aS,7aR)-N-[4-(3-Cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide 2,2,2-trifluoroacetic acid salt

The title compound was prepared from *tert-b*utyl rac-(4aS,7aR)-6-[[4-(3-cyanophenyl)-5-[2-(difluoromethyl)-6-methyl-4-pyridyl]thiazol-2-yl]carbamoyl]-2,3,4a,5,7,7a-hexahydropyrrolo[3,4-b][1,4]oxazine-4-carboxylate (step 1) and TFA analogously to Example 14 step 2.
LC-MS (Method 8B): Rt 2.73 mins; MS m/z 467.1 = [M+H]+
1H NMR (500 MHz, Methanol-d4) δ 7.94 (t, J = 1.7 Hz, 1H), 7.74 (ddt, J = 8.2, 6.9, 1.4 Hz, 2H), 7.53 (t, J = 7.8 Hz, 1H), 7.37 (s, 1H), 7.33 (s, 1H), 6.63 (t, J = 55.2 Hz, 1H), 4.45 (s, 1H), 4.15 - 4.04 (m, 2H), 4.00 (t, J = 9.4 Hz, 1H), 3.95 - 3.80 (m, 2H), 3.78 (s, 2H), 3.51 - 3.42 (m, 1H), 3.24 (dt, J = 12.6, 1.5 Hz, 1H), 2.55 (s, 3H). Exchangeable protons not observed..

### Example 14.3 rac-(4aS,7aS)-N-[4-(3-Cyanophenyl)-5-(2,6-dimethyl-4-pyridyl)thiazol-2-yl]-3,4,4a,5,7,7a-hexahydro-2H-pyrrolo[3,4-b][1,4]oxazine-6-carboxamide 2,2,2-trifluoroacetic acid salt

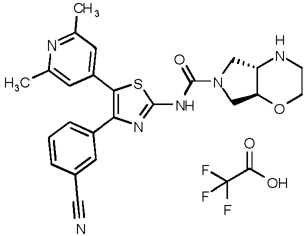

### Step 1: tert-Butyl rac-(4aS,7aS)-6-[[4-(3-cyanophenyl)-5-(2,6-dimethyl-4-pyridyl)thiazol-2-yl]carbamoyl]-2,3,4a,5,7,7a-hexahydropyrrolo[3,4-b][1,4]oxazine-4-carboxylate

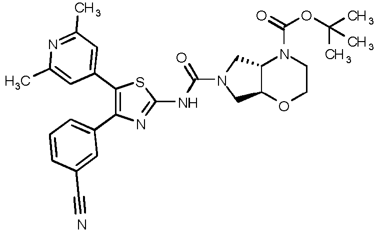

The title compound was prepared from 3-[2-amino-5-(2,6-dimethyl-4-pyridyl)thiazol-4-yl]benzonitrile (Intermediate C) and *tert*-butyl rac-(4aS,7aS)-3,4a,5,6,7,7a-hexahydro-2H-pyrrolo[3,4-b][1,4]oxazine-4-carboxylate analogously to Example 6.
LC-MS (Method 3B): Rt 1.70 mins; MS m/z 559.4 = [M-H]+
1H NMR (500 MHz, DMSO-d6) δ 11.51 (s, 1H), 7.89 (t, J = 1.6 Hz, 1H), 7.83 (dt, J = 7.8, 1.6 Hz, 1H), 7.73 - 7.69 (m, 1H), 7.57 (t, J = 7.8 Hz, 1H), 6.94 (s, 2H), 4.20 (dd, J = 10.1, 6.5 Hz, 1H), 4.13 (d, J = 12.7 Hz, 1H), 4.07 - 4.02 (m, 1H), 3.72 - 3.60 (m, 2H), 3.56 - 3.51 (m, 1H), 3.28 - 3.20 (m, 1H), 3.20 - 3.07 (m, 2H), 3.06 - 2.95 (m, 1H), 2.36 (s, 6H), 1.46 - 1.37 (m, 9H).

### Step 2: rac-(4aS,7aS)-N-[4-(3-Cyanophenyl)-5-(2,6-dimethyl-4-pyridyl)thiazol-2-yl]-3,4,4a,5,7,7a-hexahydro-2H-pyrrolo[3,4-b][1,4]oxazine-6-carboxamide 2,2,2-trifluoroacetic acid salt

The title compound was prepared from *tert-*butyl rac-(4aS,7aS)-6-[[4-(3-cyanophenyl)-5-(2,6-dimethyl-4-pyridyl)thiazol-2-yl]carbamoyl]-2,3,4a,5,7,7a-hexahydropyrrolo[3,4-b][1,4]oxazine-4-carboxylate (step 1) and TFA analogously to Example 14 step 2.
LC-MS (Method 8A): Rt 1.76 mins; MS m/z 461.1 = [M+H]+
1H NMR (500 MHz, Methanol-d4) δ 7.96 (t, J = 1.7 Hz, 1H), 7.83 - 7.78 (m, 2H), 7.59 (t, J = 7.8 Hz, 1H), 7.46 (s, 2H), 4.30 (dd, J = 11.8, 7.1 Hz, 1H), 4.18 (ddd, J = 11.8, 3.7, 1.5 Hz, 1H), 4.06 (br d, J = 12.9 Hz, 1H), 3.87 (td, J = 11.8, 3.2 Hz, 1H), 3.79 (ddd, J = 11.2, 9.6, 7.4 Hz, 1H), 3.57 (dd, J = 11.2, 7.4 Hz, 1H), 3.46 (t, J = 11.8 Hz, 1H), 3.29 - 3.21 (m, 2H), 3.13 (t, J = 11.2 Hz, 1H), 2.61 (s, 6H). Exchangeable protons not observed.

### Example 14.4

### N-[4-(3-Cyanophenyl)-5-(2,6-dimethyl-4-pyridyl)thiazol-2-yl]-2-cyclopropyl-piperazine-1-carboxamide

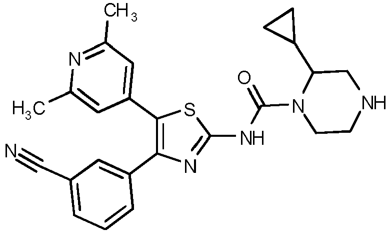

### Step 1: tert-Butyl 4-[[4-(3-cyanophenyl)-5-(2,6-dimethyl-4-pyridyl)thiazol-2-yl]carbamoyl]-3-cyclopropyl-piperazine-1-carboxylate

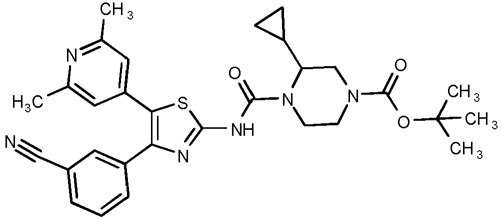

The title compound was prepared from 3-[2-amino-5-(2,6-dimethyl-4-pyridyl)thiazol-4-yl]benzonitrile (Intermediate C) and *tert-butyl* 3-cyclopropylpiperazine-1-carboxylate analogously to Example 4.
LC-MS (Method 2.5A): Rt 0.80 mins; MS m/z 559.3 = [M+H]+
1H NMR (400 MHz, DMSO-d6) δ 11.21 (s, 1H), 7.88 (t, J = 1.8 Hz, 1H), 7.82 (dt, J = 7.7, 1.5 Hz, 1H), 7.70 (dt, J = 8.0, 1.4 Hz, 1H), 7.56 (dd, J = 7.8, 7.8 Hz, 1H), 6.92 (s, 2H), 4.10 - 3.92 (m, 3H), 3.71 - 3.61 (m, 1H), 3.25 - 3.15 (m, 1H), 3.07 - 2.80 (m, 2H), 2.36 (s, 6H), 1.42 (s, 9H), 1.25 - 1.19 (m, 1H), 0.54 - 0.38 (m, 4H).

### Step 2: N-[4-(3-Cyanophenyl)-5-(2,6-dimethyl-4-pyridyl)thiazol-2-yl]-2-cyclopropyl-piperazine-1-carboxamide

The title compound was prepared from *tert-*butyl 4-[[4-(3-cyanophenyl)-5-(2,6-dimethyl-4-pyridyl)thiazol-2-yl]carbamoyl]-3-cyclopropyl-piperazine-1-carboxylate (step 1) and TFA analogously to Example 14 step 2.
LC-MS (Method 7B): Rt 2.80 mins; MS m/z 459.4 = [M+H]+
1H NMR (500 MHz, Methanol-d4) δ 7.90 (t, J = 1.7 Hz, 1H), 7.71 (dt, J = 8.0, 1.5 Hz, 1H), 7.69 (dt, J = 7.8, 1.4 Hz, 1H), 7.49 (dd, J = 7.8, 7.8 Hz, 1H), 7.00 (s, 2H), 4.00 - 3.94 (m, 1H), 3.49 (dd, J = 10.3, 3.8 Hz, 1H), 3.38 (td, J = 13.0, 3.2 Hz, 1H), 3.13 - 3.09 (m, 1H), 3.08 - 3.02 (m, 1H), 2.93 - 2.88 (m, 1H), 2.73 (td, J = 12.6, 3.6 Hz, 1H), 2.43 (s, 6H), 1.68 - 1.60 (m, 1H), 0.67 - 0.60 (m, 1H), 0.56 - 0.49 (m, 2H), 0.40 - 0.34 (m, 1H). 2x NH protons not observed.

### Example 14.5

### N-[4-(3-Cyanophenyl)-5-(2,6-dimethyl-4-pyridyl)thiazol-2-yl]-2-(cyclopropylmethyl)piperazine-1-carboxamide

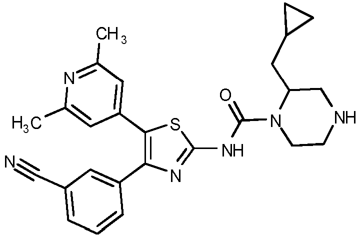

### Step 1: tert-Butyl 4-[[4-(3-cyanophenyl)-5-(2,6-dimethyl-4-pyridyl)thiazol-2-yl]carbamoyl]-3-(cyclopropylmethyl)piperazine-1-carboxylate

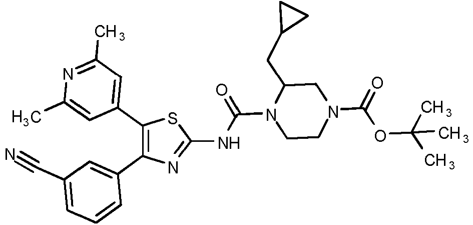

The title compound was prepared from 3-[2-amino-5-(2,6-dimethyl-4-pyridyl)thiazol-4-yl]benzonitrile (Intermediate C) and *tert*-butyl 3-(cyclopropylmethyl)piperazine-1-carboxylate analogously to Example 4.
LC-MS (Method 2A): Rt 0.87 mins; MS m/z 573.3 = [M+H]+
1H NMR (400 MHz, DMSO-d6) δ 11.28 (s, 1H), 7.88 (t, J = 1.7 Hz, 1H), 7.82 (dt, J = 7.7, 1.4 Hz, 1H), 7.71 (dt, J = 8.0, 1.4 Hz, 1H), 7.56 (dd, J = 7.8, 7.8 Hz, 1H), 6.92 (s, 2H), 4.44 - 4.36 (m, 1H), 4.03 - 3.82 (m, 3H), 3.08 - 2.95 (m, 2H), 2.35 (s, 6H), 1.42 (s, 9H), 0.84 (dd, J = 6.6, 2.4 Hz, 2H), 0.70 - 0.62 (m, 1H), 0.44 - 0.33 (m, 2H), 0.13 - 0.03 (m, 2H). 1 x CH proton not observed.

### Step 2: N-[4-(3-Cyanophenyl)-5-(2,6-dimethyl-4-pyridyl)thiazol-2-yl]-2-(cyclopropylmethyl)piperazine-1-carboxamide

The title compound was prepared from *tert*-butyl 4-[[4-(3-cyanophenyl)-5-(2,6-dimethyl-4-pyridyl)thiazol-2-yl]carbamoyl]-3-(cyclopropylmethyl)piperazine-1-carboxylate (step 1) and TFA analogously to Example 14 step 2.
LC-MS (Method 7B): Rt 3.14 mins; MS m/z 473.4 = [M+H]+
1H NMR (400 MHz, Methanol-d4) δ 7.90 (t, J = 1.4 Hz, 1H), 7.73 - 7.70 (m, 1H), 7.70 - 7.66 (m, 1H), 7.49 (dd, J = 7.8, 7.8 Hz, 1H), 7.00 (s, 2H), 4.36 - 4.29 (m, 1H), 3.96 - 3.89 (m, 1H), 3.14 - 3.04 (m, 2H), 3.03 - 2.97 (m, 1H), 2.92 (dd, J = 13.0, 4.2 Hz, 1H), 2.71 (td, J = 12.6, 3.7 Hz, 1H), 2.43 (s, 6H), 1.79 - 1.62 (m, 2H), 0.74 - 0.65 (m, 1H), 0.48 - 0.42 (m, 2H), 0.17 - 0.10 (m, 2H). - 2x NH protons not observed.

### Example 14.6

### N-[4-(3-Cyanophenyl)-5-(2,6-dimethyl-4-pyridyl)thiazol-2-yl]-3,3-dimethyl-piperazine-1-carboxamide

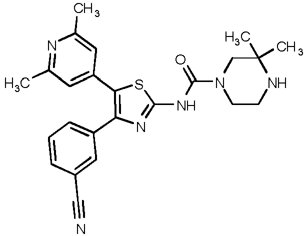

### Step 1: tert-Butyl 4-[[4-(3-cyanophenyl)-5-(2,6-dimethyl-4-pyridyl)thiazol-2-yl]carbamoyl]-2,2-dimethyl-piperazine-1-carboxylate

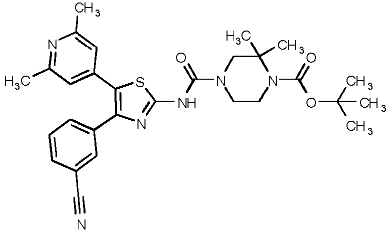

The title compound was prepared from 3-[2-amino-5-(2,6-dimethyl-4-pyridyl)thiazol-4-yl]benzonitrile (Intermediate C) and *tert-*butyl 2,2-dimethylpiperazine-1-carboxylate analogously to Example 6.
LC-MS (Method 2B): Rt 1.62 mins; MS m/z 547.2 = [M+H]+
1H NMR (500 MHz, DMSO-d6) δ 10.97 (v br s, 1H), 7.89 (t, J = 1.8 Hz, 1H), 7.82 (dt, J = 7.7, 1.5 Hz, 1H), 7.71 (dt, J = 7.9, 1.5 Hz, 1H), 7.56 (t, J = 7.8 Hz, 1H), 6.93 (s, 2H), 3.71 - 3.65 (m, 2H), 3.65 - 3.57 (m, 2H), 3.57 - 3.51 (m, 2H), 2.36 (s, 6H), 1.42 (d, J = 2.9 Hz, 9H), 1.34 (s, 6H).

### Step 2: N-[4-(3-Cyanophenyl)-5-(2,6-dimethyl-4-pyridyl)thiazol-2-yl]-3,3-dimethyl-piperazine-1-carboxamide

The title compound was prepared from *tert-*butyl 4-[[4-(3-cyanophenyl)-5-(2,6-dimethyl-4-pyridyl)thiazol-2-yl]carbamoyl]-2,2-dimethyl-piperazine-1-carboxylate (step 1) and TFA analogously to Example 14 step 2.
LC-MS (Method 8B): Rt 2.82 mins; MS m/z 447.1 = [M+H]+
1H NMR (500 MHz, Methanol-d4) δ 7.92 (t, J = 1.7 Hz, 1H), 7.73 (dt, J = 8.0, 1.5 Hz, 1H), 7.70 (dt, J = 7.7, 1.4 Hz, 1H), 7.51 (t, J = 7.8 Hz, 1H), 7.02 (s, 2H), 3.63 - 3.55 (m, 2H), 3.40 (s, 2H), 3.02 - 2.85 (m, 2H), 2.45 (s, 6H), 1.19 (s, 6H).2 x NH protons not observed.

### Example 14.7

### N-[4-(3-cyanophenyl)-5-(2,6-dimethyl-4-pyridyl)thiazol-2-yl]-4-oxa-1,9-diazaspiro[5.5]undecane-9-carboxamide

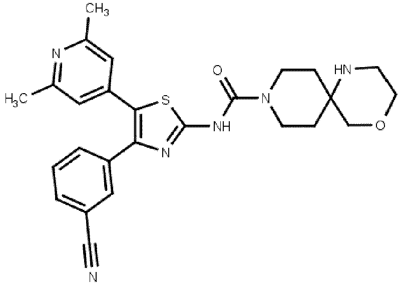

### Step 1: tert-butyl 9-[[4-(3-cyanophenyl)-5-(2,6-dimethyl-4-pyridyl)thiazol-2-yl]carbamoyl]-4-oxa-1,9-diazaspiro[5.5]undecane-1-carboxylate

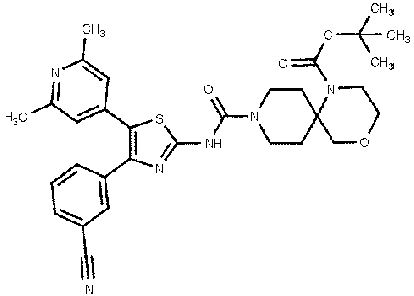

The title compound was prepared from 3-[2-amino-5-(2,6-dimethyl-4-pyridyl)thiazol-4-yl]benzonitrile (Intermediate C) and tert-butyl 4-oxa-1,9-diazaspiro[5.5]undecane-1-carboxylate analogously to Example 4.
LC-MS (Method 2A): Rt 0.74 mins; MS m/z 589.2 = [M+H]+
1H NMR (500 MHz, CDCl₃) δ 8.11 (s, 1H), 7.76 (t, J = 1.6 Hz, 1H), 7.69 (dt, J = 7.6, 1.4 Hz, 1H), 7.55 (dt, J = 7.9, 1.5 Hz, 1H), 7.48 (t, J = 7.8 Hz, 1H), 6.90 (s, 2H), 3.91 (dt, J = 11.6, 5.1 Hz, 2H), 3.77 (t, J = 5.3 Hz, 2H), 3.60 (s, 2H), 3.54 (t, J = 5.3 Hz, 2H), 3.49 - 3.41 (m, 2H), 2.60 (ddd, J = 14.0, 9.6, 4.5 Hz, 2H), 2.54 (s, 6H), 1.84 - 1.74 (m, 2H), 1.45 (s, 9H).

### Step 2: N-[4-(3-cyanophenyl)-5-(2,6-dimethyl-4-pyridyl)thiazol-2-yl]-4-oxa-1,9-diazaspiro[5.5]undecane-9-carboxamide

The title compound was prepared from tert-butyl 9-[[4-(3-cyanophenyl)-5-(2,6-dimethyl-4-pyridyl)thiazol-2-yl]carbamoyl]-4-oxa-1,9-diazaspiro[5.5]undecane-1-carboxylate (step 1) and TFA analogously to Example 14 step 2.
LC-MS (Method 7A): Rt 1.16 mins; MS m/z 489.3 = [M+H]+
1H NMR (400 MHz, MeOD) δ 7.90 (t, J = 1.7 Hz, 1H), 7.73 - 7.66 (m, 2H), 7.48 (dd, J = 7.8, 7.8 Hz, 1H), 7.00 (s, 2H), 3.70 - 3.62 (m, 4H), 3.63 - 3.56 (m, 2H), 3.54 (s, 2H), 2.92 - 2.87 (m, 2H), 2.43 (s, 6H), 1.73 (t, J = 5.9 Hz, 4H). 2 x NH protons not observed.

### Example 16

### N-[4-(3-Cyanophenyl)-5-(2-methoxy-6-methyl-4-pyridyl)thiazol-2-yl]-1-imino-1-oxo-1,4-thiazinane-4-carboxamide

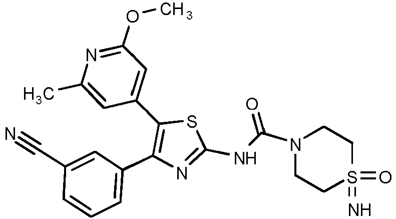

To a solution of 3-[2-amino-5-(2-methoxy-6-methyl-4-pyridyl)thiazol-4-yl]benzonitrile (Intermediate K) (75 mg, 0.23 mmol) and CDI (75 mg, 0.47 mmol) in DMA (1.2 mL) was added DIPEA (0.12 mL, 0.70 mmol) and the reaction mixture was stirred at 50 °C overnight. 1-Imino-1,4-thiazinane 1-oxide (47 mg, 0.35 mmol) was added and stirring continued at 50 °C for a further 1 h. The resulting mixture was purified by C18 reverse phase chromatography eluting with a gradient of 5 to 20% MeCN in water (+0.1% ammonium hydroxide) to afford the title compound as a white solid.
LC-MS (Method 8B): Rt 3.55 mins; MS m/z 483.3 = [M+H]+
1H NMR (500 MHz, DMSO-d6) δ 11.53 (s, 1H), 7.91 (s, 1H), 7.84 (d, J = 7.7 Hz, 1H), 7.72 (d, J = 8.0 Hz, 1H), 7.58 (t, J = 7.8 Hz, 1H), 6.74 (s, 1H), 6.42 (s, 1H), 4.11 (d, J = 14.4 Hz, 2H), 3.84 (s, 1H), 3.81 (s, 3H), 3.78 - 3.68 (m, 2H), 3.08 (d, J = 5.4 Hz, 4H), 2.34 (s, 3H).

The compounds of the following tabulated Examples (Table Ex16) were prepared analogously to Example 16 from the appropriate starting compound and amine as indicated in the table.

**Table Ex16**

| **Ex.** | **Structure and Name** | **Retention Time, [M+H]+, ¹H NMR** |
|---|---|---|
| 16.1 | 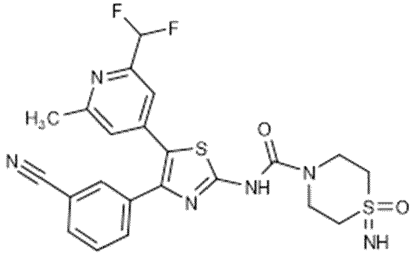 N-[4-(3-Cyanophenyl)-5-[2-(difluoromethyl)-6-methyl-4-pyridyl]thiazol-2-yl]-1-imino-1-oxo-1,4-thiazinane-4-carboxamide | LC-MS (Method 8B): Rt 3.06 mins; |
| | | MS m/z 503.1 = [M+H]+ |
| | | 1H NMR (500 MHz, DMSO-d6) δ 11.64 (s, 1H), 7.90 (d, J= 1.8 Hz, 1H), 7.87 (d, J= 7.5 Hz, 1H), 7.74 (dt, J= 7.9, 1.5 Hz, 1H), 7.60 (t, J= 7.8 Hz, 1H), 7.38 (s, 1H), 7.21 (s, 1H), 6.84 (t, J= 54.9 Hz, 1H), 4.12 (d, J= 14.3 Hz, 2H), 3.83 (s, 1H), 3.73 (d, J= 14.6 Hz, 2H), 3.09 (d, J= 9.3 Hz, 4H), 2.49 (s, 3H). |
| | Prepared from Intermediate G and 1-imino-1,4-thiazinane 1-oxide | |
| 16.2 | 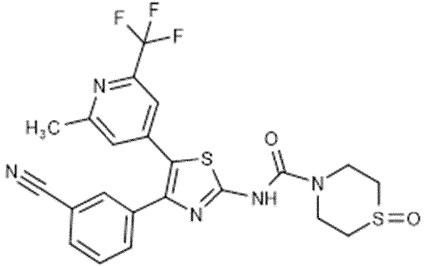 N-[4-(3-Cyanophenyl)-5-[2-methyl-6-(trifluoromethyl)-4-pyridyl]thiazol-2-yl]-1-oxo-1,4-thiazinane-4-carboxamide | LC-MS (Method 8B): Rt 3.12 mins; |
| | | MS m/z 506.0= [M+H]+ |
| | | 1H NMR (500 MHz, Methanol-d4) δ 7.91 (s, 1H), 7.73 (t, J= 9.4 Hz, 2H), 7.53 (t, J= 7.8 Hz, 1H), 7.43 (s, 1H), 7.34 (s, 1H), 4.22 (d, J= 14.7 Hz, 2H), 3.99 (t, J= 13.1 Hz, 2H), 3.02 (td, J= 12.4, 10.7, 3.4 Hz, 2H), 2.89 (d, J= 13.7 Hz, 2H), 2.55 (s, 3H). |
| | Prepared from Intermediate H and 1,4-thiazinane 1-oxide hydrochloride | |
| 16.3 | 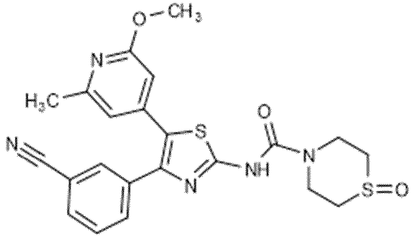 | LC-MS (Method 8B): Rt 3.22 mins; |
| | | MS m/z 468.1= [M+H]+ |
| | | 1H NMR (500 MHz, DMSO-d6) δ 11.45 (s, 1H), 7.90 (s, 1H), 7.83 (d, J= 7.7 Hz, 1H), 7.71 (d, J= 7.9 Hz, 1H), 7.57 (t, J= 7.8 Hz, 1H), 6.74 (s, 1H), 6.41 (s, 1H), 4.11 (d, J= 14.7 Hz, 2H), 3.80 (s, 3H), 3.77 (d, J= 13.2 Hz, 2H), 2.92 (t, J= 12.8 Hz, |
| | N-[4-(3-Cyanophenyl)-5-(2-methoxy-6-methyl-4-pyridyl)thiazol-2-yl]-1-oxo-1,4-thiazinane-4-carboxamide | 2H), 2.74 (d, J= 13.7 Hz, 2H), 2.34 (s, 3H). |
| | Prepared from Intermediate K and 1,4-thiazinane 1-oxide hydrochloride | |
| 16.4 | 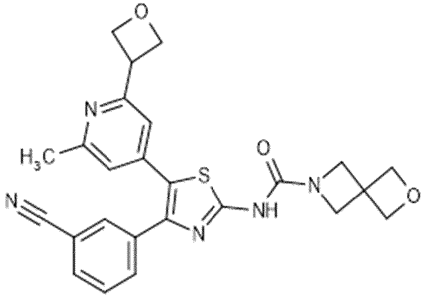 N-[4-(3-Cyanophenyl)-5-[2-methyl-6-(oxetan-3-yl)-4-pyridyl]thiazol-2-yl]-2-oxa-6-azaspiro[3.3]heptane-6-carboxamide | LC-MS (Method 8B): Rt 2.59 mins; |
| | | MS m/z 474.1 = [M+H]+ |
| | | 1H NMR (500 MHz, DMSO-d6) δ 11.37 (s, 1H), 7.88 (t, J = 1.6 Hz, 1H), 7.82 (dt, J = 7.8, 1.6 Hz, 1H), 7.72 (dt, J = 8.0, 1.6 Hz, 1H), 7.56 (apr t, J = 7.8 Hz, 1H), 7.05 (s, 1H), 6.98 (s, 1H), 4.80 (dd, J = 8.5, 5.7 Hz, 2H), 4.67 (s, 4H), 4.65 (dd, J = 7.1, 5.7 Hz, 2H), 4.27 (tt, J = 8.5, 7.1 Hz, 1H), 4.20 (s, 4H), 2.44 (s, 3H) |
| | Prepared from Intermediate L and 2-oxa-6-azaspiro[3.3]heptane | |
| 16.5 | 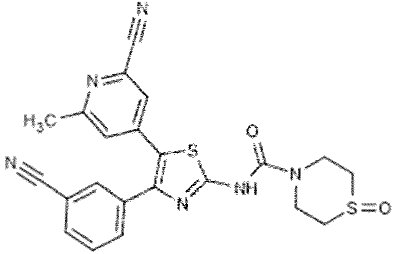 N-[5-(2-Cyano-6-methyl-4-pyridyl)-4-(3-cyanophenyl)thiazol-2-yl]-1-oxo-1,4-thiazinane-4-carboxamide | LC-MS (Method 8B): Rt 2.94 mins; |
| | | MS m/z 463.1 = [M+H]+ |
| | | 1H NMR (500 MHz, DMSO-d6) δ 11.58 (s, 1H), 7.92 (s, 1H), 7.87 (d, J = 7.7 Hz, 1H), 7.71 (d, J = 7.9 Hz, 1H), 7.63 - 7.57 (m, 2H), 7.49 (s, 1H), 4.12 (d, J = 14.2 Hz, 2H), 3.79 (t, J = 13.1 Hz, 2H), 2.92 (t, J = 12.4 Hz, 2H), 2.74 (d, J = 13.7 Hz, 2H), 2.47 (s, 3H). |
| | Prepared from Intermediate KA and 1,4-thiazinane 1-oxide hydrochloride | |
| 16.6 | 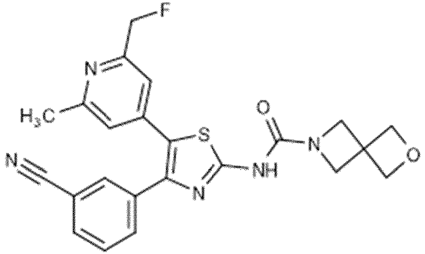 N-[4-(3-Cyanophenyl)-5-[2-(fluoromethyl)-6-methyl-4-pyridyl]thiazol-2-yl]-2-oxa-6-azaspiro[3.3]heptane-6-carboxamide Prepared from Intermediate KB and 2-oxa-6-azaspiro[3.3]heptane | LC-MS (Method 8B): Rt 2.95 mins; |
| | | MS m/z 450.3 = [M+H]+ |
| | | 1H NMR (500 MHz, DMSO-d6) δ 11.40 (s, 1H), 7.88 (t, J = 1.7 Hz, 1H), 7.83 (dt, J = 7.7, 1.4 Hz, 1H), 7.71 (dt, J = 8.0, 1.5 Hz, 1H), 7.56 (t, J = 7.8 Hz, 1H), 7.14 (s, 1H), 7.08 (s, 1H), 5.36 (d, J = 46.8 Hz, 2H), 4.67 (s, 4H), 4.20 (s, 4H), 2.42 (s, 3H). |
| 16.7 | 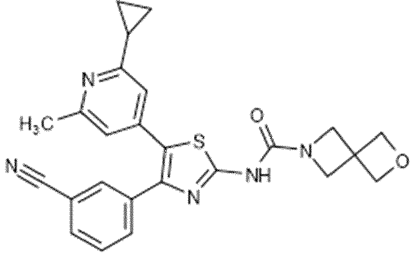 N-[4-(3-Cyanophenyl)-5-(2-cyclopropyl-6-methyl-4-pyridyl)thiazol-2-yl]-2-oxa-6-azaspiro[3.3]heptane-6-carboxamide Prepared from Intermediate KC and 2-oxa-6-azaspiro[3.3]heptane | LC-MS (Method 8B): Rt 2.33 mins; |
| | | MS m/z 458.1 = [M+H]+ |
| | | 1H NMR (500 MHz, DMSO-d6) δ 11.34 (s, 1H), 7.87 (t, J = 1.5 Hz, 1H), 7.82 (dt, J = 7.7, 1.5 Hz, 1H), 7.70 (dt, J = 8.0, 1.5 Hz, 1H), 7.57 (apr t, J = 7.8 Hz, 1H), 6.88 (d, J = 1.6 Hz, 1H), 6.87 (d, J = 1.6 Hz, 1H), 4.67 (s, 4H), 4.20 (s, 4H), 2.32 (s, 3H), 1.97 (tt, J = 8.2, 4.8 Hz, 1H), 0.90 - 0.85 (m, 2H), 0.83 - 0.78 (m, 2H) |
| 16.8 | 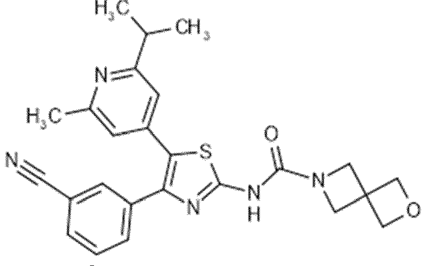 N-[4-(3-Cyanophenyl)-5-(2-isopropyl-6-methyl-4-pyridyl)thiazol-2-yl]-2-oxa-6-azaspiro[3.3]heptane-6-carboxamide Prepared from Intermediate KD and 2-oxa-6-azaspiro[3.3]heptane | LC-MS (Method 8B): Rt 3.56 mins; |
| | | MS m/z 460.3 = [M+H]+ |
| | | 1H NMR (500 MHz, DMSO-d6) δ 11.31 (s, 1H), 7.93 - 7.79 (m, 2H), 7.74 (d, J = 8.0 Hz, 1H), 7.58 (t, J = 8.0 Hz, 1H), 7.02 (s, 1H), 6.79 (s, 1H), 4.67 (s, 4H), 4.20 (s, 4H), 2.89 - 2.81 (m, 1H), 2.41 (s, 3H), 1.08 (d, J = 6.9 Hz, 6H). |
| 16.9 | 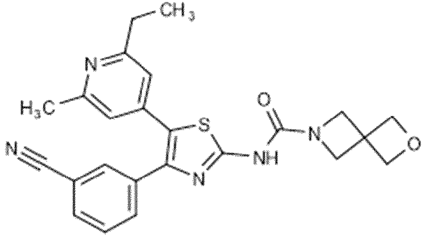 N-[4-(3-Cyanophenyl)-5-(2-ethyl-6-methyl-4-pyridyl)thiazol-2-yl]-2-oxa-6-azaspiro[3.3]heptane-6-carboxamide Prepared from Intermediate KE and 2-oxa-6-azaspiro[3.3]heptane | LC-MS (Method 8B): Rt 3.97 mins; |
| | | MS m/z 446.2 = [M+H]+ |
| | | 1H NMR (500 MHz, CDCl₃) δ 7.87 (t, J = 1.5 Hz, 1H), 7.76 (br s, 1H), 7.63 (dt, J = 7.9, 1.5 Hz, 1H), 7.60 (dt, J = 7.8, 1.5 Hz, 1H), 7.39 (apr t, J = 7.8 Hz, 1H), 6.89 (br d, J = 1.2 Hz, 1H), 6.84 (br d, J = 1.2 Hz, 1H), 4.83 (s, 4H), 4.30 (s, 4H), 2.72 (q, J = 7.6 Hz, 2H), 2.48 (s, 3H), 1.19 (t, J = 7.6 Hz, 3H) |
| 16.10 | 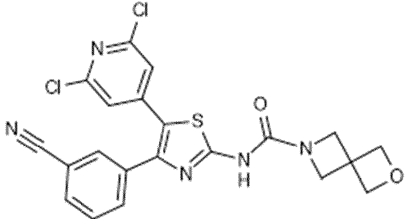 N-[4-(3-Cyanophenyl)-5-(2,6-dichloro-4-pyridyl)thiazol-2-yl]-2-oxa-6-azaspiro[3.3]heptane-6-carboxamide Prepared from Intermediate KF and 2-oxa-6-azaspiro[3.3]heptane | LC-MS (Method 8B): Rt 2.98 mins; |
| | | MS m/z 470.1 / 472.1 = [M+H]+ |
| | | 1H NMR (500 MHz, DMSO-d6) δ 11.53 (s, 1H), 7.95 (t,J= 1.6 Hz, 1H), 7.88 (dt,J= 7.8, 1.6 Hz, 1H), 7.76 (dt,J= 7.6, 1.6 Hz, 1H), 7.61 (t,J= 7.8 Hz, 1H), 7.35 (s, 2H), 4.67 (s, 4H), 4.21 (s, 4H). |

### Example 17

### N-[4-(3-Cyanophenyl)-5-(2,6-dimethyl-1-oxido-pyridin-1-ium-4-yl)thiazol-2-yl]-2-oxa-6-azaspiro[3.3]heptane-6-carboxamide

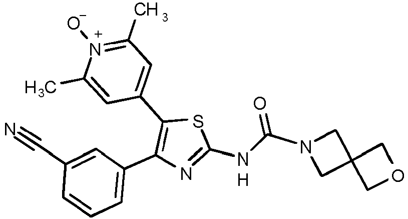

### Step 1: 3-[2-Amino-5-(2,6-dimethyl-1-oxido-pyridin-1-ium-4-yl)thiazol-4-yl]benzonitrile

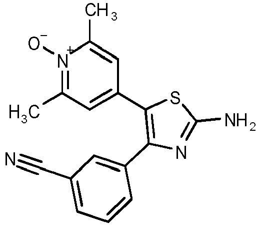

mCPBA (110 mg, 0.49 mmol) was added to a suspension of 3-[2-amino-5-(2,6-dimethyl-4-pyridyl)thiazol-4-yl]benzonitrile (Intermediate C) (100 mg, 0.33 mmol) in DCM (2 mL) at 0 °C. The reaction mixture was allowed to warm to room temperature and stirred for 16 h. Further mCPBA (73 mg, 0.33 mmol) was added and stirring continued for 1 h. The resulting solution was diluted in DCM (10 mL) and washed with sat. NaHCO₃ solution (2 x 20 mL). The organics were dried over Na₂SO₄ and concentrated *in vacuo* to afford a red oil. The oil was dry loaded onto silica and purified by chromatography on silica eluting with a gradient of 0 to 5% MeOH in DCM to afford a solid that was triturated with Et₂O, filtered and washed with Et₂O (3 x 2 mL) to afford the title compound as a yellow solid.
LC-MS (Method 5A): Rt 1.86 mins; MS m/z 323.1 = [M+H]+
1H NMR (500 MHz, DMSO-d6) δ 7.86 (d, J = 1.9 Hz, 1H), 7.77 (d, J = 7.7 Hz, 1H), 7.69 (d, J = 7.9 Hz, 1H), 7.55 - 7.49 (m, 1H), 7.46 - 7.39 (m, 2H), 7.14 (s, 2H), 2.27 (s, 6H).

### Step 2: N-[4-(3-Cyanophenyl)-5-(2,6-dimethyl-1-oxido-pyridin-1-ium-4-yl)thiazol-2-yl]-2-oxa-6-azaspiro[3.3]heptane-6-carboxamide

The title compound was prepared from 3-[2-amino-5-(2,6-dimethyl-1-oxido-pyridin-1-ium-4-yl)thiazol-4-yl]benzonitrile (step 1) and 2-oxa-6-azaspiro[3.3]heptane analogously to Example 6.
LC-MS (Method 8A): Rt 2.53 mins; MS m/z 448.2 = [M+H]+
1H NMR (500 MHz, DMSO-d6) δ 7.92 (s, 1H), 7.78 (d, J = 7.8 Hz, 1H), 7.74 (d, J = 7.8 Hz, 1H), 7.53 (t, J = 7.8 Hz, 1H), 7.20 (s, 2H), 4.67 (s, 4H), 4.13 (s, 4H), 2.29 (s, 6H). 1 x NH proton not observed.

### Example 18

### 4-Cyano-N-[4-(3-cyano-2-methyl-phenyl)-5-(2,6-dimethyl-4-pyridyl)thiazol-2-yl]-4-methyl-piperidine-1-carboxamide

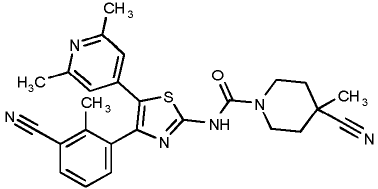

The title compound was prepared from 3-[2-amino-5-(2,6-dimethyl-4-pyridyl)thiazol-4-yl]-2-methyl-benzonitrile (Intermediate O) and 4-methylpiperidine-4-carbonitrile hydrochloride analogously to Example 6.
LC-MS (Method 8B): Rt 4.25 mins; MS m/z 471.3 = [M+H]+
1H NMR (500 MHz, DMSO-d6) δ 11.36 (s, 1H), 7.89 (d, J = 7.7 Hz, 1H), 7.53 (d, J = 7.6 Hz, 1H), 7.44 (t, J = 7.7 Hz, 1H), 6.67 (s, 2H), 4.23 (d, J = 14.0 Hz, 2H), 3.03 (t, J = 13.1 Hz, 2H), 2.29 - 2.25 (m, 9H), 1.92 (d, J = 13.5 Hz, 2H), 1.53 (td, J = 13.2, 3.9 Hz, 2H), 1.38 (s, 3H).

### Example 19

### N-[5-(2-Acetamido-6-methyl-4-pyridyl)-4-(3-cyanophenyl)thiazol-2-yl]-2-oxa-6-azaspiro[3.3]heptane-6-carboxamide

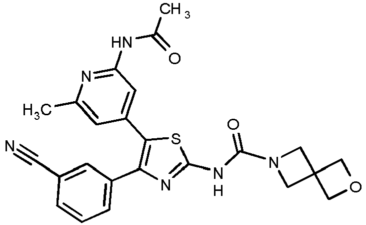

The title compound was prepared from N-[4-[2-amino-4-(3-cyanophenyl)thiazol-5-yl]-6-methyl-2-pyridyl]acetamide (Intermediate KG) and 2-oxa-6-azaspiro[3.3]heptane analogously to Example 6.
LC-MS (Method 8B): Rt 2.89 mins; MS m/z 475.3 = [M+H]+
1H NMR (500 MHz, DMSO-d6) δ 11.35 (s, 1H), 10.49 (s, 1H), 7.87 (d, J = 1.8 Hz, 1H), 7.85 (s, 1H), 7.81 (dt, J = 7.8, 1.5 Hz, 1H), 7.73 (dt, J = 7.8, 1.5 Hz, 1H), 7.55 (t, J = 7.8 Hz, 1H), 6.82 (d, J = 1.5 Hz, 1H), 4.67 (s, 4H), 4.20 (s, 4H), 2.33 (s, 3H), 2.02 (s, 3H).

### Example 20

### N-[5-[2-(Acetamidomethyl)-6-methyl-4-pyridyl]-4-(3-cyanophenyl)thiazol-2-yl]-2-oxa-6-azaspiro[3.3]heptane-6-carboxamide

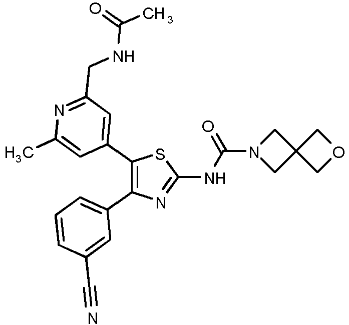

The title compound was prepared from N-[[4-[2-amino-4-(3-cyanophenyl)thiazol-5-yl]-6-methyl-2-pyridyl]methyl]acetamide (Intermediate KH) and 2-oxa-6-azaspiro[3.3]heptane analogously to Example 6.
LC-MS (Method 8B: Rt 2.59 mins; MS m/z 489.0 = [M+H]+
1H NMR (500 MHz, DMSO-d6) δ 11.37 (s, 1H), 8.30 (t, J = 6.0 Hz, 1H), 7.88 - 7.81 (m, 2H), 7.68 (dt, J = 8.0, 1.5 Hz, 1H), 7.55 (t, J = 7.8 Hz, 1H), 7.04 (s, 1H), 6.87 (s, 1H), 4.68 (s, 4H), 4.23 - 4.19 (m, 6H), 2.40 (s, 3H), 1.76 (s, 3H). Contains 1% Et2O

### Example 21

### N-[4-(3-Cyanophenyl)-5-[2-(methoxymethyl)-6-methyl-4-pyridyl]thiazol-2-yl]-2-oxa-6-azaspiro[3.3]heptane-6-carboxamide

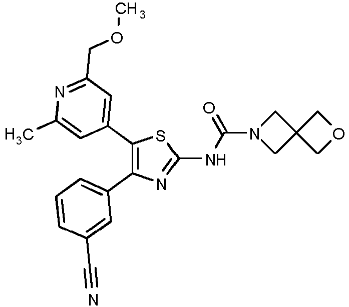

The title compound was prepared from 3-[2-amino-5-[2-(methoxymethyl)-6-methyl-4-pyridyl]thiazol-4-yl]benzonitrile (Intermediate KI) and 2-oxa-6-azaspiro[3.3]heptane analogously to Example 6.
LC-MS (Method 8B): Rt 3.30 mins; MS m/z 462.3 = [M+H]+
1H NMR (500 MHz, DMSO-d6) δ 11.38 (s, 1H), 7.87 (s, 1H), 7.83 (dt, J = 7.7, 1.5 Hz, 1H), 7.73 (dt, J = 8.0, 1.5 Hz, 1H), 7.58 (t, J = 7.8 Hz, 1H), 7.08 (s, 1H), 6.99 (s, 1H), 4.68 (s, 4H), 4.39 (s, 2H), 4.21 (s, 4H), 3.23 (s, 3H), 2.41 (s, 3H).

### Example 22

### N-[4-(3-Cyanophenyl)-5-[2-(1-methoxyethyl)-6-methyl-4-pyridyl]thiazol-2-yl]-2-oxa-6-azaspiro[3.3]heptane-6-carboxamide

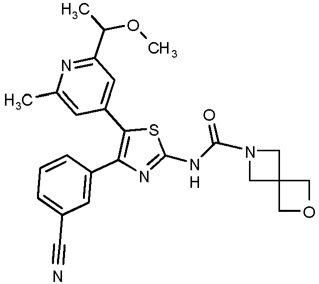

The title compound was prepared from 3-[2-amino-5-[2-(methoxymethyl)-6-methyl-4-pyridyl]thiazol-4-yl]benzonitrile (Intermediate P) and 2-oxa-6-azaspiro[3.3]heptane analogously to Example 6.
LC-MS (Method 8B): Rt 3.92 mins; MS m/z 476.3 = [M+H]+
1H NMR (500 MHz, DMSO-d6) δ 11.45 (s, 1H), 7.91 - 7.87 (m, 2H), 7.83 (dt, J = 7.9, 1.5 Hz, 1H), 7.67 - 7.63 (m, 1H), 7.21 (d, J = 1.6 Hz, 1H), 6.95 (d, J = 1.6 Hz, 1H), 4.74 (s, 4H), 4.33 - 4.20 (m, 5H), 3.10 (s, 3H), 2.51 (s, 3H), 1.28 (d, J = 6.5 Hz, 3H).

### Example 23

### N-[5-[2-Chloro-6-(1-hydroxy-1-methyl-ethyl)-4-pyridyl]-4-(3-cyanophenyl)thiazol-2-yl]-2-oxa-6-azaspiro[3.3]heptane-6-carboxamide

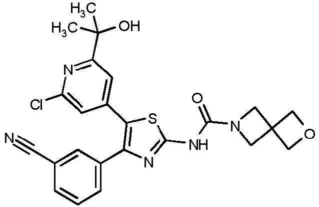

The title compound was prepared from 3-[2-amino-5-[2-chloro-6-(1-hydroxy-1-methyl-ethyl)-4-pyridyl]thiazol-4-yl]benzonitrile (Intermediate Q) and 2-oxa-6-azaspiro[3.3]heptane analogously to Example 6.
LC-MS (Method 8B): Rt 3.40 mins; MS m/z 496.1 = [M+H]+
1H NMR (500 MHz, DMSO-d6) δ 11.47 (s, 1H), 7.88 (t, J = 1.6 Hz, 1H), 7.86 (dt, J = 7.8, 1.6 Hz, 1H), 7.76 (dt, J = 7.9, 1.6 Hz, 1H), 7.61 (apr t, J = 7.8 Hz, 1H), 7.46 (d, J = 1.5 Hz, 1H), 7.15 (d, J = 1.5 Hz, 1H), 5.26 (s, 1H), 4.67 (s, 4H), 4.21 (s, 4H), 1.36 (s, 6H).

### Example 24

### 4-Cyano-N-[4-(3-cyanophenyl)-5-[2-(hydroxymethyl)-6-methyl-4-pyridyl]thiazol-2-yl]-4-methyl-piperidine-1-carboxamide

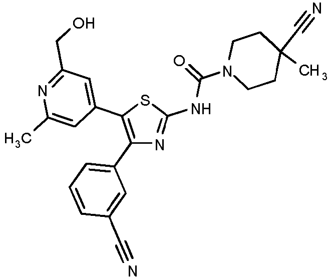

### Step 1: N-[5-[2-[[tert-Butyl(dimethyl)silyl]oxymethyl]-6-methyl-4-pyridyl]-4-(3-cyanophenyl)thiazol-2-yl]-4-cyano-4-methyl-piperidine-1-carboxamide

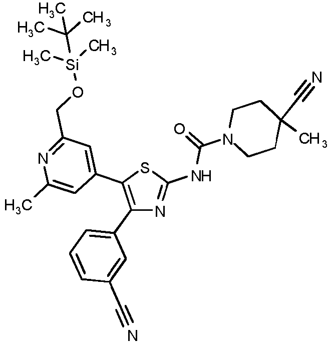

The title compound was prepared from 3-[2-amino-5-[2-[[tert-butyl(dimethyl)silyl]oxymethyl]-6-methyl-4-pyridyl]thiazol-4-yl]benzonitrile (Intermediate I) and 4-methylpiperidine-4-carbonitrile hydrochloride analogously to Example 6.
LC-MS (Method 3B): Rt 2.34 mins; MS m/z 587.3 = [M+H]+
1H NMR (500 MHz, DMSO-d6) δ 11.35 (s, 1H), 7.85 (s, 1H), 7.82 (d, J = 7.8 Hz, 1H), 7.72 (d, J = 7.9 Hz, 1H), 7.56 (t, J = 7.8 Hz, 1H), 7.12 (s, 1H), 7.01 (s, 1H), 4.62 (s, 2H), 4.22 (d, J = 14.0 Hz, 2H), 3.03 (t, J = 12.9 Hz, 2H), 2.42 (s, 3H), 1.92 (d, J = 13.6 Hz, 2H), 1.58 - 1.49 (m, 2H), 1.37 (s, 3H), 0.77 (s, 9H), -0.02 (s, 6H).

### Step 2: 4-Cyano-N-[4-(3-cyanophenyl)-5-[2-(hydroxymethyl)-6-methyl-4-pyridyl]thiazol-2-yl]-4-methyl-piperidine-1-carboxamide

To a solution of N-[5-[2-[[*tert-*butyl(dimethyl)silyl]oxymethyl]-6-methyl-4-pyridyl]-4-(3-cyanophenyl)thiazol-2-yl]-4-cyano-4-methyl-piperidine-1-carboxamide (step 1)(188 mg, 0.32 mmol) in THF (6.4 mL) was added TBAF (0.64 mL, 0.64 mmol) and the reaction mixture was stirred at room temperature for 90 mins. Additional TBAF (0.64 mL, 0.64 mmol) was added and stirring continued for a further 2.5 h. The resulting mixture was diluted with EtOAc (50 mL) and water (50 mL). The layers were separated and the aqueous phase was extracted further with EtOAc (40 mL). The combined organic extracts were washed with brine, dried over MgSO₄ and concentrated *in vacuo.* Purification by chromatography on silica eluting in 0 to 30% MeOH in EtOAc afforded a yellow solid. The solid was further purified by C18 reverse phase chromatography eluting with a gradient of 10 to 35% MeCN in water (+ 0.1% ammonium hydroxide) to afford a solid which was triturated in Et₂O and filtered to afford the title compound as a white solid.
LC-MS (Method 8B): Rt 3.75 mins; MS m/z 473.3 = [M+H]+
1H NMR (500 MHz, DMSO-d6) δ 11.32 (s, 1H), 7.89 (s, 1H), 7.82 (d, J = 7.7 Hz, 1H), 7.72 (d, J = 7.8 Hz, 1H), 7.56 (t, J = 7.8 Hz, 1H), 7.14 (s, 1H), 6.98 (s, 1H), 5.33 (t, J = 5.9 Hz, 1H), 4.46 (d, J = 5.9 Hz, 2H), 4.23 (d, J = 14.0 Hz, 2H), 3.02 (t, J = 13.1 Hz, 2H), 2.37 (s, 3H), 1.92 (d, J = 13.6 Hz, 2H), 1.52 (dt, J = 13.4, 7.1 Hz, 2H), 1.37 (s, 3H).

### Example 26

### N-[4-(3-Cyanophenyl)-5-[2-(hydroxymethyl)-6-(trifluoromethyl)-4-pyridyl]thiazol-2-yl]-2-oxa-6-azaspiro[3.3]heptane-6-carboxamide

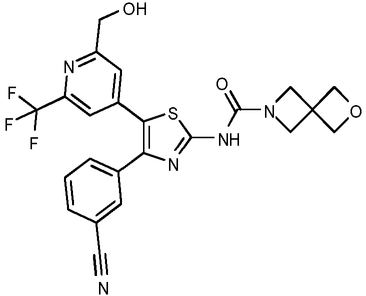

The title compound was prepared from 3-[2-amino-5-[2-[[*tert-*butyl(dimethyl)silyl]oxymethyl]-6-(trifluoromethyl)-4-pyridyl]thiazol-4-yl]benzonitrile (Intermediate IA) and 2-oxa-6-azaspiro[3.3]heptane analogously to Example 9.
LC-MS (Method 8B): Rt 3.49 mins; MS m/z 502.2 = [M+H]+
1H NMR (500 MHz, DMSO-d6) δ 11.52 (s, 1H), 7.92 (t, J = 1.8 Hz, 1H), 7.88 (dt, J = 7.8, 1.4 Hz, 1H), 7.76 (dt, J = 8.0, 1.4 Hz, 1H), 7.65 (d, J = 1.5 Hz, 1H), 7.61 (t, J = 7.8 Hz, 1H), 7.39 (d, J = 1.6 Hz, 1H), 5.63 (t, J = 5.9 Hz, 1H), 4.68 (s, 4H), 4.60 (d, J = 5.9 Hz, 2H), 4.23 (s, 4H).

### Example 27

### 3-Cyano-N-[4-(3-cyanophenyl)-5-(2,6-dimethyl-4-pyridyl)thiazol-2-yl]-3-methyl-pyrrolidine-1-carboxamide

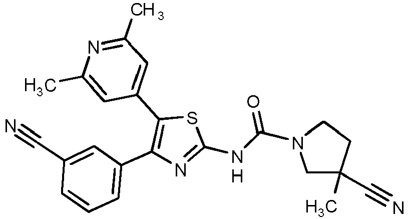

CDI (1.16 g, 7.18 mmol) and DIPEA (1.88 mL, 10.77 mmol) were added to a stirred solution of 3-[2-amino-5-(2,6-dimethyl-4-pyridyl)thiazol-4-yl]benzonitrile (Intermediate C) (1.1 g, 3.59 mmol) in DMA (11 mL) and the solution was heated at 50 °C overnight. 3-Methylpyrrolidine-3-carbonitrile hydrochloride (1.00 g, 6.82 mmol) and additional DIPEA (1.88 mL, 10.77 mmol) were added and the solution was stirred at 50 °C for 30 mins. The resulting mixture was allowed to cool to room temperature and diluted with EtOAc (150 mL). The mixture was washed with 90% aqueous brine (100 mL) and 50% aqueous brine (3 x 100 mL), dried over MgSO₄ and concentrated *in vacuo.* Purification by chromatography on silica eluting with a gradient of 1 to 2% MeOH in DCM afforded a yellow solid. Addition of MeOH (10 mL) afforded a white solid which was collected by filtration, washed with MeOH (2 mL) and Et₂O (20 mL) and dried to afford the title compound as a white solid.
LC-MS (Method 8B): Rt 3.03 mins; MS m/z 443.1 = [M+H]+
1H NMR (500 MHz, DMSO-d6) δ 11.24 (s, 1H), 7.89 (t, J = 1.8 Hz, 1H), 7.82 (dt, J = 7.7, 1.4 Hz, 1H), 7.71 (dt, J = 8.0, 1.5 Hz, 1H), 7.56 (t, J = 7.8 Hz, 1H), 6.94 (s, 2H), 3.96 - 3.86 (m, 1H), 3.64 (d, J = 34.3 Hz, 2H), 3.42 (d, J = 11.1 Hz, 1H), 2.43 - 2.38 (m, 1H), 2.36 (s, 6H), 2.14 - 2.02 (m, 1H), 1.47 (s, 3H).

### Example 27a and Example 27b

Racemic 3-cyano-N-[4-(3-cyanophenyl)-5-(2,6-dimethyl-4-pyridyl)thiazol-2-yl]-3-methyl-pyrrolidine-1-carboxamide (Example 27) was dissolved to 50 mg/mL in EtOH/DCM (4:1) and purified by SFC. The combined fractions of each enantiomer were evaporated to near dryness before being transferred into final vessels with DCM, which was removed under a stream of compressed air at 35 °C, before being stored in a vacuum oven at 35 °C and 5 mbar until constant weight was reached.

### Purification Conditions;

| | |
|---|---|
| Column Details | Lux iA3 (21.2mm x 250mm, 5um) |
| Column Temp | 40 °C |
| Flow Rate | 50 mL/min |
| BPR | 100 BarG |
| Detector Wavelength | 216 nm |
| Injection Volume | 250 uL (12.5 mg) |
| Isocratic Conditions | 35:65 EtOH:CO₂ (0.2% v/v NH₃) |

### Chiral Purity Analysis Conditions;

| | |
|---|---|
| Column Details | Chiralpak IG (4.6mm x 250mm, 5um) |
| Column Temp | 40 °C |
| Flow Rate | 4 mL/min |
| Detector Wavelength | 210-400 nm |
| Injection Volume | 1.0 uL |
| BPR | 125 BarG |
| Isocratic Conditions | 25:75 EtOH:CO₂ (0.2% v/v NH₃) |

### Example 27a

### (3R)-3-Cyano-N-[4-(3-cyanophenyl)-5-(2,6-dimethyl-4-pyridyl)thiazol-2-yl]-3-methyl-pyrrolidine-1-carboxamide

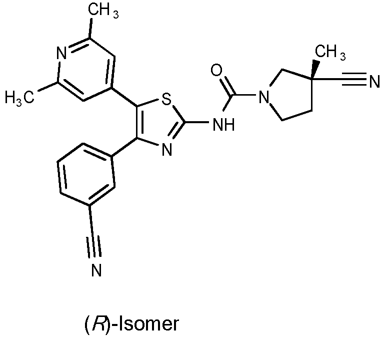

### First Eluted Peak: Chiral LCMS retention time: = 7.05 mins

LC-MS (Method 8B): Rt 3.04 mins; MS m/z 443.0 = [M+H]+
1H NMR (500 MHz, DMSO-d6) δ 11.24 (s, 1H), 7.89 (t, J = 1.6 Hz, 1H), 7.82 (dt, J = 7.7, 1.6 Hz, 1H), 7.71 (dt, J = 8.0, 1.6 Hz, 1H), 7.56 (apr t, J = 7.8 Hz, 1H), 6.93 (s, 2H), 3.98 - 3.86 (m, 1H), 3.72 - 3.63 (m, 1H), 3.64 - 3.55 (m, 1H), 3.46 - 3.38 (m, 1H), 2.43 - 2.36 (m, 1H), 2.36 (s, 6H), 2.12 - 2.03 (m, 1H), 1.47 (s, 3H).

### Example 27b

### (3S)-3-cyano-N-[4-(3-cyanophenyl)-5-(2,6-dimethyl-4-pyridyl)thiazol-2-yl]-3-methyl-pyrrolidine-1-carboxamide

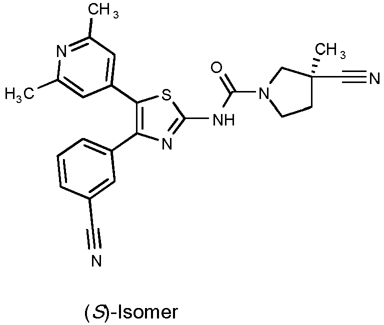

### Second Eluted Peak: Chiral LCMS retention time: = 8.59 mins

LC-MS (Method 8B): Rt 2.92 mins; MS m/z 443.0 = [M+H]+
1H NMR (500 MHz, DMSO-d6) δ 11.24 (s, 1H), 7.89 (t, J = 1.6 Hz, 1H), 7.82 (dt, J = 7.7, 1.6 Hz, 1H), 7.71 (dt, J = 8.0, 1.6 Hz, 1H), 7.56 (apr t, J = 7.8 Hz, 1H), 6.93 (s, 2H), 3.98 - 3.87 (m, 1H), 3.71 = 3.63 (m, 1H), 3.64 - 3.55 (m, 1H), 3.46 - 3.37 (m, 1H), 2.43 - 2.38 (m, 1H), 2.36 (s, 6H), 2.13 - 2.02 (m, 1H), 1.47 (s, 3H).

A further sample of Example 27b was obtained under the following conditions:
Racemic 3-Cyano-N-[4-(3-cyanophenyl)-5-(2,6-dimethyl-4-pyridyl)thiazol-2-yl]-3-methyl-pyrrolidine-1-carboxamide (Example 27) was dissolved to 25 mg/mL in EtOH/DCM (4:1) and purified by SFC. The combined fractions of each enantiomer were evaporated to near dryness. The resultant solids were transferred into final vessels with DCM, which was removed under a stream of compressed air at 35 °C, before being stored in a vacuum oven at 35 °C and 5 mbar until constant weight was reached. The material was triturated in Et₂O/MeOH under sonication and the resulting thick suspension filtered. The solids were washed with Et₂O/MeOH and vacuum dried on the sinter followed by further drying in a vacuum oven at 50 °C overnight to afford the title compound as a cream solid.

### Purification Conditions;

| | |
|---|---|
| Column Details | Lux iA3 (21.2mm x 250mm, 5um) |
| Column Temp | 40 °C |
| Flow Rate | 50 mL/min |
| BPR | 100 BarG |
| Detector Wavelength | 217 nm |
| Injection Volume | 500 uL (12.5 mg) |
| Isocratic Conditions | 25:75 EtOH:CO₂ (0.2% v/v NH₃) |

### Chiral Purity Analysis Conditions;

| | |
|---|---|
| Column Details | Chiralpak IG (4.6mm x 250mm, 5um) |
| Column Temp | 40 °C |
| Flow Rate | 4 mL/min |
| Detector Wavelength | 210-400 nm |
| Injection Volume | 1.0 uL |
| BPR | 125 BarG |
| Isocratic Conditions | 25:75 EtOH:CO₂ (0.2% v/v NH₃) |

### Second Eluted Peak: Chiral LCMS retention time: = 9.73 mins

LC-MS (Method 8A): Rt 2.37 mins; MS m/z 443.3 = [M+H]+
1H NMR (500 MHz, DMSO-d6) δ 11.23 (br s, 1H), 7.89 (br t, J = 1.5 Hz, 1H), 7.82 (br dt, J = 7.7, 1.5 Hz, 1H), 7.71 (br dt, J = 8.0, 1.5 Hz, 1H), 7.56 (apr t, J = 7.8 Hz, 1H), 6.93 (s, 2H), 3.92 (br s, 1H), 3.67 (br s, 1H), 3.60 (br s, 1H), 3.41 (br d, J = 11.2 Hz, 1H), 2.39 (s, 1H), 2.36 (s, 6H), 2.12 - 2.03 (m, 1H), 1.47 (s, 3H).

Examples 27a and 27b were alternatively prepared from 3-[2-amino-5-(2,6-dimethyl-4-pyridyl)thiazol-4-yl]benzonitrile (Intermediate C) and either (3R)-3-methylpyrrolidine-3-carbonitrile hydroiodide (Intermediate U) or (3S)-3-methylpyrrolidine-3-carbonitrile hydroiodide (Intermediate UA) analogously to Example 6.

### Example 28

### N-[5-[2,6-Bis(trideuteriomethyl)-4-pyridyl]-4-(3-cyanophenyl)thiazol-2-yl]-4-cyano-4-methyl-piperidine-1-carboxamide

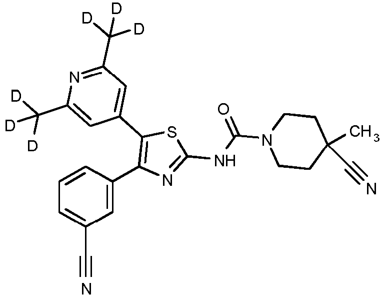

3-[2-Amino-5-[2,6-bis(trideuteriomethyl)-4-pyridyl]thiazol-4-yl]benzonitrile (Intermediate N) (465 mg, 1.49 mmol) and CDI (483 mg, 2.98 mmol) were dissolved in anhydrous DMA (7.5 mL) in a sealed tube under nitrogen. DIPEA (778 µL, 4.47 mmol) was added and the resulting solution stirred at 50 °C for 17 h. 4-Methylpiperidine-4-carbonitrile hydrochloride (478 mg, 2.98 mmol) and additional DIPEA (778 µL, 4.47mmol) were added and the mixture stirred at 50 °C for 1 h. The mixture was allowed to cool to room temperature before being diluted with EtOAc (80 mL) and washed successively with 80% brine (80 mL), 20% brine (2 x 80 mL) and 50% brine (80 mL). The organic layer was dried over Na₂SO₄, filtered and concentrated *in vacuo* to afford the crude material as a pale yellow foam. Purification by chromatography on silica eluting with a gradient of 1 to 2% MeOH in DCM afforded a clear straw coloured glass which was triturated and azeotroped with Et₂O to afford the title compound as a cream solid.
LC-MS (Method 8B): Rt 3.08 mins; MS m/z 463.1 = [M+H]+
1H NMR (500 MHz, DMSO-d6) δ 11.31 (s, 1H), 7.89 (t, J = 1.6 Hz, 1H), 7.82 (dt, J = 7.8, 1.6 Hz, 1H), 7.70 (dt, J = 8.0, 1.6 Hz, 1H), 7.56 (apr t, J = 7.8 Hz, 1H), 6.93 (s, 2H), 4.22 (d, J = 13.9 Hz, 2H), 3.03 (t, J = 13.3 Hz, 2H), 1.92 (d, J = 13.9 Hz, 2H), 1.53 (td, J = 13.3, 4.0 Hz, 2H), 1.37 (s, 3H).

### Preparation of Intermediates

### Intermediate A

### 3-(2-Aminothiazol-4-yl)benzonitrile hydrobromide

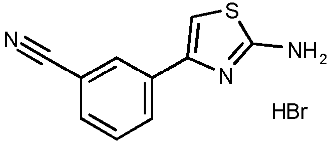

The following was prepared according to the procedure of Wang, Y. and Yang, T. in patent WO2012/100734, page 24, Example 15 step 2.

To a flask containing 3-(2-bromoacetyl)benzonitrile (5 g, 22.32 mmol) in EtOH (50 mL) was added thiourea (1.87 g, 24.55 mmol) and the mixture was stirred at room temperature for 30 mins. The volume of solvent was reduced by ~50% *in vacuo* resulting in the formation of a suspension. The suspension was sonicated, filtered and dried to afford the title compound as a colourless solid.
LC-MS (Method 3B): Rt 1.62 mins; MS m/z 202.3 = [M+H]+
¹H NMR (500 MHz, DMSO-*d*₆) δ 8.22 (t, *J =* 1.8 Hz, 1H), 8.08 (d, *J =* 8.1 Hz, 1H), 7.82 (d, *J* = 7.7 Hz, 1H), 7.65 (t, *J* = 7.9 Hz, 1H), 7.37 (s, 1H). NH₂ protons not observed.

3-(2-Aminothiazol-4-yl)benzonitrile may also be prepared as follows:
To a flask containing thiourea (2.04 g, 26.78 mmol) in pyridine (50 mL) under nitrogen was added 3-(2-bromoacetyl)benzonitrile (3.0 g, 13.39 mmol) and the mixture was stirred at room temperature for 1 h. The resulting mixture was diluted with water (100 mL) and extracted with EtOAc (2 x 100 mL). The combined organic extracts were washed with brine (50 mL), dried over MgSO₄ and concentrated *in vacuo.* The crude product was purified by chromatography on silica eluting with 0-100% EtOAc in DCM. The resulting solid was recrystallised from EtOAc to afford 3-(2-aminothiazol-4-yl)benzonitrile as a pale yellow solid LC-MS (Method 7A): Rt 1.75 mins; MS m/z 202.1 = [M+H]+
¹H NMR (400 MHz, DMSO-*d*₆) δ 8.20 (t, *J* = 1.5 Hz, 1H), 8.11 (dt, *J* = 7.9, 1.4 Hz, 1H), 7.70 (dt, *J =* 7.7, 1.3 Hz, 1H), 7.58 (t, *J =* 7.6 Hz, 1H), 7.26 (s, 1H), 7.14 (s, 2H).

### Intermediate B

### 3-(2-Amino-5-bromo-thiazol-4-yl)benzonitrile

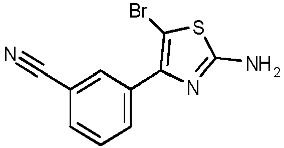

The following was prepared according to the procedure Wang, Y. and Yang, T. in patent WO2012/100734, page 24-25, Example 15 step 3.

To a solution of 3-(2-aminothiazol-4-yl)benzonitrile hydrobromide (Intermediate A)(2.6 g, 9.21 mmol) in THF (40 mL) was added NBS (1.97 g, 11.06 mmol) and the reaction mixture was stirred at room temperature for 1 h. The resulting mixture was diluted with EtOAc (60 mL) and washed with water (100 mL). The organic layer was separated and the aqueous further extracted with EtOAc (2 x 50 mL). The combined organics were dried over MgSO₄ and concentrated *in vacuo* to yield an orange solid. The solid was suspended in DCM (20 mL), filtered, washed with DCM and dried to afford the title compound as an off-white solid.
LC-MS (Method 3B): Rt 1.64 mins; MS m/z 279.9/281.9 = [M+H]+
¹H NMR (500 MHz, DMSO-*d*₆) δ 8.16 (t, *J =* 1.7 Hz, 1H), 8.15 (dt, *J =* 7.9, 1.7 Hz, 1H), 7.83 (d, *J* = 7.9 Hz, 1H), 7.66 (t, *J* = 7.9 Hz, 1H), 7.42 (s, 2H).

### Intermediate C

### 3-[2-Amino-5-(2,6-dimethyl-4-pyridyl)thiazol-4-yl]benzonitrile

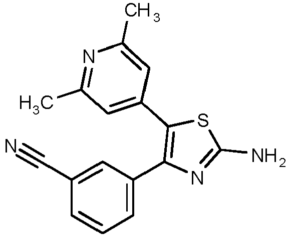

A solution of potassium carbonate (43.1 g, 312.34 mmol) in water (210 mL) was added to a solution of 3-(2-amino-5-bromo-thiazol-4-yl)benzonitrile (Intermediate B) (35.0 g, 124.94 mmol) and 2,6-dimethyl-4-(4,4,5,5-tetramethyl-1,3,2-dioxaborolan-2-yl)pyridine (34.95 g, 149.93 mmol) in 1,4-dioxane (840 mL) and the mixture de-oxygenated via nitrogen sparging for 20 mins. Bis(tri-tert-butylphosphine)palladium(0) (6.38 g, 12.49 mmol) was added, the flask sealed and the contents evacuated and backfilled with nitrogen (3 x cycles). The reaction mixture was stirred at 80 °C for 1 h and then allowed to cool to room temperature. The mixture was diluted with EtOAc (500 mL) and water (200 mL) and the layers were separated. The organic layer was washed with 20% brine (500 mL), neat brine (200 mL), dried over Na₂SO₄ and filtered through a plug of Celite^{®} (filter material), eluting with EtOAc (4 x 200 mL). The filtrate was concentrated *in vacuo* and the resulting material was triturated in DCM (150 mL) via sonication. The solids were collected by filtration, washed with DCM (3 x 50 mL) and vacuum dried to afford the title compound as a pale tan solid.
LC-MS (Method 2B): Rt 1.26 mins; MS m/z 307.0 = [M+H]+
1H NMR (500 MHz, DMSO-d6) δ 7.81 (br t, J = 1.5 Hz, 1H), 7.79 (br dt, J = 7.6, 1.5 Hz, 1H), 7.66 (br dt, J = 7.9, 1.5 Hz, 1H), 7.52 (apr t, J = 7.8 Hz, 1H), 7.50 - 7.36 (m, 2H), 6.80 (s, 2H), 2.32 (s, 6H).

### Intermediate D

### 3-[2-Amino-5-(2-chloro-6-methyl-4-pyridyl)thiazol-4-yl]benzonitrile

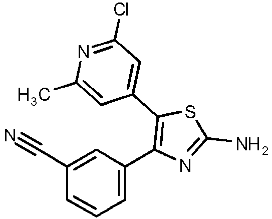

A solution of 3-(2-amino-5-bromo-thiazol-4-yl)benzonitrile (Intermediate B) (710 mg, 2.53 mmol), 2-chloro-6-methyl-4-(4,4,5,5-tetramethyl-1,3,2-dioxaborolan-2-yl)pyridine (649 mg, 2.56 mmol) and cesium carbonate (1652 mg, 5.07 mmol) in monoglyme (28.4 mL) and water (3.5 mL) was degassed with nitrogen for 5 mins. Pd(dppf)Cl₂ · CH₂Cl₂ (517 mg, 0.63 mmol) was added and the reaction mixture was heated at 80 °C under nitrogen for 1 h. After cooling to room temperature, the resulting mixture was filtered and the precipitate was washed with EtOAc (50 mL). The filtrate was partitioned between water (20 mL) and brine (20 mL) and the organic layer was separated, dried over MgSO₄ and concentrated *in vacuo.* The crude product was purified by chromatography on silica eluting with 0-50% EtOAc in DCM to afford the title compound as a brown solid.
LC-MS (Method 2A): Rt 1.12 mins; MS m/z 327.0/329.0 = [M+H]+
¹H NMR (400 MHz, DMSO-d6) δ 7.86 - 7.80 (m, 2H), 7.69 (dt, J = 7.9, 1.4 Hz, 1H), 7.60 (s, 2H), 7.59 - 7.54 (m, 1H), 7.03 - 6.99 (m, 1H), 6.92 - 6.89 (m, 1H), 2.35 (s, 3H).

### Intermediate E

### 4-(Hydroxymethyl)piperidine-4-carbonitrile hydrochloride

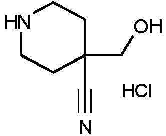

To a solution of *tert-butyl* 4-cyano-4-(hydroxymethyl)piperidine-1-carboxylate (100 mg, 0.42 mmol) in DCM (1 mL) was added 4M HCl in 1,4-dioxane (2.08 mL, 8.32 mmol) and the reaction mixture was stirred at room temperature overnight. The resulting mixture was concentrated *in vacuo* to afford the title compound as a colourless solid.
¹H NMR (400 MHz, DMSO-*d*₆) δ 9.11 (s, 2H), 5.66 (t, *J =* 5.8 Hz, 1H), 3.52 (d, *J =* 5.8 Hz, 2H), 3.41 - 3.34 (m, 2H), 2.90 (td, J = 13.1, 3.0 Hz, 2H), 2.04 (d, *J =* 14.2 Hz, 2H), 1.85 - 1.73 (m, 2H).

### Intermediate F

### Piperidine-4,4-dicarbonitrile hydrochloride

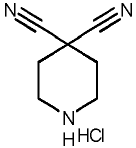

### Step 1: 1-Benzylpiperidine-4,4-dicarbonitrile

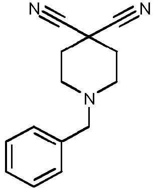

The following was prepared according to the procedure of Zahler, R. and Vath, J. in patent WO2017027684 (A1), pages 56 and 57.

K₂CO₃ (1132 mg, 8.19 mmol) was added to a solution of propanedinitrile (0.23 mL, 3.72 mmol) in DMF (5 mL) and the reaction mixture was stirred at 65 °C for 2 h. To this mixture was added N-benzyl-2-chloro-N-(2-chloroethyl)ethanamine hydrochloride (1.0 g, 3.72 mmol) in DMF (5 mL) and stirring continued at 65 °C for 16 h. After cooling to room temperature, the reaction was quenched with ice cold water. The resulting mixture was extracted with EtOAc (3 x 10 mL), dried over Na₂SO₄ and concentrated *in vacuo.* Purification of the crude material by chromatography on silica eluting with 10-40% EtOAc in petroleum ether afforded the title compound as a colourless oil.
LC-MS (Method 8B): Rt 4.31 mins; MS m/z 226.2 = [M+H]+
¹H NMR (500 MHz, CDCl₃) δ 7.37 - 7.25 (m, 5H), 3.56 (s, 2H), 2.64 (s, 4H), 2.25 (s, 4H).

### Step 2: Piperidine-4,4-dicarbonitrile hydrochloride

1-Chloroethyl carbonochloridate (0.36 mL, 3.31 mmol) was added dropwise to a stirred solution of 1-benzylpiperidine-4,4-dicarbonitrile (step 1) (622 mg, 2.76 mmol) in DCE (5 mL) at 0 °C. The reaction mixture was heated at reflux for 24 h and then concentrated *in vacuo* to give a colourless oil. Methanol (5 mL) was then added and the solution was heated at reflux for 3 h. The resulting mixture was concentrated *in vacuo* to afford the title compound as a colourless solid.
¹H NMR (500 MHz, DMSO-d6) δ 9.38 (m, 2H), 3.21 (s, 4H), 2.65 - 2.57 (m, 4H).

### Intermediate G

### 3-[2-Amino-5-(2-difluoromethyl-6-methyl-4-pyridyl)thiazol-4-yl]benzonitrile

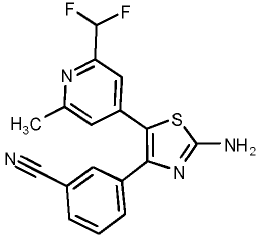

The title compound was prepared from 3-(2-amino-5-bromo-thiazol-4-yl)benzonitrile (Intermediate B) and 2-(difluoromethyl)-6-methyl-4-(4,4,5,5-tetramethyl-1,3,2-dioxaborolan-2-yl)pyridine analogously to Intermediate C.
LC-MS (Method 5B): Rt 2.83 mins; MS m/z 343.1 = [M+H]+
1H NMR (500 MHz, DMSO-d6) δ 7.84 (dd, J = 4.8, 3.0 Hz, 2H), 7.70 (d, J = 7.9 Hz, 1H), 7.63 (s, 2H), 7.56 (t, J = 8.1 Hz, 1H), 7.20 (s, 1H), 7.07 (s, 1H), 6.80 (t, J = 55.0 Hz, 1H), 2.44 (s, 3H).

### Intermediate H

### 3-[2-Amino-5-(2-trifluoromethyl-6-methyl-4-pyridyl)thiazol-4-yl]benzonitrile

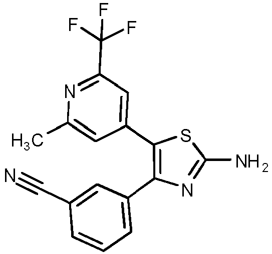

The title compound was prepared from 3-(2-amino-5-bromo-thiazol-4-yl)benzonitrile (Intermediate B) and 2-methyl-4-(4,4,5,5-tetramethyl-1,3,2-dioxaborolan-2-yl)-6-(trifluoromethyl)pyridine analogously to Intermediate C.
LC-MS (Method 3B): Rt 1.86 mins; MS m/z 359.3 = [M-H]-
1H NMR (500 MHz, DMSO-d6) δ 7.88 - 7.81 (m, 2H), 7.71 (d, J = 8.0 Hz, 1H), 7.68 (s, 2H), 7.57 (dd, J = 9.6, 6.6 Hz, 1H), 7.33 (s, 1H), 7.15 (s, 1H), 2.47 (s, 3H).

### Intermediate I

### 3-[2-Amino-5-[2-[[tert-butyl(dimethyl)silyl]oxymethyl]-6-methyl-4-pyridyl]thiazol-4-yl]benzonitrile

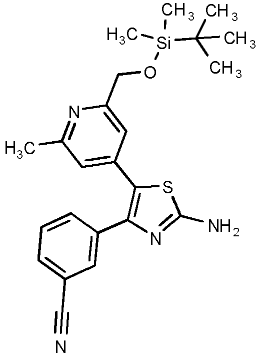

### Step 1: (4-Bromo-6-methyl-2-pyridyl)methoxy-tert-butyl-dimethyl-silane

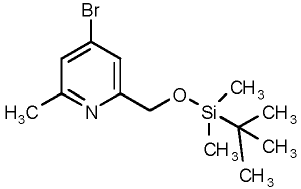

To a solution of (4-bromo-6-methyl-2-pyridyl)methanol (2.0 g, 9.9 mmol) in DMF (15 mL) at 0 °C was added imidazole (0.03 mL, 12.87 mmol) followed by *tert-*butyldimethylsilyl chloride (1.11 mL, 10.89 mmol) and the reaction mixture was stirred at room temperature for 1 h. The resulting mixture was partitioned between EtOAc (150 mL) and water (150 mL) and the layers separated. The aqueous layer was further extracted with EtOAc (100 mL) and the combined organic extracts were washed with sat. NaHCO₃ solution (150 mL), brine (150 mL), dried over MgSO₄ and concentrated *in vacuo* to afford the title compound as a yellow oil which slowly crystallised to give a yellow solid.
1H NMR (500 MHz, DMSO-d6) δ 7.45 (d, J = 0.8 Hz, 1H), 7.38 (s, 1H), 4.70 (s, 2H), 2.43 (s, 3H), 0.91 (s, 9H), 0.10 (s, 6H).

### Step 2: 2-{[(tert-butyldimethylsilyl)oxy]methyl}-6-methyl-4-(4,4,5,5-tetramethyl-1,3,2-dioxaborolan-2-yl)pyridine

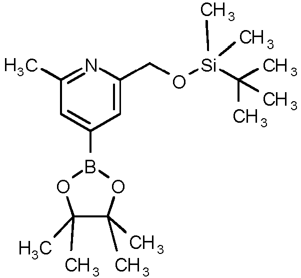

A solution of (4-bromo-6-methyl-2-pyridyl)methoxy-tert-butyl-dimethyl-silane (step 1) (2.96 g, 9.35 mmol), bis(pinacolato)diboron (3.56 g, 14.02 mmol), potassium acetate (5.16 g, 37.39 mmol) and Pd(dppf)Cl₂ (889 mg, 1.22 mmol) in 1,4-dioxane (45 mL) was heated to 80 °C overnight. The resulting mixture was concentrated *in vacuo* and the residue was dissolved in hexane (adding a small volume of DCM to aid dissolution). The mixture was filtered through Celite^{®} (filter material) and concentrated *in vacuo* to afford the title compound as a brown solid.
1H NMR (500 MHz, DMSO-d6) δ 7.46 (s, 1H), 7.31 (s, 1H), 4.71 (s, 2H), 2.44 (s, 3H), 1.30 (s, 12H), 0.91 (s, 9H), 0.09 (s, 6H).

### Step 3: 3-[2-Amino-5-[2-[[tert-butyl(dimethyl)silyl]oxymethyl]-6-methyl-4-pyridyl]thiazol-4-yl]benzonitrile

To a degassed mixture of 3-(2-amino-5-bromo-thiazol-4-yl)benzonitrile (Intermediate B)(1.0 g, 3.57mmol), 2-{[(tert-butyldimethylsilyl)oxy]methyl}-6-methyl-4-(4,4,5,5-tetramethyl-1,3,2-dioxaborolan-2-yl)pyridine (step 2)(2.59 g, 7.14 mmol) and potassium carbonate (1.97 g, 14.28 mmol) in 1,4-dioxane (20 mL) and water (2 mL) was added bis(tri-tert-butylphosphine)palladium(0) (182 mg, 0.36 mmol). The reaction mixture was heated at 80 °C for 1 h and then allowed to cool to room temperature. The resulting mixture was diluted in EtOAc (100 mL) and water (100 mL). The layers were separated and the aqueous layer was further extracted with EtOAc (2 x 50 mL). The combined organic extracts were dried over MgSO₄ and concentrated *in vacuo.* Purification by chromatography on silica eluting with a gradient of 10 to 40% EtOAc in petroleum ether afforded a solid which was triturated in diethyl ether (8 mL), collected by filtration, washed with diethyl ether (3 x 2 mL) and dried under vacuum overnight to afford the title compound as a yellow solid.
LC-MS (Method 3B): Rt 2.28 mins; MS m/z 435.4 = [M-H]-
1H NMR (500 MHz, DMSO-d6) δ 7.82 - 7.75 (m, 2H), 7.68 (dt, J = 7.9, 1.5 Hz, 1H), 7.52 (td, J = 7.6, 7.0, 1.3 Hz, 1H), 7.48 (s, 2H), 6.94 (d, J = 2.8 Hz, 2H), 4.59 (s, 2H), 2.37 (s, 3H), 0.78 (s, 9H), -0.02 (s, 6H).

### Intermediate IA

### 3-[2-Amino-5-[2-[[tert-butyl(dimethyl)silyl]oxymethyl]-6-(trifluoromethyl)-4-pyridyl]thiazol-4-yl]benzonitrile

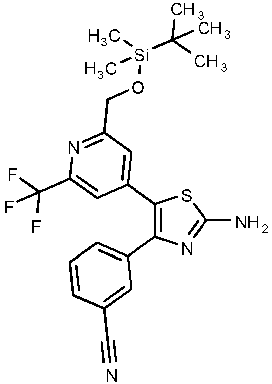

### Step 1: [4-Bromo-6-(trifluoromethyl)-2-pyridyl]methoxy-tert-butyl-dimethyl-silane

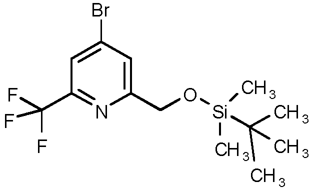

The title compound was prepared from [4-bromo-6-(trifluoromethyl)-2-pyridyl]methanol and tert-butyldimethylsilyl chloride analogously to Intermediate I step 1. LC-MS (Method 3B): Rt 2.84 mins; MS m/z 370.0 / 372.0 = [M+H]+
1H NMR (500 MHz, Chloroform-d) δ 7.88 (s, 1H), 7.73 - 7.69 (m, 1H), 4.86 (s, 2H), 0.97 (s, 9H), 0.14 (s, 6H).

### Step 2: tert-Butyl-dimethyl-[[4-(4,4,5,5-tetramethyl-1,3,2-dioxaborolan-2-yl)-6-(trifluoromethyl)-2-pyridyl]methoxy]silane

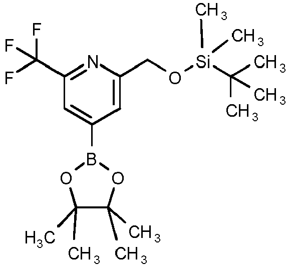

The title compound was prepared from [4-bromo-6-(trifluoromethyl)-2-pyridyl]methoxy-tert-butyl-dimethyl-silane (step 1) and bis(pinacolato)diboron analogously to Intermediate I step 2.
1H NMR (500 MHz, Chloroform-d) δ 8.04 (s, 1H), 7.88 (d, J = 0.9 Hz, 1H), 4.89 (s, 2H), 1.36 (s, 12H), 0.96 (s, 9H), 0.13 (s, 6H).

### Step 3: 3-[2-Amino-5-[2-[[tert-butyl(dimethyl)silyl]oxymethyl]-6-(trifluoromethyl)-4-pyridyl]thiazol-4-yl]benzonitrile

The title compound was prepared from *tert*-butyl-dimethyl-[[4-(4,4,5,5-tetramethyl-1,3,2-dioxaborolan-2-yl)-6-(trifluoromethyl)-2-pyridyl]methoxy]silane (step 2) and 3-(2-amino-5-bromo-thiazol-4-yl)benzonitrile (Intermediate B) analogously to Intermediate I step 3.
LC-MS (Method 3B): Rt 2.38 mins; MS m/z 491.2 = [M+H]+
1H NMR (500 MHz, DMSO-d6) δ 7.86 (t, J = 1.8 Hz, 1H), 7.83 (dt, J = 7.7, 1.5 Hz, 1H), 7.75 - 7.68 (m, 3H), 7.54 (t, J = 7.8 Hz, 1H), 7.36 (d, J = 1.6 Hz, 1H), 7.29 (d, J = 1.6 Hz, 1H), 4.70 (s, 2H), 0.79 (s, 9H), 0.00 (s, 6H).

### Intermediate J

### 3-[2-Amino-5-[2-[1-[(4-methoxyphenyl)methoxy]-1-methyl-ethyl]-6-methyl-4-pyridyl]thiazol-4-yl]benzonitrile

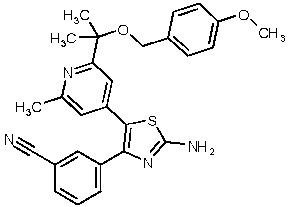

### Step 1: 2-(4-Bromo-6-methyl-2-pyridyl)propan-2-ol

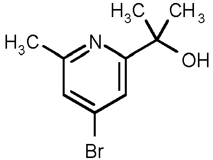

A solution of ethyl 4-bromo-6-methyl-pyridine-2-carboxylate (1.04 g, 4.25 mmol) in anhydrous THF (20 mL) was added dropwise over 10 mins to a stirred solution of methylmagnesium bromide (3.4M in 2-Me-THF) (2.75 mL, 9.36 mmol) at 0 °C under nitrogen and stirred for 5 mins. The ice bath was removed and the mixture stirred at room temperature for 20 mins. The resulting mixture was re-cooled to 5 °C and the reaction was quenched by addition of 1M HCl (9 mL). The mixture was diluted with water (30 mL) and EtOAc (30 mL) and the pH of the aqueous layer was adjusted to pH 7 by addition of saturated. NaHCO₃ solution before the layers were shaken and separated. The aqueous layer was extracted with EtOAc (30 mL) and the combined organic extracts were dried over Na₂SO₄ and concentrated *in vacuo* to afford the title compound as a pale yellow oil.
LC-MS (Method 3B): Rt 1.59 mins; MS m/z 230.1 / 232.1 = [M+H]+
1H NMR (500 MHz, CDCl₃) δ 7.33 (d, J = 1.0 Hz, 1H), 7.24 (d, J = 1.0 Hz, 1H), 5.08 (s, 1H), 2.54 (s, 3H), 1.51 (s, 6H).

### Step 2: 4-Bromo-2-[1-[(4-methoxyphenyl)methoxy]-1-methyl-ethyl]-6-methyl-pyridine

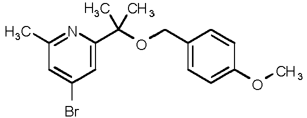

NaH (60% in oil) (229 mg, 5.74 mmol) was added to a solution of 2-(4-bromo-6-methyl-2-pyridyl)propan-2-ol (step 1)(660 mg, 2.87 mmol) in anhydrous DMF (15 mL) under nitrogen and the mixture stirred at room temperature for 5 mins. 4-Methoxybenzyl bromide (620 µL, 4.3 mmol) was added via syringe and the resulting mixture stirred at room temperature for 1 h. Additional NaH (60% in oil) (229 mg, 5.74 mmol) and 4-methoxybenzyl bromide (413 µL, 2.87 mmol) were added and the mixture stirred at room temperature overnight. The reaction was quenched by the dropwise addition of water (1 mL) before being diluted with EtOAc (75 mL). The mixture was washed with 90% brine (75 mL), 50% brine (3 x 75 mL), brine (75 mL), dried over Na₂SO₄ and concentrated *in vacuo.* Purification by chromatography on silica eluting with 5% EtOAc in n-hexane afforded the title compound as a colourless oil.
LC-MS (Method 3B): Rt 2.46 mins; MS m/z 350.1 / 352.1 = [M+H]+
1H NMR (500 MHz, Chloroform-d) δ 7.62 (apr dd, J = 1.7, 0.7 Hz, 1H), 7.30 - 7.27 (m, 2H), 7.20 (d, J = 1.7 Hz, 1H), 6.91 - 6.88 (m, 2H), 4.29 (s, 2H), 3.81 (s, 3H), 2.52 (s, 3H), 1.61 (s, 6H).

### Step 3: 2-[1-[(4-Methoxyphenyl)methoxy]-1-methyl-ethyl]-6-methyl-4-(4,4,5,5-tetramethyl-1,3,2-dioxaborolan-2-yl)pyridine

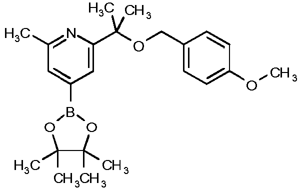

The title compound was prepared from 4-bromo-2-[1-[(4-methoxyphenyl)methoxy]-1-methylethyl]-6-methyl-pyridine (step 2) and bis(pinacolato)diboron analogously to Intermediate I step 2.
LC-MS (Method 3B): Rt 1.19 mins; MS m/z 316.2 = [M+H]+ [NB - mass ion for the parent boronic acid].
1H NMR (500 MHz, DMSO-d6) δ 7.62 (s, 1H), 7.32 (s, 1H), 7.27 - 7.23 (m, 2H), 6.90 - 6.86 (m, 2H), 4.19 (s, 2H), 3.74 (s, 3H), 2.49 (s, 3H), 1.53 (s, 6H), 1.30 (s, 12H).

### Step 4: 3-[2-Amino-5-[2-[1-[(4-methoxyphenyl)methoxy]-1-methyl-ethyl]-6-methyl-4-pyridyl]thiazol-4-yl]benzonitrile

A solution of K₂CO₃ (297 mg, 2.15 mmol) in water (1.5 mL) was added to a solution of 3-(2-amino-5-bromo-thiazol-4-yl)benzonitrile (Intermediate B) (215 mg, 0.77 mmol) and 2-[1-[(4-methoxyphenyl)methoxy]-1-methyl-ethyl]-6-methyl-4-(4,4,5,5-tetramethyl-1,3,2-dioxaborolan-2-yl)pyridine (step 3) (396 mg, 1.0 mmol) in 1,4-dioxane (6 mL) and the mixture degassed via nitrogen sparging for 10 mins. Pd(tBu₃P)₂ (39 mg, 0.08 mmol) was added and the mixture was heated at 50 °C for 1 h. The resulting mixture was allowed to cool to room temperature and diluted with EtOAc (30 mL). The mixture was washed with water (30 mL), brine (30 mL) and dried over Na₂SO₄, filtered and concentrated *in vacuo.* Purification by chromatography on silica eluting with 1% MeOH in DCM afforded a yellow gummy foam which was dissolved in Et₂O and concentrated to afford the title compound as a yellow solid.
LC-MS (Method 3B): Rt 2.21 mins; MS m/z 471.3 = [M+H]+
1H NMR (500 MHz, DMSO-d6) δ 7.78 (t, J = 1.8 Hz, 1H), 7.68 (dd, J = 7.8, 1.8 Hz, 2H), 7.50 (t, J = 7.8 Hz, 1H), 7.47 (s, 2H), 7.06 (d, J = 1.7 Hz, 1H), 7.01 (d, J = 1.7 Hz, 1H), 6.99 - 6.94 (m, 2H), 6.85 - 6.81 (m, 2H), 4.09 (s, 2H), 3.75 (s, 3H), 2.44 (s, 3H), 1.43 (s, 6H).

### Intermediate K

### 3-[2-Amino-5-(2-methoxy-6-methyl-4-pyridyl)thiazol-4-yl]benzonitrile

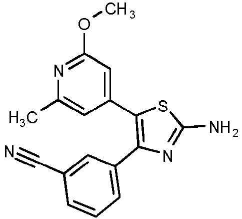

The title compound was prepared from 3-(2-amino-5-bromo-thiazol-4-yl)benzonitrile (Intermediate B) and 2-methoxy-6-methyl-4-(4,4,5,5-tetramethyl-1,3,2-dioxaborolan-2-yl)pyridine analogously to Intermediate C.
LC-MS (Method 3B): Rt 1.81 mins; MS m/z 321.2 = [M-H]-
1H NMR (500 MHz, DMSO-d6) δ 7.82 (s, 1H), 7.79 (d, J = 7.7 Hz, 1H), 7.67 (d, J = 8.1 Hz, 1H), 7.54 (t, J = 7.8 Hz, 1H), 7.46 (s, 2H), 6.60 (s, 1H), 6.29 (s, 1H), 3.34 (s, 3H), 2.29 (s, 3H).

### Intermediate KA

### 3-[2-Amino-5-(2-cyano-6-methyl-4-pyridyl)thiazol-4-yl]benzonitrile

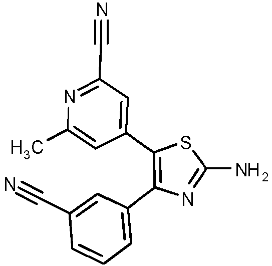

The title compound was prepared from 3-(2-amino-5-bromo-thiazol-4-yl)benzonitrile (Intermediate B) and 6-methyl-4-(4,4,5,5-tetramethyl-1,3,2-dioxaborolan-2-yl)pyridine-2-carbonitrile analogously to Intermediate C.
LC-MS (Method 3B): Rt 1.53 mins; MS m/z 318.0 = [M-H]+
1H NMR (500 MHz, DMSO-d6) δ 7.86 (s, 1H), 7.85 (s, 1H), 7.69 (s, 2H), 7.67 (s, 1H), 7.57 (t,J= 7.8 Hz, 1H), 7.47 (s, 1H), 7.32 (s, 1H), 2.42 (s, 3H).

### Intermediate KB

### 3-[2-Amino-5-[2-(fluoromethyl)-6-methyl-4-pyridyl]thiazol-4-yl]benzonitrile

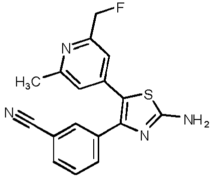

### Step 1: 4-Bromo-2-(fluoromethyl)-6-methyl-pyridine

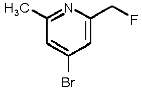

Diethylaminosulfur trifluoride (0.36 mL, 2.72 mmol) was added dropwise to a solution of (4-bromo-6-methyl-2-pyridyl)methanol (500 mg, 2.47 mmol) in DCM (5 mL) at -40 °C. The mixture was stirred at -40 °C for 30 mins and then allowed to warm to room temperature. The mixture was diluted with DCM (5 mL), water (5 mL) and sat. NaHCO₃ solution (5 mL). The layers were separated and the aqueous layer was further extracted with DCM (2 x 10 mL). The combined organic extracts were washed with brine (10 mL), dried over Na₂SO₄ and the solvent was removed *in vacuo.* Purification by chromatography on silica eluting with a gradient of 1 to 20% EtOAc in petroleum ether afforded the title compound as a white solid.
LC-MS (Method 3B): Rt 1.66 mins; MS m/z 204.0 / 205.9 = [M+H]+
1H NMR (500 MHz, Chloroform-d) δ 7.50 - 7.42 (m, 1H), 7.31 - 7.29 (m, 1H), 5.42 (d, J = 46.6 Hz, 2H), 2.52 (s, 3H).

### Step 2: 2-(Fluoromethyl)-6-methyl-4-(4,4,5,5-tetramethyl-1,3,2-dioxaborolan-2-yl)pyridine

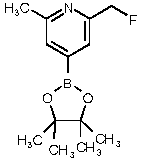

A mixture of 4-bromo-2-(fluoromethyl)-6-methyl-pyridine (step 1)(370 mg, 1.81 mmol), bis(pinacolato)diboron (553 mg, 2.18 mmol) and AcOK (534 mg, 5.44 mmol) in 1,4-dioxane (10 mL) was degassed under nitrogen for 10 mins. Pd(dppf)C_{I2} (133 mg, 0.18 mmol) was added and the flask was purged and back filled with nitrogen (x 3). The reaction mixture was heated to 95 °C for 16 h and then allowed to cool to room temperature. The resulting mixture was diluted with EtOAc (50 mL) and filtered through Celite^{®} (filter material), eluting with EtOAc. The filtrate was concentrated *in vacuo* to afford the title compound as a brown oil.
1H NMR (500 MHz, DMSO-d6) δ 7.93 (s, 1H), 7.45 (d, J = 11.9 Hz, 1H), 5.44 (d, J = 47.1 Hz, 2H), 3.57 (s, 3H), 1.31 (s, 12H).

### Step 3: 3-[2-Amino-5-[2-(fluoromethyl)-6-methyl-4-pyridyl]thiazol-4-yl]benzonitrile

The title compound was prepared from 3-(2-amino-5-bromo-thiazol-4-yl)benzonitrile (Intermediate B) and 2-(fluoromethyl)-6-methyl-4-(4,4,5,5-tetramethyl-1,3,2-dioxaborolan-2-yl)pyridine (step 2) analogously to Intermediate C.

LC-MS (Method 3B): Rt 1.56 mins; MS m/z 325.1 = [M+H]+
1H NMR (500 MHz, DMSO-d6) δ 7.84 - 7.79 (m, 2H), 7.68 (dt, J = 8.0, 1.4 Hz, 1H), 7.56 - 7.51 (m, 3H), 6.98 (d, J = 15.9 Hz, 2H), 5.32 (d, J = 46.9 Hz, 2H), 2.38 (s, 3H).

### Intermediate KC

### 3-[2-Amino-5-(2-cyclopropyl-6-methyl-4-pyridyl)thiazol-4-yl]benzonitrile

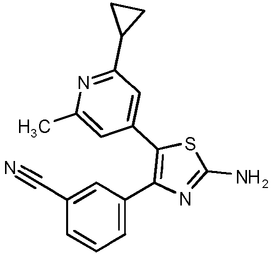

The title compound was prepared from 3-(2-amino-5-bromo-thiazol-4-yl)benzonitrile (Intermediate B) and 2-cyclopropyl-6-methyl-4-(4,4,5,5-tetramethyl-1,3,2-dioxaborolan-2-yl)pyridine analogously to Intermediate C.
LC-MS (Method 3B): Rt 1.33 mins; MS m/z 333.0 = [M+H]+
1H NMR (500 MHz, DMSO-d6) δ 7.81 (t, J = 1.5 Hz, 1H), 7.79 (dt, J = 7.7, 1.5 Hz, 1H), 7.67 (dt, J = 8.0, 1.5 Hz, 1H), 7.54 (apr t, J = 7.8 Hz, 1H), 7.44 (s, 2H), 6.76 (d, J = 1.6 Hz, 1H), 6.75 (d, J = 1.6 Hz, 1H), 2.28 (s, 3H), 1.92 (tt, J = 8.1, 4.8 Hz, 1H), 0.87 - 0.83 (m, 2H), 0.79 - 0.74 (m, 2H).

### Intermediate KD

### 3-[2-Amino-5-(2-isopropyl-6-methyl-4-pyridyl)thiazol-4-yl]benzonitrile

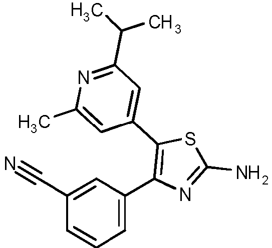

The title compound was prepared from 3-(2-amino-5-bromo-thiazol-4-yl)benzonitrile (Intermediate B) and 2-isopropyl-6-methyl-4-(4,4,5,5-tetramethyl-1,3,2-dioxaborolan-2-yl)pyridine analogously to Intermediate C.
LC-MS (Method 5B): Rt 2.86 mins; MS m/z 335.1 = [M+H]+
1H NMR (500 MHz, DMSO-d6) δ 7.80 (dd, J = 7.8, 1.5 Hz, 1H), 7.77 (d, J = 1.8 Hz, 1H), 7.71 (dt, J = 7.9, 1.5 Hz, 1H), 7.55 (t, J = 7.8 Hz, 1H), 7.45 (s, 2H), 6.89 (d, J = 1.6 Hz, 1H), 6.68 (d, J = 1.5 Hz, 1H), 3.92 (s, 3H), 2.79 (h, J = 6.8 Hz, 1H), 1.05 (s, 6H).

### Intermediate KE

### 3-[2-Amino-5-(2-ethyl-6-methyl-4-pyridyl)thiazol-4-yl]benzonitrile

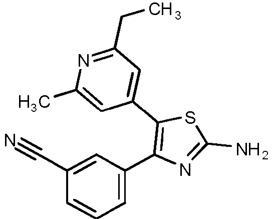

The title compound was prepared from 3-(2-amino-5-bromo-thiazol-4-yl)benzonitrile (Intermediate B) and 2-ethyl-6-methyl-4-(4,4,5,5-tetramethyl-1,3,2-dioxaborolan-2-yl)pyridine analogously to Intermediate C.
LC-MS (Method 5B): Rt 2.64 mins; MS m/z 321.1 = [M+H]+
1H NMR (500 MHz, CDCl₃) δ 7.86 (t, J = 1.5 Hz, 1H), 7.66 (dt, J = 7.9, 1.5 Hz, 1H), 7.59 (dt, J = 7.7, 1.5 Hz, 1H), 7.38 (apr t, J = 7.8 Hz, 1H), 6.82 (s, 1H), 6.76 (s, 1H), 5.10 - 4.98 (m, 2H), 2.70 (q, J = 7.6 Hz, 2H), 2.47 (s, 3H), 1.18 (t, J = 7.6 Hz, 3H).

### Intermediate KF

### 3-[2-Amino-5-(2,6-dichloro-4-pyridyl)thiazol-4-yl]benzonitrile

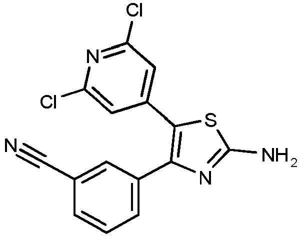

The title compound was prepared from 3-(2-amino-5-bromo-thiazol-4-yl)benzonitrile (Intermediate B) and 2,6-dichloro-4-(4,4,5,5-tetramethyl-1,3,2-dioxaborolan-2-yl)pyridine analogously to Intermediate C.
LC-MS (Method 5B): Rt 3.10 mins; MS m/z 346.9 = [M+H]+
1H NMR (500 MHz, DMSO-d6) δ 7.91 (s, 1H), 7.87 (d, J = 7.8 Hz, 1H), 7.77 (s, 2H), 7.74 (d, J = 7.8 Hz, 1H), 7.59 (t, J = 7.8 Hz, 1H), 7.15 (s, 2H).

### Intermediate KG

### N-[4-[2-Amino-4-(3-cyanophenyl)thiazol-5-yl]-6-methyl-2-pyridyl]acetamide

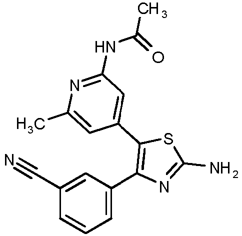

### Step 1: N-(4-Bromo-6-methyl-2-pyridyl)acetamide

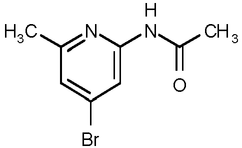

Triethylamine (0.45 mL, 3.23 mmol) and acetic anhydride (0.75 mL, 7.94 mmol) were added to a stirred solution of 4-bromo-6-methyl-pyridin-2-amine (550 mg, 2.94 mmol) in THF (20 mL) and heated to 50 °C for 22 h followed by heating at reflux for 2 h. The resulting mixture was allowed to cool to room temperature and was partitioned between EtOAc (100 mL) and sat. NaHCO₃ solution (100 mL). The layers were separated and the aqueous layer further extracted with EtOAc (2 x 100 mL). The combined organic extracts were dried over MgSO₄ and concentrated *in vacuo* to give an off-white solid. The solid was dry loaded onto silica and purification by chromatography on silica eluting with a gradient of 0 to 8% MeOH afforded the title compound as a white solid.
LC-MS (Method 5A): Rt 2.28 mins; MS m/z 228.9 / 230.9 = [M+H]+
1H NMR (500 MHz, DMSO-d6) δ 10.66 (s, 1H), 8.13 (d, J = 1.6 Hz, 1H), 7.25 (d, J = 1.6 Hz, 1H), 2.39 (s, 3H), 2.07 (s, 3H).

### Step 2: N-[6-Methyl-4-(4,4,5,5-tetramethyl-1,3,2-dioxaborolan-2-yl)-2-pyridyl]acetamide

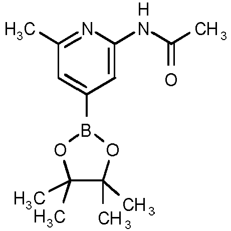

The title compound was prepared from N-(4-bromo-6-methyl-2-pyridyl)acetamide (step 1) and bis(pinacolato)diboron analogously to Intermediate KB step 2. 1H NMR (500 MHz, Chloroform-d) δ 9.12 (s, 1H), 8.39 (s, 1H), 7.27 (s, 1H), 2.49 (s, 3H), 2.21 (s, 3H), 1.32 (s, 12H).

### Step 3: N-[4-[2-Amino-4-(3-cyanophenyl)thiazol-5-yl]-6-methyl-2-pyridyl]acetamide

The title compound was prepared from 3-(2-amino-5-bromo-thiazol-4-yl)benzonitrile (Intermediate B) and N-[6-methyl-4-(4,4,5,5-tetramethyl-1,3,2-dioxaborolan-2-yl)-2-pyridyl]acetamide (step 2) analogously to Intermediate C.
LC-MS (Method 5B): Rt 2.29 mins; MS m/z 350.1 = [M+H]+
1H NMR (500 MHz, DMSO-d6) δ 10.41 (s, 1H), 7.81 (d, J = 1.7 Hz, 1H), 7.80 - 7.74 (m, 2H), 7.69 (ddd, J = 7.8, 1.7, 1.5 Hz, 1H), 7.52 (t, J = 7.8 Hz, 1H), 7.45 (s, 2H), 6.69 (d, J = 1.5 Hz, 1H), 2.29 (s, 3H), 2.01 (s, 3H).

### Intermediate KH

### N-[[4-[2-Amino-4-(3-cyanophenyl)thiazol-5-yl]-6-methyl-2-pyridyl]methyl]acetamide

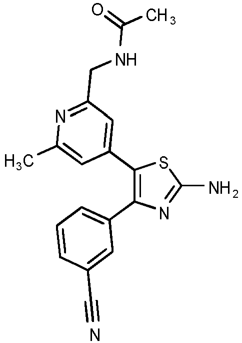

### Step 1: N-[(4-Bromo-6-methyl-2-pyridyl)methyl]acetamide

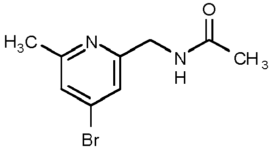

Triethylamine (0.52 mL, 4.08 mmol) and acetic anhydride (0.46 mL, 4.89 mmol) were added to a stirred solution of (4-bromo-6-methyl-2-pyridyl)methanamine (410 mg, 2.04 mmol) in DCM (15 mL) at 0 °C. The solution was allowed to warm to room temperature and stirred overnight. The resulting mixture was concentrated *in vacuo* to give an orange residue, which was diluted with water (10 mL). The solution was partitioned between sat. NaHCO₃ solution (20 mL) and EtOAc (20 mL) and the layers separated. The aqueous layer was extracted with EtOAc (2 x 20 mL) and the combined organic extracts were dried over Na₂SO₄ and concentrated *in vacuo.* Purification by chromatography on silica eluting with a gradient of 0 to 60% EtOAc in DCM afforded the title compound as a white solid.
LC-MS (Method 3B): Rt 1.28 mins; MS m/z 245.0 = [M+H]+
1H NMR (500 MHz, DMSO-d6) δ 8.44 (t, J = 6.1 Hz, 1H), 7.44 (d, J = 1.7 Hz, 1H), 7.28 (d, J = 1.7 Hz, 1H), 4.28 (d, J = 6.0 Hz, 2H), 2.44 (s, 3H), 1.91 (s, 3H).

### Step 2: N-[[6-Methyl-4-(4,4,5,5-tetramethyl-1,3,2-dioxaborolan-2-yl)-2-pyridyl]methyl]acetamide

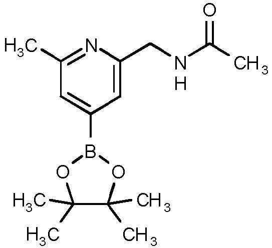

The title compound was prepared from N-[(4-bromo-6-methyl-2-pyridyl)methyl]acetamide (step 1) and bis(pinacolato)diboron analogously to Intermediate KB step 2.
1H NMR (500 MHz, DMSO-d6) δ 8.44 (t, J = 6.0 Hz, 1H), 7.32 (s, 1H), 7.27 (s, 1H), 4.30 (d, J = 6.0 Hz, 2H), 2.46 (s, 3H), 1.89 (s, 3H), 1.30 (s, 12H).

### Step 3: N-[[4-[2-Amino-4-(3-cyanophenyl)thiazol-5-yl]-6-methyl-2-pyridyl]methyl]acetamide

The title compound was prepared from 3-(2-amino-5-bromo-thiazol-4-yl)benzonitrile (Intermediate B) and N-[[6-methyl-4-(4,4,5,5-tetramethyl-1,3,2-dioxaborolan-2-yl)-2-pyridyl]methyl]acetamide (step 2) analogously to Intermediate C.
LC-MS (Method 2B): Rt 1.07 mins; MS m/z 364.0 = [M+H]+
1H NMR (500 MHz, DMSO-d6) δ 8.26 (t, J = 6.0 Hz, 1H), 7.84 - 7.77 (m, 2H), 7.65 (dt, J = 8.0, 1.5 Hz, 1H), 7.53 (d, J = 7.8 Hz, 1H), 7.48 (s, 2H), 6.90 (d, J = 1.5 Hz, 1H), 6.75 (d, J = 1.6 Hz, 1H), 4.17 (d, J = 6.0 Hz, 2H), 2.37 (s, 3H), 1.75 (s, 3H).

### Intermediate KI

### 3-[2-Amino-5-[2-(methoxymethyl)-6-methyl-4-pyridyl]thiazol-4-yl]benzonitrile

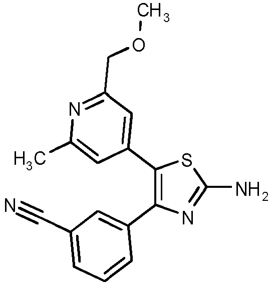

### Step 1: 4-Bromo-2-(methoxymethyl)-6-methyl-pyridine

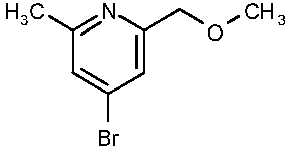

Mel (0.18 mL, 2.97 mmol) was added dropwise to a stirred solution of (4-bromo-6-methyl-2-pyridyl)methanol (300 mg, 1.48 mmol) and KOH (167 mg, 2.97 mmol) and the mixture was stirred at room temperature for 16 h. Further Mel (0.18 mL, 2.97 mmol) was added dropwise and the mixture was stirred at room temperature for 3 h. The resulting mixture was diluted in EtOAc (20 mL), washed with brine (3 x 20 mL), dried over Na₂SO₄ and concentrated *in vacuo* to afford the title compound as a brown oil.
LC-MS (Method 3B): Rt 1.62 mins; MS m/z 218.0 = [M+H]+
1H NMR (500 MHz, DMSO-d6) δ 7.48 (d, J = 1.8 Hz, 1H), 7.40 (d, J = 1.7 Hz, 1H), 4.45 (s, 2H), 3.36 (s, 3H), 2.45 (s, 3H).

### Step 2: 2-(Methoxymethyl)-6-methyl-4-(4,4,5,5-tetramethyl-1 ,3,2-dioxaborolan-2-yl)pyridine

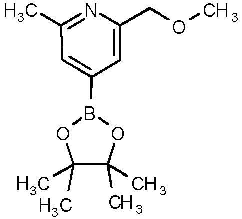

The title compound was prepared from 4-bromo-2-(methoxymethyl)-6-methylpyridine (step 1) and bis(pinacolato)diboron analogously to Intermediate KB step 2.
1H NMR (500 MHz, DMSO-d6) δ 7.41 (s, 1H), 7.34 (s, 1H), 4.45 (d, J = 4.3 Hz, 2H), 3.36 (s, 3H), 2.45 (d, J = 6.7 Hz, 3H), 1.31 (s, 12H).

### Step 3: 3-[2-Amino-5-[2-(methoxymethyl)-6-methyl-4-pyridyl]thiazol-4-yl]benzonitrile

The title compound was prepared from 3-(2-amino-5-bromo-thiazol-4-yl)benzonitrile (Intermediate B) and 2-(methoxymethyl)-6-methyl-4-(4,4,5,5-tetramethyl-1,3,2-dioxaborolan-2-yl)pyridine (step 2) analogously to Intermediate C.
LC-MS (Method 3B): Rt 1.50 mins; MS m/z 337.5 = [M+H]+
1H NMR (500 MHz, DMSO-d6) δ 7.83 - 7.78 (m, 2H), 7.70 (dt, J = 7.9, 1.5 Hz, 1H), 7.55 (t, J = 7.8 Hz, 1H), 7.49 (s, 2H), 6.94 (d, J = 1.7 Hz, 1H), 6.88 (d, J = 1.7 Hz, 1H), 4.35 (s, 2H), 3.21 (s, 3H), 2.37 (s, 3H).

### Intermediate L

### 3-[2-Amino-5-[2-methyl-6-(oxetan-3-yl)-4-pyridyl]thiazol-4-yl]benzonitrile

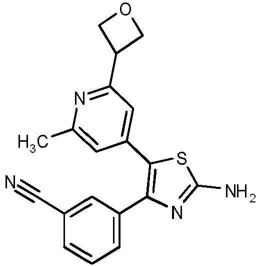

The title compound was prepared from 3-(2-amino-5-bromo-thiazol-4-yl)benzonitrile (Intermediate B) and 2-methyl-6-(oxetan-3-yl)-4-(4,4,5,5-tetramethyl-1,3,2-dioxaborolan-2-yl)pyridine analogously to Intermediate C.
LC-MS (Method 2B): Rt 1.22 mins; MS m/z 349.0 = [M+H]+
1H NMR (500 MHz, DMSO-d6) δ 7.83 - 7.81 (m, 1H), 7.80 (dt, J = 7.6, 1.4 Hz, 1H), 7.68 (dt, J = 8.0, 1.4 Hz, 1H), 7.54 (apr t, J = 7.8 Hz, 1H), 7.48 (s, 2H), 6.92 (d, J = 1.6 Hz, 1H), 6.84 (d, J = 1.6 Hz, 1H), 4.78 (dd, J = 8.5, 5.6 Hz, 2H), 4.62 (dd, J = 6.8, 5.6 Hz, 2H), 4.22 (tt, J = 8.5, 6.8 Hz, 1H), 2.40 (s, 3H).

### Intermediate M

### 3-[2-Amino-5-(3-fluoro-2,6-dimethyl-4-pyridyl)thiazol-4-yl]benzonitrile

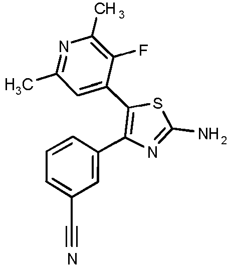

A mixture of 4-bromo-3-fluoro-2,6-dimethyl-pyridine (100 mg, 0.36 mmol), bis(pinacolato)diboron (181 mg, 0.71 mmol) and AcOK (193 mg, 1.96 mmol) in 1,4-dioxane (6 mL) was degassed under a flow of nitrogen. Pd(dppf)Cl₂ (47 mg, 0.06 mmol) was added and the reaction was heated to 100 °C overnight. The resulting mixture was cooled to room temperature, filtered and washed through with DCM (2 x 5 mL). The filtrate was concentrated *in vacuo* and the boronate was taken into 1,4-dioxane (2.5 mL) and water (0.5 mL). 3-(2-Amino-5-bromo-thiazol-4-yl)benzonitrile (Intermediate B) (100 mg, 0.36 mmol) and K₂CO₃ (197 mg, 1.43mmol) were added and the mixture was degassed under flow of nitrogen. Bis(tri-tert-butylphosphine)palladium(0) (18 mg, 0.04 mmol) was added and the reaction mixture was heated to 80 °C for 45 mins. After cooling to room temperature, the resulting mixture was concentrated *in vacuo* and purification by chromatography on silica eluting with a gradient of 0 to 3% MeOH in DCM afforded the title compound as a brown solid.
LC-MS (Method 3B): Rt 1.55 mins; MS m/z 325.0 = [M+H]+
1H NMR (500 MHz, DMSO-d6) δ 7.80 (s, 1H), 7.77 (d, J = 7.7 Hz, 1H), 7.62 (d, J = 8.0 Hz, 1H), 7.56 - 7.47 (m, 3H), 6.99 (d, J = 5.0 Hz, 1H), 2.39 - 2.30 (m, 6H).

### Intermediate N (

### 3-[2-Amino-5-[2,6-bis(trideuteriomethyl)-4-pyridyl]thiazol-4-yl]benzonitrile

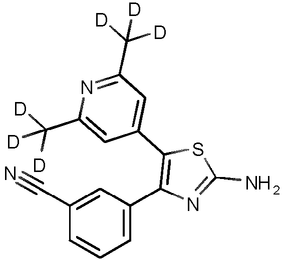

### Step 1: 2,6-Bis(trideuteriomethyl)pyridine

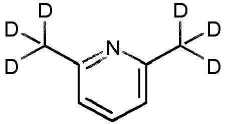

2,6-Dichloropyridine (5.0 g, 33.79 mmol) and NiCl₂(dppp) (916 mg, 1.69 mmol) were placed under nitrogen and anhydrous THF (68 mL) was added via syringe. The mixture was cooled to 0 °C and treated dropwise with a solution of methyl-d3-magnesium iodide (1M in Et₂O) (100 mL, 100 mmol) over 20 mins. The reaction mixture was heated at reflux for 1.5 h and allowed to cool to room temperature. The reaction was quenched by cautious addition of MeOH (15 mL) followed by 1M aqueous HCl (125 mL). The resulting bi-phasic mixture was stirred vigorously for 5 mins until all of the solids had dissolved and effervescence had fully ceased. The mixture was diluted with Et₂O (125 mL) and the layers separated. The organic layer was extracted with water (125 mL) and the combined aqueous layers adjusted to pH 10 with 10% aqueous NaOH before the mixture was extracted with EtOAc (2 x 300 mL) and the combined organic extracts were washed with brine (200 mL), dried over Na₂SO₄, and cautiously concentrated at 35-38 °C and 170-190 mbar then briefly at 40 °C and 100 mbar to afford the crude material as a pale orange-brown oil. The oil was purified by vacuum distillation (120-140 °C under low vacuum) to afford the title compound as a clear, colourless oil.
LC-MS (Method 2B): Rt 1.05 mins; MS m/z 114.0 = [M+H]+
1H NMR (500 MHz, DMSO-d6) δ 7.54 (t, J = 7.6 Hz, 1H), 7.02 (d, J = 7.6 Hz, 2H)

### Step 2: 4-(4,4,5,5-Tetramethyl-1,3,2-dioxaborolan-2-yl)-2,6-bis(trideuteriomethyl)pyridine

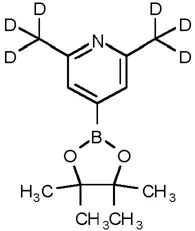

Methoxy(cyclooctadiene)iridium(I) dimer (789 mg, 1.19 mmol), 4,4'-di-*tert-*butyl-2,2'-dipyridyl (639 mg, 2.38 mmol) and bis(pinacolato)diboron (10.07 g, 39.67 mmol) were placed in a sealed flask and the contents evacuated and backfilled with nitrogen (3 x cycles). Freshly de-oxygenated anhydrous cyclohexane (50 mL) was added via syringe and the resulting mixture briefly sonicated, causing the bulk of the solids to dissolve and a deep red-brown solution to form. The mixture was stirred vigorously for 1 h at room temperature and treated with a solution of 2,6-bis(trideuteriomethyl)pyridine (step 1) (4.49 g, 39.67 mmol) in de-oxygenated anhydrous cyclohexane (50 mL) added via syringe under nitrogen. The resulting mixture was stirred at room temperature for 19.5 h and then additional bis(pinacolato)diboron (2.01 g, 7.93 mmol) in de-oxygenated anhydrous cyclohexane (10mL) was added via syringe under nitrogen. The mixture was stirred at room temperature for 2 h before additional methoxy(cyclooctadiene)iridium(I) dimer (263 mg, 0.40 mmol) and 4,4'-di-tert-butyl-2,2'-dipyridyl (213 mg, 0.79 mmol) were added. Stirring was continued under nitrogen at room temperature for a further 17.5 h after which time the mixture was concentrated *in vacuo* to afford the title compound as a dark brown solid.
LC-MS (Method 2B): Rt 0.92 mins; MS m/z 240.1 = [M+H]+
1H NMR (500 MHz, DMSO-d6) δ 7.22 (s, 2H), 1.29 (s, 12H).

### Step 3: 3-[2-Amino-5-[2,6-bis(trideuteriomethyl)-4-pyridyl]thiazol-4-yl]benzonitrile

A solution of K₂CO₃ (10.74 g, 77.68 mmol) in water (60 mL) was added to a solution of 3-(2-amino-5-bromo-thiazol-4-yl)benzonitrile (Intermediate B) (8.7 g, 31.07 mmol) and 4-(4,4,5,5-tetramethyl-1,3,2-dioxaborolan-2-yl)-2,6-bis(trideuteriomethyl)pyridine (step 2) (8.92 g, 37.29 mmol) in 1,4-dioxane (240 mL) and the reaction mixture was de-oxygenated via nitrogen sparging for 10 mins. Pd(tBu₃P)₂ (1.59 g, 3.11 mmol) was added, the flask sealed and the contents evacuated and backfilled with nitrogen (3 x cycles). The reaction mixture was stirred at 50 °C for 1 h and then allowed to cool to room temperature. The resulting mixture was diluted with EtOAc (500 mL) and washed with water (250 mL), brine (250 mL), dried over Na₂SO₄ and concentrated *in vacuo.* The crude material was re-dissolved in ~ 9 : 1 chloroform / MeOH (250 mL) and passed through a short pre-wetted Celite^{®} (filter material) plug, eluting with chloroform. The filtrate was concentrated *in vacuo* to afford a dark brown residue which was triturated in a 5:2 mixture of Et₂O / DCM (70 mL) under sonication. The resulting solids were filtered, washed with DCM (5 x 10 mL) and vacuum dried to afford the title compound as a cream solid.

LC-MS (Method 2B): Rt 1.29 mins; MS m/z 313.1 = [M+H]+

1H NMR (500 MHz, DMSO-d6) δ 7.81 (br t, J = 1.5 Hz, 1H), 7.79 (br dt, J = 7.6, 1.5 Hz, 1H), 7.66 (br dt, J = 7.9, 1.5 Hz, 1H), 7.52 (apr t, J = 7.8 Hz, 1H), 7.48 - 7.39 (m, 2H), 6.80 (s, 2H).

### Intermediate O

### 3-[2-Amino-5-(2,6-dimethyl-4-pyridyl)thiazol-4-yl]-2-methyl-benzonitrile

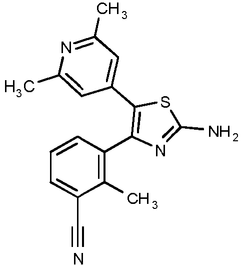

### Step 1: 3-(2-Aminothiazol-4-yl)-2-methyl-benzonitrile

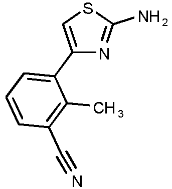

Bis(tri-tert-butylphosphine)palladium(0) (29 mg, 0.06 mmol) was added to a degassed solution of 4-bromothiazol-2-amine (100 mg, 0.56 mmol), (3-cyano-2-methylphenyl)boronic acid (180 mg, 1.12 mmol) and potassium carbonate (154 mg, 1.12 mmol) in 1,4-dioxane (4 mL) and water (0.8 mL). The flask was evacuated and backfilled with nitrogen (x 3) and the reaction mixture was stirred at 80 °C overnight. The resulting mixture was allowed to cool to room temperature and was partitioned between EtOAc and 70% brine. The organic layer was separated, washed with 70% brine, dried over Na₂SO₄ and concentrated *in vacuo.* Purification by chromatography on silica eluting with a gradient of 2 to 5% MeOH in DCM afforded a brown solid which was triturated with Et₂O (5 mL) to afford the title compound as a brown solid.
LC-MS (Method 8B): Rt 3.48 mins; MS m/z 216.1 = [M+H]+
1H NMR (500 MHz, DMSO-d6) δ 7.81 (d, J = 7.8 Hz, 1H), 7.74 (d, J = 7.6 Hz, 1H), 7.41 (t, J = 7.7 Hz, 1H), 7.11 (s, 2H), 6.76 (s, 1H), 2.60 (s, 3H).

### Step 2: 3-(2-Amino-5-bromo-thiazol-4-yl)-2-methyl-benzonitrile

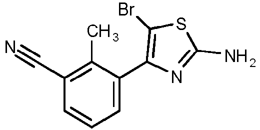

NBS (52 mg, 0.29 mmol) was added to a stirred solution of 3-(2-aminothiazol-4-yl)-2-methyl-benzonitrile (step 1) (53 mg, 0.24 mmol) in THF (1 mL) and stirred at room temperature for 5 h. The resulting solution was diluted with EtOAc (10 mL) and washed with water (10 mL), sat. NaHCO₃ solution (10 mL), brine (10 mL), dried over Na₂SO₄ and concentrated *in vacuo* to afford the title compound as a brown solid.
LC-MS (Method 8B): Rt 4.00 mins; MS m/z 294.0 / 296.0 = [M+H]+
1H NMR (500 MHz, DMSO-d6) δ 7.90 (dd, J = 7.7, 1.3 Hz, 1H), 7.66 (d, J = 7.7 Hz, 1H), 7.51 (t, J = 7.7 Hz, 1H), 7.44 (s, 2H), 2.45 (s, 3H).

### Step 3: 3-[2-Amino-5-(2,6-dimethyl-4-pyridyl)thiazol-4-yl]-2-methyl-benzonitrile

The title compound was prepared from 3-(2-amino-5-bromo-thiazol-4-yl)-2-methyl-benzonitrile (step 2) and 2,6-dimethyl-4-(4,4,5,5-tetramethyl-1,3,2-dioxaborolan-2-yl)pyridine analogously to Intermediate C.

LC-MS (Method 8B): Rt 3.71 mins; MS m/z 321.1 = [M+H]+
1H NMR (500 MHz, DMSO-d6) δ 7.92 (d, J = 7.5 Hz, 1H), 7.58 (s, 2H), 7.57 - 7.50 (m, 1H), 7.48 (t, J = 7.7 Hz, 1H), 6.55 (s, 2H), 2.34 (s, 3H), 2.29 (s, 6H).

### Intermediate P

### 3-[2-Amino-5-[2-(1-methoxyethyl)-6-methyl-4-pyridyl]thiazol-4-yl]benzonitrile

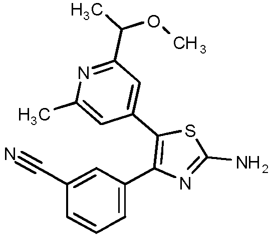

### Step 1: 4-Bromo-6-methyl-pyridine-2-carbaldehyde

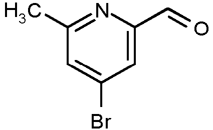

Manganese dioxide (1.72 g, 19.8 mmol) was added to a solution of (4-bromo-6-methyl-2-pyridyl)methanol (1.0 g, 4.95 mmol) in chloroform (25 mL) and the mixture stirred at reflux for 16 h. Additional manganese dioxide (215 mg, 2.47 mmol) was added and stirring continued at reflux for 5 h. The resulting mixture was cooled to room temperature and filtered through a plug of Celite^{®} (filter material) eluting with DCM. The filtrate was concentrated *in vacuo* to afford the title compound as a white solid.
LC-MS (Method 3B): Rt 1.45 mins; MS m/z 199.9 / 201.9 = [M+H]+
1H NMR (500 MHz, Chloroform-d) δ 9.99 (s, 1H), 7.93 - 7.91 (m, 1H), 7.56 (dd, J = 1.7, 0.6 Hz, 1H), 2.66 - 2.62 (m, 3H).

### Step 2: 1-(4-Bromo-6-methyl-2-pyridyl)ethanol

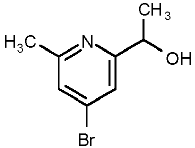

A solution of 4-bromo-6-methyl-pyridine-2-carbaldehyde (step 1) (750 mg, 3.75 mmol) in anhydrous THF (18 mL) was added dropwise over 5 mins to a stirred solution of methylmagnesium bromide (3.4M in 2-Me-THF) (2.21 mL, 7.5 mmol) at 0 °C under nitrogen and the mixture stirred for 5 mins. The ice bath was removed and the mixture stirred at room temperature for 1 h. The resulting mixture was re-cooled to ~ 5 °C and the reaction was quenched by addition of 1M HCl (7.5 mL). The mixture was diluted with water (25 mL) and EtOAc (25 mL) and the pH of the aqueous layer was adjusted to pH 8 by addition of sat. NaHCO₃ solution before the layers were shaken and separated. The organic layer was washed with additional sat. NaHCO₃ solution (20 mL) and brine (20 mL) before being dried over Na₂SO₄ and concentrated *in vacuo* to afford the title compound as a clear yellow oil.
LC-MS (Method 3B): Rt 1.40 mins; MS m/z 216.2 / 218.0 = [M+H]+
1H NMR (500 MHz, DMSO-d6) δ 7.48 (d, J = 1.8 Hz, 1H), 7.41 (d, J = 1.8 Hz, 1H), 5.50 - 5.42 (m, 1H), 4.65 (qd, J = 6.6, 4.8 Hz, 1H), 2.43 (s, 3H), 1.33 (d, J = 6.6 Hz, 3H).

### Step 3: 4-Bromo-2-(1-methoxyethyl)-6-methyl-pyridine

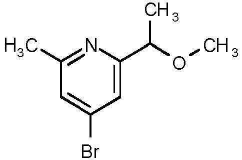

NaH (60% in mineral oil) (74 mg, 1.85 mmol) was added to a stirred solution of 1-(4-bromo-6-methyl-2-pyridyl)ethanol (200 mg, 0.93 mmol) in anhydrous DMF (4 mL) under nitrogen and the resulting solution stirred at room temperature for 10 mins before iodomethane (173 uL, 2.78 mmol) was added. The resulting mixture was stirred at room temperature for 1 h before being carefully quenched by addition of water (1 mL), diluted with EtOAc (20 mL) and washed with 90% brine (25 mL) and 50% brine (3 x 25 mL). The organic layer was dried over Na₂SO4, filtered and concentrated *in vacuo.* Purification by column chromatography on silica eluting with 2% MeOH in DCM afforded the title compound as a yellow oil.LC-MS (Method 3B): Rt 1.75 mins; MS m/z 230.0 / 232.0 = [M+H]+
1H NMR (500 MHz, Chloroform-d) δ 7.41 (s, 1H), 7.23 (d, J = 1.8 Hz, 1H), 4.36 (q, J = 6.6 Hz, 1H), 3.32 (s, 3H), 2.52 (s, 3H), 1.43 (d, J = 6.6 Hz, 3H).

### Step 4: 2-(1-Methoxyethyl)-6-methyl-4-(4,4,5,5-tetramethyl-1,3,2-dioxaborolan-2-yl)pyridine

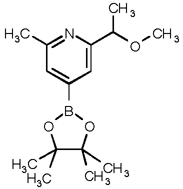

The title compound was prepared from 4-bromo-2-(1-methoxyethyl)-6-methylpyridine (step 3) and bis(pinacolato)diboron analogously to Intermediate J step 3.
LC-MS (Method 3B): Rt 0.93 mins; MS m/z 278.1 = [M+H]+ .

### Step 5: 3-[2-Amino-5-[2-(1-methoxyethyl)-6-methyl-4-pyridyl]thiazol-4-yl]benzonitrile

The title compound was prepared from 2-(1-methoxyethyl)-6-methyl-4-(4,4,5,5-tetramethyl-1,3,2-dioxaborolan-2-yl)pyridine (step 4) and 3-(2-amino-5-bromo-thiazol-4-yl)benzonitrile (Intermediate B) analogously to Intermediate J step 4.
LC-MS (Method 3B): Rt 1.57 mins; MS m/z 349.2 = [M-H]-
1H NMR (500 MHz, DMSO-d6) δ 7.80 (dt, J = 7.8, 1.5 Hz, 1H), 7.78 (t, J = 1.5Hz, 1H), 7.73 (dt, J = 7.8, 1.5 Hz, 1H), 7.56 (t, J = 7.8 Hz, 1H), 7.49 (s, 2H), 7.00 (d, J = 1.6 Hz, 1H), 6.78 (d, J = 1.6 Hz, 1H), 4.18 (q, J = 6.6 Hz, 1H), 3.02 (s, 3H), 2.41 (s, 3H), 1.19 (d, J = 6.6 Hz, 3H).

### Intermediate PA

### 3-[2-Amino-5-[2-[1-[(4-methoxyphenyl)methoxy]ethyl]-6-methyl-4-pyridyl]thiazol-4-yl]benzonitrile

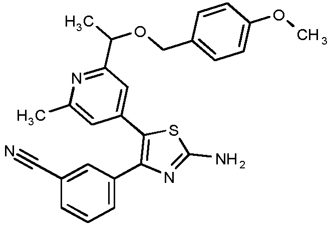

### Step 1: 4-Bromo-2-[1-[(4-methoxyphenyl)methoxy]ethyl]-6-methyl-pyridine

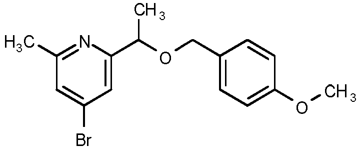

The title compound was prepared from 1-(4-bromo-6-methyl-2-pyridyl)ethanol (Intermediate P step 2) and 4-methoxybenzyl bromide analogously to Intermediate J step 2. LC-MS (Method 3B): Rt 2.23 mins; MS m/z 336.0 / 338.0 = [M+H]+
1H NMR (500 MHz, CDCl3) δ 7.49 (s, 1H), 7.27 - 7.23 (m, 3H), 6.90 - 6.86 (m, 2H), 4.61 - 4.55 (m, 1H), 4.44 (d, J = 11.3 Hz, 1H), 4.38 (d, J = 11.3 Hz, 1H), 3.81 (s, 3H), 2.53 (s, 3H), 1.46 (d, J = 6.6 Hz, 3H).

### Step 2: 2-[1-[(4-Methoxyphenyl)methoxy]ethyl]-6-methyl-4-(4,4,5,5-tetramethyl-1,3,2-dioxaborolan-2-yl)pyridine

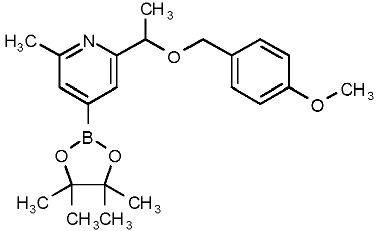

The title compound was prepared from 4-bromo-2-[1-[(4-methoxyphenyl)methoxy]ethyl]-6-methyl-pyridine (step 1) and bis(pinacolato)diboron analogously to Intermediate J step 3.
LC-MS (Method 3B): Rt 1.00 mins; MS m/z 302.1 = [M+H]+ [NB - mass ion for the parent boronic acid].

### Step 3: 3-[2-Amino-5-[2-[1-[(4-methoxyphenyl)methoxy]ethyl]-6-methyl-4-pyridyl]thiazol-4-yl]benzonitrile

The title compound was prepared from 2-[1-[(4-methoxyphenyl)methoxy]ethyl]-6-methyl-4-(4,4,5,5-tetramethyl-1,3,2-dioxaborolan-2-yl)pyridine (step 2) and 3-(2-amino-5-bromo-thiazol-4-yl)benzonitrile (Intermediate B) analogously to Intermediate J step 4. LC-MS (Method 3B): Rt 1.91 mins; MS m/z 455.3 = [M-H]-
1H NMR (500 MHz, DMSO-d6) δ 7.80 (t, J = 1.7 Hz, 1H), 7.72 - 7.70 (m, 1H), 7.72 - 7.67 (m, 1H), 7.53 - 7.47 (m, 3H), 7.05 - 7.02 (m, 2H), 7.01 (d, J = 1.6 Hz, 1H), 6.89 (d, J = 1.6 Hz, 1H), 6.88 - 6.85 (m, 2H), 4.39 (q, J = 6.5 Hz, 1H), 4.19 (d, J = 11.6 Hz, 1H), 4.15 (d, J = 11.6 Hz, 1H), 3.75 (s, 3H), 2.42 (s, 3H), 1.25 (d, J = 6.5 Hz, 3H).

### Intermediate Q

### 3-[2-Amino-5-[2-chloro-6-(1-hydroxy-1-methyl-ethyl)-4-pyridyl]thiazol-4-yl]benzonitrile

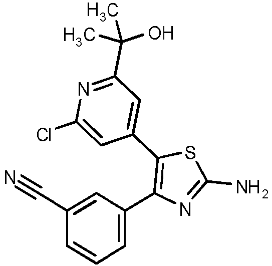

### Step 1: 2-(6-Chloro-2-pyridyl)propan-2-ol

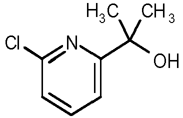

The title compound was prepared from methyl 6-chloropyridine-2-carboxylate and methylmagnesium bromide analogously to Intermediate J step 1.
LC-MS (Method 3B): Rt 1.42 mins; MS m/z 172.0 / 174.0 = [M+H]+
1H NMR (500 MHz, DMSO-d6) δ 7.83 (t, J = 7.8 Hz, 1H), 7.64 (dd, J = 7.8, 0.8 Hz, 1H), 7.33 (dd, J = 7.8, 0.8 Hz, 1H), 5.32 (s, 1H), 1.41 (s, 6H).

### Step 2: 2-[6-Chloro-4-(4,4,5,5-tetramethyl-1,3,2-dioxaborolan-2-yl)-2-pyridyl]propan-2-ol

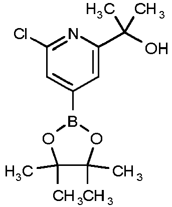

The title compound was prepared from 2-(6-chloro-2-pyridyl)propan-2-ol (step 1) and bis(pinacolato)diboron analogously to Intermediate N step 2.
LC-MS (Method 3B): Rt 1.03 mins; MS m/z 298.2 / 300.2= [M+H]+

### Step 3: 3-[2-Amino-5-[2-chloro-6-(1-hydroxy-1-methyl-ethyl)-4-pyridyl]thiazol-4-yl]benzonitrile

The title compound was prepared from 2-[6-chloro-4-(4,4,5,5-tetramethyl-1,3,2-dioxaborolan-2-yl)-2-pyridyl]propan-2-ol (step 2) and 3-(2-amino-5-bromo-thiazol-4-yl)benzonitrile (Intermediate B) analogously to Intermediate J step 4.

LC-MS (Method 3B): Rt 1.67 mins; MS m/z 371.1 / 373.1 = [M+H]+
1H NMR (500 MHz, DMSO-d6) δ 7.85 - 7.81 (m, 2H), 7.73 (dt, J = 8.0, 1.5 Hz, 1H), 7.64 - 7.61 (m, 2H), 7.60 - 7.56 (m, 1H), 7.32 (d, J = 1.4 Hz, 1H), 6.99 (d, J = 1.4 Hz, 1H), 5.19 (s, 1H), 1.33 (s, 6H).

### Intermediate R

### 3-[2-Amino-5-(2-methyl-6-morpholino-4-pyridyl)thiazol-4-yl]benzonitrile

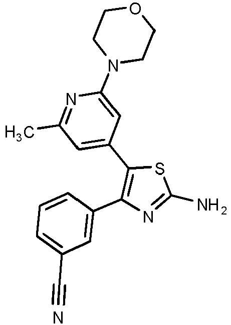

### Step 1: (4-Bromo-6-methyl-2-pyridyl) trifluoromethanesulfonate N121-87-1 hbarlow

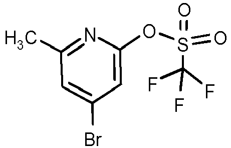

To a cooled (0 °C) suspension of 4-bromo-6-methyl-pyridin-2-ol (500 mg, 2.66 mmol) in DCM (13.5 mL) was added triethylamine (0.44 mL, 3.19 mmol) followed by dropwise addition of trifluoromethylsulfonyl trifluoromethanesulfonate (0.54 mL, 3.19 mmol) and the mixture was stirred at 0 °C for 10 mins. Additional trifluoromethylsulfonyl trifluoromethanesulfonate (0.26 mL, 1.6 mmol) and triethylamine (0.11 mL, 0.80 mmol) were added and the mixture was stirred for 10 mins at 0 °C. The resulting mixture was added to sat.NaHCO₃ solution (50 mL) and extracted with DCM (2 x 50 mL). The combined organic extracts were dried over MgSO₄ and concentrated *in vacuo.* Purification by chromatography on silica eluting with a gradient of 0 to 15% EtOAc in petroleum ether afforded the title compound as a colourless oil.
LC-MS (Method 3B): Rt 2.08 mins; MS m/z 317.9 / 319.9 = [M-H]-
1H NMR (500 MHz, DMSO-d6) δ 7.84 (s, 1H), 7.83 (s, 1H), 2.48 (s, 3H).

### Step 2: 4-(4-Bromo-6-methyl-2-pyridyl)morpholine

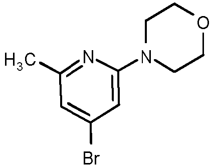

A mixture of (4-bromo-6-methyl-2-pyridyl) trifluoromethanesulfonate (step 1) (150 mg, 0.47 mmol) and morpholine (82 µL, 0.94 mmol) in DMSO (3 mL) was stirred at 80 °C for 30 mins. The reaction mixture was allowed to cool to room temperature and was diluted with EtOAc (20 mL) and water (20 mL). The layers were separated and the aqueous layer was further extracted with EtOAc (2 x 20 mL). The combined organic extracts were washed with brine (3 x 20 mL), dried over MgSO₄ and concentrated *in vacuo.* Purification by chromatography on silica eluting with a gradient of 0 to 15% EtOAc in petroleum ether afforded the title compound as a colourless oil.
LC-MS (Method 3A): Rt 1.47 mins; MS m/z 258.0 = [M+H]+
1H NMR (500 MHz, DMSO-d6) δ 6.83 (s, 1H), 6.76 (d, J = 1.0 Hz, 1H), 3.68 - 3.64 (m, 4H), 3.46 - 3.42 (m, 4H), 2.29 (s, 3H).

### Step 3: 4-[6-Methyl-4-(4,4,5,5-tetramethyl-1,3,2-dioxaborolan-2-yl)-2-pyridyl]morpholine

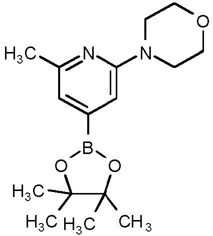

The title compound was prepared from 4-(4-bromo-6-methyl-2-pyridyl)morpholine (step 2) and bis(pinacolato)diboron analogously to Intermediate I step 2. 1H NMR (500 MHz, DMSO-d6) δ 6.75 (s, 1H), 6.74 (s, 1H), 3.69 - 3.65 (m, 4H), 3.42 - 3.38 (m, 4H), 2.31 (s, 3H), 1.28 (s, 12H).

### Step 4: 3-[2-Amino-5-(2-methyl-6-morpholino-4-pyridyl)thiazol-4-yl]benzonitrile

The title compound was prepared from 4-[6-methyl-4-(4,4,5,5-tetramethyl-1,3,2-dioxaborolan-2-yl)-2-pyridyl]morpholine (step 3) and 3-(2-amino-5-bromo-thiazol-4-yl)benzonitrile (Intermediate B) analogously to Intermediate I step 3.
LC-MS (Method 5B): Rt 2.84 mins; MS m/z 378.0 = [M+H]+
1H NMR (500 MHz, DMSO-d6) δ 7.82 (t, J = 1.5 Hz, 1H), 7.76 (dt, J = 7.8, 1.5 Hz, 1H), 7.70 (dt, J = 7.8, 1.5 Hz, 1H), 7.52 (t, J = 7.8 Hz, 1H), 7.37 (s, 2H), 6.34 (s, 1H), 6.32 (s, 1H), 3.65 - 3.58 (m, 4H), 3.43 - 3.36 (m, 4H), 2.23 (s, 3H).

### Intermediate S

### 1-Methylimino-1,4-thiazinane 1-oxide

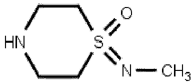

### Step 1 - tert-butyl 1-methylimino-1-oxo-1,4-thiazinane-4-carboxylate

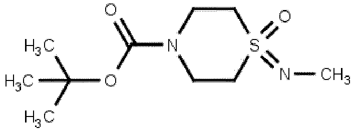

To a mixture of tert-butyl 1-imino-1-oxo-1,4-thiazinane-4-carboxylate (200 mg, 0.85 mmol) in DMF (5 mL) was added sodium hydride (60% in mineral oil) (68 mg, 1.71 mmol) followed by iodomethane (266 µL, 4.27 mmol) and the reaction mixture was stirred at room temperature for 1.5 h. Additional sodium hydride (60% in mineral oil) (68 mg, 1.71 mmol) followed by iodomethane (266 µL, 4.27 mmol) were added and the reaction mixture was stirred for a further 1.5 h. The mixture was then diluted with 9:1 EtOAc / MeOH (30 mL) and washed with 90% brine (4 x 30 mL), dried over MgSO₄, filtered and concentrated *in vacuo* to afford the title compound.
1H NMR (500 MHz, DMSO-d6) δ 3.86 (d, J = 14.7 Hz, 2H), 3.49 - 3.42 (m, 2H), 3.18 - 3.11 (m, 2H), 3.05 - 2.96 (m, 2H), 2.64 (s, 3H), 1.42 (s, 9H).

### Step 2 - 1-Methylimino-1,4-thiazinane 1-oxide

To a stirred solution of tert-butyl 1-methylimino-1-oxo-1,4-thiazinane-4-carboxylate (step 1) (167 mg, 0.67 mmol) in DCM (5 mL) was added 4 HCl in dioxane (3.36 mL, 13.45 mmol) dropwise. The reaction mixture was stirred at room temperature for 2h. The precipitated solid was collected by filtration, washing with DCM (2 x 2 mL) and concentrated *in vacuo* from MeOH to afford the title compound as a white solid.

### Intermediate T

### 2-[(2S)-Piperazin-2-yl]propan-2-ol dihydrochloride

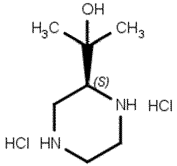

### Step 1: 1,4-Di-tert-butyl 2-methyl (2S)-piperazine-1,2,4-tricarboxylate

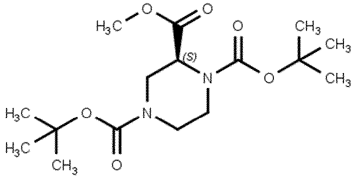

A mixture of di-tert-butyl dicarbonate (479 mg, 2.19 mmol), 1-tert-butyl 2-methyl (2S)-piperazine-1,2-dicarboxylate (487 mg, 1.99 mmol) and triethylamine (0.39 mL, 2.79 mmol) in DCM (15 mL) was stirred at room temperature for 18 h. The reaction mixture was diluted with DCM (40 mL) and washed with sat. NaHCO₃ solution (20 mL). The organic phase was dried over MgSO₄ and concentrated *in vacuo* to afford the title compound as a clear oil which solidified on standing.
1H NMR (500 MHz, DMSO-d6) δ 4.56 (d, J = 37.3 Hz, 1H), 4.28 (dd, J = 31.7, 13.8 Hz, 1H), 3.83 (s, 1H), 3.68 (s, 3H), 3.23 - 2.72 (m, 4H), 1.44 - 1.31 (m, 18H).

### Step 2: Di-tert-butyl (2S)-2-(1-hydroxy-1-methyl-ethyl)piperazine-1,4-dicarboxylate

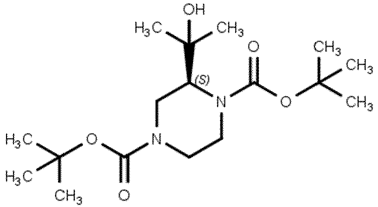

To a solution of 1,4-di-tert-butyl 2-methyl (2S)-piperazine-1,2,4-tricarboxylate (step 1)(712 mg, 2.07 mmol) in THF (20 mL) at 0°C was added MeMgCl (3 M in THF) (1.72 mL, 5.17 mmol) dropwise. The reaction was allowed to warm to room temperature and was stirred at this temperature for 3 h. The reaction was diluted with DCM (70 mL) and washed with sat. NH₄Cl solution (20 mL). The aq. layer was extracted with DCM (20 mL) and the combined organic extracts were dried over MgSO₄ and concentrated *in vacuo.* Purification by chromatography on silica eluting with a gradient of 1 to 2% MeOH in DCM afforded the title compound as a clear oil.
1H NMR (500 MHz, DMSO-d6) δ 4.39 (s, 1H), 4.03 - 3.43 (m, 4H), 3.17 - 3.04 (m, 2H), 3.01 - 2.82 (m, 1H), 1.39 (s, 18H), 1.17 - 1.01 (m, 6H)

### Step 3: 2-[(2S)-Piperazin-2-yl]propan-2-ol dihydrochloride

To a solution of di-tert-butyl (2S)-2-(1-hydroxy-1-methyl-ethyl)piperazine-1,4-dicarboxylate (step 2)(331 mg, 0.96 mmol) in MeOH (8 mL) was added 4N HCl in dioxane (2.4 mL, 9.60 mmol) and the reaction mixture was stirred at room temperature for 6 h before being concentrated *in vacuo* to afford the title compound as a white solid.
1H NMR (500 MHz, DMSO-d6) δ 9.67 (s, 2H), 9.41 (s, 1H), 9.07 (s, 1H), 5.57 (s, 1H), 3.62 - 3.53 (m, 1H), 3.49 - 3.38 (m, 3H), 3.29 - 3.21 (m, 2H), 2.98 (t, J = 12.8 Hz, 1H), 1.26 (s, 3H), 1.19 (s, 3H).

### Intermediate U

### (3R)-3-Methylpyrrolidine-3-carbonitrile hydroiodide

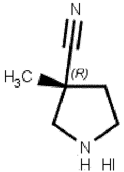

### Step 1 - benzyl (3R)-3-carbamoyl-3-methyl-pyrrolidine-1-carboxylate

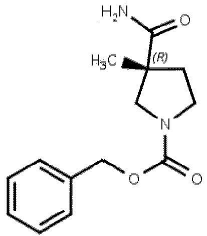

Anhydrous THF (19 mL) was added to a mixture of (3R)-1-benzyloxycarbonyl-3-methyl-pyrrolidine-3-carboxylic acid (1.0 g, 3.80 mmol) and CDI (678 mg, 4.18 mmol) under nitrogen and the reaction mixture stirred at room temperature for 1.5 hours. Ammonium hydroxide (35% in water) (8.64 mL, 75.96 mmol) was added and the mixture was stirred at room temperature for 15 mins before the organic solvent was removed *in vacuo.* The crude reaction mixture was diluted with EtOAc (50 mL) and washed with 1M HCl (25 mL), sat. NaHCO₃ solution (25 mL) and brine (25 mL). The organic layer was dried over Na₂SO₄ and concentrated **in vacuo** to afford the title compound as a white solid.
LC-MS (Method 2B): Rt 1.17 mins; MS m/z 263.0 = [M+H]+
1H NMR (500 MHz, DMSO) δ 7.40 - 7.28 (m, 6H), 6.97 (s, 1H), 5.05 (s, 2H), 3.68 (dd, J = 25.8, 10.6 Hz, 1H), 3.42 - 3.33 (m, 1H), 3.31 - 3.25 (m, 1H), 3.11 (dd, J = 13.2, 10.6 Hz, 1H), 2.24 - 2.15 (m, 1H), 1.75 - 1.66 (m, 1H), 1.22 (s, 3H).

### Step 2 - benzyl (3R)-3-cyano-3-methyl-pyrrolidine-1-carboxylate

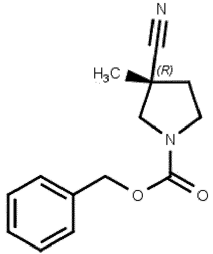

Benzyl (3R)-3-carbamoyl-3-methyl-pyrrolidine-1-carboxylate (step 1) (996 mg, 3.80 mmol) and Burgess reagent (1.36 g, 5.70 mmol) were dissolved in DCM (9.5 mL) under nitrogen and the resulting mixture stirred at 35 °C for 17 hours. The mixture was cooled to room temperature before being diluted with DCM (40 mL) and washed with 1M HCl (50 mL) and brine (25 mL). The organic layer was dried over Na₂SO₄, and concentrated *in vacuo.* Purification by chromatography on silica eluting with a gradient of 10 to 50% EtOAc in petrol afforded the title compound as a colourless syrup.
LC-MS (Method 2B): Rt 1.39 mins; MS m/z 245.0 = [M+H]+
1H NMR (500 MHz, DMSO) δ 7.40 - 7.28 (m, 5H), 5.09 (s, 2H), 3.78 (dd, J = 22.9, 10.9 Hz, 1H), 3.57 - 3.39 (m, 2H), 3.30 - 3.25 (m, 1H), 2.35 - 2.27 (m, 1H), 2.05 - 1.94 (m, 1H), 1.43 (d, J = 3.1 Hz, 3H).

### Step 3 - (3R)-3-methylpyrrolidine-3-carbonitrile hydroiodide

Trimethylsilyl iodide (0.80 mL, 5.65 mmol) was added drop wise to a stirred solution of benzyl (3R)-3-cyano-3-methyl-pyrrolidine-1-carboxylate (step 2) (575 mg, 2.35 mmol) in DCM (24 mL) under nitrogen at 0 °C. The reaction mixture was warmed to room temperature and stirred for 1 hour. Methanol (0.95 mL, 23.54 mmol) was added drop wise and the resulting mixture was stirred at room temperature for 30 mins. The formed solid was collected by filtration, washed with DCM (2 x 5 mL) and dried to afford the title compound as a cream solid.
1H NMR (500 MHz, DMSO) δ 9.10 (br s, 2H), 3.66 (d, J = 12.2 Hz, 1H), 3.40 (ddd, J = 11.9, 8.0, 5.4 Hz, 1H), 3.35 - 3.29 (m, 1H), 3.19 (d, J = 12.2 Hz, 1H), 2.43 (ddd, J = 13.2, 7.6, 5.4 Hz, 1H), 2.06 (dt, J = 13.2, 8.1 Hz, 1H), 1.50 (s, 3H).

### Intermediate UA

### (3S)-3-Methylpyrrolidine-3-carbonitrile hydroiodide

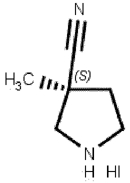

### Step 1 - benzyl (3S)-3-carbamoyl-3-methyl-pyrrolidine-1-carboxylate

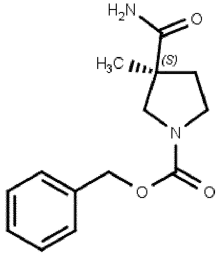

The title compound was prepared from (3S)-1-benzyloxycarbonyl-3-methyl-pyrrolidine-3-carboxylic acid, CDI and ammonium hydroxide (35% in water) analogously to Intermediate U, step 1.
LC-MS (Method 2B): Rt 1.17 mins; MS m/z 263.0 = [M+H]+ (90% @ 210nm).
1H NMR (500 MHz, DMSO) δ 7.44 - 7.26 (m, 6H), 6.97 (s, 1H), 5.06 (s, 2H), 3.69 (dd, J = 25.8, 10.6 Hz, 1H), 3.46 - 3.34 (m, 1H), 3.31 - 3.28 (m, 1H), 3.11 (dd, J = 13.3, 10.6 Hz, 1H), 2.31 - 2.12 (m, 1H), 1.78 - 1.61 (m, 1H), 1.23 (s, 3H).

### Step 2 - benzyl (3S)-3-cyano-3-methyl-pyrrolidine-1-carboxylate

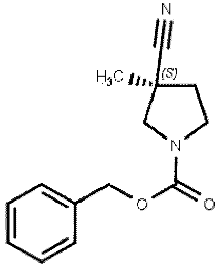

The title compound was prepared from benzyl (3S)-3-carbamoyl-3-methyl-pyrrolidine-1-carboxylate (step 1) and Burgess reagent analogously to Intermediate U, step 2. LC-MS (Method 2B): Rt 1.39 mins; MS m/z 245.0 = [M+H]+ (100% @ 254nm).
1H NMR (500 MHz, DMSO) δ 7.38 (d, J = 4.4 Hz, 4H), 7.36 - 7.30 (m, 1H), 5.10 (s, 2H), 3.79 (dd, J = 22.7, 10.9 Hz, 1H), 3.59 - 3.37 (m, 2H), 3.31 - 3.24 (m, 1H), 2.37 - 2.26 (m, 1H), 2.07 - 1.93 (m, 1H), 1.44 (d, J = 3.1 Hz, 3H).

### Step 3 - (3S)-3-methylpyrrolidine-3-carbonitrile hydroiodide

The title compound was prepared from benzyl (3S)-3-cyano-3-methyl-pyrrolidine-1-carboxylate (step 2) and trimethylsilyl iodide analogously to Intermediate U, step 3.

### BIOLOGICAL EXAMPLES

### Biological Example 1 - Adenosine receptor time-resolved fluorescence resonance energy transfer (TRFRET) binding assay

All FRET binding experiments were conducted at room temperature in white 384-well plates, in assay binding buffer containing 1x LabMed (Cisbio, France), 100µg/mL saponin, 1% DMSO and 0.02% pluronic acid. Binding of the fluorescently labelled Adenosine receptor antagonist XAC (CA200645, FRET acceptor) to terbium-labelled A1, A2a, A2b and A3 adenosine receptors (FRET donors) was detected by time-resolved FRET due to the close proximity of the donor and acceptor in a binding event. To investigate the ability of unlabelled test compounds to bind to Adenosine A1, A2a, A2b and A3 receptors, dose response curves were constructed that determined the ability of a range of concentrations to inhibit the binding of 30nM CA200645 to the A2b receptor and 100nM CA200645 to the A1, A2a, and A3 receptor.

Serial dilution (1:3 dilutions) of test compounds in neat DMSO and transfer of a 400nL sample of test compound into the assay plate was carried out using the Mosquito (TTP Labtech, UK). The compound samples were incubated for 2 hours at room temperature with a fixed concentration of CA200645 defined for each receptor (see above) and CHO cell membranes containing the human Adenosine A1 (0.5µg/well), A2a (0.3µg/well), A2b (1µg/well) or A3 (1 µg/well) receptor in 40 µL of assay buffer. Total and non-specific binding of CA200645 was determined in the absence and presence of 10µM XAC, respectively. Following 2 hours incubation, the level of CA200645 binding was detected on a Pherastar FSX (BMG Labtech, Germany) using standard TR-FRET settings. The terbium donor was excited with three laser flashes at a wavelength of 337 nm, and donor and acceptor emission was detected at 620 nm and 665 nm wavelengths, respectively. FRET ratios were obtained by multiplying the acceptor/donor ratio value by 10,000. Specific binding was determined by subtracting the non-specific binding FRET ratio from the total binding FRET ratio. Compound IC50 curves were analysed using GraphPad Prism 7.0 (GraphPad, USA) and Ki affinity values were determined from the obtained IC50 values using the method of Cheng and Prusoff. The results are presented in Table 1.

**Table 1**

| **Example Number** | **A2a Ki (nM)** | **A2b Ki (nM)** | **A1 Ki (nM)** | **A3 Ki (nM)** |
|---|---|---|---|---|
| **1** | **0.42** | **1.1** | **9** | **305** |
| **2** | **0.40** | **1.0** | **234** | **849** |
| **3** | **58.24** | **50.6** | **1000** | **1976** |
| **3.1** | **3.11** | **6.6** | **94** | **267** |
| **3.2** | **0.40** | **0.7** | **12** | **1039** |
| **3.3** | **2.15** | **3.9** | **136** | |
| **3.4** | **14.34** | **14.3** | | **2046** |
| **3.5** | **11.55** | **11.4** | **434** | **928** |
| **3.6** | **0.64** | **1.5** | **88** | **481** |
| **3.7** | **0.20** | **1.1** | **14** | **382** |
| **3.8** | **0.09** | **0.9** | **116** | **538** |
| **3.9** | **0.49** | **2.2** | **81** | **338** |
| **3.10** | **8.17** | **12.2** | **135** | **144** |
| **3.11** | **0.52** | **1.2** | **102** | **1300** |
| **3.12** | **0.25** | **1.0** | **18** | **531** |
| **4** | **1.01** | **0.7** | **60** | **1000** |
| **4.1** | **0.11** | **0.6** | **11** | **145** |
| **4.2** | **0.27** | **4.4** | **28** | **881** |
| **4.3** | **0.70** | **3.8** | **187** | **603** |
| **4.4** | **0.86** | **4.5** | **263** | **568** |
| **4.5** | **1.54** | **1.4** | **16** | **94** |
| **4.6** | **0.18** | **1.2** | **41** | **170** |
| **4.7** | **0.05** | **1.3** | **6** | **125** |
| **4.8** | **0.37** | **1.5** | **17** | **180** |
| **4.9** | **0.14** | **1.5** | **9** | **133** |
| **4.10** | **0.38** | **2.5** | **50** | **157** |
| **4.11** | **1.30** | **4.7** | **225** | **482** |
| **4.12** | **0.26** | **1.1** | **31** | **389** |
| **4.13** | **1.50** | **1.7** | **50** | **195** |
| **4.14** | **2.00** | **2.4** | **67** | **200** |
| **4.15** | **2.30** | **1.4** | **91** | **339** |
| **4.16** | **0.90** | **2.2** | **41** | **265** |
| **4.17** | **1.90** | **2.8** | **77** | **293** |
| **4.18** | **0.60** | **2.3** | **94** | **414** |
| **4.19** | **0.60** | **1.3** | **46** | **232** |
| **4.20** | **1.50** | **2.6** | **161** | **967** |
| **4.21** | **0.30** | **1.0** | **10** | **265** |
| **4.22** | **0.10** | **0.9** | **35** | **165** |
| **4.23** | **0.20** | **0.7** | **54** | **267** |
| **4.24** | **0.18** | **2.4** | **15** | **383** |
| **4.25** | **0.12** | **1.8** | **14** | **120** |
| **4.26** | **0.05** | **0.4** | **4** | **130** |
| **4.27** | **0.10** | **0.8** | **6** | **423** |
| **4.28** | **0.48** | **2.4** | **135** | **77** |
| **4.29** | **0.13** | **1.3** | **24** | **112** |
| **4.30** | **0.41** | **0.6** | **7** | **106** |
| **4.31** | **3.75** | **4.7** | **210** | **425** |
| **4.32** | **0.15** | **1.1** | **26** | **457** |
| **4.33** | **1.91** | **5.9** | **62** | **410** |
| **4.34** | **12.2** | **13** | **268** | |
| **4.36** | **2.48** | **13.8** | **316** | |
| **4.37** | **4.29** | **27.1** | **545** | |
| **4.38** | **1.8** | **8.1** | **180** | |
| **4.38** | **0.4** | **3.5** | **75** | |
| **5** | **5.75** | **5.5** | **601** | **696** |
| **5.1** | **0.62** | **1.6** | **45** | **229** |
| **5.2** | **0.84** | **0.9** | **40** | **70** |
| **5.3** | **2.31** | **2.1** | **90** | **98** |
| **5.4** | **1.03** | **5.8** | **111** | **33** |
| **5.5** | **0.94** | **2.4** | **114** | **70** |
| **5.6** | **2.13** | **4.2** | **22** | **31** |
| **5.7** | **2.90** | **1.8** | **48** | **247** |
| **5.8** | **0.68** | **1.8** | **34** | **474** |
| **5.9** | **2.32** | **4.3** | **82** | **125** |
| **6** | **0.12** | **1.1** | **37** | **283** |
| **6.1** | **1.05** | **2.1** | **40** | **228** |
| **6.2** | **1.28** | **0.9** | **24** | **166** |
| **6.3** | **0.87** | **1.5** | **32** | **125** |
| **6.4** | **0.67** | **3.1** | **103** | **360** |
| **6.5** | **4.63** | **23.1** | **93** | |
| **6.6** | **1.44** | **1.3** | **81** | **233** |
| **6.7** | **0.11** | **0.7** | **14** | **281** |
| **6.8** | **0.72** | **1.1** | **23** | **312** |
| **6.9** | **0.88** | **0.7** | **44** | **412** |
| **6.10** | **0.30** | **1.2** | **14** | **316** |
| **6.11** | **0.39** | **2.6** | **42** | **319** |
| **6.12** | **0.23** | **2.4** | **137** | **298** |
| **6.13** | **0.40** | **3.4** | **23** | |
| **6.14** | **0.60** | **2.6** | **167** | **772** |
| **6.15** | **2.02** | **5.9** | **263** | **259** |
| **6.16** | **3.99** | **17.6** | **422** | **565** |
| **6.17** | **0.46** | **6.5** | **35** | **405** |
| **6.18** | **0.33** | **6.1** | **70** | **332** |
| **6.19** | **0.61** | **1.1** | **20** | **130** |
| **6.20** | **0.14** | **1.9** | **53** | **287** |
| **6.21** | **0.86** | **1.3** | **6** | **96** |
| **6.22** | **0.38** | **1.8** | **4** | **107** |
| **6.23** | **2.99** | **6.5** | **92** | **394** |
| **6.24** | **0.99** | **2.5** | **129** | **224** |
| **6.25** | **1.73** | **1.1** | **2** | **94** |
| **6.26** | **0.79** | **3.8** | **86** | **302** |
| **6.27** | **1.01** | **1.4** | **45** | **151** |
| **6.28** | **4.84** | **3.2** | **32** | **226** |
| **6.29** | **0.95** | **3.4** | **228** | **635** |
| **6.30** | **0.82** | **2.0** | **97** | **187** |
| **6.31** | **1.50** | **2.1** | **19** | **186** |
| **6.32** | **0.83** | **5.4** | **109** | **719** |
| **6.33** | **3.65** | **3.6** | **51** | **268** |
| **6.34** | **0.64** | **2.1** | **26** | **166** |
| **6.35** | **9.58** | **20.9** | **243** | **303** |
| **6.36** | **0.87** | **4.2** | **133** | **377** |
| **6.37** | **1.82** | **10.1** | **91** | **356** |
| **6.38** | **0.83** | **3.6** | **170** | **278** |
| **6.39** | **10.1** | **39.6** | **383** | **389** |
| **6.40** | **0.41** | **4.6** | **110** | **330** |
| **6.41** | **0.18** | **1.3** | **111** | **235** |
| **6.42** | **0.39** | **9.0** | **44** | **438** |
| **6.43** | **1.12** | **8.2** | **51** | **175** |
| **6.44** | **0.40** | **2.4** | **151** | **299** |
| **6.45** | **0.44** | **2.0** | **104** | **183** |
| **6.46** | **2.06** | **1.9** | **113** | **235** |
| **6.47** | **0.37** | **2.5** | **38** | **465** |
| **6.48** | **1.11** | **1.7** | **96** | **245** |
| **6.49** | **1.27** | **2.2** | **51** | **324** |
| **6.50** | **1.63** | **3.4** | **136** | **472** |
| **6.51** | **1.26** | **1.2** | **120** | **318** |
| **6.52** | **4.30** | **8.6** | **120** | **238** |
| **6.53** | **0.27** | **1.6** | **110** | **662** |
| **6.54** | **0.38** | **1.9** | **53** | **268** |
| **6.55** | **0.60** | **2.4** | **86** | **273** |
| **6.56** | **0.59** | **1.2** | **75** | **368** |
| **6.57** | **0.88** | **1.3** | **32** | **212** |
| **6.58** | **2.27** | **7.8** | **559** | **848** |
| **6.59** | **1.47** | **2.2** | **128** | **184** |
| **6.60** | **3.09** | **6.2** | **185** | **455** |
| **6.61** | **1.52** | **3.2** | **112** | **57** |
| **6.62** | **35.7** | **23.3** | **219** | **358** |
| **6.63** | **34.3** | **29.2** | **343** | **200** |
| **6.64** | **0.14** | **1.4** | **27** | **235** |
| **6.65** | **45.6** | **17.3** | **185** | **295** |
| **6.66** | **0.43** | **0.6** | **37** | **1215** |
| **6.67** | **2.06** | **1.0** | **22** | **201** |
| **6.68** | **0.36** | **0.6** | **30** | **217** |
| **6.69** | **0.94** | **1.4** | **124** | **156** |
| **6.70** | **9.59** | **13.1** | **163** | **192** |
| **6.71** | **3.45** | **4.2** | **110** | **62** |
| **6.72** | **1.12** | **1.2** | **3** | **94** |
| **6.73** | **2.51** | **3.7** | **92** | **165** |
| **6.74** | **0.63** | **1.9** | **23** | **219** |
| **6.75** | **0.81** | **4.8** | **99** | **361** |
| **6.76** | **12.9** | **21.7** | **39** | **321** |
| **6.77** | **1.44** | **5.4** | **133** | **193** |
| **6.78** | **0.67** | **0.9** | **24** | **37** |
| **6.79** | **1.50** | **6.0** | **177** | **537** |
| **6.80** | **0.80** | **2.4** | **130** | **450** |
| **6.81** | **0.47** | **2.3** | **49** | **292** |
| **6.82** | **3.67** | **5.1** | **116** | **246** |
| **6.83** | **67.6** | **102.1** | **129** | **294** |
| **6.84** | **4.46** | **8.4** | **216** | **568** |
| **6.85** | **12.1** | **16.0** | **44** | **216** |
| **6.86** | **0.68** | **1.9** | **2** | **181** |
| **6.87** | **1.75** | **6.7** | **57** | **37** |
| **6.88** | **1.90** | **8.0** | **65** | **385** |
| **6.89** | **1.01** | **3.5** | **326** | **300** |
| **6.90** | **3.16** | **10.7** | **63** | **106** |
| **6.91** | **1.66** | **5.3** | **20** | **162** |
| **6.92** | **0.39** | **2.6** | **66** | **98** |
| **6.93** | **0.31** | **1.5** | **44** | **161** |
| **6.94** | **5.63** | **11.3** | **407** | **719** |
| **6.95** | **1.05** | **4.6** | **29** | **560** |
| **6.96** | **0.53** | **1.8** | **10** | **502** |
| **6.97** | **0.23** | **1.1** | **17** | **250** |
| **6.98** | **0.91** | **7.0** | **309** | **819** |
| **6.99** | **0.13** | **0.7** | **14** | **77** |
| **6.100** | **0.16** | **0.6** | **25** | **549** |
| **6.101** | **1.10** | **3.0** | **30** | **853** |
| **6.102** | **7.42** | **10.5** | **64** | **195** |
| **6.103** | **0.68** | **1.7** | **61** | **475** |
| **6.104** | **0.20** | **1.0** | **31** | **564** |
| **6.105** | **1.27** | **1.3** | **71** | **368** |
| **6.106** | **1.07** | **4.5** | **139** | **195** |
| **6.107** | **1.37** | **2.3** | **69** | **156** |
| **6.108** | **0.59** | **0.9** | **34** | **145** |
| **6.109** | **10.8** | **45.5** | **471** | **298** |
| **6.110** | **0.26** | **3.6** | **13** | **956** |
| **6.111** | **0.20** | **6.4** | **18** | |
| **6.112** | **4.30** | **23.6** | **339** | |
| **6.113** | **10.75** | **8.3** | **361** | |
| **6.114** | **9.95** | **23** | **240** | |
| **6.115** | **8.00** | **29** | **177** | |
| **6.116** | **7.39** | **10** | **281** | |
| **6.120** | **11.11** | **63.9** | **123** | |
| **6.121** | **1.94** | **6.8** | **123** | |
| **7** | **2.81** | **11.1** | **42** | **304** |
| **7.1** | **0.99** | **7.6** | **47** | **418** |
| **7.2** | **0.20** | **1.0** | **8** | **337** |
| **7.3** | **1.02** | **5.7** | **208** | |
| **7.4** | **2.73** | **13.5** | **58** | |
| **7.5** | **1.26** | **11.9** | **9** | |
| **7.6** | **4.09** | **22.6** | **162** | |
| **7.7** | **5.27** | **18.2** | **49** | |
| **7.8** | **6.73** | **60.2** | **194** | |
| **7.9** | **10.33** | **44.2** | **61** | |
| **7.10** | **29.38** | **90.0** | **122** | |
| **7.11** | **12.75** | **78.7** | **110** | |
| **8** | **1.83** | **1.8** | **59** | **776** |
| **9** | **0.61** | **5.3** | **47** | **2023** |
| **9.1** | **0.78** | **7.0** | **95** | **1003** |
| **9.2** | **0.79** | **5.3** | **102** | **772** |
| **9.3** | **1.11** | **8.1** | **29** | **1507** |
| **9.4** | **4.19** | **20.1** | **410** | **1545** |
| **9.5** | **2.28** | **7.5** | **174** | **699** |
| **9.6** | **0.51** | **2.9** | **12** | **369** |
| **9.7** | **0.42** | **3.2** | **68** | **742** |
| **9.8** | **0.37** | **2.4** | **24** | **615** |
| **9.9** | **0.31** | **3.1** | **48** | **491** |
| **10** | **1.19** | **1.4** | **43** | **283** |
| **11** | **0.62** | **1.3** | **51** | **515** |
| **12** | **0.16** | **3.1** | **6** | **930** |
| **12.1** | **0.20** | **3.1** | **7** | **1236** |
| **12.2** | **0.28** | **8.4** | **6** | **1525** |
| **12.3** | **0.44** | **5.0** | **27** | **1812** |
| **12.3a** | **0.18** | **2.6** | **23** | **1091** |
| **12.3b** | **0.42** | **1.2** | **19** | |
| **13** | **24.34** | **195.6** | **373** | |
| **13.1** | **0.64** | **1.9** | **164** | |
| **13.2** | **1.90** | **4.4** | **27** | |
| **13.3** | **11.37** | **77.0** | **322** | |
| **13.4** | **5.13** | **19.6** | **54** | **54** |
| **13.5** | **9.87** | **18.8** | **84** | **226** |
| **13.6** | **16.78** | **79.7** | **220** | **133** |
| **13.7** | **7.46** | **13.7** | **174** | **256** |
| **13.8** | **2.38** | **12.3** | **47** | **41** |
| **13.9** | **2.01** | **14.9** | **225** | **646** |
| **13.10** | **4.56** | **14.5** | **61** | **573** |
| **13.11** | **12.90** | **58.4** | **132** | **226** |
| **13.12** | **0.84** | **4.6** | **41** | **426** |
| **13.13** | **0.48** | **8.0** | **19** | **871** |
| **13.14** | **0.18** | **4.2** | **13** | |
| **13.15** | **0.33** | **5.1** | **41** | |
| **14** | **2.71** | **7.4** | **45** | **47** |
| **14.1** | **2.67** | **8.6** | **41** | **112** |
| **14.2** | **2.09** | **8.3** | **183** | **132** |
| **14.3** | **3.49** | **7.6** | **57** | **520** |
| **14.4** | **5.79** | **37.1** | **58** | |
| **14.5** | **0.43** | **2.5** | **61** | |
| **14.6** | **15.6** | **33** | **185** | |
| **16** | **2.71** | **2.4** | **55** | **334** |
| **16.1** | **0.29** | **2.2** | **22** | **257** |
| **16.2** | **0.38** | **1.4** | **16** | **135** |
| **16.3** | **5.71** | **6.2** | **82** | **383** |
| **16.4** | **0.13** | **3.3** | **12** | **1712** |
| **16.5** | **11.87** | **19.9** | **87** | **325** |
| **16.6** | **0.16** | **1.6** | **42** | **985** |
| **16.7** | **0.13** | **1.6** | **13** | **478** |
| **16.8** | **0.13** | **1.1** | **6** | **537** |
| **16.9** | **0.10** | **0.8** | **9** | **350** |
| **16.10** | **0.57** | **1.7** | **43** | **254** |
| **17** | **1.29** | **10.0** | **12** | |
| **18** | **0.39** | **3.4** | **248** | **852** |
| **19** | **1.86** | **7.9** | **137** | |
| **20** | **6.85** | **39.1** | **133** | **2543** |
| **21** | **0.21** | **3.9** | **29** | **1558** |
| **22** | **0.20** | **3.2** | **7** | **2060** |
| **23** | **0.16** | **5.7** | **9** | **1838** |
| **24** | **0.24** | **2.7** | **71** | **683** |
| **26** | **0.24** | **1.2** | **38** | **456** |
| **27** | **0.41** | **0.8** | **21** | **225** |
| **27a** | **0.54** | **1.2** | **37** | **313** |
| **27b** | **0.14** | **1.0** | **33** | **271** |
| **28** | **0.09** | **1.0** | **30** | **381** |

### Biological Example - CD3/CD28 stimulated IL-2 release NECA reversal assay in human PBMCs

Blood is drawn from healthy volunteers using sodium citrate as the anticoagulant (0.3% final concentration). After centrifugation of the blood over Histopaque-1077, PBMCs are collected from the Histopaque/plasma interface and washed twice in PBS (300g for 10 mins at room temp). Cells are plated at 50,000 cells/well in 150µl RPMI/10% FCS in 96-well cell culture plates that have been precoated with 1ug/ml CD3 antibody. 50µl diluted compound mix is added to the cells, to obtain final concentrations of 1ug/ml CD28 antibody, 1uM NECA and 0.003-10µM adenosine receptor antagonist. Assay plates are incubated for 24 hours at 37°C in a humidified incubator. Culture supernatant is tested for IL-2 levels using the human IL-2 Tissue Culture Kit (Meso Scale Discovery). Data for dose-response curves is calculated as % inhibition with 100% inhibition defined from no agonist control wells (+CD3/28 -NECA)

**Table 2**

| **Example** | **IL-2 IC50 (µM)** |
|---|---|
| **2** | **0.0020** |
| **3.1** | **0.0130** |
| **3.6** | **0.0075** |
| **3.7** | **0.0033** |
| **3.8** | **0.0010** |
| **3.9** | **0.0179** |
| **3.11** | **0.0104** |
| **3.12** | **0.008** |
| **4** | **0.0038** |
| **4.1** | **0.0003** |
| **4.2** | **0.0079** |
| **4.3** | **0.0182** |
| **4.4** | **0.0062** |
| **4.5** | **0.0085** |
| **4.7** | **0.0008** |
| **4.8** | **0.0011** |
| **4.9** | **0.0032** |
| **4.10** | **0.0029** |
| **4.11** | **0.0038** |
| **4.12** | **0.002** |
| **4.13** | **0.0141** |
| **4.15** | **0.0103** |
| **4.17** | **0.0085** |
| **4.18** | **0.0061** |
| **4.19** | **0.0054** |
| **4.20** | **0.0013** |
| **4.22** | **0.0011** |
| **4.23** | **0.0006** |
| **4.24** | **0.0028** |
| **4.25** | **0.0011** |
| **4.26** | **0.0004** |
| **4.28** | **0.0047** |
| **4.29** | **0.0021** |
| **4.30** | **0.0013** |
| **4.32** | **0.0007** |
| **5.2** | **0.0202** |
| **5.4** | **0.0718** |
| **5.5** | **0.0306** |
| **5.6** | **0.0305** |
| **5.8** | **0.0184** |
| **5.9** | **0.0183** |
| **6** | **0.0006** |
| **6.1** | **0.0036** |
| **6.4** | **0.0014** |
| **6.6** | **0.0082** |
| **6.7** | **0.0008** |
| **6.9** | **0.0043** |
| **6.10** | **0.0029** |
| **6.11** | **0.0052** |
| **6.12** | **0.0027** |
| **6.13** | **0.0037** |
| **6.14** | **0.0053** |
| **6.17** | **0.0053** |
| **6.18** | **0.005** |
| **6.20** | **0.0018** |
| **6.24** | **0.0047** |
| **6.26** | **0.0032** |
| **6.29** | **0.0053** |
| **6.30** | **0.0069** |
| **6.32** | **0.0086** |
| **6.36** | **0.0042** |
| **6.38** | **0.006** |
| **6.40** | **0.0022** |
| **6.41** | **0.0027** |
| **6.42** | **0.005** |
| **6.44** | **0.0031** |
| **6.45** | **0.0094** |
| **6.46** | **0.0151** |
| **6.47** | **0.0019** |
| **6.48** | **0.0037** |
| **6.50** | **0.0036** |
| **6.51** | **0.0061** |
| **6.53** | **0.0025** |
| **6.54** | **0.0033** |
| **6.55** | **0.0022** |
| **6.56** | **0.0035** |
| **6.57** | **0.0026** |
| **6.58** | **0.0085** |
| **6.59** | **0.0059** |
| **6.61** | **0.0172** |
| **6.64** | **0.0011** |
| **6.66** | **0.0043** |
| **6.67** | **0.0106** |
| **6.68** | **0.0031** |
| **6.69** | **0.0109** |
| **6.73** | **0.0135** |
| **6.74** | **0.0056** |
| **6.75** | **0.0059** |
| **6.79** | **0.0145** |
| **6.81** | **0.0076** |
| **6.88** | **0.0078** |
| **6.92** | **0.0012** |
| **6.93** | **0.0013** |
| **6.94** | **0.0105** |
| **6.99** | **0.0007** |
| **7** | **0.0160** |
| **7.1** | **0.0205** |
| **9.2** | **0.0027** |
| **9.7** | **0.001** |
| **9.8** | **0.0007** |
| **9.9** | **0.0013** |
| **10** | **0.0247** |
| **11** | **0.0105** |
| **12.2** | **0.0004** |
| **16.1** | **0.0067** |
| **16.6** | **0.0026** |
| **16.7** | **0.0009** |
| **18** | **0.0072** |
| **24** | **0.0016** |
| **27** | **0.0009** |
| **27b** | **0.001** |
| **28** | **0.0004** |

### Biological Example - Measurement of pCREB in CD8+T cells in human whole blood

Heparinised human whole blood was pre-incubated at 37°C with serial dilutions of A2a antagonists for 20 min. and the phosphodiesterase inhibitor rolipram to amplify the pCREB response. The adenosine receptor agonist NECA is then added at a final concentration of 3µM and following a 60 min incubation the blood is fixed and red blood cells lysed. White blood cells are isolated, permeabilized and stained with directly conjugated fluorescent antibodies to phospho-CREB (Alexa Fluor 488) and CD8 (Alexa Fluor 647) and the level of phospho-CREB in CD8+ T cells is measured by FACS using a BD Accuri C6 Flow Cytometer.

**Table 3**

| **Example** | **pCREB IC50 (µM)** |
|---|---|
| **2** | **0.28** |
| **3.1** | **1.77** |
| **3.6** | **0.99** |
| **3.7** | **0.29** |
| **3.8** | **0.75** |
| **3.9** | **0.47** |
| **3.11** | **0.67** |
| **3.12** | **0.60** |
| **4** | **0.13** |
| **4.1** | **0.01** |
| **4.2** | **0.09** |
| **4.3** | **0.17** |
| **4.4** | **0.17** |
| **4.5** | **0.16** |
| **4.7** | **0.09** |
| **4.8** | **0.04** |
| **4.9** | **0.05** |
| **4.10** | **0.10** |
| **4.11** | **0.22** |
| **4.12** | **0.05** |
| **4.13** | **0.55** |
| **4.15** | **0.65** |
| **4.17** | **0.37** |
| **4.18** | **0.35** |
| **4.19** | **5.52** |
| **4.20** | **0.13** |
| **4.22** | **0.11** |
| **4.23** | **0.03** |
| **4.24** | **0.08** |
| **4.25** | **0.05** |
| **4.26** | **0.02** |
| **4.28** | **0.67** |
| **4.29** | **0.05** |
| **4.30** | **0.06** |
| **4.32** | **0.06** |
| **5.2** | **1.18** |
| **5.4** | **1.95** |
| **5.5** | **2.69** |
| **5.6** | **0.72** |
| **5.8** | **0.98** |
| **5.9** | **1.00** |
| **6** | **0.07** |
| **6.1** | **0.15** |
| **6.4** | **0.19** |
| **6.6** | **0.60** |
| **6.7** | **0.02** |
| **6.9** | **0.16** |
| **6.10** | **0.04** |
| **6.11** | **0.05** |
| **6.12** | **0.11** |
| **6.13** | **0.14** |
| **6.14** | **0.05** |
| **6.17** | **0.07** |
| **6.18** | **0.04** |
| **6.20** | **0.02** |
| **6.24** | **0.09** |
| **6.26** | **0.08** |
| **6.29** | **0.51** |
| **6.30** | **0.53** |
| **6.32** | **0.23** |
| **6.36** | **0.09** |
| **6.38** | **0.58** |
| **6.40** | **0.08** |
| **6.41** | **0.12** |
| **6.42** | **0.13** |
| **6.44** | **0.10** |
| **6.45** | **0.31** |
| **6.46** | **0.36** |
| **6.47** | **0.08** |
| **6.48** | **0.14** |
| **6.50** | **0.19** |
| **6.51** | **0.32** |
| **6.53** | **0.13** |
| **6.54** | **0.14** |
| **6.55** | **0.01** |
| **6.56** | **0.07** |
| **6.57** | **0.06** |
| **6.58** | **0.36** |
| **6.59** | **0.10** |
| **6.61** | **0.35** |
| **6.64** | **0.01** |
| **6.66** | **0.30** |
| **6.67** | **0.25** |
| **6.68** | **0.05** |
| **6.69** | **0.43** |
| **6.73** | **0.20** |
| **6.74** | **0.24** |
| **6.75** | **0.18** |
| **6.79** | **0.35** |
| **6.81** | **0.23** |
| **6.88** | **0.48** |
| **6.92** | **0.03** |
| **6.93** | **0.05** |
| **6.94** | **0.19** |
| **6.96** | **0.03** |
| **6.97** | **0.05** |
| **6.98** | **0.05** |
| **6.99** | **0.04** |
| **7** | **0.58** |
| **7.1** | **0.65** |
| **9.2** | **0.02** |
| **9.7** | **0.01** |
| **9.8** | **0.01** |
| **9.9** | **0.02** |
| **10** | **1.06** |
| **11** | **1.13** |
| **12.2** | **0.01** |
| **12.3** | **0.02** |
| **12.3a** | **0.01** |
| **12.3b** | **0.01** |
| **13.12** | **0.07** |
| **13.13** | **0.11** |
| **16.1** | **0.12** |
| **16.6** | **0.10** |
| **16.7** | **0.06** |
| **18** | **0.41** |
| **19** | **0.29** |
| **21** | **0.02** |
| **23** | **0.02** |
| **24** | **0.02** |
| **26** | **0.05** |
| **27** | **0.08** |
| **27a** | **0.08** |
| **27b** | **0.04** |
| **28** | **0.06** |

### MoDC pCREB Assay

Monocytes are isolated from human whole blood using CD14 microbeads (Miltenyi Biotec 130-050-201) and differentiated to immature monocyte-derived dendritic cells (MoDC) by culture for 5 days in Mo-DC Differentiation Medium (Miltenyi Biotec 130-094-812). MoDCs are seeded into fresh medium into U bottomed 96 well culture plates and pre-incubated at 37°C with serial dilutions of A_{2A/B} antagonists for 15 min. At the same time the cells are co-incubated with the phosphodiesterase inhibitor rolipram (final concentration of 20 µM) to amplify the subsequent pCREB response. Adenosine receptor agonist NECA is then added at 1µM final concentration, leading to an increase in CREB phosphorylation. Following a 45 min incubation at 37°C, the cells are fixed, permeabilized and stained with directly conjugated fluorescent antibodies to phospho-CREB (Alexa Fluor 488), CD209 (PerCP-Cy5.5) and CD86 (APC). Samples are analysed by FACS to measure fluorescence in corresponding channels, and also forward and Side scatter. The population of CD209+/ CD86-low cells corresponding to immature dendritic cells is gated for analysis of pCREB levels. The adenosine receptor response in this cell population is largely specific to A_{2B}, based on specific response to the highly selective antagonist PSB-603.

Approximately 5% of the analysed cells are positive for detection of pCREB in the absence of agonist stimulation, representing the background signalling of all pathways upstream of CREB. Following NECA stimulation this is increased to ~80% pCREB +ve, and this increase is essentially fully reversible by A_{2B} or dual A_{2A/B} antagonists.

IC50 is represented as the point of inflection of a sigmoidal curve

**Table 4**

| **Example** | **MoDC IC50 (µM)** |
|---|---|
| **3.6** | **0.051** |
| **3.8** | **0.011** |
| **4.1** | **0.015** |
| **4.4** | **0.095** |
| **4.8** | **0.012** |
| **4.9** | **0.026** |
| **4.12** | **0.018** |
| **4.22** | **0.011** |
| **4.23** | **0.02** |
| **4.24** | **0.079** |
| **4.28** | **0.01** |
| **4.32** | **0.014** |
| **6** | **0.009** |
| **6.6** | **0.024** |
| **6.7** | **0.007** |
| **6.2** | **0.028** |
| **6.36** | **0.016** |
| **6.46** | **0.038** |
| **6.53** | **0.04** |
| **6.56** | **0.007** |
| **6.68** | **0.003** |
| **6.69** | **0.003** |
| **6.74** | **0.001** |
| **6.79** | **0.083** |
| **6.81** | **0.019** |
| **6.92** | **0.011** |
| **6.93** | **0.009** |
| **6.97** | **0.013** |
| **6.98** | **0.072** |
| **6.99** | **0.008** |
| **6.100** | **0.01** |
| **6.104** | **0.012** |
| **6.105** | **0.004** |
| **6.108** | **0.062** |
| **9.8** | **0.008** |
| **12** | **0.073** |
| **12.2** | **0.055** |
| **12.3** | **0.031** |
| **12.3a** | **0.016** |
| **13.12** | **0.073** |
| **16.4** | **0.036** |
| **16.6** | **0.013** |
| **16.7** | **0.014** |
| **16.9** | **0.018** |
| **21** | **0.019** |
| **23** | **0.182** |
| **24** | **0.021** |
| **26** | **0.017** |
| **27a** | **0.01** |
| **27b** | **0.004** |
| **28** | **0.009** |

### REFERENCES

1. Sukari A Nagasaka M Al-Hadidi A and Lum LG (2016). Anticancer Res. 36(11):5593-5606.
2. Vijayan D, Young A, Teng MWL, and Smyth MJ (2017), Nat Rev Cancer. 17(12):709-724.
3. Houthuys, E, Marillier R, Deregnaucourt,T, Brouwer, M, Pirson, R, Marchante, J , et al (2016). SITC 2017 Conference, Maryland.
4. Gao ZW, Dong K, Zhang HZ (2014). "The roles of CD73 in cancer". Biomed Res Int: 2014:460654.
5. Loi S, Pommey S, Haibe-Kains B, Beavis PA, Darcy PK, Smyth MJ, et al. (2013), "CD73 promotes anthracycline resistance and poor prognosis in triple negative breast cancer" Proc Natl Acad Sci U S A.;110(27):11091-6*.*
6. Deaglio S, Dwyer KM , Gao W, Friedman D, Usheva A, Erat A et al (2007). J. Exp Med.. 204, No. 6, June 11, 2007 1257-1265.
7. Csóka B, Selmeczy Z, Koscsó B, Németh ZH, Pacher P, Murray PJ, Kepka-Lenhart D, Morris SM Jr, Gause WC, Leibovich SJ, Haskó G (2012). Adenosine promotes alternative macrophage activation via A2A and A2B receptors. FASEB J 26:376-86.
8. Ryzhov S, Novitskiy SV, Goldstein AE, Biktasova A, Blackburn MR, Biaggioni I, Dikov MM, Feoktistov I (2011). Adenosinergic regulation of the expansion and immunosuppressive activity of CD11b+Gr1+ cells. J Immunol. 187(11):6120-9.
9. Novitskiy SV, Ryzhov S, Zaynagetdinov R, Goldstein AE, Huang Y, Tikhomirov OY, Blackburn MR, Biaggioni I, Carbone DP, Feoktistov I, Dikov MM (2008). Adenosine receptors in regulation of dendritic cell differentiation and function. Blood 2008 112 :1822-31
10. Wilson JM, Ross WG, Agbai ON, Frazier R, Figler RA, Rieger J, Linden J, Ernst PB (2009). The A2B adenosine receptor impairs the maturation and immunogenicity of dendritic cells. J. Immunol. 18:4616-23.
11. Cekic C, Sag D, Li Y, Theodorescu D, Strieter RM, Linden J.J (2012). Adenosine A2B receptor blockade slows growth of bladder and breast tumors. J Immunol. 188:198-205.
12. Sorrentino C, Miele L, Porta A, Pinto A, Morello S (2015). Myeloid-derived suppressor cells contribute to A2B adenosine receptor-induced VEGF production and angiogenesis in a mouse melanoma model. Oncotarget. 6:27478-89.
13. lannone R, Miele L, Maiolino P, Pinto A, Morello S (2013). Blockade of A2b adenosine receptor reduces tumor growth and immune suppression mediated by myeloid-derived suppressor cells in a mouse model of melanoma. Neoplasia. 2013 15(12):1400-9.

### NUMBERED PARAGRAPHS

### The following paragraphs define certain aspects and embodiments of the invention.

1. A compound, or pharmaceutically acceptable salt thereof, having the structural formula I' shown below: 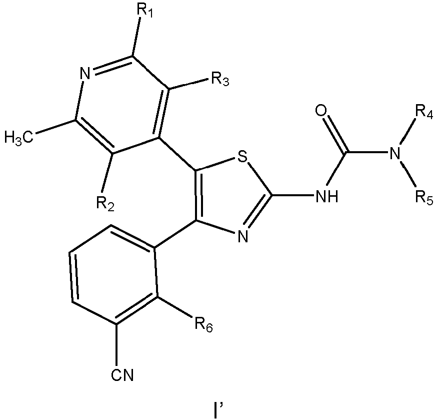 wherein:
   R₁ is selected from (1-2C)alkyl, halo, (1-2C)haloalkyl, (1-2C)haloalkoxy, cyano, -(CH₂)_{q1}-OR_{1A}, -(CH₂)_{q1}-C(O)R_{1A}, -(CH₂)_{q1}-C(O)OR_{1A}, -(CH₂)_{q1}-OC(O)R_{1A}, -(CH₂)_{q1}-C(O)N(R_{1B})R_{1A}, -(CH₂)_{q1}-N(R_{1B})C(O)R_{1A}, -(CH₂)_{q1}-S(O)ₚR_{1A} (where p is 0, 1 or 2), -(CH₂)_{q1}-SO₂N(R_{1B})R_{1A}, or -(CH₂)_{q1}-N(R_{1B})SO₂R_{1A},
      wherein q1 is 0, 1, or 2; and
      wherein R_{1A} and R_{1B} are each independently selected from hydrogen, (1-2C)alkyl, (3-4C)cycloalkyl or (3-4C)cycloalkyl(1-2C)alkyl;

   R₂ and R₃ are both hydrogen or one of R₂ and R₃ is hydrogen and the other is selected from fluoro, methyl or methoxy;
   R₄ and R₅ are linked such that, together with the nitrogen atom to which they are attached, they form heterocyclic ring which is optionally substituted on any available carbon atom by one or more R_{10C} substituent groups,
      wherein R_{10C} is selected from oxo, thioxo, halo, or cyano substituents, or a group of the formula:

         -[CR₇ₐR_{7b}]ₙ₋L-Z
      wherein
      n is 0 to 6;
      R₇ₐ and R_{7b} are each independently selected from hydrogen, fluoro or (1-2C)alkyl;
      L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -C(S)N(Rₐ)-, -N(Rₐ)C(S)-, -N(Rₐ)C(S)N(R_{b})-, -S(O)₂N(Rₐ)-, or -N(Rₐ)SO₂-, wherein Rₐ and R_{b} are each independently selected from hydrogen or (1-2C)alkyl; and
      Z is selected from hydrogen, (1-6C)alkyl, (3-6C)cycloalkyl, (3-6C)cycloalkyl(1-2C)alkyl, aryl, aryl(1-2C)alkyl, heterocyclyl, heterocyclyl(1-2C)alkyl, heteroaryl or heteroaryl(1-2C)alkyl; and wherein Z is optionally substituted by one or more substituents selected from (1-6C)alkyl, halo, (1-6C)haloalkyl, (1-6C)haloalkoxy, cyano, nitro, -NR_{c}R_{d}, -OR_{c}, -C(O)R_{c}, -C(O)OR_{c}, -OC(O)_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1, 2 or 3) or oxo,
      wherein any alkyl moiety in a substituent group on Z is optionally further substituted by cyano, halo, hydroxy, amino, oxo, (1-2C)alkyl or (1-2C)alkoxy and R_{c} and R_{d} are each independently selected from hydrogen, (1-6C)alkyl, (1-6C)haloalkyl or (3-6C)cycloalkyl;
   and wherein any available N atoms are optionally in the form of a N-oxide or are optionally substituted by one or more R_{10N},
      wherein R_{10N} is -S(O)₂NH₂ or selected from:
         (iv) -Z₁;
         (v) -L₁ₐ-Z₁; or
         (vi) -[CR₈ₐR_{8b}]₁₋₆-L_{1b}-Z₁;
      wherein
      R₈ₐ and R_{8b} are each independently selected from hydrogen, fluoro or (1-2C)alkyl;
      L₁ₐ is selected from -C(O)-, -S(O)₂-, -C(O)O-, -C(O)N(Rₐ₁)-, -S(O)₂N(Rₐ₁)- or -N(Rₐ₁)-, wherein Rₐ₁ is selected from hydrogen or (1-2C)alkyl;
      L_{1b} is absent or selected from -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, -N(Rₐ₂)C(S)N(R_{b2})-, -S(O)₂N(Rₐ₂)-, or -N(Rₐ₂)SO₂, wherein Rₐ₂ and R_{b2} are each independently selected from hydrogen or (1-2C)alkyl; and
      Z₁ is selected from (1-6C)alkyl, (3-6C)cycloalkyl, aryl, heterocyclyl, heteroaryl, (3-6C)cycloalkyl(1-2C)alkyl, aryl(1-2C)alkyl, heterocyclyl(1-2C)alkyl or heteroaryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-6C)alkyl, halo, (1-6C)haloalkoxy, cyano, nitro, - NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen, (1-6C)alkyl, (1-6C)haloalkyl or (3-6C)cycloalkyl;
   and wherein any S atoms present in the heterocyclic ring may optionally be present as S(=O), S(=O)₂ or S(=O)(=NRₑ) wherein Rₑ is selected from hydrogen, (1-3C)alkyl or (2-3C)alkanoyl; and
   R₆ is selected from hydrogen, halo, methyl, methoxy and trifluoromethyl.
2. A compound according to paragraph 1, or a pharmaceutically acceptable salt thereof, wherein R₁ is selected from methyl, halo or -CF₃;
3. A compound according to any one of the preceding paragraphs, or a pharmaceutically acceptable salt thereof, wherein R₁ is selected from methyl or chloro;
4. A compound according to any one of the preceding paragraphs, or a pharmaceutically acceptable salt thereof, wherein R₂ and R₃ are both hydrogen or one of R₂ and R₃ is hydrogen and the other is fluoro or methyl.
5. A compound according to any one of the preceding paragraphs, or a pharmaceutically acceptable salt thereof, wherein R₂ and R₃ are both hydrogen.
6. A compound according to any one of the preceding paragraphs, or a pharmaceutically acceptable salt thereof, wherein R₄ and R₅ are linked such that, together with the nitrogen atom to which they are attached, they form heterocyclic ring which is optionally substituted on any available carbon atom by one or more R_{10C} substituent groups,
   wherein R_{10C} is selected from oxo, halo, or cyano substituents, or a group of the formula:

      -[CH₂]ₙ-L-Z
   wherein
      n is 0 to 2;
      L is absent or selected from -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)-, or -N(Rₐ₂)SO₂-, wherein Rₐ₂ and R_{b2} are each independently selected from hydrogen or methyl; and
      Z is selected from hydrogen, (1-6C)alkyl, (3-6C)cycloalkyl, (3-6C)cycloalkyl(1-2C)alkyl, aryl, heterocyclyl or heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, -C(O)OR_{c}, -OC(O)_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1 or 2) or oxo;
      wherein any alkyl moiety in a substituent group on Z₁ is optionally further substituted by cyano, halo, hydroxy, amino, oxo, (1-2C)alkyl or (1-2C)alkoxy and R_{c} and R_{d} are each independently selected from hydrogen, (1-2C)alkyl, (1-2C)haloalkyl or (3-6C)cycloalkyl;
   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]₁₋₂-L_{1b}-Z₁;
         wherein
         R₈ₐ and R_{8b} are both hydrogen;
         L₁ₐ is selected from -C(O)-, -S(O)₂- or -S(O)₂N(Rₐ₁)-, wherein Rₐ₁ is hydrogen or methyl;
         L_{1b} is absent or selected from -O-, -S-, -SO-, -SO₂₋, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)-, or -N(Rₐ₂)SO₂, wherein Rₐ₂ and R_{b2} are each independently selected from hydrogen or methyl; and
         Z₁ is selected from (1-6C)alkyl, (3-6C)cycloalkyl, aryl, heterocyclyl, heteroaryl, (3-6C)cycloalkyl(1-2C)alkyl or aryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, nitro, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen or (1-2C)alkyl;
   and wherein any S atoms present in the heterocyclic ring may optionally be present as S(=O), S(=O)₂ or S(=O)(=NRₑ) wherein Rₑ is selected from hydrogen, (1-2C)alkyl or (2C)alkanoyl.
7. A compound according to any one of the preceding paragraphs, or a pharmaceutically acceptable salt thereof, wherein R₄ and R₅ are linked such that, together with the nitrogen atom to which they are attached, they form heterocyclic ring which is optionally substituted on any available carbon atom by one or more R_{10C} substituent groups,
   wherein R_{10C} is selected from oxo, halo, or cyano substituents, or a group of the formula:

      -[CH₂]ₙ-L-Z
   wherein
      n is 0 to 2;
      L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)-, or -N(Rₐ)SO₂-, wherein Rₐ and R_{b} are hydrogen; and
      Z is selected from hydrogen, (1-4C)alkyl, phenyl, a 4-7 membered heterocyclyl or a 5 or 6-membered heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, -C(O)N(R_{c})R_{d}, -N(R_{c})C(O)R_{d} or -S(O)_{y}R_{c} (where y is 0, 1 or 2) or oxo;
      wherein R_{c} and R_{d} are each independently selected from hydrogen or (1-2C)alkyl;
   and wherein any available N atoms are optionally in the form of a N-oxide or are optionally substituted by one or more R_{10N}, wherein R_{10N} is -S(O)₂NH₂ or selected from:
      (i) -Z₁;
      (ii) -L₁ₐ-Z₁; or
      (iii) -[CR₈ₐR_{8b}]1₋₂-L_{1b}-Z₁;
         wherein
         R₈ₐ and R_{8b} are both hydrogen;
         L₁ₐ is selected from -C(O)-, -S(O)₂- or -S(O)₂N(Rₐ₁)-, wherein Rₐ₁ is hydrogen;
         L_{1b} is absent or selected from -O-, -S-, -SO-, -SO₂₋, -N(Rₐ₂)-, -C(O)N(Rₐ₂)-, -N(Rₐ₂)C(O)-, -S(O)₂N(Rₐ₂)- or -N(Rₐ₂)SO₂, wherein Rₐ₂ is hydrogen; and
         Z₁ is selected from (1-4C)alkyl, phenyl, a 4-7 membered heterocyclyl or a 5 or 6-membered heteroaryl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, -NRₑR_{f}, -ORₑ, -C(O)Rₑ, -C(O)N(Rₑ)R_{f}, -N(Rₑ)C(O)R_{f}, -S(O)_{y}Rₑ (where y is 0, 1 or 2); and wherein Rₑ and R_{f} are each independently selected from hydrogen or methyl;
   and wherein any S atoms present in the heterocyclic ring may optionally be present as S(=O), S(=O)₂ or S(=O)(=NRₑ) wherein Rₑ is selected from hydrogen, (1-2C)alkyl or (2C)alkanoyl.
8. A compound according to any one of the preceding paragraphs, or a pharmaceutically acceptable salt thereof, wherein R₄ and R₅ are linked such that, together with the nitrogen atom to which they are attached, they form a heterocyclic ring selected from any one of the following options: 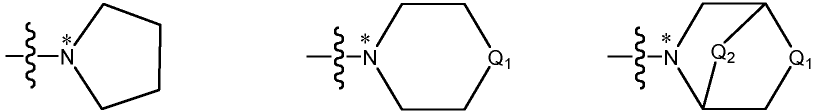 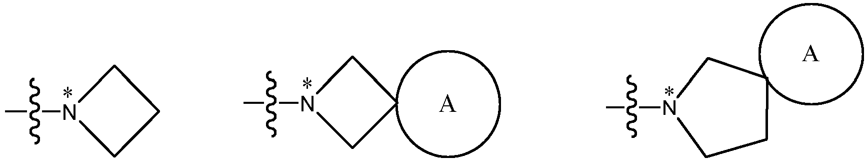 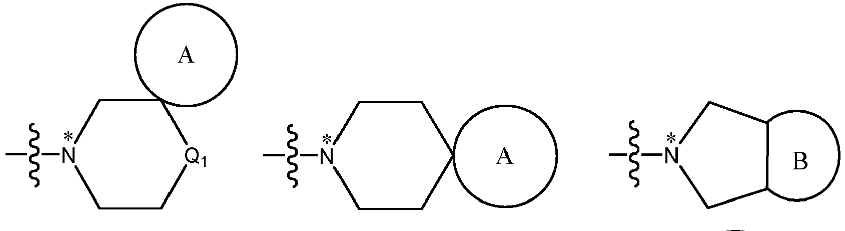 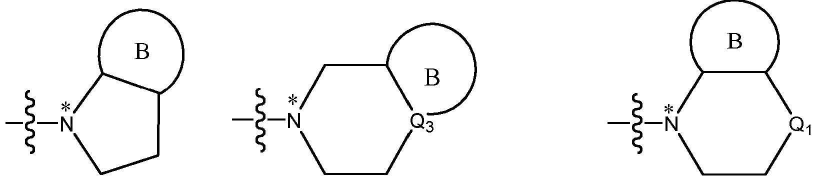 wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-, -CHR_{10C}-, -C(R_{10c})₂-, -CHR_{10C}-CH₂-, -CH₂-CHR_{10C}-, -CHR_{10C}-CHR_{10C}-, -C(R_{10c})₂-CH₂-, or -CH₂-C(R_{10c})₂-;
   Q₃ is C or N;
   Ring A is a spiro-fused 4, 5 or 6 membered carbocyclic or heterocyclic ring;
   Ring B is a fused 4, 5 or 6 membered carbocyclic or heterocyclic ring;
   wherein Rₑ is selected from hydrogen, (1-2C)alkyl or (2C)alkanoyl;
   and wherein each of the heterocyclic ring systems is optionally substituted with one or more R_{10c} or R_{10N} substituent groups as defined in any one of paragraphs 1, 6 or 7.
9. A compound according to any one of the preceding paragraphs, or a pharmaceutically acceptable salt thereof, wherein R₄ and R₅ are linked such that, together with the nitrogen atom to which they are attached, they form a heterocyclic ring selected from any one of the following options: 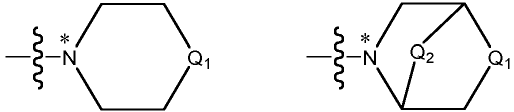
   wherein * denotes the N atom to which R₄ and R₅ are attached;
   Q₁ is O, NH, S, S(O), S(O)₂, S(O)(=NRₑ), N-R_{10N}, CH₂, CHR_{10C} or C(R_{10c})₂;
   Q₂ is -CH₂-;
   wherein Rₑ is selected from hydrogen, methyl, or (2C)alkanoyl;
   and R_{10c} or R_{10N} are as defined in any one of paragraphs 1, 6 or 7.
10. A compound according to any one of the preceding paragraphs, or a pharmaceutically acceptable salt thereof, wherein R₄ and R₅ are linked such that, together with the nitrogen atom to which they are attached, they form a heterocyclic ring of the structure: 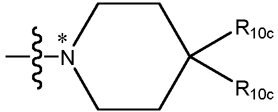
   wherein * denotes the N atom to which R₄ and R₅ are attached;
   and each R_{10c} is independently selected from the options defined in any one of paragraphs 1, 6 or 7 above.
11. A compound according to any one of the preceding paragraphs, or a pharmaceutically acceptable salt thereof, wherein R₆ is selected from hydrogen, halo or methyl.
12. A compound according to any one of the preceding paragraphs, or a pharmaceutically acceptable salt thereof, wherein R₆ is hydrogen.
13. A compound of the formula: 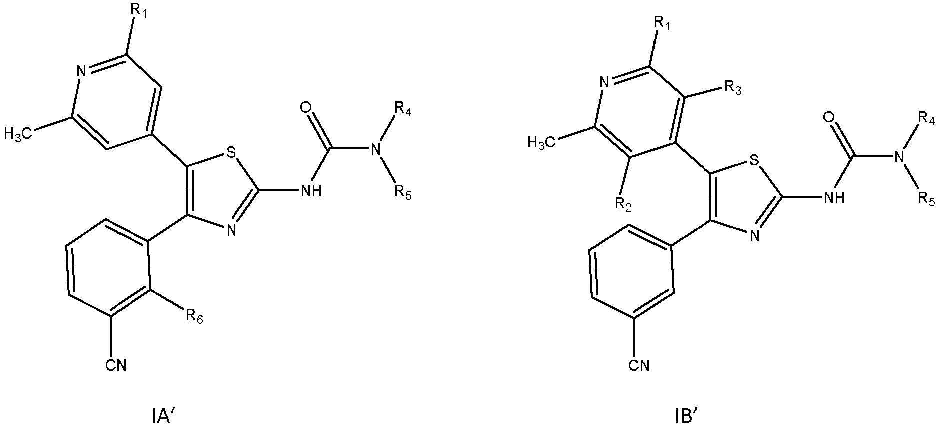 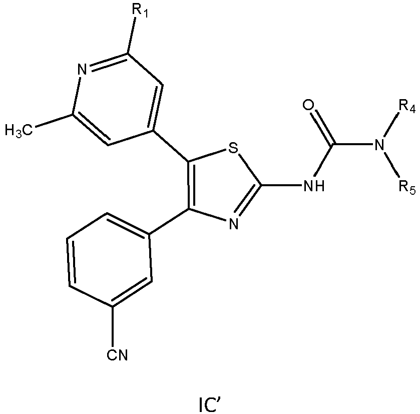 wherein R₁, R₂, R₃, R₄, R₅ and R₆ are each as defined in any one of paragraphs 1 to 12;
14. A compound, or a pharmaceutically acceptable salt thereof, selected from any one of the following:
   N-[5-(2-chloro-6-methyl-4-pyridyl)-4-(3-cyanophenyl)thiazol-2-yl]morpholine-4-carboxamide;
   N-[5-(2-chloro-6-methyl-4-pyridyl)-4-(3-cyanophenyl)thiazol-2-yl]-3-(1-hydroxy-1-methylethyl)pyrrolidine-1-carboxamide;
   (2R)-N-[5-(2-chloro-6-methyl-4-pyridyl)-4-(3-cyanophenyl)thiazol-2-yl]-2-(1-hydroxy-1-methylethyl)pyrrolidine-1-carboxamide;
   N-[5-(2-chloro-6-methyl-4-pyridyl)-4-(3-cyanophenyl)thiazol-2-yl]piperazine-1-carboxamide;
   N-[5-(2-chloro-6-methyl-4-pyridyl)-4-(3-cyanophenyl)thiazol-2-yl]pyrrolidine-1-carboxamide;
   N-[5-(2-chloro-6-methyl-4-pyridyl)-4-(3-cyanophenyl)thiazol-2-yl]-2-(2-hydroxy-2-methylpropyl)pyrrolidine-1-carboxamide;
   N-[5-(2-chloro-6-methyl-4-pyridyl)-4-(3-cyanophenyl)thiazol-2-yl]-3-(1-hydroxy-1-methylethyl)morpholine-4-carboxamide;
   (2S)-4-[[5-(2-chloro-6-methyl-4-pyridyl)-4-(3-cyanophenyl)thiazol-2-yl]carbamoyl]piperazine-2-carboxylic acid;
   N-[5-(2-chloro-6-methyl-4-pyridyl)-4-(3-cyanophenyl)thiazol-2-yl]-4-methyl-piperazine-1-carboxamide;
   N-[5-(2-chloro-6-methyl-4-pyridyl)-4-(3-cyanophenyl)thiazol-2-yl]-4-hydroxy-4-methylpiperidine-1-carboxamide;
   N-[5-(2-chloro-6-methyl-4-pyridyl)-4-(3-cyanophenyl)thiazol-2-yl]-4-cyano-4-methylpiperidine-1-carboxamide;
   (3S)-N-[5-(2-chloro-6-methyl-4-pyridyl)-4-(3-cyanophenyl)thiazol-2-yl]-3-(1-hydroxy-1-methylethyl)piperazine-1-carboxamide formate;
   (3R)-N-[5-(2-chloro-6-methyl-4-pyridyl)-4-(3-cyanophenyl)thiazol-2-yl]-3-(1-hydroxy-1-methylethyl)piperazine-1-carboxamide formate
   N-[5-(2-chloro-6-methyl-4-pyridyl)-4-(3-cyanophenyl)thiazol-2-yl]-4-(oxetan-3-yl)piperazine-1-carboxamide;
   N-[5-(2-chloro-6-methyl-4-pyridyl)-4-(3-cyanophenyl)thiazol-2-yl]-1-imino-1-oxo-1,4-thiazinane-4-carboxamide;
   N-[4-(3-cyanophenyl)-5-(2,6-dimethyl-4-pyridyl)thiazol-2-yl]-4-methyl-piperazine-1-carboxamide;
   4-cyano-N-[4-(3-cyanophenyl)-5-(2,6-dimethyl-4-pyridyl)thiazol-2-yl]piperidine-1-carboxamide;
   N-[4-(3-cyanophenyl)-5-(2,6-dimethyl-4-pyridyl)thiazol-2-yl]-1-imino-1-oxo-1,4-thiazinane-4-carboxamide;
   N-[4-(3-cyanophenyl)-5-(2,6-dimethyl-4-pyridyl)thiazol-2-yl]-4-(2-hydroxyethyl)piperazine-1-carboxamide;
   N-[4-(3-cyanophenyl)-5-(2,6-dimethyl-4-pyridyl)thiazol-2-yl]-4-(oxetan-3-yl)piperazine-1-carboxamide;
   (3S)-N-[4-(3-cyanophenyl)-5-(2,6-dimethyl-4-pyridyl)thiazol-2-yl]-3-hydroxy-pyrrolidine-1-carboxamide;
   4-cyano-N-[4-(3-cyanophenyl)-5-(2,6-dimethyl-4-pyridyl)thiazol-2-yl]-4-(methoxymethyl)piperidine-1-carboxamide;
   4-cyano-N-[4-(3-cyanophenyl)-5-(2,6-dimethyl-4-pyridyl)thiazol-2-yl]-4-fluoro-piperidine-1-carboxamide;
   (3S)-3-cyano-N-[4-(3-cyanophenyl)-5-(2,6-dimethyl-4-pyridyl)thiazol-2-yl]pyrrolidine-1-carboxamide;
   N-[4-(3-cyanophenyl)-5-(2,6-dimethyl-4-pyridyl)thiazol-2-yl]-3-hydroxy-3-methyl-azetidine-1-carboxamide;
   (3R)-3-cyano-N-[4-(3-cyanophenyl)-5-(2,6-dimethyl-4-pyridyl)thiazol-2-yl]pyrrolidine-1-carboxamide;
   N-[4-(3-cyanophenyl)-5-(2,6-dimethyl-4-pyridyl)thiazol-2-yl]-3,4,6,7,9,9a-hexahydro-1H-pyrazino[2,1-c][1,4]oxazine-8-carboxamide;
   N-[4-(3-cyanophenyl)-5-(2,6-dimethyl-4-pyridyl)thiazol-2-yl]-2-oxa-6-azaspiro[3.3]heptane-6-carboxamide;
   N-[4-(3-cyanophenyl)-5-(2,6-dimethyl-4-pyridyl)thiazol-2-yl]-3-oxo-piperazine-1-carboxamide
   N4-[4-(3-cyanophenyl)-5-(2,6-dimethyl-4-pyridyl)thiazol-2-yl]morpholine-2,4-dicarboxamide
   N-[4-(3-cyanophenyl)-5-(2,6-dimethyl-4-pyridyl)thiazol-2-yl]-4-methyl-3-oxo-piperazine-1-carboxamide;
   (2R)-N-[4-(3-cyanophenyl)-5-(2,6-dimethyl-4-pyridyl)thiazol-2-yl]-2-(hydroxymethyl)morpholine-4-carboxamide;
   4-acetyl-N-[4-(3-cyanophenyl)-5-(2,6-dimethyl-4-pyridyl)thiazol-2-yl]piperazine-1-carboxamide;
   N-[4-(3-cyanophenyl)-5-(2,6-dimethyl-4-pyridyl)thiazol-2-yl]-4-methylsulfonyl-piperazine-1-carboxamide;
   N-[4-(3-cyanophenyl)-5-(2,6-dimethyl-4-pyridyl)thiazol-2-yl]-4-sulfamoyl-piperazine-1-carboxamide;
   N-[4-(3-cyanophenyl)-5-(2,6-dimethyl-4-pyridyl)thiazol-2-yl]-2,4-dioxo-1,3,8-triazaspiro[4.5]decane-8-carboxamide;
   (2S)-N-[4-(3-cyanophenyl)-5-(2,6-dimethyl-4-pyridyl)thiazol-2-yl]-2-(hydroxymethyl)morpholine-4-carboxamide;
   4-cyano-N-[4-(3-cyanophenyl)-5-(2,6-dimethyl-4-pyridyl)thiazol-2-yl]-4-methoxy-piperidine-1-carboxamide;
   4-cyano-N-[4-(3-cyanophenyl)-5-(2,6-dimethyl-4-pyridyl)thiazol-2-yl]-4-(hydroxymethyl)piperidine-1-carboxamide;
   N-[4-(3-cyanophenyl)-5-(2,6-dimethyl-4-pyridyl)thiazol-2-yl]-1-oxo-1,4-thiazinane-4-carboxamide;
   N-[4-(3-cyanophenyl)-5-(2,6-dimethyl-4-pyridyl)thiazol-2-yl]-3-(hydroxymethyl)azetidine-1-carboxamide;
   3-cyano-N-[4-(3-cyanophenyl)-5-(2,6-dimethyl-4-pyridyl)thiazol-2-yl]azetidine-1-carboxamide;
   N-[4-(3-cyanophenyl)-5-(2,6-dimethyl-4-pyridyl)thiazol-2-yl]-1,1-dioxo-1,4-thiazinane-4-carboxamide
   N-[4-(3-cyanophenyl)-5-(2,6-dimethyl-4-pyridyl)thiazol-2-yl]-1,3-dimethyl-2,4-dioxo-1,3,8-triazaspiro[4.5]decane-8-carboxamide
   N 1-[4-(3-cyanophenyl)-5-(2,6-dimethyl-4-pyridyl)thiazol-2-yl]piperidine-1,4-dicarboxamide
   N-[4-(3-cyanophenyl)-5-(2,6-dimethyl-4-pyridyl)thiazol-2-yl]-3-hydroxy-azetidine-1-carboxamide
   N-[4-(3-cyanophenyl)-5-(2,6-dimethyl-4-pyridyl)thiazol-2-yl]-4-(2-hydroxyethyl)-3-oxo-piperazine-1-carboxamide;
   4,4-Dicyano-N-[4-(3-cyanophenyl)-5-(2,6-dimethyl-4-pyridyl)thiazol-2-yl]piperidine-1-carboxamide;
   N-[4-(3-cyanophenyl)-5-(2,6-dimethyl-4-pyridyl)thiazol-2-yl]-3-(1-hydroxy-1-methylethyl)azetidine-1-carboxamide;
   (2R)-4-[[5-(2-chloro-6-methyl-4-pyridyl)-4-(3-cyanophenyl)thiazol-2-yl]carbamoyl]piperazine-2-carboxylic acid;
   methyl (2R)-4-[[5-(2-chloro-6-methyl-4-pyridyl)-4-(3-cyanophenyl)thiazol-2-yl]carbamoyl]piperazine-2-carboxylate;
   (3S)-N-[5-(2-chloro-6-methyl-4-pyridyl)-4-(3-cyanophenyl)thiazol-2-yl]-3-(hydroxymethyl)piperazine-1-carboxamide;
   (3R)-N-[5-(2-chloro-6-methyl-4-pyridyl)-4-(3-cyanophenyl)thiazol-2-yl]-3-(hydroxymethyl)piperazine-1-carboxamide;
   (3S)-N-[5-(2-chloro-6-methyl-4-pyridyl)-4-(3-cyanophenyl)thiazol-2-yl]-3-methyl-piperazine-1-carboxamide;
   (3R)-N-[5-(2-chloro-6-methyl-4-pyridyl)-4-(3-cyanophenyl)thiazol-2-yl]-3-methyl-piperazine-1-carboxamide;
   (1S,4S)-N-[5-(2-chloro-6-methyl-4-pyridyl)-4-(3-cyanophenyl)thiazol-2-yl]-2,5-diazabicyclo[2.2.1]heptane-2-carboxamide;
   (3R)-N 1-[5-(2-chloro-6-methyl-4-pyridyl)-4-(3-cyanophenyl)thiazol-2-yl]piperazine-1,3-dicarboxamide;
   (3S)-N1-[5-(2-chloro-6-methyl-4-pyridyl)-4-(3-cyanophenyl)thiazol-2-yl]piperazine-1,3-dicarboxamide;
   4-Amino-N-[5-(2-chloro-6-methyl-4-pyridyl)-4-(3-cyanophenyl)thiazol-2-yl]-4-methylpiperidine-1-carboxamide;
   4-cyano-N-[4-(3-cyanophenyl)-5-(2,6-dimethyl-4-pyridyl)thiazol-2-yl]-4-methyl-piperidine-1-carboxamide;
   N-[4-(3-cyanophenyl)-5-(2,6-dimethyl-4-pyridyl)thiazol-2-yl]piperazine-1-carboxamide;
   (3R)-N-[4-(3-cyanophenyl)-5-(2,6-dimethyl-4-pyridyl)thiazol-2-yl]-3-methyl-piperazine-1-carboxamide;
   4-Amino-4-cyano-N-[4-(3-cyanophenyl)-5-(2,6-dimethyl-4-pyridyl)thiazol-2-yl]piperidine-1-carboxamide; and
   1-acetylimino-N-[4-(3-cyanophenyl)-5-(2,6-dimethyl-4-pyridyl)thiazol-2-yl]-1-oxo-1,4-thiazinane-4-carboxamide.
15. A pharmaceutical composition comprising a compound according to any one of paragraphs 1 to 14, or a pharmaceutically acceptable salt thereof, in admixture with a pharmaceutically acceptable diluent or carrier.
16. A compound according to any one of paragraphs 1 to 14, or a pharmaceutically acceptable salt thereof, or a pharmaceutical composition according to paragraph 15, for use in therapy.
17. A compound according to any one of paragraphs 1 to 14, or a pharmaceutically acceptable salt thereof, or a pharmaceutical composition according to paragraph 15, for use:
   (i) in the treatment of a proliferative condition;
   (ii) in the treatment of cancer;
   (iii) in the treatment of cancer, wherein the compound or pharmaceutical composition is administered in combination with one or more additional anticancer agents;
   (iv) in the treatment of cancer, wherein the compound or pharmaceutical composition is administered in combination with one or more additional anticancer agents selected from the group consisting of:
      other forms of cancer immunotherapy and anti-cancer chemotherapeutic agents;
      A2b antagonists;
      anti-PD-1 and PDL-1 antibodies (e.g. pembrolizumab, nivolumab, durvalumab, avelumab and atezolizumab); and
      anti-CTLA4 antibodies (e.g ipilimumab).
18. A method of treating a proliferative disorder in a patient in need of such treatment, the method comprising administering a therapeutically effective amount of a compound according to any one of paragraphs 1 to 14, or a pharmaceutically acceptable salt thereof, or a pharmaceutical composition according to paragraph 15.
19. A method of treating cancer in a patient in need of such treatment, the method comprising administering a therapeutically effective amount of a compound according to any one of paragraphs 1 to 14, or a pharmaceutically acceptable salt thereof, or a pharmaceutical composition according to paragraph 15.
20. A method of treating a proliferative disorder in a patient in need of such treatment, the method comprising administering a therapeutically effective amount of a compound according to any one of paragraphs 1 to 14, or a pharmaceutically acceptable salt thereof, or a pharmaceutical composition according to paragraph 15 in combination with one or more additional anticancer agents.
21. A method according to paragraph 20, wherein the one or more additional anticancer agents is selected from:
   other forms of cancer immunotherapy and anti-cancer chemotherapeutic agents;
   A2b antagonists;
   anti-PD-1 and PDL-1 antibodies (e.g. pembrolizumab, nivolumab, durvalumab, avelumab and atezolizumab); and
   anti-CTLA4 antibodies (e.g ipilimumab).

## Claims

1. A compound, or pharmaceutically acceptable salt thereof, having the structural formula I shown below: 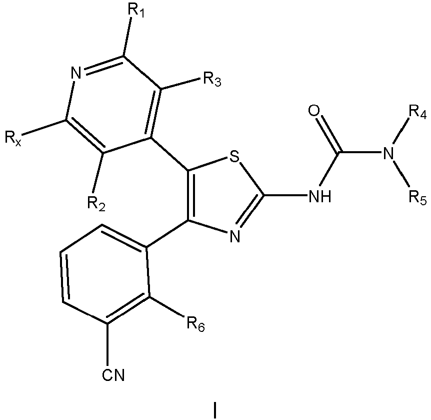 wherein:
Rₓ is selected from methyl, CD₃, chloro and CF₃;
R₁ is selected from (1-3C)alkyl, halo, (1-3C)haloalkyl, (1-3C)haloalkoxy, (1-3C)hydroxyalkyl, cyano, cyclopropyl, oxetan-3-yl, -(CR_{1C}R_{1D})_{q1}-OR_{1A}, -(CR_{1C}R_{1D})_{q1}-C(O)R_{1A}, -(CR_{1C}R_{1D})_{q1}-C(O)OR_{1A}, -(CR_{1C}R_{1D})_{q1}-OC(O)R_{1A}, -( CR_{1C}R_{1D})_{q1}-C(O)N(R_{1B})R_{1A}, -(CR_{1C}R_{1D})_{q1}-N(R_{1B})C(O)R_{1A}, -( CR_{1C}R_{1D})_{q1}-S(O)ₚR_{1A} (where p is 0, 1 or 2), -(CR_{1C}R_{1D})_{q1}-SO₂N(R_{1B})R_{1A}, or -(CR_{1C}R_{1D})_{q1}-N(R_{1B})SO₂R_{1A},
wherein q1 is 0, 1, or 2; and
wherein R_{1A} and R_{1B} are each independently selected from hydrogen, (1-2C)alkyl, (3-4C)cycloalkyl or (3-4C)cycloalkyl(1-2C)alkyl;
wherein R_{1C} and R_{1D} are each independently selected from hydrogen or (1-2C)alkyl;
R₂ and R₃ are both hydrogen or one of R₂ and R₃ is hydrogen and the other is selected from fluoro, methyl or methoxy;
R₄ and R₅ are linked such that, together with the nitrogen atom to which they are attached, they form heterocyclic ring which is optionally substituted on any available carbon atom by one or more R_{10C} substituent groups,
wherein R_{10C} is selected from oxo, thioxo, halo, or cyano substituents, or a group of the formula:
-[CR₇ₐR_{7b}]ₙ-L-Z
wherein
n is 0 to 6;
R₇ₐ and R_{7b} are each independently selected from hydrogen, fluoro or (1-2C)alkyl;
L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -C(S)N(Rₐ)-, -N(Rₐ)C(S)-, -N(Rₐ)C(S)N(R_{b})-, -S(O)₂N(Rₐ)-, or -N(Rₐ)SO₂-, wherein Rₐ and R_{b} are each independently selected from hydrogen or (1-2C)alkyl; and
Z is selected from hydrogen, (1-6C)alkyl, (3-6C)cycloalkyl, (3-6C)cycloalkyl(1-2C)alkyl, aryl, aryl(1-2C)alkyl, heterocyclyl, heterocyclyl(1-2C)alkyl, heteroaryl or heteroaryl(1-2C)alkyl; and wherein Z is optionally substituted by one or more substituents selected from (1-6C)alkyl, halo, (1-6C)haloalkyl, (1-6C)haloalkoxy, cyano, nitro, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1, 2 or 3) or oxo, wherein any alkyl moiety in a substituent group on Z is optionally further substituted by cyano, halo, hydroxy, amino, oxo, (1-2C)alkyl or (1-2C)alkoxy and R_{c} and R_{d} are each independently selected from hydrogen, (1-6C)alkyl, (1-6C)haloalkyl or (3-6C)cycloalkyl;
and wherein any available N atoms are optionally in the form of a N-oxide or are optionally substituted by one or more R_{10N},
wherein R_{10N} is -S(O)₂NH₂ or selected from:
(i) -Z₁;
(ii) -L₁ₐ-Z₁; or
(iii) -[CR₈ₐR_{8b}]₁₋₆-L_{1b}-Z₁;
wherein
R₈ₐ and R_{8b} are each independently selected from hydrogen, fluoro or (1-2C)alkyl;
L₁ₐ is selected from -C(O)-, -S(O)₂-, -C(O)O-, -C(O)N(Rₐ₁)-, -S(O)₂N(Rₐ₁)- or -N(Rₐ₁)-, wherein Rₐ₁ is selected from hydrogen or (1-2C)alkyl;
L_{1b} is absent or selected from -O-, -S-, -SO-, -SO₂₋, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, -N(Rₐ₂)C(S)N(R_{b2})-, -S(O)₂N(Rₐ₂)-, or -N(Rₐ₂)SO₂, wherein Rₐ₂ and R_{b2} are each independently selected from hydrogen or (1-2C)alkyl; and
Z₁ is selected from (1-6C)alkyl, (3-6C)cycloalkyl, aryl, heterocyclyl, heteroaryl, (3-6C)cycloalkyl(1-2C)alkyl, aryl(1-2C)alkyl, heterocyclyl(1-2C)alkyl or heteroaryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-6C)alkyl, halo, (1-6C)haloalkoxy, cyano, nitro, - NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)_{z}NRₑR_{f} (where z is 1, 2 or 3) or oxo, and wherein Rₑ and R_{f} are each independently selected from hydrogen, (1-6C)alkyl, (1-6C)haloalkyl or (3-6C)cycloalkyl;
and wherein any S atoms present in the heterocyclic ring may optionally be present as S(=O), S(=O)₂ or S(=O)(=NRₑ) wherein Rₑ is selected from hydrogen, (1-3C)alkyl or (2-3C)alkanoyl; and
R₆ is selected from hydrogen, halo, methyl, methoxy and trifluoromethyl.

2. A compound according to claim 1, or a pharmaceutically acceptable salt thereof, wherein any one or more of the following statements (A)-(C) applies:
(A) Rₓ is selected from methyl or CD₃;
(B) R₁ is selected from (1-3C)alkyl, halo, (1-3C)haloalkyl, (1-3C)haloalkoxy, (1-3C)hydroxyalkyl, cyano, cyclopropyl, oxetan-3-yl, -(CH₂)_{q1}-OR_{1A} and -(CH₂)_{q1}-C(O)R_{1A};
wherein q1 is 0 or 1; and
wherein R_{1A} is selected from hydrogen or (1-2C)alkyl;
(C) R₁ is selected from methyl, CD₃, isopropyl, halo, (1-3C)hydroxyalkyl, cyano, cyclopropyl, oxetan-3-yl, -OMe, -CH₂F, -CHF₂ or -CF₃.

3. A compound according to any one of the preceding claims, or a pharmaceutically acceptable salt thereof, wherein any one or both of the following statements (A)-(B) applies:
(A) R₂ and R₃ are both hydrogen or one of R₂ and R₃ is hydrogen and the other is fluoro or methyl;
(B) R₂ and R₃ are both hydrogen.

4. A compound according to any one of the preceding claims, or a pharmaceutically acceptable salt thereof, wherein R₄ and R₅ are linked such that, together with the nitrogen atom to which they are attached, they form heterocyclic ring which is optionally substituted on any available carbon atom by one or more R_{10C} substituent groups,
wherein R_{10C} is selected from oxo, halo, or cyano substituents, or a group of the formula:
-[CH₂]ₙ-L-Z
wherein
n is 0 to 2;
L is absent or selected from -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)-, or -N(Rₐ₂)SO₂-, wherein Rₐ₂ and R_{b2} are each independently selected from hydrogen or methyl; and
Z is selected from hydrogen, (1-6C)alkyl, (3-6C)cycloalkyl, (3-6C)cycloalkyl(1-2C)alkyl, aryl, heterocyclyl or heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, -C(O)ORₑ, -OC(O)Rₑ, -C(O)N(R_{c})R_{d}, -N(R_{c})C(O)R_{d}, -S(O)_{y}R_{c} (where y is 0, 1 or 2), -SO₂N(R_{c})R_{d}, -N(R_{c})SO₂R_{d}, -(CH₂)_{z}NR_{c}R_{d} (where z is 1 or 2) or oxo;
wherein any alkyl moiety in a substituent group on Z₁ is optionally further substituted by cyano, halo, hydroxy, amino, oxo, (1-2C)alkyl or (1-2C)alkoxy and R_{c} and R_{d} are each independently selected from hydrogen, (1-2C)alkyl, (1-2C)haloalkyl or (3-6C)cycloalkyl;
and wherein any available N atoms are optionally in the form of a N-oxide or are optionally substituted by one or more R_{10N}, wherein R_{10N} is -S(O)₂NH₂ or selected from:
(i) -Z₁;
(ii) -L₁ₐ-Z₁; or
(iii) -[CR₈ₐR_{8b}]₁₋₂-L_{1b}-Z₁;
wherein
R₈ₐ and R_{8b} are both hydrogen;
L₁ₐ is selected from -C(O)-, -S(O)₂- or -S(O)₂N(Rₐ₁)-, wherein Rₐ₁ is hydrogen or methyl;
L_{1b} is absent or selected from -O-, -S-, -SO-, -SO₂₋, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)-, or -N(Rₐ₂)SO₂, wherein Rₐ₂ and R_{b2} are each independently selected from hydrogen or methyl; and
Z₁ is selected from (1-6C)alkyl, (3-6C)cycloalkyl, aryl, heterocyclyl, heteroaryl, (3-6C)cycloalkyl(1-2C)alkyl or aryl(1-2C)alkyl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, nitro,
-NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (where y is 0, 1 or 2), -SO₂N(Rₑ)R_{f}, -N(Rₑ)SO₂R_{f}, -(CH₂)₂NRₑR_{f} (where z is 1, 2 or 3) or oxo, and wherein Rₑ and R_{f} are each independently selected from hydrogen or (1-2C)alkyl;
and wherein any S atoms present in the heterocyclic ring may optionally be present as S(=O), S(=O)₂ or S(=O)(=NRₑ) wherein Rₑ is selected from hydrogen, (1-2C)alkyl or (2C)alkanoyl.

5. A compound according to any one of the preceding claims, or a pharmaceutically acceptable salt thereof, wherein R₄ and R₅ are linked such that, together with the nitrogen atom to which they are attached, they form heterocyclic ring which is optionally substituted on any available carbon atom by one or more R_{10C} substituent groups,
wherein R_{10C} is selected from oxo, halo, or cyano substituents, or a group of the formula:
-[CH₂]ₙ-L-Z
wherein
n is 0 to 2;
L is absent or selected from -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)-, or -N(Rₐ)SO₂-, wherein Rₐ and R_{b} are hydrogen; and
Z is selected from hydrogen, (1-4C)alkyl, (3-6C)cycloalkyl, phenyl, a 4-7 membered heterocyclyl or a 5 or 6-membered heteroaryl; and wherein Z is optionally substituted by one or more substituents selected from (1-2C)alkyl, halo, (1-2C)haloalkyl, (1-2C)haloalkoxy, cyano, -NR_{c}R_{d}, -OR_{c}, -C(O)R_{c}, - C(O)N(R_{c})R_{d}, -N(R_{c})C(O)R_{d} or -S(O)_{y}R_{c} (where y is 0, 1 or 2) or oxo;
wherein R_{c} and R_{d} are each independently selected from hydrogen or (1-2C)alkyl;
and wherein any available N atoms are optionally in the form of a N-oxide or are optionally substituted by one or more R_{10N}, wherein R_{10N} is -S(O)₂NH₂ or selected from:
(i) -Z₁;
(ii) -L₁ₐ-Z₁; or
(iii) -[CR₈ₐR_{8b}]₁₋₂-L_{1b}-Z₁;
wherein
R₈ₐ and R_{8b} are both hydrogen;
L₁ₐ is selected from -C(O)-, -S(O)₂- or -S(O)₂N(Rₐ₁)-, wherein Rₐ₁ is hydrogen;
L_{1b} is absent or selected from -O-, -S-, -SO-, -SO₂₋, -N(Rₐ₂)-, -C(O)N(Rₐ₂)-, -N(Rₐ₂)C(O)-, -S(O)₂N(Rₐ₂)- or -N(Rₐ₂)SO₂, wherein Rₐ₂ is hydrogen; and
Z₁ is selected from (1-4C)alkyl, phenyl, a 4-7 membered heterocyclyl or a 5 or 6-membered heteroaryl, with the proviso that when Z₁ is a heterocyclyl group directly linked to a N atom it is a carbon-linked heterocyclyl; and wherein Z₁ is optionally substituted by one or more substituents selected from (1-2C)alkyl, halo, (1-2C)haloalkoxy, cyano, -NRₑR_{f}, -ORₑ, -C(O)Rₑ, -C(O)N(Rₑ)R_{f}, -N(Rₑ)C(O)R_{f}, -S(O)_{y}Rₑ (where y is 0, 1 or 2); and wherein Rₑ and R_{f} are each independently selected from hydrogen or methyl;
and wherein any S atoms present in the heterocyclic ring may optionally be present as S(=O), S(=O)₂ or S(=O)(=NRₑ) wherein Rₑ is selected from hydrogen, (1-2C)alkyl or (2C)alkanoyl.

6. A compound according to any one of the preceding claims, or a pharmaceutically acceptable salt thereof, wherein R₄ and R₅ are linked such that, together with the nitrogen atom to which they are attached, they form heterocyclic ring which is optionally substituted on any available carbon atom by one or more R_{10C} substituent groups, wherein R_{10C} is selected from oxo, halo, cyano, (1-4C)alkyl or (3-6C)cycloalkyl;
and wherein any available N atoms are optionally in the form of a N-oxide or are optionally substituted by one or more R_{10N}, wherein R_{10N} is -S(O)₂NH₂ or (1-4C)alkyl,
and wherein any S atoms present in the heterocyclic ring may optionally be present as S(=O), S(=O)₂ or S(=O)(=NRₑ) wherein Rₑ is selected from hydrogen, methyl or (2C)alkanoyl.

7. A compound according to any one of the preceding claims, or a pharmaceutically acceptable salt thereof, wherein R₄ and R₅ are linked such that, together with the nitrogen atom to which they are attached, they form a heterocyclic ring selected from any one of the following options: 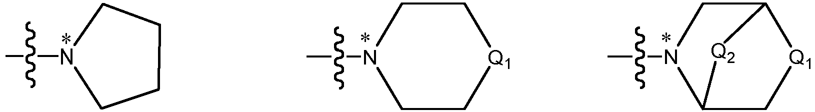 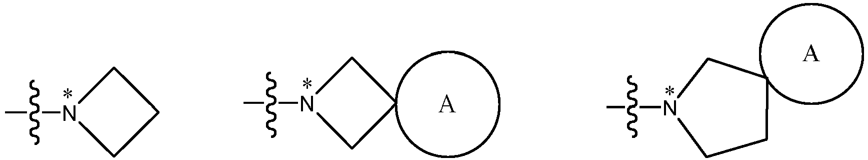 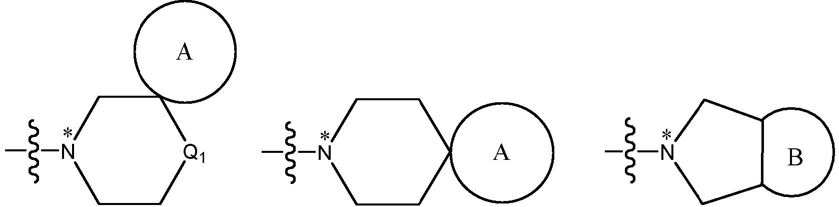 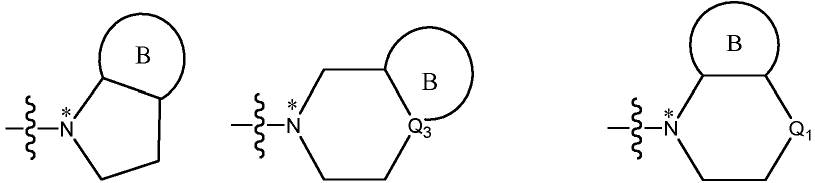 wherein * denotes the N atom to which R₄ and R₅ are attached;
Q₁ is O, NH, S, S(O), S(O)₂, S(O)(=NRₑ), N-R_{10N}, CH₂, CHR_{10C} or C(R_{10c})₂;
Q₂ is -CH₂-, -CHR_{10C}-, -C(R_{10c})₂-, -CHR_{10C}-CH₂-, -CH₂-CHR_{10C}-, -CHR_{10C}-CHR_{10C}-, -C(R_{10c})₂-CH₂-, or -CH₂-C(R_{10c})₂-;
Q₃ is CH, CR_{10C} or N;
Ring A is a spiro-fused 4, 5 or 6 membered carbocyclic or heterocyclic ring;
Ring B is a fused 4, 5 or 6 membered carbocyclic or heterocyclic ring;
wherein Rₑ is selected from hydrogen, (1-2C)alkyl or (2C)alkanoyl;
and wherein each of the heterocyclic ring systems is optionally substituted with one or more R_{10c} or R_{10N} substituent groups as defined in any one of claims 1, 4 or 5.

8. A compound according to any one of the preceding claims, or a pharmaceutically acceptable salt thereof, wherein R₄ and R₅ are linked such that, together with the nitrogen atom to which they are attached, they form a heterocyclic ring selected from any one of the following options: 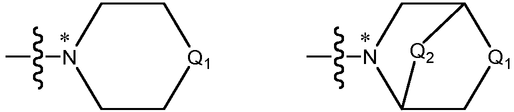
wherein * denotes the N atom to which R₄ and R₅ are attached;
Q₁ is O, NH, S, S(O), S(O)₂, S(O)(=NRₑ), N-R_{10N}, CH₂, CHR_{10C} or C(R_{10c})₂;
Q₂ is -CH₂-;
wherein Rₑ is selected from hydrogen, methyl, or (2C)alkanoyl;
and R_{10c} or R_{10N} are as defined in any one of claims 1, 4 or 5.

9. A compound according to any one of the preceding claims, or a pharmaceutically acceptable salt thereof, wherein R₄ and R₅ are linked such that, together with the nitrogen atom to which they are attached, they form a heterocyclic ring of the structure: 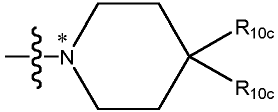
wherein * denotes the N atom to which R₄ and R₅ are attached;
and each R_{10c} is independently selected from the options defined in any one of claims 1, 4 or 5 above.

10. A compound according to any one of the preceding claims, or a pharmaceutically acceptable salt thereof, wherein any one or both of the following statements (A)-(B) applies:
(A) R₆ is selected from hydrogen, halo or methyl;
(B) R₆ is hydrogen.

11. A compound according to any one of the preceding claims, or a pharmaceutically acceptable salt thereof, wherein the compound is of the formula IA, IB or IC: 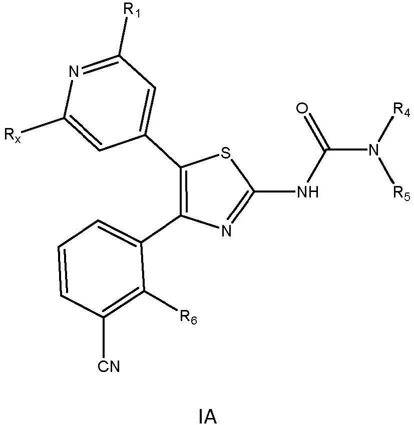 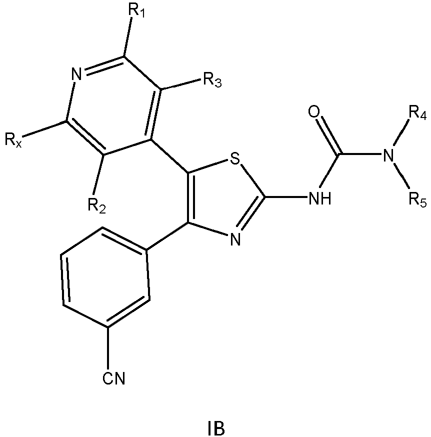 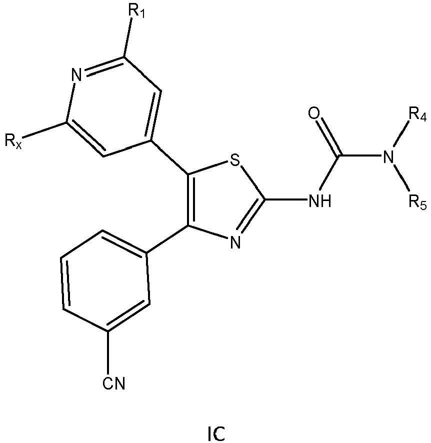 wherein Rₓ, R₁, R₂, R₃, R₄, R₅ and R₆ are each as defined in any one of claims 1 to 10.

12. A compound according to claim 1, or a pharmaceutically acceptable salt thereof, selected from any one of the following:
N-[5-(2-chloro-6-methyl-4-pyridyl)-4-(3-cyanophenyl)thiazol-2-yl]morpholine-4-carboxamide
N-[5-(2-chloro-6-methyl-4-pyridyl)-4-(3-cyanophenyl)thiazol-2-yl]-3-(1-hydroxy-1-methylethyl)pyrrolidine-1-carboxamide
(2R)-N-[5-(2-chloro-6-methyl-4-pyridyl)-4-(3-cyanophenyl)thiazol-2-yl]-2-(1-hydroxy-1-methylethyl)pyrrolidine-1-carboxamide
N-[5-(2-chloro-6-methyl-4-pyridyl)-4-(3-cyanophenyl)thiazol-2-yl]piperazine-1-carboxamide (3R)-N-[5-(2-chloro-6-methyl-4-pyridyl)-4-(3-cyanophenyl)thiazol-2-yl]-3-(1-hydroxy-1-methylethyl)piperazine-1-carboxamide
N-[5-(2-chloro-6-methyl-4-pyridyl)-4-(3-cyanophenyl)thiazol-2-yl]-4-(oxetan-3-yl)piperazine-1-carboxamide
N-[5-(2-chloro-6-methyl-4-pyridyl)-4-(3-cyanophenyl)thiazol-2-yl]-1-imino-1-oxo-1,4-thiazinane-4-carboxamide
N-[5-(2-chloro-6-methyl-4-pyridyl)-4-(3-cyanophenyl)thiazol-2-yl]pyrrolidine-1-carboxamide
N-[5-(2-chloro-6-methyl-4-pyridyl)-4-(3-cyanophenyl)thiazol-2-yl]-2-(2-hydroxy-2-methylpropyl)pyrrolidine-1-carboxamide
N-[5-(2-chloro-6-methyl-4-pyridyl)-4-(3-cyanophenyl)thiazol-2-yl]-3-(1-hydroxy-1-methylethyl)morpholine-4-carboxamide
(2S)-4-[[5-(2-chloro-6-methyl-4-pyridyl)-4-(3-cyanophenyl)thiazol-2-yl]carbamoyl]piperazine-2-carboxylic acid
N-[5-(2-chloro-6-methyl-4-pyridyl)-4-(3-cyanophenyl)thiazol-2-yl]-4-methyl-piperazine-1-carboxamide
N-[5-(2-chloro-6-methyl-4-pyridyl)-4-(3-cyanophenyl)thiazol-2-yl]-4-hydroxy-4-methylpiperidine-1-carboxamide
N-[5-(2-chloro-6-methyl-4-pyridyl)-4-(3-cyanophenyl)thiazol-2-yl]-4-cyano-4-methylpiperidine-1-carboxamide
(3S)-N-[5-(2-chloro-6-methyl-4-pyridyl)-4-(3-cyanophenyl)thiazol-2-yl]-3-(1-hydroxy-1-methylethyl)piperazine-1-carboxamide
N-[4-(3-cyanophenyl)-5-(2,6-dimethyl-4-pyridyl)thiazol-2-yl]-4-methyl-piperazine-1-carboxamide
4-cyano-N-[4-(3-cyanophenyl)-5-(2,6-dimethyl-4-pyridyl)thiazol-2-yl]piperidine-1-carboxamide
(3R)-3-cyano-N-[4-(3-cyanophenyl)-5-(2,6-dimethyl-4-pyridyl)thiazol-2-yl]pyrrolidine-1-carboxamide
N-[4-(3-cyanophenyl)-5-(2,6-dimethyl-4-pyridyl)thiazol-2-yl]-3,4,6,7,9,9a-hexahydro-1H-pyrazino[2,1-c][1,4]oxazine-8-carboxamide
N-[4-(3-cyanophenyl)-5-(2,6-dimethyl-4-pyridyl)thiazol-2-yl]-2-oxa-6-azaspiro[3.3]heptane-6-carboxamide
N-[4-(3-cyanophenyl)-5-(2,6-dimethyl-4-pyridyl)thiazol-2-yl]-3-oxo-piperazine-1-carboxamide
N4-[4-(3-cyanophenyl)-5-(2,6-dimethyl-4-pyridyl)thiazol-2-yl]morpholine-2,4-dicarboxamide
N-[4-(3-cyanophenyl)-5-(2,6-dimethyl-4-pyridyl)thiazol-2-yl]-4-methyl-3-oxo-piperazine-1-carboxamide
(2R)-N-[4-(3-cyanophenyl)-5-(2,6-dimethyl-4-pyridyl)thiazol-2-yl]-2-(hydroxymethyl)morpholine-4-carboxamide
4-acetyl-N-[4-(3-cyanophenyl)-5-(2,6-dimethyl-4-pyridyl)thiazol-2-yl]piperazine-1-carboxamide
N-[4-(3-cyanophenyl)-5-(2,6-dimethyl-4-pyridyl)thiazol-2-yl]-4-methylsulfonyl-piperazine-1-carboxamide
N-[4-(3-cyanophenyl)-5-(2,6-dimethyl-4-pyridyl)thiazol-2-yl]-4-sulfamoyl-piperazine-1-carboxamide
N-[4-(3-cyanophenyl)-5-(2,6-dimethyl-4-pyridyl)thiazol-2-yl]-1-imino-1-oxo-1,4-thiazinane-4-carboxamide
N-[4-(3-cyanophenyl)-5-(2,6-dimethyl-4-pyridyl)thiazol-2-yl]-2,4-dioxo-1,3,8-triazaspiro[4.5]decane-8-carboxamide
(2S)-N-[4-(3-cyanophenyl)-5-(2,6-dimethyl-4-pyridyl)thiazol-2-yl]-2-(hydroxymethyl)morpholine-4-carboxamide
4-cyano-N-[4-(3-cyanophenyl)-5-(2,6-dimethyl-4-pyridyl)thiazol-2-yl]-4-methoxy-piperidine-1-carboxamide
4-cyano-N-[4-(3-cyanophenyl)-5-(2,6-dimethyl-4-pyridyl)thiazol-2-yl]-4-(hydroxymethyl)piperidine-1-carboxamide
N-[4-(3-cyanophenyl)-5-(2,6-dimethyl-4-pyridyl)thiazol-2-yl]-1-oxo-1,4-thiazinane-4-carboxamide
N-[4-(3-cyanophenyl)-5-(2,6-dimethyl-4-pyridyl)thiazol-2-yl]-3-(hydroxymethyl)azetidine-1-carboxamide
3-cyano-N-[4-(3-cyanophenyl)-5-(2,6-dimethyl-4-pyridyl)thiazol-2-yl]azetidine-1-carboxamide
N-[4-(3-cyanophenyl)-5-(2,6-dimethyl-4-pyridyl)thiazol-2-yl]-1,1-dioxo-1,4-thiazinane-4-carboxamide
N-[4-(3-cyanophenyl)-5-(2,6-dimethyl-4-pyridyl)thiazol-2-yl]-1,3-dimethyl-2,4-dioxo-1,3,8-triazaspiro[4.5]decane-8-carboxamide
N 1-[4-(3-cyanophenyl)-5-(2,6-dimethyl-4-pyridyl)thiazol-2-yl]piperidine-1,4-dicarboxamide
N-[4-(3-cyanophenyl)-5-(2,6-dimethyl-4-pyridyl)thiazol-2-yl]-4-(2-hydroxyethyl)piperazine-1-carboxamide
N-[4-(3-cyanophenyl)-5-(2,6-dimethyl-4-pyridyl)thiazol-2-yl]-3-hydroxy-azetidine-1-carboxamide
N-[4-(3-cyanophenyl)-5-(2,6-dimethyl-4-pyridyl)thiazol-2-yl]-4-(2-hydroxyethyl)-3-oxo-piperazine-1-carboxamide
4,4-dicyano-N-[4-(3-cyanophenyl)-5-(2,6-dimethyl-4-pyridyl)thiazol-2-yl]piperidine-1-carboxamide
N-[4-(3-cyanophenyl)-5-(2,6-dimethyl-4-pyridyl)thiazol-2-yl]-3-(1-hydroxy-1-methylethyl)azetidine-1-carboxamide
N-[4-(3-cyanophenyl)-5-(2,6-dimethyl-4-pyridyl)thiazol-2-yl]-4-(2-hydroxy-2-methylpropyl)piperazine-1-carboxamide
N-[4-(3-cyanophenyl)-5-(2,6-dimethyl-4-pyridyl)thiazol-2-yl]-1-methyl-1,7-diazaspiro[3.4]octane-7-carboxamide
N-[4-(3-cyanophenyl)-5-(2,6-dimethyl-4-pyridyl)thiazol-2-yl]-4-(2-hydroxy-1,1-dimethylethyl)piperazine-1-carboxamide
(8aS)-N-[4-(3-cyanophenyl)-5-(2,6-dimethyl-4-pyridyl)thiazol-2-yl]-3,4,6,7,8,8a-hexahydro-1H-pyrrolo[1,2-a]pyrazine-2-carboxamide
(8aR)-N-[4-(3-cyanophenyl)-5-(2,6-dimethyl-4-pyridyl)thiazol-2-yl]-3,4,6,7,8,8a-hexahydro-1H-pyrrolo[1,2-a]pyrazine-2-carboxamide
N-[4-(3-cyanophenyl)-5-(2,6-dimethyl-4-pyridyl)thiazol-2-yl]-4-(oxetan-3-yl)piperazine-1-carboxamide
(3S)-N-[4-(3-cyanophenyl)-5-(2,6-dimethyl-4-pyridyl)thiazol-2-yl]-3-hydroxy-pyrrolidine-1-carboxamide
4-cyano-N-[4-(3-cyanophenyl)-5-(2,6-dimethyl-4-pyridyl)thiazol-2-yl]-4-(methoxymethyl)piperidine-1-carboxamide
4-cyano-N-[4-(3-cyanophenyl)-5-(2,6-dimethyl-4-pyridyl)thiazol-2-yl]-4-fluoro-piperidine-1-carboxamide
(3S)-3-cyano-N-[4-(3-cyanophenyl)-5-(2,6-dimethyl-4-pyridyl)thiazol-2-yl]pyrrolidine-1-carboxamide
N-[4-(3-cyanophenyl)-5-(2,6-dimethyl-4-pyridyl)thiazol-2-yl]-3-hydroxy-3-methyl-azetidine-1-carboxamide
(2R)-4-[[5-(2-chloro-6-methyl-4-pyridyl)-4-(3-cyanophenyl)thiazol-2-yl]carbamoyl]piperazine-2-carboxylic acid
methyl (2R)-4-[[5-(2-chloro-6-methyl-4-pyridyl)-4-(3-cyanophenyl)thiazol-2-yl]carbamoyl]piperazine-2-carboxylate
(3S)-N-[5-(2-chloro-6-methyl-4-pyridyl)-4-(3-cyanophenyl)thiazol-2-yl]-3-(hydroxymethyl)piperazine-1-carboxamide
(3R)-N-[5-(2-chloro-6-methyl-4-pyridyl)-4-(3-cyanophenyl)thiazol-2-yl]-3-(hydroxymethyl)piperazine-1-carboxamide
(3S)-N-[5-(2-chloro-6-methyl-4-pyridyl)-4-(3-cyanophenyl)thiazol-2-yl]-3-methyl-piperazine-1-carboxamide
(3R)-N-[5-(2-chloro-6-methyl-4-pyridyl)-4-(3-cyanophenyl)thiazol-2-yl]-3-methyl-piperazine-1-carboxamide
(1S,4S)-N-[5-(2-chloro-6-methyl-4-pyridyl)-4-(3-cyanophenyl)thiazol-2-yl]-2,5-diazabicyclo[2.2.1]heptane-2-carboxamide
(3R)-N1-[5-(2-chloro-6-methyl-4-pyridyl)-4-(3-cyanophenyl)thiazol-2-yl]piperazine-1,3-dicarboxamide
(3S)-N1-[5-(2-chloro-6-methyl-4-pyridyl)-4-(3-cyanophenyl)thiazol-2-yl]piperazine-1,3-dicarboxamide
4-amino-N-[5-(2-chloro-6-methyl-4-pyridyl)-4-(3-cyanophenyl)thiazol-2-yl]-4-methylpiperidine-1-carboxamide
4-cyano-N-[4-(3-cyanophenyl)-5-(2,6-dimethyl-4-pyridyl)thiazol-2-yl]-4-methyl-piperidine-1-carboxamide
(3S)-N-[4-(3-cyanophenyl)-5-(2,6-dimethyl-4-pyridyl)thiazol-2-yl]-3-methylsulfonyl-pyrrolidine-1-carboxamide
N-[4-(3-cyanophenyl)-5-(2,6-dimethyl-4-pyridyl)thiazol-2-yl]-2-oxa-7-azaspiro[3.5]nonane-7-carboxamide
N-[5-(2-chloro-6-methyl-4-pyridyl)-4-(3-cyanophenyl)thiazol-2-yl]-2-oxa-6-azaspiro[3.3]heptane-6-carboxamide
(1R,4R)-5-acetyl-N-[4-(3-cyanophenyl)-5-(2,6-dimethyl-4-pyridyl)thiazol-2-yl]-2,5-diazabicyclo[2.2.1]heptane-2-carboxamide
(1S,4S)-N-[4-(3-cyanophenyl)-5-(2,6-dimethyl-4-pyridyl)thiazol-2-yl]-5-methyl-2,5-diazabicyclo[2.2.1]heptane-2-carboxamide
N-[4-(3-cyanophenyl)-5-(2,6-dimethyl-4-pyridyl)thiazol-2-yl]-3-methyl-2-oxo-1,3,8-triazaspiro[4.5]decane-8-carboxamide
N-[5-[2,6-bis(trideuteriomethyl)-4-pyridyl]-4-(3-cyanophenyl)thiazol-2-yl]-2-oxa-6-azaspiro[3.3]heptane-6-carboxamide
N-[4-(3-cyanophenyl)-5-(3-fluoro-2,6-dimethyl-4-pyridyl)thiazol-2-yl]-2-oxa-6-azaspiro[3.3]heptane-6-carboxamide
(4aS,7aR)-N-[4-(3-cyanophenyl)-5-[2-(difluoromethyl)-6-methyl-4-pyridyl]thiazol-2-yl]-4-methyl-2,3,4a,5,7,7a-hexahydropyrrolo[3,4-b][1,4]oxazine-6-carboxamide
N-[4-(3-cyanophenyl)-5-[2-(difluoromethyl)-6-methyl-4-pyridyl]thiazol-2-yl]-9-methyl-6-oxa-2,9-diazaspiro[4.5]decane-2-carboxamide
N-[4-(3-cyanophenyl)-5-[2-(difluoromethyl)-6-methyl-4-pyridyl]thiazol-2-yl]-6-oxa-2,9-diazaspiro[4.5]decane-2-carboxamide
(4aS,7aS)-N-[4-(3-cyanophenyl)-5-[2-(difluoromethyl)-6-methyl-4-pyridyl]thiazol-2-yl]-4-methyl-2,3,4a,5,7,7a-hexahydropyrrolo[3,4-b][1,4]oxazine-6-carboxamide
N-[4-(3-cyanophenyl)-5-(2,6-dimethyl-4-pyridyl)thiazol-2-yl]-2-oxo-1,3,8-triazaspiro[4.5]decane-8-carboxamide
3-cyano-N-[4-(3-cyanophenyl)-5-[2-methyl-6-(oxetan-3-yl)-4-pyridyl]thiazol-2-yl]-3-methyl-pyrrolidine-1-carboxamide
4-cyano-N-[4-(3-cyanophenyl)-5-[2-methyl-6-(oxetan-3-yl)-4-pyridyl]thiazol-2-yl]-4-methylpiperidine-1-carboxamide
N-[4-(3-cyanophenyl)-5-(2,6-dimethyl-4-pyridyl)thiazol-2-yl]-3-morpholino-azetidine-1-carboxamide
N-[4-(3-cyanophenyl)-5-(2,6-dimethyl-4-pyridyl)thiazol-2-yl]-4-tetrahydropyran-4-yl-piperazine-1-carboxamide
N-[4-(3-cyanophenyl)-5-(2,6-dimethyl-4-pyridyl)thiazol-2-yl]-3-pyrrolidin-1-yl-azetidine-1-carboxamide
N-[4-(3-cyanophenyl)-5-(2,6-dimethyl-4-pyridyl)thiazol-2-yl]-3-(dimethylamino)azetidine-1-carboxamide
N-[4-(3-cyanophenyl)-5-(2,6-dimethyl-4-pyridyl)thiazol-2-yl]-4-(morpholinomethyl)piperidine-1-carboxamide
N-[4-(3-cyanophenyl)-5-(2,6-dimethyl-4-pyridyl)thiazol-2-yl]-3-methyl-2-oxo-1-oxa-3,8-diazaspiro[4.5]decane-8-carboxamide
N-[4-(3-cyanophenyl)-5-(2,6-dimethyl-4-pyridyl)thiazol-2-yl]-3-methyl-3,6-diazabicyclo[3.2.0]heptane-6-carboxamide
N-[4-(3-cyanophenyl)-5-(2,6-dimethyl-4-pyridyl)thiazol-2-yl]-1,4-diazabicyclo[3.2.1]octane-4-carboxamide
N-[4-(3-cyanophenyl)-5-(2,6-dimethyl-4-pyridyl)thiazol-2-yl]-3-oxo-5,6,8,8a-tetrahydro-1H-oxazolo[3,4-a]pyrazine-7-carboxamide
N-[4-(3-cyanophenyl)-5-(2,6-dimethyl-4-pyridyl)thiazol-2-yl]-2,2-dioxo-3,4,6,7,9,9a-hexahydro-1H-pyrazino[2,1-c][1,4]thiazine-8-carboxamide
N-[4-(3-cyanophenyl)-5-(2,6-dimethyl-4-pyridyl)thiazol-2-yl]-4-oxo-3,6,7,8,9,9a-hexahydro-1H-pyrido[1,2-a]pyrazine-2-carboxamide
N-[4-(3-cyanophenyl)-5-(2,6-dimethyl-4-pyridyl)thiazol-2-yl]-9-oxo-3,4,6,7,8,9a-hexahydro-1H-pyrazino[1,2-a]pyrazine-2-carboxamide
N-[4-(3-cyanophenyl)-5-(2,6-dimethyl-4-pyridyl)thiazol-2-yl]-6-oxo-1,3,4,8,9,9a-hexahydropyrazino[1,2-c][1,3]oxazine-2-carboxamide
N-[4-(3-cyanophenyl)-5-(2,6-dimethyl-4-pyridyl)thiazol-2-yl]-6-oxo-1,3,4,7,8,8a-hexahydropyrrolo[1,2-a]pyrazine-2-carboxamide
N-[4-(3-cyanophenyl)-5-[2-(difluoromethyl)-6-methyl-4-pyridyl]thiazol-2-yl]-1-oxo-1,4-thiazinane-4-carboxamide
N-[4-(3-cyanophenyl)-5-(2,6-dimethyl-4-pyridyl)thiazol-2-yl]-3-sulfamoyl-pyrrolidine-1-carboxamide
N-[4-(3-cyanophenyl)-5-(2,6-dimethyl-4-pyridyl)thiazol-2-yl]-3-oxo-2,7-diazaspiro[3.5]nonane-7-carboxamide
N-[4-(3-cyanophenyl)-5-(2,6-dimethyl-4-pyridyl)thiazol-2-yl]-4-pyrazol-1-yl-piperidine-1-carboxamide
N-[4-(3-cyanophenyl)-5-(2,6-dimethyl-4-pyridyl)thiazol-2-yl]-4-(1,2,4-oxadiazol-3-yl)piperidine-1-carboxamide
N-[4-(3-cyanophenyl)-5-(2,6-dimethyl-4-pyridyl)thiazol-2-yl]-1,3-dioxo-5,6,8,8a-tetrahydroimidazo[1,5-a]pyrazine-7-carboxamide
N-[4-(3-cyanophenyl)-5-(2,6-dimethyl-4-pyridyl)thiazol-2-yl]-1-oxo-2,8-diazaspiro[4.5]decane-8-carboxamide
N-[4-(3-cyanophenyl)-5-(2,6-dimethyl-4-pyridyl)thiazol-2-yl]-4-(1H-imidazol-2-yl)piperidine-1-carboxamide
N-[4-(3-cyanophenyl)-5-(2,6-dimethyl-4-pyridyl)thiazol-2-yl]-10-oxo-3,9-diazaspiro[5.5]undecane-3-carboxamide
N-[4-(3-cyanophenyl)-5-(2,6-dimethyl-4-pyridyl)thiazol-2-yl]-6,8-dihydro-5H-imidazo[1,2-a]pyrazine-7-carboxamide
N 1-[4-(3-cyanophenyl)-5-(2,6-dimethyl-4-pyridyl)thiazol-2-yl]-N4-methyl-piperidine-1,4-dicarboxamide
N-[4-(3-cyanophenyl)-5-(2,6-dimethyl-4-pyridyl)thiazol-2-yl]-8-oxa-2-azaspiro[4.5]decane-2-carboxamide
(3S)-N1-[4-(3-cyanophenyl)-5-(2,6-dimethyl-4-pyridyl)thiazol-2-yl]pyrrolidine-1 ,3-dicarboxamide
N-[4-(3-cyanophenyl)-5-(2,6-dimethyl-4-pyridyl)thiazol-2-yl]-4-(2-oxoimidazolidin-1-yl)piperidine-1-carboxamide
N-[4-(3-cyanophenyl)-5-(2,6-dimethyl-4-pyridyl)thiazol-2-yl]-4-(1,2,4-oxadiazol-5-yl)piperidine-1-carboxamide
N-[4-(3-cyanophenyl)-5-(2,6-dimethyl-4-pyridyl)thiazol-2-yl]-1-methylimino-1-oxo-1,4-thiazinane-4-carboxamide
N 1-[4-(3-cyanophenyl)-5-(2,6-dimethyl-4-pyridyl)thiazol-2-yl]-N4-(2-hydroxyethyl)piperidine-1,4-dicarboxamide
N-[4-(3-cyanophenyl)-5-(2,6-dimethyl-4-pyridyl)thiazol-2-yl]-6-oxo-7-oxa-2,5-diazaspiro[3.5]nonane-2-carboxamide
N-[4-(3-cyanophenyl)-5-(2,6-dimethyl-4-pyridyl)thiazol-2-yl]-6,9-dioxo-1,3,4,7,8,9a-hexahydropyrazino[1,2-a]pyrazine-2-carboxamide
(3R)-N-[4-(3-cyanophenyl)-5-(2,6-dimethyl-4-pyridyl)thiazol-2-yl]-3-(1-hydroxy-1-methylethyl)pyrrolidine-1-carboxamide
N-[4-(3-cyanophenyl)-5-(2,6-dimethyl-4-pyridyl)thiazol-2-yl]-4-oxo-6,7,9,9a-tetrahydro-1H-pyrazino[2,1-c][1,4]oxazine-8-carboxamide
N-[4-(3-cyanophenyl)-5-(2,6-dimethyl-4-pyridyl)thiazol-2-yl]-2-methyl-3-oxo-2,8-diazaspiro[4.5]decane-8-carboxamide
N-[4-(3-cyanophenyl)-5-(2,6-dimethyl-4-pyridyl)thiazol-2-yl]-8-methyl-6,9-dioxo-3,4,7,9a-tetrahydro-1H-pyrazino[1,2-a]pyrazine-2-carboxamide
N-[4-(3-cyanophenyl)-5-(2,6-dimethyl-4-pyridyl)thiazol-2-yl]-3-oxo-2,8-diazaspiro[4.5]decane-8-carboxamide
N-[4-(3-cyanophenyl)-5-(2,6-dimethyl-4-pyridyl)thiazol-2-yl]-3-oxo-2,9-diazaspiro[5.5]undecane-9-carboxamide
4-cyano-N-[4-(3-cyanophenyl)-5-(2,6-dimethyl-4-pyridyl)thiazol-2-yl]-4-(1-hydroxy-1-methylethyl)piperidine-1-carboxamide
N-[4-(3-cyanophenyl)-5-(2,6-dimethyl-4-pyridyl)thiazol-2-yl]-6-oxo-3,4,7,8,9,9a-hexahydro-1H-pyrazino[1,2-c]pyrimidine-2-carboxamide
N-[4-(3-cyanophenyl)-5-(2,6-dimethyl-4-pyridyl)thiazol-2-yl]-6-oxo-3,4,7,8,9,9a-hexahydro-1H-pyrido[1,2-a]pyrazine-2-carboxamide
N-[4-(3-cyanophenyl)-5-(2,6-dimethyl-4-pyridyl)thiazol-2-yl]-6-hydroxy-6-methyl-2-azaspiro[3.3]heptane-2-carboxamide
N-[4-(3-cyanophenyl)-5-(2,6-dimethyl-4-pyridyl)thiazol-2-yl]-2-oxo-1,8-diazaspiro[4.5]decane-8-carboxamide
N-[4-(3-cyanophenyl)-5-(2,6-dimethyl-4-pyridyl)thiazol-2-yl]-2,2-dioxo-2λ^{6}-thia-6-azaspiro[3.3]heptane-6-carboxamide
N-[4-(3-cyanophenyl)-5-(2,6-dimethyl-4-pyridyl)thiazol-2-yl]-2-(1-hydroxy-1-methylethyl)morpholine-4-carboxamide
(3S)-N-[4-(3-cyanophenyl)-5-(2,6-dimethyl-4-pyridyl)thiazol-2-yl]-3-(1-hydroxy-1-methylethyl)pyrrolidine-1-carboxamide
(3aR,6aS)-N-[4-(3-cyanophenyl)-5-(2,6-dimethyl-4-pyridyl)thiazol-2-yl]-1,3,3a,4,6,6a-hexahydrofuro[3,4-c]pyrrole-5-carboxamide
N-[4-(3-cyanophenyl)-5-(2,6-dimethyl-4-pyridyl)thiazol-2-yl]-2-oxo-3-oxa-1,9-diazaspiro[5.5]undecane-9-carboxamide
(1S,5S)-N-[4-(3-cyanophenyl)-5-(2,6-dimethyl-4-pyridyl)thiazol-2-yl]-6-methyl-3,6-diazabicyclo[3.2.2]nonane-3-carboxamide
N-[4-(3-cyanophenyl)-5-(2,6-dimethyl-4-pyridyl)thiazol-2-yl]-1-methyl-2-oxo-1,3,8-triazaspiro[4.5]decane-8-carboxamide
(3aS,6aS)-N-[4-(3-cyanophenyl)-5-(2,6-dimethyl-4-pyridyl)thiazol-2-yl]-3-oxo-1,2,3a,4,6,6a-hexahydropyrrolo[3,4-c]pyrrole-5-carboxamide
N-[4-(3-cyanophenyl)-5-(2,6-dimethyl-4-pyridyl)thiazol-2-yl]-6-oxa-2-azaspiro[3.4]octane-2-carboxamide
N-[4-(3-cyanophenyl)-5-(2,6-dimethyl-4-pyridyl)thiazol-2-yl]-7-oxa-2-azaspiro[3.5]nonane-2-carboxamide
N-[4-(3-cyanophenyl)-5-(2,6-dimethyl-4-pyridyl)thiazol-2-yl]-6-oxo-2,7-diazaspiro[3.4]octane-2-carboxamide
N-[4-(3-cyanophenyl)-5-(2,6-dimethyl-4-pyridyl)thiazol-2-yl]-2-oxo-1-oxa-3,8-diazaspiro[4.5]decane-8-carboxamide
N-[4-(3-cyanophenyl)-5-(2,6-dimethyl-4-pyridyl)thiazol-2-yl]-6-oxo-2,5,7-triazaspiro[3.4]octane-2-carboxamide
N-[4-(3-cyanophenyl)-5-(2,6-dimethyl-4-pyridyl)thiazol-2-yl]-2-oxo-1,9-diazaspiro[5.5]undecane-9-carboxamide
N-[4-(3-cyanophenyl)-5-(2,6-dimethyl-4-pyridyl)thiazol-2-yl]-5-oxo-2,6-diazaspiro[3.4]octane-2-carboxamide
N-[4-(3-cyanophenyl)-5-(2,6-dimethyl-4-pyridyl)thiazol-2-yl]-3-methyl-2,4-dioxo-1,3,8-triazaspiro[4.5]decane-8-carboxamide
N-[4-(3-cyanophenyl)-5-(2,6-dimethyl-4-pyridyl)thiazol-2-yl]-4-ethyl-piperazine-1-carboxamide
N-[4-(3-cyanophenyl)-5-[2-methyl-6-(trifluoromethyl)-4-pyridyl]thiazol-2-yl]-4-(oxetan-3-yl)piperazine-1-carboxamide
1-[[4-(3-cyanophenyl)-5-(2,6-dimethyl-4-pyridyl)thiazol-2-yl]carbamoyl]piperidine-4-carboxylic acid
(3S)-1-[[4-(3-cyanophenyl)-5-(2,6-dimethyl-4-pyridyl)thiazol-2-yl]carbamoyl]pyrrolidine-3-carboxylic acid
3-cyano-N-[4-(3-cyanophenyl)-5-(2,6-dimethyl-4-pyridyl)thiazol-2-yl]-3-(hydroxymethyl)azetidine-1-carboxamide
(3R)-1-[[4-(3-cyanophenyl)-5-(2,6-dimethyl-4-pyridyl)thiazol-2-yl]carbamoyl]pyrrolidine-3-carboxylic acid
4-cyano-N-[4-(3-cyanophenyl)-5-(3-fluoro-2,6-dimethyl-4-pyridyl)thiazol-2-yl]-4-methylpiperidine-1-carboxamide
N-[4-(3-cyanophenyl)-5-(3-fluoro-2,6-dimethyl-4-pyridyl)thiazol-2-yl]-3-hydroxy-3-methyl-azetidine-1-carboxamide
3-cyano-N-[4-(3-cyanophenyl)-5-(3-fluoro-2,6-dimethyl-4-pyridyl)thiazol-2-yl]-3-methyl-azetidine-1-carboxamide
N-[4-(3-cyanophenyl)-5-(3-fluoro-2,6-dimethyl-4-pyridyl)thiazol-2-yl]-3-oxo-2,7-diazaspiro[3.5]nonane-7-carboxamide
3-cyano-N-[4-(3-cyanophenyl)-5-(2,6-dimethyl-4-pyridyl)thiazol-2-yl]-3-methyl-azetidine-1-carboxamide
1-[[4-(3-cyanophenyl)-5-(2,6-dimethyl-4-pyridyl)thiazol-2-yl]carbamoyl]azetidine-3-carboxylic acid
(2S)-N-[4-(3-cyanophenyl)-5-(2,6-dimethyl-4-pyridyl)thiazol-2-yl]-2-(hydroxymethyl)pyrrolidine-1-carboxamide
(2S)-N-[4-(3-cyanophenyl)-5-(2,6-dimethyl-4-pyridyl)thiazol-2-yl]-2-methyl-azetidine-1-carboxamide
(2S)-N-[4-(3-cyanophenyl)-5-(2,6-dimethyl-4-pyridyl)thiazol-2-yl]-2-(hydroxymethyl)azetidine-1-carboxamide
(3R)-N-[4-(3-cyanophenyl)-5-(2,6-dimethyl-4-pyridyl)thiazol-2-yl]-3-methyl-morpholine-4-carboxamide
(3S)-N-[4-(3-cyanophenyl)-5-(2,6-dimethyl-4-pyridyl)thiazol-2-yl]-3-(1-hydroxy-1-methylethyl)piperazine-1-carboxamide
(3S)-N-[4-(3-cyanophenyl)-5-(2,6-dimethyl-4-pyridyl)thiazol-2-yl]-3-(hydroxymethyl)morpholine-4-carboxamide
N-[4-(3-cyanophenyl)-5-(2,6-dimethyl-4-pyridyl)thiazol-2-yl]-2-methyl-1-oxo-2,8-diazaspiro[4.5]decane-8-carboxamide
(2R)-N-[4-(3-cyanophenyl)-5-(2,6-dimethyl-4-pyridyl)thiazol-2-yl]-2-methyl-azetidine-1-carboxamide
N-[4-(3-cyano-2-methyl-phenyl)-5-(2,6-dimethyl-4-pyridyl)thiazol-2-yl]-2-oxa-6-azaspiro[3.3]heptane-6-carboxamide
(3R)-N 1-[4-(3-cyanophenyl)-5-(2,6-dimethyl-4-pyridyl)thiazol-2-yl]pyrrolidine-1,3-dicarboxamide
N-[4-(3-cyanophenyl)-5-(2,6-dimethyl-4-pyridyl)thiazol-2-yl]-2-oxa-7-azaspiro[4.4]nonane-7-carboxamide
N-[4-(3-cyanophenyl)-5-(2,6-dimethyl-4-pyridyl)thiazol-2-yl]-2-oxa-7-azaspiro[3.4]octane-7-carboxamide
(2S)-N-[4-(3-cyanophenyl)-5-(2,6-dimethyl-4-pyridyl)thiazol-2-yl]-2-(1-hydroxy-1-methylethyl)azetidine-1-carboxamide
(2R)-N-[4-(3-cyanophenyl)-5-(2,6-dimethyl-4-pyridyl)thiazol-2-yl]-2-(1-hydroxy-1-methylethyl)azetidine-1-carboxamide
N-[4-(3-cyanophenyl)-5-(2,6-dimethyl-4-pyridyl)thiazol-2-yl]-3-morpholino-pyrrolidine-1-carboxamide
(3R)-N-[4-(3-cyanophenyl)-5-(2,6-dimethyl-4-pyridyl)thiazol-2-yl]-3-methyl-5-oxo-piperazine-1-carboxamide
(3S)-N-[4-(3-cyanophenyl)-5-(2,6-dimethyl-4-pyridyl)thiazol-2-yl]-3-methyl-morpholine-4-carboxamide
N-[4-(3-cyanophenyl)-5-(2,6-dimethyl-4-pyridyl)thiazol-2-yl]-6-oxa-1-azaspiro[3.3]heptane-1-carboxamide
(3R)-N-[4-(3-cyanophenyl)-5-(2,6-dimethyl-4-pyridyl)thiazol-2-yl]-3-(hydroxymethyl)morpholine-4-carboxamide
N-[4-(3-cyanophenyl)-5-(2,6-dimethyl-4-pyridyl)thiazol-2-yl]-1,3-dimethyl-2-oxo-1,3,8-triazaspiro[4.5]decane-8-carboxamide
(3R)-N-[4-(3-cyanophenyl)-5-(2,6-dimethyl-4-pyridyl)thiazol-2-yl]-3-methylsulfonyl-pyrrolidine-1-carboxamide
(3S)-N-[4-(3-cyanophenyl)-5-(2,6-dimethyl-4-pyridyl)thiazol-2-yl]-3-methyl-5-oxo-piperazine-1-carboxamide
(2R)-N-[4-(3-cyanophenyl)-5-(2,6-dimethyl-4-pyridyl)thiazol-2-yl]-2-(hydroxymethyl)azetidine-1-carboxamide
N-[4-(3-cyanophenyl)-5-(2,6-dimethyl-4-pyridyl)thiazol-2-yl]-6-methyl-5-oxo-2,6-diazaspiro[3.4]octane-2-carboxamide
N-[4-(3-cyanophenyl)-5-(2,6-dimethyl-4-pyridyl)thiazol-2-yl]-1-oxa-6-azaspiro[3.3]heptane-6-carboxamide
N-[4-(3-cyanophenyl)-5-(2,6-dimethyl-4-pyridyl)thiazol-2-yl]-1-methyl-2-oxo-1,8-diazaspiro[4.5]decane-8-carboxamide
N-[4-(3-cyanophenyl)-5-(2,6-dimethyl-4-pyridyl)thiazol-2-yl]-4-methylsulfonyl-piperidine-1-carboxamide
N-[4-(3-cyanophenyl)-5-(2,6-dimethyl-4-pyridyl)thiazol-2-yl]-4-hydroxy-4-methyl-piperidine-1-carboxamide
N-[4-(3-cyanophenyl)-5-[2-(difluoromethyl)-6-methyl-4-pyridyl]thiazol-2-yl]-2-oxa-6-azaspiro[3.3]heptane-6-carboxamide
(4aR,7aS)-N-[4-(3-cyanophenyl)-5-(2,6-dimethyl-4-pyridyl)thiazol-2-yl]-4-methyl-2,3,4a,5,7,7a-hexahydropyrrolo[3,4-b][1,4]oxazine-6-carboxamide
N-[4-(3-cyanophenyl)-5-[2-methyl-6-(trifluoromethyl)-4-pyridyl]thiazol-2-yl]-2-oxa-6-azaspiro[3.3]heptane-6-carboxamide
N-[4-(3-cyanophenyl)-5-(2,6-dimethyl-4-pyridyl)thiazol-2-yl]piperazine-1-carboxamide
(3R)-N-[4-(3-cyanophenyl)-5-(2,6-dimethyl-4-pyridyl)thiazol-2-yl]-3-methyl-piperazine-1-carboxamide
N-[4-(3-cyanophenyl)-5-(2,6-dimethyl-4-pyridyl)thiazol-2-yl]-5,8-diazaspiro[3.5]nonane-5-carboxamide
trans-N-[4-(3-cyanophenyl)-5-(2,6-dimethyl-4-pyridyl)thiazol-2-yl]-2,5-dimethyl-piperazine-1-carboxamide
4-amino-4-cyano-N-[4-(3-cyanophenyl)-5-(2,6-dimethyl-4-pyridyl)thiazol-2-yl]piperidine-1-carboxamide
N-[4-(3-cyanophenyl)-5-(2,6-dimethyl-4-pyridyl)thiazol-2-yl]-1-oxa-4,9-diazaspiro[5.5]undecane-9-carboxamide
N-[4-(3-cyanophenyl)-5-(2,6-dimethyl-4-pyridyl)thiazol-2-yl]-3,8-diazabicyclo[3.2.1]octane-8-carboxamide
N-[4-(3-cyanophenyl)-5-(2,6-dimethyl-4-pyridyl)thiazol-2-yl]-3,6-diazabicyclo[3.1.1]heptane-6-carboxamide
(1R,4R)-N-[4-(3-cyanophenyl)-5-(2,6-dimethyl-4-pyridyl)thiazol-2-yl]-2,5-diazabicyclo[2.2.2]octane-2-carboxamide
cis-(2S,5S)-N-[4-(3-cyanophenyl)-5-(2,6-dimethyl-4-pyridyl)thiazol-2-yl]-2,5-dimethylpiperazine-1-carboxamide
(2R,6R)-N-[4-(3-cyanophenyl)-5-(2,6-dimethyl-4-pyridyl)thiazol-2-yl]-2,6-dimethyl-piperazine-1-carboxamide
N-[4-(3-cyanophenyl)-5-(2,6-dimethyl-4-pyridyl)thiazol-2-yl]-2,2-dimethyl-piperazine-1-carboxamide
1-acetylimino-N-[4-(3-cyanophenyl)-5-(2,6-dimethyl-4-pyridyl)thiazol-2-yl]-1-oxo-1,4-thiazinane-4-carboxamide
N-[4-(3-cyanophenyl)-5-[2-(hydroxymethyl)-6-methyl-4-pyridyl]thiazol-2-yl]-2-oxa-6-azaspiro[3.3]heptane-6-carboxamide
N-[4-(3-cyanophenyl)-5-[2-(hydroxymethyl)-6-methyl-4-pyridyl]thiazol-2-yl]-3-oxo-2,7-diazaspiro[3.5]nonane-7-carboxamide
(3R)-N-[4-(3-cyanophenyl)-5-[2-(hydroxymethyl)-6-methyl-4-pyridyl]thiazol-2-yl]-3-(1-hydroxy-1-methyl-ethyl)pyrrolidine-1-carboxamide
N-[4-(3-cyanophenyl)-5-[2-(hydroxymethyl)-6-methyl-4-pyridyl]thiazol-2-yl]-1-oxo-1,4-thiazinane-4-carboxamide
N-[4-(3-cyanophenyl)-5-[2-(hydroxymethyl)-6-methyl-4-pyridyl]thiazol-2-yl]-4-(oxetan-3-yl)piperazine-1-carboxamide
N-[4-(3-cyanophenyl)-5-[2-(hydroxymethyl)-6-methyl-4-pyridyl]thiazol-2-yl]-1,3-dimethyl-2,4-dioxo-1,3,8-triazaspiro[4.5]decane-8-carboxamide
4-cyano-N-[4-(3-cyanophenyl)-5-[2-(hydroxymethyl)-6-methyl-4-pyridyl]thiazol-2-yl]piperidine-1-carboxamide
4-cyano-N-[4-(3-cyanophenyl)-5-[2-(hydroxymethyl)-6-methyl-4-pyridyl]thiazol-2-yl]-4-(hydroxymethyl)piperidine-1-carboxamide
4,4-dicyano-N-[4-(3-cyanophenyl)-5-[2-(hydroxymethyl)-6-methyl-4-pyridyl]thiazol-2-yl]piperidine-1-carboxamide
4-cyano-N-[4-(3-cyanophenyl)-5-[2-(hydroxymethyl)-6-methyl-4-pyridyl]thiazol-2-yl]-4-methoxy-piperidine-1-carboxamide
N-[4-(3-cyanophenyl)-5-(3-fluoro-2,6-dimethyl-4-pyridyl)thiazol-2-yl]-1-oxo-1,4-thiazinane-4-carboxamide
1-cyanoimino-N-[4-(3-cyanophenyl)-5-(2,6-dimethyl-4-pyridyl)thiazol-2-yl]-1-oxo-1,4-thiazinane-4-carboxamide
4-cyano-N-[4-(3-cyanophenyl)-5-[2-(1-hydroxy-1-methyl-ethyl)-6-methyl-4-pyridyl]thiazol-2-yl]-4-methyl-piperidine-1-carboxamide
N-[4-(3-cyanophenyl)-5-[2-(1-hydroxy-1-methyl-ethyl)-6-methyl-4-pyridyl]thiazol-2-yl]-6-oxa-2-azaspiro[3.4]octane-2-carboxamide
N-[4-(3-cyanophenyl)-5-[2-(1-hydroxy-1-methyl-ethyl)-6-methyl-4-pyridyl]thiazol-2-yl]-2-oxa-6-azaspiro[3.3]heptane-6-carboxamide
N-[4-(3-cyanophenyl)-5-[2-(1-hydroxyethyl)-6-methyl-4-pyridyl]thiazol-2-yl]-2-oxa-6-azaspiro[3.3]heptane-6-carboxamide
rac-(3S,5R)-N-[4-(3-cyanophenyl)-5-(2,6-dimethyl-4-pyridyl)thiazol-2-yl]-3,5-dimethylpiperazine-1-carboxamide
(3S)-N-[4-(3-cyanophenyl)-5-(2,6-dimethyl-4-pyridyl)thiazol-2-yl]-3-(trifluoromethyl)piperazine-1-carboxamide
N-[4-(3-cyanophenyl)-5-(2,6-dimethyl-4-pyridyl)thiazol-2-yl]-5-oxa-2,8-diazaspiro[3.5]nonane-2-carboxamide
N-[4-(3-cyanophenyl)-5-(2,6-dimethyl-4-pyridyl)thiazol-2-yl]-3,8-diazabicyclo[3.2.1]octane-3-carboxamide
N-[4-(3-cyanophenyl)-5-(2,6-dimethyl-4-pyridyl)thiazol-2-yl]-6-oxa-2,9-diazaspiro[4.5]decane-2-carboxamide
(3R)-N-[4-(3-cyanophenyl)-5-(2,6-dimethyl-4-pyridyl)thiazol-2-yl]-3-(trifluoromethyl)piperazine-1-carboxamide
(3S)-3-cyano-N-[4-(3-cyanophenyl)-5-(2,6-dimethyl-4-pyridyl)thiazol-2-yl]piperazine-1-carboxamide
(3R)-3-cyano-N-[4-(3-cyanophenyl)-5-(2,6-dimethyl-4-pyridyl)thiazol-2-yl]piperazine-1-carboxamide
N-[4-(3-cyanophenyl)-5-(2,6-dimethyl-4-pyridyl)thiazol-2-yl]-4,7-diazaspiro[2.5]octane-7-carboxamide
(3S,5S)-N-[4-(3-cyanophenyl)-5-(2,6-dimethyl-4-pyridyl)thiazol-2-yl]-3,5-dimethyl-piperazine-1-carboxamide
(2S)-N-[4-(3-cyanophenyl)-5-(2,6-dimethyl-4-pyridyl)thiazol-2-yl]-2-methyl-piperazine-1-carboxamide
(2R)-N-[4-(3-cyanophenyl)-5-(2,6-dimethyl-4-pyridyl)thiazol-2-yl]-2-(hydroxymethyl)piperazine-1-carboxamide
(2S)-N-[4-(3-cyanophenyl)-5-(2,6-dimethyl-4-pyridyl)thiazol-2-yl]-2-(hydroxymethyl)piperazine-1-carboxamide
N-[4-(3-cyanophenyl)-5-(2,6-dimethyl-4-pyridyl)thiazol-2-yl]-3,6-diazabicyclo[3.1.1]heptane-3-carboxamide
(2R)-N-[4-(3-cyanophenyl)-5-(2,6-dimethyl-4-pyridyl)thiazol-2-yl]-2-methyl-piperazine-1-carboxamide
N-[4-(3-cyanophenyl)-5-(2,6-dimethyl-4-pyridyl)thiazol-2-yl]-9-oxa-2,6-diazaspiro[4.5]decane-2-carboxamide
(3R)-N-[4-(3-cyanophenyl)-5-[2-methyl-6-(trifluoromethyl)-4-pyridyl]thiazol-2-yl]-3-methylpiperazine-1-carboxamide
(4aS,7aS)-N-[4-(3-cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide
(4aS,7aR)-N-[4-(3-cyanophenyl)-5-[2-(difluoromethyl)-6-methyl-4-pyridyl]thiazol-2-yl]-3,4,4a,5,7,7a-hexahydro-2H-pyrrolo[3,4-b][1,4]oxazine-6-carboxamide
rac-(4aS,7aS)-N-[4-(3-cyanophenyl)-5-(2,6-dimethyl-4-pyridyl)thiazol-2-yl]-3,4,4a,5,7,7a-hexahydro-2H-pyrrolo[3,4-b][1,4]oxazine-6-carboxamide
N-[4-(3-cyanophenyl)-5-(2,6-dimethyl-4-pyridyl)thiazol-2-yl]-2-cyclopropyl-piperazine-1-carboxamide
N-[4-(3-cyanophenyl)-5-(2,6-dimethyl-4-pyridyl)thiazol-2-yl]-2-(cyclopropylmethyl)piperazine-1-carboxamide
N-[4-(3-cyanophenyl)-5-(2,6-dimethyl-4-pyridyl)thiazol-2-yl]-3,3-dimethyl-piperazine-1-carboxamide
N-[4-(3-cyanophenyl)-5-(2-methoxy-6-methyl-4-pyridyl)thiazol-2-yl]-1-imino-1-oxo-1,4-thiazinane-4-carboxamide
N-[4-(3-cyanophenyl)-5-[2-(difluoromethyl)-6-methyl-4-pyridyl]thiazol-2-yl]-1-imino-1-oxo-1,4-thiazinane-4-carboxamide
N-[4-(3-cyanophenyl)-5-(2,6-dichloro-4-pyridyl)thiazol-2-yl]-2-oxa-6-azaspiro[3.3]heptane-6-carboxamide
N-[4-(3-cyanophenyl)-5-[2-methyl-6-(trifluoromethyl)-4-pyridyl]thiazol-2-yl]-1-oxo-1,4-thiazinane-4-carboxamide
N-[4-(3-cyanophenyl)-5-(2-methoxy-6-methyl-4-pyridyl)thiazol-2-yl]-1-oxo-1,4-thiazinane-4-carboxamide
N-[4-(3-cyanophenyl)-5-[2-methyl-6-(oxetan-3-yl)-4-pyridyl]thiazol-2-yl]-2-oxa-6-azaspiro[3.3]heptane-6-carboxamide
N-[5-(2-cyano-6-methyl-4-pyridyl)-4-(3-cyanophenyl)thiazol-2-yl]-1-oxo-1,4-thiazinane-4-carboxamide
N-[4-(3-cyanophenyl)-5-[2-(fluoromethyl)-6-methyl-4-pyridyl]thiazol-2-yl]-2-oxa-6-azaspiro[3.3]heptane-6-carboxamide
N-[4-(3-cyanophenyl)-5-(2-cyclopropyl-6-methyl-4-pyridyl)thiazol-2-yl]-2-oxa-6-azaspiro[3.3]heptane-6-carboxamide
N-[4-(3-cyanophenyl)-5-(2-isopropyl-6-methyl-4-pyridyl)thiazol-2-yl]-2-oxa-6-azaspiro[3.3]heptane-6-carboxamide
N-[4-(3-cyanophenyl)-5-(2-ethyl-6-methyl-4-pyridyl)thiazol-2-yl]-2-oxa-6-azaspiro[3.3]heptane-6-carboxamide
N-[4-(3-cyanophenyl)-5-(2,6-dimethyl-1-oxido-pyridin-1-ium-4-yl)thiazol-2-yl]-2-oxa-6-azaspiro[3.3]heptane-6-carboxamide
4-cyano-N-[4-(3-cyano-2-methyl-phenyl)-5-(2,6-dimethyl-4-pyridyl)thiazol-2-yl]-4-methylpiperidine-1-carboxamide
N-[5-(2-acetamido-6-methyl-4-pyridyl)-4-(3-cyanophenyl)thiazol-2-yl]-2-oxa-6-azaspiro[3.3]heptane-6-carboxamide
N-[5-[2-(acetamidomethyl)-6-methyl-4-pyridyl]-4-(3-cyanophenyl)thiazol-2-yl]-2-oxa-6-azaspiro[3.3]heptane-6-carboxamide
N-[4-(3-cyanophenyl)-5-[2-(methoxymethyl)-6-methyl-4-pyridyl]thiazol-2-yl]-2-oxa-6-azaspiro[3.3]heptane-6-carboxamide
N-[4-(3-cyanophenyl)-5-[2-(1-methoxyethyl)-6-methyl-4-pyridyl]thiazol-2-yl]-2-oxa-6-azaspiro[3.3]heptane-6-carboxamide
N-[5-[2-chloro-6-(1-hydroxy-1-methyl-ethyl)-4-pyridyl]-4-(3-cyanophenyl)thiazol-2-yl]-2-oxa-6-azaspiro[3.3]heptane-6-carboxamide
4-cyano-N-[4-(3-cyanophenyl)-5-[2-(hydroxymethyl)-6-methyl-4-pyridyl]thiazol-2-yl]-4-methylpiperidine-1-carboxamide
N-[4-(3-cyanophenyl)-5-[2-(hydroxymethyl)-6-(trifluoromethyl)-4-pyridyl]thiazol-2-yl]-2-oxa-6-azaspiro[3.3]heptane-6-carboxamide
3-cyano-N-[4-(3-cyanophenyl)-5-(2,6-dimethyl-4-pyridyl)thiazol-2-yl]-3-methyl-pyrrolidine-1-carboxamide
N-[5-[2,6-bis(trideuteriomethyl)-4-pyridyl]-4-(3-cyanophenyl)thiazol-2-yl]-4-cyano-4-methylpiperidine-1-carboxamide
N-[4-(3-cyanophenyl)-5-[2-[(1R)-1-hydroxyethyl]-6-methyl-4-pyridyl]thiazol-2-yl]-2-oxa-6-azaspiro[3.3]heptane-6-carboxamide
N-[4-(3-cyanophenyl)-5-[2-[(1S)-1-hydroxyethyl]-6-methyl-4-pyridyl]thiazol-2-yl]-2-oxa-6-azaspiro[3.3]heptane-6-carboxamide
(3R)-3-cyano-N-[4-(3-cyanophenyl)-5-(2,6-dimethyl-4-pyridyl)thiazol-2-yl]-3-methyl-pyrrolidine-1-carboxamide
(3S)-3-cyano-N-[4-(3-cyanophenyl)-5-(2,6-dimethyl-4-pyridyl)thiazol-2-yl]-3-methyl-pyrrolidine-1-carboxamide.

13. A pharmaceutical composition comprising a compound according to any one of claims 1 to 12, or a pharmaceutically acceptable salt thereof, in admixture with a pharmaceutically acceptable diluent or carrier.

14. A compound according to any one of claims 1 to 12, or a pharmaceutically acceptable salt thereof, or a pharmaceutical composition according to claim 13, for use in therapy.

15. A compound according to any one of claims 1 to 12, or a pharmaceutically acceptable salt thereof, or a pharmaceutical composition according to claim 13, for use:
(i) in the treatment of a proliferative condition;
(ii) in the treatment of cancer;
(iii) in the treatment of cancer, wherein the compound or pharmaceutical composition is administered in combination with one or more additional anticancer agents;
(iv) in the treatment of cancer, wherein the compound or pharmaceutical composition is administered in combination with one or more additional anticancer agents selected from the group consisting of:
1) other forms of cancer immunotherapy and anti-cancer chemotherapeutic agents;
2) adenosine pathway modulators, including, but not limited to A2b antagonists, CD73 inhibitors and CD39 inhibitors;
3) anti-PD-1 and PDL-1 antibodies (e.g. cetrelimab, pembrolizumab, nivolumab, durvalumab, avelumab and atezolizumab); and
4) anti-CTLA4 antibodies (e.g ipilimumab).

## Patentansprüche

1. Verbindung oder pharmazeutisch unbedenkliches Salz davon mit der nachfolgend gezeigten Strukturformel I: 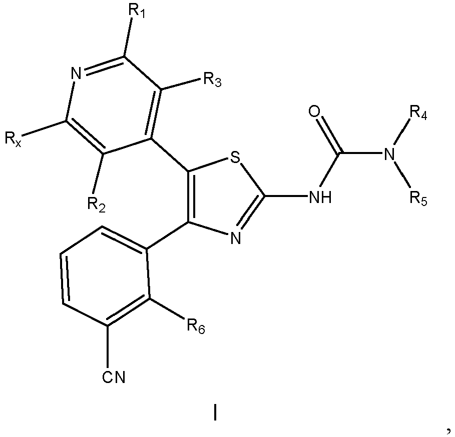 wobei:
Rₓ ausgewählt ist aus Methyl, CD₃, Chlor und CF₃;
R₁ ausgewählt ist aus (1-3C)Alkyl, Halogen, (1-3C)Haloalkyl, (1-3C)Haloalkoxy, (1-3C)Hydroxyalkyl, Cyano, Cyclopropyl, Oxetan-3-yl, -(CR_{1C}R_{1D})_{q1}-OR_{1A}, -(CR_{1C}R_{1D})_{q1}-C(O)R_{1A}, -(CR_{1C}R_{1D})_{q1}-C(O)OR_{1A}, -(CR_{1C}R_{1D})_{q1}-OC(O)R_{1A}, -(CR_{1C}R_{1D})_{q1}-C(O)N(R_{1B})R_{1A}, -(CR_{1C}R_{1D})_{q1}-N(R_{1B})C(O)R_{1A}, -(CR_{1C}R_{1D})_{q1}-S(O)ₚR_{1A} (wobei p 0, 1 oder 2 ist), -(CR_{1C}R_{1D})_{q1}-SO_{2N}(R_{1B})R_{1A} oder -(CR_{1C}R_{1D})_{q1}-N(R_{1B})SO₂R_{1A},
wobei q1 0, 1 oder 2 ist; und
wobei R_{1A} und R_{1B} jeweils unabhängig ausgewählt sind aus Wasserstoff, (1-2C)Alkyl, (3-4C)Cycloalkyl oder (3-4C)Cycloalkyl(1-2C)alkyl;
wobei R_{1C} and R_{1D} jeweils unabhängig ausgewählt sind aus Wasserstoff oder (1-2C)Alkyl;
R₂ und R₃ beide Wasserstoff sind oder eines von R₂ und R₃ Wasserstoff ist und das andere aus Fluor, Methyl oder Methoxy ausgewählt ist;
R₄ und R₅ gebunden sind, sodass sie zusammen mit dem Stickstoffatom, an welches sie angefügt sind, einen heterocyclischen Ring bilden, welcher optional an einem beliebigen verfügbaren Kohlenstoffatom durch eine oder mehrere R_{10C}-Substituentengruppen substituiert ist,
wobei R_{10C} ausgewählt ist aus Oxo-, Thioxo-, Halo- oder Cyano-Substituenten oder einer Gruppe der Formel:
-[CR₇ₐR_{7b}]ₙ-L-Z,
wobei
n 0 bis 6 ist;
R₇ₐ und R_{7b} jeweils unabhängig ausgewählt sind aus Wasserstoff, Fluor oder (1-2C)Alkyl;
L fehlt oder ausgewählt ist aus -O-, -S-, -SO-, -SO₂-, -N(Rₐ)-, -C(O)-, -C(O)O-, -OC(O)-, -C(O)N(Rₐ)-, -N(Rₐ)C(O)-, -N(Rₐ)C(O)N(R_{b})-, -C(S)N(Rₐ)-, -N(Rₐ)C(S)-, -N(Rₐ)C(S)N(R_{b})-, - S(O)₂N(Rₐ)- oder -N(Rₐ)SO₂-, wobei Rₐ und R_{b} jeweils unabhängig ausgewählt sind aus Wasserstoff oder (1-2C)Alkyl; und
Z ausgewählt ist aus Wasserstoff, (1-6C)Alkyl, (3-6C)Cycloalkyl, (3-6C)Cycloalkyl(1-2C)alkyl, Aryl, Aryl(1-2C)alkyl, Heterocyclyl, Heterocyclyl(1-2C)alkyl, Heteroaryl oder Heteroaryl(1-2C)alkyl; und wobei Z optional substituiert ist durch einen oder mehrere Substituenten ausgewählt aus (1-6C)Alkyl, Halogen, (1-6C)Haloalkyl, (1-6C)Haloalkoxy, Cyano, Nitro, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}R_{c} (wobei y 0, 1 oder 2 ist), -SO₂N(R_{c})R_{d}, -N(R_{c})SO₂R_{d}, -(CH₂)_{z}NR_{c}R_{d} (wobei z 1, 2 oder 3 ist) oder Oxo,
wobei ein beliebiger Alkylanteil in einer Substituentengruppe an Z optional ferner durch Cyano, Halogen, Hydroxy, Amino, Oxo, (1-2C)Alkyl oder (1-2C)Alkoxy substituiert ist und R_{c} und R_{d} jeweils unabhängig ausgewählt sind aus Wasserstoff, (1-6C)Alkyl, (1-6C)Haloalkyl oder (3-6C)Cycloalkyl;
und wobei beliebige verfügbare N-Atome optional in der Form eines N-Oxids vorliegen oder optional durch ein oder mehrere R_{10N} substituiert sind,
wobei R_{10N} -S(O)₂NH₂ ist oder ausgewählt ist aus:
(i) -Z₁;
(ii) -L₁ₐ-Z₁; oder
(iii) -[CR₈ₐR_{8b}]₁₋₆-L_{1b}-Z₁;
wobei
R₈ₐ und R_{8b} jeweils unabhängig ausgewählt sind aus Wasserstoff, Fluor oder (1-2C)Alkyl;
L₁ₐ ausgewählt ist aus -C(O)-, -S(O)₂-, -C(O)O-, -C(O)N(Rₐ₁)-, -S(O)₂N(Rₐ₁)- oder - N(Rₐ₁)-, wobei Rₐ₁ ausgewählt ist aus Wasserstoff oder (1-2C)Alkyl;
L_{1b} fehlt oder ausgewählt ist aus -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, - N(Rₐ₂)C(S)N(R_{b2})-, -S(O)₂N(Rₐ₂)- oder -N(Rₐ₂)SO₂, wobei Rₐ₂ und R_{b2} jeweils unabhängig ausgewählt sind aus Wasserstoff oder (1-2C)Alkyl; und
Z₁ ausgewählt ist aus (1-6C)Alkyl, (3-6C)Cycloalkyl, Aryl, Heterocyclyl, Heteroaryl, (3-6C)Cycloalkyl(1-2C)alkyl, Aryl(1-2C)alkyl, Heterocyclyl(1-2C)alkyl oder Heteroaryl(1-2C)alkyl, mit der Maßgabe, dass, wenn Z₁ eine Heterocyclylgruppe ist, die direkt an ein N-Atom gebunden ist, es ein kohlenstoffgebundenes Heterocyclyl ist; und wobei Z₁ optional substituiert ist durch einen oder mehrere Substituenten ausgewählt aus (1-6C)Alkyl, Halogen, (1-6C)Haloalkoxy, Cyano, Nitro, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, - S(O)_{y}Rₑ (wobei y 0, 1 oder 2 ist), -SO₂N(Rₑ)R_{f}, -N(Rₑ)SO₂R_{f}, -(CH₂)_{z}NRₑR_{f} (wobei z 1, 2 oder 3 ist) oder Oxo, und wobei Rₑ und R_{f} jeweils unabhängig ausgewählt sind aus Wasserstoff, (1-6C)Alkyl, (1-6C)Haloalkyl oder (3-6C)Cycloalkyl;
und wobei beliebige S-Atome, die in dem heterocyclischen Ring vorhanden sind, optional als S(=O), S(=O)₂ oder S(=O)(=NRₑ) vorhanden sein können, wobei Rₑ ausgewählt ist aus Wasserstoff, (1-3C)Alkyl oder (2-3C)Alkanoyl; und
R₆ ausgewählt ist aus Wasserstoff, Halogen, Methyl, Methoxy und Trifluormethyl.

2. Verbindung gemäß Anspruch 1 oder ein pharmazeutisch unbedenkliches Salz davon, wobei eine beliebige oder mehrere der folgenden Aussagen (A)-(C) zutreffen:
(A) Rₓ ausgewählt ist aus Methyl oder CD₃;
(B) R₁ ausgewählt ist aus (1-3C)Alkyl, Halogen, (1-3C)Haloalkyl, (1-3C)Haloalkoxy, (1-3C)Hydroxyalkyl, Cyano, Cyclopropyl, Oxetan-3-yl, -(CH₂)_{q1}-OR_{1A} und -(CH₂)_{q1}-C(O)R_{1A};
wobei q1 0 oder 1 ist; und
wobei R_{1A} ausgewählt ist aus Wasserstoff oder (1-2C)Alkyl;
(C) R₁ ausgewählt ist aus Methyl, CD₃, Isopropyl, Halogen, (1-3C)Hydroxyalkyl, Cyano, Cyclopropyl, Oxetan-3-yl, -OMe, -CH₂F, -CHF₂ oder -CF₃.

3. Verbindung gemäß einem beliebigen der vorhergehenden Ansprüche oder ein pharmazeutisch unbedenkliches Salz davon, wobei eine beliebige oder beide der folgenden Aussagen (A)-(B) zutreffen:
(A) R₂ und R₃ beide Wasserstoff sind oder eines von R₂ und R₃ Wasserstoff ist und das andere Fluor oder Methyl ist;
(B) R₂ und R₃ beide Wasserstoff sind.

4. Verbindung gemäß einem beliebigen der vorhergehenden Ansprüche oder ein pharmazeutisch unbedenkliches Salz davon, wobei R₄ und R₅ gebunden sind, sodass sie zusammen mit dem Stickstoffatom, an welches sie angefügt sind, einen heterocyclischen Ring bilden, welcher optional an einem beliebigen verfügbaren Kohlenstoffatom durch eine oder mehrere R_{10C}-Substituentengruppen substituiert ist,
wobei R_{10C} ausgewählt ist aus Oxo-, Halo- oder Cyano-Substituenten oder einer Gruppe der Formel:
-[CH₂]ₙ-L-Z,
wobei
n 0 bis 2 ist;
L fehlt oder ausgewählt ist aus -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)- oder -N(Rₐ₂)SO₂-, wobei Rₐ₂ und R_{b2} jeweils unabhängig ausgewählt sind aus Wasserstoff oder Methyl; und
Z ausgewählt ist aus Wasserstoff, (1-6C)Alkyl, (3-6C)Cycloalkyl, (3-6C)Cycloalkyl(1-2C)alkyl, Aryl, Heterocyclyl oder Heteroaryl; und wobei Z optional substituiert ist durch einen oder mehrere Substituenten ausgewählt aus (1-2C)Alkyl, Halogen, (1-2C)Haloalkyl, (1-2C)Haloalkoxy, Cyano, -NR_{c}R_{d}, -OR_{c}, -C(O)R_{c}, -C(O)OR_{c}, -OC(O)R_{c}, -C(O)N(Re)Rd, - N(R_{c})C(O)R_{d}, -S(O)_{y}R_{c} (wobei y 0, 1 oder 2 ist), -SO₂N(R_{c})R_{d}, -N(R_{c})SO₂R_{d}, -(CH₂)_{z}NR_{c}R_{d} (wobei z 1 oder 2 ist) oder Oxo;
wobei ein beliebiger Alkylanteil in einer Substituentengruppe an Z₁ optional ferner durch Cyano, Halogen, Hydroxy, Amino, Oxo, (1-2C)Alkyl oder (1-2C)Alkoxy substituiert ist und R_{c} und R_{d} jeweils unabhängig ausgewählt sind aus Wasserstoff, (1-2C)Alkyl, (1-2C)Haloalkyl oder (3-6C)Cycloalkyl;
und wobei beliebige verfügbare N-Atome optional in der Form eines N-Oxids vorliegen oder optional durch ein oder mehrere R_{10N} substituiert sind, wobei R_{10N} -S(O)₂NH₂ ist oder ausgewählt ist aus:
(i) -Z₁;
(ii) -L₁ₐ-Z₁; oder
(iii) -[CR₈ₐR_{8b}]₁₋₂-L_{1b}-Z₁;
wobei
R₈ₐ und R_{8b} beide Wasserstoff sind;
L₁ₐ ausgewählt ist aus -C(O)-, -S(O)₂- oder -S(O)₂N(Rₐ₁)-, wobei Rₐ₁ Wasserstoff oder Methyl ist;
L_{1b} fehlt oder ausgewählt ist aus -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)- oder -N(Rₐ₂)SO₂, wobei Rₐ₂ und R_{b2} jeweils unabhängig ausgewählt sind aus Wasserstoff oder Methyl; und
Z₁ ausgewählt ist aus (1-6C)Alkyl, (3-6C)Cycloalkyl, Aryl, Heterocyclyl, Heteroaryl, (3-6C)Cycloalkyl(1-2C)alkyl, Aryl(1-2C)alkyl, mit der Maßgabe, dass, wenn Z₁ eine Heterocyclylgruppe ist, die direkt an ein N-Atom gebunden ist, es ein kohlenstoffgebundenes Heterocyclyl ist; und wobei Z₁ optional substituiert ist durch einen oder mehrere Substituenten ausgewählt aus (1-2C)Alkyl, Halogen, (1-2C)Haloalkoxy, Cyano, Nitro, -NRₑR_{f}, -ORₑ, -C(O)Rₑ, -C(O)ORₑ, -OC(O)Rₑ, -C(O)N(Rₑ)R_{f}, -N(Rₑ)C(O)R_{f}, -S(O)_{y}Rₑ (wobei y 0, 1 oder 2 ist), - SO₂N(Rₑ)R_{f}, -N(Rₑ)SO₂R_{f}, -(CH₂)_{z}NRₑR_{f} (wobei z 1, 2 oder 3 ist) oder Oxo, und wobei Rₑ und R_{f} jeweils unabhängig ausgewählt sind aus Wasserstoff oder (1-2C)Alkyl;
und wobei beliebige S-Atome, die in dem heterocyclischen Ring vorhanden sind, optional als S(=O), S(=O)₂ oder S(=O)(=NRₑ) vorhanden sein können, wobei Rₑ ausgewählt ist aus Wasserstoff, (1-2C)Alkyl oder (2C)Alkanoyl.

5. Verbindung gemäß einem beliebigen der vorhergehenden Ansprüche oder ein pharmazeutisch unbedenkliches Salz davon, wobei R₄ und R₅ gebunden sind, sodass sie zusammen mit dem Stickstoffatom, an welches sie angefügt sind, einen heterocyclischen Ring bilden, welcher optional an einem beliebigen verfügbaren Kohlenstoffatom durch eine oder mehrere R_{10C}-Substituentengruppen substituiert ist,
wobei R_{10C} ausgewählt ist aus Oxo-, Halo- oder Cyano-Substituenten oder einer Gruppe der Formel:
-[CH₂]ₙ-L-Z,
wobei
n 0 bis 2 ist;
L fehlt oder ausgewählt ist aus -O-, -S-, -SO-, -SO₂-, -N(Rₐ)-, -C(O)-, -C(O)O-, -OC(O)-, -C(O)N(Rₐ)-, -N(Rₐ)C(O)-, -S(O)₂N(Rₐ)- oder -N(Rₐ)SO₂-, wobei Rₐ und R_{b} Wasserstoff sind; und
Z ausgewählt ist aus Wasserstoff, (1-4C)Alkyl, (3-6C)Cycloalkyl, Phenyl, einem 4-7-gliedrigen Heterocyclyl oder einem 5- oder 6-gliedrigen Heteroaryl; und wobei Z optional substituiert ist durch einen oder mehrere Substituenten ausgewählt aus (1-2C)Alkyl, Halogen, (1-2C)Haloalkyl, (1-2C)Haloalkoxy, Cyano, -NR_{c}R_{d}, -OR_{c}, -C(O)R_{c}, -C(O)N(R_{c})R_{d}, -N(R_{c})C(O)R_{d} oder -S(O)_{y}R_{c} (wobei y 0, 1 oder 2 ist) oder Oxo;
wobei R_{c} und R_{d} jeweils unabhängig ausgewählt sind aus Wasserstoff oder (1-2C)Alkyl; und wobei beliebige verfügbare N-Atome optional in der Form eines N-Oxids vorliegen oder optional durch ein oder mehrere R_{10N} substituiert sind, wobei R_{10N} -S(O)₂NH₂ ist oder ausgewählt ist aus:
(i) -Z₁;
(ii) -L₁ₐ-Z₁; oder
(iii) -[CR₈ₐR_{8b}]₁₋₂-L_{1b}-Z₁;
wobei
R₈ₐ und R_{8b} beide Wasserstoff sind;
L₁ₐ ausgewählt ist aus -C(O)-, -S(O)₂- oder -S(O)₂N(Rₐ₁)-, wobei Rₐ₁ Wasserstoff ist;
L_{1b} fehlt oder ausgewählt ist aus -O-, -S-, -SO-, -SO₂-, -N(Rₐ₂)-, -C(O)N(Rₐ₂)-, - N(Rₐ₂)C(O)-, -S(O)₂N(Rₐ₂)- oder -N(Rₐ₂)SO₂, wobei Rₐ₂ Wasserstoff ist; und
Z₁ ausgewählt ist aus (1-4C)Alkyl, Phenyl, einem 4-7-gliedrigen Heterocyclyl oder einem 5- oder 6-gliedrigen Heteroaryl, mit der Maßgabe, dass, wenn Z₁ eine Heterocyclylgruppe ist, die direkt an ein N-Atom gebunden ist, es ein kohlenstoffgebundenes Heterocyclyl ist; und wobei Z₁ optional substituiert ist durch einen oder mehrere Substituenten ausgewählt aus (1-2C)Alkyl, Halogen, (1-2C)Haloalkoxy, Cyano, -NRₑR_{f}, -ORₑ, -C(O)Rₑ, -C(O)N(Rₑ)R_{f}, -N(Rₑ)C(O)R_{f}, - S(O)_{y}Rₑ (wobei y 0, 1 oder 2 ist); und wobei Rₑ und R_{f} jeweils unabhängig ausgewählt sind aus Wasserstoff oder Methyl;
und wobei beliebige S-Atome, die in dem heterocyclischen Ring vorhanden sind, optional als S(=O), S(=O)₂ oder S(=O)(=NRₑ) vorhanden sein können, wobei Rₑ ausgewählt ist aus Wasserstoff, (1-2C)Alkyl oder (2C)Alkanoyl.

6. Verbindung gemäß einem beliebigen der vorhergehenden Ansprüche oder ein pharmazeutisch unbedenkliches Salz davon, wobei R₄ und R₅ gebunden sind, sodass sie zusammen mit dem Stickstoffatom, an welches sie gebunden sind, einen heterocyclischen Ring bilden, welcher optional an einem beliebigen verfügbaren Kohlenstoffatom durch eine oder mehrere R_{10C}-Substituentengruppen substituiert ist, wobei R_{10C} ausgewählt ist aus Oxo, Halogen, Cyano, (1-4C)Alkyl oder (3-6C)Cycloalkyl;
und wobei beliebige verfügbare N-Atome optional in der Form eines N-Oxids vorliegen oder optional durch ein oder mehrere R_{10N} substituiert sind, wobei R_{10N} -S(O)₂NH₂ oder (1-4C)Alkyl ist,
und wobei beliebige S-Atome, die in dem heterocyclischen Ring vorhanden sind, optional als S(=O), S(=O)₂ oder S(=O)(=NRₑ) vorhanden sein können, wobei Rₑ ausgewählt ist aus Wasserstoff, Methyl oder (2C)Alkanoyl.

7. Verbindung gemäß einem beliebigen der vorhergehenden Ansprüche oder ein pharmazeutisch unbedenkliches Salz davon, wobei R₄ und R₅ gebunden sind, sodass sie zusammen mit dem Stickstoffatom, an welches sie angefügt sind, einen heterocyclischen Ring bilden, ausgewählt aus einer beliebigen der folgenden Optionen: 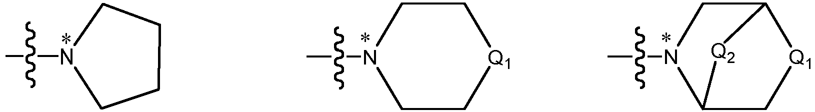 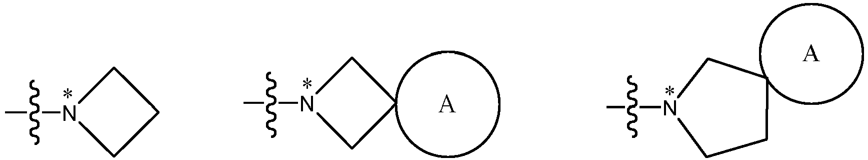 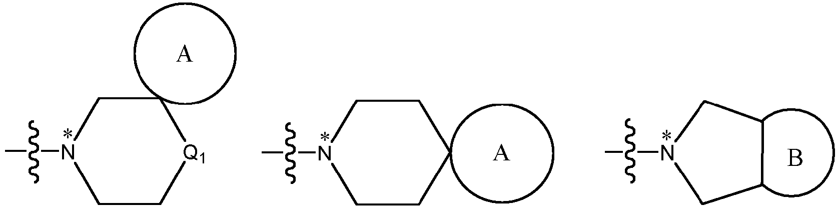 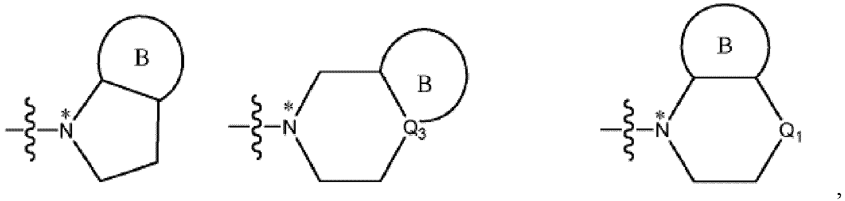
wobei * das N-Atom bezeichnet, an welches R₄ und R₅ angefügt sind;
Q₁ O, NH, S, S(O), S(O)₂, S(O)(=NRₑ), N-R_{10N}, CH₂, CHR_{10C} oder C(R_{10c})₂ ist;
Q₂ -CH₂-, -CHR_{10C}-, -C(R_{10C})₂-, -CHR_{10C}-CH₂-, -CH₂-CHR_{10C}-, -CHR_{10C}-CHR_{10C}-, - C(R_{10C})₂-CH₂- oder -CH₂-C(R_{10C})₂- ist;
Q₃ CH, CR_{10C} oder N ist;
Ring A ein spirokondensierter 4-, 5- oder 6-gliedriger carbocyclischer oder heterocyclischer Ring ist;
Ring B ein fusionierter 4-, 5- oder 6-gliedriger carbocyclischer oder heterocyclischer Ring ist;
wobei Rₑ ausgewählt ist aus Wasserstoff, (1-2C)Alkyl oder (2C)Alkanoyl;
und wobei jedes der heterocyclischen Ringsysteme optional mit einer oder mehreren R_{10c}- oder R_{10N}-Substituentengruppen substituiert ist, wie in einem beliebigen der Ansprüche 1, 4 oder 5 definiert.

8. Verbindung gemäß einem beliebigen der vorhergehenden Ansprüche oder ein pharmazeutisch unbedenkliches Salz davon, wobei R₄ und R₅ gebunden sind, sodass sie zusammen mit dem Stickstoffatom, an welches sie angefügt sind, einen heterocyclischen Ring bilden, ausgewählt aus einer beliebigen der folgenden Optionen: 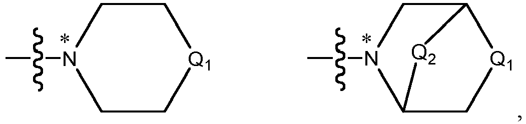
wobei * das N-Atom bezeichnet, an welches R₄ und R₅ angefügt sind;
Q₁ O, NH, S, S(O), S(O)₂, S(O)(=NRₑ), N-R_{10N}, CH₂, CHR_{10C} oder C(R_{10c})₂ ist;
Q₂ -CH₂- ist;
wobei Rₑ ausgewählt ist aus Wasserstoff, Methyl oder (2C)Alkanoyl;
und R_{10c} oder R_{10N} wie in einem beliebigen der Ansprüche 1, 4 oder 5 definiert sind.

9. Verbindung gemäß einem beliebigen der vorhergehenden Ansprüche oder ein pharmazeutisch unbedenkliches Salz davon, wobei R₄ und R₅ gebunden sind, sodass sie zusammen mit dem Stickstoffatom, an welches sie angefügt sind, einen heterocyclischen Ring der folgenden Struktur bilden: 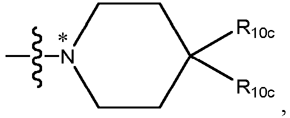
wobei * das N-Atom bezeichnet, an welches R₄ und R₅ angefügt sind;
und jedes R_{10c} unabhängig aus den in einem beliebigen der obigen Ansprüche 1, 4 oder 5 definierten Optionen ausgewählt ist.

10. Verbindung gemäß einem beliebigen der vorhergehenden Ansprüche oder ein pharmazeutisch unbedenkliches Salz davon, wobei eine beliebige oder beide der folgenden Aussagen (A)-(B) zutreffen:
(A) R₆ ausgewählt ist aus Wasserstoff, Halogen oder Methyl;
(B) R₆ Wasserstoff ist.

11. Verbindung gemäß einem beliebigen der vorhergehenden Ansprüche oder ein pharmazeutisch unbedenkliches Salz davon, wobei die Verbindung die Formel IA, IB oder IC ist: 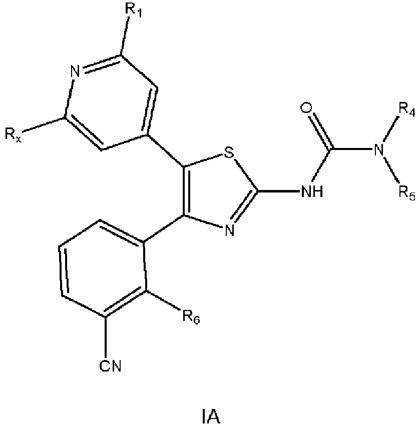 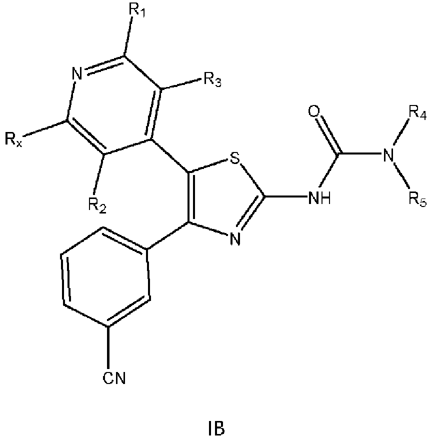 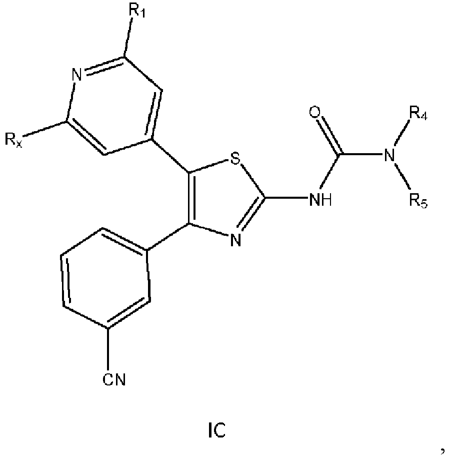 wobei Rₓ, R₁, R₂, R₃, R₄, R₅ und R₆ jeweils wie in einem beliebigen der Ansprüche 1 bis 10 definiert sind.

12. Verbindung gemäß Anspruch 1 oder ein pharmazeutisch unbedenkliches Salz davon, ausgewählt aus einem beliebigen der Folgenden:
N-[5-(2-Chlor-6-methyl-4-pyridyl)-4-(3-cyanophenyl)thiazol-2-yl]morpholin-4-carboxamid
N-[5-(2-Chlor-6-methyl-4-pyridyl)-4-(3-cyanophenyl)thiazol-2-yl]-3-(1-hydroxy-1-methyl-ethyl)pyrrolidin-1-carboxamid
(2R)-N-[5-(2-Chlor-6-methyl-4-pyridyl)-4-(3-cyanophenyl)thiazol-2-yl]-2-(1-hydroxy-1-methyl-ethyl)pyrrolidin-1-carboxamid
N-[5-(2-Chlor-6-methyl-4-pyridyl)-4-(3-cyanophenyl)thiazol-2-yl]piperazin-1-carboxamid
(3R)-N-[5-(2-Chlor-6-methyl-4-pyridyl)-4-(3-cyanophenyl)thiazol-2-yl]-3-(1-hydroxy-1-methyl-ethyl)piperazin-1-carboxamid
N-[5-(2-Chlor-6-methyl-4-pyridyl)-4-(3-cyanophenyl)thiazol-2-yl]-4-(oxetan-3-yl)piperazin-1-carboxamid
N-[5-(2-Chlor-6-methyl-4-pyridyl)-4-(3-cyanophenyl)thiazol-2-yl]-1-imino-1-oxo-1,4-thiazinan-4-carboxamid
N-[5-(2-Chlor-6-methyl-4-pyridyl)-4-(3-cyanophenyl)thiazol-2-yl]pyrrolidin-1-carboxamid
N-[5-(2-Chlor-6-methyl-4-pyridyl)-4-(3-cyanophenyl)thiazol-2-yl]-2-(2-hydroxy-2-methyl-propyl)pyrrolidin-1-carboxamid
N-[5-(2-Chlor-6-methyl-4-pyridyl)-4-(3-cyanophenyl)thiazol-2-yl]-3-(1-hydroxy-1-methyl-ethyl)morpholin-4-carboxamid
(2S)-4-[[5-(2-Chlor-6-methyl-4-pyridyl)-4-(3-cyanophenyl)thiazol-2-yl]carbamoyl]piperazin-2-carbonsäure
N-[5-(2-Chlor-6-methyl-4-pyridyl)-4-(3-cyanophenyl)thiazol-2-yl]-4-methyl-piperazin-1-carboxamid
N-[5-(2-Chlor-6-methyl-4-pyridyl)-4-(3-cyanophenyl)thiazol-2-yl]-4-hydroxy-4-methylpiperidin-1-carboxamid
N-[5-(2-Chlor-6-methyl-4-pyridyl)-4-(3-cyanophenyl)thiazol-2-yl]-4-cyano-4-methylpiperidin-1-carboxamid
(3S)-N-[5-(2-Chlor-6-methyl-4-pyridyl)-4-(3-cyanophenyl)thiazol-2-yl]-3-(1-hydroxy-1-methyl-ethyl)piperazin-1-carboxamid
N-[4-(3-Cyanophenyl)-5-(2,6-dimethyl-4-pyridyl)thiazol-2-yl]-4-methyl-piperazin-1-carboxamid
4-Cyano-N-[4-(3-cyanophenyl)-5-(2,6-dimethyl-4-pyridyl)thiazol-2-yl]piperidin-1-carboxamid
(3R)-3-Cyano-N-[4-(3-cyanophenyl)-5-(2,6-dimethyl-4-pyridyl)thiazol-2-yl]pyrrolidin-1-carboxamid
N-[4-(3-Cyanophenyl)-5-(2,6-dimethyl-4-pyridyl)thiazol-2-yl]-3,4,6,7,9,9a-hexahydro-1H-pyrazino[2,1-c][1,4]oxazin-8-carboxamid
N-[4-(3-Cyanophenyl)-5-(2,6-dimethyl-4-pyridyl)thiazol-2-yl]-2-oxa-6-azaspiro[3 .3]heptan-6-carboxamid
N-[4-(3-Cyanophenyl)-5-(2,6-dimethyl-4-pyridyl)thiazol-2-yl]-3-oxo-piperazin-1-carboxamid
N4-[4-(3-Cyanophenyl)-5-(2,6-dimethyl-4-pyridyl)thiazol-2-yl]morpholin-2,4-dicarboxamid
N-[4-(3-Cyanophenyl)-5-(2,6-dimethyl-4-pyridyl)thiazol-2-yl]-4-methyl-3-oxo-piperazin-1-carboxamid
(2R)-N-[4-(3-Cyanophenyl)-5-(2,6-dimethyl-4-pyridyl)thiazol-2-yl]-2-(hydroxymethyl)morpholin-4-carboxamid
4-Acetyl-N-[4-(3-Cyanophenyl)-5-(2,6-dimethyl-4-pyridyl)thiazol-2-yl]piperazin-1-carboxamid
N-[4-(3-Cyanophenyl)-5-(2,6-dimethyl-4-pyridyl)thiazol-2-yl]-4-methylsulfonyl-piperazin-1-carboxamid
N-[4-(3-Cyanophenyl)-5-(2,6-dimethyl-4-pyridyl)thiazol-2-yl]-4-sulfamoyl-piperazin-1-carboxamid
N-[4-(3-Cyanophenyl)-5-(2,6-dimethyl-4-pyridyl)thiazol-2-yl]-1-imino-1-oxo-1,4-thiazinan-4-carboxamid
N-[4-(3-Cyanophenyl)-5-(2,6-dimethyl-4-pyridyl)thiazol-2-yl]-2,4-dioxo-1,3,8-triazaspiro[4.5]decan-8-carboxamid
(2S)-N-[4-(3-Cyanophenyl)-5-(2,6-dimethyl-4-pyridyl)thiazol-2-yl]-2-(hydroxymethyl)morpholin-4-carboxamid
4-Cyano-N-[4-(3-cyanophenyl)-5-(2,6-dimethyl-4-pyridyl)thiazol-2-yl]-4-methoxy-piperidin-1-carboxamid
4-Cyano-N-[4-(3-cyanophenyl)-5-(2,6-dimethyl-4-pyridyl)thiazol-2-yl]-4-(hydroxymethyl)piperidin-1-carboxamid
N-[4-(3-Cyanophenyl)-5-(2,6-dimethyl-4-pyridyl)thiazol-2-yl]-1-oxo-1,4-thiazinan-4-carboxamid
N-[4-(3-Cyanophenyl)-5-(2,6-dimethyl-4-pyridyl)thiazol-2-yl]-3-(hydroxymethyl)azetidin-1-carboxamid
3-Cyano-N-[4-(3-Cyanophenyl)-5-(2,6-dimethyl-4-pyridyl)thiazol-2-yl]azetidin-1-carboxamid
N-[4-(3-Cyanophenyl)-5-(2,6-dimethyl-4-pyridyl)thiazol-2-yl]-1,1-dioxo-1,4-thiazinan-4-carboxamid
N-[4-(3-Cyanophenyl)-5-(2,6-dimethyl-4-pyridyl)thiazol-2-yl]-1,3-dimethyl-2,4-dioxo-1,3,8-triazaspiro[4.5]decan-8-carboxamid
N1-[4-(3-Cyanophenyl)-5-(2,6-dimethyl-4-pyridyl)thiazol-2-yl]piperidin-1,4-dicarboxamid
N-[4-(3-Cyanophenyl)-5-(2,6-dimethyl-4-pyridyl)thiazol-2-yl]-4-(2-hydroxyethyl)piperazin-1-carboxamid
N-[4-(3-Cyanophenyl)-5-(2,6-dimethyl-4-pyridyl)thiazol-2-yl]-3-hydroxy-azetidin-1-carboxamid
N-[4-(3-Cyanophenyl)-5-(2,6-dimethyl-4-pyridyl)thiazol-2-yl]-4-(2-hydroxyethyl)-3-oxo-piperazin-1-carboxamid
4,4-Dicyano-N-[4-(3-cyanophenyl)-5-(2,6-dimethyl-4-pyridyl)thiazol-2-yl]piperidin-1-carboxamid
N-[4-(3-Cyanophenyl)-5-(2,6-dimethyl-4-pyridyl)thiazol-2-yl]-3-(1-hydroxy-1-methyl-ethyl)azetidin-1-carboxamid
N-[4-(3-Cyanophenyl)-5-(2,6-dimethyl-4-pyridyl)thiazol-2-yl]-4-(2-hydroxy-2-methylpropyl)piperazin-1-carboxamid
N-[4-(3-Cyanophenyl)-5-(2,6-dimethyl-4-pyridyl)thiazol-2-yl]-1-methyl-1,7-diazaspiro[3.4]octan-7-carboxamid
N-[4-(3-Cyanophenyl)-5-(2,6-dimethyl-4-pyridyl)thiazol-2-yl]-4-(2-hydroxy-1,1-dimethyl-ethyl)piperazin-1-carboxamid
(8aS)-N-[4-(3-Cyanophenyl)-5-(2,6-dimethyl-4-pyridyl)thiazol-2-yl]-3,4,6,7,8,8a-hexahydro-1H-pyrrolo[1,2-a]pyrazin-2-carboxamid
(8aR)-N-[4-(3-Cyanophenyl)-5-(2,6-dimethyl-4-pyridyl)thiazol-2-yl]-3,4,6,7,8,8a-hexahydro-1H-pyrrolo[1,2-a]pyrazin-2-carboxamid
N-[4-(3-Cyanophenyl)-5-(2,6-dimethyl-4-pyridyl)thiazol-2-yl]-4-(oxetan-3-yl)piperazin-1-carboxamid
(3S)-N-[4-(3-Cyanophenyl)-5-(2,6-dimethyl-4-pyridyl)thiazol-2-yl]-3-hydroxy-pyrrolidin-1-carboxamid
4-Cyano-N-[4-(3-cyanophenyl)-5-(2,6-dimethyl-4-pyridyl)thiazol-2-yl]-4-(methoxymethyl)piperidin-1-carboxamid
4-Cyano-N-[4-(3-cyanophenyl)-5-(2,6-dimethyl-4-pyridyl)thiazol-2-yl]-4-fluor-piperidin-1-carboxamid
(3S)-3-Cyano-N-[4-(3-cyanophenyl)-5-(2,6-dimethyl-4-pyridyl)thiazol-2-yl]pyrrolidin-1-carboxamid
N-[4-(3-Cyanophenyl)-5-(2,6-dimethyl-4-pyridyl)thiazol-2-yl]-3-hydroxy-3-methyl-azetidin-1-carboxamid
(2R)-4-[[5-(2-Chlor-6-methyl-4-pyridyl)-4-(3-cyanophenyl)thiazol-2-yl]carbamoyl]piperazin-2-carbonsäure
Methyl-(2R)-4-[[5-(2-chlor-6-methyl-4-pyridyl)-4-(3-cyanophenyl)thiazol-2-yl]carbamoyl]piperazin-2-carboxylat
(3S)-N-[5-(2-Chlor-6-methyl-4-pyridyl)-4-(3-cyanophenyl)thiazol-2-yl]-3-(hydroxymethyl)piperazin-1-carboxamid
(3R)-N-[5-(2-Chlor-6-methyl-4-pyridyl)-4-(3-cyanophenyl)thiazol-2-yl]-3-(hydroxymethyl)piperazin-1-carboxamid
(3S)-N-[5-(2-Chlor-6-methyl-4-pyridyl)-4-(3-cyanophenyl)thiazol-2-yl]-3-methylpiperazin-1-carboxamid
(3R)-N-[5-(2-Chlor-6-methyl-4-pyridyl)-4-(3-cyanophenyl)thiazol-2-yl]-3-methylpiperazin-1-carboxamid
(1S,4S)-N-[5-(2-Chlor-6-methyl-4-pyridyl)-4-(3-cyanophenyl)thiazol-2-yl]-2,5-diazabicyclo[2.2.1]heptan-2-carboxamid
(3R)-N1-[5-(2-Chlor-6-methyl-4-pyridyl)-4-(3-cyanophenyl)thiazol-2-yl]piperazin-1,3-dicarboxamid
(3S)-N1-[5-(2-Chlor-6-methyl-4-pyridyl)-4-(3-cyanophenyl)thiazol-2-yl]piperazin-1,3-dicarboxamid
4-Amino-N-[5-(2-chlor-6-methyl-4-pyridyl)-4-(3-cyanophenyl)thiazol-2-yl]-4-methylpiperidin-1-carboxamid
4-Cyano-N-[4-(3-cyanophenyl)-5-(2,6-dimethyl-4-pyridyl)thiazol-2-yl]-4-methylpiperidin-1-carboxamid
(3S)-N-[4-(3-cyanophenyl)-5-(2,6-dimethyl-4-pyridyl)thiazol-2-yl]-3-methylsulfonyl-pyrrolidin-1-carboxamid
N-[4-(3-cyanophenyl)-5-(2,6-dimethyl-4-pyridyl)thiazol-2-yl]-2-oxa-7-azaspiro[3.5]nonan-7-carboxamid
N-[5-(2-Chlor-6-methyl-4-pyridyl)-4-(3-cyanophenyl)thiazol-2-yl]-2-oxa-6-azaspiro[3.3]heptan-6-carboxamid
(1R,4R)-5-Acetyl-N-[4-(3-cyanophenyl)-5-(2,6-dimethyl-4-pyridyl)thiazol-2-yl]-2,5-diazabicyclo[2.2.1]heptan-2-carboxamid
(1S,4S)-N-[4-(3-Cyanophenyl)-5-(2,6-dimethyl-4-pyridyl)thiazol-2-yl]-5-methyl-2,5-diazabicyclo[2.2.1]heptan-2-carboxamid
N-[4-(3-Cyanophenyl)-5-(2,6-dimethyl-4-pyridyl)thiazol-2-yl]-3-methyl-2-oxo-1,3,8-triazaspiro[4.5]decan-8-carboxamid
N-[5-[2,6-Bis(trideuteriomethyl)-4-pyridyl]-4-(3-cyanophenyl)thiazol-2-yl]-2-oxa-6-azaspiro[3.3]heptan-6-carboxamid
N-[4-(3-Cyanophenyl)-5-(3-fluor-2,6-dimethyl-4-pyridyl)thiazol-2-yl]-2-oxa-6-azaspiro[3.3]heptan-6-carboxamid
(4aS,7aR)-N-[4-(3-Cyanophenyl)-5-[2-(difluormethyl)-6-methyl-4-pyridyl]thiazol-2-yl]-4-methyl-2,3,4a,5,7,7a-hexahydropyrrolo[3,4-b][1,4]oxazin-6-carboxamid
N-[4-(3-Cyanophenyl)-5-[2-(difluormethyl)-6-methyl-4-pyridyl]thiazol-2-yl]-9-methyl-6-oxa-2,9-diazaspiro[4.5]decan-2-carboxamid
N-[4-(3-Cyanophenyl)-5-[2-(difluormethyl)-6-methyl-4-pyridyl]thiazol-2-yl]-6-oxa-2,9-diazaspiro[4.5]decan-2-carboxamid
(4aS,7aS)-N-[4-(3-Cyanophenyl)-5-[2-(difluormethyl)-6-methyl-4-pyridyl]thiazol-2-yl]-4-methyl-2,3,4a,5,7,7a-hexahydropyrrolo[3,4-b][1,4]oxazin-6-carboxamid
N-[4-(3-Cyanophenyl)-5-(2,6-dimethyl-4-pyridyl)thiazol-2-yl]-2-oxo-1,3,8-triazaspiro[4.5]decan-8-carboxamid
3-Cyano-N-[4-(3-cyanophenyl)-5-[2-methyl-6-(oxetan-3-yl)-4-pyridyl]thiazol-2-yl]-3-methyl-pyrrolidin-1-carboxamid
4-Cyano-N-[4-(3-cyanophenyl)-5-[2-methyl-6-(oxetan-3-yl)-4-pyridyl]thiazol-2-yl]-4-methyl-piperidin-1-carboxamid
N-[4-(3-Cyanophenyl)-5-(2,6-dimethyl-4-pyridyl)thiazol-2-yl]-3-morpholino-azetidin-1-carboxamid
N-[4-(3-Cyanophenyl)-5-(2,6-dimethyl-4-pyridyl)thiazol-2-yl]-4-tetrahydropyran-4-yl-piperazin-1-carboxamid
N-[4-(3-Cyanophenyl)-5-(2,6-dimethyl-4-pyridyl)thiazol-2-yl]-3-pyrrolidin-1-yl-azetidin-1-carboxamid
N-[4-(3-Cyanophenyl)-5-(2,6-dimethyl-4-pyridyl)thiazol-2-yl]-3-(dimethylamino)azetidin-1-carboxamid
N-[4-(3-Cyanophenyl)-5-(2,6-dimethyl-4-pyridyl)thiazol-2-yl]-4-(morpholinomethyl)piperidin-1-carboxamid
N-[4-(3-Cyanophenyl)-5-(2,6-dimethyl-4-pyridyl)thiazol-2-yl]-3-methyl-2-oxo-1-oxa-3,8-diazaspiro[4.5]decan-8-carboxamid
N-[4-(3-Cyanophenyl)-5-(2,6-dimethyl-4-pyridyl)thiazol-2-yl]-3-methyl-3,6-diazabicyclo[3.2.0]heptan-6-carboxamid
N-[4-(3-Cyanophenyl)-5-(2,6-dimethyl-4-pyridyl)thiazol-2-yl]-1,4-diazabicyclo[3.2.1]octan-4-carboxamid
N-[4-(3-Cyanophenyl)-5-(2,6-dimethyl-4-pyridyl)thiazol-2-yl]-3-oxo-5,6,8,8a-tetrahydro-1H-oxazolo[3,4-a]pyrazin-7-carboxamid
N-[4-(3-Cyanophenyl)-5-(2,6-dimethyl-4-pyridyl)thiazol-2-yl]-2,2-dioxo-3,4,6,7,9,9a-hexahydro-1H-pyrazino[2,1-c][1,4]thiazin-8-carboxamid
N-[4-(3-Cyanophenyl)-5-(2,6-dimethyl-4-pyridyl)thiazol-2-yl]-4-oxo-3,6,7,8,9,9a-hexahydro-1H-pyrido[1,2-a]pyrazin-2-carboxamid
N-[4-(3-Cyanophenyl)-5-(2,6-dimethyl-4-pyridyl)thiazol-2-yl]-9-oxo-3,4,6,7,8,9a-hexahydro-1H-pyrazino[1,2-a]pyrazin-2-carboxamid
N-[4-(3-Cyanophenyl)-5-(2,6-dimethyl-4-pyridyl)thiazol-2-yl]-6-oxo-1,3,4,8,9,9a-hexahydropyrazino[1,2-c][1,3]oxazin-2-carboxamid
N-[4-(3-Cyanophenyl)-5-(2,6-dimethyl-4-pyridyl)thiazol-2-yl]-6-oxo-1,3,4,7,8,8a-hexahydropyrrolo[1,2-a]pyrazin-2-carboxamid
N-[4-(3-Cyanophenyl)-5-[2-(difluormethyl)-6-methyl-4-pyridyl]thiazol-2-yl]-1-oxo-1,4-thiazinan-4-carboxamid
N-[4-(3-Cyanophenyl)-5-(2,6-dimethyl-4-pyridyl)thiazol-2-yl]-3-sulfamoyl-pyrrolidin-1-carboxamid
N-[4-(3-Cyanophenyl)-5-(2,6-dimethyl-4-pyridyl)thiazol-2-yl]-3-oxo-2,7-diazaspiro[3.5]nonan-7-carboxamid
N-[4-(3-Cyanophenyl)-5-(2,6-dimethyl-4-pyridyl)thiazol-2-yl]-4-pyrazol-1-yl-piperidin-1-carboxamid
N-[4-(3-Cyanophenyl)-5-(2,6-dimethyl-4-pyridyl)thiazol-2-yl]-4-(1,2,4-oxadiazol-3-yl)piperidin-1-carboxamid
N-[4-(3-Cyanophenyl)-5-(2,6-dimethyl-4-pyridyl)thiazol-2-yl]-1,3-dioxo-5,6,8,8a-tetrahydroimidazo[1,5-a]pyrazin-7-carboxamid
N-[4-(3-Cyanophenyl)-5-(2,6-dimethyl-4-pyridyl)thiazol-2-yl]-1-oxo-2,8-diazaspiro[4.5]decan-8-carboxamid
N-[4-(3-Cyanophenyl)-5-(2,6-dimethyl-4-pyridyl)thiazol-2-yl]-4-(1H-imidazol-2-yl)piperidin-1-carboxamid
N-[4-(3-Cyanophenyl)-5-(2,6-dimethyl-4-pyridyl)thiazol-2-yl]-10-oxo-3,9-diazaspiro[5.5]undecan-3-carboxamid
N-[4-(3-Cyanophenyl)-5-(2,6-dimethyl-4-pyridyl)thiazol-2-yl]-6,8-dihydro-5H-imidazo[1,2-a]pyrazin-7-carboxamid
N1-[4-(3-Cyanophenyl)-5-(2,6-dimethyl-4-pyridyl)thiazol-2-yl]-N4-methyl-piperidin-1,4-dicarboxamid
N-[4-(3-Cyanophenyl)-5-(2,6-dimethyl-4-pyridyl)thiazol-2-yl]-8-oxa-2-azaspiro[4.5]decan-2-carboxamid
(3S)-N1-[4-(3-Cyanophenyl)-5-(2,6-dimethyl-4-pyridyl)thiazol-2-yl]pyrrolidin-1,3-dicarboxamid
N-[4-(3-Cyanophenyl)-5-(2,6-dimethyl-4-pyridyl)thiazol-2-yl]-4-(2-oxoimidazolidin-1-yl)piperidin-1-carboxamid
N-[4-(3-Cyanophenyl)-5-(2,6-dimethyl-4-pyridyl)thiazol-2-yl]-4-(1,2,4-oxadiazol-5-yl)piperidin-1-carboxamid
N-[4-(3-Cyanophenyl)-5-(2,6-dimethyl-4-pyridyl)thiazol-2-yl]-1-methylimino-1-oxo-1,4-thiazinan-4-carboxamid
N1-[4-(3-Cyanophenyl)-5-(2,6-dimethyl-4-pyridyl)thiazol-2-yl]-N4-(2-hydroxyethyl)piperidin-1,4-dicarboxamid
N-[4-(3-Cyanophenyl)-5-(2,6-dimethyl-4-pyridyl)thiazol-2-yl]-6-oxo-7-oxa-2,5-diazaspiro[3.5]nonan-2-carboxamid
N-[4-(3-Cyanophenyl)-5-(2,6-dimethyl-4-pyridyl)thiazol-2-yl]-6,9-dioxo-1,3,4,7,8,9a-hexahydropyrazino[1,2-a]pyrazin-2-carboxamid
(3R)-N-[4-(3-Cyanophenyl)-5-(2,6-dimethyl-4-pyridyl)thiazol-2-yl]-3-(1-hydroxy-1-methyl-ethyl)pyrrolidin-1-carboxamid
N-[4-(3-Cyanophenyl)-5-(2,6-dimethyl-4-pyridyl)thiazol-2-yl]-4-oxo-6,7,9,9a-tetrahydro-1H-pyrazino[2,1-c][1,4]oxazin-8-carboxamid
N-[4-(3-Cyanophenyl)-5-(2,6-dimethyl-4-pyridyl)thiazol-2-yl]-2-methyl-3-oxo-2,8-diazaspiro[4.5]decan-8-carboxamid
N-[4-(3-Cyanophenyl)-5-(2,6-dimethyl-4-pyridyl)thiazol-2-yl]-8-methyl-6,9-dioxo-3,4,7,9a-tetrahydro-1H-pyrazino[1,2-a]pyrazin-2-carboxamid
N-[4-(3-Cyanophenyl)-5-(2,6-dimethyl-4-pyridyl)thiazol-2-yl]-3-oxo-2,8-diazaspiro[4.5]decan-8-carboxamid
N-[4-(3-Cyanophenyl)-5-(2,6-dimethyl-4-pyridyl)thiazol-2-yl]-3-oxo-2,9-diazaspiro[5.5]undecan-9-carboxamid
4-Cyano-N-[4-(3-cyanophenyl)-5-(2,6-dimethyl-4-pyridyl)thiazol-2-yl]-4-(1-hydroxy-1-methyl-ethyl)piperidin-1-carboxamid
N-[4-(3-Cyanophenyl)-5-(2,6-dimethyl-4-pyridyl)thiazol-2-yl]-6-oxo-3,4,7,8,9,9a-hexahydro-1H-pyrazino[1,2-c]pyrimidin-2-carboxamid
N-[4-(3-Cyanophenyl)-5-(2,6-dimethyl-4-pyridyl)thiazol-2-yl]-6-oxo-3,4,7,8,9,9a-hexahydro-1H-pyrido[1,2-a]pyrazin-2-carboxamid
N-[4-(3-Cyanophenyl)-5-(2,6-dimethyl-4-pyridyl)thiazol-2-yl]-6-hydroxy-6-methyl-2-azaspiro[3.3]heptan-2-carboxamid
N-[4-(3-Cyanophenyl)-5-(2,6-dimethyl-4-pyridyl)thiazol-2-yl]-2-oxo-1,8-diazaspiro[4.5]decan-8-carboxamid
N-[4-(3-Cyanophenyl)-5-(2,6-dimethyl-4-pyridyl)thiazol-2-yl]-2,2-dioxo-2λ^{6}-thia-6-azaspiro[3.3]heptan-6-carboxamid
N-[4-(3-Cyanophenyl)-5-(2,6-dimethyl-4-pyridyl)thiazol-2-yl]-2-(1-hydroxy-1-methyl-ethyl)morpholin-4-carboxamid
(3S)-N-[4-(3-Cyanophenyl)-5-(2,6-dimethyl-4-pyridyl)thiazol-2-yl]-3-(1-hydroxy-1-methyl-ethyl)pyrrolidin-1-carboxamid
(3aR,6aS)-N-[4-(3-Cyanophenyl)-5-(2,6-dimethyl-4-pyridyl)thiazol-2-yl]-1,3,3a,4,6,6a-hexahydrofuro[3,4-c]pyrrol-5-carboxamid
N-[4-(3-Cyanophenyl)-5-(2,6-dimethyl-4-pyridyl)thiazol-2-yl]-2-oxo-3-oxa-1,9-diazaspiro[5.5]undecan-9-carboxamid
(1S,5S)-N-[4-(3-Cyanophenyl)-5-(2,6-dimethyl-4-pyridyl)thiazol-2-yl]-6-methyl-3,6-diazabicyclo[3.2.2]nonan-3-carboxamid
N-[4-(3-Cyanophenyl)-5-(2,6-dimethyl-4-pyridyl)thiazol-2-yl]-1-methyl-2-oxo-1,3,8-triazaspiro[4.5]decan-8-carboxamid
(3aS,6aS)-N-[4-(3-cyanophenyl)-5-(2,6-dimethyl-4-pyridyl)thiazol-2-yl]-3-oxo-1,2,3a,4,6,6a-hexahydropyrrolo[3,4-c]pyrrol-5-carboxamid
N-[4-(3-Cyanophenyl)-5-(2,6-dimethyl-4-pyridyl)thiazol-2-yl]-6-oxa-2-azaspiro[3.4]octan-2-carboxamid
N-[4-(3-Cyanophenyl)-5-(2,6-dimethyl-4-pyridyl)thiazol-2-yl]-7-oxa-2-azaspiro[3.5]nonan-2-carboxamid
N-[4-(3-Cyanophenyl)-5-(2,6-dimethyl-4-pyridyl)thiazol-2-yl]-6-oxo-2,7-diazaspiro[3.4]octan-2-carboxamid
N-[4-(3-Cyanophenyl)-5-(2,6-dimethyl-4-pyridyl)thiazol-2-yl]-2-oxo-1-oxa-3,8-diazaspiro[4.5]decan-8-carboxamid
N-[4-(3-Cyanophenyl)-5-(2,6-dimethyl-4-pyridyl)thiazol-2-yl]-6-oxo-2,5,7-triazaspiro[3.4]octan-2-carboxamid
N-[4-(3-Cyanophenyl)-5-(2,6-dimethyl-4-pyridyl)thiazol-2-yl]-2-oxo-1,9-diazaspiro[5.5]undecan-9-carboxamid
N-[4-(3-Cyanophenyl)-5-(2,6-dimethyl-4-pyridyl)thiazol-2-yl]-5-oxo-2,6-diazaspiro[3.4]octan-2-carboxamid
N-[4-(3-Cyanophenyl)-5-(2,6-dimethyl-4-pyridyl)thiazol-2-yl]-3-methyl-2,4-dioxo-1,3,8-triazaspiro[4.5]decan-8-carboxamid
N-[4-(3-Cyanophenyl)-5-(2,6-dimethyl-4-pyridyl)thiazol-2-yl]-4-ethyl-piperazin-1-carboxamid
N-[4-(3-Cyanophenyl)-5-[2-methyl-6-(trifluormethyl)-4-pyridyl]thiazol-2-yl]-4-(oxetan-3-yl)piperazin-1-carboxamid
1-[[4-(3-Cyanophenyl)-5-(2,6-dimethyl-4-pyridyl)thiazol-2-yl]carbamoyl]piperidin-4-carbonsäure
(3S)-1-[[4-(3-Cyanophenyl)-5-(2,6-dimethyl-4-pyridyl)thiazol-2-yl]carbamoyl]pyrrolidin-3-carbonsäure
3-Cyano-N-[4-(3-cyanophenyl)-5-(2,6-dimethyl-4-pyridyl)thiazol-2-yl]-3-(hydroxymethyl)azetidin-1-carboxamid
(3R)-1-[[4-(3-Cyanophenyl)-5-(2,6-dimethyl-4-pyridyl)thiazol-2-yl]carbamoyl]pyrrolidin-3-carbonsäure
4-Cyano-N-[4-(3-cyanophenyl)-5-(3-fluor-2,6-dimethyl-4-pyridyl)thiazol-2-yl]-4-methyl-piperidin-1-carboxamid
N-[4-(3-Cyanophenyl)-5-(3-fluor-2,6-dimethyl-4-pyridyl)thiazol-2-yl]-3-hydroxy-3-methyl-azetidin-1-carboxamid
3-Cyano-N-[4-(3-cyanophenyl)-5-(3-fluor-2,6-dimethyl-4-pyridyl)thiazol-2-yl]-3-methyl-azetidin-1-carboxamid
N-[4-(3-Cyanophenyl)-5-(3-fluor-2,6-dimethyl-4-pyridyl)thiazol-2-yl]-3-oxo-2,7-diazaspiro[3.5]nonan-7-carboxamid
3-Cyano-N-[4-(3-Cyanophenyl)-5-(2,6-dimethyl-4-pyridyl)thiazol-2-yl]-3-methyl-azetidin-1-carboxamid
1-[[4-(3-Cyanophenyl)-5-(2,6-dimethyl-4-pyridyl)thiazol-2-yl]carbamoyl]azetidin-3-carbonsäure
(2S)-N-[4-(3-Cyanophenyl)-5-(2,6-dimethyl-4-pyridyl)thiazol-2-yl]-2-(hydroxymethyl)pyrrolidin-1-carboxamid
(2S)-N-[4-(3-Cyanophenyl)-5-(2,6-dimethyl-4-pyridyl)thiazol-2-yl]-2-methyl-azetidin-1-carboxamid
(2S)-N-[4-(3-Cyanophenyl)-5-(2,6-dimethyl-4-pyridyl)thiazol-2-yl]-2-(hydroxymethyl)azetidin-1-carboxamid
(3R)-N-[4-(3-Cyanophenyl)-5-(2,6-dimethyl-4-pyridyl)thiazol-2-yl]-3-methylmorpholin-4-carboxamid
(3S)-N-[4-(3-Cyanophenyl)-5-(2,6-dimethyl-4-pyridyl)thiazol-2-yl]-3-(1-hydroxy-1-methyl-ethyl)piperazin-1-carboxamid
(3S)-N-[4-(3-Cyanophenyl)-5-(2,6-dimethyl-4-pyridyl)thiazol-2-yl]-3-(hydroxymethyl)morpholin-4-carboxamid
N-[4-(3-Cyanophenyl)-5-(2,6-dimethyl-4-pyridyl)thiazol-2-yl]-2-methyl-1-oxo-2,8-diazaspiro[4.5]decan-8-carboxamid
(2R)-N-[4-(3-Cyanophenyl)-5-(2,6-dimethyl-4-pyridyl)thiazol-2-yl]-2-methyl-azetidin-1-carboxamid
N-[4-(3-Cyano-2-methyl-phenyl)-5-(2,6-dimethyl-4-pyridyl)thiazol-2-yl]-2-oxa-6-azaspiro[3.3]heptan-6-carboxamid
(3R)-N1-[4-(3-Cyanophenyl)-5-(2,6-dimethyl-4-pyridyl)thiazol-2-yl]pyrrolidin-1,3-dicarboxamid
N-[4-(3-cyanophenyl)-5-(2,6-dimethyl-4-pyridyl)thiazol-2-yl]-2-oxa-7-azaspiro[4.4]nonan-7-carboxamid
N-[4-(3-Cyanophenyl)-5-(2,6-dimethyl-4-pyridyl)thiazol-2-yl]-2-oxa-7-azaspiro[3.4]octan-7-carboxamid
(2S)-N-[4-(3-Cyanophenyl)-5-(2,6-dimethyl-4-pyridyl)thiazol-2-yl]-2-(1-hydroxy-1-methyl-ethyl)azetidin-1-carboxamid
(2R)-N-[4-(3-Cyanophenyl)-5-(2,6-dimethyl-4-pyridyl)thiazol-2-yl]-2-(1-hydroxy-1-methyl-ethyl)azetidin-1-carboxamid
N-[4-(3-Cyanophenyl)-5-(2,6-dimethyl-4-pyridyl)thiazol-2-yl]-3-morpholino-pyrrolidin-1-carboxamid
(3R)-N-[4-(3-Cyanophenyl)-5-(2,6-dimethyl-4-pyridyl)thiazol-2-yl]-3-methyl-5-oxo-piperazin-1-carboxamid
(3S)-N-[4-(3-Cyanophenyl)-5-(2,6-dimethyl-4-pyridyl)thiazol-2-yl]-3-methyl-morpholin-4-carboxamid
N-[4-(3-Cyanophenyl)-5-(2,6-dimethyl-4-pyridyl)thiazol-2-yl]-6-oxa-1-azaspiro[3.3]heptan-1-carboxamid
(3R)-N-[4-(3-Cyanophenyl)-5-(2,6-dimethyl-4-pyridyl)thiazol-2-yl]-3-(hydroxymethyl)morpholin-4-carboxamid
N-[4-(3-Cyanophenyl)-5-(2,6-dimethyl-4-pyridyl)thiazol-2-yl]-1,3-dimethyl-2-oxo-1,3,8-triazaspiro[4.5]decan-8-carboxamid
(3R)-N-[4-(3-cyanophenyl)-5-(2,6-dimethyl-4-pyridyl)thiazol-2-yl]-3-methylsulfonyl-pyrrolidin-1-carboxamid
(3S)-N-[4-(3-Cyanophenyl)-5-(2,6-dimethyl-4-pyridyl)thiazol-2-yl]-3-methyl-5-oxo-piperazin-1-carboxamid
(2R)-N-[4-(3-Cyanophenyl)-5-(2,6-dimethyl-4-pyridyl)thiazol-2-yl]-2-(hydroxymethyl)azetidin-1-carboxamid
N-[4-(3-Cyanophenyl)-5-(2,6-dimethyl-4-pyridyl)thiazol-2-yl]-6-methyl-5-oxo-2,6-diazaspiro[3.4]octan-2-carboxamid
N-[4-(3-Cyanophenyl)-5-(2,6-dimethyl-4-pyridyl)thiazol-2-yl]-1-oxa-6-azaspiro[3.3]heptan-6-carboxamid
N-[4-(3-Cyanophenyl)-5-(2,6-dimethyl-4-pyridyl)thiazol-2-yl]-1-methyl-2-oxo-1,8-diazaspiro[4.5]decan-8-carboxamid
N-[4-(3-Cyanophenyl)-5-(2,6-dimethyl-4-pyridyl)thiazol-2-yl]-4-methylsulfonyl-piperidin-1-carboxamid
N-[4-(3-Cyanophenyl)-5-(2,6-dimethyl-4-pyridyl)thiazol-2-yl]-4-hydroxy-4-methylpiperidin-1-carboxamid
N-[4-(3-Cyanophenyl)-5-[2-(difluormethyl)-6-methyl-4-pyridyl]thiazol-2-yl]-2-oxa-6-azaspiro[3.3]heptan-6-carboxamid
(4aR,7aS)-N-[4-(3-Cyanophenyl)-5-(2,6-dimethyl-4-pyridyl)thiazol-2-yl]-4-methyl-2,3,4a,5,7,7a-hexahydropyrrolo[3,4-b][1,4]oxazin-6-carboxamid
N-[4-(3-Cyanophenyl)-5-[2-methyl-6-(trifluormethyl)-4-pyridyl]thiazol-2-yl]-2-oxa-6-azaspiro[3.3]heptan-6-carboxamid
N-[4-(3-Cyanophenyl)-5-(2,6-dimethyl-4-pyridyl)thiazol-2-yl]piperazin-1-carboxamid
(3R)-N-[4-(3-Cyanophenyl)-5-(2,6-dimethyl-4-pyridyl)thiazol-2-yl]-3-methyl-piperazin-1-carboxamid
N-[4-(3-Cyanophenyl)-5-(2,6-dimethyl-4-pyridyl)thiazol-2-yl]-5,8-diazaspiro[3.5]nonan-5-carboxamid
trans-N-[4-(3-Cyanophenyl)-5-(2,6-dimethyl-4-pyridyl)thiazol-2-yl]-2,5-dimethyl-piperazin-1-carboxamid
4-Amino-4-cyano-N-[4-(3-cyanophenyl)-5-(2,6-dimethyl-4-pyridyl)thiazol-2-yl]piperidin-1-carboxamid
N-[4-(3-Cyanophenyl)-5-(2,6-dimethyl-4-pyridyl)thiazol-2-yl]-1-oxa-4,9-diazaspiro[5.5]undecan-9-carboxamid
N-[4-(3-Cyanophenyl)-5-(2,6-dimethyl-4-pyridyl)thiazol-2-yl]-3,8-diazabicyclo[3.2.1]octan-8-carboxamid
N-[4-(3-Cyanophenyl)-5-(2,6-dimethyl-4-pyridyl)thiazol-2-yl]-3,6-diazabicyclo[3.1.1]heptan-6-carboxamid
(1R,4R)-N-[4-(3-Cyanophenyl)-5-(2,6-dimethyl-4-pyridyl)thiazol-2-yl]-2,5-diazabicyclo[2.2.2]octan-2-carboxamid
cis-(2S,5S)-N-[4-(3-Cyanophenyl)-5-(2,6-dimethyl-4-pyridyl)thiazol-2-yl]-2,5-dimethyl-piperazin-1-carboxamid
(2R,6R)-N-[4-(3-Cyanophenyl)-5-(2,6-dimethyl-4-pyridyl)thiazol-2-yl]-2,6-dimethyl-piperazin-1-carboxamid
N-[4-(3-Cyanophenyl)-5-(2,6-dimethyl-4-pyridyl)thiazol-2-yl]-2,2-dimethyl-piperazin-1-carboxamid
1-Acetylimino-N-[4-(3-Cyanophenyl)-5-(2,6-dimethyl-4-pyridyl)thiazol-2-yl]-1-oxo-1,4-thiazinan-4-carboxamid
N-[4-(3-Cyanophenyl)-5-[2-(hydroxymethyl)-6-methyl-4-pyridyl]thiazol-2-yl]-2-oxa-6-azaspiro[3.3]heptan-6-carboxamid
N-[4-(3-Cyanophenyl)-5-[2-(hydroxymethyl)-6-methyl-4-pyridyl]thiazol-2-yl]-3-oxo-2,7-diazaspiro[3.5]nonan-7-carboxamid
(3R)-N-[4-(3-Cyanophenyl)-5-[2-(hydroxymethyl)-6-methyl-4-pyridyl]thiazol-2-yl]-3-(1-hydroxy-1-methyl-ethyl)pyrrolidin-1-carboxamid
N-[4-(3-Cyanophenyl)-5-[2-(hydroxymethyl)-6-methyl-4-pyridyl]thiazol-2-yl]-1-oxo-1,4-thiazinan-4-carboxamid
N-[4-(3-Cyanophenyl)-5-[2-(hydroxymethyl)-6-methyl-4-pyridyl]thiazol-2-yl]-4-(oxetan-3-yl)piperazin-1-carboxamid
N-[4-(3-Cyanophenyl)-5-[2-(hydroxymethyl)-6-methyl-4-pyridyl]thiazol-2-yl]-1,3-dimethyl-2,4-dioxo-1,3,8-triazaspiro[4.5]decan-8-carboxamid
4-Cyano-N-[4-(3-cyanophenyl)-5-[2-(hydroxymethyl)-6-methyl-4-pyridyl]thiazol-2-yl]piperidin-1-carboxamid
4-Cyano-N-[4-(3-cyanophenyl)-5-[2-(hydroxymethyl)-6-methyl-4-pyridyl]thiazol-2-yl]-4-(hydroxymethyl)piperidin-1-carboxamid
4,4-Dicyano-N-[4-(3-cyanophenyl)-5-[2-(hydroxymethyl)-6-methyl-4-pyridyl]thiazol-2-yl]piperidin-1-carboxamid
4-Cyano-N-[4-(3-cyanophenyl)-5-[2-(hydroxymethyl)-6-methyl-4-pyridyl]thiazol-2-yl]-4-methoxy-piperidin-1-carboxamid
N-[4-(3-Cyanophenyl)-5-(3-fluor-2,6-dimethyl-4-pyridyl)thiazol-2-yl]-1-oxo-1,4-thiazinan-4-carboxamid
1-Cyanoimino-N-[4-(3-Cyanophenyl)-5-(2,6-dimethyl-4-pyridyl)thiazol-2-yl]-1-oxo-1,4-thiazinan-4-carboxamid
4-Cyano-N-[4-(3-cyanophenyl)-5-[2-(1-hydroxy-1-methyl-ethyl)-6-methyl-4-pyridyl]thiazol-2-yl]-4-methyl-piperidin-1-carboxamid
N-[4-(3-Cyanophenyl)-5-[2-(1-hydroxy-1-methyl-ethyl)-6-methyl-4-pyridyl]thiazol-2-yl]-6-oxa-2-azaspiro[3.4]octan-2-carboxamid
N-[4-(3-Cyanophenyl)-5-[2-(1-hydroxy-1-methyl-ethyl)-6-methyl-4-pyridyl]thiazol-2-yl]-2-oxa-6-azaspiro[3.3]heptan-6-carboxamid
N-[4-(3-Cyanophenyl)-5-[2-(1-hydroxyethyl)-6-methyl-4-pyridyl]thiazol-2-yl]-2-oxa-6-azaspiro[3.3]heptan-6-carboxamid
rac-(3S,5R)-N-[4-(3-Cyanophenyl)-5-(2,6-dimethyl-4-pyridyl)thiazol-2-yl]-3,5-dimethyl-piperazin-1-carboxamid
(3S)-N-[4-(3-Cyanophenyl)-5-(2,6-dimethyl-4-pyridyl)thiazol-2-yl]-3-(trifluormethyl)piperazin-1-carboxamid
N-[4-(3-Cyanophenyl)-5-(2,6-dimethyl-4-pyridyl)thiazol-2-yl]-5-oxa-2,8-diazaspiro[3.5]nonan-2-carboxamid
N-[4-(3-Cyanophenyl)-5-(2,6-dimethyl-4-pyridyl)thiazol-2-yl]-3,8-diazabicyclo[3.2.1]octan-3-carboxamid
N-[4-(3-Cyanophenyl)-5-(2,6-dimethyl-4-pyridyl)thiazol-2-yl]-6-oxa-2,9-diazaspiro[4.5]decan-2-carboxamid
(3R)-N-[4-(3-Cyanophenyl)-5-(2,6-dimethyl-4-pyridyl)thiazol-2-yl]-3-(trifluormethyl)piperazin-1-carboxamid
(3S)-3-Cyano-N-[4-(3-cyanophenyl)-5-(2,6-dimethyl-4-pyridyl)thiazol-2-yl]piperazin-1-carboxamid
(3R)-3-Cyano-N-[4-(3-cyanophenyl)-5-(2,6-dimethyl-4-pyridyl)thiazol-2-yl]piperazin-1-carboxamid
N-[4-(3-Cyanophenyl)-5-(2,6-dimethyl-4-pyridyl)thiazol-2-yl]-4,7-diazaspiro[2.5]octan-7-carboxamid
(3S,5S)-N-[4-(3-Cyanophenyl)-5-(2,6-dimethyl-4-pyridyl)thiazol-2-yl]-3,5-dimethyl-piperazin-1-carboxamid
(2S)-N-[4-(3-Cyanophenyl)-5-(2,6-dimethyl-4-pyridyl)thiazol-2-yl]-2-methyl-piperazin-1-carboxamid
(2R)-N-[4-(3-Cyanophenyl)-5-(2,6-dimethyl-4-pyridyl)thiazol-2-yl]-2-(hydroxymethyl)piperazin-1-carboxamid
(2S)-N-[4-(3-Cyanophenyl)-5-(2,6-dimethyl-4-pyridyl)thiazol-2-yl]-2-(hydroxymethyl)piperazin-1-carboxamid
N-[4-(3-Cyanophenyl)-5-(2,6-dimethyl-4-pyridyl)thiazol-2-yl]-3,6-diazabicyclo[3.1.1]heptan-3-carboxamid
(2R)-N-[4-(3-Cyanophenyl)-5-(2,6-dimethyl-4-pyridyl)thiazol-2-yl]-2-methyl-piperazin-1-carboxamid
N-[4-(3-Cyanophenyl)-5-(2,6-dimethyl-4-pyridyl)thiazol-2-yl]-9-oxa-2,6-diazaspiro[4.5]decan-2-carboxamid
(3R)-N-[4-(3-Cyanophenyl)-5-[2-methyl-6-(trifluormethyl)-4-pyridyl]thiazol-2-yl]-3-methyl-piperazin-1-carboxamid
(4aS,7aS)-N-[4-(3-Cyanophenyl)-5-[2-(difluormethyl)-6-methyl-4-pyridyl]thiazol-2-yl]-3,4,4a,5,7,7a-hexahydro-2H-pyrrolo[3,4-b][1,4]oxazin-6-carboxamid
(4aS,7aR)-N-[4-(3-Cyanophenyl)-5-[2-(difluormethyl)-6-methyl-4-pyridyl]thiazol-2-yl]-3,4,4a,5,7,7a-hexahydro-2H-pyrrolo[3,4-b][1,4]oxazin-6-carboxamid
rac-(4aS,7aS)-N-[4-(3-Cyanophenyl)-5-(2,6-dimethyl-4-pyridyl)thiazol-2-yl]-3,4,4a,5,7,7a-hexahydro-2H-pyrrolo[3,4-b][1,4]oxazin-6-carboxamid
N-[4-(3-Cyanophenyl)-5-(2,6-dimethyl-4-pyridyl)thiazol-2-yl]-2-cyclopropyl-piperazin-1-carboxamid
N-[4-(3-Cyanophenyl)-5-(2,6-dimethyl-4-pyridyl)thiazol-2-yl]-2-(cyclopropylmethyl)piperazin-1-carboxamid
N-[4-(3-Cyanophenyl)-5-(2,6-dimethyl-4-pyridyl)thiazol-2-yl]-3,3-dimethyl-piperazin-1-carboxamid
N-[4-(3-Cyanophenyl)-5-(2-methoxy-6-methyl-4-pyridyl)thiazol-2-yl]-1-imino-1-oxo-1,4-thiazinan-4-carboxamid
N-[4-(3-Cyanophenyl)-5-[2-(difluormethyl)-6-methyl-4-pyridyl]thiazol-2-yl]-1-imino-1-oxo-1,4-thiazinan-4-carboxamid
N-[4-(3-Cyanophenyl)-5-(2,6-dichlor-4-pyridyl)thiazol-2-yl]-2-oxa-6-azaspiro[3.3]heptan-6-carboxamid
N-[4-(3-Cyanophenyl)-5-[2-methyl-6-(trifluormethyl)-4-pyridyl]thiazol-2-yl]-1-oxo-1,4-thiazinan-4-carboxamid
N-[4-(3-Cyanophenyl)-5-(2-methoxy-6-methyl-4-pyridyl)thiazol-2-yl]-1-oxo-1,4-thiazinan-4-carboxamid
N-[4-(3-Cyanophenyl)-5-[2-methyl-6-(oxetan-3-yl)-4-pyridyl]thiazol-2-yl]-2-oxa-6-azaspiro[3 .3]heptan-6-carboxamid
N-[5-(2-Cyano-6-methyl-4-pyridyl)-4-(3-cyanophenyl)thiazol-2-yl]-1-oxo-1,4-thiazinan-4-carboxamid
N-[4-(3-Cyanophenyl)-5-[2-(fluormethyl)-6-methyl-4-pyridyl]thiazol-2-yl]-2-oxa-6-azaspiro[3 .3]heptan-6-carboxamid
N-[4-(3-Cyanophenyl)-5-(2-cyclopropyl-6-methyl-4-pyridyl)thiazol-2-yl]-2-oxa-6-azaspiro[3 .3]heptan-6-carboxamid
N-[4-(3-Cyanophenyl)-5-(2-isopropyl-6-methyl-4-pyridyl)thiazol-2-yl]-2-oxa-6-azaspiro[3 .3]heptan-6-carboxamid
N-[4-(3-Cyanophenyl)-5-(2-ethyl-6-methyl-4-pyridyl)thiazol-2-yl]-2-oxa-6-azaspiro[3 .3]heptan-6-carboxamid
N-[4-(3-Cyanophenyl)-5-(2,6-dimethyl-1-oxido-pyridin-1-ium-4-yl)thiazol-2-yl]-2-oxa-6-azaspiro[3.3]heptan-6-carboxamid
4-Cyano-N-[4-(3-cyano-2-methyl-phenyl)-5-(2,6-dimethyl-4-pyridyl)thiazol-2-yl]-4-methyl-piperidin-1-carboxamid
N-[5-(2-Acetamido-6-methyl-4-pyridyl)-4-(3-cyanophenyl)thiazol-2-yl]-2-oxa-6-azaspiro[3 .3]heptan-6-carboxamid
N-[5-[2-(Acetamidomethyl)-6-methyl-4-pyridyl]-4-(3-cyanophenyl)thiazol-2-yl]-2-oxa-6-azaspiro[3.3]heptan-6-carboxamid
N-[4-(3-Cyanophenyl)-5-[2-(methoxymethyl)-6-methyl-4-pyridyl]thiazol-2-yl]-2-oxa-6-azaspiro[3 .3]heptan-6-carboxamid
N-[4-(3-Cyanophenyl)-5-[2-(1-methoxyethyl)-6-methyl-4-pyridyl]thiazol-2-yl]-2-oxa-6-azaspiro[3 .3]heptan-6-carboxamid
N-[5-[2-Chlor-6-(1-hydroxy-1-methyl-ethyl)-4-pyridyl]-4-(3-cyanophenyl)thiazol-2-yl]-2-oxa-6-azaspiro[3 .3]heptan-6-carboxamid
4-Cyano-N-[4-(3-cyanophenyl)-5-[2-(hydroxymethyl)-6-methyl-4-pyridyl]thiazol-2-yl]-4-methyl-piperidin-1-carboxamid
N-[4-(3-Cyanophenyl)-5-[2-(hydroxymethyl)-6-(trifluormethyl)-4-pyridyl]thiazol-2-yl]-2-oxa-6-azaspiro[3 .3]heptan-6-carboxamid
3-Cyano-N-[4-(3-Cyanophenyl)-5-(2,6-dimethyl-4-pyridyl)thiazol-2-yl]-3-methylpyrrolidin-1-carboxamid
N-[5-[2,6-Bis(trideuteriomethyl)-4-pyridyl]-4-(3-cyanophenyl)thiazol-2-yl]-4-cyano-4-methyl-piperidin-1-carboxamid
N-[4-(3-Cyanophenyl)-5-[2-[(1R)-1-hydroxyethyl]-6-methyl-4-pyrid^{y}l]thiazol-2-yl]-2-oxa-6-azaspiro[3 .3]heptan-6-carboxamid
N-[4-(3-Cyanophenyl)-5-[2-[(1S)-1-hydroxyethyl]-6-methyl-4-pyridyl]thiazol-2-yl]-2-oxa-6-azaspiro[3 .3]heptan-6-carboxamid
(3R)-3-Cyano-N-[4-(3-cyanophenyl)-5-(2,6-dimethyl-4-pyridyl)thiazol-2-yl]-3-methylpyrrolidin-1-carboxamid
(3S)-3-Cyano-N-[4-(3-cyanophenyl)-5-(2,6-dimethyl-4-pyridyl)thiazol-2-yl]-3-methylpyrrolidin-1-carboxamid.

13. Pharmazeutische Zusammensetzung, umfassend eine Verbindung gemäß einem beliebigen der Ansprüche 1 bis 12 oder ein pharmazeutisch unbedenkliches Salz davon in Beimengung mit einem pharmazeutisch unbedenklichen Verdünnungsmittel oder Träger.

14. Verbindung gemäß einem beliebigen der Ansprüche 1 bis 12 oder ein pharmazeutisch unbedenkliches Salz davon oder eine pharmazeutische Zusammensetzung gemäß Anspruch 13 zur Verwendung bei Therapie.

15. Verbindung gemäß einem beliebigen der Ansprüche 1 bis 12 oder ein pharmazeutisch unbedenkliches Salz davon oder eine pharmazeutische Zusammensetzung gemäß Anspruch 13 zur Verwendung:
(i) bei der Behandlung eines proliferativen Zustands;
(ii) bei der Behandlung von Krebs;
(iii) bei der Behandlung von Krebs, wobei die Verbindung oder pharmazeutische Zusammensetzung in Kombination mit einem oder mehreren zusätzlichen Antikrebsmitteln verabreicht wird;
(iv) bei der Behandlung von Krebs, wobei die Verbindung oder pharmazeutische Zusammensetzung in Kombination mit einem oder mehreren zusätzlichen Antikrebsmitteln verabreicht wird, ausgewählt aus der Gruppe bestehend aus:
1) anderen Formen von Krebsimmuntherapie und Antikrebs-Chemotherapeutika;
2) Adenosinwegmodulatoren, einschließlich, aber nicht beschränkt auf A2b-Antagonisten, CD73-Inhibitoren und CD39-Inhibitoren;
3) Anti-PD-1- und PDL-1-Antikörper (z. B. Cetrelimab, Pembrolizumab, Nivolumab, Durvalumab, Avelumab und Atezolizumab); und
4) Anti-CTLA4-Antikörper (z. B. Ipilimumab).

## Revendications

1. Composé, ou sel pharmaceutiquement acceptable de celui-ci, répondant à la formule structurelle 1 représentée ci-dessous : 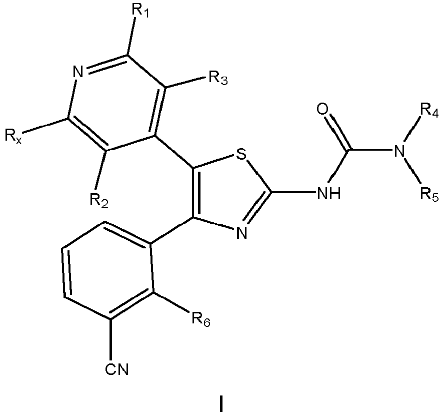 dans lequel :
Rₓ est choisi parmi un méthyle, CD₃, un chloro et CF₃ ;
R₁ est choisi parmi un alkyle en (C1-3), un halogéno, un halogénoalkyle en (C1-3), un halogénoalcoxy en (C1-3), un hydroxyalkyle en (C1-3), un cyano, un cyclopropyle, un oxétan-3-yle, -(CR_{1C}R_{1D})_{q1}-OR_{1A}, -(CR_{1C}R_{1D})_{q1}-C(O)R_{1A}, -(CR_{1C}R_{1D})_{q1}-C(O)OR_{1A}, -(CR_{1C}R_{1D})_{q1}-OC(O)R_{1A}, -(CR_{1C}R_{1D})_{q1}-C(O)N(R_{1B})R_{1A}, -(CR_{1C}R_{1D})_{q1}-N(R_{1B})C(O)R_{1A}, -(CR_{1C}R_{1D})_{q1}-S(O)ₚR_{1A} (où p vaut 0, 1 ou 2), -(CR_{1c}R₁ₚ)_{q1}-SO₂N(R_{1B})R_{1A} ou -(CR_{1C}R_{1D})_{g1}-N(R_{1B})SO₂R_{1A},
dans lequel q1 vaut 0, 1 ou 2 ; et
dans lequel R_{1A} et R_{1B} sont chacun indépendamment choisis parmi un hydrogène, un alkyle en (C1-2), un cycloalkyle en (C3-4) ou un cycloalkyle en (C3-4)-alkyle en (C1-2) ;
dans lequel R_{1C} et R_{1D} sont chacun indépendamment choisis parmi un hydrogène ou un alkyle en (C1-2) ;
R₂ et R₃ sont tous deux un hydrogène ou l'un de R₂ et R₃ est un hydrogène et l'autre est choisi parmi un fluoro, un méthyle ou un méthoxy ;
R₄ et R₅ sont liés de sorte que, conjointement avec l'atome d'azote auquel ils sont attachés, ils forment un cycle hétérocyclique qui est éventuellement substitué sur tout atome de carbone disponible par un ou plusieurs groupes substituants R_{10C},
dans lequel R_{10C} est choisi parmi des substituants oxo, thioxo, halogéno ou cyano, ou un groupe de la formule :
-[CR₇ₐR_{7b}]ₙ-L-Z
dans lequel
n vaut 0 à 6 ;
R₇ₐ et R_{7b} sont chacun indépendamment choisis parmi un hydrogène, un fluoro ou un alkyle en (C1-2) ;
L est absent ou choisi parmi -O-, -S-, -SO-, -SO₂-, -N(Rₐ)-, -C(O)-, -C(O)O-, -OC(O)-, - C(O)N(Rₐ)-, -N(Rₐ)C(O)-, -N(Rₐ)C(O)N(R_{b})-, -C(S)N(Rₐ)-, -N(Rₐ)C(S)-, -N(Rₐ)C(S)N(R_{b})-, - S(O)₂N(Rₐ)- ou -N(Rₐ)SO₂-, Rₐ et R_{b} étant chacun indépendamment choisis parmi un hydrogène ou un alkyle en (C1-2) ; et
Z est choisi parmi un hydrogène, un alkyle en (C1-6), un cycloalkyle en (C3-6), un cycloalkyle en (C3-6)-alkyle en (C1-2), un aryle, un aryle-alkyle en (C1-2), un hétérocyclyle, un hétérocyclyle-alkyle en (C1-2), un hétéroaryle ou un hétéroaryle-alkyle en (C1-2) ; et Z étant éventuellement substitué par un ou plusieurs substituants choisis parmi un alkyle en (C1-6), un halogéno, un halogénoalkyle en (C1-6), un halogénoalcoxy en (C1-6), un cyano, un nitro, -NR_{c}R_{d}, -OR_{c}, -C(O)R_{c}, -C(O)OR_{c}, -OC(O)R_{c}, -C(O)N(R_{c})R_{d}, -N(R_{c})C(O)R_{d}, -S(O)_{y}R_{c} (où y vaut 0, 1 ou 2), -SO₂N(R_{c})R_{d}, -N(R_{c})SO₂R_{d}, -(CH₂)_{z}NRₑR_{d} (où z vaut 1, 2 ou 3) ou un oxo,
dans lequel toute fraction alkyle dans un groupe substituant sur Z est éventuellement en outre substituée par un cyano, un halogéno, un hydroxy, un amino, un oxo, un alkyle en (C1-2) ou un alcoxy en (C1-2) et R_{c} et R_{d} sont chacun indépendamment choisis parmi un hydrogène, un alkyle en (C1-6), un halogénoalkyle en (C1-6) ou un cycloalkyle en (C3-6) ;
et dans lequel tous les atomes N disponibles sont éventuellement sous la forme d'un N-oxyde ou sont éventuellement substitués par un ou plusieurs R_{10N},
dans lequel R_{10N} est -S(O)₂NH₂ ou choisi parmi :
(i) -Z₁ ;
(ii) -L₁ₐ-Z₁ ; ou
(iii) -[CR₈ₐR_{8b}]₁₋₆-L_{1b}-Z₁ ;
dans lequel
R₈ₐ et R_{8b} sont chacun indépendamment choisis parmi un hydrogène, un fluoro ou un alkyle en (C1-2) ;
L₁ₐ est choisi parmi -C(O)-, -S(O)₂-, -C(O)O-, -C(O)N(Rₐ₁)-, -S(O)₂N(Rₐ₁)- ou -N(Rₐ₁)-, Rₐ₁ étant choisi parmi un hydrogène ou un alkyle en (C1-2) ;
L_{1b} est absent ou choisi parmi -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -C(S)N(Rₐ₂)-, -N(Rₐ₂)C(S)-, - N(Rₐ₂)C(S)N(R_{b2})-, -S(O)₂N(Rₐ₂)- ou -N(Rₐ₂)SO₂, Rₐ₂ et R_{b2} étant chacun indépendamment choisis parmi un hydrogène ou un alkyle en (C1-2) ; et
Z₁ est choisi parmi un alkyle en (C1-6), un cycloalkyle en (C3-6), un aryle, un hétérocyclyle, un hétéroaryle, un cycloalkyle en (C3-6)-alkyle en (C1-2), un aryle-alkyle en (C1-2), un hétérocyclyle-alkyle en (C1-2) ou un hétéroaryle-alkyle en (C1-2), à condition que lorsque Z₁ est un groupe hétérocyclyle directement lié à un atome N, il s'agisse d'un hétérocyclyle lié à un carbone ; et Z₁ étant éventuellement substitué par un ou plusieurs substituants choisis parmi un alkyle en (C1-6), un halogéno, halogénoalcoxy en (C1-6), un cyano, un nitro, -NRₑR_{f}, -ORₑ, - C(O)Rₑ, -C(O)ORₑ, -OC(O)Rₑ, -C(O)N(Rₑ)R_{f}, -N(Rₑ)C(O)R_{f}, -S(O)_{y}Rₑ (où y vaut 0, 1 ou 2), - SO₂N(Rₑ)R_{f}, -N(Rₑ)SO₂R_{f}, -(CH₂)_{z}NRₑR_{f} (où z vaut 1, 2 ou 3) ou un oxo, et Rₑ et R_{f} étant chacun indépendamment choisis parmi un hydrogène, un alkyle en (C1-6), un halogénoalkyle en (C1-6) ou un cycloalkyle en (C3-6) ;
et dans lequel tous les atomes S présents dans le cycle hétérocyclique peuvent éventuellement être présents sous forme de S(=O), S(=O)₂ ou S(=O)(=NRₑ), Rₑ étant choisi parmi un hydrogène, un alkyle en (C1-3) ou un alcanoyle en (C2-3) ; et
R₆ est choisi parmi un hydrogène, un halogéno, un méthyle, un méthoxy et un trifluorométhyle.

2. Composé selon la revendication 1, ou sel pharmaceutiquement acceptable de celui-ci, dans lequel l'une quelconque ou plusieurs des affirmations (A) à (C) suivantes s'appliquent :
(A) Rₓ est choisi parmi un méthyle ou CD₃ ;
(B) R₁ est choisi parmi un alkyle en (C1-3), un halogéno, un halogénoalkyle en (C1-3), un halogénoalcoxy en (C1-3), un hydroxyalkyle en (C1-3), un cyano, un cyclopropyle, un oxétan-3-yle, -(CH₂)_{q1}-OR_{1A} et -(CH₂)_{q1}-C(O)R_{1A} ;
dans lequel q1 vaut 0 ou 1 ; et
dans lequel R_{1A} est choisi parmi un hydrogène ou un alkyle en (C1-2) ;
(C) R₁ est choisi parmi un méthyle, CD₃, un isopropyle, un halogéno, un hydroxyalkyle en (C1-3), un cyano, un cyclopropyle, un oxétan-3-yle, -OMe, -CH₂F, -CHF₂ ou-CF₃.

3. Composé selon l'une quelconque des revendications précédentes, ou sel pharmaceutiquement acceptable de celui-ci, dans lequel l'une quelconque ou les deux affirmations (A) et (B) suivantes s'appliquent :
(A) R₂ et R₃ sont tous deux un hydrogène ou l'un de R₂ et R₃ est un hydrogène et l'autre est un fluoro ou un méthyle ;
(B) R₂ et R₃ sont tous deux un hydrogène.

4. Composé selon l'une quelconque des revendications précédentes, ou sel pharmaceutiquement acceptable de celui-ci, dans lequel R₄ et R₅ sont liés de sorte que, conjointement avec l'atome d'azote auquel ils sont attachés, ils forment un cycle hétérocyclique qui est éventuellement substitué sur tout atome de carbone disponible par un ou plusieurs groupes substituants R_{10C},
dans lequel R_{10C} est choisi parmi des substituants oxo, halogéno ou cyano, ou un groupe de la formule :
-[CH₂]ₙ-L-Z
dans lequel
n vaut 0 à 2 ;
L est absent ou choisi parmi -O-, -S-, -SO-, -SO₂-, -N(Rₐ₂)-, -C(O)-, -C(O)O-, -OC(O)-, - C(O)N(Rₐ₂)-, -N(Rₐ₂)C(O)-, -N(Rₐ₂)C(O)N(R_{b2})-, -S(O)₂N(Rₐ₂)- ou -N(Rₐ₂)SO₂-, Rₐ₂ et R_{b2} étant chacun indépendamment choisis parmi un hydrogène ou un méthyle ; et
Z est choisi parmi un hydrogène, un alkyle en (C1-6), un cycloalkyle en (C3-6), un cycloalkyle en (C3-6)-alkyle en (C1-2), un aryle, un hétérocyclyle ou un hétéroaryle ; et Z étant éventuellement substitué par un ou plusieurs substituants choisis parmi un alkyle en (C1-2), un halogéno, un halogénoalkyle en (C1-2), un halogénoalcoxy en (C1-2), un cyano, -NR_{c}R_{d}, -OR_{c}, - C(O)R_{c}, -C(O)OR_{c}, -OC(O)R_{c}, -C(O)N(R_{c})R_{d}, -N(R_{c})C(O)R_{d}, -S(O)_{y}R_{c} (où y vaut 0, 1 ou 2), - SO₂N(R_{c})R_{d}, -N(R_{c})SO₂R_{d}, -(CH₂)_{z}NR_{c}R_{d} (où Z vaut 1 ou 2) ou un oxo ;
dans lequel toute fraction alkyle dans un groupe substituant sur Z₁ est éventuellement en outre substituée par un cyano, un halogéno, un hydroxy, un amino, un oxo, un alkyle en (C1-2) ou un alcoxy en (C1-2) et R_{c} et R_{d} sont chacun indépendamment choisis parmi un hydrogène, un alkyle en (C1-2), un halogénoalkyle en (C1-2) ou un cycloalkyle en (C3-6) ;
et dans lequel tous les atomes N disponibles sont éventuellement sous la forme d'un N-oxyde ou sont éventuellement substitués par un ou plusieurs R_{10N}, R_{10N} étant -S(O)₂NH₂ ou choisi parmi :
(i) -Z₁ ;
(ii) -L₁ₐ-Z₁ ; ou
(iii) -[CR₈ₐR_{8b}]₁₋₂-L_{1b}-Z₁ ;
dans lequel
R₈ₐ et R_{8b} sont tous deux un hydrogène ;
L₁ₐ est choisi parmi -C(O)-, -S(O)₂- ou -S(O)₂N(Rₐ₁)-, Rₐ₁ étant un hydrogène ou un méthyle ;
L_{1b} est absent ou choisi parmi -O-, -S-, -SO-, -SO₂-, -N(Rₐ₂)-, -C(O)-, -C(O)O-, -OC(O)-, -C(O)N(Rₐ₂)-, -N(Rₐ₂)C(O)-, -N(Rₐ₂)C(O)N(R_{b2})-, -S(O)₂N(Rₐ₂)- ou -N(Rₐ₂)SO₂, Rₐ₂ et R_{b2} étant chacun indépendamment choisis parmi un hydrogène ou un méthyle ; et
Z₁ est choisi parmi un alkyle en (C1-6), un cycloalkyle en (C3-6), un aryle, un hétérocyclyle, un hétéroaryle, un cycloalkyle en (C3-6)-alkyle en (C1-2) ou un aryle-alkyle en (C1-2), à condition que lorsque Z₁ est un groupe hétérocyclyle directement lié à un atome N, il s'agisse d'un hétérocyclyle lié à un carbone ; et Z₁ étant éventuellement substitué par un ou plusieurs substituants choisis parmi un alkyle en (C1-2), un halogéno, un halogénoalcoxy en (C1-2), un cyano, un nitro, -NRₑR_{f}, -ORₑ, -C(O)Rₑ, -C(O)ORₑ, -OC(O)Rₑ, -C(O)N(Rₑ)R_{f}, - N(Rₑ)C(O)R_{f}, -S(O)_{y}Rₑ (où y vaut 0, 1 ou 2), -SO₂N(Rₑ)R_{f}, -N(Rₑ)SO₂R_{f}, -(CH₂)_{z}NRₑR_{f} (où z vaut 1, 2 ou 3) ou un oxo, et Rₑ et R_{f} étant chacun indépendamment choisis parmi un hydrogène ou un alkyle en (C1-2) ;
et dans lequel tous les atomes S présents dans le cycle hétérocyclique peuvent éventuellement être présents sous forme de S(=O), S(=O)₂ ou S(=O)(=NRₑ), Rₑ étant choisi parmi un hydrogène, un alkyle en (C1-2) ou un alcanoyle en (2C).

5. Composé selon l'une quelconque des revendications précédentes, ou sel pharmaceutiquement acceptable de celui-ci, dans lequel R₄ et R₅ sont liés de sorte que, conjointement avec l'atome d'azote auquel ils sont attachés, ils forment un cycle hétérocyclique qui est éventuellement substitué sur tout atome de carbone disponible par un ou plusieurs groupes substituants R_{10C},
dans lequel R_{10C} est choisi parmi des substituants oxo, halogéno ou cyano, ou un groupe de la formule :
-[CH₂]ₙ-L-Z
dans lequel
n vaut 0 à 2 ;
L est absent ou choisi parmi -O-, -S-, -SO-, -SO₂-, -N(Rₐ)-, -C(O)-, -C(O)O-, -OC(O)-, - C(O)N(Rₐ)-, -N(Rₐ)C(O)-, -S(O)₂N(Rₐ)- ou -N(Rₐ)SO₂-, Rₐ et R_{b} étant un hydrogène ; et
Z est choisi parmi un hydrogène, un alkyle en (C1-4), un cycloalkyle en (C3-6), un phényle, un hétérocyclyle à 4-7 chaînons ou un hétéroaryle à 5 ou 6 chaînons ; et Z étant éventuellement substitué par un ou plusieurs substituants choisis parmi un alkyle en (C1-2), un halogéno, un halogénoalkyle en (C1-2), un halogénoalcoxy en (C1-2), un cyano, -NR_{c}R_{d}, -OR_{c}, -C(O)R_{c}, - C(O)N(R_{c})R_{d}, -N(R_{c})C(O)R_{d} ou -S(O)_{y}R_{c} (où y vaut 0, 1 ou 2) ou un oxo ;
dans lequel R_{c} et Rₐ sont chacun indépendamment choisis parmi un hydrogène ou un alkyle en (C1-2) ;
et dans lequel tous les atomes N disponibles sont éventuellement sous la forme d'un N-oxyde ou sont éventuellement substitués par un ou plusieurs R_{10N}, R_{10N} étant -S(O)₂NH₂ ou choisi parmi :
(i) -Z₁ ;
(ii) -L₁ₐ-Z₁ ; ou
(iii) -[CR₈ₐR_{8b}]₁₋₂-L_{1b}-Z₁ ;
dans lequel
R₈ₐ et R_{8b} sont tous deux un hydrogène ;
L₁ₐ est choisi parmi -C(O)-, -S(O)₂- ou -S(O)₂N(Rₐ₁)-, Rₐ₁ étant un hydrogène ;
L_{1b} est absent ou choisi parmi -O-, -S-, -SO-, -SO₂-, -N(Rₐ₂)-, -C(O)N(Rₐ₂)-, -N(Rₐ₂)C(O)-, -S(O)₂N(Rₐ₂)- ou -N(Rₐ₂)SO₂, Rₐ₂ étant un hydrogène ; et
Z₁ est choisi parmi un alkyle en (C1-4), un phényle, un hétérocyclyle à 4-7 chaînons ou un hétéroaryle à 5 ou 6 chaînons, à condition que lorsque Z₁ est un groupe hétérocyclyle directement lié à un atome N, il s'agisse d'un hétérocyclyle lié à un carbone ; et Z₁ étant éventuellement substitué par un ou plusieurs substituants choisis parmi un alkyle en (C1-2), un halogéno, un halogénoalcoxy en (C1-2), un cyano, -NRₑR_{f}, -ORₑ, -C(O)Rₑ, -C(O)N(Rₑ)R_{f}, -N(Rₑ)C(O)R_{f}, - S(O)_{y}Rₑ (où y vaut 0, 1 ou 2) ; et Rₑ et R_{f} étant chacun indépendamment choisis parmi un hydrogène ou un méthyle ;
et dans lequel tous les atomes S présents dans le cycle hétérocyclique peuvent éventuellement être présents sous forme de S(=O), S(=O)₂ ou S(=O)(=NRₑ), Rₑ étant choisi parmi un hydrogène, un alkyle en (C1-2) ou un alcanoyle en (2C).

6. Composé selon l'une quelconque des revendications précédentes, ou sel pharmaceutiquement acceptable de celui-ci, dans lequel R₄ et R₅ sont liés de sorte que, conjointement avec l'atome d'azote auquel ils sont attachés, ils forment un cycle hétérocyclique qui est éventuellement substitué sur tout atome de carbone disponible par un ou plusieurs groupes substituants R_{10C},R_{10C} étant choisi parmi un oxo, un halogéno, un cyano, un alkyle en (C1-4) ou un cycloalkyle en (C3-6) ;
et dans lequel tous les atomes N disponibles sont éventuellement sous la forme d'un N-oxyde ou sont éventuellement substitués par un ou plusieurs R_{10N}, R_{10N} étant -S(O)₂NH₂ ou un alkyle en (C1-4),
et dans lequel tous les atomes S présents dans le cycle hétérocyclique peuvent éventuellement être présents sous forme de S(=O), S(=O)₂ ou S(=O)(=NRₑ), Rₑ étant choisi parmi un hydrogène, un méthyle ou un alcanoyle en (2C).

7. Composé selon l'une quelconque des revendications précédentes, ou sel pharmaceutiquement acceptable de celui-ci, dans lequel R₄ et R₅ sont liés de sorte que, conjointement avec l'atome d'azote auquel ils sont attachés, ils forment un cycle hétérocyclique choisi parmi l'une quelconque des options suivantes : 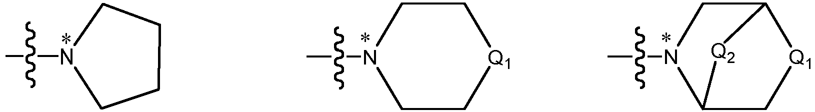 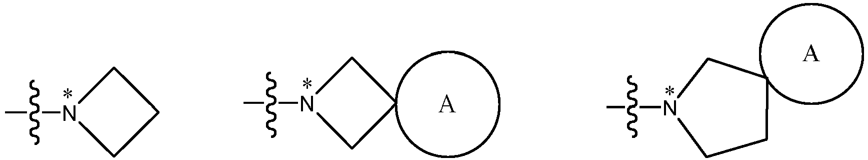 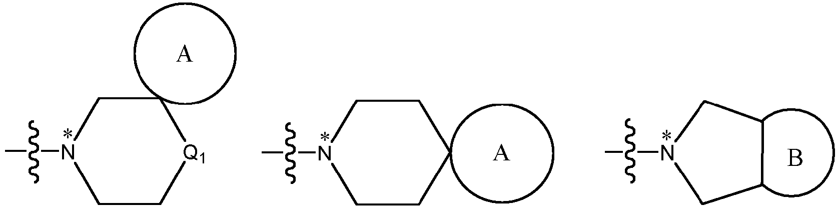 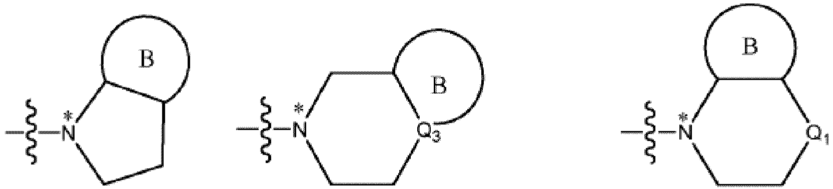
dans lequel * désigne l'atome N auquel R₄ et R₅ sont attachés ;
Q₁ est O, NH, S, S(O), S(O)₂, S(O)(=NRₑ), N-R_{10N}, CH₂, CHR_{10C} ou C(R_{10C})₂ ;
Q₂ est -CH₂-, -CHR_{10C}-, -C(R_{10C})₂-, -CHR_{10C}-CH₂-, -CH₂-CHR_{10C}-, -CHR_{10C}-CHR_{10C}-, - C(R_{10C})_{z}-CH₂-, ou -CH₂-C(R_{10C})_{z}- ;
Q₃ est CH, CR_{10C} ou N ;
le cycle A est un cycle carbocyclique ou hétérocyclique spiro-fusionné à **4,** 5 ou 6 chaînons ;
le cycle B est un cycle carbocyclique ou hétérocyclique fusionné à **4,** 5 ou 6 chaînons ;
dans lequel Rₑ est choisi parmi un hydrogène, un alkyle en (C1-2) ou un alcanoyle en (2C) ;
et dans lequel chacun des systèmes cycliques hétérocycliques est éventuellement substitué par un ou plusieurs groupes substituants R_{10C} ou R_{10N} tels que définis dans l'une quelconque des revendications 1, 4 ou 5.

8. Composé selon l'une quelconque des revendications précédentes, ou sel pharmaceutiquement acceptable de celui-ci, dans lequel R₄ et R₅ sont liés de sorte que, conjointement avec l'atome d'azote auquel ils sont attachés, ils forment un cycle hétérocyclique choisi parmi l'une quelconque des options suivantes : 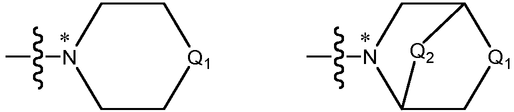
dans lequel * désigne l'atome N auquel R₄ et R₅ sont attachés ;
Q₁ est O, NH, S, S(O), S(O)₂, S(O)(=NRₑ), N-R_{10N}, CH₂, CHR_{10C} ou C(R_{10c})₂ ;
Q₂ est -CH₂- ;
dans lequel Rₑ est choisi parmi un hydrogène, un méthyle ou un alcanoyle en (2C) ;
et R_{10C} ou R_{10N} sont tels que définis dans l'une quelconque des revendications 1, 4 ou 5.

9. Composé selon l'une quelconque des revendications précédentes, ou sel pharmaceutiquement acceptable de celui-ci, dans lequel R₄ et R₅ sont liés de sorte que, conjointement avec l'atome d'azote auquel ils sont attachés, ils forment un cycle hétérocyclique de structure : 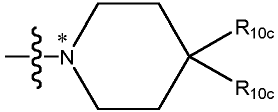
dans lequel * désigne l'atome N auquel R₄ et R₅ sont attachés ;
et chaque R_{10C} est indépendamment choisi parmi les options définies dans l'une quelconque des revendications 1, 4 ou 5 ci-dessus.

10. Composé selon l'une quelconque des revendications précédentes, ou sel pharmaceutiquement acceptable de celui-ci, dans lequel l'une quelconque ou les deux affirmations (A) et (B) suivantes s'appliquent :
(A) R₆ est choisi parmi un hydrogène, un halogéno ou un méthyle ;
(B) R₆ est un hydrogène.

11. Composé selon l'une quelconque des revendications précédentes, ou sel pharmaceutiquement acceptable de celui-ci, ledit composé répondant à la formule IA, IB ou IC : 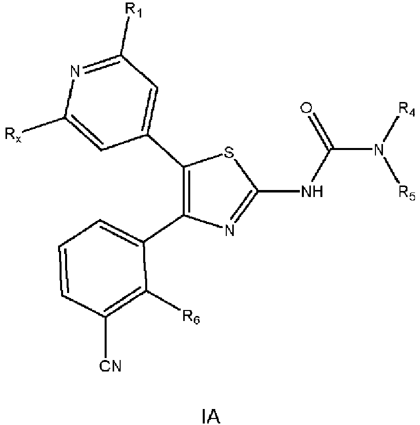 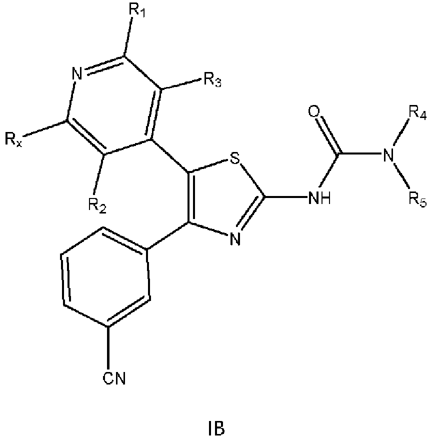 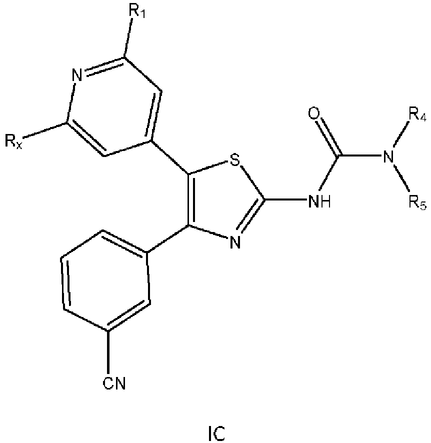 dans lequel Rₓ, R₁, R₂, R₃, R₄, R₅ et R₆ sont chacun tels que définis dans l'une quelconque des revendications 1 à 10.

12. Composé selon la revendication 1, ou sel pharmaceutiquement acceptable de celui-ci, choisi parmi l'un quelconque de ce qui suit :
le N-[5-(2-chloro-6-méthyl-4-pyridyl)-4-(3-cyanophényl)thiazol-2-yl]morpholine-4-carboxamide
le N-[5-(2-chloro-6-méthyl-4-pyridyl)-4-(3-cyanophényl)thiazol-2-yl]-3-(1-hydroxy-1-méthyl-éthyl)pyrrolidine-1-carboxamide
le (2R)-N-[5-(2-chloro-6-méthyl-4-pyridyl)-4-(3-cyanophényl)thiazol-2-yl]-2-(1-hydroxy-1-méthyl-éthyl)pyrrolidine-1-carboxamide
le N-[5-(2-chloro-6-méthyl-4-pyridyl)-4-(3-cyanophényl)thiazol-2-yl]pipérazine-1-carboxamide
le (3R)-N-[5-(2-chloro-6-méthyl-4-pyridyl)-4-(3-cyanophényl)thiazol-2-yl]-3-(1-hydroxy-1-méthyl-éthyl)pipérazine-1-carboxamide
le N-[5-(2-chloro-6-méthyl-4-pyridyl)-4-(3-cyanophényl)thiazol-2-yl]-4-(oxétan-3-yl)pipérazine-1-carboxamide
le N-[5-(2-chloro-6-méthyl-4-pyridyl)-4-(3-cyanophényl)thiazol-2-yl]-1-imino-1-oxo-1,4-thiazinane-4-carboxamide
le N-[5-(2-chloro-6-méthyl-4-pyridyl)-4-(3-cyanophényl)thiazol-2-yl]pyrrolidine-1-carboxamide
le N-[5-(2-chloro-6-méthyl-4-pyridyl)-4-(3-cyanophényl)thiazol-2-yl]-2-(2-hydroxy-2-méthyl-propyl)pyrrolidine-1-carboxamide
le N-[5-(2-chloro-6-méthyl-4-pyridyl)-4-(3-cyanophényl)thiazol-2-yl]-3-(1-hydroxy-1-méthyl-éthyl)morpholine-4-carboxamide
l'acide (2S)-4-[[5-(2-chloro-6-méthyl-4-pyridyl)-4-(3-cyanophényl)thiazol-2-yl]carbamoyl]pipérazine-2-carboxylique
le N-[5-(2-chloro-6-méthyl-4-pyridyl)-4-(3-cyanophényl)thiazol-2-yl]-4-méthyl-pipérazine-1-carboxamide
le N-[5-(2-chloro-6-méthyl-4-pyridyl)-4-(3-cyanophényl)thiazol-2-yl]-4-hydroxy-4-méthyl-pipéridine-1-carboxamide
le N-[5-(2-chloro-6-méthyl-4-pyridyl)-4-(3-cyanophényl)thiazol-2-yl]-4-cyano-4-méthyl-pipéridine-1-carboxamide
le (3S)-N-[5-(2-chloro-6-méthyl-4-pyridyl)-4-(3-cyanophényl)thiazol-2-yl]-3-(1-hydroxy-1-méthyl-éthyl)pipérazine-1-carboxamide
le N-[4-(3-cyanophényl)-5-(2,6-diméthyl-4-pyridyl)thiazol-2-yl]-4-méthyl-pipérazine-1-carboxamide
le 4-cyano-N-[4-(3-cyanophényl)-5-(2,6-diméthyl-4-pyridyl)thiazol-2-yl]pipéridine-1-carboxamide
le (3R)-3-cyano-N-[4-(3-cyanophényl)-5-(2,6-diméthyl-4-pyridyl)thiazol-2-yl]pyrrolidine-1-carboxamide
le N-[4-(3-cyanophényl)-5-(2,6-diméthyl-4-pyridyl)thiazol-2-yl]-3,4,6,7,9,9a-hexahydro-1H-pyrazino[2,1-c][1,4]oxazine-8-carboxamide
le N-[4-(3-cyanophényl)-5-(2,6-diméthyl-4-pyridyl)thiazol-2-yl]-2-oxa-6-azaspiro[3.3]heptane-6-carboxamide
le N-[4-(3-cyanophényl)-5-(2,6-diméthyl-4-pyridyl)thiazol-2-yl]-3-oxo-pipérazine-1-carboxamide
le N4-[4-(3-cyanophényl)-5-(2,6-diméthyl-4-pyridyl)thiazol-2-yl]morpholine-2,4-dicarboxamide
le N-[4-(3-cyanophényl)-5-(2,6-diméthyl-4-pyridyl)thiazol-2-yl]-4-méthyl-3-oxo-pipérazine-1-carboxamide
le (2R)-N-[4-(3-cyanophényl)-5-(2,6-diméthyl-4-pyridyl)thiazol-2-yl]-2-(hydroxyméthyl)morpholine-4-carboxamide
le 4-acétyl-N-[4-(3-cyanophényl)-5-(2,6-diméthyl-4-pyridyl)thiazol-2-yl]pipérazine-1-carboxamide
le N-[4-(3-cyanophényl)-5-(2,6-diméthyl-4-pyridyl)thiazol-2-yl]-4-méthylsulfonyl-pipérazine-1-carboxamide
le N-[4-(3-cyanophényl)-5-(2,6-diméthyl-4-pyridyl)thiazol-2-yl]-4-sulfamoyl-pipérazine-1-carboxamide
le N-[4-(3-cyanophényl)-5-(2,6-diméthyl-4-pyridyl)thiazol-2-yl]-1-imino-1-oxo-1,4-thiazinane-4-carboxamide
le N-[4-(3-cyanophényl)-5-(2,6-diméthyl-4-pyridyl)thiazol-2-yl]-2,4-dioxo-1,3,8-triazaspiro[4.5]décane-8-carboxamide
le (2S)-N-[4-(3-cyanophényl)-5-(2,6-diméthyl-4-pyridyl)thiazol-2-yl]-2-(hydroxyméthyl)morpholine-4-carboxamide
le 4-cyano-N-[4-(3-cyanophényl)-5-(2,6-diméthyl-4-pyridyl)thiazol-2-yl]-4-méthoxy-pipéridine-1-carboxamide
le 4-cyano-N-[4-(3-cyanophényl)-5-(2,6-diméthyl-4-pyridyl)thiazol-2-yl]-4-(hydroxyméthyl)pipéridine-1-carboxamide
le N-[4-(3-cyanophényl)-5-(2,6-diméthyl-4-pyridyl)thiazol-2-yl]-1-oxo-1,4-thiazinane-4-carboxamide
le N-[4-(3-cyanophényl)-5-(2,6-diméthyl-4-pyridyl)thiazol-2-yl]-3-(hydroxyméthyl)azétidine-1-carboxamide
le 3-cyano-N-[4-(3-cyanophényl)-5-(2,6-diméthyl-4-pyridyl)thiazol-2-yl]azétidine-1-carboxamide
le N-[4-(3-cyanophényl)-5-(2,6-diméthyl-4-pyridyl)thiazol-2-yl]-1,1-dioxo-1,4-thiazinane-4-carboxamide
le N-[4-(3-cyanophényl)-5-(2,6-diméthyl-4-pyridyl)thiazol-2-yl]-1,3-diméthyl-2,4-dioxo-1,3,8-triazaspiro[4.5]décane-8-carboxamide
le N1-[4-(3-cyanophényl)-5-(2,6-diméthyl-4-pyridyl)thiazol-2-yl]pipéridine-1,4-dicarboxamide
le N-[4-(3-cyanophényl)-5-(2,6-diméthyl-4-pyridyl)thiazol-2-yl]-4-(2-hydroxyéthyl)pipérazine-1-carboxamide
le N-[4-(3-cyanophényl)-5-(2,6-diméthyl-4-pyridyl)thiazol-2-yl]-3-hydroxy-azétidine-1-carboxamide
le N-[4-(3-cyanophényl)-5-(2,6-diméthyl-4-pyridyl)thiazol-2-yl]-4-(2-hydroxyéthyl)-3-oxo-pipérazine-1-carboxamide
le 4,4-dicyano-N-[4-(3-cyanophényl)-5-(2,6-diméthyl-4-pyridyl)thiazol-2-yl]pipéridine-1-carboxamide
le N-[4-(3-cyanophényl)-5-(2,6-diméthyl-4-pyridyl)thiazol-2-yl]-3-(1-hydroxy-1-méthyl-éthyl)azétidine-1-carboxamide
le N-[4-(3-cyanophényl)-5-(2,6-diméthyl-4-pyridyl)thiazol-2-yl]-4-(2-hydroxy-2-méthyl-propyl)pipérazine-1-carboxamide
le N-[4-(3-cyanophényl)-5-(2,6-diméthyl-4-pyridyl)thiazol-2-yl]-1-méthyl-1,7-diazaspiro[3.4]octane-7-carboxamide
le N-[4-(3-cyanophényl)-5-(2,6-diméthyl-4-pyridyl)thiazol-2-yl]-4-(2-hydroxy-1,1-diméthyl-éthyl)pipérazine-1-carboxamide
le (8aS)-N-[4-(3-cyanophényl)-5-(2,6-diméthyl-4-pyridyl)thiazol-2-yl]-3,4,6,7,8,8a-hexahydro-1H-pyrrolo[1,2-a]pyrazine-2-carboxamide
le (8aR)-N-[4-(3-cyanophényl)-5-(2,6-diméthyl-4-pyridyl)thiazol-2-yl]-3,4,6,7,8,8a-hexahydro-1H-pyrrolo[1,2-a]pyrazine-2-carboxamide
le N-[4-(3-cyanophényl)-5-(2,6-diméthyl-4-pyridyl)thiazol-2-yl]-4-(oxétan-3-yl)pipérazine-1-carboxamide
le (3S)-N-[4-(3-cyanophényl)-5-(2,6-diméthyl-4-pyridyl)thiazol-2-yl]-3-hydroxy-pyrrolidine-1-carboxamide
le 4-cyano-N-[4-(3-cyanophényl)-5-(2,6-diméthyl-4-pyridyl)thiazol-2-yl]-4-(méthoxyméthyl)pipéridine-1-carboxamide
le 4-cyano-N-[4-(3-cyanophényl)-5-(2,6-diméthyl-4-pyridyl)thiazol-2-yl]-4-fluoro-pipéridine-1-carboxamide
le (3S)-3-cyano-N-[4-(3-cyanophényl)-5-(2,6-diméthyl-4-pyridyl)thiazol-2-yl]pyrrolidine-1-carboxamide
le N-[4-(3-cyanophényl)-5-(2,6-diméthyl-4-pyridyl)thiazol-2-yl]-3-hydroxy-3-méthyl-azétidine-1-carboxamide
l'acide (2R)-4-[[5-(2-chloro-6-méthyl-4-pyridyl)-4-(3-cyanophényl)thiazol-2-yl]carbamoyl]pipérazine-2-carboxylique
le (2R)-4-[[5-(2-chloro-6-méthyl-4-pyridyl)-4-(3-cyanophényl)thiazol-2-yl]carbamoyl]pipérazine-2-carboxylate de méthyle
le (3S)-N-[5-(2-chloro-6-méthyl-4-pyridyl)-4-(3-cyanophényl)thiazol-2-yl]-3-(hydroxyméthyl)pipérazine-1-carboxamide
le (3R)-N-[5-(2-chloro-6-méthyl-4-pyridyl)-4-(3-cyanophényl)thiazol-2-yl]-3-(hydroxyméthyl)pipérazine-1-carboxamide
le (3S)-N-[5-(2-chloro-6-méthyl-4-pyridyl)-4-(3-cyanophényl)thiazol-2-yl]-3-méthyl-pipérazine-1-carboxamide
le (3R)-N-[5-(2-chloro-6-méthyl-4-pyridyl)-4-(3-cyanophényl)thiazol-2-yl]-3-méthyl-pipérazine-1-carboxamide
le (1S,4S)-N-[5-(2-chloro-6-méthyl-4-pyridyl)-4-(3-cyanophényl)thiazol-2-yl]-2,5-diazabicyclo[2.2.1]heptane-2-carboxamide
le (3R)-N1-[5-(2-chloro-6-méthyl-4-pyridyl)-4-(3-cyanophényl)thiazol-2-yl]pipérazine-1,3-dicarboxamide
le (3S)-N1-[5-(2-chloro-6-méthyl-4-pyridyl)-4-(3-cyanophényl)thiazol-2-yl]pipérazine-1,3-dicarboxamide
le 4-amino-N-[5-(2-chloro-6-méthyl-4-pyridyl)-4-(3-cyanophényl)thiazol-2-yl]-4-méthyl-pipéridine-1-carboxamide
le 4-cyano-N-[4-(3-cyanophényl)-5-(2,6-diméthyl-4-pyridyl)thiazol-2-yl]-4-méthyl-pipéridine-1-carboxamide
le (3S)-N-[4-(3-cyanophényl)-5-(2,6-diméthyl-4-pyridyl)thiazol-2-yl]-3-méthylsulfonyl-pyrrolidine-1-carboxamide
le N-[4-(3-cyanophényl)-5-(2,6-diméthyl-4-pyridyl)thiazol-2-yl]-2-oxa-7-azaspiro[3.5]nonane-7-carboxamide
le N-[5-(2-chloro-6-méthyl-4-pyridyl)-4-(3-cyanophényl)thiazol-2-yl]-2-oxa-6-azaspiro[3.3]heptane-6-carboxamide
le (1R,4R)-5-acétyl-N-[4-(3-cyanophényl)-5-(2,6-diméthyl-4-pyridyl)thiazol-2-yl]-2,5-diazabicyclo[2.2.1]heptane-2-carboxamide
le (1S,4S)-N-[4-(3-cyanophényl)-5-(2,6-diméthyl-4-pyridyl)thiazol-2-yl]-5-méthyl-2,5-diazabicyclo[2.2.1]heptane-2-carboxamide
le N-[4-(3-cyanophényl)-5-(2,6-diméthyl-4-pyridyl)thiazol-2-yl]-3-méthyl-2-oxo-1,3,8-triazaspiro[4.5]décane-8-carboxamide
le N-[5-[2,6-bis(trideutériométhyl)-4-pyridyl]-4-(3-cyanophényl)thiazol-2-yl]-2-oxa-6-azaspiro[3.3]heptane-6-carboxamide
le N-[4-(3-cyanophényl)-5-(3-fluoro-2,6-diméthyl-4-pyridyl)thiazol-2-yl]-2-oxa-6-azaspiro[3.3]heptane-6-carboxamide
le (4aS,7aR)-N-[4-(3-cyanophényl)-5-[2-(difluorométhyl)-6-méthyl-4-pyridyl]thiazol-2-yl]-4-méthyl-2,3,4a,5,7,7a-hexahydropyrrolo[3,4-b][1,4]oxazine-6-carboxamide
le N-[4-(3-cyanophényl)-5-[2-(difluorométhyl)-6-méthyl-4-pyridyl]thiazol-2-yl]-9-méthyl-6-oxa-2,9-diazaspiro[4.5]décane-2-carboxamide
le N-[4-(3-cyanophényl)-5-[2-(difluorométhyl)-6-méthyl-4-pyridyl]thiazol-2-yl]-6-oxa-2,9-diazaspiro[4.5]décane-2-carboxamide
le (4aS,7aS)-N-[4-(3-cyanophényl)-5-[2-(difluorométhyl)-6-méthyl-4-pyridyl]thiazol-2-yl]-4-méthyl-2,3,4a,5,7,7a-hexahydropyrrolo[3,4-b][1,4]oxazine-6-carboxamide
le N-[4-(3-cyanophényl)-5-(2,6-diméthyl-4-pyridyl)thiazol-2-yl]-2-oxo-1,3,8-triazaspiro[4.5]décane-8-carboxamide
le 3-cyano-N-[4-(3-cyanophényl)-5-[2-méthyl-6-(oxétan-3-yl)-4-pyridyl]thiazol-2-yl]-3-méthyl-pyrrolidine-1-carboxamide
le 4-cyano-N-[4-(3-cyanophényl)-5-[2-méthyl-6-(oxétan-3-yl)-4-pyridyl]thiazol-2-yl]-4-méthyl-pipéridine-1-carboxamide
le N-[4-(3-cyanophényl)-5-(2,6-diméthyl-4-pyridyl)thiazol-2-yl]-3-morpholino-azétidine-1-carboxamide
le N-[4-(3-cyanophényl)-5-(2,6-diméthyl-4-pyridyl)thiazol-2-yl]-4-tétrahydropyran-4-yl-pipérazine-1-carboxamide
le N-[4-(3-cyanophényl)-5-(2,6-diméthyl-4-pyridyl)thiazol-2-yl]-3-pyrrolidin-1-yl-azétidine-1-carboxamide
le N-[4-(3-cyanophényl)-5-(2,6-diméthyl-4-pyridyl)thiazol-2-yl]-3-(diméthylamino)azétidine-1-carboxamide
le N-[4-(3-cyanophényl)-5-(2,6-diméthyl-4-pyridyl)thiazol-2-yl]-4-(morpholinométhyl)pipéridine-1-carboxamide
le N-[4-(3-cyanophényl)-5-(2,6-diméthyl-4-pyridyl)thiazol-2-yl]-3-méthyl-2-oxo-1-oxa-3,8-diazaspiro[4.5]décane-8-carboxamide
le N-[4-(3-cyanophényl)-5-(2,6-diméthyl-4-pyridyl)thiazol-2-yl]-3-méthyl-3,6-diazabicyclo[3.2.0]heptane-6-carboxamide
le N-[4-(3-cyanophényl)-5-(2,6-diméthyl-4-pyridyl)thiazol-2-yl]-1,4-diazabicyclo[3.2.1]octane-4-carboxamide
le N-[4-(3-cyanophényl)-5-(2,6-diméthyl-4-pyridyl)thiazol-2-yl]-3-oxo-5,6,8,8a-tétrahydro-1H-oxazolo[3,4-a]pyrazine-7-carboxamide
le N-[4-(3-cyanophényl)-5-(2,6-diméthyl-4-pyridyl)thiazol-2-yl]-2,2-dioxo-3,4,6,7,9,9a-hexahydro-1H-pyrazino[2,1-c][1,4]thiazine-8-carboxamide
le N-[4-(3-cyanophényl)-5-(2,6-diméthyl-4-pyridyl)thiazol-2-yl]-4-oxo-3,6,7,8,9,9a-hexahydro-1H-pyrido[1,2-a]pyrazine-2-carboxamide
le N-[4-(3-cyanophényl)-5-(2,6-diméthyl-4-pyridyl)thiazol-2-yl]-9-oxo-3,4,6,7,8,9a-hexahydro-1H-pyrazino[1,2-a]pyrazine-2-carboxamide
le N-[4-(3-cyanophényl)-5-(2,6-diméthyl-4-pyridyl)thiazol-2-yl]-6-oxo-1,3,4,8,9,9a-hexahydropyrazino[1,2-c][1,3]oxazine-2-carboxamide
le N-[4-(3-cyanophényl)-5-(2,6-diméthyl-4-pyridyl)thiazol-2-yl]-6-oxo-1,3,4,7,8,8a-hexahydropyrrolo[1,2-a]pyrazine-2-carboxamide
le N-[4-(3-cyanophényl)-5-[2-(difluorométhyl)-6-méthyl-4-pyridyl]thiazol-2-yl]-1-oxo-1,4-thiazinane-4-carboxamide
le N-[4-(3-cyanophényl)-5-(2,6-diméthyl-4-pyridyl)thiazol-2-yl]-3-sulfamoyl-pyrrolidine-1-carboxamide
le N-[4-(3-cyanophényl)-5-(2,6-diméthyl-4-pyridyl)thiazol-2-yl]-3-oxo-2,7-diazaspiro[3.5]nonane-7-carboxamide
le N-[4-(3-cyanophényl)-5-(2,6-diméthyl-4-pyridyl)thiazol-2-yl]-4-pyrazol-1-yl-pipéridine-1-carboxamide
le N-[4-(3-cyanophényl)-5-(2,6-diméthyl-4-pyridyl)thiazol-2-yl]-4-(1,2,4-oxadiazol-3-yl)pipéridine-1-carboxamide
le N-[4-(3-cyanophényl)-5-(2,6-diméthyl-4-pyridyl)thiazol-2-yl]-1,3-dioxo-5,6,8,8a-tétrahydroimidazo[1,5-a]pyrazine-7-carboxamide
le N-[4-(3-cyanophényl)-5-(2,6-diméthyl-4-pyridyl)thiazol-2-yl]-1-oxo-2,8-diazaspiro[4.5]décane-8-carboxamide
le N-[4-(3-cyanophényl)-5-(2,6-diméthyl-4-pyridyl)thiazol-2-yl]-4-(1H-imidazol-2-yl)pipéridine-1-carboxamide
le N-[4-(3-cyanophényl)-5-(2,6-diméthyl-4-pyridyl)thiazol-2-yl]-10-oxo-3,9-diazaspiro[5.5]undécane-3-carboxamide
le N-[4-(3-cyanophényl)-5-(2,6-diméthyl-4-pyridyl)thiazol-2-yl]-6,8-dihydro-5H-imidazo[1,2-a]pyrazine-7-carboxamide
le N1-[4-(3-cyanophényl)-5-(2,6-diméthyl-4-pyridyl)thiazol-2-yl]-N4-méthyl-pipéridine-1,4-dicarboxamide
le N-[4-(3-cyanophényl)-5-(2,6-diméthyl-4-pyridyl)thiazol-2-yl]-8-oxa-2-azaspiro[4.5]décane-2-carboxamide
le (3S)-N1-[4-(3-cyanophényl)-5-(2,6-diméthyl-4-pyridyl)thiazol-2-yl]pyrrolidine-1,3-dicarboxamide
le N-[4-(3-cyanophényl)-5-(2,6-diméthyl-4-pyridyl)thiazol-2-yl]-4-(2-oxoimidazolidin-1-yl)pipéridine-1-carboxamide
le N-[4-(3-cyanophényl)-5-(2,6-diméthyl-4-pyridyl)thiazol-2-yl]-4-(1,2,4-oxadiazol-5-yl)pipéridine-1-carboxamide
le N-[4-(3-cyanophényl)-5-(2,6-diméthyl-4-pyridyl)thiazol-2-yl]-1-méthylimino-1-oxo-1,4-thiazinane-4-carboxamide
le N1-[4-(3-cyanophényl)-5-(2,6-diméthyl-4-pyridyl)thiazol-2-yl]-N4-(2-hydroxyéthyl)pipéridine-1,4-dicarboxamide
le N-[4-(3-cyanophényl)-5-(2,6-diméthyl-4-pyridyl)thiazol-2-yl]-6-oxo-7-oxa-2,5-diazaspiro[3.5]nonane-2-carboxamide
le N-[4-(3-cyanophényl)-5-(2,6-diméthyl-4-pyridyl)thiazol-2-yl]-6,9-dioxo-1,3,4,7,8,9a-hexahydropyrazino[1,2-a]pyrazine-2-carboxamide
le (3R)-N-[4-(3-cyanophényl)-5-(2,6-diméthyl-4-pyridyl)thiazol-2-yl]-3-(1-hydroxy-1-méthyl-éthyl)pyrrolidine-1-carboxamide
le N-[4-(3-cyanophényl)-5-(2,6-diméthyl-4-pyridyl)thiazol-2-yl]-4-oxo-6,7,9,9a-tétrahydro-1H-pyrazino[2,1-c][1,4]oxazine-8-carboxamide
le N-[4-(3-cyanophényl)-5-(2,6-diméthyl-4-pyridyl)thiazol-2-yl]-2-méthyl-3-oxo-2,8-diazaspiro[4.5]décane-8-carboxamide
le N-[4-(3-cyanophényl)-5-(2,6-diméthyl-4-pyridyl)thiazol-2-yl]-8-méthyl-6,9-dioxo-3,4,7,9a-tétrahydro-1H-pyrazino[1,2-a]pyrazine-2-carboxamide
le N-[4-(3-cyanophényl)-5-(2,6-diméthyl-4-pyridyl)thiazol-2-yl]-3-oxo-2,8-diazaspiro[4.5]décane-8-carboxamide
le N-[4-(3-cyanophényl)-5-(2,6-diméthyl-4-pyridyl)thiazol-2-yl]-3-oxo-2,9-diazaspiro[5.5]undécane-9-carboxamide
le 4-cyano-N-[4-(3-cyanophényl)-5-(2,6-diméthyl-4-pyridyl)thiazol-2-yl]-4-(1-hydroxy-1-méthyl-éthyl)pipéridine-1-carboxamide
le N-[4-(3-cyanophényl)-5-(2,6-diméthyl-4-pyridyl)thiazol-2-yl]-6-oxo-3,4,7,8,9,9a-hexahydro-1H-pyrazino[1,2-c]pyrimidine-2-carboxamide
le N-[4-(3-cyanophényl)-5-(2,6-diméthyl-4-pyridyl)thiazol-2-yl]-6-oxo-3,4,7,8,9,9a-hexahydro-1H-pyrido[1,2-a]pyrazine-2-carboxamide
le N-[4-(3-cyanophényl)-5-(2,6-diméthyl-4-pyridyl)thiazol-2-yl]-6-hydroxy-6-méthyl-2-azaspiro[3.3]heptane-2-carboxamide
le N-[4-(3-cyanophényl)-5-(2,6-diméthyl-4-pyridyl)thiazol-2-yl]-2-oxo-1,8-diazaspiro[4.5]décane-8-carboxamide
le N-[4-(3-cyanophényl)-5-(2,6-diméthyl-4-pyridyl)thiazol-2-yl]-2,2-dioxo-2λ^{6}-thia-6-azaspiro[3.3]heptane-6-carboxamide
le N-[4-(3-cyanophényl)-5-(2,6-diméthyl-4-pyridyl)thiazol-2-yl]-2-(1-hydroxy-1-méthyl-éthyl)morpholine-4-carboxamide
le (3S)-N-[4-(3-cyanophényl)-5-(2,6-diméthyl-4-pyridyl)thiazol-2-yl]-3-(1-hydroxy-1-méthyl-éthyl)pyrrolidine-1-carboxamide
le (3aR,6aS)-N-[4-(3-cyanophényl)-5-(2,6-diméthyl-4-pyridyl)thiazol-2-yl]-1,3,3a,4,6,6a-hexahydrofuro[3,4-c]pyrrole-5-carboxamide
le N-[4-(3-cyanophényl)-5-(2,6-diméthyl-4-pyridyl)thiazol-2-yl]-2-oxo-3-oxa-1,9-diazaspiro[5.5]undécane-9-carboxamide
le (1S,5S)-N-[4-(3-cyanophényl)-5-(2,6-diméthyl-4-pyridyl)thiazol-2-yl]-6-méthyl-3,6-diazabicyclo[3.2.2]nonane-3-carboxamide
le N-[4-(3-cyanophényl)-5-(2,6-diméthyl-4-pyridyl)thiazol-2-yl]-1-méthyl-2-oxo-1,3,8-triazaspiro[4.5]décane-8-carboxamide
le (3aS,6aS)-N-[4-(3-cyanophényl)-5-(2,6-diméthyl-4-pyridyl)thiazol-2-yl]-3-oxo-1,2,3a,4,6,6a-hexahydropyrrolo[3,4-c]pyrrole-5-carboxamide
le N-[4-(3-cyanophényl)-5-(2,6-diméthyl-4-pyridyl)thiazol-2-yl]-6-oxa-2-azaspiro[3.4]octane-2-carboxamide
le N-[4-(3-cyanophényl)-5-(2,6-diméthyl-4-pyridyl)thiazol-2-yl]-7-oxa-2-azaspiro[3.5]nonane-2-carboxamide
le N-[4-(3-cyanophényl)-5-(2,6-diméthyl-4-pyridyl)thiazol-2-yl]-6-oxo-2,7-diazaspiro[3.4]octane-2-carboxamide
le N-[4-(3-cyanophényl)-5-(2,6-diméthyl-4-pyridyl)thiazol-2-yl]-2-oxo-1-oxa-3,8-diazaspiro[4.5]décane-8-carboxamide
le N-[4-(3-cyanophényl)-5-(2,6-diméthyl-4-pyridyl)thiazol-2-yl]-6-oxo-2,5,7-triazaspiro[3.4]octane-2-carboxamide
le N-[4-(3-cyanophényl)-5-(2,6-diméthyl-4-pyridyl)thiazol-2-yl]-2-oxo-1,9-diazaspiro[5.5]undécane-9-carboxamide
le N-[4-(3-cyanophényl)-5-(2,6-diméthyl-4-pyridyl)thiazol-2-yl]-5-oxo-2,6-diazaspiro[3.4]octane-2-carboxamide
le N-[4-(3-cyanophényl)-5-(2,6-diméthyl-4-pyridyl)thiazol-2-yl]-3-méthyl-2,4-dioxo-1,3,8-triazaspiro[4.5]décane-8-carboxamide
le N-[4-(3-cyanophényl)-5-(2,6-diméthyl-4-pyridyl)thiazol-2-yl]-4-éthyl-pipérazine-1-carboxamide
le N-[4-(3-cyanophényl)-5-[2-méthyl-6-(trifluorométhyl)-4-pyridyl]thiazol-2-yl]-4-(oxétan-3-yl)pipérazine-1-carboxamide
l'acide 1-[[4-(3-cyanophényl)-5-(2,6-diméthyl-4-pyridyl)thiazol-2-yl]carbamoyl]pipéridine-4-carboxylique
l'acide (3S)-1-[[4-(3-cyanophényl)-5-(2,6-diméthyl-4-pyridyl)thiazol-2-yl]carbamoyl]pyrrolidine-3-carboxylique
le 3-cyano-N-[4-(3-cyanophényl)-5-(2,6-diméthyl-4-pyridyl)thiazol-2-yl]-3-(hydroxyméthyl)azétidine-1-carboxamide
l'acide (3R)-1-[[4-(3-cyanophényl)-5-(2,6-diméthyl-4-pyridyl)thiazol-2-yl]carbamoyl]pyrrolidine-3-carboxylique
le 4-cyano-N-[4-(3-cyanophényl)-5-(3-fluoro-2,6-diméthyl-4-pyridyl)thiazol-2-yl]-4-méthyl-pipéridine-1-carboxamide
le N-[4-(3-cyanophényl)-5-(3-fluoro-2,6-diméthyl-4-pyridyl)thiazol-2-yl]-3-hydroxy-3-méthyl-azétidine-1-carboxamide
le 3-cyano-N-[4-(3-cyanophényl)-5-(3-fluoro-2,6-diméthyl-4-pyridyl)thiazol-2-yl]-3-méthyl-azétidine-1-carboxamide
le N-[4-(3-cyanophényl)-5-(3-fluoro-2,6-diméthyl-4-pyridyl)thiazol-2-yl]-3-oxo-2,7-diazaspiro[3.5]nonane-7-carboxamide
le 3-cyano-N-[4-(3-cyanophényl)-5-(2,6-diméthyl-4-pyridyl)thiazol-2-yl]-3-méthyl-azétidine-1-carboxamide
l'acide 1-[[4-(3-cyanophényl)-5-(2,6-diméthyl-4-pyridyl)thiazol-2-yl]carbamoyl]azétidine-3-carboxylique
le (2S)-N-[4-(3-cyanophényl)-5-(2,6-diméthyl-4-pyridyl)thiazol-2-yl]-2-(hydroxyméthyl)pyrrolidine-1-carboxamide
le (2S)-N-[4-(3-cyanophényl)-5-(2,6-diméthyl-4-pyridyl)thiazol-2-yl]-2-méthyl-azétidine-1-carboxamide
le (2S)-N-[4-(3-cyanophényl)-5-(2,6-diméthyl-4-pyridyl)thiazol-2-yl]-2-(hydroxyméthyl)azétidine-1-carboxamide
le (3R)-N-[4-(3-cyanophényl)-5-(2,6-diméthyl-4-pyridyl)thiazol-2-yl]-3-méthyl-morpholine-4-carboxamide
le (3S)-N-[4-(3-cyanophényl)-5-(2,6-diméthyl-4-pyridyl)thiazol-2-yl]-3-(1-hydroxy-1-méthyl-éthyl)pipérazine-1-carboxamide
le (3S)-N-[4-(3-cyanophényl)-5-(2,6-diméthyl-4-pyridyl)thiazol-2-yl]-3-(hydroxyméthyl)morpholine-4-carboxamide
le N-[4-(3-cyanophényl)-5-(2,6-diméthyl-4-pyridyl)thiazol-2-yl]-2-méthyl-1-oxo-2,8-diazaspiro[4.5]décane-8-carboxamide
le (2R)-N-[4-(3-cyanophényl)-5-(2,6-diméthyl-4-pyridyl)thiazol-2-yl]-2-méthyl-azétidine-1-carboxamide
le N-[4-(3-cyano-2-méthyl-phényl)-5-(2,6-diméthyl-4-pyridyl)thiazol-2-yl]-2-oxa-6-azaspiro[3 .3]heptane-6-carboxamide
le (3R)-N1-[4-(3-cyanophényl)-5-(2,6-diméthyl-4-pyridyl)thiazol-2-yl]pyrrolidine-1,3-dicarboxamide
le N-[4-(3-cyanophényl)-5-(2,6-diméthyl-4-pyridyl)thiazol-2-yl]-2-oxa-7-azaspiro[4.4]nonane-7-carboxamide
le N-[4-(3-cyanophényl)-5-(2,6-diméthyl-4-pyridyl)thiazol-2-yl]-2-oxa-7-azaspiro[3.4]octane-7-carboxamide
le (2S)-N-[4-(3-cyanophényl)-5-(2,6-diméthyl-4-pyridyl)thiazol-2-yl]-2-(1-hydroxy-1-méthyl-éthyl)azétidine-1-carboxamide
le (2R)-N-[4-(3-cyanophényl)-5-(2,6-diméthyl-4-pyridyl)thiazol-2-yl]-2-(1-hydroxy-1-méthyl-éthyl)azétidine-1-carboxamide
le N-[4-(3-cyanophényl)-5-(2,6-diméthyl-4-pyridyl)thiazol-2-yl]-3-morpholino-pyrrolidine-1-carboxamide
le (3R)-N-[4-(3-cyanophényl)-5-(2,6-diméthyl-4-pyridyl)thiazol-2-yl]-3-méthyl-5-oxo-pipérazine-1-carboxamide
le (3S)-N-[4-(3-cyanophényl)-5-(2,6-diméthyl-4-pyridyl)thiazol-2-yl]-3-méthyl-morpholine-4-carboxamide
le N-[4-(3-cyanophényl)-5-(2,6-diméthyl-4-pyridyl)thiazol-2-yl]-6-oxa-1-azaspiro[3.3]heptane-1-carboxamide
le (3R)-N-[4-(3-cyanophényl)-5-(2,6-diméthyl-4-pyridyl)thiazol-2-yl]-3-(hydroxyméthyl)morpholine-4-carboxamide
le N-[4-(3-cyanophényl)-5-(2,6-diméthyl-4-pyridyl)thiazol-2-yl]-1,3-diméthyl-2-oxo-1,3,8-triazaspiro[4.5]décane-8-carboxamide
le (3R)-N-[4-(3-cyanophényl)-5-(2,6-diméthyl-4-pyridyl)thiazol-2-yl]-3-méthylsulfonyl-pyrrolidine-1-carboxamide
le (3S)-N-[4-(3-cyanophényl)-5-(2,6-diméthyl-4-pyridyl)thiazol-2-yl]-3-méthyl-5-oxo-pipérazine-1-carboxamide
le (2R)-N-[4-(3-cyanophényl)-5-(2,6-diméthyl-4-pyridyl)thiazol-2-yl]-2-(hydroxyméthyl)azétidine-1-carboxamide
le N-[4-(3-cyanophényl)-5-(2,6-diméthyl-4-pyridyl)thiazol-2-yl]-6-méthyl-5-oxo-2,6-diazaspiro[3.4]octane-2-carboxamide
le N-[4-(3-cyanophényl)-5-(2,6-diméthyl-4-pyridyl)thiazol-2-yl]-1-oxa-6-azaspiro[3.3]heptane-6-carboxamide
le N-[4-(3-cyanophényl)-5-(2,6-diméthyl-4-pyridyl)thiazol-2-yl]-1-méthyl-2-oxo-1,8-diazaspiro[4.5]décane-8-carboxamide
le N-[4-(3-cyanophényl)-5-(2,6-diméthyl-4-pyridyl)thiazol-2-yl]-4-méthylsulfonyl-pipéridine-1-carboxamide
le N-[4-(3-cyanophényl)-5-(2,6-diméthyl-4-pyridyl)thiazol-2-yl]-4-hydroxy-4-méthyl-pipéridine-1-carboxamide
le N-[4-(3-cyanophényl)-5-[2-(difluorométhyl)-6-méthyl-4-pyridyl]thiazol-2-yl]-2-oxa-6-azaspiro[3.3]heptane-6-carboxamide
le (4aR,7aS)-N-[4-(3-cyanophényl)-5-(2,6-diméthyl-4-pyridyl)thiazol-2-yl]-4-méthyl-2,3,4a,5,7,7a-hexahydropyrrolo[3,4-b][1,4]oxazine-6-carboxamide
le N-[4-(3-cyanophényl)-5-[2-méthyl-6-(trifluorométhyl)-4-pyridyl]thiazol-2-yl]-2-oxa-6-azaspiro[3.3]heptane-6-carboxamide
le N-[4-(3-cyanophényl)-5-(2,6-diméthyl-4-pyridyl)thiazol-2-yl]pipérazine-1-carboxamide
le (3R)-N-[4-(3-cyanophényl)-5-(2,6-diméthyl-4-pyridyl)thiazol-2-yl]-3-méthyl-pipérazine-1-carboxamide
le N-[4-(3-cyanophényl)-5-(2,6-diméthyl-4-pyridyl)thiazol-2-yl]-5,8-diazaspiro[3.5]nonane-5-carboxamide
le trans-N-[4-(3-cyanophényl)-5-(2,6-diméthyl-4-pyridyl)thiazol-2-yl]-2,5-diméthyl-pipérazine-1-carboxamide
le 4-amino-4-cyano-N-[4-(3-cyanophényl)-5-(2,6-diméthyl-4-pyridyl)thiazol-2-yl]pipéridine-1-carboxamide
le N-[4-(3-cyanophényl)-5-(2,6-diméthyl-4-pyridyl)thiazol-2-yl]-1-oxa-4,9-diazaspiro[5.5]undécane-9-carboxamide
le N-[4-(3-cyanophényl)-5-(2,6-diméthyl-4-pyridyl)thiazol-2-yl]-3,8-diazabicyclo[3.2.1]octane-8-carboxamide
le N-[4-(3-cyanophényl)-5-(2,6-diméthyl-4-pyridyl)thiazol-2-yl]-3,6-diazabicyclo[3.1.1]heptane-6-carboxamide
le (1R,4R)-N-[4-(3-cyanophényl)-5-(2,6-diméthyl-4-pyridyl)thiazol-2-yl]-2,5-diazabicyclo[2.2.2]octane-2-carboxamide
le cis-(2S,5S)-N-[4-(3-cyanophényl)-5-(2,6-diméthyl-4-pyridyl)thiazol-2-yl]-2,5-diméthyl-pipérazine-1-carboxamide
le (2R,6R)-N-[4-(3-cyanophényl)-5-(2,6-diméthyl-4-pyridyl)thiazol-2-yl]-2,6-diméthyl-pipérazine-1-carboxamide
le N-[4-(3-cyanophényl)-5-(2,6-diméthyl-4-pyridyl)thiazol-2-yl]-2,2-diméthyl-pipérazine-1-carboxamide
le 1-acétylimino-N-[4-(3-cyanophényl)-5-(2,6-diméthyl-4-pyridyl)thiazol-2-yl]-1-oxo-1,4-thiazinane-4-carboxamide
le N-[4-(3-cyanophényl)-5-[2-(hydroxyméthyl)-6-méthyl-4-pyridyl]thiazol-2-yl]-2-oxa-6-azaspiro[3.3]heptane-6-carboxamide
le N-[4-(3-cyanophényl)-5-[2-(hydroxyméthyl)-6-méthyl-4-pyridyl]thiazol-2-yl]-3-oxo-2,7-diazaspiro[3.5]nonane-7-carboxamide
le (3R)-N-[4-(3-cyanophényl)-5-[2-(hydroxyméthyl)-6-méthyl-4-pyridyl]thiazol-2-yl]-3-(1-hydroxy-1-méthyl-éthyl)pyrrolidine-1-carboxamide
le N-[4-(3-cyanophényl)-5-[2-(hydroxyméthyl)-6-méthyl-4-pyridyl]thiazol-2-yl]-1-oxo-1,4-thiazinane-4-carboxamide
le N-[4-(3-cyanophényl)-5-[2-(hydroxyméthyl)-6-méthyl-4-pyridyl]thiazol-2-yl]-4-(oxétan-3-yl)pipérazine-1-carboxamide
le N-[4-(3-cyanophényl)-5-[2-(hydroxyméthyl)-6-méthyl-4-pyridyl]thiazol-2-yl]-1,3-diméthyl-2,4-dioxo-1,3,8-triazaspiro[4.5]décane-8-carboxamide
le 4-cyano-N-[4-(3-cyanophényl)-5-[2-(hydroxyméthyl)-6-méthyl-4-pyridyl]thiazol-2-yl]pipéridine-1-carboxamide
le 4-cyano-N-[4-(3-cyanophényl)-5-[2-(hydroxyméthyl)-6-méthyl-4-pyridyl]thiazol-2-yl]-4-(hydroxyméthyl)pipéridine-1-carboxamide
le 4,4-dicyano-N-[4-(3-cyanophényl)-5-[2-(hydroxyméthyl)-6-méthyl-4-pyridyl]thiazol-2-yl]pipéridine-1-carboxamide
le 4-cyano-N-[4-(3-cyanophényl)-5-[2-(hydroxyméthyl)-6-méthyl-4-pyridyl]thiazol-2-yl]-4-méthoxy-pipéridine-1-carboxamide
le N-[4-(3-cyanophényl)-5-(3-fluoro-2,6-diméthyl-4-pyridyl)thiazol-2-yl]-1-oxo-1,4-thiazinane-4-carboxamide
le 1-cyanoimino-N-[4-(3-cyanophényl)-5-(2,6-diméthyl-4-pyridyl)thiazol-2-yl]-1-oxo-1,4-thiazinane-4-carboxamide
le 4-cyano-N-[4-(3-cyanophényl)-5-[2-(1-hydroxy-1-méthyl-éthyl)-6-méthyl-4-pyridyl]thiazol-2-yl]-4-méthyl-pipéridine-1-carboxamide
le N-[4-(3-cyanophényl)-5-[2-(1-hydroxy-1-méthyl-éthyl)-6-méthyl-4-pyridyl]thiazol-2-yl]-6-oxa-2-azaspiro[3.4]octane-2-carboxamide
le N-[4-(3-cyanophényl)-5-[2-(1-hydroxy-1-méthyl-éthyl)-6-méthyl-4-pyridyl]thiazol-2-yl]-2-oxa-6-azaspiro[3.3]heptane-6-carboxamide
le N-[4-(3-cyanophényl)-5-[2-(1-hydroxyéthyl)-6-méthyl-4-pyridyl]thiazol-2-yl]-2-oxa-6-azaspiro[3.3]heptane-6-carboxamide
le rac-(3S,5R)-N-[4-(3-cyanophényl)-5-(2,6-diméthyl-4-pyridyl)thiazol-2-yl]-3,5-diméthyl-pipérazine-1-carboxamide
le (3S)-N-[4-(3-cyanophényl)-5-(2,6-diméthyl-4-pyridyl)thiazol-2-yl]-3-(trifluorométhyl)pipérazine-1-carboxamide
le N-[4-(3-cyanophényl)-5-(2,6-diméthyl-4-pyridyl)thiazol-2-yl]-5-oxa-2,8-diazaspiro[3.5]nonane-2-carboxamide
le N-[4-(3-cyanophényl)-5-(2,6-diméthyl-4-pyridyl)thiazol-2-yl]-3,8-diazabicyclo[3.2.1]octane-3-carboxamide
le N-[4-(3-cyanophényl)-5-(2,6-diméthyl-4-pyridyl)thiazol-2-yl]-6-oxa-2,9-diazaspiro[4.5]décane-2-carboxamide
le (3R)-N-[4-(3-cyanophényl)-5-(2,6-diméthyl-4-pyridyl)thiazol-2-yl]-3-(trifluorométhyl)pipérazine-1-carboxamide
le (3S)-3-cyano-N-[4-(3-cyanophényl)-5-(2,6-diméthyl-4-pyridyl)thiazol-2-yl]pipérazine-1-carboxamide
le (3R)-3-cyano-N-[4-(3-cyanophényl)-5-(2,6-diméthyl-4-pyridyl)thiazol-2-yl]pipérazine-1-carboxamide
le N-[4-(3-cyanophényl)-5-(2,6-diméthyl-4-pyridyl)thiazol-2-yl]-4,7-diazaspiro[2.5]octane-7-carboxamide
le (3S,5S)-N-[4-(3-cyanophényl)-5-(2,6-diméthyl-4-pyridyl)thiazol-2-yl]-3,5-diméthyl-pipérazine-1-carboxamide
le (2S)-N-[4-(3-cyanophényl)-5-(2,6-diméthyl-4-pyridyl)thiazol-2-yl]-2-méthyl-pipérazine-1-carboxamide
le (2R)-N-[4-(3-cyanophényl)-5-(2,6-diméthyl-4-pyridyl)thiazol-2-yl]-2-(hydroxyméthyl)pipérazine-1-carboxamide
le (2S)-N-[4-(3-cyanophényl)-5-(2,6-diméthyl-4-pyridyl)thiazol-2-yl]-2-(hydroxyméthyl)pipérazine-1-carboxamide
le N-[4-(3-cyanophényl)-5-(2,6-diméthyl-4-pyridyl)thiazol-2-yl]-3,6-diazabicyclo[3.1.1]heptane-3-carboxamide
le (2R)-N-[4-(3-cyanophényl)-5-(2,6-diméthyl-4-pyridyl)thiazol-2-yl]-2-méthyl-pipérazine-1-carboxamide
le N-[4-(3-cyanophényl)-5-(2,6-diméthyl-4-pyridyl)thiazol-2-yl]-9-oxa-2,6-diazaspiro[4.5]décane-2-carboxamide
le (3R)-N-[4-(3-cyanophényl)-5-[2-méthyl-6-(trifluorométhyl)-4-pyridyl]thiazol-2-yl]-3-méthyl-pipérazine-1-carboxamide
le (4aS,7aS)-N-[4-(3-cyanophényl)-5-[2-(difluorométhyl)-6-méthyl-4-pyridyl]thiazol-2-yl]-3,4,4a,5,7,7a-hexahydro-2H-pyrrolo[3,4-b][1,4]oxazine-6-carboxamide
le (4aS,7aR)-N-[4-(3-cyanophényl)-5-[2-(difluorométhyl)-6-méthyl-4-pyridyl]thiazol-2-yl]-3,4,4a,5,7,7a-hexahydro-2H-pyrrolo[3,4-b][1,4]oxazine-6-carboxamide
le rac-(4aS,7aS)-N-[4-(3-cyanophényl)-5-(2,6-diméthyl-4-pyridyl)thiazol-2-yl]-3,4,4a,5,7,7a-hexahydro-2H-pyrrolo[3,4-b][1,4]oxazine-6-carboxamide
le N-[4-(3-cyanophényl)-5-(2,6-diméthyl-4-pyridyl)thiazol-2-yl]-2-cyclopropyl-pipérazine-1-carboxamide
le N-[4-(3-cyanophényl)-5-(2,6-diméthyl-4-pyridyl)thiazol-2-yl]-2-(cyclopropylméthyl)pipérazine-1-carboxamide
le N-[4-(3-cyanophényl)-5-(2,6-diméthyl-4-pyridyl)thiazol-2-yl]-3,3-diméthyl-pipérazine-1-carboxamide
le N-[4-(3-cyanophényl)-5-(2-méthoxy-6-méthyl-4-pyridyl)thiazol-2-yl]-1-imino-1-oxo-1,4-thiazinane-4-carboxamide
le N-[4-(3-cyanophényl)-5-[2-(difluorométhyl)-6-méthyl-4-pyridyl]thiazol-2-yl]-1-imino-1-oxo-1,4-thiazinane-4-carboxamide
le N-[4-(3-cyanophényl)-5-(2,6-dichloro-4-pyridyl)thiazol-2-yl]-2-oxa-6-azaspiro[3 .3]heptane-6-carboxamide
le N-[4-(3-cyanophényl)-5-[2-méthyl-6-(trifluorométhyl)-4-pyridyl]thiazol-2-yl]-1-oxo-1,4-thiazinane-4-carboxamide
le N-[4-(3-cyanophényl)-5-(2-méthoxy-6-méthyl-4-pyridyl)thiazol-2-yl]-1-oxo-1,4-thiazinane-4-carboxamide
le N-[4-(3-cyanophényl)-5-[2-méthyl-6-(oxétan-3-yl)-4-pyridyl]thiazol-2-yl]-2-oxa-6-azaspiro[3.3]heptane-6-carboxamide
le N-[5-(2-cyano-6-méthyl-4-pyridyl)-4-(3-cyanophényl)thiazol-2-yl]-1-oxo-1,4-thiazinane-4-carboxamide
le N-[4-(3-cyanophényl)-5-[2-(fluorométhyl)-6-méthyl-4-pyridyl]thiazol-2-yl]-2-oxa-6-azaspiro[3.3]heptane-6-carboxamide
le N-[4-(3-cyanophényl)-5-(2-cyclopropyl-6-méthyl-4-pyridyl)thiazol-2-yl]-2-oxa-6-azaspiro[3.3]heptane-6-carboxamide
le N-[4-(3-cyanophényl)-5-(2-isopropyl-6-méthyl-4-pyridyl)thiazol-2-yl]-2-oxa-6-azaspiro[3.3]heptane-6-carboxamide
le N-[4-(3-cyanophényl)-5-(2-éthyl-6-méthyl-4-pyridyl)thiazol-2-yl]-2-oxa-6-azaspiro[3.3]heptane-6-carboxamide
le N-[4-(3-cyanophényl)-5-(2,6-diméthyl-1-oxido-pyridin-1-ium-4-yl)thiazol-2-yl]-2-oxa-6-azaspiro[3.3]heptane-6-carboxamide
le 4-cyano-N-[4-(3-cyano-2-méthyl-phényl)-5-(2,6-diméthyl-4-pyridyl)thiazol-2-yl]-4-méthyl-pipéridine-1-carboxamide
le N-[5-(2-acétamido-6-méthyl-4-pyridyl)-4-(3-cyanophényl)thiazol-2-yl]-2-oxa-6-azaspiro[3.3]heptane-6-carboxamide
le N-[5-[2-(acétamidométhyl)-6-méthyl-4-pyridyl]-4-(3-cyanophényl)thiazol-2-yl]-2-oxa-6-azaspiro[3.3]heptane-6-carboxamide
le N-[4-(3-cyanophényl)-5-[2-(méthoxyméthyl)-6-méthyl-4-pyridyl]thiazol-2-yl]-2-oxa-6-azaspiro[3.3]heptane-6-carboxamide
le N-[4-(3-cyanophényl)-5-[2-(1-méthoxyéthyl)-6-méthyl-4-pyridyl]thiazol-2-yl]-2-oxa-6-azaspiro[3.3]heptane-6-carboxamide
le N-[5-[2-chloro-6-(1-hydroxy-1-méthyl-éthyl)-4-pyridyl]-4-(3-cyanophényl)thiazol-2-yl]-2-oxa-6-azaspiro[3.3]heptane-6-carboxamide
le 4-cyano-N-[4-(3-cyanophényl)-5-[2-(hydroxyméthyl)-6-méthyl-4-pyridyl]thiazol-2-yl]-4-méthyl-pipéridine-1-carboxamide
le N-[4-(3-cyanophényl)-5-[2-(hydroxyméthyl)-6-(trifluorométhyl)-4-pyridyl]thiazol-2-yl]-2-oxa-6-azaspiro[3.3]heptane-6-carboxamide
le 3-cyano-N-[4-(3-cyanophényl)-5-(2,6-diméthyl-4-pyridyl)thiazol-2-yl]-3-méthyl-pyrrolidine-1-carboxamide
le N-[5-[2,6-bis(trideutériométhyl)-4-pyridyl]-4-(3-cyanophényl)thiazol-2-yl]-4-cyano-4-méthyl-pipéridine-1-carboxamide
le N-[4-(3-cyanophényl)-5-[2-[(1R)-1-hydroxyéthyl]-6-méthyl-4-pyridyl]thiazol-2-yl]-2-oxa-6-azaspiro[3.3]heptane-6-carboxamide
le N-[4-(3-cyanophényl)-5-[2-[(1S)-1-hydroxyéthyl]-6-méthyl-4-pyridyl]thiazol-2-yl]-2-oxa-6-azaspiro[3.3]heptane-6-carboxamide
le (3R)-3-cyano-N-[4-(3-cyanophényl)-5-(2,6-diméthyl-4-pyridyl)thiazol-2-yl]-3-méthyl-pyrrolidine-1-carboxamide
le (3S)-3-cyano-N-[4-(3-cyanophényl)-5-(2,6-diméthyl-4-pyridyl)thiazol-2-yl]-3-méthyl-pyrrolidine-1-carboxamide.

13. Composition pharmaceutique comprenant un composé selon l'une quelconque des revendications 1 à 12, ou sel pharmaceutiquement acceptable de celui-ci, en mélange avec un diluant ou un support pharmaceutiquement acceptable.

14. Composé selon l'une quelconque des revendications 1 à 12, ou sel pharmaceutiquement acceptable de celui-ci ou composition pharmaceutique selon la revendication 13, destiné(e) à être utilisé(e) en thérapie.

15. Composé selon l'une quelconque des revendications 1 à 12, ou sel pharmaceutiquement acceptable de celui-ci ou composition pharmaceutique selon la revendication 13, destiné(e) à être utilisé(e) :
(i) dans le traitement d'une affection proliférative ;
(ii) dans le traitement d'un cancer ;
(iii) dans le traitement d'un cancer, ledit composé ou ladite composition pharmaceutique étant administré(e) en combinaison avec un ou plusieurs agents anticancéreux supplémentaires ;
(iv) dans le traitement d'un cancer, ledit composé ou ladite composition pharmaceutique étant administré(e) en combinaison avec un ou plusieurs agents anticancéreux supplémentaires choisis dans le groupe constitué par :
1) d'autres formes d'immunothérapie anticancéreuse et d'agents chimiothérapeutiques anticancéreux ;
2) des modulateurs de la voie de l'adénosine, y compris, mais sans s'y limiter, des antagonistes d'A2b, des inhibiteurs de CD73 et des inhibiteurs de CD39 ;
3) des anticorps anti-PD-1 et PDL-1 (par exemple le cétrélimab, le pembrolizumab, le nivolumab, le durvalumab, l'avélumab et l'atézolizumab) ; et
4) des anticorps anti-CTLA4 (par exemple l'ipilimumab).
